# EUROPEAN PATENT APPLICATION

(11) **EP 2 439 280 A1**
(43) Date of publication of application: **11.04.2012**
(21) Application number: 11194324.7
(22) Date of filing: 30.03.2007
(51) Int. Cl.: C12N 15/82, C07K 14/47, C07K 14/415

(54) **Plants having enhanced yield-related traits and a method for making the same**

(30) Priority: 07.04.2006 US 79011606 P; 07.04.2006 US 79015106 P; 14.04.2006 US 79222506 P; 08.05.2006 US 79860206 P; 19.05.2006 US 80168706 P; 15.02.2007 US 88995807 P; 31.03.2006 EP 06075822; 31.03.2006 EP 06075823; 10.04.2006 EP 06075918; 27.04.2006 EP 06075957; 18.05.2006 EP 06114140; 14.07.2006 EP 06117235; 19.01.2007 EP 07100833
(62) Divisional of application: 07727553.5
(71) Applicant: BASF Plant Science GmbH, 67056 Ludwigshafen (DE)
(72) Inventor: Frankard, Valerie, 1410 Waterloo (BE); Reuzeau, Christophe, 24350 La Chapelle Gonaguet (FR); Sanz Molinero, Ana Isabel, 9050 Gentbrugge (BE)
(74) Representative: Mistry, Meeta

(57) **Abstract**

The present invention relates generally to the field of molecular biology and concerns a method for enhancing yield-related traits relative to control plants. More specifically, the present invention concerns a method for increasing plant yield comprising increasing expression of a nucleic acid encoding a PLT transcription factor polypeptide in a plant. The present invention also concerns plants having increased expression of a nucleic acid encoding a PLT transcription factor polypeptide, which plants have increased yield relative to suitable control plants. The invention also provides constructs useful in the methods of the invention.

SEQUENCE LISTING

<110>  CropDesign N.V.

<120>  Plants having enhanced yield-related traits and a method for
       making the same

<130>  PF58328

<160>  301

<170>  PatentIn version 3.3

<210>  1
<211>  630
<212>  DNA
<213>  Arabidopsis thaliana

<400>  1
atggagaatg ggaaaagaga cagacaagac atggaagtga ataccacacc gaggaagcct      60
cgtgtactac tcgctgcaag tggaagcgtc gctgctatca aattcggcaa tctctgccat     120
tgcttcaccg aatgggcaga agtcagagcc gtcgttacga aatcatctct acatttcctc     180
gataaactct ctctcccaca agaagtgact ctgtatactg atgaagatga atggtctagc     240
tggaacaaga tcggtgatcc tgtccttcac atcgagctta gacgttgggc tgatgtttta     300
gtcattgctc ctttgtctgc taacacctta ggcaagattg ctggtgggct ttgtgataat     360
cttctgactt gcattatacg agcttgggac tataccaaac cactgtttgt tgctccagct     420
atgaatactt tgatgtggaa caatcctttc actgaaaggc atcttttgtc tcttgatgaa     480
ctgggaatca cacttattcc tcctatcaag aagagacttg cctgtggaga ctacggtaat     540
ggagctatgg ctgagccctc tcttatctat tccactgtca gactcttctg ggagtctcag     600
gctcatcagc aaaccggtgg aactagttaa                                      630

<210>  2
<211>  209
<212>  PRT
<213>  Arabidopsis thaliana

<400>  2
Met Glu Asn Gly Lys Arg Asp Arg Gln Asp Met Glu Val Asn Thr Thr
1               5                   10                  15
Pro Arg Lys Pro Arg Val Leu Leu Ala Ala Ser Gly Ser Val Ala Ala
            20                  25                  30
Ile Lys Phe Gly Asn Leu Cys His Cys Phe Thr Glu Trp Ala Glu Val
        35                  40                  45
Arg Ala Val Val Thr Lys Ser Ser Leu His Phe Leu Asp Lys Leu Ser
    50                  55                  60
Leu Pro Gln Glu Val Thr Leu Tyr Thr Asp Glu Asp Glu Trp Ser Ser
65                  70                  75                  80
Trp Asn Lys Ile Gly Asp Pro Val Leu His Ile Glu Leu Arg Arg Trp
                85                  90                  95
Ala Asp Val Leu Val Ile Ala Pro Leu Ser Ala Asn Thr Leu Gly Lys
            100                 105                 110
Ile Ala Gly Gly Leu Cys Asp Asn Leu Leu Thr Cys Ile Ile Arg Ala
        115                 120                 125
Trp Asp Tyr Thr Lys Pro Leu Phe Val Ala Pro Ala Met Asn Thr Leu
    130                 135                 140
Met Trp Asn Asn Pro Phe Thr Glu Arg His Leu Leu Ser Leu Asp Glu
145                 150                 155                 160
Leu Gly Ile Thr Leu Ile Pro Pro Ile Lys Lys Arg Leu Ala Cys Gly
                165                 170                 175

Asp Tyr Gly Asn Gly Ala Met Ala Glu Pro Ser Leu Ile Tyr Ser Thr
            180                 185                 190
Val Arg Leu Phe Trp Glu Ser Gln Ala His Gln Gln Thr Gly Gly Thr
        195                 200                 205
Ser

<210>  3
<211>  37
<212>  DNA
<213>  Artificial sequence

<220>
<223>  primer: prm00957

<400>  3
aaaaagcagg ctcacaatgg agaatgggaa aagagac                              37

<210>  4
<211>  33
<212>  DNA
<213>  Artificial sequence

<220>
<223>  primer: prm00958

<400>  4
agaaagctgg gttggtttta actagttcca ccg                                 33

<210>  5
<211>  1243
<212>  DNA
<213>  Oryza sativa

<400>  5
aaaaccaccg agggacctga tctgcaccgg ttttgatagt tgagggaccc gttgtgtctg      60
gttttccgat cgagggacga aaatcggatt cggtgtaaag ttaagggacc tcagatgaac     120
ttattccgga gcatgattgg gaagggagga cataaggccc atgtcgcatg tgtttggacg     180
gtccagatct ccagatcact cagcaggatc ggccgcgttc gcgtagcacc cgcggtttga     240
ttcggcttcc cgcaaggcgg cggccggtgg ccgtgccgcc gtagcttccg ccggaagcga     300
gcacgccgcc gccgccgacc cggctctgcg tttgcaccgc cttgcacgcg atacatcggg     360
atagatagct actactctct ccgtttcaca atgtaaatca ttctactatt ttccacattc     420
atattgatgt taatgaatat agacatatat atctatttag attcattaac atcaatatga     480
atgtaggaaa tgctagaatg acttacattg tgaattgtga aatggacgaa gtacctacga     540
tggatggatg caggatcatg aaagaattaa tgcaagatcg tatctgccgc atgcaaaatc     600
ttactaattg cgctgcatat atgcatgaca gcctgcatgc gggcgtgtaa gcgtgttcat     660
ccattaggaa gtaaccttgt cattacttat accagtacta catactatat agtattgatt     720
tcatgagcaa atctacaaaa ctggaaagca ataaggaata cgggactgga aaagactcaa     780
cattaatcac caaatatttc gccttctcca gcagaatata tatctctcca tcttgatcac     840
tgtacacact gacagtgtac gcataaacgc agcagccagc ttaactgtcg tctcaccgtc     900
gcacactggc cttccatctc aggctagctt tctcagccac ccatcgtaca tgtcaactcg     960
gcgcgcgcac aggcacaaat tacgtacaaa acgcatgacc aaatcaaaac caccggagaa    1020
gaatcgctcc cgcgcgcggc ggcggcgcgc acgtacgaat gcacgcacgc acgcccaacc    1080
ccacgacacg atcgcgcgcg acgccggcga caccggccat ccacccgcgc cctcacctcg    1140
ccgactataa atacgtaggc atctgcttga tcttgtcatc catctcacca ccaaaaaaaa    1200
aggaaaaaaa aacaaaacac accaagccaa ataaaagcga caa                      1243

<210>  6
<211>  34

<212>  PRT
<213>  Artificial sequence

<220>
<223>  conserved motif 1

<220>
<221>  VARIANT
<222>  (1)..(1)
<223>  /replace = "Arg"

<220>
<221>  VARIANT
<222>  (4)..(4)
<223>  /replace = "Ile"

<220>
<221>  VARIANT
<222>  (9)..(9)
<223>  /replace = "Thr"

<220>
<221>  VARIANT
<222>  (14)..(14)
<223>  /replace = "Ser"

<220>
<221>  VARIANT
<222>  (15)..(15)
<223>  /replace = "Met" /replace = "Val"

<220>
<221>  VARIANT
<222>  (18)..(18)
<223>  /replace = "Glu" /replace = "Ala" /replace = "Ser"

<220>
<221>  VARIANT
<222>  (21)..(21)
<223>  /replace = "Val"

<220>
<221>  VARIANT
<222>  (22)..(22)
<223>  /replace = "Arg" /replace = "Gly"

<220>
<221>  VARIANT
<222>  (23)..(23)
<223>  /replace = "Ser" /replace = "Ile"

<220>
<221>  VARIANT
<222>  (25)..(25)
<223>  /replace = "Ser"

<220>
<221>  VARIANT
<222>  (26)..(26)

<223>  /replace = "Asp"

<220>
<221>  VARIANT
<222>  (29)..(29)
<223>  /replace = "Asp"

<220>
<221>  VARIANT
<222>  (31)..(31)
<223>  /replace = "Lys"

<220>
<221>  VARIANT
<222>  (34)..(34)
<223>  /replace = "Ala"

<400>  6
Lys Pro Arg Val Leu Leu Ala Ala Ser Gly Ser Val Ala Ala Ile Lys
1               5                   10                  15
Phe Gly Asn Leu Cys His Cys Phe Thr Glu Trp Ala Glu Val Arg Ala
            20                  25                  30
Val Val


<210>  7
<211>  98
<212>  PRT
<213>  Artificial sequence

<220>
<223>  conserved motif 2

<220>
<221>  VARIANT
<222>  (8)..(8)
<223>  /replace = "Lys" /replace = "Gln"

<220>
<221>  VARIANT
<222>  (12)..(12)
<223>  /replace = "Ile"

<220>
<221>  VARIANT
<222>  (13)..(13)
<223>  /replace = "Met"

<220>
<221>  VARIANT
<222>  (14)..(14)
<223>  /replace = "Ile"

<220>
<221>  VARIANT
<222>  (24)..(24)
<223>  /replace = "Ala"

<220>

<221>  VARIANT
<222>  (30)..(30)
<223>  /replace = "Met"

<220>
<221>  VARIANT
<222>  (38)..(38)
<223>  /replace = "Val"

<220>
<221>  VARIANT
<222>  (39)..(39)
<223>  /replace = "Val"

<220>
<221>  VARIANT
<222>  (44)..(44)
<223>  /replace = "Phe"

<220>
<221>  VARIANT
<222>  (45)..(45)
<223>  /replace = "Ser" /replace = "Asn" /replace = "Asp" /replace =
      "Lys"

<220>
<221>  VARIANT
<222>  (48)..(48)
<223>  /replace = "Phe" /replace = "Met" /replace = "Ile"

<220>
<221>  VARIANT
<222>  (50)..(50)
<223>  /replace = "Ala"

<220>
<221>  VARIANT
<222>  (57)..(57)
<223>  /replace = "Phe"

<220>
<221>  VARIANT
<222>  (60)..(60)
<223>  /replace = "Ser"

<220>
<221>  VARIANT
<222>  (65)..(65)
<223>  /replace = "Ser" /replace = "Gln" /replace = "Ala"

<220>
<221>  VARIANT
<222>  (66)..(66)
<223>  /replace = "Lys"

<220>
<221>  VARIANT
<222>  (68)..(68)
<223>  /replace = "Phe" /replace = "Ile"

<220>
<221> UNSURE
<222> (69)..(70)
<223> Xaa can be any naturally occurring amino acid

<220>
<221> VARIANT
<222> (71)..(71)
<223> /replace = "Ile" /replace = "Met"

<220>
<221> VARIANT
<222> (72)..(72)
<223> /replace = "Asn" /replace = "Ser"

<220>
<221> VARIANT
<222> (73)..(73)
<223> /replace = "Leu" /replace = "Gln"

<220>
<221> VARIANT
<222> (74)..(74)
<223> /replace = "Met"

<220>
<221> VARIANT
<222> (76)..(76)
<223> /replace = "Val"

<220>
<221> VARIANT
<222> (77)..(77)
<223> /replace = "Ser" /replace = "Ala" /replace = "Ile"

<220>
<221> VARIANT
<222> (79)..(79)
<223> /replace = "Val"

<220>
<221> VARIANT
<222> (82)..(82)
<223> /replace = "Val" /replace = "Thr"

<220>
<221> VARIANT
<222> (83)..(83)
<223> /replace = "Thr" /replace = "Ser"

<220>
<221> VARIANT
<222> (85)..(85)
<223> /replace = "Thr" /replace = "Lys"

<220>
<221> VARIANT
<222> (91)..(91)

<223>  /replace = "His"

<220>
<221>  VARIANT
<222>  (93)..(93)
<223>  /replace = "Thr"

<220>
<221>  VARIANT
<222>  (97)..(97)
<223>  /replace = "Ser"

<400>  7
Val Leu His Ile Glu Leu Arg Arg Trp Ala Asp Val Leu Val Ile Ala
1               5                   10                  15
Pro Leu Ser Ala Asn Thr Leu Gly Lys Ile Ala Gly Gly Leu Cys Asp
            20                  25                  30
Asn Leu Leu Thr Cys Ile Ile Arg Ala Trp Asp Tyr Thr Lys Pro Leu
        35                  40                  45
Phe Val Ala Pro Ala Met Asn Thr Leu Met Trp Asn Asn Pro Phe Thr
    50                  55                  60
Glu Arg His Leu Xaa Xaa Leu Asp Glu Leu Gly Ile Thr Leu Ile Pro
65                  70                  75                  80
Pro Ile Lys Lys Arg Leu Ala Cys Gly Asp Tyr Gly Asn Gly Ala Met
                85                  90                  95
Ala Glu

<210>  8
<211>  171
<212>  PRT
<213>  Arabidopsis thaliana

<400>  8
Lys Pro Arg Val Leu Leu Ala Ala Ser Gly Ser Val Ala Ala Ile Lys
1               5                   10                  15
Phe Gly Asn Leu Cys His Cys Phe Thr Glu Trp Ala Glu Val Arg Ala
            20                  25                  30
Val Val Thr Lys Ser Ser Leu His Phe Leu Asp Lys Leu Ser Leu Pro
        35                  40                  45
Gln Glu Val Thr Leu Tyr Thr Asp Glu Asp Glu Trp Ser Ser Trp Asn
    50                  55                  60
Lys Ile Gly Asp Pro Val Leu His Ile Glu Leu Arg Arg Trp Ala Asp
65                  70                  75                  80
Val Leu Val Ile Ala Pro Leu Ser Ala Asn Thr Leu Gly Lys Ile Ala
                85                  90                  95
Gly Gly Leu Cys Asp Asn Leu Leu Thr Cys Ile Ile Arg Ala Trp Asp
            100                 105                 110
Tyr Thr Lys Pro Leu Phe Val Ala Pro Ala Met Asn Thr Leu Met Trp
        115                 120                 125
Asn Asn Pro Phe Thr Glu Arg His Leu Leu Ser Leu Asp Glu Leu Gly
    130                 135                 140
Ile Thr Leu Ile Pro Pro Ile Lys Lys Arg Leu Ala Cys Gly Asp Tyr
145                 150                 155                 160
Gly Asn Gly Ala Met Ala Glu Pro Ser Leu Ile
                165                 170

<210>  9
<211>  606

<212>  DNA
<213>  Arabidopsis thaliana

<400>  9
atgaatatgg aagtggatac agtaacaagg aagcctcgta tcttactagc tgcaagtgga      60
agtgtggctt caattaagtt cagtaatctc tgccattgtt tctcagaatg ggctgaagtc     120
aaagccgtcg cttcaaaatc atctctcaat ttcgttgata aaccttctct acctcagaat     180
gtgactctct atacagatga agatgaatgg tctagctgga acaagattgg tgatcccgtt     240
cttcatatcg agctcagacg ctgggctgat gttatgatca ttgctccttt gtctgctaac     300
acattagcca agattgctgg tgggttatgt gataatctat tgacatgtat agtaagagca     360
tgggattata gcaaaccgtt gtttgttgca ccggcgatga acactttgat gtggaacaat     420
cctttcacag aacggcacct tgtcttgctt gatgaacttg gaatcaccct aattcctccc     480
atcaagaaga aactggcctg tggagactac ggtaatggcg caatggctga gccttctctg     540
atttattcca ctgttagact gttctgggag tcacaagctc gtaaacaaag agatggaacc     600
agttga                                                                606

<210>  10
<211>  201
<212>  PRT
<213>  Arabidopsis thaliana

<400>  10
Met Asn Met Glu Val Asp Thr Val Thr Arg Lys Pro Arg Ile Leu Leu
1               5                   10                  15
Ala Ala Ser Gly Ser Val Ala Ser Ile Lys Phe Ser Asn Leu Cys His
            20                  25                  30
Cys Phe Ser Glu Trp Ala Glu Val Lys Ala Val Ala Ser Lys Ser Ser
        35                  40                  45
Leu Asn Phe Val Asp Lys Pro Ser Leu Pro Gln Asn Val Thr Leu Tyr
    50                  55                  60
Thr Asp Glu Asp Glu Trp Ser Ser Trp Asn Lys Ile Gly Asp Pro Val
65                  70                  75                  80
Leu His Ile Glu Leu Arg Arg Trp Ala Asp Val Met Ile Ile Ala Pro
                85                  90                  95
Leu Ser Ala Asn Thr Leu Ala Lys Ile Ala Gly Gly Leu Cys Asp Asn
            100                 105                 110
Leu Leu Thr Cys Ile Val Arg Ala Trp Asp Tyr Ser Lys Pro Leu Phe
        115                 120                 125
Val Ala Pro Ala Met Asn Thr Leu Met Trp Asn Asn Pro Phe Thr Glu
    130                 135                 140
Arg His Leu Val Leu Leu Asp Glu Leu Gly Ile Thr Leu Ile Pro Pro
145                 150                 155                 160
Ile Lys Lys Lys Leu Ala Cys Gly Asp Tyr Gly Asn Gly Ala Met Ala
                165                 170                 175
Glu Pro Ser Leu Ile Tyr Ser Thr Val Arg Leu Phe Trp Glu Ser Gln
            180                 185                 190
Ala Arg Lys Gln Arg Asp Gly Thr Ser
        195                 200

<210>  11
<211>  1128
<212>  DNA
<213>  Oryza sativa

<400>  11
caaccaaagt ccaaaggcta ttctgaaccg aagtcctccc acaccaaaac ttcgaggccc      60
ccgtcgcggc accctcctcc gccgccggat ctccaccgga gtacggcgcc ggccaccccc     120
tcctcccccg gatcttcacc gccggtgaag tactgaggtg atgactacat cagagtcagt     180
acaagaaacc ttgggattgg atttccctca tcctagcaaa cctcgggtcc tccttgctgc     240

ctctggaagc gtcgctgcta taaaatttga gagcctttgc cgtagcttct cggaatgggc 300
agaagtcaga gccgtcgcca ccaaggcttc attacatttt attgatagaa cgtctctgcc 360
tagcaatatt attctttaca ctgatgatga tgaatggtct acctggaaga agatagggga 420
tgaagttttg cacattgaac tgcgaaaatg ggcagatatc atggtgattg cgccattatc 480
agctaatacc ctagctaaga ttgctggtgg tttatgtgac aacctcttga catgcatagt 540
gagagcatgg gactacagca aaccactctt tgttgcccca gctatgaaca ccttcatgtg 600
gaacaacccg ttcaccagtc gtcatcttga gacaatcaac ctgctaggta tatctttggt 660
ccctcccatt accaaaaggc tggcctgtgg tgattatggt aatggtgcaa tggctgagcc 720
ttctgtgatc gattccaccg tcaggcttgc ttgcaagaga cagccactta atacaaatag 780
ttcacctgtg gttcctgccg gcagaaacct cccatctagc tgatgcggca actattctgt 840
tcaagattaa actctggacc tagttttcta tggtaaagag tacttcgtgt cacaaaatga 900
aatgttaagt gatgtctatg tcggccaaca tagcaccttt attggccagt tgttgtacta 960
ctattagtga tatggtagga cgtggagatt ggagaaaggc tattgtccca gcactttaat 1020
gttgcttttc caaatttctt gtgacataat gctaaggtgc tgatgaatat gttcatgttg 1080
tagcactatt tttttctgca aatgtttgca aagactcgtg atggaatc 1128

<210> 12
<211> 220
<212> PRT
<213> Oryza sativa

<400> 12
Met Thr Thr Ser Glu Ser Val Gln Glu Thr Leu Gly Leu Asp Phe Pro
1               5                   10                  15
His Pro Ser Lys Pro Arg Val Leu Leu Ala Ala Ser Gly Ser Val Ala
            20                  25                  30
Ala Ile Lys Phe Glu Ser Leu Cys Arg Ser Phe Ser Glu Trp Ala Glu
        35                  40                  45
Val Arg Ala Val Ala Thr Lys Ala Ser Leu His Phe Ile Asp Arg Thr
    50                  55                  60
Ser Leu Pro Ser Asn Ile Ile Leu Tyr Thr Asp Asp Asp Glu Trp Ser
65                  70                  75                  80
Thr Trp Lys Lys Ile Gly Asp Glu Val Leu His Ile Glu Leu Arg Lys
                85                  90                  95
Trp Ala Asp Ile Met Val Ile Ala Pro Leu Ser Ala Asn Thr Leu Ala
            100                 105                 110
Lys Ile Ala Gly Gly Leu Cys Asp Asn Leu Leu Thr Cys Ile Val Arg
        115                 120                 125
Ala Trp Asp Tyr Ser Lys Pro Leu Phe Val Ala Pro Ala Met Asn Thr
    130                 135                 140
Phe Met Trp Asn Asn Pro Phe Thr Ser Arg His Leu Glu Thr Ile Asn
145                 150                 155                 160
Leu Leu Gly Ile Ser Leu Val Pro Pro Ile Thr Lys Arg Leu Ala Cys
                165                 170                 175
Gly Asp Tyr Gly Asn Gly Ala Met Ala Glu Pro Ser Val Ile Asp Ser
            180                 185                 190
Thr Val Arg Leu Ala Cys Lys Arg Gln Pro Leu Asn Thr Asn Ser Ser
        195                 200                 205
Pro Val Val Pro Ala Gly Arg Asn Leu Pro Ser Ser
    210                 215                 220

<210> 13
<211> 1164
<212> DNA
<213> Triticum aestivum

<400> 13
gcacgagcag ccgcggagcc gccagagaca gacgctgccg ccgtcggccg tcctccgccc 60
tccagttgag cggttcaagc agagctgatg gctacatcag agccagtaca agagagtttg 120

gtggtgcact actcacaacc tagtaggccc cgggtcctcc ttgctgcttc aggaagtgta 180
gccgctataa aatttgagag cctttgccgt agcttctctg agtggcggga tgtccgagct 240
gtggccacca cgccatcctt gcacttcgtt gatagatcat ctctaccaag tggcatcgtt 300
ctttacactg atgacgatga atggtctacc tggaagaaga taggagatga agtcttacac 360
atcgagctgc ggaaatgggc agacgttatg gtgatcgctc cattgtcagc aaataccctg 420
gctaagatcg ccggtgggtt atgcgacaac ctcttgacct gcatcgtgag agcgtgggac 480
tacagcaaac cgctctttgt tgccccagcc atgaacacgt taatgtggaa caacccattc 540
acggagcggc atcttcacac aatcaaccaa ctgggcatag ccttgatccc cccagttacc 600
aaaaggctgg cctgtggcga ttacggaaac ggcgcaatgg ccgaaacctc gcagatccat 660
acttccgtga ggctcgcgtg caagacgcaa ccgcacgatg cgagcagttc actcgcgggt 720
cctgtcagta ataaccggcc atctagctga tgcagcagtt ggccacttga ttgtcaagct 780
taggaatttg ttttatatgc agtgtgcatc tggagtgttg taacagattt tttttccaac 840
tagtgtttgt gtgtattgaa attggggggg aaaggctgtt gtcacaggat aacaataact 900
tcctccctgc tcagtaatta tgagtctatt cagtttgtaa ttgtcggtgg gagtacaatt 960
atgctacaat attgtttgtt tgtgtgtgtg tgggagttgg ggagtgctgc tgccacagag 1020
atagcttcct ccctgcccag ccagtaatga tagtgattat tcagtttgta gttgtcagtg 1080
gaagtgtcca gcaaactttt ctactaccct ttgatattcc tggtacaaat gttggaacga 1140
ctgtcctaaa aaaaaaaaaa aaaa 1164

<210> 14
<211> 220
<212> PRT
<213> Triticum aestivum

<400> 14
Met Ala Thr Ser Glu Pro Val Gln Glu Ser Leu Val Val His Tyr Ser
1               5                   10                  15
Gln Pro Ser Arg Pro Arg Val Leu Leu Ala Ala Ser Gly Ser Val Ala
            20                  25                  30
Ala Ile Lys Phe Glu Ser Leu Cys Arg Ser Phe Ser Glu Trp Ala Asp
        35                  40                  45
Val Arg Ala Val Ala Thr Thr Pro Ser Leu His Phe Val Asp Arg Ser
    50                  55                  60
Ser Leu Pro Ser Gly Ile Val Leu Tyr Thr Asp Asp Asp Glu Trp Ser
65                  70                  75                  80
Thr Trp Lys Lys Ile Gly Asp Glu Val Leu His Ile Glu Leu Arg Lys
                85                  90                  95
Trp Ala Asp Val Met Val Ile Ala Pro Leu Ser Ala Asn Thr Leu Ala
            100                 105                 110
Lys Ile Ala Gly Gly Leu Cys Asp Asn Leu Leu Thr Cys Ile Val Arg
        115                 120                 125
Ala Trp Asp Tyr Ser Lys Pro Leu Phe Val Ala Pro Ala Met Asn Thr
    130                 135                 140
Leu Met Trp Asn Asn Pro Phe Thr Glu Arg His Leu His Thr Ile Asn
145                 150                 155                 160
Gln Leu Gly Ile Ala Leu Ile Pro Pro Val Thr Lys Arg Leu Ala Cys
                165                 170                 175
Gly Asp Tyr Gly Asn Gly Ala Met Ala Glu Thr Ser Gln Ile His Thr
            180                 185                 190
Ser Val Arg Leu Ala Cys Lys Thr Gln Pro His Asp Ala Ser Ser Ser
        195                 200                 205
Leu Ala Gly Pro Val Ser Asn Asn Arg Pro Ser Ser
    210                 215                 220

<210> 15
<211> 1165
<212> DNA
<213> Zea mays

<400> 15
gcacgaggtc agatcgacct ggactttggc ggcagctact tctagaacgc ggcggcacgg 60
cagcaagcca gcaaggagaa cgcggcggca cgggcgtgct gctacttctg cttcctctcc 120
gacgcttgct gttgagcggt tcaagcagag ctgatggcta catcagagcc agtacaagag 180
agtttggtgg tgcactactc acaacctagt aggccccggg tcctccttgc tgcttcagga 240
agtgtagccg ctataaaatt tgagagcctt tgccgtagct tctctgagtg ggcggatgtc 300
cgagctgtgg ccaccacgcc atccttgcac ttcgttgata gatcatctct accaagtggc 360
atcgttcttt acactgatga cgatgaatgg tctacctgga agaagatagg agatgaagtc 420
ttacacatcg agctgcggaa atgggcagac gttatggtga tcgctccatt gtcagcaaat 480
accctggcta agatcgccgg tgggttatgc gacaacctct tgacctgcat cgtgagagcg 540
tgggactaca gcaaaccgct ctttgttgcc ccagccatga acacgttaat gtggaacaac 600
ccattcacgg agcggcatct tcacacaatc aaccaactgg gcatagcctt gatcccccca 660
gttaccaaaa ggctggcctg tggcgattac gggaacggcg caatggccga aacctcgcag 720
atccatactt ccgtgaggct cgcgtgcaag acgcaaccgc acgatgcgag cagttcactc 780
gcgggtcctg tcagtaataa ccggccatct agctgatgca gcagttggcc acttgattgt 840
caagcttagg aatttgtttt atatgcagtg tgcatctgga gtgttgtaac agattttttt 900
tccaactagt gtttgtgtgt attgaaattg ggggggaaag gctgttgtca caggataaca 960
ataacttcct ccctgctcag taattatgag tctattcagt ttgtaattgt cggtgggagt 1020
acaattatgc tacaatattg tttgtttgtg tgtgtgtggg agttggggag tgctgctgcc 1080
acagagatag cttcctccct gcccagccag taatgatagt gattattcag tttgtaaaaa 1140
aaaaaaaaaa aaaaaaaaaa aaaaa 1165

<210> 16
<211> 220
<212> PRT
<213> Zea mays

<400> 16
Met Ala Thr Ser Glu Pro Val Gln Glu Ser Leu Val Val His Tyr Ser
1               5                   10                  15
Gln Pro Ser Arg Pro Arg Val Leu Leu Ala Ala Ser Gly Ser Val Ala
            20                  25                  30
Ala Ile Lys Phe Glu Ser Leu Cys Arg Ser Phe Ser Glu Trp Ala Asp
        35                  40                  45
Val Arg Ala Val Ala Thr Thr Pro Ser Leu His Phe Val Asp Arg Ser
    50                  55                  60
Ser Leu Pro Ser Gly Ile Val Leu Tyr Thr Asp Asp Asp Glu Trp Ser
65                  70                  75                  80
Thr Trp Lys Lys Ile Gly Asp Glu Val Leu His Ile Glu Leu Arg Lys
                85                  90                  95
Trp Ala Asp Val Met Val Ile Ala Pro Leu Ser Ala Asn Thr Leu Ala
            100                 105                 110
Lys Ile Ala Gly Gly Leu Cys Asp Asn Leu Leu Thr Cys Ile Val Arg
        115                 120                 125
Ala Trp Asp Tyr Ser Lys Pro Leu Phe Val Ala Pro Ala Met Asn Thr
    130                 135                 140
Leu Met Trp Asn Asn Pro Phe Thr Glu Arg His Leu His Thr Ile Asn
145                 150                 155                 160
Gln Leu Gly Ile Ala Leu Ile Pro Pro Val Thr Lys Arg Leu Ala Cys
                165                 170                 175
Gly Asp Tyr Gly Asn Gly Ala Met Ala Glu Thr Ser Gln Ile His Thr
            180                 185                 190
Ser Val Arg Leu Ala Cys Lys Thr Gln Pro His Asp Ala Ser Ser Ser
        195                 200                 205
Leu Ala Gly Pro Val Ser Asn Asn Arg Pro Ser Ser
    210                 215                 220

<210> 17
<211> 631

<212>  DNA
<213>  Picea abies

<220>
<221>  misc_feature
<222>  (546)..(546)
<223>  n is a, c, g, or t

<400>  17
ggacgcgtgg gtactgtact gcatatagaa ctccggcaat gggctgatgc tatggtgatt      60
gctccactat cagcaaacac gcttgctaag atagcaggtg gcctatgcga caatctactg     120
acttgcataa tacgtgcatg ggactttaac aagcctctct ttgtagctcc tgcaatgaat     180
actttcatgt ggaacaaccc atttactcaa cggcatttgg actctatctc ggagatggga     240
gtatcactta tcccccctat aacaaagacg ttagcttgtg gtgattatgg aaatggtgca     300
atgtcagaac cttcctcaat agatacaact cttaggtttt cactcgatcc ttcaattaaa     360
tgaagtatct gatctccctt tctccaattc ttgttatgaa tctaacatgc ttagtactat     420
ggactgtggt atagactcag attcgtagct gggctagaca gcatgaaccc agctcgtgtg     480
tggctatgtt aagaaattct gatgataaac aatctcgaac ttatgattta tgtaacattt     540
gttccnccat tctggacact tacaagaact gtgtggatac tcctgcttat aagaaaatgt     600
gtcaatctac attgcatatc atctaatttg g                                    631

<210>  18
<211>  120
<212>  PRT
<213>  Picea abies

<400>  18
Gly Arg Val Gly Thr Val Leu His Ile Glu Leu Arg Gln Trp Ala Asp
1               5                   10                  15
Ala Met Val Ile Ala Pro Leu Ser Ala Asn Thr Leu Ala Lys Ile Ala
            20                  25                  30
Gly Gly Leu Cys Asp Asn Leu Leu Thr Cys Ile Ile Arg Ala Trp Asp
        35                  40                  45
Phe Asn Lys Pro Leu Phe Val Ala Pro Ala Met Asn Thr Phe Met Trp
    50                  55                  60
Asn Asn Pro Phe Thr Gln Arg His Leu Asp Ser Ile Ser Glu Met Gly
65                  70                  75                  80
Val Ser Leu Ile Pro Pro Ile Thr Lys Thr Leu Ala Cys Gly Asp Tyr
                85                  90                  95
Gly Asn Gly Ala Met Ser Glu Pro Ser Ser Ile Asp Thr Thr Leu Arg
            100                 105                 110
Phe Ser Leu Asp Pro Ser Ile Lys
        115                 120

<210>  19
<211>  769
<212>  DNA
<213>  Brassica napus

<400>  19
gagatctgta agggttttca aaaagagcca tggagaacgg gaaaagagac agagaagaca      60
tggaagtgca gctctcccct agaaagcccc gcgtactcct cgcagcaacc ggaagcgtcg     120
ccgccatcaa attcggcaac ctctgccact gcttcacaga gtgggcggaa gtgagagccg     180
tcgtctcgaa atcgtctctc cacttcctcg acaagctctc tctcccacag gaagtgactc     240
tctacaccga cgaagacgag tggtcgagct ggaacaagat cggcgatccc gtgcttcaca     300
tcgagctcag acgctgggct gacgtcatgg tcatcgctcc tttgtctgct aacactttag     360
ccaagatagc tggtgggatg tgtgataatc ttctgacttg tatcataaga gcttgggatt     420
atagcaaacc gcttttcgtt gcgccggcta tgaatacttt gatgtggaac aatcctttta     480
cggagaggca tcttttgtcg cttgatgagc ttggaatcac tcttattcct ccgatcaaga     540

agaggttggc ttgtggtgac tatggtaatg gcgcgatggc tgagccgtct cttatctatt 600
ccactgttag actcttctgg gagtctcagg ctcatcagca aagtggtgga actagttaat 660
accatgatgg ttttgcactt tgcaatggtt ggtcagtgtc atagattgtt ctgtctgaaa 720
tgctcgtcct tgtatatgtt aagaacagct gtctgggttg atgtctctt 769

<210> 20
<211> 209
<212> PRT
<213> Brassica napus

<400> 20
Met Glu Asn Gly Lys Arg Asp Arg Glu Asp Met Glu Val Gln Leu Ser
1               5                   10                  15
Pro Arg Lys Pro Arg Val Leu Leu Ala Ala Thr Gly Ser Val Ala Ala
            20                  25                  30
Ile Lys Phe Gly Asn Leu Cys His Cys Phe Thr Glu Trp Ala Glu Val
        35                  40                  45
Arg Ala Val Val Ser Lys Ser Ser Leu His Phe Leu Asp Lys Leu Ser
    50                  55                  60
Leu Pro Gln Glu Val Thr Leu Tyr Thr Asp Glu Asp Glu Trp Ser Ser
65                  70                  75                  80
Trp Asn Lys Ile Gly Asp Pro Val Leu His Ile Glu Leu Arg Arg Trp
                85                  90                  95
Ala Asp Val Met Val Ile Ala Pro Leu Ser Ala Asn Thr Leu Ala Lys
            100                 105                 110
Ile Ala Gly Gly Met Cys Asp Asn Leu Leu Thr Cys Ile Ile Arg Ala
        115                 120                 125
Trp Asp Tyr Ser Lys Pro Leu Phe Val Ala Pro Ala Met Asn Thr Leu
    130                 135                 140
Met Trp Asn Asn Pro Phe Thr Glu Arg His Leu Leu Ser Leu Asp Glu
145                 150                 155                 160
Leu Gly Ile Thr Leu Ile Pro Pro Ile Lys Lys Arg Leu Ala Cys Gly
                165                 170                 175
Asp Tyr Gly Asn Gly Ala Met Ala Glu Pro Ser Leu Ile Tyr Ser Thr
            180                 185                 190
Val Arg Leu Phe Trp Glu Ser Gln Ala His Gln Gln Ser Gly Gly Thr
        195                 200                 205
Ser

<210> 21
<211> 636
<212> DNA
<213> Brassica oleracea var. alboglabra

<220>
<221> misc_feature
<222> (622)..(622)
<223> n is a, c, g, or t

<400> 21
gacgcgtggg cggacgcgtg gggggttttc aaaaagagcc atggagaacg ggaaaagaga 60
cagagaagac atggaagtgc agctctcccc tagaaagccc cgcgtactcc tcgcagcaac 120
cggaagcgtc gccgccatca aattcggcaa cctctgccac tgcttcacag agtgggcgga 180
agtgagagcc gtcgtctcga aatcgtctct ccacttcctc gacaagctct ctctcccaca 240
ggaagtgact ctctacaccg acgaagacga gtggtcgagc tggaacaaga tcggcgatcc 300
cgtgcttcac atcgagctca gacgctgggc tgacgtcatg gtcatcgctc ctttgtctgc 360
taacacttta gccaagatag ctggtgggat gtgtgataat cttctgactt gtatcataag 420
agcttgggat tatagcaaac cgcttttcgt tgcgccggct atgaatactt tgatgtggaa 480

caatcctttt acggagaggc atcttttgtc gcttgatgag cttggaatca ctcttattcc 540
tccgatcaag aagaggttgg cttgtggtga ctatggtaat ggcgcgatgg ctgagccgtc 600
tcttatctat tccactgtta gnctcttctg ggagtc 636

<210> 22
<211> 198
<212> PRT
<213> Brassica oleracea var. alboglabra

<220>
<221> UNSURE
<222> (194)..(194)
<223> Xaa can be any naturally occurring amino acid

<400> 22
Met Glu Asn Gly Lys Arg Asp Arg Glu Asp Met Glu Val Gln Leu Ser
1               5                   10                  15
Pro Arg Lys Pro Arg Val Leu Leu Ala Ala Thr Gly Ser Val Ala Ala
            20                  25                  30
Ile Lys Phe Gly Asn Leu Cys His Cys Phe Thr Glu Trp Ala Glu Val
        35                  40                  45
Arg Ala Val Val Ser Lys Ser Ser Leu His Phe Leu Asp Lys Leu Ser
    50                  55                  60
Leu Pro Gln Glu Val Thr Leu Tyr Thr Asp Glu Asp Glu Trp Ser Ser
65                  70                  75                  80
Trp Asn Lys Ile Gly Asp Pro Val Leu His Ile Glu Leu Arg Arg Trp
                85                  90                  95
Ala Asp Val Met Val Ile Ala Pro Leu Ser Ala Asn Thr Leu Ala Lys
            100                 105                 110
Ile Ala Gly Gly Met Cys Asp Asn Leu Leu Thr Cys Ile Ile Arg Ala
        115                 120                 125
Trp Asp Tyr Ser Lys Pro Leu Phe Val Ala Pro Ala Met Asn Thr Leu
    130                 135                 140
Met Trp Asn Asn Pro Phe Thr Glu Arg His Leu Leu Ser Leu Asp Glu
145                 150                 155                 160
Leu Gly Ile Thr Leu Ile Pro Pro Ile Lys Lys Arg Leu Ala Cys Gly
                165                 170                 175
Asp Tyr Gly Asn Gly Ala Met Ala Glu Pro Ser Leu Ile Tyr Ser Thr
            180                 185                 190
Val Xaa Leu Phe Trp Glu
        195

<210> 23
<211> 958
<212> DNA
<213> Nicotiana tabacum

<400> 23
ggagctgtgt tgccgcagaa ggagcaagaa ttgttggtgc ttcaaggata attggtgttg 60
atttaattcc tagcagattt gatttagatt agggtagata tggagacttc agagatggaa 120
ccggttcaga ttaacaatgc accgaggaga cctcgcattc tgcttgcagc aagtggaagt 180
gtggctgcta tcaagtttgc cagtctatgc cgttccttta ctgattgggc tgaagttaaa 240
gcggttgcta caaaagcttc tcttcatttc atagacaaag cttcacttcc ggaagatgtc 300
attctttata ctgatgagga tgaatggtca acttggacga agataggtga ccgtgtgcta 360
cacatcgagc tccggaggtg ggctgatatt atgattattg cccctttgtc agcaaataca 420
cttgggaaga ttgctggtgg actatgtgat aacttgttaa cctgcatcgt acgagcatgg 480
gactacgata aacccctttt cgtggcacca gcaatgaata cattgatgtg gaataatcca 540
ttcacagaaa agcaccttat ggcaattgat gagcttggga tctctctcat accaccagta 600
tcaaagagac tagcctgtgg agattacggg aatggagcaa tggctgaacc gtctctgatc 660

ttccaagctg taagactcta ttatgacgca caattacgat caggtggcag caacgtggcg 720
tgatccacag gtcatgaaat ttcattcatc ggtttgtaca agtgatagaa gttgttgaaa 780
ttcaggacca ggagcccagc tgtgttattt ttctcctaaa ttcttcacct ccctagtatc 840
tttcttgtgt cctagagcta ctttcaatca aggttcatgt tcattttagt cgataaaatg 900
agaatatcac gtaactgctg ttactaatgg atgtgatcat gatcatgaac atgaaaaa 958

<210> 24
<211> 207
<212> PRT
<213> Nicotiana tabacum

<400> 24
Met Glu Thr Ser Glu Met Glu Pro Val Gln Ile Asn Asn Ala Pro Arg
1               5                   10                  15
Arg Pro Arg Ile Leu Leu Ala Ala Ser Gly Ser Val Ala Ala Ile Lys
            20                  25                  30
Phe Ala Ser Leu Cys Arg Ser Phe Thr Asp Trp Ala Glu Val Lys Ala
        35                  40                  45
Val Ala Thr Lys Ala Ser Leu His Phe Ile Asp Lys Ala Ser Leu Pro
    50                  55                  60
Glu Asp Val Ile Leu Tyr Thr Asp Glu Asp Glu Trp Ser Thr Trp Thr
65                  70                  75                  80
Lys Ile Gly Asp Arg Val Leu His Ile Glu Leu Arg Arg Trp Ala Asp
                85                  90                  95
Ile Met Ile Ile Ala Pro Leu Ser Ala Asn Thr Leu Gly Lys Ile Ala
            100                 105                 110
Gly Gly Leu Cys Asp Asn Leu Leu Thr Cys Ile Val Arg Ala Trp Asp
        115                 120                 125
Tyr Asp Lys Pro Leu Phe Val Ala Pro Ala Met Asn Thr Leu Met Trp
    130                 135                 140
Asn Asn Pro Phe Thr Glu Lys His Leu Met Ala Ile Asp Glu Leu Gly
145                 150                 155                 160
Ile Ser Leu Ile Pro Pro Val Ser Lys Arg Leu Ala Cys Gly Asp Tyr
                165                 170                 175
Gly Asn Gly Ala Met Ala Glu Pro Ser Leu Ile Phe Gln Ala Val Arg
            180                 185                 190
Leu Tyr Tyr Asp Ala Gln Leu Arg Ser Gly Gly Ser Asn Val Ala
        195                 200                 205

<210> 25
<211> 1016
<212> DNA
<213> Solanum tuberosum

<400> 25
gaattgatcg gcgccggact tcgatcaccg tcggcgtttc taagccgtcg ccggaactaa 60
gccggagaaa atctcaatca agtgagctaa gcactcggcg tttcaacttc tggttataaa 120
tttattaaag cctttgctcc attaggttag gtagatacgg atcctatgac ttcagagatg 180
gaaccagttc agattaatgg tgcacctagg agacctcgta ttctgctggc agcaagtgga 240
agtgtggctg caattaagtt tgcaaatcta tgtggttgtt tttctgaatg ggcagaagtt 300
aaagcagttg caacaaaacc ttctcttcat ttcatagaca aagcttcact tccggaagat 360
gccattctat atactgatga ggaggaatgg tccacttgga agaaaattgg tgatagtgtg 420
ctacacattg agctccgcag gtgggctgat attatggtta ttgccccttt gtcagcaaac 480
acacttggga agattgcagg tggactatgt gataacttgt taacctgcat cgtacgagca 540
tgggactaca ataaacccct ttttgtggca ccagccatga atacattgat gtggaataat 600
ccattcacag aacgacacct tatggtaatt gatgagcttg gaatctctct cataccacca 660
gtttctaaaa gactagcttg tggagattat ggaaacggcg ctatggctga accttctctc 720
atctactcaa ctgtaagact cttctatgag tcacggtcac aatcaggtgg catcaacttg 780
gcttgatcca cggatcatta aattttattc gtcggtttgt acaagtggta gaaattgttg 840

aaattcagga cctggaacag tgttacttag catacaccag ttgtgtttat ttttctcctt 900
aattagtgtc atgtttgtat ccaagagctg actttcaatc aagtttcatg ttcattgtag 960
tctattgact ctgaatatta tgcaactcta tatagtaccc gctactaatt atgtat 1016

<210> 26
<211> 206
<212> PRT
<213> Solanum tuberosum

<400> 26
Met Thr Ser Glu Met Glu Pro Val Gln Ile Asn Gly Ala Pro Arg Arg
1               5                   10                  15
Pro Arg Ile Leu Leu Ala Ala Ser Gly Ser Val Ala Ala Ile Lys Phe
            20                  25                  30
Ala Asn Leu Cys Gly Cys Phe Ser Glu Trp Ala Glu Val Lys Ala Val
        35                  40                  45
Ala Thr Lys Pro Ser Leu His Phe Ile Asp Lys Ala Ser Leu Pro Glu
    50                  55                  60
Asp Ala Ile Leu Tyr Thr Asp Glu Glu Glu Trp Ser Thr Trp Lys Lys
65                  70                  75                  80
Ile Gly Asp Ser Val Leu His Ile Glu Leu Arg Arg Trp Ala Asp Ile
                85                  90                  95
Met Val Ile Ala Pro Leu Ser Ala Asn Thr Leu Gly Lys Ile Ala Gly
            100                 105                 110
Gly Leu Cys Asp Asn Leu Leu Thr Cys Ile Val Arg Ala Trp Asp Tyr
        115                 120                 125
Asn Lys Pro Leu Phe Val Ala Pro Ala Met Asn Thr Leu Met Trp Asn
    130                 135                 140
Asn Pro Phe Thr Glu Arg His Leu Met Val Ile Asp Glu Leu Gly Ile
145                 150                 155                 160
Ser Leu Ile Pro Pro Val Ser Lys Arg Leu Ala Cys Gly Asp Tyr Gly
                165                 170                 175
Asn Gly Ala Met Ala Glu Pro Ser Leu Ile Tyr Ser Thr Val Arg Leu
            180                 185                 190
Phe Tyr Glu Ser Arg Ser Gln Ser Gly Gly Ile Asn Leu Ala
        195                 200                 205

<210> 27
<211> 967
<212> DNA
<213> Glycine max

<400> 27
ggttgaacag aagagattta ggcagcccga accgacgatg ttgtttttaa ctgacagcag 60
cacaaagcga acactaacac taacgtgaaa cgcgttgcgt tgtcggaaaa tcaccctcac 120
ctgatcactg tggaagtctc taggtgatgg ccggttcaga acctgttagg gcagagggag 180
agactatggc tgtggatgct gccccaagga agccccggat tctacttgct gctagtggga 240
gtgttgctgc tgtcaaattt gcaaatcttt gtcactgttt ctctgaatgg gcagatgtaa 300
gagcagtttc cacaagtgca tctttgcatt tcattgatag agcagcaatg cccaaggatg 360
taattctata cacggatgac aatgaatggt ctagttggaa gaaattaggt gatagcgtgc 420
ttcacattga gcttcgcaaa tgggctgata tcatggtcat cgctccatta tcagcaaaca 480
cccttggcaa gattgctgga gggttgtgtg acaatctact gacatgcatc gttcgagcct 540
gggactacag caagccattc tttgttgcac cagccatgaa cactttgatg tggaacaatc 600
ctttcactga gcggcatttc atctccattg atgagcttgg catttctctc atcccgcctg 660
ttacaaagag gttagcttgt ggggattatg gcaatggtgc catggctgaa ccctctacca 720
tttactcaac tgtaaggctc ttctatgagt caaaggctca gcaaggtaga gctgtggtaa 780
ccttaaggtg aatgaagggg tagtcctacg cccccattca tggtagtcaa tagcactcta 840
tagcaggata acggagcgca gcagccctga gttactatgg aagtcgaaat cgctgagcga 900
ttttcaataa ccgctgtagc ggctgcaata gtggtctaaa tacagctttt cgggtgctac 960

agcgcac                                                              967

<210>  28
<211>  214
<212>  PRT
<213>  Glycine max

<400>  28
Met Ala Gly Ser Glu Pro Val Arg Ala Glu Gly Glu Thr Met Ala Val
1               5                   10                  15
Asp Ala Ala Pro Arg Lys Pro Arg Ile Leu Leu Ala Ala Ser Gly Ser
            20                  25                  30
Val Ala Ala Val Lys Phe Ala Asn Leu Cys His Cys Phe Ser Glu Trp
        35                  40                  45
Ala Asp Val Arg Ala Val Ser Thr Ser Ala Ser Leu His Phe Ile Asp
    50                  55                  60
Arg Ala Ala Met Pro Lys Asp Val Ile Leu Tyr Thr Asp Asp Asn Glu
65                  70                  75                  80
Trp Ser Ser Trp Lys Lys Leu Gly Asp Ser Val Leu His Ile Glu Leu
                85                  90                  95
Arg Lys Trp Ala Asp Ile Met Val Ile Ala Pro Leu Ser Ala Asn Thr
            100                 105                 110
Leu Gly Lys Ile Ala Gly Gly Leu Cys Asp Asn Leu Leu Thr Cys Ile
        115                 120                 125
Val Arg Ala Trp Asp Tyr Ser Lys Pro Phe Phe Val Ala Pro Ala Met
    130                 135                 140
Asn Thr Leu Met Trp Asn Asn Pro Phe Thr Glu Arg His Phe Ile Ser
145                 150                 155                 160
Ile Asp Glu Leu Gly Ile Ser Leu Ile Pro Pro Val Thr Lys Arg Leu
                165                 170                 175
Ala Cys Gly Asp Tyr Gly Asn Gly Ala Met Ala Glu Pro Ser Thr Ile
            180                 185                 190
Tyr Ser Thr Val Arg Leu Phe Tyr Glu Ser Lys Ala Gln Gln Gly Arg
        195                 200                 205
Ala Val Val Thr Leu Arg
    210

<210>  29
<211>  1067
<212>  DNA
<213>  Vitis vinifera

<400>  29
ttgggagaga tgccacggcc ctcattcgct ctcgatcccc gacctcggct tcactctctc      60
taattttccc gagcaattct ccgagttgag gctgatttga aagttaatga tgatgacata     120
tgcagaacct ttgagtccag aagttgatgc gataccagtc aacattgctc ccagaagacc     180
ccggattcta cttgctgcta gtgggagtgt agctgctatg aagtttggga atctcgtcca     240
ttctttttgt gaatgggcag aagtaagagc agttgtcaca aaggcttctt tacacttcat     300
tgatagagca gcactgccta aggatttata tctttacact gatgatgatg aatggtccag     360
ttggacaaaa ttaggagaca gtgtgcttca cattgagctc cgcaggtggg ctgatgtcat     420
ggtaatcgcc ccattatcag caaatacact tggcaagatt gccgggggac tgtgtgacaa     480
cctgctgaca tgcattgtgc gagcgtggga ctacagcaag ccaatgtttg ttgcgccagc     540
tatgaacacc ttcatgtgga ccaatccttt cacagaacgc catcttatga caattgatga     600
acttggaatt tctcttattc cacctgtcac taaaaggctg gcttgcggag attatgggac     660
tggtgcaatg gctgaacctt ttctcatcca ctcaaccgta agactcttct tggagacacg     720
ggctcaatca agtagcagta atgtgcagta attgggtatg gttaatctgt ctgttggaga     780
agtcaccaga gagttggctg aaatggcatg ttggaaatgc taaacgtagt tcacttggac     840
cgcattgatt ttgttatgac cgtccattaa acttactacg tcttgtagat tgttggtatc     900
ggtggatttg catatcctgt ttctcaatat ttctagtagc gccttttgaa tgttatgtgc     960

cttgtgtatt aatggtgagg gagaatgtat cagtctccta aatttctcaa tctctgtgta 1020
gacatgcttg atgatacatt cctataaata gactctgatt tctgggc 1067

<210> 30
<211> 214
<212> PRT
<213> Vitis vinifera

<400> 30
Met Met Met Thr Tyr Ala Glu Pro Leu Ser Pro Glu Val Asp Ala Ile
1               5                   10                  15
Pro Val Asn Ile Ala Pro Arg Arg Pro Arg Ile Leu Leu Ala Ala Ser
            20                  25                  30
Gly Ser Val Ala Ala Met Lys Phe Gly Asn Leu Val His Ser Phe Cys
        35                  40                  45
Glu Trp Ala Glu Val Arg Ala Val Val Thr Lys Ala Ser Leu His Phe
    50                  55                  60
Ile Asp Arg Ala Ala Leu Pro Lys Asp Leu Tyr Leu Tyr Thr Asp Asp
65                  70                  75                  80
Asp Glu Trp Ser Ser Trp Thr Lys Leu Gly Asp Ser Val Leu His Ile
                85                  90                  95
Glu Leu Arg Arg Trp Ala Asp Val Met Val Ile Ala Pro Leu Ser Ala
            100                 105                 110
Asn Thr Leu Gly Lys Ile Ala Gly Gly Leu Cys Asp Asn Leu Leu Thr
        115                 120                 125
Cys Ile Val Arg Ala Trp Asp Tyr Ser Lys Pro Met Phe Val Ala Pro
    130                 135                 140
Ala Met Asn Thr Phe Met Trp Thr Asn Pro Phe Thr Glu Arg His Leu
145                 150                 155                 160
Met Thr Ile Asp Glu Leu Gly Ile Ser Leu Ile Pro Pro Val Thr Lys
                165                 170                 175
Arg Leu Ala Cys Gly Asp Tyr Gly Thr Gly Ala Met Ala Glu Pro Phe
            180                 185                 190
Leu Ile His Ser Thr Val Arg Leu Phe Leu Glu Thr Arg Ala Gln Ser
        195                 200                 205
Ser Ser Ser Asn Val Gln
    210

<210> 31
<211> 1081
<212> DNA
<213> Hordeum vulgare

<400> 31
cggcacgagg ctccccaatt ccctccgccc gcggcgcgcc gctccgggca gcctcggtcg 60
ccgccgccgc cgccgctgcc tccacctacc gccggccgac gacgcccttc gaacctaccg 120
cctccgccgc catcttcaac cgcttatttg cctgcgctcc taccagatcg cctctgagtt 180
gagggctcca agtgaagcag atggctacat cagaaccggt acagcaaagc tgggagctgg 240
aatccagcag gcctcgggtc ctccttgctg cgtcagggag tgtagctgct ataaaattcg 300
agagcctctg ccgtatcttc tccgagtggg cggaagtccg agctgtggcg accaagtcag 360
cattgcactt tgttgacaga tcatctctgc caagcgacgt cgtcctttac actgatgatg 420
atgagtggtc tacctggaca aagataggag acgaggttct gcacatagag ctgcgaaagt 480
gggcagacat catggtgatc gcccccttat cagcaaacac tctggccaag atcgccggcg 540
ggttatgcga caacctcctg acgtgcatta tccgagcgtg ggactacaag aagccgatct 600
tcgccgcgcc agccatgaac accttcatgt ggaacaaccc attcacggcg cgccacatcg 660
agaccatgaa ccaactgggc atctccctgg tcccgcccac cacgaaacgg ctggcctgcg 720
gcgactacgg gaacggcgcg atggctgagc cctcgcagat ccacacgact gtgaggctcg 780
cgtgcaagtc gcagacgttt ggcacgggca tttcgcccgc gcacccttcc agcggccacc 840
ccgtctagcc gatgcggtga tggtcactat gttcaggtgg tctagtactc gaggtttttc 900

tatgccgccc acatcatcag ggtttgagaa agtgaagggt atcaccaggt gttctgttca 960
tcggtggtgt ctgtattaac cgaagtatcg tacctgtggg atatggatca gcttatttgt 1020
tatgtggtag tagacgttag ggcgtagacg tagagattgg ggaattgctg ttgctccagc 1080
c 1081

<210> 32
<211> 215
<212> PRT
<213> Hordeum vulgare

<400> 32
Met Ala Thr Ser Glu Pro Val Gln Gln Ser Trp Glu Leu Glu Ser Ser
1               5                   10                  15
Arg Pro Arg Val Leu Leu Ala Ala Ser Gly Ser Val Ala Ala Ile Lys
            20                  25                  30
Phe Glu Ser Leu Cys Arg Ile Phe Ser Glu Trp Ala Glu Val Arg Ala
        35                  40                  45
Val Ala Thr Lys Ser Ala Leu His Phe Val Asp Arg Ser Ser Leu Pro
    50                  55                  60
Ser Asp Val Val Leu Tyr Thr Asp Asp Asp Glu Trp Ser Thr Trp Thr
65                  70                  75                  80
Lys Ile Gly Asp Glu Val Leu His Ile Glu Leu Arg Lys Trp Ala Asp
                85                  90                  95
Ile Met Val Ile Ala Pro Leu Ser Ala Asn Thr Leu Ala Lys Ile Ala
            100                 105                 110
Gly Gly Leu Cys Asp Asn Leu Leu Thr Cys Ile Ile Arg Ala Trp Asp
        115                 120                 125
Tyr Lys Lys Pro Ile Phe Ala Ala Pro Ala Met Asn Thr Phe Met Trp
    130                 135                 140
Asn Asn Pro Phe Thr Ala Arg His Ile Glu Thr Met Asn Gln Leu Gly
145                 150                 155                 160
Ile Ser Leu Val Pro Pro Thr Thr Lys Arg Leu Ala Cys Gly Asp Tyr
                165                 170                 175
Gly Asn Gly Ala Met Ala Glu Pro Ser Gln Ile His Thr Thr Val Arg
            180                 185                 190
Leu Ala Cys Lys Ser Gln Thr Phe Gly Thr Gly Ile Ser Pro Ala His
        195                 200                 205
Pro Ser Ser Gly His Pro Val
    210                 215

<210> 33
<211> 1175
<212> DNA
<213> Gossypium hirsutum

<400> 33
cccattgtca cctttagtgc tggtgagcat ttaagagagg gcagagcaac ggaggaaatc 60
cccttttttg tgcagtggaa gtaaaaatta cctaccatta gaaaaggaga aggattggta 120
aagtattgca gcgctccgta aaggcatttt ggtatagcaa gtagcagcaa ttcatcaaaa 180
ggcagcattt ctttttgcac caggggccct ttaaaaagct caactctcgc gcttctttgc 240
gtttcaaatc tctctgcaag tcgcaaggac atctgaagca gaaagcccag attcctctct 300
aatcttagat tccagatttg cataggttat ggcgtatcct gagcctcaag gtgcagatag 360
ggagatggtt aaggtcaatc ctaccccaag aaaaccccgg gttttactcg ctgccagtgg 420
aagtgtagct gccataaagt ttgggaatct ctgccattgt ttctctgaat gggcagaagt 480
aaaagcagtt gccacgaaag cttctttgca tttcattgac agagcatcac ttcctaagga 540
tctaaagctt tacactgatg aggaggaatg gtctagttgg gggaaaatag gtgacagtgt 600
gcttcacatt gagcttcgtc gatggctgat tattatggtc attgccccat tgtcagcaaa 660
cacacttggc aagattgctg gaggattatg tgacaatttg ttaacttgtg tcgtacgagc 720
atgggactac agcaagccaa tgtttgttgc accagctatg aacactttca tgtggagcaa 780

ccctttcaca gaaaagcatc tcatgacaat tgatgagctt ggtatttctc tcatcccccc 840
tgtctccaaa agactagctt gtggggacta tggaaacggc gcaatggcag aaccttctct 900
aatccactcg actgtaagat tattcttgga gtcacgacct caaccaagtg actgaagatc 960
tatttattcg ccatgaatta taaatactat attagttgta tggtagccca gttgactcta 1020
ggttgggtgt atgtctatta gctgtctaac aagcttattg tacatttata gttgcatttc 1080
cgagtttgct taactttgca tatcatgagg agtggtcttt gaatactgct gaaaatttga 1140
tcctgtaagc tgatgatact gatgagagtt ggcag 1175

<210> 34
<211> 208
<212> PRT
<213> Gossypium hirsutum

<400> 34
Met Ala Tyr Pro Glu Pro Gln Gly Ala Asp Arg Glu Met Val Lys Val
1               5                   10                  15
Asn Pro Thr Pro Arg Lys Pro Arg Val Leu Leu Ala Ala Ser Gly Ser
            20                  25                  30
Val Ala Ala Ile Lys Phe Gly Asn Leu Cys His Cys Phe Ser Glu Trp
        35                  40                  45
Ala Glu Val Lys Ala Val Ala Thr Lys Ala Ser Leu His Phe Ile Asp
    50                  55                  60
Arg Ala Ser Leu Pro Lys Asp Leu Lys Leu Tyr Thr Asp Glu Glu Glu
65                  70                  75                  80
Trp Ser Ser Trp Gly Lys Ile Gly Asp Ser Val Leu His Ile Glu Leu
                85                  90                  95
Arg Arg Trp Ala Asp Ile Met Val Ile Ala Pro Leu Ser Ala Asn Thr
            100                 105                 110
Leu Gly Lys Ile Ala Gly Gly Leu Cys Asp Asn Leu Leu Thr Cys Val
        115                 120                 125
Val Arg Ala Trp Asp Tyr Ser Lys Pro Met Phe Val Ala Pro Ala Met
    130                 135                 140
Asn Thr Phe Met Trp Ser Asn Pro Phe Thr Glu Lys His Leu Met Thr
145                 150                 155                 160
Ile Asp Glu Leu Gly Ile Ser Leu Ile Pro Pro Val Ser Lys Arg Leu
                165                 170                 175
Ala Cys Gly Asp Tyr Gly Asn Gly Ala Met Ala Glu Pro Ser Leu Ile
            180                 185                 190
His Ser Thr Val Arg Leu Phe Leu Glu Ser Arg Pro Gln Pro Ser Asp
        195                 200                 205

<210> 35
<211> 1119
<212> DNA
<213> Sorghum bicolor

<400> 35
gcacgagggc tctctccgct cacctccact tccccgcccc cgccccggtc tccgtccttg 60
acacgggcgc gcacccgtcc gcctccgact gacccggagc cgactcgacc ttgttcagga 120
gggagggaga gcccgaaccg cggcggggcg ggagggctcc ttttcgtttg ccccgccgga 180
gggccagccg ccccatgggc cggtcgcctg agcgcgctac gtatcagctc atcgaccggc 240
ggaaagctga gcgttagcgg agctgatggc tacatcagag ccagtacaag agaatttggc 300
gatggactac tcacaaccta gtaagcctcg ggtactcctt gctgcttcag gaagtgtagc 360
cgctataaaa tttgagaacc tttgtcgtag tttctctgag tgggcggatg tccgagccgt 420
ggccaccgca tcatctttgc actttattga tagatcatct cttccaagtg acattgttct 480
ttacactgat gatgatgagt ggtctacctg gaagaagata ggagatgaag ttttacacat 540
tgagctgcgt aaatgggcag atattatggt gattgctccg ttatcagcaa ataccctggc 600
taagatcgcc ggtgggttat gtgacaacct cttaacatgc atcgtgagag cgtgggacta 660
cagcaaaccg ctctttgttg ccccagctat gaacacatta atgtggaaca acccattcac 720

agagcgtcat cttcaaacga tcaaccaact gggcataatc ttgatccccc cagttaccaa 780
aaggttggct tgtggcgatc acgggaatgg tgcaatggct gaaacctcgc agatctatac 840
ttctgtgagg cttgcatgga agacgcaacc acatgatgca agcagttcac ttgtggttcc 900
tgtcagtaat aaccgcccat ctagctggtg tttgacctct taattaagag cttaggaatt 960
tgttttatct gcagtgtgca tcttgatgtt agaaatgttg taccaatttt ttttaaccag 1020
tgttttagtg tgtattgtga tgagaatgtg gggaaaggaa ggttgtcagt tgtcacggtg 1080
ataaattact gccagctcag tatcgatagt gaggatgag 1119

<210> 36
<211> 225
<212> PRT
<213> Sorghum bicolor

<400> 36
Met Ala Thr Ser Glu Pro Val Gln Glu Asn Leu Ala Met Asp Tyr Ser
1               5                   10                  15
Gln Pro Ser Lys Pro Arg Val Leu Leu Ala Ala Ser Gly Ser Val Ala
            20                  25                  30
Ala Ile Lys Phe Glu Asn Leu Cys Arg Ser Phe Ser Glu Trp Ala Asp
        35                  40                  45
Val Arg Ala Val Ala Thr Ala Ser Ser Leu His Phe Ile Asp Arg Ser
    50                  55                  60
Ser Leu Pro Ser Asp Ile Val Leu Tyr Thr Asp Asp Asp Glu Trp Ser
65                  70                  75                  80
Thr Trp Lys Lys Ile Gly Asp Glu Val Leu His Ile Glu Leu Arg Lys
                85                  90                  95
Trp Ala Asp Ile Met Val Ile Ala Pro Leu Ser Ala Asn Thr Leu Ala
            100                 105                 110
Lys Ile Ala Gly Gly Leu Cys Asp Asn Leu Leu Thr Cys Ile Val Arg
        115                 120                 125
Ala Trp Asp Tyr Ser Lys Pro Leu Phe Val Ala Pro Ala Met Asn Thr
    130                 135                 140
Leu Met Trp Asn Asn Pro Phe Thr Glu Arg His Leu Gln Thr Ile Asn
145                 150                 155                 160
Gln Leu Gly Ile Ile Leu Ile Pro Pro Val Thr Lys Arg Leu Ala Cys
                165                 170                 175
Gly Asp His Gly Asn Gly Ala Met Ala Glu Thr Ser Gln Ile Tyr Thr
            180                 185                 190
Ser Val Arg Leu Ala Trp Lys Thr Gln Pro His Asp Ala Ser Ser Ser
        195                 200                 205
Leu Val Val Pro Val Ser Asn Asn Arg Pro Ser Ser Trp Cys Leu Thr
    210                 215                 220
Ser
225

<210> 37
<211> 975
<212> DNA
<213> Lycopersicon esculentum

<400> 37
ctacaattga tcggcgccgg actttgatca ccgtcggcgt ttctaagccg ttgccggact 60
atgccggagc aaatctcaat caagtgagct aaccactctg tgtttcaact tctggttata 120
aattttttaa agcctttgct ccgttaggtt aggtagatac ggatcctatg acttcagaga 180
tggaaccagt tcagattaat ggtgcaccta ggagacctcg tattctgctg gcagcaagtg 240
gaagtgtggc ttcaattaag tttgctaatc tatgtcgttg tttttctgaa tgggcagaag 300
ttaaagcagt tgcaacgaaa ccttctcttc atttcataga caaagcttcg cttccggaag 360
atgtcattct ttatactgat gaggaggaat ggtccacttg gtgcagattg gtgatagtgt 420
gctacacatt gagctccgca gatggctgaa tattatggtt attgcccctt tgtcagcaaa 480

cacacttggg aagattgcag gtggactatg tgataacttg ttaacctgca tcgtacgagc 540
atgggactac aataaacccc tttttgtggc accagccatg aatacattga tgtggaataa 600
tccattcaca gaacgacacc ttatggtaat tgatgagctt ggaatctctc tcataccccc 660
agtttcaaaa agactagcat gtggagatta tggaaatggc gctatggctg aaccttctct 720
catttactca actgtaagac tcttttatga gtcacggtca caatcaggtg gcatcaactt 780
ggcttgattc gcagatcatt aaattttatt catcggtttg tacaagtggt aaaaattgtt 840
gaaattcagg acctggaaca gtgttactta gcatacacca gttgggttta tttttctcct 900
aaattcttca cctctttaac ttgtgtcatg tttgtatcca agcggtaact ttcaatcaag 960
tttcatgttc attgt 975

<210> 38
<211> 206
<212> PRT
<213> Lycopersicon esculentum

<220>
<221> UNSURE
<222> (74)..(74)
<223> Xaa can be any naturally occurring amino acid

<400> 38
Met Thr Ser Glu Met Glu Pro Val Gln Ile Asn Gly Ala Pro Arg Arg
1               5                   10                  15
Pro Arg Ile Leu Leu Ala Ala Ser Gly Ser Val Ala Ser Ile Lys Phe
            20                  25                  30
Ala Asn Leu Cys Arg Cys Phe Ser Glu Trp Ala Glu Val Lys Ala Val
        35                  40                  45
Ala Thr Lys Pro Ser Leu His Phe Ile Asp Lys Ala Ser Leu Pro Glu
    50                  55                  60
Asp Val Ile Leu Tyr Thr Asp Glu Glu Xaa Met Val His Leu Val Gln
65                  70                  75                  80
Ile Gly Asp Ser Val Leu His Ile Glu Leu Arg Arg Trp Ala Asp Ile
                85                  90                  95
Met Val Ile Ala Pro Leu Ser Ala Asn Thr Leu Gly Lys Ile Ala Gly
            100                 105                 110
Gly Leu Cys Asp Asn Leu Leu Thr Cys Ile Val Arg Ala Trp Asp Tyr
        115                 120                 125
Asn Lys Pro Leu Phe Val Ala Pro Ala Met Asn Thr Leu Met Trp Asn
    130                 135                 140
Asn Pro Phe Thr Glu Arg His Leu Met Val Ile Asp Glu Leu Gly Ile
145                 150                 155                 160
Ser Leu Ile Pro Pro Val Ser Lys Arg Leu Ala Cys Gly Asp Tyr Gly
                165                 170                 175
Asn Gly Ala Met Ala Glu Pro Ser Leu Ile Tyr Ser Thr Val Arg Leu
            180                 185                 190
Phe Tyr Glu Ser Arg Ser Gln Ser Gly Gly Ile Asn Leu Ala
        195                 200                 205

<210> 39
<211> 1395
<212> DNA
<213> Arabidopsis thaliana

<400> 39
atggcgaaaa cagctcttac tcctcccgcg tctggttccg aagttccaag atccggtaca 60
cctggagatg cgtctggcaa caaacctcaa acggatgcca ccggcgtctc agctactgat 120
actgcttctc agaaacgcgg tcgtggtcga ccgccaaagg ctaaatctga ctcttcccaa 180
atcggtgccg tttctgcgaa ggcgagtact aaaccaagtg gtcgtccgaa aagaaacgta 240
gctcaggctg ttccttctac gtctgtggcg gcggctgtga agaaacgtgg tagggcaaag 300

aggtcgactg taacggcggc tgtggttact actgctactg gagagggttc tagaaaacga 360
gggaggccaa agaaagatga cgtggcggct gcaactgttc cagcagaaac tgtggtggct 420
ccagctaaga gacgtggaag gaaacctact gtcgaagtag ctgcacagcc tgtgcgcagg 480
actaggaagg tattcgggtt ttctatgcat gaacaaaaga gcacttcagt ggcaccggta 540
gctgcaaacg tcggagatct caagaaaaga accgcactct tacaaaagaa agtgaaggaa 600
gctgcagcta agttgaaaca agcagtaaca gcaattgacg aggtccagaa gttagcggat 660
ggaatattga ccagcgatga cgtcgacttc tctgttttgt tttcaaactt aaagaccctc 720
gcggcatttg atttcgattt ccgattaggg ttcctcgcag atcctccttc ctcggctata 780
gagaagaaga tgaatatgga agtggataca gtaacaagga agcctcgtat cttactagct 840
gcaagtggaa gtgtggcttc aattaagttc agtaatctct gccattgttt ctcagaatgg 900
gctgaagtca aagccgtcgc ttcaaaatca tctctcaatt tcgttgataa accttctcta 960
cctcagaatg tgactctcta tacagatgaa gatgaatggt ctagctggaa caagattggt 1020
gatcccgttc ttcatatcga gctcagacgc tgggctgatg ttatgatcat tgctcctttg 1080
tctgctaaca cattagccaa gattgctggt gggttatgtg ataatctatt gacatgtata 1140
gtaagagcat gggattatag caaaccgttg tttgttgcac cggcgatgaa cactttgatg 1200
tggaacaatc ctttcacaga acggcacctt gtcttgcttg atgaacttgg aatcacccta 1260
attcctccca tcaagaagaa actggcctgt ggagactacg gtaatggcgc aatggctgag 1320
ccttctctga tttattccac tgttagactg ttctgggagt cacaagctcg taaacaaaga 1380
gatggaacca gttga 1395

<210> 40
<211> 464
<212> PRT
<213> Arabidopsis thaliana

<400> 40
Met Ala Lys Thr Ala Leu Thr Pro Pro Ala Ser Gly Ser Glu Val Pro
1               5                   10                  15
Arg Ser Gly Thr Pro Gly Asp Ala Ser Gly Asn Lys Pro Gln Thr Asp
            20                  25                  30
Ala Thr Gly Val Ser Ala Thr Asp Thr Ala Ser Gln Lys Arg Gly Arg
        35                  40                  45
Gly Arg Pro Pro Lys Ala Lys Ser Asp Ser Ser Gln Ile Gly Ala Val
    50                  55                  60
Ser Ala Lys Ala Ser Thr Lys Pro Ser Gly Arg Pro Lys Arg Asn Val
65                  70                  75                  80
Ala Gln Ala Val Pro Ser Thr Ser Val Ala Ala Ala Val Lys Lys Arg
                85                  90                  95
Gly Arg Ala Lys Arg Ser Thr Val Thr Ala Ala Val Val Thr Thr Ala
            100                 105                 110
Thr Gly Glu Gly Ser Arg Lys Arg Gly Arg Pro Lys Lys Asp Asp Val
        115                 120                 125
Ala Ala Ala Thr Val Pro Ala Glu Thr Val Val Ala Pro Ala Lys Arg
    130                 135                 140
Arg Gly Arg Lys Pro Thr Val Glu Val Ala Ala Gln Pro Val Arg Arg
145                 150                 155                 160
Thr Arg Lys Val Phe Gly Phe Ser Met His Glu Gln Lys Ser Thr Ser
                165                 170                 175
Val Ala Pro Val Ala Ala Asn Val Gly Asp Leu Lys Lys Arg Thr Ala
            180                 185                 190
Leu Leu Gln Lys Lys Val Lys Glu Ala Ala Ala Lys Leu Lys Gln Ala
        195                 200                 205
Val Thr Ala Ile Asp Glu Val Gln Lys Leu Ala Asp Gly Ile Leu Thr
    210                 215                 220
Ser Asp Asp Val Asp Phe Ser Val Leu Phe Ser Asn Leu Lys Thr Leu
225                 230                 235                 240
Ala Ala Phe Asp Phe Asp Phe Arg Leu Gly Phe Leu Ala Asp Pro Pro
                245                 250                 255
Ser Ser Ala Ile Glu Lys Lys Met Asn Met Glu Val Asp Thr Val Thr

                    260                     265                     270
Arg Lys Pro Arg Ile Leu Leu Ala Ala Ser Gly Ser Val Ala Ser Ile
        275                     280                     285
Lys Phe Ser Asn Leu Cys His Cys Phe Ser Glu Trp Ala Glu Val Lys
    290                     295                     300
Ala Val Ala Ser Lys Ser Ser Leu Asn Phe Val Asp Lys Pro Ser Leu
305                     310                     315                     320
Pro Gln Asn Val Thr Leu Tyr Thr Asp Glu Asp Glu Trp Ser Ser Trp
                    325                     330                     335
Asn Lys Ile Gly Asp Pro Val Leu His Ile Glu Leu Arg Arg Trp Ala
                340                     345                     350
Asp Val Met Ile Ile Ala Pro Leu Ser Ala Asn Thr Leu Ala Lys Ile
        355                     360                     365
Ala Gly Gly Leu Cys Asp Asn Leu Leu Thr Cys Ile Val Arg Ala Trp
    370                     375                     380
Asp Tyr Ser Lys Pro Leu Phe Val Ala Pro Ala Met Asn Thr Leu Met
385                     390                     395                     400
Trp Asn Asn Pro Phe Thr Glu Arg His Leu Val Leu Leu Asp Glu Leu
                    405                     410                     415
Gly Ile Thr Leu Ile Pro Pro Ile Lys Lys Lys Leu Ala Cys Gly Asp
                420                     425                     430
Tyr Gly Asn Gly Ala Met Ala Glu Pro Ser Leu Ile Tyr Ser Thr Val
        435                     440                     445
Arg Leu Phe Trp Glu Ser Gln Ala Arg Lys Gln Arg Asp Gly Thr Ser
    450                     455                     460

<210>  41
<211>  1182
<212>  DNA
<213>  Pinus sp.

<400>  41
atattgaaac tcatttttaa acacttattc tgcactataa gaagcagcag gcccccagaa      60
atatgagctt catacacaca acgggggagt aatcagcacc atcaaagggg ggatttgcgg     120
caattgtgag atcaagtgga ggaagctgga atagatctaa atctaggccc ttgaggagaa     180
tgctctccta atggtggttt taaaaatcaa tactctagtg caaaacatgg atgcaacaaa     240
ttctcaacct gatgaacctg gaagggatgc taattcatct cagaaaccac gaatcattct     300
agcagccagc gggagtgtgg cagcaataaa atttggaatt cttgcccatt gtctatgtca     360
atgggcagaa gtcaaagcag tggtcacaaa atctgctttg catttcattg acaagatgtc     420
tcttccggct aatgttaagc tctacactga tgaaaatgaa tggtctagct ggagcaaaat     480
aggtgatact gtactgcata tagaactccg gcaatgggct gatgctatgg tgattgctcc     540
actctcagca aacacgcttg ctaagatagc aggtggccta tgcgacaatc tacttacttg     600
catagtacgt gcatgggact ttaacaagcc tctctttgta gctcctgcaa tgaatacttt     660
catgtggaac aatccattta ctcaacggca tttggactca atctcagagc tcggactatc     720
acttatcccc ccaataacaa agaagttagc ttgtggtgat tatggaaatg gtgcaatggc     780
tgaaccttct acgatagata caactcttag gttttcactt gatccttcaa ttgtatgaag     840
tgtctgatgt cccgttcaac aattcttgtt atgaatctaa catgcttagt acaatggact     900
atggtataga cccagatagt gtttcttggt agctgggtta gacagcatga actcagcttg     960
tgtgtggtga agttaagttc tgatgataaa caatcatgaa cttgtgattt tatgtaacat    1020
ttgctcattt attctggaca cttacaagaa ctgtgtggat actcctgctt ataagagaat    1080
gtgtcaaact actatgcaaa tcatctaatt tggaacttga tgatgtaaga ggagtatgca    1140
ctttattttc ttccctgtga taattattaa tctctatgat tg                       1182

<210>  42
<211>  215
<212>  PRT
<213>  Pinus sp.

<400>  42

Met Val Val Leu Lys Ile Asn Thr Leu Val Gln Asn Met Asp Ala Thr
1               5                   10                  15
Asn Ser Gln Pro Asp Glu Pro Gly Arg Asp Ala Asn Ser Ser Gln Lys
            20                  25                  30
Pro Arg Ile Ile Leu Ala Ala Ser Gly Ser Val Ala Ala Ile Lys Phe
        35                  40                  45
Gly Ile Leu Ala His Cys Leu Cys Gln Trp Ala Glu Val Lys Ala Val
    50                  55                  60
Val Thr Lys Ser Ala Leu His Phe Ile Asp Lys Met Ser Leu Pro Ala
65                  70                  75                  80
Asn Val Lys Leu Tyr Thr Asp Glu Asn Glu Trp Ser Ser Trp Ser Lys
                85                  90                  95
Ile Gly Asp Thr Val Leu His Ile Glu Leu Arg Gln Trp Ala Asp Ala
            100                 105                 110
Met Val Ile Ala Pro Leu Ser Ala Asn Thr Leu Ala Lys Ile Ala Gly
        115                 120                 125
Gly Leu Cys Asp Asn Leu Leu Thr Cys Ile Val Arg Ala Trp Asp Phe
    130                 135                 140
Asn Lys Pro Leu Phe Val Ala Pro Ala Met Asn Thr Phe Met Trp Asn
145                 150                 155                 160
Asn Pro Phe Thr Gln Arg His Leu Asp Ser Ile Ser Glu Leu Gly Leu
                165                 170                 175
Ser Leu Ile Pro Pro Ile Thr Lys Lys Leu Ala Cys Gly Asp Tyr Gly
            180                 185                 190
Asn Gly Ala Met Ala Glu Pro Ser Thr Ile Asp Thr Thr Leu Arg Phe
        195                 200                 205
Ser Leu Asp Pro Ser Ile Val
    210                 215

<210>  43
<211>  804
<212>  DNA
<213>  Oryza sativa

<400>  43
atggggcggg ggaaggtgca gctgaagcgg atagagaaca agatcaacag gcaggtgacg      60
ttctccaaga ggaggaatgg attgctgaag aaggcgcacg agatctccgt cctctgcgac     120
gccgaggtcg ccgccatcgt cttctccccc aagggcaagc tctacgagta cgccactgac     180
tccaggatgg acaaaatcct tgaacgttat gagcgctatt catatgctga aaaggctctt     240
atttcagctg aatccgagag tgagggaaat tggtgccatg aatacaggaa acttaaggca     300
aagattgaga ccatacaaaa atgtcacaaa cacctcatgg gagaggatct agaatccctg     360
aatctcaaag aactccaaca gctagagcag cagctggaga gttcattgaa gcacataata     420
tcaagaaaga gccaccttat gcttgagtcc atttccgagc tgcagaaaaa ggagaggtca     480
ctgcaggagg agaacaaggc tctgcagaag gaactggtgg agaggcagaa gaatgtgagg     540
ggccagcagc aagtagggca gtgggaccaa acccaggtcc aggcccaggc ccaagcccaa     600
ccccaagccc agacaagctc ctcctcctcc tccatgctga gggatcagca ggcacttctt     660
ccaccacaaa atatctgcta cccgccggtg atgatgggcg agagaaatga tgcggcggcg     720
gcggcggcgg tggcggcgca gggccaggtg caactccgca tcggaggtct tccgccatgg     780
atgctgagcc acctcaatgc ttaa                                            804

<210>  44
<211>  267
<212>  PRT
<213>  Oryza sativa

<400>  44
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala

20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Ile Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Ser Ser
        195                 200                 205
Ser Ser Ser Met Leu Arg Asp Gln Gln Ala Leu Leu Pro Pro Gln Asn
    210                 215                 220
Ile Cys Tyr Pro Pro Val Met Met Gly Glu Arg Asn Asp Ala Ala Ala
225                 230                 235                 240
Ala Ala Ala Val Ala Ala Gln Gly Gln Val Gln Leu Arg Ile Gly Gly
                245                 250                 255
Leu Pro Pro Trp Met Leu Ser His Leu Asn Ala
            260                 265

<210>  45
<211>  52
<212>  DNA
<213>  Artificial sequence

<220>
<223>  primer

<400>  45
ggggacaagt ttgtacaaaa aagcaggctt aaacaatggg gcgggggaag gt          52

<210>  46
<211>  47
<212>  DNA
<213>  Artificial sequence

<220>
<223>  primer

<400>  46
ggggaccact ttgtacaaga aagctgggtt tggccgacga cgacgac               47

<210>  47
<211>  2193
<212>  DNA
<213>  Oryza sativa

<400>  47
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt      420
ttgtgcattc gtcatatcgc acatcattaa ggacatgtct tactccatcc caatttttat     480
ttagtaatta aagacaattg acttattttt attatttatc ttttttcgat tagatgcaag    540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa      780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga attttttttc ccaaatatct taaaaagtca     1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccatttttaa ttatatgaaa tgaactgtag cataagcagt attcatttgg    1920
attatttttt ttattagctc tcaccccttc attattctga gctgaaagtc tggcatgaac    1980
tgtcctcaat tttgttttca aattcacatc gattatctat gcattatcct cttgtatcta    2040
cctgtagaag tttctttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193

<210>  48
<211>  42
<212>  PRT
<213>  Artificial sequence

<220>
<223>  artificial sequence

<220>
<221>  VARIANT
<222>  (2)..(2)
<223>  /replace = "Val"

<220>
<221>  VARIANT
<222>  (6)..(6)
<223>  /replace = "Asn"

<220>
<221>  VARIANT

<222>  (10)..(10)
<223>  /replace = "Ile"

<220>
<221>  VARIANT
<222>  (12)..(12)
<223>  /replace = "Tyr"

<220>
<221>  VARIANT
<222>  (16)..(16)
<223>  /replace = "Ile" /replace = "Leu"

<220>
<221>  VARIANT
<222>  (17)..(17)
<223>  /replace = "Gly"

<220>
<221>  VARIANT
<222>  (18)..(18)
<223>  /replace = "Ala" /replace = "Val"

<220>
<221>  VARIANT
<222>  (19)..(19)
<223>  /replace = "Val"

<220>
<221>  VARIANT
<222>  (20)..(20)
<223>  /replace = "Val"

<220>
<221>  VARIANT
<222>  (23)..(23)
<223>  /replace = "Pro" /replace = "Ala" /replace = "Asn"

<220>
<221>  VARIANT
<222>  (30)..(30)
<223>  /replace = "Phe"

<220>
<221>  VARIANT
<222>  (31)..(31)
<223>  /replace = "Ser"

<220>
<221>  VARIANT
<222>  (32)..(32)
<223>  /replace = "Ser"

<220>
<221>  VARIANT
<222>  (33)..(33)
<223>  /replace = "Asn" /replace = "Glu"

<220>

<221>  VARIANT
<222>  (35)..(35)
<223>  /replace = "Cys" /replace = "Arg" /replace = "Ser"

<220>
<221>  VARIANT
<222>  (37)..(37)
<223>  /replace = "Glu"

<220>
<221>  VARIANT
<222>  (38)..(38)
<223>  /replace = "Ile" /replace = "Arg" /replace = "Lys"

<220>
<221>  VARIANT
<222>  (41)..(41)
<223>  /replace = "Glu"

<400>  48
Leu Leu Lys Lys Ala His Glu Ile Ser Val Leu Cys Asp Ala Glu Val
1               5                   10                  15
Ala Leu Ile Ile Phe Ser Thr Lys Gly Lys Leu Tyr Glu Tyr Ala Thr
            20                  25                  30
Asp Ser Lys Met Asp Asn Ile Leu Glu Arg
        35                  40

<210>  49
<211>  19
<212>  PRT
<213>  Artificial sequence

<220>
<223>  artificial sequence

<220>
<221>  VARIANT
<222>  (4)..(4)
<223>  /replace = "Ser"

<220>
<221>  VARIANT
<222>  (5)..(5)
<223>  /replace = "Arg"

<220>
<221>  VARIANT
<222>  (6)..(6)
<223>  /replace = "Ile"

<220>
<221>  VARIANT
<222>  (8)..(8)
<223>  /replace = "Thr" /replace = "Ser"

<220>
<221>  VARIANT
<222>  (9)..(9)
<223>  /replace = "Ile"

<220>
<221>  VARIANT
<222>  (10)..(10)
<223>  /replace = "Asn"

<220>
<221>  VARIANT
<222>  (11)..(11)
<223>  /replace = "Arg" /replace = "Asn"

<220>
<221>  VARIANT
<222>  (12)..(12)
<223>  /replace = "Cys" /replace = "Arg"

<220>
<221>  VARIANT
<222>  (13)..(13)
<223>  /replace = "His"

<220>
<221>  VARIANT
<222>  (14)..(14)
<223>  /replace = "Lys"

<400>  49
Lys Leu Lys Ala Lys Val Glu Ala Leu Gln Lys Ser Gln Arg His Leu
1               5                   10                  15
Met Gly Glu


<210>  50
<211>  11
<212>  PRT
<213>  Artificial sequence

<220>
<223>  artificial sequence

<220>
<221>  VARIANT
<222>  (2)..(2)
<223>  /replace = "Gln" /replace = "Val" /replace = "Ala"

<220>
<221>  VARIANT
<222>  (6)..(6)
<223>  /replace = "Phe"

<220>
<221>  VARIANT
<222>  (7)..(7)
<223>  /replace = "Phe"

<220>
<221>  VARIANT
<222>  (8)..(8)
<223>  /replace = "Phe"

<220>
<221> VARIANT
<222> (9)..(9)
<223> /replace = "Phe"

<220>
<221> VARIANT
<222> (10)..(10)
<223> /replace = "Cys" /replace = "Phe"

<220>
<221> VARIANT
<222> (11)..(11)
<223> /replace = "Met"

<400> 50
Gln Pro Gln Thr Ser Ser Ser Ser Ser Ser Phe
1               5                   10

<210> 51
<211> 7
<212> PRT
<213> Artificial sequence

<220>
<223> artificial sequence

<220>
<221> VARIANT
<222> (1)..(1)
<223> /replace = "Ala" /replace = "Val" /replace = "Leu"

<220>
<221> VARIANT
<222> (2)..(2)
<223> /replace = "Pro"

<220>
<221> UNSURE
<222> (3)..(3)
<223> Xaa can be any naturally occurring amino acid, preferably Leu,
      Pro or His

<220>
<221> UNSURE
<222> (3)..(3)
<223> Xaa can be any naturally occurring amino acid, preferably Leu,
      Pro or His

<220>
<221> UNSURE
<222> (6)..(6)
<223> Xaa can be any naturally occurring amino acid, preferably Leu,
      Pro or His

<220>
<221> VARIANT
<222> (7)..(7)

<223>  /replace = "His"

<400>  51
Gly Leu Xaa Trp Met Xaa Ser
1               5

<210>  52
<211>  735
<212>  DNA
<213>  Hordeum vulgare

<400>  52
atggggcgcg ggaaggtgca gctgaagcgg atcgagaaca agatcaaccg ccaggtcacc      60
ttctccaagc gccgctcggg gctgctcaag aaggcgcacg agatctccgt gctctgcgac     120
gccgaggtcg gcctcatcat cttctccacc aagggaaagc tctacgagtt ctccaccgag     180
tcatgtatgg acaaaattct tgaacggtat gagcgctact cttatgcaga aaaggttctc     240
gtttcaagtg aatctgaaat tcagggaaac tggtgtcacg aatataggaa actgaaggcg     300
aaggttgaga caatacagaa atgtcaaaag catctcatgg gagaggatct tgaatctttg     360
aatctcaagg agttgcagca actggagcag cagctggaaa gctcactgaa acatatcaga     420
gccaggaaga accaacttat gcacgaatcc atttctgagc ttcagaagaa ggagaggtca     480
ctgcaggagg agaataaagt tctccagaag gaacttgtgg agaagcagaa ggcccaggcg     540
gcgcagcaag atcaaactca gcctcaaacc agctcttctt cttcttcctt catgatgagg     600
gatgctcccc ctgtcgcaga taccagcaat cacccagcgg cggcaggcga gagggcagag     660
gatgtggcag tgcagcctca ggtcccactc cggacggcgc ttccactgtg gatggtgagc     720
cacatcaacg gctga                                                      735

<210>  53
<211>  244
<212>  PRT
<213>  Hordeum vulgare

<400>  53
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Val Ala Asp Thr
        195                 200                 205
Ser Asn His Pro Ala Ala Ala Gly Glu Arg Ala Glu Asp Val Ala Val

210                 215                 220
Gln Pro Gln Val Pro Leu Arg Thr Ala Leu Pro Leu Trp Met Val Ser
225                 230                 235                 240
His Ile Asn Gly

<210>  54
<211>  1196
<212>  DNA
<213>  Hordeum vulgare

<400>  54
ctctcccctc ccacttcacc caaccacctg acagccatgg ctccgccacc tcgcctccgc      60
ccgcgcctct gagagtagcc gtcgcggtcg ctcgctcgct cgctcgctgc tgccggtgtt     120
ggcccggtcc tcgagcggag atggggcgca ggaaggtgca gctgaagcgg atcgagaaca     180
agatcaaccg ccaggtcacc ttctccaagc gccgctcggg gctgctcaag aaggcgcacg     240
agatctccgt gctctacgac gccgaggtcg gcctcatcat cttctccacc aagggaaagc     300
tctacgagtt ctccaccgag tcatgtatgg acaaaattct tgaacggtat gagcgctact     360
cttatgcaga aaaggttctc gtttcaagtg aatctgaaat tcagggaaac tggtgtcacg     420
aatataggaa actgaaggcg aaggttgaga caatacagaa atgtcaaaag catctcatgg     480
gagaggatct tgaatctttg aatctcaagg agttgcagca actggagcag cagctggaaa     540
gctcactgaa acatatcaga gccaggaaga accaacttat gcacgaatcc atttctgagc     600
ttcagaagaa ggagaggtca ctgcaggagg agaataaagt tctccagaag gaacttgtgg     660
agaagcagaa ggcccaggcg gcgcagcaag atcaaactca gcctcaaacc agctcttctt     720
cttcttcctt catgatgagg gatgctcccc ctgtcgcaga taccagcaat cacccagcgg     780
cggcaggcga gagggcagag gatgtggcag tgcagcctca ggtcccactc cggacggcgc     840
ttccactgtg gatggtgagc cacatcaacg gctgaagggc ttccagccca tgtaagcgta     900
ctattcagta cgagtaacaa gttgcagcgg ccagcctggt gtatcatgcg gttgcgaaca     960
tgctaacccc atggagggga gaggaaaaga aatcagagta aagcagcaag ctgcaggaat    1020
gtgtatattt cacttcgtcc acctcagttt cctttccacc tgggctgaga tggctgtacg    1080
agtaatctac catgtaattt atatgtagca tgagtgacga attttcaact ttcgatgata    1140
tccgttgctc ctgggtgttg tttctgtgaa ttaacctatc gaatatgagc gttgtg        1196

<210>  55
<211>  244
<212>  PRT
<213>  Hordeum vulgare

<400>  55
Met Gly Arg Arg Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Tyr Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140
Gln Leu Met His Glu Ser Ile Ser Glu Leu Gln Lys Lys Glu Arg Ser
145                 150                 155                 160

Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Val Ala Asp Thr
        195                 200                 205
Ser Asn His Pro Ala Ala Ala Gly Glu Arg Ala Glu Asp Val Ala Val
    210                 215                 220
Gln Pro Gln Val Pro Leu Arg Thr Ala Leu Pro Leu Trp Met Val Ser
225                 230                 235                 240
His Ile Asn Gly

<210> 56
<211> 1161
<212> DNA
<213> Triticum aestivum

<400> 56
ggcacgagct ctctctctct ctctctctct ctctctctct ctctctcctc gtgccgaatt      60
cggcacgagc ggagatgggg cgcgggaagg tgcagctgaa gcggatcgag aacaagatca     120
accggcaggt gaccttctcc aagcgccgct cggggctgct caagaaggcg cacgagatct     180
ccgtgctctg cgacgccgag gtcggcctca tcatcttctc caccaaggga aagctctacg     240
agttctccac cgagtcatgt atggacaaaa ttcttgaacg gtatgagcgc tactcttatg     300
cagaaaaggt tctcgtttca agtgaatctg aaattcaggg aaactggtgt cacgaatata     360
ggaaactgaa ggcgaaggtt gagacaatac agaaatgtca aaagcatctg atgggagagg     420
atcttgaatc tttgaatctc aaggagttgc agcaactgga gcagcagctg gaaagctcac     480
tgaaacatat cagatccagg aagaaccaac ttatgcacga atccatttct gagcttcaga     540
agaaggagag gtcactgcag gaggagaata aagttctcca gaaggaactc gtcgagaagc     600
agaaggccca ggcggcgcaa caagatcaga ctcagcctca aacaagctct tcttcttctt     660
ccttcatgat gagggatgct ccccctgccg cagctaccag cattcatcca gcggcggcag     720
gcgagagggc aggggatgcg gcagtgcagc cgcaggcccc accccggacg gggcttccac     780
tgtggatggt gagccacatc aacggctgaa gggcttccag cccatataag cgtactattc     840
agtagagagt aacaagttgc accggccagt ctggtgtatg ttgcggttgc tagcacgcct     900
gaccccttgg aggggaaagg aaaagaaatc agagtaaagt agcaagctgc agcgatgtgt     960
atatttcact ttgtccaccc cagtttccct cccagctggg ctcaatttac catgtaatct    1020
atatgtagct tgagtgatga attttcaagt ttccatgata cccgtctcta gtgggatgtt    1080
gtttatgtga attaacctat caaatatgag cattgtgtat attgtgattc ttgaaaataa    1140
ataaatcagg atctttgtct t                                              1161

<210> 57
<211> 244
<212> PRT
<213> Triticum aestivum

<400> 57
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu

            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Ala Ala Ala Thr
        195                 200                 205
Ser Ile His Pro Ala Ala Ala Gly Glu Arg Ala Gly Asp Ala Ala Val
    210                 215                 220
Gln Pro Gln Ala Pro Pro Arg Thr Gly Leu Pro Leu Trp Met Val Ser
225                 230                 235                 240
His Ile Asn Gly

<210> 58
<211> 1210
<212> DNA
<213> Triticum aestivum

<400> 58
tccctctcct ccctctcttc cgcctcaccc aaccacctga cagccatggc tccgcccccc 60
cgcccccgcc tgcgcctgtc ggagtagccg tcgcggtctg ccggtgttgg aggcttgggg 120
tgtagggttg gccccgttct ccagcggaga tggggcgcgg gaaggtgcag ctgaagcgga 180
tcgagaacaa gatcaaccgg caggtgacct tctccaagcg ccgctcgggg ctgctcaaga 240
aggcgcacga gatctccgtg ctctgcgacg ccgaggtcgg cctcatcatc ttctccacca 300
agggaaagct ctacgagttc tccaccgagt catgtatgga caaaattctt gaacggtatg 360
agcgctactc ttatgcagaa aaggttctcg tttcaagtga atctgaaatt cagggaaact 420
ggtgtcacga atataggaaa ctgaaggcga aggttgagac aatacagaaa tgtcaaaagc 480
atctgatggg agaggatctt gaatctttga atctcaagga gttgcagcaa ctggagcagc 540
agctggaaag ctcactgaaa catatcagat ccaggaagaa ccaacttatg cacgaatcca 600
tttctgagct tcagaagaag gagaggtcac tgcaggagga gaataaagtt ctccagaagg 660
aactcgtcga gaagcagaag gcccaggcgg cgcaacaaga tcagactcag cctcaaacaa 720
gctcttcttc ttcttccttc atgatgaggg atgctccccc tgccgcaact accagcattc 780
atccagcggc atcaggagag agggcagagg atgcggcagt gcagccgcag gccccacccc 840
ggacggggct tccactgtgg atggttagcc acatcaacgg ctgaagggct tccagcccat 900
ataagcgtac tattcagtag agagtaacaa gttgcaccgg ccagcctggt gtatgttgcg 960
gttgctagca tgcctgaccc cttggagggg aaaggaaaag aaatcagagt aaagtagcaa 1020
gctgcagtga tgtgtatatt tcactttgtc cacctcagtt tccctcccag ctgggctcaa 1080
tttaccatgt aatctatatg tagcttgagt gatgaatttt caagtttcca tgatacccgt 1140
ctcgagcggg tgttgtttat gtgaattaac ctatcaaata tgagcattgt gtaaaaaaaa 1200
aaaaaaaaaa 1210

<210> 59
<211> 244
<212> PRT
<213> Triticum aestivum

<400> 59
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Ala Ala Thr Thr
        195                 200                 205
Ser Ile His Pro Ala Ala Ser Gly Glu Arg Ala Glu Asp Ala Ala Val
    210                 215                 220
Gln Pro Gln Ala Pro Pro Arg Thr Gly Leu Pro Leu Trp Met Val Ser
225                 230                 235                 240
His Ile Asn Gly

<210> 60
<211> 735
<212> DNA
<213> Triticum monococcum

<400> 60
atggggcgcg ggaaggtgca gctgaagcgg atcgagaaca agatcaaccg gcaggtgacc 60
ttctccaagc gccgctcggg gcttctcaag aaggcgcacg agatctccgt gctctgcgac 120
gccgaggtcg gcctcatcat cttctccacc aagggaaagc tctacgagtt ctccaccgag 180
tcatgtatgg acaaaattct tgaacggtat gagcgctatt cttatgcaga aaaggttctc 240
gtttcaagtg aatctgaaat tcagggaaac tggtgtcacg aatataggaa actgaaggcg 300
aaggttgaga caatacagaa atgtcaaaaa catctcatgg gagaggatct tgaatctttg 360
aatctcaagg agttgcagca actggagcag cagctggaaa gctcactgaa acatatcaga 420
tccaggaaga accaacttat gcacgaatcc atttctgagc tgcagaagaa ggagaggtca 480
ctgcaggagg agaataaagt tctccagaag gaactcgtgg agaagcagaa ggcccatgcg 540
gcgcagcaag atcaaactca gcctcaaacc agctcttctt cttcttcctt catgctgagg 600
gatgctcccc ctgccgcaaa taccagcatt catccagcgg cggcaggcga gagggcagag 660
gatgcggcag tgcagccgca ggccccaccc cggacggggc ttccaccgtg gatggtgagc 720
cacatcaacg ggtga 735

<210> 61
<211> 244
<212> PRT
<213> Triticum monococcum

<400> 61
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala His Ala Ala Gln Gln Asp Gln Thr Gln Pro Gln Thr Ser Ser
            180                 185                 190
Ser Ser Ser Ser Phe Met Leu Arg Asp Ala Pro Pro Ala Ala Asn Thr
        195                 200                 205
Ser Ile His Pro Ala Ala Ala Gly Glu Arg Ala Glu Asp Ala Ala Val
    210                 215                 220
Gln Pro Gln Ala Pro Pro Arg Thr Gly Leu Pro Pro Trp Met Val Ser
225                 230                 235                 240
His Ile Asn Gly


<210>  62
<211>  735
<212>  DNA
<213>  Triticum aestivum

<400>  62
atggggcggg ggaaggtgca gctgaagcgg atcgagaaca agatcaaccg gcaggtgacc      60
ttctccaagc gccgctcggg gcttctcaag aaggcgcacg agatctccgt gctctgcgac     120
gccgaggtcg gcctcatcat cttctccacc aagggaaagc tctacgagtt ctccaccgag     180
tcatgtatgg acaaaattct tgaacggtat gagcgctatt cttatgcaga aaaggttctc     240
gtttcaagtg aatctgaaat tcagggaaac tggtgtcacg aatataggaa actgaaggcg     300
aaggttgaga caatacagaa atgtcaaaag catctcatgg gagaggatct tgaatctttg     360
aatctcaagg agttgcagca actggagcag cagctggaaa gctcactgaa acatatcaga     420
tccaggaaga accaacttat gcacgaatcc atttctgagc ttcagaagaa ggagaggtca     480
ctgcaggagg agaataaagt tctccagaag gaactcgtgg agaagcagaa ggcccatgcg     540
gcgcagcaag atcaaactca gcctcaaacc agctcttcat cttcttcctt catgctgagg     600
gatgctcccc ctgccgcaaa taccagcatt catccagcgg caacaggcga gagggcagag     660
gatgcggcag tgcagccgca ggccccaccc cggacggggc ttccaccgtg gatggtgagc     720
cacatcaacg ggtga                                                      735

<210>  63
<211>  244
<212>  PRT
<213>  Triticum aestivum

<400>  63
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala His Ala Ala Gln Gln Asp Gln Thr Gln Pro Gln Thr Ser Ser
            180                 185                 190
Ser Ser Ser Ser Phe Met Leu Arg Asp Ala Pro Pro Ala Ala Asn Thr
        195                 200                 205
Ser Ile His Pro Ala Ala Thr Gly Glu Arg Ala Glu Asp Ala Ala Val
    210                 215                 220
Gln Pro Gln Ala Pro Pro Arg Thr Gly Leu Pro Pro Trp Met Val Ser
225                 230                 235                 240
His Ile Asn Gly

<210> 64
<211> 738
<212> DNA
<213> Lolium perenne

<400> 64
atggggcgcg gcaaggtgca gctcaagcgg atcgagaaca agatcaaccg ccaggtcacc 60
ttctccaagc gccgctcggg gctgctcaag aaggcgcacg agatctccgt gctctgcgac 120
gccgaggtcg ggctcatcat cttctccacc aagggaaagc tctacgagtt cgcaaccgac 180
tcatgtatgg acaaaattct tgagcggtat gagcgctact cctatgcaga gaaagtgctc 240
atttcaaccg aatctgaaat tcagggaaac tggtgtcatg aatataggaa actgaaggcg 300
aaggttgaga caatacagag atgtcaaaag catctaatgg gagaggatct tgaatcattg 360
aatctcaagg agttgcagca actagagcag cagctggaaa gttcactgaa acatattaga 420
gccagaaaga accagcttat gcacgaatcc atatctgagc ttcaaaagaa ggagaggtca 480
ctgcaggagg agaataaaat tctccagaag gaactcatag agaagcagaa ggcccacacg 540
cagcaagcgc agtgggagca aactcagccc caaaccagct cttcctcctc ctcctttatg 600
atgggggaag ctaccccagc aacaaattgc agtaatcccc cagcagcggc cagcgacaga 660
gcagaggatg cgacggggca gccttcagct cgcacggtgc ttccaccatg gatggtgagt 720
cacatcaaca atggctga 738

<210> 65
<211> 245
<212> PRT
<213> Lolium perenne

<400> 65
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

        35                  40                  45
Ser Thr Lys Gly Lys Leu Tyr Glu Phe Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Thr Glu Ser Glu Ile Gln Gly Asn Trp Cys His Glu Tyr Arg
                85                  90                  95
Lys Leu Lys Ala Lys Val Glu Thr Ile Gln Arg Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Ile Leu Gln Lys Glu Leu Ile Glu Lys Gln
                165                 170                 175
Lys Ala His Thr Gln Gln Ala Gln Trp Glu Gln Thr Gln Pro Gln Thr
            180                 185                 190
Ser Ser Ser Ser Ser Ser Phe Met Met Gly Glu Ala Thr Pro Ala Thr
        195                 200                 205
Asn Cys Ser Asn Pro Pro Ala Ala Ala Ser Asp Arg Ala Glu Asp Ala
    210                 215                 220
Thr Gly Gln Pro Ser Ala Arg Thr Val Leu Pro Pro Trp Met Val Ser
225                 230                 235                 240
His Ile Asn Asn Gly
                245

<210> 66
<211> 738
<212> DNA
<213> Lolium temulentum

<400> 66
atggggcgcg gcaaggtgca gctcaagcgg atcgagaaca agatcaaccg ccaggtcacc 60
ttctccaagc gccgctcagg cctgctcaag aaggcgcacg agatctccgt gctctgcgac 120
gcagaggtcg ggctcatcat cttctccacc aagggaaagc tctacgagtt cgccaccgac 180
tcatgtatgg acaaaattct tgagcggtat gagcgctact cctatgcaga gaaagtgctc 240
atttcaactg aatctgaaat tcagggaaac tggtgtcatg aatataggaa actgaaggcg 300
aaggttgaga caatacagag atgtcaaaag catctaatgg gagaggatct tgaatcattg 360
aatctcaagg agttgcagca actagagcag cagctggaaa gttcactgaa acatattaga 420
tccagaaaga gccagcttat gcacgaatcc atatctgagc ttcaaaagaa ggagaggtca 480
ctgcaagagg agaataaaat tctccagaag gaactcatag agaagcagaa ggcccacacg 540
cagcaagcgc agttggagca aactcagccc caaaccagct cttcctcctc ctcctttatg 600
atgggggaag ctaccccagc aacaaatcgc agtaatcccc cagcagcggc cagcgacaga 660
gcagaggatg cgacggggca gcctccagct cgcacggtgc ttccaccatg gatggtgagt 720
cacctcaaca atggctga 738

<210> 67
<211> 245
<212> PRT
<213> Lolium temulentum

<400> 67
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

35                  40                  45
Ser Thr Lys Gly Lys Leu Tyr Glu Phe Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Thr Glu Ser Glu Ile Gln Gly Asn Trp Cys His Glu Tyr Arg
                85                  90                  95
Lys Leu Lys Ala Lys Val Glu Thr Ile Gln Arg Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
Gln Leu Met His Glu Ser Ile Ser Glu Leu Gln Lys Lys Glu Arg Ser
145                 150                 155                 160
Leu Gln Glu Glu Asn Lys Ile Leu Gln Lys Glu Leu Ile Glu Lys Gln
                165                 170                 175
Lys Ala His Thr Gln Gln Ala Gln Leu Glu Gln Thr Gln Pro Gln Thr
            180                 185                 190
Ser Ser Ser Ser Ser Ser Phe Met Met Gly Glu Ala Thr Pro Ala Thr
        195                 200                 205
Asn Arg Ser Asn Pro Pro Ala Ala Ala Ser Asp Arg Ala Glu Asp Ala
    210                 215                 220
Thr Gly Gln Pro Pro Ala Arg Thr Val Leu Pro Pro Trp Met Val Ser
225                 230                 235                 240
His Leu Asn Asn Gly
                245

<210>  68
<211>  738
<212>  DNA
<213>  Zea mays

<400>  68
atggggcgcg ggaaggtgca gctgaagcgg atcgagaaca agatcaaccg ccaggtgacc      60
ttctccaagc gccgctcggg gctgctcaag aaggcgcacg agatctccgt gctctgcgac     120
gccgaggtcg cgctcatcat cttctccacc aaagggaagc tctacgagta ttccaccgat     180
tcatgtatgg acaaaattct tgaccggtac gagcgctact cctatgcaga aaaggttctt     240
atttcagcag aatctgaaac tcagggcaat tggtgccacg agtatagaaa actaaaggcg     300
aaggtcgaga caatacaaaa atgtcaaaag cacctcatgg gagaggatct tgaaacgttg     360
aatctcaaag agcttcagca actagagcag cagctggaga gttcactgaa acatatcaga     420
accaggaaga accaacttat gctcgagtca atttcggagc tccaacggaa ggagaagtcg     480
ctgcaggagg agaacaaggt tctgcagaag gagctcgcgg agaagcagaa agcccagcgg     540
aagcaagtgc aatggggcca aacccaacag cagaccagtt cgtcttcctc gtgcttcgtg     600
ataagggaag ctgccccaac aacaaatatc agcatttttc ctgtggcagc aggcgggagg     660
ttggtggaag gtgcagcagc gcagccacag gctcgcgttg gactaccacc atggatgctt     720
agccacctga gcagctga                                                   738

<210>  69
<211>  245
<212>  PRT
<213>  Zea mays

<400>  69
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Ile Phe

35                  40                  45
Ser Thr Lys Gly Lys Leu Tyr Glu Tyr Ser Thr Asp Ser Cys Met Asp
    50                  55                  60
Lys Ile Leu Asp Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Thr Leu Asn Leu Lys Glu Leu Gln Gln Leu
        115                 120                 125
Glu Gln Gln Leu Glu Ser Ser Leu Lys His Ile Arg Thr Arg Lys Asn
    130                 135                 140
Gln Leu Met Leu Glu Ser Ile Ser Glu Leu Gln Arg Lys Glu Lys Ser
145                 150                 155                 160
Leu Gln Glu Glu Asn Lys Val Leu Gln Lys Glu Leu Ala Glu Lys Gln
                165                 170                 175
Lys Ala Gln Arg Lys Gln Val Gln Trp Gly Gln Thr Gln Gln Gln Thr
            180                 185                 190
Ser Ser Ser Ser Ser Cys Phe Val Ile Arg Glu Ala Ala Pro Thr Thr
        195                 200                 205
Asn Ile Ser Ile Phe Pro Val Ala Ala Gly Gly Arg Leu Val Glu Gly
    210                 215                 220
Ala Ala Ala Gln Pro Gln Ala Arg Val Gly Leu Pro Pro Trp Met Leu
225                 230                 235                 240
Ser His Leu Ser Ser
                245

<210>  70
<211>  738
<212>  DNA
<213>  Zea mays

<400>  70
atggggcgcg ggaaggtgca gctgaagcgg atcgagaaca agatcaaccg ccaggtgaca      60
ttctccaagc gccgctcggg gctactcaag aaggcgcacg agatctccgt gctctgcgac     120
gccgaggtcg cgctcatcat cttctccacc aagggcaagc tctacgagta ctctaccgat     180
tcatgtatgg acaaaattct tgaacggtat gagcgctact cctatgcaga aaaggttctc     240
atttccgcag aatatgaaac tcagggcaat tggtgccatg aatatagaaa actaaaggcg     300
aaggtcgaga caatacagaa atgtcaaaag cacctcatgg gagaggatct tgaaactttg     360
aatctcaaag agcttcagca actagagcag cagctggaga gttcactgaa acatatcaga     420
acaaggaaga gccagcttat ggtcgagtca atttcagcgc tccaacggaa ggagaagtca     480
ctgcaggagg agaacaaggt tctgcagaag gagctcgcgg agaagcagaa agaccagcgg     540
cagcaagtgc aacgggacca aactcaacag cagaccagtt cgtcttccac gtccttcatg     600
ttaagggaag ctgccccaac aacaaatgtc agcatcttcc ctgtggcagc aggcgggagg     660
gtggtggaag gggcagcagc gcagccgcag gctcgcgttg gactgccacc atggatgctt     720
agccatctga gctgctga                                                   738

<210>  71
<211>  245
<212>  PRT
<213>  Zea mays

<400>  71
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Ile Phe

        35                  40                  45
Ser Thr Lys Gly Lys Leu Tyr Glu Tyr Ser Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Tyr Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Thr Leu Asn Leu Lys Glu Leu Gln Gln Leu
        115                 120                 125
Glu Gln Gln Leu Glu Ser Ser Leu Lys His Ile Arg Thr Arg Lys Ser
    130                 135                 140
Gln Leu Met Val Glu Ser Ile Ser Ala Leu Gln Arg Lys Glu Lys Ser
145                 150                 155                 160
Leu Gln Glu Glu Asn Lys Val Leu Gln Lys Glu Leu Ala Glu Lys Gln
                165                 170                 175
Lys Asp Gln Arg Gln Gln Val Gln Arg Asp Gln Thr Gln Gln Gln Thr
            180                 185                 190
Ser Ser Ser Ser Thr Ser Phe Met Leu Arg Glu Ala Ala Pro Thr Thr
        195                 200                 205
Asn Val Ser Ile Phe Pro Val Ala Ala Gly Gly Arg Val Val Glu Gly
    210                 215                 220
Ala Ala Ala Gln Pro Gln Ala Arg Val Gly Leu Pro Pro Trp Met Leu
225                 230                 235                 240
Ser His Leu Ser Cys
                245

<210> 72
<211> 762
<212> DNA
<213> Oryza sativa

<400> 72
atggggcggg gcaaggtgca gctgaagcgg atcgagaaca agatcaaccg gcaggtgacc 60
ttctccaagc gcaggtcggg gctgctcaag aaggcgaatg agatctccgt gctctgcgac 120
gccgaggtcg cgctcatcat cttctccacc aagggcaagc tctacgagta cgccaccgac 180
tcatgtatgg acaaaatcct tgaacgttat gagcgctact cctatgcaga aaaggtcctt 240
atttcagctg aatctgacac tcagggcaac tggtgccacg aatataggaa actgaaggct 300
aaggttgaga caatacagaa atgtcaaaag cacctcatgg gagaggatct tgaatctttg 360
aatctcaaag agctgcagca gctggagcag cagctggaaa attcgttgaa acatatcaga 420
tccagaaaga gccaactaat gctcgagtcc attaacgagc ttcaacggaa ggaaaagtca 480
ctgcaggagg agaataaggt cctacagaaa gaaaaccctt gctccttcct acagctggtg 540
gagaagcaga aagtccagaa gcaacaagtg caatggaccc agacacaacc tcaaacaagt 600
tcctcatcat cctccttcat gatgagggaa gcccttccaa caactaatat cagtaactac 660
cctgcagcag ctggcgaaag gatagaggat gtagcagcag ggcagccaca gcatgttcgc 720
attgggctgc caccatggat gctgagccac atcaacggct aa 762

<210> 73
<211> 253
<212> PRT
<213> Oryza sativa

<400> 73
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
Asn Glu Ile Ser Val Leu Cys Asp Ala Glu Val Ala Leu Ile Ile Phe

        35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Asp Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Asn Ser Leu Lys His Ile Arg Ser Arg Lys Ser
    130                 135                 140
Gln Leu Met Leu Glu Ser Ile Asn Glu Leu Gln Arg Lys Glu Lys Ser
145                 150                 155                 160
Leu Gln Glu Glu Asn Lys Val Leu Gln Lys Glu Asn Pro Cys Ser Phe
                165                 170                 175
Leu Gln Leu Val Glu Lys Gln Lys Val Gln Lys Gln Gln Val Gln Trp
            180                 185                 190
Asp Gln Thr Gln Pro Gln Thr Ser Ser Ser Ser Ser Ser Phe Met Met
        195                 200                 205
Arg Glu Ala Leu Pro Thr Thr Asn Ile Ser Asn Tyr Pro Ala Ala Ala
    210                 215                 220
Gly Glu Arg Ile Glu Asp Val Ala Ala Gly Gln Pro Gln His Val Arg
225                 230                 235                 240
Ile Gly Leu Pro Pro Trp Met Leu Ser His Ile Asn Gly
                245                 250

<210> 74
<211> 741
<212> DNA
<213> Oryza sativa

<400> 74
atggggcggg gcaaggtgca gctgaagcgg atcgagaaca agatcaaccg gcaggtgacc 60
ttctccaagc gcaggtcaaa actgctcaag aaggcgaatg agatctccgt gctctgcgac 120
gccgaggtcg cgctcatcat cttctccacc aagggcaagc tctacgagta cgccaccgac 180
tcatgtatgg acaaaatcct tgaacgttat gagcgctact cctatgcaga aaaggtcctt 240
atttcagctg aatctgacac tcagggcaac tggtgccacg aatataggaa actgaaggct 300
aaggttgaga caatacagaa atgtcaaaag cacctcatgg gagaggatct tgaatctttg 360
aatctcaaag agctgcagca gctggagcag cagctggaaa attcgttgaa acatatcaga 420
tccagaaaga gccaactaat gctcgagtcc attaacgagc ttcaacggaa ggaaaagtca 480
ctgcaggagg agaataaggt cctacagaaa gaactggtgg agaagcagaa agtccagaag 540
caacaagtgc aatggaccca gacacaacct caaacaagtt cctcatcatc ctccttcatg 600
atgagggaag cccttccaac aactaatatc agtaactacc ctgcagcagc tggcgaaagg 660
atagaggatg tagcagcagg gcagccacag catgaacgca ttgggctgcc accatggatg 720
ctgagccaca tcaacggcta a 741

<210> 75
<211> 246
<212> PRT
<213> Oryza sativa

<400> 75
Met Gly Arg Gly Lys Val Gln Leu Lys Arg Ile Glu Asn Lys Ile Asn
1               5                   10                  15
Arg Gln Val Thr Phe Ser Lys Arg Arg Ser Lys Leu Leu Lys Lys Ala
            20                  25                  30
Asn Glu Ile Ser Val Leu Cys Asp Ala Glu Val Ala Leu Ile Ile Phe

35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Asp Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Asn Ser Leu Lys His Ile Arg Ser Arg Lys Ser
    130                 135                 140
Gln Leu Met Leu Glu Ser Ile Asn Glu Leu Gln Arg Lys Glu Lys Ser
145                 150                 155                 160
Leu Gln Glu Glu Asn Lys Val Leu Gln Lys Glu Leu Val Glu Lys Gln
                165                 170                 175
Lys Val Gln Lys Gln Gln Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Asn Tyr Pro Ala Ala Ala Gly Glu Arg Ile Glu Asp Val
    210                 215                 220
Ala Ala Gly Gln Pro Gln His Glu Arg Ile Gly Leu Pro Pro Trp Met
225                 230                 235                 240
Leu Ser His Ile Asn Gly
                245

<210>  76
<211>  735
<212>  DNA
<213>  Dendrocalamus latiflorus

<400>  76
atggggcgcg ggaaggtgca gctgaagcgg atcgagaaca agatcaaccg gcaagtgact      60
ttctccaagc gccggtcggg gctgctcaag aaggcgcatg agatctccgt cctctgcgac     120
gccgaggtcg gccttatcat cttctccacc aagggcaagc tctacgagta cgccaccgac     180
tcatgtatgg acaaaattct tgaacggtac gagcgttact cctatgcaga aaaggttctt     240
atttcagccg aatctgaaac tcagggcaac tggtgtcacg aatataggaa actgaaggcg     300
aaggttgaga cgatacagaa atgtcaaaag cacctcatgg gagaggatcc tgaatctttg     360
aatctcaagg agctgcagca actcgagcag cagctggaaa gttcagtgaa acatatcaga     420
tccagaaaga gccagcttat gctcgagtcc atttccgagc ttcaaaagaa ggagaagtca     480
ctgcaggagg agaacaaggt tctgcagaag gaactcgtgg agaagcagca ggtccataaa     540
cggttagtgc aatggaccca aactcagccg caaactagtt cctcttcctc gtccttcatg     600
atgagggaag ctctcccaac aacaaatatc agtatttacg ctgcggcagc cggcgagagg     660
gcagaggacg cagcagggca gcctcagatt cacattgggc tgccgccatg gatggtgagc     720
cacatcaacg gctaa                                                      735

<210>  77
<211>  244
<212>  PRT
<213>  Dendrocalamus latiflorus

<400>  77
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

        35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Pro Glu Ser Leu Asn Leu Lys Glu Leu Gln Gln Leu
        115                 120                 125
Glu Gln Gln Leu Glu Ser Ser Val Lys His Ile Arg Ser Arg Lys Ser
    130                 135                 140
Gln Leu Met Leu Glu Ser Ile Ser Glu Leu Gln Lys Lys Glu Lys Ser
145                 150                 155                 160
Leu Gln Glu Glu Asn Lys Val Leu Gln Lys Glu Leu Val Glu Lys Gln
                165                 170                 175
Gln Val His Lys Arg Leu Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Ile Tyr Ala Ala Ala Ala Gly Glu Arg Ala Glu Asp Ala
    210                 215                 220
Ala Gly Gln Pro Gln Ile His Ile Gly Leu Pro Pro Trp Met Val Ser
225                 230                 235                 240
His Ile Asn Gly

<210> 78
<211> 735
<212> DNA
<213> Dendrocalamus latiflorus

<400> 78
atggggcgcg ggaaggtgca gctgaagcgg atcgagaaca agatcaaccg gcaagtgact 60
ttctccaagc gccggtcggg gctgctcaag aaggcgcatg agatctccgt cctctgcgac 120
gccgaggtcg gccttatcat cttctccacc aagggcaagc tctacgagta cgccaccgac 180
tcatgtatgg acaaaattct tgaacggtac gagcgttact cctatgcaga aaaggttctt 240
atttcagccg aatctgaaac tcagggcaac tggtgtcacg aatataggaa actgaaggcg 300
aaggttgaga cgatacagaa atgtcaaaag cacctcatgg gagaggatct tgaatctttg 360
aatctcaagg agctgcagca actcgagcag cagctggaaa gttcagtgaa acatatcaga 420
tccagaaaga gccagcttat gctcgagtcc atttccgagc ttcaaaagaa ggagaagtca 480
ctgcaggagg agaacaaggt tctgcagaag gaactcgtgg agaagcagca ggtccataaa 540
cggttagtgc aatggaccca aactcagccg caaactagtt cctcttcctc gtccttcatg 600
atgagggaag ctctcccaac aacaaatatc agtatttacg ctgcggcagc cggcgagagg 660
gcagaggacg cagcagggca gcctcagatt cacattgggc tgccgccatg gatggtgagc 720
cacatcaacg gctaa 735

<210> 79
<211> 244
<212> PRT
<213> Dendrocalamus latiflorus

<400> 79
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe

        35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Val Lys His Ile Arg Ser Arg Lys Ser
    130                 135                 140
Gln Leu Met Leu Glu Ser Ile Ser Glu Leu Gln Lys Lys Glu Lys Ser
145                 150                 155                 160
Leu Gln Glu Glu Asn Lys Val Leu Gln Lys Glu Leu Val Glu Lys Gln
                165                 170                 175
Gln Val His Lys Arg Leu Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Ile Tyr Ala Ala Ala Ala Gly Glu Arg Ala Glu Asp Ala
    210                 215                 220
Ala Gly Gln Pro Gln Ile His Ile Gly Leu Pro Pro Trp Met Val Ser
225                 230                 235                 240
His Ile Asn Gly

<210> 80
<211> 813
<212> DNA
<213> Zea mays

<400> 80
atggggcgcg gcaaggtgca gctgaagcgg atagagaaca agataaaccg gcaggtgacc 60
ttctccaagc gccggaacgg gctgctgaag aaggcgcacg agatctccgt cctctgcgac 120
gccgaggtcg ccgtcatcgt cttctccccc aagggcaagc tctacgagta cgcctccgac 180
tcccgcatgg acaaaattct agaacgttat gagcgatatt cctatgctga aaaggctctt 240
atttcagctg aatctgaaag tgagggaaat tggtgccacg aatacaggaa actgaaggcc 300
aaaattgaga ccatacaaag atgccacaag cacctgatgg gagaggatct agagtctttg 360
aatccaaaag agctccaaca actagagcag cagctggaga gctcactgaa gcacatcaga 420
tcaagaaaga gccaccttat ggccgagtca atttctgagc tacagaagaa ggagaggtca 480
ctgcaggagg agaacaagat tctacagaag gaactttcag agaggcagaa ggcggtcgct 540
agccggcagc agcagcagca gcaggtgcag tgggaccagc agacacaggt ccaggtccag 600
acaagctcat cgtcttcttc cttcatgatg aggcaggatc agcagggact gccacctcca 660
caaaacatct gcttcccgcc gttgagcatc ggagagagag gcgaagaggt ggctgcggcg 720
gcgcagcagc agctgcctcc tccggggcag gcgcaaccac agctccgcat cgcaggtctg 780
ccgccatgga tgctgagcca cctcaatgca taa 813

<210> 81
<211> 270
<212> PRT
<213> Zea mays

<400> 81
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30

His Glu Ile Ser Val Leu Cys Asp Ala Glu Val Ala Val Ile Val Phe
        35                  40                  45
Ser Pro Lys Gly Lys Leu Tyr Glu Tyr Ala Ser Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Arg Cys His Lys His Leu
            100                 105                 110
Met Gly Glu Asp Leu Glu Ser Leu Asn Pro Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Ala Glu Ser Ile Ser Glu Leu Gln Lys Lys Glu Arg Ser
145                 150                 155                 160
Leu Gln Glu Glu Asn Lys Ile Leu Gln Lys Glu Leu Ser Glu Arg Gln
                165                 170                 175
Lys Ala Val Ala Ser Arg Gln Gln Gln Gln Gln Gln Val Gln Trp Asp
            180                 185                 190
Gln Gln Thr Gln Val Gln Val Gln Thr Ser Ser Ser Ser Ser Ser Phe
        195                 200                 205
Met Met Arg Gln Asp Gln Gln Gly Leu Pro Pro Pro Gln Asn Ile Cys
    210                 215                 220
Phe Pro Pro Leu Ser Ile Gly Glu Arg Gly Glu Glu Val Ala Ala Ala
225                 230                 235                 240
Ala Gln Gln Gln Leu Pro Pro Pro Gly Gln Ala Gln Pro Gln Leu Arg
                245                 250                 255
Ile Ala Gly Leu Pro Pro Trp Met Leu Ser His Leu Asn Ala
            260                 265                 270

<210>  82
<211>  689
<212>  DNA
<213>  Sorghum bicolor

<400>  82
gcaaggtgca gctcaagcgg atagagaaca agataaaccg gcaggtgacc ttctccaagc      60
gccgcaacgg gctgctcaag aaggcgcacg agatctccgt cctctgcgac gccgaggtcg     120
ccgtcatcgt cttctccccc aagggcaagc tctatgagta cgccaccgac tcccgcatgg     180
acaaaattct cgaacgttat gagcgatatt cctatgctga aaaggctctt atttcagctg     240
aatctgaaag tgagggaaac tggtgccacg aatacaggaa actgaaggcc aaaattgaga     300
ccattcaaaa atgccacaag cacctgatgg gagaggatct agagtctttg aatcccaaag     360
agctccaaca actagagcag cagctggaga gctcactgaa gcacatcaga tcaagaaaga     420
gccaccttat ggctgagtct atttctgaac tacagaagaa ggagaggtca ctgcaggagg     480
agaacaaggc tctacagaag gaacttgcgg agaggcagaa ggcggccgcg agcaggcagc     540
agcagcaagg tgcagtggga ccagcagaca cagacccagg cccagacaag ctcatcatcg     600
tcctccttca tgatgaggca ggatcagcag ggtctgccgc ctccacaaaa catatgcttc     660
ccgccgctga taatcggaga gagaggtga                                       689

<210>  83
<211>  228
<212>  PRT
<213>  Sorghum bicolor

<400>  83
Lys Val Gln Leu Lys Arg Ile Glu Asn Lys Ile Asn Arg Gln Val Thr
1               5                   10                  15
Phe Ser Lys Arg Arg Asn Gly Leu Leu Lys Lys Ala His Glu Ile Ser

                20                  25                  30
Val Leu Cys Asp Ala Glu Val Ala Val Ile Val Phe Ser Pro Lys Gly
        35                  40                  45
Lys Leu Tyr Glu Tyr Ala Thr Asp Ser Arg Met Asp Lys Ile Leu Glu
    50                  55                  60
Arg Tyr Glu Arg Tyr Ser Tyr Ala Glu Lys Ala Leu Ile Ser Ala Glu
65                  70                  75                  80
Ser Glu Ser Glu Gly Asn Trp Cys His Glu Tyr Arg Lys Leu Lys Ala
                85                  90                  95
Lys Ile Glu Thr Ile Gln Lys Cys His Lys His Leu Met Gly Glu Asp
            100                 105                 110
Leu Glu Ser Leu Asn Pro Lys Glu Leu Gln Gln Leu Glu Gln Gln Leu
        115                 120                 125
Glu Ser Ser Leu Lys His Ile Arg Ser Arg Lys Ser His Leu Met Ala
    130                 135                 140
Glu Ser Ile Ser Glu Leu Gln Lys Lys Glu Arg Ser Leu Gln Glu Glu
145                 150                 155                 160
Asn Lys Ala Leu Gln Lys Glu Leu Ala Glu Arg Gln Lys Ala Ala Ala
                165                 170                 175
Ser Arg Gln Gln Gln Gln Gly Ala Val Gly Pro Ala Asp Thr Asp Pro
            180                 185                 190
Gly Pro Asp Lys Leu Ile Ile Val Leu Leu His Asp Glu Ala Gly Ser
        195                 200                 205
Ala Gly Ser Ala Ala Ser Thr Lys His Met Leu Pro Ala Ala Asp Asn
    210                 215                 220
Arg Arg Glu Arg
225

<210>  84
<211>  822
<212>  DNA
<213>  Zea mays

<400>  84
atggggcgcg gcaaggtaca gctgaagcgg atagagaaca agataaaccg gcaggtgacc      60
ttctccaagc gccggaacgg cctgctcaag aaggcgcacg agatctccgt cctctgcgat     120
gccgaggtcg ccgtcatcgt cttctccccc aagggcaagc tctacgagta cgccaccgac     180
tcccgcatgg acaaaattct tgaacgctat gagcgatatt cctatgctga aaaggctctt     240
atttcagctg aatctgaaag tgagggaaat tggtgccacg aatacaggaa actgaaggcc     300
aaaattgaga ccatacaaaa atgccacaag cacctgatgg gagaggatct agagtctttg     360
aatcccaaag agctccagca actagagcag cagctggata gctcactgaa gcacatcaga     420
tcaaggaaga gccaccttat ggccgagtct atttctgagc tacagaagaa ggagaggtca     480
ctgcaggagg agaacaaggc tctgcagaag gaacttgcgg agaggcagaa ggccgtcgcg     540
agccggcagc agcagcaaca gcagcaggtg cagtgggacc agcagacaca tgcccaggcc     600
cagacaagct catcatcgtc ctccttcatg atgaggcagg atcagcaggg actgccgcct     660
ccacacaaca tctgcttccc gccgttgaca atgggagata gaggtgaaga gctggctgcg     720
gcggcggcgg cgcagcagca gcagccactg ccggggcagg cgcaaccgca gctccgcatc     780
gcaggtctgc caccatggat gctgagccac ctcaatgcat aa                        822

<210>  85
<211>  273
<212>  PRT
<213>  Zea mays

<400>  85
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30

His Glu Ile Ser Val Leu Cys Asp Ala Glu Val Ala Val Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Pro Lys Glu Leu Gln Gln Leu
        115                 120                 125
Glu Gln Gln Leu Asp Ser Ser Leu Lys His Ile Arg Ser Arg Lys Ser
    130                 135                 140
His Leu Met Ala Glu Ser Ile Ser Glu Leu Gln Lys Lys Glu Arg Ser
145                 150                 155                 160
Leu Gln Glu Glu Asn Lys Ala Leu Gln Lys Glu Leu Ala Glu Arg Gln
                165                 170                 175
Lys Ala Val Ala Ser Arg Gln Gln Gln Gln Gln Gln Gln Val Gln Trp
            180                 185                 190
Asp Gln Gln Thr His Ala Gln Ala Gln Thr Ser Ser Ser Ser Ser Ser
        195                 200                 205
Phe Met Met Arg Gln Asp Gln Gln Gly Leu Pro Pro Pro His Asn Ile
    210                 215                 220
Cys Phe Pro Pro Leu Thr Met Gly Asp Arg Gly Glu Glu Leu Ala Ala
225                 230                 235                 240
Ala Ala Ala Ala Gln Gln Gln Gln Pro Leu Pro Gly Gln Ala Gln Pro
                245                 250                 255
Gln Leu Arg Ile Ala Gly Leu Pro Pro Trp Met Leu Ser His Leu Asn
            260                 265                 270
Ala

<210> 86
<211> 786
<212> DNA
<213> Lolium temulentum

<400> 86
atgggtcgcg gcaaggtgca gctgaagcgg atagagaaca agataaaccg tcaggtgaca 60
ttctccaagc gccgcaacgg gctactcaag aaggcgcacg agatctccgt cctctgcgac 120
gccgaggtcg ccgtcgtcgt cttctccccg aaagggaagc tctatgagta cgccactgac 180
tccagcatgg acaaaattct tgaacgttat gaacgctact cttatgctga aaaggctttg 240
atttcagctg aatctgaaag tgagggaaat tggtgccatg aatacaggaa gctgaaggcg 300
aagattgaga ctatacaaaa atgtcacaag cacctcatgg gggaggatct ggagtgtcta 360
aacctgaaag agctccaaca actagagcag cagctggaga gttcattgaa gcacatcaga 420
tcgagaaaga gccaccttat gatggagtcc atttctgagc tacagaagaa ggagcggtca 480
ctccaggagg agaacaaggc tctacagaag gaactggtgg agaggcagaa ggcggccagg 540
cagcagcagc aagagcagtg ggaccgtcag acccaaacac aacaagccca aaaccaacct 600
caggcccaga cgagctcatc atcttcctcc ttcatgatga gggatcagca ggcccatgct 660
caacaaaaca tctgttaccc gctggtgaca atgggtggag aggctgtggc cgcggcgcca 720
gggcagcagg ggcagcttcg catcggaggc ctgccaccat ggatgctgag ccacctcaac 780
gcttga 786

<210> 87
<211> 261
<212> PRT
<213> Lolium temulentum

<400> 87
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Val Val Val Phe
        35                  40                  45
Ser Pro Lys Gly Lys Leu Tyr Glu Tyr Ala Thr Asp Ser Ser Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Cys Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Met Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Ala Ala Arg Gln Gln Gln Gln Glu Gln Trp Asp Arg Gln Thr Gln
            180                 185                 190
Thr Gln Gln Ala Gln Asn Gln Pro Gln Ala Gln Thr Ser Ser Ser Ser
        195                 200                 205
Ser Ser Phe Met Met Arg Asp Gln Gln Ala His Ala Gln Gln Asn Ile
    210                 215                 220
Cys Tyr Pro Leu Val Thr Met Gly Gly Glu Ala Val Ala Ala Ala Pro
225                 230                 235                 240
Gly Gln Gln Gly Gln Leu Arg Ile Gly Gly Leu Pro Pro Trp Met Leu
                245                 250                 255
Ser His Leu Asn Ala
            260

<210>  88
<211>  786
<212>  DNA
<213>  Lolium perenne

<400>  88
atgggtcgcg gcaaggtgca gctgaagcgg atagagaaca agataaaccg tcaggtgacc      60
ttctccaagc gccgcaacgg gctactcaag aaggcgcacg agatctccgt cctctgcgac     120
gccgaggtcg ccgtcgtcgt cttctccccg aaagggaagc tctatgagta cgccactgac     180
tccagcatgg acaaaattct tgaacgttat gaacgctact cttatgctga aaaggctttg     240
atttcagctg aatctgaaag tgagggaaat tggtgccatg aatacaggaa actgaaggcg     300
aagattgaga ctatacaaaa atgtcacaag cacctcatgg gggaggatct ggagtgtcta     360
aacctgaaag agctccaaca actagagcag cagctggaga gttcattgaa gcacatcaga     420
tcgagaaaga gccaccttat gatggagtcc atttctgagc tacagaagaa ggagcggtca     480
ctccaggagg agaacaaggc tctacagaag gaactggtgg agaggcagaa ggcggccagg     540
cagcagcagc aagagcagtg ggaccgtcag acccaaacac aacaagccca aaaccaacct     600
caggcccaga cgagctcatc atcttcctcc ttcatgatga gggatcagca ggcccatgct     660
caacaaaaca tctgttaccc gccggtgaca atgggtggag aggctgtggc cgcggcgcca     720
gggcagcagg ggcagcttcg catcggaggc ctgccaccat ggatgctgag ccacctcaac     780
gcttga                                                                786

<210>  89
<211>  261

<212>  PRT
<213>  Lolium perenne

<400>  89
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Val Val Val Phe
        35                  40                  45
Ser Pro Lys Gly Lys Leu Tyr Glu Tyr Ala Thr Asp Ser Ser Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Cys Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Met Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Ala Ala Arg Gln Gln Gln Gln Glu Gln Trp Asp Arg Gln Thr Gln
            180                 185                 190
Thr Gln Gln Ala Gln Asn Gln Pro Gln Ala Gln Thr Ser Ser Ser Ser
        195                 200                 205
Ser Ser Phe Met Met Arg Asp Gln Gln Ala His Ala Gln Gln Asn Ile
    210                 215                 220
Cys Tyr Pro Pro Val Thr Met Gly Gly Glu Ala Val Ala Ala Ala Pro
225                 230                 235                 240
Gly Gln Gln Gly Gln Leu Arg Ile Gly Gly Leu Pro Pro Trp Met Leu
                245                 250                 255
Ser His Leu Asn Ala
            260

<210>  90
<211>  831
<212>  DNA
<213>  Hordeum vulgare

<400>  90
atgggtcgcg gtaaggtgca gctgaagcgg atagagaaca agataaatcg gcaggtgacc      60
ttctccaagc gccgcaacgg gctcctgaag aaggcgcacg agatctccgt cctctgtgac     120
gcagaggtcg ccgtcatcgt cttctccccc aaaggcaagc tctatgagta cgccaccgac     180
tccagcatgg acaaaattct tgaacgttat gagcgctact cttatgctga aaaggctctt     240
atttcagctg aatctgaaag tgaggggaat tggtgtcatg aatacaggaa acttaaggcg     300
aagattgaga ccatacagaa gtgtcacaag cacctcatgg gagaggatct ggattctctg     360
aacctcaaag aactccaaca actggagcag cagctggaga gttcattgaa gcacatcaga     420
tcgagaaaga gccatcttat gatggagtcc atttctgagc tacagaagaa ggagaggtca     480
ctgcaggagg agaacaaggc cctacagaag gaactggtgg agaggcagaa ggcggccagc     540
aggcagcagc agctgcagca gcagcaacaa caacaacaaa tgcaatggga gcaccaagcc     600
cagacccaaa cccataccca tactcaaaac cagccccaag cccagactag ctcatcatct     660
tcctctttca tgatgaggga tcagcaggcc catgcccctc aacagaacat ttgtagctac     720
ccaccggtga cgatgggtgg ggaggcgacg gcggcggcgg cggcgccgga gcagcaggct     780
cagcttcgca tatgcctacc gccatggatg ctgagccacc tcaacgcttg a              831

<210> 91
<211> 276
<212> PRT
<213> Hordeum vulgare

<400> 91
   Met Gly Arg Gly Lys Val Gln Leu Lys Arg Ile Glu Asn Lys Ile Asn
   1 5 10 15
   Arg Gln Val Thr Phe Ser Lys Arg Arg Asn Gly Leu Leu Lys Lys Ala
   20 25 30
   His Glu Ile Ser Val Leu Cys Asp Ala Glu Val Ala Val Ile Val Phe
   35 40 45
   Ser Pro Lys Gly Lys Leu Tyr Glu Tyr Ala Thr Asp Ser Ser Met Asp
   50 55 60
   Lys Ile Leu Glu Arg Tyr Glu Arg Tyr Ser Tyr Ala Glu Lys Ala Leu
   65 70 75 80
   Ile Ser Ala Glu Ser Glu Ser Glu Gly Asn Trp Cys His Glu Tyr Arg
   85 90 95
   Lys Leu Lys Ala Lys Ile Glu Thr Ile Gln Lys Cys His Lys His Leu
   100 105 110
   Met Gly Glu Asp Leu Asp Ser Leu Asn Leu Lys Glu Leu Gln Gln Leu
   115 120 125
   Glu Gln Gln Leu Glu Ser Ser Leu Lys His Ile Arg Ser Arg Lys Ser
   130 135 140
   His Leu Met Met Glu Ser Ile Ser Glu Leu Gln Lys Lys Glu Arg Ser
   145 150 155 160
   Leu Gln Glu Glu Asn Lys Ala Leu Gln Lys Glu Leu Val Glu Arg Gln
   165 170 175
   Lys Ala Ala Ser Arg Gln Gln Gln Leu Gln Gln Gln Gln Gln Gln Gln
   180 185 190
   Gln Met Gln Trp Glu His Gln Ala Gln Thr Gln Thr His Thr His Thr
   195 200 205
   Gln Asn Gln Pro Gln Ala Gln Thr Ser Ser Ser Ser Ser Ser Phe Met
   210 215 220
   Met Arg Asp Gln Gln Ala His Ala Pro Gln Gln Asn Ile Cys Ser Tyr
   225 230 235 240
   Pro Pro Val Thr Met Gly Gly Glu Ala Thr Ala Ala Ala Ala Ala Pro
   245 250 255
   Glu Gln Gln Ala Gln Leu Arg Ile Cys Leu Pro Pro Trp Met Leu Ser
   260 265 270
   His Leu Asn Ala
   275

<210> 92
<211> 804
<212> DNA
<213> Oryza sativa

<400> 92
atggggcggg ggaaggtgca gctgaagcgg atagagaact cgatgaaccg gcaggtgacg 60
ttctccaaga ggaggaatgg attgctgaag aaggcgcacg agatctccgt cctctgcgac 120
gccgaggtcg ccgccatcgt cttctccccc aagggcaagc tctacgagta cgccactgac 180
tccaggatgg acaaaatcct tgaacgttat gagcgctatt catatgctga aaaggctctt 240
atttcagctg aatctgagag tgagggaaat tggtgccatg aatacaggaa gcttaaggca 300
aagattgaga ccatacaaaa atgtcacaaa cacctcatgg gagaggatct agaatccctg 360
aatctcaaag aactccaaca gctagagcag cagctggaga gttcattgaa gcacataaga 420
tcaagaaaga gccaccttat gcttgagtcc atttccgagc tgcagaaaaa ggagaggtca 480
ctgcaggagg agaacaaggc tctgcagaag gaactggtgg agaggcagaa gaatgtgagg 540

ggccagcagc aagtagggca gtgggaccaa acccaggtcc aggcccaggc ccaagcccaa 600
ccccaagccc agacaagctt cttcttcttc ttcatgctga gggatcagca ggcacttctt 660
tcaccacaaa atatctgcta cccgccggtg atgatgggcc agagaaatga tgcggcggcg 720
cggcggcggt ggcggcccaa ggccaggtgc aacttccgca ttggaggctt tccgccatgg 780
atgctgagca ccttcaaggc ttaa 804

<210> 93
<211> 267
<212> PRT
<213> Oryza sativa

<400> 93
Met Gly Arg Gly Lys Val Gln Leu Lys Arg Ile Glu Asn Ser Met Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Phe Phe
        195                 200                 205
Phe Phe Phe Met Leu Arg Asp Gln Gln Ala Leu Leu Ser Pro Gln Asn
    210                 215                 220
Ile Cys Tyr Pro Pro Val Met Met Gly Gln Arg Asn Asp Ala Ala Ala
225                 230                 235                 240
Arg Arg Arg Trp Arg Pro Lys Ala Arg Cys Asn Phe Arg Ile Gly Gly
                245                 250                 255
Phe Pro Pro Trp Met Leu Ser Thr Phe Lys Ala
            260                 265

<210> 94
<211> 804
<212> DNA
<213> Oryza sativa

<400> 94
atggggcggg ggaaggtgca gctgaagcgg atagagaaca agatcaacag gcaggtgacg 60
ttctccaaga ggaggaatgg attgctgaag aaggcgcacg agatctccgt cctctgcgac 120
gccgaggtcg ccgccatcgt cttctccccc aagggcaagc tctacgagta cgccactgac 180
tccaggatgg acaaaatcct tgaacgttat gagcgctatt catatgctga aaaggctctt 240
atttcagctg aatccgagag tgagggaaat tggtgccatg aatacaggaa acttaaggca 300
aagattgaga ccatacaaaa atgtcacaaa cacctcatgg gagaggatca tgaatccctg 360

aatctcaaag aactccaaca gctagagcag cagctggaga gttcattgaa gcacataata 420
tcaagaaaga gccaccttat gcttgagtcc atttccgagc tgcagaaaaa ggagaggtca 480
ctgcaggagg agaacaaggc tctgcagaag gaactggtgg agaggcagaa gaatgtgagg 540
ggccagcagc aagtagggca gtgggaccaa acccaggtcc aggcccaggc ccaagcccaa 600
ccccaagccc agacaagctc ctcctcctcc tccatgctga gggatcagca ggcacttctt 660
ccaccacaaa atatctgcta cccgccggtg atgatgggcg agagaaatga tgcggcggcg 720
gcggcggcgg tggcggcgca gggccaggtg caactccgca tcggaggtct tccgccatgg 780
atgctgagcc acctcaatgc ttaa 804

<210> 95
<211> 267
<212> PRT
<213> Oryza sativa

<400> 95
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp His Glu Ser Leu Asn Leu Lys Glu Leu Gln Gln Leu
        115                 120                 125
Glu Gln Gln Leu Glu Ser Ser Leu Lys His Ile Ile Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Ser Ser
        195                 200                 205
Ser Ser Ser Met Leu Arg Asp Gln Gln Ala Leu Leu Pro Pro Gln Asn
    210                 215                 220
Ile Cys Tyr Pro Pro Val Met Met Gly Glu Arg Asn Asp Ala Ala Ala
225                 230                 235                 240
Ala Ala Ala Val Ala Ala Gln Gly Gln Val Gln Leu Arg Ile Gly Gly
                245                 250                 255
Leu Pro Pro Trp Met Leu Ser His Leu Asn Ala
            260                 265

<210> 96
<211> 668
<212> DNA
<213> Tradescantia virginiana

<400> 96
gggctggtga agaaggctca tgagatctcd atkytrtgtg atgcygaggt tgcgcttatt 60
attttctcta ctaaaggcaa actatacgag tatgccactg attccaaaat ggaaaatatc 120
cttgaacgct atgaacgtta ctcatatgct gagaaggctt taacttcatc agatcctgaa 180

ttacagggaa attggtgcca agagtatgtt aaacttaagg ctaaggttga ggccttacat 240
aaaagccaaa ggcatcttat gggagagcaa ctagaagcgt tggatctcaa agaattgcag 300
caactagagc atcaacttga aggttctttg aggcttgtca ggtcaagaaa gactcaaatg 360
atgttggact ccatttccga acttcagagg aaggaaaagt ctctggaaga gcaaaacaag 420
aacctagaga aggagatttt ggagaagcag aaagaaaagg ctctggcaca ccaagctcac 480
tgggaacagc agaatcagcc actacaaagc actaattcgc ctccaaggcc cttcgtgatt 540
gcagaaactc atccaacact aaacattgga aatttccaag gtagaacaaa taccgtccat 600
gcagaagaaa gtctgcagcg tcagatgagg atcagcagca gcctactgcc mycmtggatg 660
mttcacma 668

<210> 97
<211> 222
<212> PRT
<213> Tradescantia virginiana

<220>
<221> VARIANT
<222> (11)..(11)
<223> Xaa can be any naturally occurring amino acid

<220>
<221> VARIANT
<222> (218)..(218)
<223> Xaa can be any naturally occurring amino acid

<220>
<221> VARIANT
<222> (221)..(221)
<223> Xaa can be any naturally occurring amino acid

<400> 97
Gly Leu Val Lys Lys Ala His Glu Ile Ser Xaa Leu Cys Asp Ala Glu
1               5                   10                  15
Val Ala Leu Ile Ile Phe Ser Thr Lys Gly Lys Leu Tyr Glu Tyr Ala
            20                  25                  30
Thr Asp Ser Lys Met Glu Asn Ile Leu Glu Arg Tyr Glu Arg Tyr Ser
        35                  40                  45
Tyr Ala Glu Lys Ala Leu Thr Ser Ser Asp Pro Glu Leu Gln Gly Asn
    50                  55                  60
Trp Cys Gln Glu Tyr Val Lys Leu Lys Ala Lys Val Glu Ala Leu His
65                  70                  75                  80
Lys Ser Gln Arg His Leu Met Gly Glu Gln Leu Glu Ala Leu Asp Leu
                85                  90                  95
Lys Glu Leu Gln Gln Leu Glu His Gln Leu Glu Gly Ser Leu Arg Leu
            100                 105                 110
Val Arg Ser Arg Lys Thr Gln Met Met Leu Asp Ser Ile Ser Glu Leu
        115                 120                 125
Gln Arg Lys Glu Lys Ser Leu Glu Glu Gln Asn Lys Asn Leu Glu Lys
    130                 135                 140
Glu Ile Leu Glu Lys Gln Lys Glu Lys Ala Leu Ala His Gln Ala His
145                 150                 155                 160
Trp Glu Gln Gln Asn Gln Pro Leu Gln Ser Thr Asn Ser Pro Pro Arg
                165                 170                 175
Pro Phe Val Ile Ala Glu Thr His Pro Thr Leu Asn Ile Gly Asn Phe
            180                 185                 190
Gln Gly Arg Thr Asn Thr Val His Ala Glu Glu Ser Leu Gln Arg Gln
        195                 200                 205
Met Arg Ile Ser Ser Ser Leu Leu Pro Xaa Trp Met Xaa His
    210                 215                 220

<210> 98
<211> 723
<212> DNA
<213> Tradescantia virginiana

<400> 98
gtgcagctga aacggatgga gaacaagatt aacaggcagg tgacgttttc taaacgtcga 60
ggagggctgc tgaagaaagc tcatgagatc tctattctat gtgatgctga gattgctctt 120
attattttct ctactaaagg gaagctctat gagtatgcca ccaattccaa aatggacaat 180
attcttgaac gctatgagcg ttactcatat gctgaaaagg ctctaacttc atcagatcct 240
gatatacagg gaaattggtg ccaagagtat gctaaactta agtctaaggt tgaggcttta 300
tgtaaaagcc aaaggcatct tatgggagag cagcttgaaa cattgaatct caaagaattg 360
cagcaactag agcaacagct cgaaggttct ctaaagcatg tcaggtcaag aaagactcaa 420
gttatgctgg actctatttc tgaacttcag aggaaggaaa agtcactaga ggagcaaaac 480
aagaacctag agaaggagat tttggagaag cagaaaatca aggctcttgc acagcaggct 540
cactgggaac accagaatca accagcacca aggggttcac ctcctaggcc atttgtgatt 600
gcagagtctc atccgacact aaatattgga catttccaag gcaggacaaa tgcagtcgaa 660
gcagaagaaa atcagcagcc tcakatgaga atttgcagta gcctcctgcc cccctggatg 720
ctt 723

<210> 99
<211> 241
<212> PRT
<213> Tradescantia virginiana

<220>
<221> UNSURE
<222> (228)..(228)
<223> Xaa can be any naturally occurring amino acid

<400> 99
Val Gln Leu Lys Arg Met Glu Asn Lys Ile Asn Arg Gln Val Thr Phe
1               5                   10                  15
Ser Lys Arg Arg Gly Gly Leu Leu Lys Lys Ala His Glu Ile Ser Ile
            20                  25                  30
Leu Cys Asp Ala Glu Ile Ala Leu Ile Ile Phe Ser Thr Lys Gly Lys
        35                  40                  45
Leu Tyr Glu Tyr Ala Thr Asn Ser Lys Met Asp Asn Ile Leu Glu Arg
    50                  55                  60
Tyr Glu Arg Tyr Ser Tyr Ala Glu Lys Ala Leu Thr Ser Ser Asp Pro
65                  70                  75                  80
Asp Ile Gln Gly Asn Trp Cys Gln Glu Tyr Ala Lys Leu Lys Ser Lys
                85                  90                  95
Val Glu Ala Leu Cys Lys Ser Gln Arg His Leu Met Gly Glu Gln Leu
            100                 105                 110
Glu Thr Leu Asn Leu Lys Glu Leu Gln Gln Leu Glu Gln Gln Leu Glu
        115                 120                 125
Gly Ser Leu Lys His Val Arg Ser Arg Lys Thr Gln Val Met Leu Asp
    130                 135                 140
Ser Ile Ser Glu Leu Gln Arg Lys Glu Lys Ser Leu Glu Glu Gln Asn
145                 150                 155                 160
Lys Asn Leu Glu Lys Glu Ile Leu Glu Lys Gln Lys Ile Lys Ala Leu
                165                 170                 175
Ala Gln Gln Ala His Trp Glu His Gln Asn Gln Pro Ala Pro Arg Gly
            180                 185                 190
Ser Pro Pro Arg Pro Phe Val Ile Ala Glu Ser His Pro Thr Leu Asn
        195                 200                 205
Ile Gly His Phe Gln Gly Arg Thr Asn Ala Val Glu Ala Glu Glu Asn

210                 215                 220
Gln Gln Pro Xaa Met Arg Ile Cys Ser Ser Leu Leu Pro Pro Trp Met
225                 230                 235                 240
Leu

<210>  100
<211>  599
<212>  DNA
<213>  Tradescantia virginiana

<400>  100
gggctkgtga agaaagctca tgagatctcg gtactttgtg atgctgagct tgctcttatt      60
atcttctctc ccaaaggcaa gctctatgag tatgccaccg attccaaaat ggaaattatt     120
cttgaacgct atgaacgtta cacctacgct gaaaaagctt taattgcatc agatcctgat     180
gtacagggaa actggtgtca tgagtacatt aagcttaaag ctaaatttga ggccttgaat     240
aaaagccaga ggcatcttat gggagaacaa ctagatacgt tgaaccaaaa ggaattgctg     300
caactagaga ctaagcttga aggttctctg aaaaacgtca ggtcaagaaa gactcaactt     360
atgttggatt ccatttctga gcttcaagaa aagggaaagt cactccagga gcaaaacacc     420
tgcctagaaa aggagatttt gggaaaacag aaagacaagg ctcccaaaca gcatgttcag     480
tgggaaaaac agaatcaacc accacctacc tcttctgcgc caatgccatt cctcattggt     540
gatattcacc caacccctaa tatcagaaat ttccaaggca gaacagtagc tgatgcaga      599

<210>  101
<211>  199
<212>  PRT
<213>  Tradescantia virginiana

<400>  101
Gly Leu Val Lys Lys Ala His Glu Ile Ser Val Leu Cys Asp Ala Glu
1               5                   10                  15
Leu Ala Leu Ile Ile Phe Ser Pro Lys Gly Lys Leu Tyr Glu Tyr Ala
            20                  25                  30
Thr Asp Ser Lys Met Glu Ile Ile Leu Glu Arg Tyr Glu Arg Tyr Thr
        35                  40                  45
Tyr Ala Glu Lys Ala Leu Ile Ala Ser Asp Pro Asp Val Gln Gly Asn
    50                  55                  60
Trp Cys His Glu Tyr Ile Lys Leu Lys Ala Lys Phe Glu Ala Leu Asn
65                  70                  75                  80
Lys Ser Gln Arg His Leu Met Gly Glu Gln Leu Asp Thr Leu Asn Gln
                85                  90                  95
Lys Glu Leu Leu Gln Leu Glu Thr Lys Leu Glu Gly Ser Leu Lys Asn
            100                 105                 110
Val Arg Ser Arg Lys Thr Gln Leu Met Leu Asp Ser Ile Ser Glu Leu
        115                 120                 125
Gln Glu Lys Gly Lys Ser Leu Gln Glu Gln Asn Thr Cys Leu Glu Lys
    130                 135                 140
Glu Ile Leu Gly Lys Gln Lys Asp Lys Ala Pro Lys Gln His Val Gln
145                 150                 155                 160
Trp Glu Lys Gln Asn Gln Pro Pro Pro Thr Ser Ser Ala Pro Met Pro
                165                 170                 175
Phe Leu Ile Gly Asp Ile His Pro Thr Pro Asn Ile Arg Asn Phe Gln
            180                 185                 190
Gly Arg Thr Val Ala Asp Ala
        195

<210>  102
<211>  753
<212>  DNA

<213>  Elaeis guineensis

<400>  102
atggggagag ggagggtgca gctgaggcgg atcgagaaca agataaaccg gcaggtgacg      60
ttctcgaagc gccggtcggg gctcctgaag aaagcccacg agatctccgt cctctgcgac     120
gccgaggtcg ccctcatcat cttctcgacc aagggcaagc tctacgagta cgccaccgac     180
tcctgcatgg aaaggattct tgaacgctat gaacgttaca cctatgcaga aaaagcacta     240
atttcatctg gacccgaatt gcagggtaac tggtgccatg aatttggcaa actcaaagct     300
aaggttgagg ctttacaaaa aagccaaagg catctcatgg gtgagcaact tgagcccttg     360
aatctcaaag aactccagca actagagcaa cagcttgaaa gttctttaaa gcatataaga     420
accagaaagt gccaactcat gtttgaatcc atctctgagc ttcaaaaaaa ggaaaagtca     480
ctgcaggagc agaacaagat gctggagaag gagctcatgg agaagcagaa ggtgaaggca     540
ctaaaccagc aggcaccttg ggagcagcaa ggcccgccgc agacaagctc atcatcccca     600
acctccttcc tgatcggaga ctctctcccc accctgaata ttgggacata ccaatgtagc     660
ggaaatgaac atggggagga agcagcacaa ccccaggttc gtataggaaa cagcctgtta     720
ccaccttgga tgcttagcca cttgaacggg tag                                  753

<210>  103
<211>  250
<212>  PRT
<213>  Elaeis guineensis

<400>  103
Met Gly Arg Gly Arg Val Gln Leu Arg Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Ile Phe
        35                  40                  45
Ser Thr Lys Gly Lys Leu Tyr Glu Tyr Ala Thr Asp Ser Cys Met Glu
    50                  55                  60
Arg Ile Leu Glu Arg Tyr Glu Arg Tyr Thr Tyr Ala Glu Lys Ala Leu
65                  70                  75                  80
Ile Ser Ser Gly Pro Glu Leu Gln Gly Asn Trp Cys His Glu Phe Gly
                85                  90                  95
Lys Leu Lys Ala Lys Val Glu Ala Leu Gln Lys Ser Gln Arg His Leu
            100                 105                 110
Met Gly Glu Gln Leu Glu Pro Leu Asn Leu Lys Glu Leu Gln Gln Leu
        115                 120                 125
Glu Gln Gln Leu Glu Ser Ser Leu Lys His Ile Arg Thr Arg Lys Cys
    130                 135                 140
Gln Leu Met Phe Glu Ser Ile Ser Glu Leu Gln Lys Lys Glu Lys Ser
145                 150                 155                 160
Leu Gln Glu Gln Asn Lys Met Leu Glu Lys Glu Leu Met Glu Lys Gln
                165                 170                 175
Lys Val Lys Ala Leu Asn Gln Gln Ala Pro Trp Glu Gln Gln Gly Pro
            180                 185                 190
Pro Gln Thr Ser Ser Ser Ser Pro Thr Ser Phe Leu Ile Gly Asp Ser
        195                 200                 205
Leu Pro Thr Leu Asn Ile Gly Thr Tyr Gln Cys Ser Gly Asn Glu His
    210                 215                 220
Gly Glu Glu Ala Ala Gln Pro Gln Val Arg Ile Gly Asn Ser Leu Leu
225                 230                 235                 240
Pro Pro Trp Met Leu Ser His Leu Asn Gly
                245                 250

<210>  104
<211>  699
<212>  DNA

<213>  Allium sp.

<400>  104
gtgcaattga agaggatgga aaacaagatt aatagacaag tgaccttctc aaaaagaaga      60
aatggtttgt tgaagaaagc tcatgagatt tcwgtgcttt gtgatgcaga agttgcactt     120
attgttttct ctgctaaagg aaaactctat gaatattcaa ctgattcaag tatggaaaaa     180
attctggaga ggtatgaacg ttattgcttt gcggagaaat catcaacaat gagtgacatt     240
gactcccagg aggattggag ccttgaatat cacaaactga aggctaaggt tgagagttta     300
aacaacaggc aaaggcatct tatgggagag caacttgaat ctctgagtct tcgagaaatt     360
ggacagcttg agcaacaact tgagaattct ctcaaaactg ttcggacgcg caagagccaa     420
gaattgttaa gttctatttc agagcttcag gacaaggaga aaactttgcg agatgagaac     480
aaagctttag aaaatgagct tatgaaaagg gccagggcaa aagctattct ggaacaacaa     540
gcacgatgga agcatcataa tcataaacaa caggataatc ttcataatcc aaatatcaac     600
attggaaatt accaaacaag gaacaatgag ggaggagttg agccagcaac ggatgttcaa     660
gtacgtgttg ttagaaattt gttgccccac tggatgctt                            699

<210>  105
<211>  233
<212>  PRT
<213>  Allium sp.

<400>  105
Val Gln Leu Lys Arg Met Glu Asn Lys Ile Asn Arg Gln Val Thr Phe
1               5                   10                  15
Ser Lys Arg Arg Asn Gly Leu Leu Lys Lys Ala His Glu Ile Ser Val
            20                  25                  30
Leu Cys Asp Ala Glu Val Ala Leu Ile Val Phe Ser Ala Lys Gly Lys
        35                  40                  45
Leu Tyr Glu Tyr Ser Thr Asp Ser Ser Met Glu Lys Ile Leu Glu Arg
    50                  55                  60
Tyr Glu Arg Tyr Cys Phe Ala Glu Lys Ser Ser Thr Met Ser Asp Ile
65                  70                  75                  80
Asp Ser Gln Glu Asp Trp Ser Leu Glu Tyr His Lys Leu Lys Ala Lys
                85                  90                  95
Val Glu Ser Leu Asn Asn Arg Gln Arg His Leu Met Gly Glu Gln Leu
            100                 105                 110
Glu Ser Leu Ser Leu Arg Glu Ile Gly Gln Leu Glu Gln Gln Leu Glu
        115                 120                 125
Asn Ser Leu Lys Thr Val Arg Thr Arg Lys Ser Gln Glu Leu Leu Ser
    130                 135                 140
Ser Ile Ser Glu Leu Gln Asp Lys Glu Lys Thr Leu Arg Asp Glu Asn
145                 150                 155                 160
Lys Ala Leu Glu Asn Glu Leu Met Lys Arg Ala Arg Ala Lys Ala Ile
                165                 170                 175
Leu Glu Gln Gln Ala Arg Trp Lys His His Asn His Lys Gln Gln Asp
            180                 185                 190
Asn Leu His Asn Pro Asn Ile Asn Ile Gly Asn Tyr Gln Thr Arg Asn
        195                 200                 205
Asn Glu Gly Gly Val Glu Pro Ala Thr Asp Val Gln Val Arg Val Val
    210                 215                 220
Arg Asn Leu Leu Pro His Trp Met Leu
225                 230

<210>  106
<211>  744
<212>  DNA
<213>  Dendrobium grex Madame Thong-IN

<400>  106

atgggtcgtg gcagggtgca gctgaagcga atcgagaata aaataaaccg gcaggtgacg 60
ttctcgaagc ggagatctgg tttgcttaag aaggcgcacg agatctccgt gctctgtgac 120
gctgaagttg ctctgatcgt tttttccaat aagggaaagc tttatgagta ttccaccgat 180
tccagcatgg agaaaattct tgaacggtat gagcgttatt catatgctga aagagcatta 240
ttttccaatg aggccaaccc ccaggctgat tggcgccttg aatataataa actgaaggca 300
agggttgaaa gcttacagaa gagccaaagg caccttatgg gggagcaact tgactccttg 360
agcattaaag aactccaacg tctagagcaa cagcttgaaa gttccttgaa gtttatacga 420
tccagaaaga cacagctcat actacattca atttccgagc tacaaaagat ggaaaaaata 480
ttgctggagc aaaacaagac cttagagaag gagattatag ctaaagagaa agccaaagct 540
ttggtgcagc atgccccatg ggagaagcaa aaccagtccc aatatagctc tgcactcccg 600
cctgtgattt cggattctgt cccaactccc accagcagaa cgtttcaagc cagagccaat 660
gaagaagaat cacctcagcc acagttaaga gtaagcaaca ctctgctgcc cccatggatg 720
ctcagtcata tgaatggaca ataa 744

<210> 107
<211> 247
<212> PRT
<213> Dendrobium grex Madame Thong-IN

<400> 107
Met Gly Arg Gly Arg Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Val Phe
        35                  40                  45
Ser Asn Lys Gly Lys Leu Tyr Glu Tyr Ser Thr Asp Ser Ser Met Glu
    50                  55                  60
Lys Ile Leu Glu Arg Tyr Glu Arg Tyr Ser Tyr Ala Glu Arg Ala Leu
65                  70                  75                  80
Phe Ser Asn Glu Ala Asn Pro Gln Ala Asp Trp Arg Leu Glu Tyr Asn
                85                  90                  95
Lys Leu Lys Ala Arg Val Glu Ser Leu Gln Lys Ser Gln Arg His Leu
            100                 105                 110
Met Gly Glu Gln Leu Asp Ser Leu Ser Ile Lys Glu Leu Gln Arg Leu
        115                 120                 125
Glu Gln Gln Leu Glu Ser Ser Leu Lys Phe Ile Arg Ser Arg Lys Thr
    130                 135                 140
Gln Leu Ile Leu His Ser Ile Ser Glu Leu Gln Lys Met Glu Lys Ile
145                 150                 155                 160
Leu Leu Glu Gln Asn Lys Thr Leu Glu Lys Glu Ile Ile Ala Lys Glu
                165                 170                 175
Lys Ala Lys Ala Leu Val Gln His Ala Pro Trp Glu Lys Gln Asn Gln
            180                 185                 190
Ser Gln Tyr Ser Ser Ala Leu Pro Pro Val Ile Ser Asp Ser Val Pro
        195                 200                 205
Thr Pro Thr Ser Arg Thr Phe Gln Ala Arg Ala Asn Glu Glu Glu Ser
    210                 215                 220
Pro Gln Pro Gln Leu Arg Val Ser Asn Thr Leu Leu Pro Pro Trp Met
225                 230                 235                 240
Leu Ser His Met Asn Gly Gln
                245

<210> 108
<211> 2194
<212> DNA
<213> Oryza sativa

<400> 108

aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 109
<211> 804
<212> DNA
<213> Oryza sativa

<400> 109
atggggcggg ggaaggtgca gctgaagcgg atagagaaca agatcaacag gcaggtgacg 60
ttctccaaga ggaggaatgg attgctgaag aaggcgcacg agatctccgt cctctgcgac 120
gccgaggtcg ccgccatcgt cttctccccc aagggcaagc tctacgagta cgccactgac 180
tccaggatgg acaaaatcct tgaacgttat gagcgctatt catatgctga aaaggctctt 240
atttcagctg aatccgagag tgagggaaat tggtgccatg aatacaggaa acttaaggca 300
aagattgaga ccatacaaaa atgtcacaaa cacctcatgg gagaggatct agaatccctg 360
aatctcaaag aactccaaca gctagagcag cagctggaga gttcattgaa gcacataata 420
tcaagaaaga gccaccttat gcttgagtcc atttccgagc tgcagaaaaa ggagaggtca 480
ctgcaggagg agaacaaggc tctgcagaag gaactggtgg agaggcagaa gaatgtgagg 540
ggccagcagc aagtagggca gtgggaccaa acccaggtcc aggcccaggc ccaagcccaa 600
ccccaagccc agacaagctc ctcctcctcc tccatgctga gggatcagca ggcacttctt 660
ccaccacaaa atatctgcta cccgccggtg atgatgggcg agagaaatga tgcggcggcg 720
gcggcggcgg tggcggcgca gggccaggtg caactccgca tcggaggtct tccgccatgg 780
atgctgagcc acctcaatgc ttaa 804

<210> 110
<211> 267
<212> PRT
<213> Oryza sativa

<400> 110
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Ile Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Ser Ser
        195                 200                 205
Ser Ser Ser Met Leu Arg Asp Gln Gln Ala Leu Leu Pro Pro Gln Asn
    210                 215                 220
Ile Cys Tyr Pro Pro Val Met Met Gly Glu Arg Asn Asp Ala Ala Ala
225                 230                 235                 240
Ala Ala Ala Val Ala Ala Gln Gly Gln Val Gln Leu Arg Ile Gly Gly
                245                 250                 255
Leu Pro Pro Trp Met Leu Ser His Leu Asn Ala
            260                 265

<210> 111
<211> 52
<212> DNA
<213> Artificial sequence

<220>
<223> primer

<400> 111
ggggacaagt ttgtacaaaa aagcaggctt aaacaatggg gcgggggaag gt 52

<210> 112
<211> 47
<212> DNA
<213> Artificial sequence

<220>
<223> primer

<400> 112
ggggaccact ttgtacaaga aagctgggtt tggccgacga cgacgac 47

<210> 113
<211> 2193
<212> DNA
<213> Oryza sativa

<400> 113
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga attttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 114
<211> 42
<212> PRT
<213> Artificial sequence

<220>
<223> artificial sequence

<220>
<221> VARIANT
<222> (2)..(2)

<223> /replace = "Val"

<220>
<221> VARIANT
<222> (6)..(6)
<223> /replace = "Asn"

<220>
<221> VARIANT
<222> (10)..(10)
<223> /replace = "Ile"

<220>
<221> VARIANT
<222> (12)..(12)
<223> /replace = "Tyr"

<220>
<221> VARIANT
<222> (16)..(16)
<223> /replace = "Ile" /replace = "Leu"

<220>
<221> VARIANT
<222> (17)..(17)
<223> /replace = "Gly"

<220>
<221> VARIANT
<222> (18)..(18)
<223> /replace = "Ala" /replace = "Val"

<220>
<221> VARIANT
<222> (19)..(19)
<223> /replace = "Val"

<220>
<221> VARIANT
<222> (20)..(20)
<223> /replace = "Val"

<220>
<221> VARIANT
<222> (23)..(23)
<223> /replace = "Pro" /replace = "Ala" /replace = "Asn"

<220>
<221> VARIANT
<222> (30)..(30)
<223> /replace = "Phe"

<220>
<221> VARIANT
<222> (31)..(31)
<223> /replace = "Ser"

<220>
<221> VARIANT

<222>  (32)..(32)
<223>  /replace = "Ser"

<220>
<221>  VARIANT
<222>  (33)..(33)
<223>  /replace = "Asn" /replace = "Glu"

<220>
<221>  VARIANT
<222>  (35)..(35)
<223>  /replace = "Cys /replace = "Arg" /replace = "Ser"

<220>
<221>  VARIANT
<222>  (37)..(37)
<223>  /replace = "Glu"

<220>
<221>  VARIANT
<222>  (38)..(38)
<223>  /replace = "Ile" /replace = "Arg" /replace = "Lys"

<220>
<221>  VARIANT
<222>  (41)..(41)
<223>  /replace = "Asp"

<400>  114
Leu Leu Lys Lys Ala His Glu Ile Ser Val Leu Cys Asp Ala Glu Val
1               5                   10                  15
Ala Leu Ile Ile Phe Ser Thr Lys Gly Lys Leu Tyr Glu Tyr Ala Thr
            20                  25                  30
Asp Ser Lys Met Asp Asn Ile Leu Glu Arg
        35                  40

<210>  115
<211>  19
<212>  PRT
<213>  Artificial sequence

<220>
<223>  artificial sequence

<220>
<221>  VARIANT
<222>  (4)..(4)
<223>  /replace = "Ser"

<220>
<221>  VARIANT
<222>  (5)..(5)
<223>  /replace = "Arg"

<220>
<221>  VARIANT
<222>  (6)..(6)
<223>  /replace = "Ile"

<220>
<221>  VARIANT
<222>  (8)..(8)
<223>  /replace = "Thr" /replace = "Ser"

<220>
<221>  VARIANT
<222>  (9)..(9)
<223>  /replace = "Ile"

<220>
<221>  VARIANT
<222>  (10)..(10)
<223>  /replace = "Asn"

<220>
<221>  VARIANT
<222>  (11)..(11)
<223>  /replace = "Arg" /replace = "Asn"

<220>
<221>  VARIANT
<222>  (12)..(12)
<223>  /replace = "Cys" /replace = "Arg"

<220>
<221>  VARIANT
<222>  (13)..(13)
<223>  /replace = "His"

<220>
<221>  VARIANT
<222>  (14)..(14)
<223>  /replace = "Lys"

<400>  115
Lys Leu Lys Ala Lys Val Glu Ala Leu Gln Lys Ser Gln Arg His Leu
1               5                   10                  15
Met Gly Glu


<210>  116
<211>  11
<212>  PRT
<213>  Artificial sequence

<220>
<223>  artificial sequence

<220>
<221>  VARIANT
<222>  (2)..(2)
<223>  /replace = "Gln" /replace = "Val" /replace = "Ala"

<220>
<221>  VARIANT
<222>  (6)..(6)
<223>  /replace = "Phe"

<220>
<221> VARIANT
<222> (7)..(7)
<223> /replace = "Phe"

<220>
<221> VARIANT
<222> (8)..(8)
<223> /replace = "Phe"

<220>
<221> VARIANT
<222> (9)..(9)
<223> /replace = "Phe"

<220>
<221> VARIANT
<222> (10)..(10)
<223> /replace = "Cys" /replace = "Phe"

<220>
<221> VARIANT
<222> (11)..(11)
<223> /replace = "Met"

<400> 116
Gln Pro Gln Thr Ser Ser Ser Ser Ser Ser Phe
1               5                   10

<210> 117
<211> 7
<212> PRT
<213> Artificial sequence

<220>
<223> artificial sequence

<220>
<221> VARIANT
<222> (1)..(1)
<223> /replace = "Ala" /replace = "Val" /replace = "Leu"

<220>
<221> VARIANT
<222> (2)..(2)
<223> /replace = "Pro"

<220>
<221> UNSURE
<222> (3)..(3)
<223> Xaa can be any amino acid, preferably Leu, Phe or His

<220>
<221> UNSURE
<222> (6)..(6)
<223> Xaa can be any naturally occuring amino acid, preferably Val or
      Leu

<220>

<221>  VARIANT
<222>  (7)..(7)
<223>  /replace = "His"

<400>  117
Gly Leu Xaa Trp Met Xaa Ser
1               5

<210>  118
<211>  735
<212>  DNA
<213>  Hordeum vulgare

<400>  118
atggggcgcg ggaaggtgca gctgaagcgg atcgagaaca agatcaaccg ccaggtcacc      60
ttctccaagc gccgctcggg gctgctcaag aaggcgcacg agatctccgt gctctgcgac     120
gccgaggtcg gcctcatcat cttctccacc aagggaaagc tctacgagtt ctccaccgag     180
tcatgtatgg acaaaattct tgaacggtat gagcgctact cttatgcaga aaaggttctc     240
gtttcaagtg aatctgaaat tcagggaaac tggtgtcacg aatataggaa actgaaggcg     300
aaggttgaga caatacagaa atgtcaaaag catctcatgg gagaggatct tgaatctttg     360
aatctcaagg agttgcagca actggagcag cagctggaaa gctcactgaa acatatcaga     420
gccaggaaga accaacttat gcacgaatcc atttctgagc ttcagaagaa ggagaggtca     480
ctgcaggagg agaataaagt tctccagaag gaacttgtgg agaagcagaa ggcccaggcg     540
gcgcagcaag atcaaactca gcctcaaacc agctcttctt cttcttcctt catgatgagg     600
gatgctcccc ctgtcgcaga taccagcaat cacccagcgg cggcaggcga gagggcagag     660
gatgtggcag tgcagcctca ggtcccactc cggacggcgc ttccactgtg gatggtgagc     720
cacatcaacg gctga                                                      735

<210>  119
<211>  244
<212>  PRT
<213>  Hordeum vulgare

<400>  119
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Val Ala Asp Thr

195                 200                 205
Ser Asn His Pro Ala Ala Ala Gly Glu Arg Ala Glu Asp Val Ala Val
    210                 215                 220
Gln Pro Gln Val Pro Leu Arg Thr Ala Leu Pro Leu Trp Met Val Ser
225                 230                 235                 240
His Ile Asn Gly

<210>  120
<211>  1196
<212>  DNA
<213>  Hordeum vulgare

<400>  120
ctctcccctc ccacttcacc caaccacctg acagccatgg ctccgccacc tcgcctccgc      60
ccgcgcctct gagagtagcc gtcgcggtcg ctcgctcgct cgctcgctgc tgccggtgtt     120
ggcccggtcc tcgagcggag atggggcgca ggaaggtgca gctgaagcgg atcgagaaca     180
agatcaaccg ccaggtcacc ttctccaagc gccgctcggg gctgctcaag aaggcgcacg     240
agatctccgt gctctacgac gccgaggtcg gcctcatcat cttctccacc aagggaaagc     300
tctacgagtt ctccaccgag tcatgtatgg acaaaattct tgaacggtat gagcgctact     360
cttatgcaga aaaggttctc gtttcaagtg aatctgaaat tcagggaaac tggtgtcacg     420
aatataggaa actgaaggcg aaggttgaga caatacagaa atgtcaaaag catctcatgg     480
gagaggatct tgaatctttg aatctcaagg agttgcagca actggagcag cagctggaaa     540
gctcactgaa acatatcaga gccaggaaga accaacttat gcacgaatcc atttctgagc     600
ttcagaagaa ggagaggtca ctgcaggagg agaataaagt tctccagaag gaacttgtgg     660
agaagcagaa ggcccaggcg gcgcagcaag atcaaactca gcctcaaacc agctcttctt     720
cttcttcctt catgatgagg gatgctcccc ctgtcgcaga taccagcaat cacccagcgg     780
cggcaggcga gagggcagag gatgtggcag tgcagcctca ggtcccactc cggacggcgc     840
ttccactgtg gatggtgagc cacatcaacg gctgaagggc ttccagccca tgtaagcgta     900
ctattcagta cgagtaacaa gttgcagcgg ccagcctggt gtatcatgcg gttgcgaaca     960
tgctaacccc atggagggga gaggaaaaga aatcagagta aagcagcaag ctgcaggaat    1020
gtgtatattt cacttcgtcc acctcagttt cctttccacc tgggctgaga tggctgtacg    1080
agtaatctac catgtaattt atatgtagca tgagtgacga attttcaact ttcgatgata    1140
tccgttgctc ctgggtgttg tttctgtgaa ttaacctatc gaatatgagc gttgtg        1196

<210>  121
<211>  244
<212>  PRT
<213>  Hordeum vulgare

<400>  121
Met Gly Arg Arg Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Tyr Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140

Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Val Ala Asp Thr
        195                 200                 205
Ser Asn His Pro Ala Ala Ala Gly Glu Arg Ala Glu Asp Val Ala Val
    210                 215                 220
Gln Pro Gln Val Pro Leu Arg Thr Ala Leu Pro Leu Trp Met Val Ser
225                 230                 235                 240
His Ile Asn Gly

<210>  122
<211>  1161
<212>  DNA
<213>  Triticum aestivum

<400>  122
ggcacgagct ctctctctct ctctctctct ctctctctct ctctctcctc gtgccgaatt      60
cggcacgagc ggagatgggg cgcgggaagg tgcagctgaa gcggatcgag aacaagatca     120
accggcaggt gaccttctcc aagcgccgct cggggctgct caagaaggcg cacgagatct     180
ccgtgctctg cgacgccgag gtcggcctca tcatcttctc caccaaggga aagctctacg     240
agttctccac cgagtcatgt atggacaaaa ttcttgaacg gtatgagcgc tactcttatg     300
cagaaaaggt tctcgtttca agtgaatctg aaattcaggg aaactggtgt cacgaatata     360
ggaaactgaa ggcgaaggtt gagacaatac agaaatgtca aaagcatctg atgggagagg     420
atcttgaatc tttgaatctc aaggagttgc agcaactgga gcagcagctg gaaagctcac     480
tgaaacatat cagatccagg aagaaccaac ttatgcacga atccatttct gagcttcaga     540
agaaggagag gtcactgcag gaggagaata aagttctcca gaaggaactc gtcgagaagc     600
agaaggccca ggcggcgcaa caagatcaga ctcagcctca aacaagctct tcttcttctt     660
ccttcatgat gagggatgct ccccctgccg cagctaccag cattcatcca gcggcggcag     720
gcgagagggc aggggatgcg gcagtgcagc cgcaggcccc accccggacg gggcttccac     780
tgtggatggt gagccacatc aacggctgaa gggcttccag cccatataag cgtactattc     840
agtagagagt aacaagttgc accggccagt ctggtgtatg ttgcggttgc tagcacgcct     900
gaccccttgg aggggaaagg aaaagaaatc agagtaaagt agcaagctgc agcgatgtgt     960
atatttcact ttgtccaccc cagtttccct cccagctggg ctcaatttac catgtaatct    1020
atatgtagct tgagtgatga attttcaagt ttccatgata cccgtctcta gtgggatgtt    1080
gtttatgtga attaacctat caaatatgag cattgtgtat attgtgattc ttgaaaataa    1140
ataaatcagg atctttgtct t                                              1161

<210>  123
<211>  244
<212>  PRT
<213>  Triticum aestivum

<400>  123
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg

                    85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Ala Ala Ala Thr
        195                 200                 205
Ser Ile His Pro Ala Ala Ala Gly Glu Arg Ala Gly Asp Ala Ala Val
    210                 215                 220
Gln Pro Gln Ala Pro Pro Arg Thr Gly Leu Pro Leu Trp Met Val Ser
225                 230                 235                 240
His Ile Asn Gly

<210>  124
<211>  1210
<212>  DNA
<213>  Triticum aestivum

<400>  124
tccctctcct ccctctcttc cgcctcaccc aaccacctga cagccatggc tccgcccccc      60
cgcccccgcc tgcgcctgtc ggagtagccg tcgcggtctg ccggtgttgg aggcttgggg     120
tgtagggttg gccccgttct ccagcggaga tggggcgcgg gaaggtgcag ctgaagcgga     180
tcgagaacaa gatcaaccgg caggtgacct tctccaagcg ccgctcgggg ctgctcaaga     240
aggcgcacga gatctccgtg ctctgcgacg ccgaggtcgg cctcatcatc ttctccacca     300
agggaaagct ctacgagttc tccaccgagt catgtatgga caaaattctt gaacggtatg     360
agcgctactc ttatgcagaa aaggttctcg tttcaagtga atctgaaatt cagggaaact     420
ggtgtcacga atataggaaa ctgaaggcga aggttgagac aatacagaaa tgtcaaaagc     480
atctgatggg agaggatctt gaatctttga atctcaagga gttgcagcaa ctggagcagc     540
agctggaaag ctcactgaaa catatcagat ccaggaagaa ccaacttatg cacgaatcca     600
tttctgagct tcagaagaag gagaggtcac tgcaggagga gaataaagtt ctccagaagg     660
aactcgtcga gaagcagaag gcccaggcgg cgcaacaaga tcagactcag cctcaaacaa     720
gctcttcttc ttcttccttc atgatgaggg atgctccccc tgccgcaact accagcattc     780
atccagcggc atcaggagag agggcagagg atgcggcagt gcagccgcag gccccacccc     840
ggacggggct tccactgtgg atggttagcc acatcaacgg ctgaagggct tccagcccat     900
ataagcgtac tattcagtag agagtaacaa gttgcaccgg ccagcctggt gtatgttgcg     960
gttgctagca tgcctgaccc cttggagggg aaaggaaaag aaatcagagt aaagtagcaa    1020
gctgcagtga tgtgtatatt tcactttgtc cacctcagtt tccctcccag ctgggctcaa    1080
tttaccatgt aatctatatg tagcttgagt gatgaatttt caagtttcca tgatacccgt    1140
ctcgagcggg tgttgtttat gtgaattaac ctatcaaata tgagcattgt gtaaaaaaaa    1200
aaaaaaaaaa                                                           1210

<210>  125
<211>  244
<212>  PRT
<213>  Triticum aestivum

<400>  125
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
            35                  40                  45
Ser Thr Lys Gly Lys Leu Tyr Glu Phe Ser Thr Glu Ser Cys Met Asp
        50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala Gln Ala Ala Gln Gln Asp Gln Thr Gln Pro Gln Thr Ser Ser
            180                 185                 190
Ser Ser Ser Ser Phe Met Met Arg Asp Ala Pro Pro Ala Ala Thr Thr
        195                 200                 205
Ser Ile His Pro Ala Ala Ser Gly Glu Arg Ala Glu Asp Ala Ala Val
    210                 215                 220
Gln Pro Gln Ala Pro Pro Arg Thr Gly Leu Pro Leu Trp Met Val Ser
225                 230                 235                 240
His Ile Asn Gly

<210>  126
<211>  735
<212>  DNA
<213>  Triticum monococcum

<400>  126
atggggcgcg ggaaggtgca gctgaagcgg atcgagaaca agatcaaccg gcaggtgacc      60
ttctccaagc gccgctcggg gcttctcaag aaggcgcacg agatctccgt gctctgcgac     120
gccgaggtcg gcctcatcat cttctccacc aagggaaagc tctacgagtt ctccaccgag     180
tcatgtatgg acaaaattct tgaacggtat gagcgctatt cttatgcaga aaaggttctc     240
gtttcaagtg aatctgaaat tcagggaaac tggtgtcacg aatataggaa actgaaggcg     300
aaggttgaga caatacagaa atgtcaaaaa catctcatgg gagaggatct tgaatctttg     360
aatctcaagg agttgcagca actggagcag cagctggaaa gctcactgaa acatatcaga     420
tccaggaaga accaacttat gcacgaatcc atttctgagc tgcagaagaa ggagaggtca     480
ctgcaggagg agaataaagt tctccagaag gaactcgtgg agaagcagaa ggcccatgcg     540
gcgcagcaag atcaaactca gcctcaaacc agctcttctt cttcttcctt catgctgagg     600
gatgctcccc ctgccgcaaa taccagcatt catccagcgg cggcaggcga gagggcagag     660
gatgcggcag tgcagccgca ggccccaccc cggacggggc ttccaccgtg gatggtgagc     720
cacatcaacg ggtga                                                      735

<210>  127
<211>  244
<212>  PRT
<213>  Triticum monococcum

<400>  127
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala His Ala Ala Gln Gln Asp Gln Thr Gln Pro Gln Thr Ser Ser
            180                 185                 190
Ser Ser Ser Ser Phe Met Leu Arg Asp Ala Pro Pro Ala Ala Asn Thr
        195                 200                 205
Ser Ile His Pro Ala Ala Ala Gly Glu Arg Ala Glu Asp Ala Ala Val
    210                 215                 220
Gln Pro Gln Ala Pro Pro Arg Thr Gly Leu Pro Pro Trp Met Val Ser
225                 230                 235                 240
His Ile Asn Gly


<210>  128
<211>  735
<212>  DNA
<213>  Triticum aestivum

<400>  128
atggggcggg ggaaggtgca gctgaagcgg atcgagaaca agatcaaccg gcaggtgacc      60
ttctccaagc gccgctcggg gcttctcaag aaggcgcacg agatctccgt gctctgcgac     120
gccgaggtcg gcctcatcat cttctccacc aagggaaagc tctacgagtt ctccaccgag     180
tcatgtatgg acaaaattct tgaacggtat gagcgctatt cttatgcaga aaaggttctc     240
gtttcaagtg aatctgaaat tcagggaaac tggtgtcacg aatataggaa actgaaggcg     300
aaggttgaga caatacagaa atgtcaaaag catctcatgg gagaggatct tgaatctttg     360
aatctcaagg agttgcagca actggagcag cagctggaaa gctcactgaa acatatcaga     420
tccaggaaga accaacttat gcacgaatcc atttctgagc ttcagaagaa ggagaggtca     480
ctgcaggagg agaataaagt tctccagaag gaactcgtgg agaagcagaa ggcccatgcg     540
gcgcagcaag atcaaactca gcctcaaacc agctcttcat cttcttcctt catgctgagg     600
gatgctcccc ctgccgcaaa taccagcatt catccagcgg caacaggcga gagggcagag     660
gatgcggcag tgcagccgca ggccccaccc cggacggggc ttccaccgtg gatggtgagc     720
cacatcaacg ggtga                                                      735

<210>  129
<211>  244
<212>  PRT
<213>  Triticum aestivum

<400>  129
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
        35                  40                  45
Ser Thr Lys Gly Lys Leu Tyr Glu Phe Ser Thr Glu Ser Cys Met Asp
    50                  55                  60
Lys Ile Leu Glu Arg Tyr Glu Arg Tyr Ser Tyr Ala Glu Lys Val Leu
65                  70                  75                  80
Val Ser Ser Glu Ser Glu Ile Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Val Leu Gln Lys Glu Leu Val Glu Lys Gln
                165                 170                 175
Lys Ala His Ala Ala Gln Gln Asp Gln Thr Gln Pro Gln Thr Ser Ser
            180                 185                 190
Ser Ser Ser Ser Phe Met Leu Arg Asp Ala Pro Pro Ala Ala Asn Thr
        195                 200                 205
Ser Ile His Pro Ala Ala Thr Gly Glu Arg Ala Glu Asp Ala Ala Val
    210                 215                 220
Gln Pro Gln Ala Pro Pro Arg Thr Gly Leu Pro Pro Trp Met Val Ser
225                 230                 235                 240
His Ile Asn Gly

<210>  130
<211>  738
<212>  DNA
<213>  Lolium perenne

<400>  130
atggggcgcg gcaaggtgca gctcaagcgg atcgagaaca agatcaaccg ccaggtcacc      60
ttctccaagc gccgctcggg gctgctcaag aaggcgcacg agatctccgt gctctgcgac     120
gccgaggtcg ggctcatcat cttctccacc aagggaaagc tctacgagtt cgcaaccgac     180
tcatgtatgg acaaaattct tgagcggtat gagcgctact cctatgcaga gaaagtgctc     240
atttcaaccg aatctgaaat tcagggaaac tggtgtcatg aatataggaa actgaaggcg     300
aaggttgaga caatacagag atgtcaaaag catctaatgg gagaggatct tgaatcattg     360
aatctcaagg agttgcagca actagagcag cagctggaaa gttcactgaa acatattaga     420
gccagaaaga accagcttat gcacgaatcc atatctgagc ttcaaaagaa ggagaggtca     480
ctgcaggagg agaataaaat tctccagaag gaactcatag agaagcagaa ggcccacacg     540
cagcaagcgc agtgggagca aactcagccc caaaccagct cttcctcctc ctcctttatg     600
atgggggaag ctaccccagc aacaaattgc agtaatcccc cagcagcggc cagcgacaga     660
gcagaggatg cgacggggca gccttcagct cgcacggtgc ttccaccatg gatggtgagt     720
cacatcaaca atggctga                                                   738

<210>  131
<211>  245
<212>  PRT
<213>  Lolium perenne

<400>  131
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
            35                  40                  45
Ser Thr Lys Gly Lys Leu Tyr Glu Phe Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Thr Glu Ser Glu Ile Gln Gly Asn Trp Cys His Glu Tyr Arg
                85                  90                  95
Lys Leu Lys Ala Lys Val Glu Thr Ile Gln Arg Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ala Arg Lys Asn
    130                 135                 140
Gln Leu Met His Glu Ser Ile Ser Glu Leu Gln Lys Lys Glu Arg Ser
145                 150                 155                 160
Leu Gln Glu Glu Asn Lys Ile Leu Gln Lys Glu Leu Ile Glu Lys Gln
                165                 170                 175
Lys Ala His Thr Gln Gln Ala Gln Trp Glu Gln Thr Gln Pro Gln Thr
            180                 185                 190
Ser Ser Ser Ser Ser Ser Phe Met Met Gly Glu Ala Thr Pro Ala Thr
        195                 200                 205
Asn Cys Ser Asn Pro Pro Ala Ala Ala Ser Asp Arg Ala Glu Asp Ala
    210                 215                 220
Thr Gly Gln Pro Ser Ala Arg Thr Val Leu Pro Pro Trp Met Val Ser
225                 230                 235                 240
His Ile Asn Asn Gly
                245

<210>  132
<211>  738
<212>  DNA
<213>  Lolium temulentum

<400>  132
atggggcgcg gcaaggtgca gctcaagcgg atcgagaaca agatcaaccg ccaggtcacc      60
ttctccaagc gccgctcagg cctgctcaag aaggcgcacg agatctccgt gctctgcgac     120
gcagaggtcg ggctcatcat cttctccacc aagggaaagc tctacgagtt cgccaccgac     180
tcatgtatgg acaaaattct tgagcggtat gagcgctact cctatgcaga gaaagtgctc     240
atttcaactg aatctgaaat tcagggaaac tggtgtcatg aatataggaa actgaaggcg     300
aaggttgaga caatacagag atgtcaaaag catctaatgg gagaggatct tgaatcattg     360
aatctcaagg agttgcagca actagagcag cagctggaaa gttcactgaa acatattaga     420
tccagaaaga gccagcttat gcacgaatcc atatctgagc ttcaaaagaa ggagaggtca     480
ctgcaagagg agaataaaat tctccagaag gaactcatag agaagcagaa ggcccacacg     540
cagcaagcgc agttggagca aactcagccc caaaccagct cttcctcctc ctcctttatg     600
atgggggaag ctaccccagc aacaaatcgc agtaatcccc cagcagcggc cagcgacaga     660
gcagaggatg cgacggggca gcctccagct cgcacggtgc ttccaccatg gatggtgagt     720
cacctcaaca atggctga                                                   738

<210>  133
<211>  245
<212>  PRT
<213>  Lolium temulentum

<400>  133
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
            35                  40                  45
Ser Thr Lys Gly Lys Leu Tyr Glu Phe Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Thr Glu Ser Glu Ile Gln Gly Asn Trp Cys His Glu Tyr Arg
                85                  90                  95
Lys Leu Lys Ala Lys Val Glu Thr Ile Gln Arg Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
Gln Leu Met His Glu Ser Ile Ser Glu Leu Gln Lys Lys Glu Arg Ser
145                 150                 155                 160
Leu Gln Glu Glu Asn Lys Ile Leu Gln Lys Glu Leu Ile Glu Lys Gln
                165                 170                 175
Lys Ala His Thr Gln Gln Ala Gln Leu Glu Gln Thr Gln Pro Gln Thr
            180                 185                 190
Ser Ser Ser Ser Ser Ser Phe Met Met Gly Glu Ala Thr Pro Ala Thr
        195                 200                 205
Asn Arg Ser Asn Pro Pro Ala Ala Ala Ser Asp Arg Ala Glu Asp Ala
    210                 215                 220
Thr Gly Gln Pro Pro Ala Arg Thr Val Leu Pro Pro Trp Met Val Ser
225                 230                 235                 240
His Leu Asn Asn Gly
                245

<210>  134
<211>  738
<212>  DNA
<213>  Zea mays

<400>  134
atggggcgcg ggaaggtgca gctgaagcgg atcgagaaca agatcaaccg ccaggtgacc      60
ttctccaagc gccgctcggg gctgctcaag aaggcgcacg agatctccgt gctctgcgac     120
gccgaggtcg cgctcatcat cttctccacc aaagggaagc tctacgagta ttccaccgat     180
tcatgtatgg acaaaattct tgaccggtac gagcgctact cctatgcaga aaaggttctt     240
atttcagcag aatctgaaac tcagggcaat tggtgccacg agtatagaaa actaaaggcg     300
aaggtcgaga caatacaaaa atgtcaaaag cacctcatgg gagaggatct tgaaacgttg     360
aatctcaaag agcttcagca actagagcag cagctggaga gttcactgaa acatatcaga     420
accaggaaga accaacttat gctcgagtca atttcggagc tccaacggaa ggagaagtcg     480
ctgcaggagg agaacaaggt tctgcagaag gagctcgcgg agaagcagaa agcccagcgg     540
aagcaagtgc aatgggccaa aacccaacag cagaccagtt cgtcttcctc gtgcttcgtg     600
ataagggaag ctgccccaac aacaaatatc agcatttttc ctgtggcagc aggcgggagg     660
ttggtggaag gtgcagcagc gcagccacag gctcgcgttg gactaccacc atggatgctt     720
agccacctga gcagctga                                                   738

<210>  135
<211>  245
<212>  PRT
<213>  Zea mays

<400>  135
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

20                  25                  30
His Glu Ile Ser Val Leu Cys Asp Ala Glu Val Ala Leu Ile Ile Phe
        35                  40                  45
Ser Thr Lys Gly Lys Leu Tyr Glu Tyr Ser Thr Asp Ser Cys Met Asp
    50                  55                  60
Lys Ile Leu Asp Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Thr Leu Asn Leu Lys Glu Leu Gln Gln Leu
        115                 120                 125
Glu Gln Gln Leu Glu Ser Ser Leu Lys His Ile Arg Thr Arg Lys Asn
    130                 135                 140
Gln Leu Met Leu Glu Ser Ile Ser Glu Leu Gln Arg Lys Glu Lys Ser
145                 150                 155                 160
Leu Gln Glu Glu Asn Lys Val Leu Gln Lys Glu Leu Ala Glu Lys Gln
                165                 170                 175
Lys Ala Gln Arg Lys Gln Val Gln Trp Gly Gln Thr Gln Gln Gln Thr
            180                 185                 190
Ser Ser Ser Ser Ser Cys Phe Val Ile Arg Glu Ala Ala Pro Thr Thr
        195                 200                 205
Asn Ile Ser Ile Phe Pro Val Ala Ala Gly Gly Arg Leu Val Glu Gly
    210                 215                 220
Ala Ala Ala Gln Pro Gln Ala Arg Val Gly Leu Pro Pro Trp Met Leu
225                 230                 235                 240
Ser His Leu Ser Ser
                245

<210>  136
<211>  738
<212>  DNA
<213>  Zea mays

<400>  136
atggggcgcg ggaaggtgca gctgaagcgg atcgagaaca agatcaaccg ccaggtgaca      60
ttctccaagc gccgctcggg gctactcaag aaggcgcacg agatctccgt gctctgcgac     120
gccgaggtcg cgctcatcat cttctccacc aagggcaagc tctacgagta ctctaccgat     180
tcatgtatgg acaaaattct tgaacggtat gagcgctact cctatgcaga aaaggttctc     240
atttccgcag aatatgaaac tcagggcaat tggtgccatg aatatagaaa actaaaggcg     300
aaggtcgaga caatacagaa atgtcaaaag cacctcatgg gagaggatct tgaaactttg     360
aatctcaaag agcttcagca actagagcag cagctggaga gttcactgaa acatatcaga     420
acaaggaaga gccagcttat ggtcgagtca atttcagcgc tccaacggaa ggagaagtca     480
ctgcaggagg agaacaaggt tctgcagaag gagctcgcgg agaagcagaa agaccagcgg     540
cagcaagtgc aacgggacca aactcaacag cagaccagtt cgtcttccac gtccttcatg     600
ttaagggaag ctgccccaac aacaaatgtc agcatcttcc ctgtggcagc aggcgggagg     660
gtggtggaag gggcagcagc gcagccgcag gctcgcgttg gactgccacc atggatgctt     720
agccatctga gctgctga                                                   738

<210>  137
<211>  245
<212>  PRT
<213>  Zea mays

<400>  137
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Ala Leu Ile Ile Phe
            35                  40                  45
Ser Thr Lys Gly Lys Leu Tyr Glu Tyr Ser Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Tyr Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Thr Leu Asn Leu Lys Glu Leu Gln Gln Leu
        115                 120                 125
Glu Gln Gln Leu Glu Ser Ser Leu Lys His Ile Arg Thr Arg Lys Ser
    130                 135                 140
Gln Leu Met Val Glu Ser Ile Ser Ala Leu Gln Arg Lys Glu Lys Ser
145                 150                 155                 160
Leu Gln Glu Glu Asn Lys Val Leu Gln Lys Glu Leu Ala Glu Lys Gln
                165                 170                 175
Lys Asp Gln Arg Gln Gln Val Gln Arg Asp Gln Thr Gln Gln Gln Thr
            180                 185                 190
Ser Ser Ser Ser Thr Ser Phe Met Leu Arg Glu Ala Ala Pro Thr Thr
        195                 200                 205
Asn Val Ser Ile Phe Pro Val Ala Ala Gly Gly Arg Val Val Glu Gly
    210                 215                 220
Ala Ala Ala Gln Pro Gln Ala Arg Val Gly Leu Pro Pro Trp Met Leu
225                 230                 235                 240
Ser His Leu Ser Cys
                245

<210>  138
<211>  762
<212>  DNA
<213>  Oryza sativa

<400>  138
atggggcggg gcaaggtgca gctgaagcgg atcgagaaca agatcaaccg gcaggtgacc      60
ttctccaagc gcaggtcggg gctgctcaag aaggcgaatg agatctccgt gctctgcgac     120
gccgaggtcg cgctcatcat cttctccacc aagggcaagc tctacgagta cgccaccgac     180
tcatgtatgg acaaaatcct tgaacgttat gagcgctact cctatgcaga aaaggtcctt     240
atttcagctg aatctgacac tcagggcaac tggtgccacg aatataggaa actgaaggct     300
aaggttgaga caatacagaa atgtcaaaag cacctcatgg gagaggatct tgaatctttg     360
aatctcaaag agctgcagca gctggagcag cagctggaaa attcgttgaa acatatcaga     420
tccagaaaga gccaactaat gctcgagtcc attaacgagc ttcaacggaa ggaaaagtca     480
ctgcaggagg agaataaggt cctacagaaa gaaaaccctt gctccttcct acagctggtg     540
gagaagcaga aagtccagaa gcaacaagtg caatgggacc agacacaacc tcaaacaagt     600
tcctcatcat cctccttcat gatgagggaa gcccttccaa caactaatat cagtaactac     660
cctgcagcag ctggcgaaag gatagaggat gtagcagcag ggcagccaca gcatgttcgc     720
attgggctgc caccatggat gctgagccac atcaacggct aa                        762

<210>  139
<211>  253
<212>  PRT
<213>  Oryza sativa

<400>  139
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

20                  25                  30
Asn Glu Ile Ser Val Leu Cys Asp Ala Glu Val Ala Leu Ile Ile Phe
        35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Asp Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Asn Ser Leu Lys His Ile Arg Ser Arg Lys Ser
    130                 135                 140
Gln Leu Met Leu Glu Ser Ile Asn Glu Leu Gln Arg Lys Glu Lys Ser
145                 150                 155                 160
Leu Gln Glu Glu Asn Lys Val Leu Gln Lys Glu Asn Pro Cys Ser Phe
                165                 170                 175
Leu Gln Leu Val Glu Lys Gln Lys Val Gln Lys Gln Gln Val Gln Trp
            180                 185                 190
Asp Gln Thr Gln Pro Gln Thr Ser Ser Ser Ser Ser Ser Phe Met Met
        195                 200                 205
Arg Glu Ala Leu Pro Thr Thr Asn Ile Ser Asn Tyr Pro Ala Ala Ala
    210                 215                 220
Gly Glu Arg Ile Glu Asp Val Ala Ala Gly Gln Pro Gln His Val Arg
225                 230                 235                 240
Ile Gly Leu Pro Pro Trp Met Leu Ser His Ile Asn Gly
                245                 250

<210>  140
<211>  741
<212>  DNA
<213>  Oryza sativa

<400>  140
atggggcggg gcaaggtgca gctgaagcgg atcgagaaca agatcaaccg gcaggtgacc      60
ttctccaagc gcaggtcaaa actgctcaag aaggcgaatg agatctccgt gctctgcgac     120
gccgaggtcg cgctcatcat cttctccacc aagggcaagc tctacgagta cgccaccgac     180
tcatgtatgg acaaaatcct tgaacgttat gagcgctact cctatgcaga aaaggtcctt     240
atttcagctg aatctgacac tcagggcaac tggtgccacg aatataggaa actgaaggct     300
aaggttgaga caatacagaa atgtcaaaag cacctcatgg gagaggatct tgaatctttg     360
aatctcaaag agctgcagca gctggagcag cagctggaaa attcgttgaa acatatcaga     420
tccagaaaga gccaactaat gctcgagtcc attaacgagc ttcaacggaa ggaaaagtca     480
ctgcaggagg agaataaggt cctacagaaa gaactggtgg agaagcagaa agtccagaag     540
caacaagtgc aatgggacca gacacaacct caaacaagtt cctcatcatc ctccttcatg     600
atgagggaag cccttccaac aactaatatc agtaactacc ctgcagcagc tggcgaaagg     660
atagaggatg tagcagcagg gcagccacag catgaacgca ttgggctgcc accatggatg     720
ctgagccaca tcaacggcta a                                               741

<210>  141
<211>  246
<212>  PRT
<213>  Oryza sativa

<400>  141
Met Gly Arg Gly Lys Val Gln Leu Lys Arg Ile Glu Asn Lys Ile Asn
1               5                   10                  15
Arg Gln Val Thr Phe Ser Lys Arg Arg Ser Lys Leu Leu Lys Lys Ala

                20                  25                  30
Asn Glu Ile Ser Val Leu Cys Asp Ala Glu Val Ala Leu Ile Ile Phe
        35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Asp Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Asn Ser Leu Lys His Ile Arg Ser Arg Lys Ser
    130                 135                 140
Gln Leu Met Leu Glu Ser Ile Asn Glu Leu Gln Arg Lys Glu Lys Ser
145                 150                 155                 160
Leu Gln Glu Glu Asn Lys Val Leu Gln Lys Glu Leu Val Glu Lys Gln
                165                 170                 175
Lys Val Gln Lys Gln Gln Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Asn Tyr Pro Ala Ala Ala Gly Glu Arg Ile Glu Asp Val
    210                 215                 220
Ala Ala Gly Gln Pro Gln His Glu Arg Ile Gly Leu Pro Pro Trp Met
225                 230                 235                 240
Leu Ser His Ile Asn Gly
                245

<210>  142
<211>  735
<212>  DNA
<213>  Dendrocalamus latiflorus

<400>  142
atggggcgcg ggaaggtgca gctgaagcgg atcgagaaca agatcaaccg gcaagtgact      60
ttctccaagc gccggtcggg gctgctcaag aaggcgcatg agatctccgt cctctgcgac     120
gccgaggtcg gccttatcat cttctccacc aagggcaagc tctacgagta cgccaccgac     180
tcatgtatgg acaaaattct tgaacggtac gagcgttact cctatgcaga aaaggttctt     240
atttcagccg aatctgaaac tcagggcaac tggtgtcacg aatataggaa actgaaggcg     300
aaggttgaga cgatacagaa atgtcaaaag cacctcatgg gagaggatcc tgaatctttg     360
aatctcaagg agctgcagca actcgagcag cagctggaaa gttcagtgaa acatatcaga     420
tccagaaaga gccagcttat gctcgagtcc atttccgagc ttcaaaagaa ggagaagtca     480
ctgcaggagg agaacaaggt tctgcagaag gaactcgtgg agaagcagca ggtccataaa     540
cggttagtgc aatgggacca aactcagccg caaactagtt cctcttcctc gtccttcatg     600
atgagggaag ctctcccaac aacaaatatc agtatttacg ctgcggcagc cggcgagagg     660
gcagaggacg cagcagggca gcctcagatt cacattgggc tgccgccatg gatggtgagc     720
cacatcaacg gctaa                                                      735

<210>  143
<211>  244
<212>  PRT
<213>  Dendrocalamus latiflorus

<400>  143
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
            35                  40                  45
Ser Thr Lys Gly Lys Leu Tyr Glu Tyr Ala Thr Asp Ser Cys Met Asp
    50                  55                  60
Lys Ile Leu Glu Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Pro Glu Ser Leu Asn Leu Lys Glu Leu Gln Gln Leu
        115                 120                 125
Glu Gln Gln Leu Glu Ser Ser Val Lys His Ile Arg Ser Arg Lys Ser
    130                 135                 140
Gln Leu Met Leu Glu Ser Ile Ser Glu Leu Gln Lys Lys Glu Lys Ser
145                 150                 155                 160
Leu Gln Glu Glu Asn Lys Val Leu Gln Lys Glu Leu Val Glu Lys Gln
                165                 170                 175
Gln Val His Lys Arg Leu Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Ile Tyr Ala Ala Ala Ala Gly Glu Arg Ala Glu Asp Ala
    210                 215                 220
Ala Gly Gln Pro Gln Ile His Ile Gly Leu Pro Pro Trp Met Val Ser
225                 230                 235                 240
His Ile Asn Gly

<210>  144
<211>  735
<212>  DNA
<213>  Dendrocalamus latiflorus

<400>  144
atggggcgcg ggaaggtgca gctgaagcgg atcgagaaca agatcaaccg gcaagtgact      60
ttctccaagc gccggtcggg gctgctcaag aaggcgcatg agatctccgt cctctgcgac     120
gccgaggtcg gccttatcat cttctccacc aagggcaagc tctacgagta cgccaccgac     180
tcatgtatgg acaaaattct tgaacggtac gagcgttact cctatgcaga aaaggttctt     240
atttcagccg aatctgaaac tcagggcaac tggtgtcacg aatataggaa actgaaggcg     300
aaggttgaga cgatacagaa atgtcaaaag cacctcatgg gagaggatct tgaatctttg     360
aatctcaagg agctgcagca actcgagcag cagctggaaa gttcagtgaa acatatcaga     420
tccagaaaga gccagcttat gctcgagtcc atttccgagc ttcaaaagaa ggagaagtca     480
ctgcaggagg agaacaaggt tctgcagaag gaactcgtgg agaagcagca ggtccataaa     540
cggttagtgc aatggaccca aactcagccg caaactagtt cctcttcctc gtccttcatg     600
atgagggaag ctctcccaac aacaaatatc agtatttacg ctgcggcagc cggcgagagg     660
gcagaggacg cagcagggca gcctcagatt cacattgggc tgccgccatg gatggtgagc     720
cacatcaacg gctaa                                                      735

<210>  145
<211>  244
<212>  PRT
<213>  Dendrocalamus latiflorus

<400>  145
Met Gly Arg Gly Lys Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala

                20                  25                  30
His Glu Ile Ser Val Leu Cys Asp Ala Glu Val Gly Leu Ile Ile Phe
            35                  40                  45
Ser Thr Lys Gly Lys Leu Tyr Glu Tyr Ala Thr Asp Ser Cys Met Asp
        50                  55                  60
Lys Ile Leu Glu Arg Tyr Glu Arg Tyr Ser Tyr Ala Glu Lys Val Leu
65                  70                  75                  80
Ile Ser Ala Glu Ser Glu Thr Gln Gly Asn Trp Cys His Glu Tyr Arg
                85                  90                  95
Lys Leu Lys Ala Lys Val Glu Thr Ile Gln Lys Cys Gln Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Val Lys His Ile Arg Ser Arg Lys Ser
    130                 135                 140
Gln Leu Met Leu Glu Ser Ile Ser Glu Leu Gln Lys Lys Glu Lys Ser
145                 150                 155                 160
Leu Gln Glu Glu Asn Lys Val Leu Gln Lys Glu Leu Val Glu Lys Gln
                165                 170                 175
Gln Val His Lys Arg Leu Val Gln Trp Asp Gln Thr Gln Pro Gln Thr
            180                 185                 190
Ser Ser Ser Ser Ser Ser Phe Met Met Arg Glu Ala Leu Pro Thr Thr
        195                 200                 205
Asn Ile Ser Ile Tyr Ala Ala Ala Ala Gly Glu Arg Ala Glu Asp Ala
    210                 215                 220
Ala Gly Gln Pro Gln Ile His Ile Gly Leu Pro Pro Trp Met Val Ser
225                 230                 235                 240
His Ile Asn Gly

<210>  146
<211>  813
<212>  DNA
<213>  Zea mays

<400>  146
atggggcgcg gcaaggtgca gctgaagcgg atagagaaca agataaaccg gcaggtgacc      60
ttctccaagc gccggaacgg gctgctgaag aaggcgcacg agatctccgt cctctgcgac     120
gccgaggtcg ccgtcatcgt cttctccccc aagggcaagc tctacgagta cgcctccgac     180
tcccgcatgg acaaaattct agaacgttat gagcgatatt cctatgctga aaaggctctt     240
atttcagctg aatctgaaag tgagggaaat tggtgccacg aatacaggaa actgaaggcc     300
aaaattgaga ccatacaaag atgccacaag cacctgatgg gagaggatct agagtctttg     360
aatccaaaag agctccaaca actagagcag cagctggaga gctcactgaa gcacatcaga     420
tcaagaaaga gccaccttat ggccgagtca atttctgagc tacagaagaa ggagaggtca     480
ctgcaggagg agaacaagat tctacagaag gaactttcag agaggcagaa ggcggtcgct     540
agccggcagc agcagcagca gcaggtgcag tgggaccagc agacacaggt ccaggtccag     600
acaagctcat cgtcttcttc cttcatgatg aggcaggatc agcagggact gccacctcca     660
caaaacatct gcttcccgcc gttgagcatc ggagagagag gcgaagaggt ggctgcggcg     720
gcgcagcagc agctgcctcc tccggggcag gcgcaaccac agctccgcat cgcaggtctg     780
ccgccatgga tgctgagcca cctcaatgca taa                                  813

<210>  147
<211>  270
<212>  PRT
<213>  Zea mays

<400>  147
Met Gly Arg Gly Lys Val Gln Leu Lys Arg Ile Glu Asn Lys Ile Asn
1               5                   10                  15

Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Val Ile Val Phe
        35                  40                  45
Ser Pro Lys Gly Lys Leu Tyr Glu Tyr Ala Ser Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Arg Cys His Lys His Leu
            100                 105                 110
Met Gly Glu Asp Leu Glu Ser Leu Asn Pro Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Ala Glu Ser Ile Ser Glu Leu Gln Lys Lys Glu Arg Ser
145                 150                 155                 160
Leu Gln Glu Glu Asn Lys Ile Leu Gln Lys Glu Leu Ser Glu Arg Gln
                165                 170                 175
Lys Ala Val Ala Ser Arg Gln Gln Gln Gln Gln Gln Val Gln Trp Asp
            180                 185                 190
Gln Gln Thr Gln Val Gln Val Gln Thr Ser Ser Ser Ser Ser Ser Phe
        195                 200                 205
Met Met Arg Gln Asp Gln Gln Gly Leu Pro Pro Pro Gln Asn Ile Cys
    210                 215                 220
Phe Pro Pro Leu Ser Ile Gly Glu Arg Gly Glu Glu Val Ala Ala Ala
225                 230                 235                 240
Ala Gln Gln Gln Leu Pro Pro Pro Gly Gln Ala Gln Pro Gln Leu Arg
                245                 250                 255
Ile Ala Gly Leu Pro Pro Trp Met Leu Ser His Leu Asn Ala
            260                 265                 270

<210>  148
<211>  689
<212>  DNA
<213>  Sorghum bicolor

<400>  148
gcaaggtgca gctcaagcgg atagagaaca agataaaccg gcaggtgacc ttctccaagc      60
gccgcaacgg gctgctcaag aaggcgcacg agatctccgt cctctgcgac gccgaggtcg     120
ccgtcatcgt cttctccccc aagggcaagc tctatgagta cgccaccgac tcccgcatgg     180
acaaaattct cgaacgttat gagcgatatt cctatgctga aaaggctctt atttcagctg     240
aatctgaaag tgagggaaac tggtgccacg aatacaggaa actgaaggcc aaaattgaga     300
ccattcaaaa atgccacaag cacctgatgg gagaggatct agagtctttg aatcccaaag     360
agctccaaca actagagcag cagctggaga gctcactgaa gcacatcaga tcaagaaaga     420
gccaccttat ggctgagtct atttctgaac tacagaagaa ggagaggtca ctgcaggagg     480
agaacaaggc tctacagaag gaacttgcgg agaggcagaa ggcggccgcg agcaggcagc     540
agcagcaagg tgcagtggga ccagcagaca cagacccagg cccagacaag ctcatcatcg     600
tcctccttca tgatgaggca ggatcagcag ggtctgccgc ctccacaaaa catatgcttc     660
ccgccgctga taatcggaga gagaggtga                                       689

<210>  149
<211>  228
<212>  PRT
<213>  Sorghum bicolor

<400>  149
Lys Val Gln Leu Lys Arg Ile Glu Asn Lys Ile Asn Arg Gln Val Thr

1                   5                   10                  15
Phe Ser Lys Arg Arg Asn Gly Leu Leu Lys Lys Ala His Glu Ile Ser
            20                  25                  30
Val Leu Cys Asp Ala Glu Val Ala Val Ile Val Phe Ser Pro Lys Gly
        35                  40                  45
Lys Leu Tyr Glu Tyr Ala Thr Asp Ser Arg Met Asp Lys Ile Leu Glu
    50                  55                  60
Arg Tyr Glu Arg Tyr Ser Tyr Ala Glu Lys Ala Leu Ile Ser Ala Glu
65                  70                  75                  80
Ser Glu Ser Glu Gly Asn Trp Cys His Glu Tyr Arg Lys Leu Lys Ala
                85                  90                  95
Lys Ile Glu Thr Ile Gln Lys Cys His Lys His Leu Met Gly Glu Asp
            100                 105                 110
Leu Glu Ser Leu Asn Pro Lys Glu Leu Gln Gln Leu Glu Gln Gln Leu
        115                 120                 125
Glu Ser Ser Leu Lys His Ile Arg Ser Arg Lys Ser His Leu Met Ala
    130                 135                 140
Glu Ser Ile Ser Glu Leu Gln Lys Lys Glu Arg Ser Leu Gln Glu Glu
145                 150                 155                 160
Asn Lys Ala Leu Gln Lys Glu Leu Ala Glu Arg Gln Lys Ala Ala Ala
                165                 170                 175
Ser Arg Gln Gln Gln Gln Gly Ala Val Gly Pro Ala Asp Thr Asp Pro
            180                 185                 190
Gly Pro Asp Lys Leu Ile Ile Val Leu Leu His Asp Glu Ala Gly Ser
        195                 200                 205
Ala Gly Ser Ala Ala Ser Thr Lys His Met Leu Pro Ala Ala Asp Asn
    210                 215                 220
Arg Arg Glu Arg
225

<210>  150
<211>  822
<212>  DNA
<213>  Zea mays

<400>  150
atggggcgcg gcaaggtaca gctgaagcgg atagagaaca agataaaccg gcaggtgacc      60
ttctccaagc gccggaacgg cctgctcaag aaggcgcacg agatctccgt cctctgcgat     120
gccgaggtcg ccgtcatcgt cttctccccc aagggcaagc tctacgagta cgccaccgac     180
tcccgcatgg acaaaattct tgaacgctat gagcgatatt cctatgctga aaaggctctt     240
atttcagctg aatctgaaag tgagggaaat tggtgccacg aatacaggaa actgaaggcc     300
aaaattgaga ccatacaaaa atgccacaag cacctgatgg gagaggatct agagtctttg     360
aatcccaaag agctccagca actagagcag cagctggata gctcactgaa gcacatcaga     420
tcaaggaaga gccaccttat ggccgagtct atttctgagc tacagaagaa ggagaggtca     480
ctgcaggagg agaacaaggc tctgcagaag gaacttgcgg agaggcagaa ggccgtcgcg     540
agccggcagc agcagcaaca gcagcaggtg cagtgggacc agcagacaca tgcccaggcc     600
cagacaagct catcatcgtc ctccttcatg atgaggcagg atcagcaggg actgccgcct     660
ccacacaaca tctgcttccc gccgttgaca atgggagata gaggtgaaga gctggctgcg     720
gcggcggcgg cgcagcagca gcagccactg ccggggcagg cgcaaccgca gctccgcatc     780
gcaggtctgc caccatggat gctgagccac ctcaatgcat aa                        822

<210>  151
<211>  273
<212>  PRT
<213>  Zea mays

<400>  151
Met Gly Arg Gly Lys Val Gln Leu Lys Arg Ile Glu Asn Lys Ile Asn
1               5                   10                  15

Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Val Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Pro Lys Glu Leu Gln Gln Leu
        115                 120                 125
Glu Gln Gln Leu Asp Ser Ser Leu Lys His Ile Arg Ser Arg Lys Ser
    130                 135                 140
His Leu Met Ala Glu Ser Ile Ser Glu Leu Gln Lys Lys Glu Arg Ser
145                 150                 155                 160
Leu Gln Glu Glu Asn Lys Ala Leu Gln Lys Glu Leu Ala Glu Arg Gln
                165                 170                 175
Lys Ala Val Ala Ser Arg Gln Gln Gln Gln Gln Gln Gln Val Gln Trp
            180                 185                 190
Asp Gln Gln Thr His Ala Gln Ala Gln Thr Ser Ser Ser Ser Ser Ser
        195                 200                 205
Phe Met Met Arg Gln Asp Gln Gln Gly Leu Pro Pro Pro His Asn Ile
    210                 215                 220
Cys Phe Pro Pro Leu Thr Met Gly Asp Arg Gly Glu Glu Leu Ala Ala
225                 230                 235                 240
Ala Ala Ala Ala Gln Gln Gln Gln Pro Leu Pro Gly Gln Ala Gln Pro
                245                 250                 255
Gln Leu Arg Ile Ala Gly Leu Pro Pro Trp Met Leu Ser His Leu Asn
            260                 265                 270
Ala

<210>  152
<211>  786
<212>  DNA
<213>  Lolium temulentum

<400>  152
atgggtcgcg gcaaggtgca gctgaagcgg atagagaaca agataaaccg tcaggtgaca      60
ttctccaagc gccgcaacgg gctactcaag aaggcgcacg agatctccgt cctctgcgac     120
gccgaggtcg ccgtcgtcgt cttctccccg aaagggaagc tctatgagta cgccactgac     180
tccagcatgg acaaaattct tgaacgttat gaacgctact cttatgctga aaaggctttg     240
atttcagctg aatctgaaag tgagggaaat tggtgccatg aatacaggaa gctgaaggcg     300
aagattgaga ctatacaaaa atgtcacaag cacctcatgg gggaggatct ggagtgtcta     360
aacctgaaag agctccaaca actagagcag cagctggaga gttcattgaa gcacatcaga     420
tcgagaaaga gccaccttat gatggagtcc atttctgagc tacagaagaa ggagcggtca     480
ctccaggagg agaacaaggc tctacagaag gaactggtgg agaggcagaa ggcggccagg     540
cagcagcagc aagagcagtg ggaccgtcag acccaaacac aacaagccca aaaccaacct     600
caggcccaga cgagctcatc atcttcctcc ttcatgatga gggatcagca ggcccatgct     660
caacaaaaca tctgttaccc gctggtgaca atgggtggag aggctgtggc cgcggcgcca     720
gggcagcagg ggcagcttcg catcggaggc ctgccaccat ggatgctgag ccacctcaac     780
gcttga                                                                786

<210>  153
<211>  261
<212>  PRT

<213>  Lolium temulentum

<400>  153
   Met Gly Arg Gly Lys Val Gln Leu Lys Arg Ile Glu Asn Lys Ile Asn
   1               5                   10                  15
   Arg Gln Val Thr Phe Ser Lys Arg Arg Asn Gly Leu Leu Lys Lys Ala
               20                  25                  30
   His Glu Ile Ser Val Leu Cys Asp Ala Glu Val Ala Val Val Val Phe
           35                  40                  45
   Ser Pro Lys Gly Lys Leu Tyr Glu Tyr Ala Thr Asp Ser Ser Met Asp
       50                  55                  60
   Lys Ile Leu Glu Arg Tyr Glu Arg Tyr Ser Tyr Ala Glu Lys Ala Leu
   65                  70                  75                  80
   Ile Ser Ala Glu Ser Glu Ser Glu Gly Asn Trp Cys His Glu Tyr Arg
                   85                  90                  95
   Lys Leu Lys Ala Lys Ile Glu Thr Ile Gln Lys Cys His Lys His Leu
               100                 105                 110
   Met Gly Glu Asp Leu Glu Cys Leu Asn Leu Lys Glu Leu Gln Gln Leu
           115                 120                 125
   Glu Gln Gln Leu Glu Ser Ser Leu Lys His Ile Arg Ser Arg Lys Ser
       130                 135                 140
   His Leu Met Met Glu Ser Ile Ser Glu Leu Gln Lys Lys Glu Arg Ser
   145                 150                 155                 160
   Leu Gln Glu Glu Asn Lys Ala Leu Gln Lys Glu Leu Val Glu Arg Gln
                   165                 170                 175
   Lys Ala Ala Arg Gln Gln Gln Gln Glu Gln Trp Asp Arg Gln Thr Gln
               180                 185                 190
   Thr Gln Gln Ala Gln Asn Gln Pro Gln Ala Gln Thr Ser Ser Ser Ser
           195                 200                 205
   Ser Ser Phe Met Met Arg Asp Gln Gln Ala His Ala Gln Gln Asn Ile
       210                 215                 220
   Cys Tyr Pro Leu Val Thr Met Gly Gly Glu Ala Val Ala Ala Ala Pro
   225                 230                 235                 240
   Gly Gln Gln Gly Gln Leu Arg Ile Gly Gly Leu Pro Pro Trp Met Leu
                   245                 250                 255
   Ser His Leu Asn Ala
               260

<210>  154
<211>  786
<212>  DNA
<213>  Lolium perenne

<400>  154
atgggtcgcg gcaaggtgca gctgaagcgg atagagaaca agataaaccg tcaggtgacc      60
ttctccaagc gccgcaacgg gctactcaag aaggcgcacg agatctccgt cctctgcgac     120
gccgaggtcg ccgtcgtcgt cttctccccg aaagggaagc tctatgagta cgccactgac     180
tccagcatgg acaaaattct tgaacgttat gaacgctact cttatgctga aaaggctttg     240
atttcagctg aatctgaaag tgagggaaat tggtgccatg aatacaggaa actgaaggcg     300
aagattgaga ctatacaaaa atgtcacaag cacctcatgg gggaggatct ggagtgtcta     360
aacctgaaag agctccaaca actagagcag cagctggaga gttcattgaa gcacatcaga     420
tcgagaaaga gccaccttat gatggagtcc atttctgagc tacagaagaa ggagcggtca     480
ctccaggagg agaacaaggc tctacagaag gaactggtgg agaggcagaa ggcggccagg     540
cagcagcagc aagagcagtg ggaccgtcag acccaaacac aacaagccca aaaccaacct     600
caggcccaga cgagctcatc atcttcctcc ttcatgatga gggatcagca ggcccatgct     660
caacaaaaca tctgttaccc gccggtgaca atgggtggag aggctgtggc cgcggcgcca     720
gggcagcagg ggcagcttcg catcggaggc ctgccaccat ggatgctgag ccacctcaac     780
gcttga                                                                786

<210>  155
<211>  261
<212>  PRT
<213>  Lolium perenne

<400>  155
   Met Gly Arg Gly Lys Val Gln Leu Lys Arg Ile Glu Asn Lys Ile Asn
   1 5 10 15
   Arg Gln Val Thr Phe Ser Lys Arg Arg Asn Gly Leu Leu Lys Lys Ala
   20 25 30
   His Glu Ile Ser Val Leu Cys Asp Ala Glu Val Ala Val Val Val Phe
   35 40 45
   Ser Pro Lys Gly Lys Leu Tyr Glu Tyr Ala Thr Asp Ser Ser Met Asp
   50 55 60
   Lys Ile Leu Glu Arg Tyr Glu Arg Tyr Ser Tyr Ala Glu Lys Ala Leu
   65 70 75 80
   Ile Ser Ala Glu Ser Glu Ser Glu Gly Asn Trp Cys His Glu Tyr Arg
   85 90 95
   Lys Leu Lys Ala Lys Ile Glu Thr Ile Gln Lys Cys His Lys His Leu
   100 105 110
   Met Gly Glu Asp Leu Glu Cys Leu Asn Leu Lys Glu Leu Gln Gln Leu
   115 120 125
   Glu Gln Gln Leu Glu Ser Ser Leu Lys His Ile Arg Ser Arg Lys Ser
   130 135 140
   His Leu Met Met Glu Ser Ile Ser Glu Leu Gln Lys Lys Glu Arg Ser
   145 150 155 160
   Leu Gln Glu Glu Asn Lys Ala Leu Gln Lys Glu Leu Val Glu Arg Gln
   165 170 175
   Lys Ala Ala Arg Gln Gln Gln Gln Glu Gln Trp Asp Arg Gln Thr Gln
   180 185 190
   Thr Gln Gln Ala Gln Asn Gln Pro Gln Ala Gln Thr Ser Ser Ser Ser
   195 200 205
   Ser Ser Phe Met Met Arg Asp Gln Gln Ala His Ala Gln Gln Asn Ile
   210 215 220
   Cys Tyr Pro Pro Val Thr Met Gly Gly Glu Ala Val Ala Ala Ala Pro
   225 230 235 240
   Gly Gln Gln Gly Gln Leu Arg Ile Gly Gly Leu Pro Pro Trp Met Leu
   245 250 255
   Ser His Leu Asn Ala
   260

<210>  156
<211>  831
<212>  DNA
<213>  Hordeum vulgare

<400>  156
atgggtcgcg gtaaggtgca gctgaagcgg atagagaaca agataaatcg gcaggtgacc      60
ttctccaagc gccgcaacgg gctcctgaag aaggcgcacg agatctccgt cctctgtgac     120
gcagaggtcg ccgtcatcgt cttctccccc aaaggcaagc tctatgagta cgccaccgac     180
tccagcatgg acaaaattct tgaacgttat gagcgctact cttatgctga aaaggctctt     240
atttcagctg aatctgaaag tgaggggaat tggtgtcatg aatacaggaa acttaaggcg     300
aagattgaga ccatacagaa gtgtcacaag cacctcatgg gagaggatct ggattctctg     360
aacctcaaag aactccaaca actggagcag cagctggaga gttcattgaa gcacatcaga     420
tcgagaaaga gccatcttat gatggagtcc atttctgagc tacagaagaa ggagaggtca     480
ctgcaggagg agaacaaggc cctacagaag gaactggtgg agaggcagaa ggcggccagc     540
aggcagcagc agctgcagca gcagcaacaa caacaacaaa tgcaatggga gcaccaagcc     600
cagacccaaa cccataccca tactcaaaac cagccccaag cccagactag ctcatcatct     660
tcctctttca tgatgaggga tcagcaggcc catgcccctc aacagaacat ttgtagctac     720

ccaccggtga cgatgggtgg ggaggcgacg gcggcggcgg cggcgccgga gcagcaggct 780
cagcttcgca tatgcctacc gccatggatg ctgagccacc tcaacgcttg a 831

<210> 157
<211> 276
<212> PRT
<213> Hordeum vulgare

<400> 157
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Val Ile Val Phe
        35                  40                  45
Ser Pro Lys Gly Lys Leu Tyr Glu Tyr Ala Thr Asp Ser Ser Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Asp Ser Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Met Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Ala Ala Ser Arg Gln Gln Gln Leu Gln Gln Gln Gln Gln Gln Gln
            180                 185                 190
Gln Met Gln Trp Glu His Gln Ala Gln Thr Gln Thr His Thr His Thr
        195                 200                 205
Gln Asn Gln Pro Gln Ala Gln Thr Ser Ser Ser Ser Ser Ser Phe Met
    210                 215                 220
Met Arg Asp Gln Gln Ala His Ala Pro Gln Gln Asn Ile Cys Ser Tyr
225                 230                 235                 240
Pro Pro Val Thr Met Gly Gly Glu Ala Thr Ala Ala Ala Ala Ala Pro
                245                 250                 255
Glu Gln Gln Ala Gln Leu Arg Ile Cys Leu Pro Pro Trp Met Leu Ser
            260                 265                 270
His Leu Asn Ala
        275

<210> 158
<211> 804
<212> DNA
<213> Oryza sativa

<400> 158
atggggcggg ggaaggtgca gctgaagcgg atagagaact cgatgaaccg gcaggtgacg 60
ttctccaaga ggaggaatgg attgctgaag aaggcgcacg agatctccgt cctctgcgac 120
gccgaggtcg ccgccatcgt cttctccccc aagggcaagc tctacgagta cgccactgac 180
tccaggatgg acaaaatcct tgaacgttat gagcgctatt catatgctga aaaggctctt 240
atttcagctg aatctgagag tgagggaaat tggtgccatg aatacaggaa gcttaaggca 300
aagattgaga ccatacaaaa atgtcacaaa cacctcatgg gagaggatct agaatccctg 360
aatctcaaag aactccaaca gctagagcag cagctggaga gttcattgaa gcacataaga 420

tcaagaaaga gccaccttat gcttgagtcc atttccgagc tgcagaaaaa ggagaggtca 480
ctgcaggagg agaacaaggc tctgcagaag gaactggtgg agaggcagaa gaatgtgagg 540
ggccagcagc aagtagggca gtgggaccaa acccaggtcc aggcccaggc ccaagcccaa 600
ccccaagccc agacaagctt cttcttcttc ttcatgctga gggatcagca ggcacttctt 660
tcaccacaaa atatctgcta cccgccggtg atgatgggcc agagaaatga tgcggcggcg 720
cggcggcggt ggcggcccaa ggccaggtgc aacttccgca ttggaggctt tccgccatgg 780
atgctgagca ccttcaaggc ttaa 804

<210> 159
<211> 267
<212> PRT
<213> Oryza sativa

<400> 159
Met Gly Arg Gly Lys Val Gln Leu Lys Arg Ile Glu Asn Ser Met Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp Leu Glu Ser Leu Asn Leu Lys Glu Leu Gln Gln Leu
        115                 120                 125
Glu Gln Gln Leu Glu Ser Ser Leu Lys His Ile Arg Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Phe Phe
        195                 200                 205
Phe Phe Phe Met Leu Arg Asp Gln Gln Ala Leu Leu Ser Pro Gln Asn
    210                 215                 220
Ile Cys Tyr Pro Pro Val Met Met Gly Gln Arg Asn Asp Ala Ala Ala
225                 230                 235                 240
Arg Arg Arg Trp Arg Pro Lys Ala Arg Cys Asn Phe Arg Ile Gly Gly
                245                 250                 255
Phe Pro Pro Trp Met Leu Ser Thr Phe Lys Ala
            260                 265

<210> 160
<211> 804
<212> DNA
<213> Oryza sativa

<400> 160
atggggcggg ggaaggtgca gctgaagcgg atagagaaca agatcaacag gcaggtgacg 60
ttctccaaga ggaggaatgg attgctgaag aaggcgcacg agatctccgt cctctgcgac 120
gccgaggtcg ccgccatcgt cttctccccc aagggcaagc tctacgagta cgccactgac 180
tccaggatgg acaaaatcct tgaacgttat gagcgctatt catatgctga aaaggctctt 240

atttcagctg aatccgagag tgagggaaat tggtgccatg aatacaggaa acttaaggca 300
aagattgaga ccatacaaaa atgtcacaaa cacctcatgg gagaggatca tgaatccctg 360
aatctcaaag aactccaaca gctagagcag cagctggaga gttcattgaa gcacataata 420
tcaagaaaga gccaccttat gcttgagtcc atttccgagc tgcagaaaaa ggagaggtca 480
ctgcaggagg agaacaaggc tctgcagaag gaactggtgg agaggcagaa gaatgtgagg 540
ggccagcagc aagtagggca gtgggaccaa acccaggtcc aggcccaggc ccaagcccaa 600
ccccaagccc agacaagctc ctcctcctcc tccatgctga gggatcagca ggcacttctt 660
ccaccacaaa atatctgcta cccgccggtg atgatgggcg agagaaatga tgcggcggcg 720
gcggcggcgg tggcggcgca gggccaggtg caactccgca tcggaggtct tccgccatgg 780
atgctgagcc acctcaatgc ttaa 804

<210> 161
<211> 267
<212> PRT
<213> Oryza sativa

<400> 161
Met Gly Arg Gly Lys Val Gln Leu Lys Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Ala Ile Val Phe
        35                  40                  45
Ser Pro Lys Gly Lys Leu Tyr Glu Tyr Ala Thr Asp Ser Arg Met Asp
    50                  55                  60
Lys Ile Leu Glu Arg Tyr Glu Arg Tyr Ser Tyr Ala Glu Lys Ala Leu
65                  70                  75                  80
Ile Ser Ala Glu Ser Glu Ser Glu Gly Asn Trp Cys His Glu Tyr Arg
                85                  90                  95
Lys Leu Lys Ala Lys Ile Glu Thr Ile Gln Lys Cys His Lys His Leu
            100                 105                 110
Met Gly Glu Asp His Glu Ser Leu Asn Leu Lys Glu Leu Gln Gln Leu
        115                 120                 125
Glu Gln Gln Leu Glu Ser Ser Leu Lys His Ile Ile Ser Arg Lys Ser
    130                 135                 140
His Leu Met Leu Glu Ser Ile Ser Glu Leu Gln Lys Lys Glu Arg Ser
145                 150                 155                 160
Leu Gln Glu Glu Asn Lys Ala Leu Gln Lys Glu Leu Val Glu Arg Gln
                165                 170                 175
Lys Asn Val Arg Gly Gln Gln Gln Val Gly Gln Trp Asp Gln Thr Gln
            180                 185                 190
Val Gln Ala Gln Ala Gln Ala Gln Pro Gln Ala Gln Thr Ser Ser Ser
        195                 200                 205
Ser Ser Ser Met Leu Arg Asp Gln Gln Ala Leu Leu Pro Pro Gln Asn
    210                 215                 220
Ile Cys Tyr Pro Pro Val Met Met Gly Glu Arg Asn Asp Ala Ala Ala
225                 230                 235                 240
Ala Ala Ala Val Ala Ala Gln Gly Gln Val Gln Leu Arg Ile Gly Gly
                245                 250                 255
Leu Pro Pro Trp Met Leu Ser His Leu Asn Ala
            260                 265

<210> 162
<211> 668
<212> DNA
<213> Tradescantia virginiana

<400> 162
gggctggtga agaaggctca tgagatctcd atkytrtgtg atgcygaggt tgcgcttatt 60

attttctcta ctaaaggcaa actatacgag tatgccactg attccaaaat ggaaaatatc 120
cttgaacgct atgaacgtta ctcatatgct gagaaggctt taacttcatc agatcctgaa 180
ttacagggaa attggtgcca agagtatgtt aaacttaagg ctaaggttga ggccttacat 240
aaaagccaaa ggcatcttat gggagagcaa ctagaagcgt tggatctcaa agaattgcag 300
caactagagc atcaacttga aggttctttg aggcttgtca ggtcaagaaa gactcaaatg 360
atgttggact ccatttccga acttcagagg aaggaaaagt ctctggaaga gcaaaacaag 420
aacctagaga aggagatttt ggagaagcag aaagaaaagg ctctggcaca ccaagctcac 480
tgggaacagc agaatcagcc actacaaagc actaattcgc ctccaaggcc cttcgtgatt 540
gcagaaactc atccaacact aaacattgga aatttccaag gtagaacaaa taccgtccat 600
gcagaagaaa gtctgcagcg tcagatgagg atcagcagca gcctactgcc mycmtggatg 660
mttcacma 668

<210> 163
<211> 222
<212> PRT
<213> Tradescantia virginiana

<220>
<221> UNSURE
<222> (11)..(11)
<223> Xaa can be any naturally occurring amino acid

<220>
<221> UNSURE
<222> (218)..(218)
<223> Xaa can be any naturally occurring amino acid

<220>
<221> UNSURE
<222> (221)..(221)
<223> Xaa can be any naturally occurring amino acid

<400> 163
Gly Leu Val Lys Lys Ala His Glu Ile Ser Xaa Leu Cys Asp Ala Glu
1               5                   10                  15
Val Ala Leu Ile Ile Phe Ser Thr Lys Gly Lys Leu Tyr Glu Tyr Ala
            20                  25                  30
Thr Asp Ser Lys Met Glu Asn Ile Leu Glu Arg Tyr Glu Arg Tyr Ser
        35                  40                  45
Tyr Ala Glu Lys Ala Leu Thr Ser Ser Asp Pro Glu Leu Gln Gly Asn
    50                  55                  60
Trp Cys Gln Glu Tyr Val Lys Leu Lys Ala Lys Val Glu Ala Leu His
65                  70                  75                  80
Lys Ser Gln Arg His Leu Met Gly Glu Gln Leu Glu Ala Leu Asp Leu
                85                  90                  95
Lys Glu Leu Gln Gln Leu Glu His Gln Leu Glu Gly Ser Leu Arg Leu
            100                 105                 110
Val Arg Ser Arg Lys Thr Gln Met Met Leu Asp Ser Ile Ser Glu Leu
        115                 120                 125
Gln Arg Lys Glu Lys Ser Leu Glu Glu Gln Asn Lys Asn Leu Glu Lys
    130                 135                 140
Glu Ile Leu Glu Lys Gln Lys Glu Lys Ala Leu Ala His Gln Ala His
145                 150                 155                 160
Trp Glu Gln Gln Asn Gln Pro Leu Gln Ser Thr Asn Ser Pro Pro Arg
                165                 170                 175
Pro Phe Val Ile Ala Glu Thr His Pro Thr Leu Asn Ile Gly Asn Phe
            180                 185                 190
Gln Gly Arg Thr Asn Thr Val His Ala Glu Glu Ser Leu Gln Arg Gln
        195                 200                 205

Met Arg Ile Ser Ser Ser Leu Leu Pro Xaa Trp Met Xaa His
    210                 215                 220

<210>  164
<211>  723
<212>  DNA
<213>  Tradescantia virginiana

<400>  164
gtgcagctga aacggatgga gaacaagatt aacaggcagg tgacgttttc taaacgtcga      60
ggagggctgc tgaagaaagc tcatgagatc tctattctat gtgatgctga gattgctctt     120
attattttct ctactaaagg gaagctctat gagtatgcca ccaattccaa aatggacaat     180
attcttgaac gctatgagcg ttactcatat gctgaaaagg ctctaacttc atcagatcct     240
gatatacagg gaaattggtg ccaagagtat gctaaactta agtctaaggt tgaggcttta     300
tgtaaaagcc aaaggcatct tatgggagag cagcttgaaa cattgaatct caaagaattg     360
cagcaactag agcaacagct cgaaggttct ctaaagcatg tcaggtcaag aaagactcaa     420
gttatgctgg actctatttc tgaacttcag aggaaggaaa agtcactaga ggagcaaaac     480
aagaacctag agaaggagat tttggagaag cagaaaatca aggctcttgc acagcaggct     540
cactgggaac accagaatca accagcacca aggggttcac ctcctaggcc atttgtgatt     600
gcagagtctc atccgacact aaatattgga catttccaag gcaggacaaa tgcagtcgaa     660
gcagaagaaa atcagcagcc tcakatgaga atttgcagta gcctcctgcc cccctggatg     720
ctt                                                                   723

<210>  165
<211>  241
<212>  PRT
<213>  Tradescantia virginiana

<220>
<221>  UNSURE
<222>  (228)..(228)
<223>  Xaa can be any naturally occurring amino acid

<400>  165
Val Gln Leu Lys Arg Met Glu Asn Lys Ile Asn Arg Gln Val Thr Phe
1               5                   10                  15
Ser Lys Arg Arg Gly Gly Leu Leu Lys Lys Ala His Glu Ile Ser Ile
            20                  25                  30
Leu Cys Asp Ala Glu Ile Ala Leu Ile Ile Phe Ser Thr Lys Gly Lys
        35                  40                  45
Leu Tyr Glu Tyr Ala Thr Asn Ser Lys Met Asp Asn Ile Leu Glu Arg
    50                  55                  60
Tyr Glu Arg Tyr Ser Tyr Ala Glu Lys Ala Leu Thr Ser Ser Asp Pro
65                  70                  75                  80
Asp Ile Gln Gly Asn Trp Cys Gln Glu Tyr Ala Lys Leu Lys Ser Lys
                85                  90                  95
Val Glu Ala Leu Cys Lys Ser Gln Arg His Leu Met Gly Glu Gln Leu
            100                 105                 110
Glu Thr Leu Asn Leu Lys Glu Leu Gln Gln Leu Glu Gln Gln Leu Glu
        115                 120                 125
Gly Ser Leu Lys His Val Arg Ser Arg Lys Thr Gln Val Met Leu Asp
    130                 135                 140
Ser Ile Ser Glu Leu Gln Arg Lys Glu Lys Ser Leu Glu Glu Gln Asn
145                 150                 155                 160
Lys Asn Leu Glu Lys Glu Ile Leu Glu Lys Gln Lys Ile Lys Ala Leu
                165                 170                 175
Ala Gln Gln Ala His Trp Glu His Gln Asn Gln Pro Ala Pro Arg Gly
            180                 185                 190
Ser Pro Pro Arg Pro Phe Val Ile Ala Glu Ser His Pro Thr Leu Asn

        195                 200                 205
Ile Gly His Phe Gln Gly Arg Thr Asn Ala Val Glu Ala Glu Glu Asn
    210                 215                 220
Gln Gln Pro Xaa Met Arg Ile Cys Ser Ser Leu Leu Pro Pro Trp Met
225                 230                 235                 240
Leu

<210> 166
<211> 599
<212> DNA
<213> Tradescantia virginiana

<400> 166
gggctkgtga agaaagctca tgagatctcg gtactttgtg atgctgagct tgctcttatt 60
atcttctctc ccaaaggcaa gctctatgag tatgccaccg attccaaaat ggaaattatt 120
cttgaacgct atgaacgtta cacctacgct gaaaaagctt taattgcatc agatcctgat 180
gtacagggaa actggtgtca tgagtacatt aagcttaaag ctaaatttga ggccttgaat 240
aaaagccaga ggcatcttat gggagaacaa ctagatacgt tgaaccaaaa ggaattgctg 300
caactagaga ctaagcttga aggttctctg aaaaacgtca ggtcaagaaa gactcaactt 360
atgttggatt ccatttctga gcttcaagaa aagggaaagt cactccagga gcaaaacacc 420
tgcctagaaa aggagatttt gggaaaacag aaagacaagg ctcccaaaca gcatgttcag 480
tgggaaaaac agaatcaacc accacctacc tcttctgcgc caatgccatt cctcattggt 540
gatattcacc caacccctaa tatcagaaat ttccaaggca gaacagtagc tgatgcaga 599

<210> 167
<211> 199
<212> PRT
<213> Tradescantia virginiana

<400> 167
Gly Leu Val Lys Lys Ala His Glu Ile Ser Val Leu Cys Asp Ala Glu
1               5                   10                  15
Leu Ala Leu Ile Ile Phe Ser Pro Lys Gly Lys Leu Tyr Glu Tyr Ala
            20                  25                  30
Thr Asp Ser Lys Met Glu Ile Ile Leu Glu Arg Tyr Glu Arg Tyr Thr
        35                  40                  45
Tyr Ala Glu Lys Ala Leu Ile Ala Ser Asp Pro Asp Val Gln Gly Asn
    50                  55                  60
Trp Cys His Glu Tyr Ile Lys Leu Lys Ala Lys Phe Glu Ala Leu Asn
65                  70                  75                  80
Lys Ser Gln Arg His Leu Met Gly Glu Gln Leu Asp Thr Leu Asn Gln
                85                  90                  95
Lys Glu Leu Leu Gln Leu Glu Thr Lys Leu Glu Gly Ser Leu Lys Asn
            100                 105                 110
Val Arg Ser Arg Lys Thr Gln Leu Met Leu Asp Ser Ile Ser Glu Leu
        115                 120                 125
Gln Glu Lys Gly Lys Ser Leu Gln Glu Gln Asn Thr Cys Leu Glu Lys
    130                 135                 140
Glu Ile Leu Gly Lys Gln Lys Asp Lys Ala Pro Lys Gln His Val Gln
145                 150                 155                 160
Trp Glu Lys Gln Asn Gln Pro Pro Pro Thr Ser Ser Ala Pro Met Pro
                165                 170                 175
Phe Leu Ile Gly Asp Ile His Pro Thr Pro Asn Ile Arg Asn Phe Gln
            180                 185                 190
Gly Arg Thr Val Ala Asp Ala
        195

<210> 168

<211>  753
<212>  DNA
<213>  Elaeis guineensis

<400>  168
atggggagag ggagggtgca gctgaggcgg atcgagaaca agataaaccg gcaggtgacg      60
ttctcgaagc gccggtcggg gctcctgaag aaagcccacg agatctccgt cctctgcgac     120
gccgaggtcg ccctcatcat cttctcgacc aagggcaagc tctacgagta cgccaccgac     180
tcctgcatgg aaaggattct tgaacgctat gaacgttaca cctatgcaga aaaagcacta     240
atttcatctg gacccgaatt gcagggtaac tggtgccatg aatttggcaa actcaaagct     300
aaggttgagg ctttacaaaa aagccaaagg catctcatgg gtgagcaact tgagcccttg     360
aatctcaaag aactccagca actagagcaa cagcttgaaa gttctttaaa gcatataaga     420
accagaaagt gccaactcat gtttgaatcc atctctgagc ttcaaaaaaa ggaaaagtca     480
ctgcaggagc agaacaagat gctggagaag gagctcatgg agaagcagaa ggtgaaggca     540
ctaaaccagc aggcaccttg ggagcagcaa ggcccgccgc agacaagctc atcatcccca     600
acctccttcc tgatcggaga ctctctcccc accctgaata ttgggacata ccaatgtagc     660
ggaaatgaac atggggagga agcagcacaa ccccaggttc gtataggaaa cagcctgtta     720
ccaccttgga tgcttagcca cttgaacggg tag                                  753

<210>  169
<211>  250
<212>  PRT
<213>  Elaeis guineensis

<400>  169
Met Gly Arg Gly Arg Val Gln Leu Arg Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Ile Phe
        35                  40                  45
Ser Thr Lys Gly Lys Leu Tyr Glu Tyr Ala Thr Asp Ser Cys Met Glu
    50                  55                  60
Arg Ile Leu Glu Arg Tyr Glu Arg Tyr Thr Tyr Ala Glu Lys Ala Leu
65                  70                  75                  80
Ile Ser Ser Gly Pro Glu Leu Gln Gly Asn Trp Cys His Glu Phe Gly
                85                  90                  95
Lys Leu Lys Ala Lys Val Glu Ala Leu Gln Lys Ser Gln Arg His Leu
            100                 105                 110
Met Gly Glu Gln Leu Glu Pro Leu Asn Leu Lys Glu Leu Gln Gln Leu
        115                 120                 125
Glu Gln Gln Leu Glu Ser Ser Leu Lys His Ile Arg Thr Arg Lys Cys
    130                 135                 140
Gln Leu Met Phe Glu Ser Ile Ser Glu Leu Gln Lys Lys Glu Lys Ser
145                 150                 155                 160
Leu Gln Glu Gln Asn Lys Met Leu Glu Lys Glu Leu Met Glu Lys Gln
                165                 170                 175
Lys Val Lys Ala Leu Asn Gln Gln Ala Pro Trp Glu Gln Gln Gly Pro
            180                 185                 190
Pro Gln Thr Ser Ser Ser Ser Pro Thr Ser Phe Leu Ile Gly Asp Ser
        195                 200                 205
Leu Pro Thr Leu Asn Ile Gly Thr Tyr Gln Cys Ser Gly Asn Glu His
    210                 215                 220
Gly Glu Glu Ala Ala Gln Pro Gln Val Arg Ile Gly Asn Ser Leu Leu
225                 230                 235                 240
Pro Pro Trp Met Leu Ser His Leu Asn Gly
                245                 250

<210>  170

<211>  699
<212>  DNA
<213>  Allium sp.

<400>  170
gtgcaattga agaggatgga aaacaagatt aatagacaag tgaccttctc aaaaagaaga      60
aatggtttgt tgaagaaagc tcatgagatt tcwgtgcttt gtgatgcaga agttgcactt     120
attgttttct ctgctaaagg aaaactctat gaatattcaa ctgattcaag tatggaaaaa     180
attctggaga ggtatgaacg ttattgcttt gcggagaaat catcaacaat gagtgacatt     240
gactcccagg aggattggag ccttgaatat cacaaactga aggctaaggt tgagagttta     300
aacaacaggc aaaggcatct tatgggagag caacttgaat ctctgagtct tcgagaaatt     360
ggacagcttg agcaacaact tgagaattct ctcaaaactg ttcggacgcg caagagccaa     420
gaattgttaa gttctatttc agagcttcag gacaaggaga aaactttgcg agatgagaac     480
aaagctttag aaaatgagct tatgaaaagg gccagggcaa aagctattct ggaacaacaa     540
gcacgatgga agcatcataa tcataaacaa caggataatc ttcataatcc aaatatcaac     600
attggaaatt accaaacaag gaacaatgag ggaggagttg agccagcaac ggatgttcaa     660
gtacgtgttg ttagaaattt gttgccccac tggatgctt                            699

<210>  171
<211>  233
<212>  PRT
<213>  Allium sp.

<400>  171
Val Gln Leu Lys Arg Met Glu Asn Lys Ile Asn Arg Gln Val Thr Phe
1               5                   10                  15
Ser Lys Arg Arg Asn Gly Leu Leu Lys Lys Ala His Glu Ile Ser Val
            20                  25                  30
Leu Cys Asp Ala Glu Val Ala Leu Ile Val Phe Ser Ala Lys Gly Lys
        35                  40                  45
Leu Tyr Glu Tyr Ser Thr Asp Ser Ser Met Glu Lys Ile Leu Glu Arg
    50                  55                  60
Tyr Glu Arg Tyr Cys Phe Ala Glu Lys Ser Ser Thr Met Ser Asp Ile
65                  70                  75                  80
Asp Ser Gln Glu Asp Trp Ser Leu Glu Tyr His Lys Leu Lys Ala Lys
                85                  90                  95
Val Glu Ser Leu Asn Asn Arg Gln Arg His Leu Met Gly Glu Gln Leu
            100                 105                 110
Glu Ser Leu Ser Leu Arg Glu Ile Gly Gln Leu Glu Gln Gln Leu Glu
        115                 120                 125
Asn Ser Leu Lys Thr Val Arg Thr Arg Lys Ser Gln Glu Leu Leu Ser
    130                 135                 140
Ser Ile Ser Glu Leu Gln Asp Lys Glu Lys Thr Leu Arg Asp Glu Asn
145                 150                 155                 160
Lys Ala Leu Glu Asn Glu Leu Met Lys Arg Ala Arg Ala Lys Ala Ile
                165                 170                 175
Leu Glu Gln Gln Ala Arg Trp Lys His His Asn His Lys Gln Gln Asp
            180                 185                 190
Asn Leu His Asn Pro Asn Ile Asn Ile Gly Asn Tyr Gln Thr Arg Asn
        195                 200                 205
Asn Glu Gly Gly Val Glu Pro Ala Thr Asp Val Gln Val Arg Val Val
    210                 215                 220
Arg Asn Leu Leu Pro His Trp Met Leu
225                 230

<210>  172
<211>  744
<212>  DNA
<213>  Dendrobium grex Madame Thong-IN

<400>  172
atgggtcgtg gcagggtgca gctgaagcga atcgagaata aaataaaccg gcaggtgacg      60
ttctcgaagc ggagatctgg tttgcttaag aaggcgcacg agatctccgt gctctgtgac     120
gctgaagttg ctctgatcgt tttttccaat aagggaaagc tttatgagta ttccaccgat     180
tccagcatgg agaaaattct tgaacggtat gagcgttatt catatgctga aagagcatta     240
ttttccaatg aggccaaccc ccaggctgat tggcgccttg aatataataa actgaaggca     300
agggttgaaa gcttacagaa gagccaaagg caccttatgg gggagcaact tgactccttg     360
agcattaaag aactccaacg tctagagcaa cagcttgaaa gttccttgaa gtttatacga     420
tccagaaaga cacagctcat actacattca atttccgagc tacaaaagat ggaaaaaata     480
ttgctggagc aaaacaagac cttagagaag gagattatag ctaaagagaa agccaaagct     540
ttggtgcagc atgccccatg ggagaagcaa aaccagtccc aatatagctc tgcactcccg     600
cctgtgattt cggattctgt cccaactccc accagcagaa cgtttcaagc cagagccaat     660
gaagaagaat cacctcagcc acagttaaga gtaagcaaca ctctgctgcc cccatggatg     720
ctcagtcata tgaatggaca ataa                                            744

<210>  173
<211>  247
<212>  PRT
<213>  Dendrobium grex Madame Thong-IN

<400>  173
Met Gly Arg Gly Arg Val Gln Leu Lys Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Val Phe
        35                  40                  45
Ser Asn Lys Gly Lys Leu Tyr Glu Tyr Ser Thr Asp Ser Ser Met Glu
    50                  55                  60
Lys Ile Leu Glu Arg Tyr Glu Arg Tyr Ser Tyr Ala Glu Arg Ala Leu
65                  70                  75                  80
Phe Ser Asn Glu Ala Asn Pro Gln Ala Asp Trp Arg Leu Glu Tyr Asn
                85                  90                  95
Lys Leu Lys Ala Arg Val Glu Ser Leu Gln Lys Ser Gln Arg His Leu
            100                 105                 110
Met Gly Glu Gln Leu Asp Ser Leu Ser Ile Lys Glu Leu Gln Arg Leu
        115                 120                 125
Glu Gln Gln Leu Glu Ser Ser Leu Lys Phe Ile Arg Ser Arg Lys Thr
    130                 135                 140
Gln Leu Ile Leu His Ser Ile Ser Glu Leu Gln Lys Met Glu Lys Ile
145                 150                 155                 160
Leu Leu Glu Gln Asn Lys Thr Leu Glu Lys Glu Ile Ile Ala Lys Glu
                165                 170                 175
Lys Ala Lys Ala Leu Val Gln His Ala Pro Trp Glu Lys Gln Asn Gln
            180                 185                 190
Ser Gln Tyr Ser Ser Ala Leu Pro Pro Val Ile Ser Asp Ser Val Pro
        195                 200                 205
Thr Pro Thr Ser Arg Thr Phe Gln Ala Arg Ala Asn Glu Glu Glu Ser
    210                 215                 220
Pro Gln Pro Gln Leu Arg Val Ser Asn Thr Leu Leu Pro Pro Trp Met
225                 230                 235                 240
Leu Ser His Met Asn Gly Gln
                245

<210>  174
<211>  2194
<212>  DNA
<213>  Oryza sativa

<400>  174
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                2194

<210>  175
<211>  1725
<212>  DNA
<213>  Arabidopsis thaliana

<400>  175
atgaattcta acaactggct tggctttcct ctttcaccga acaactcttc tttgcctcct      60
catgaataca accttggctt ggtcagcgac catatggaca acccttttca aacacaagag     120
tggaatatga tcaatccaca cggtggagga ggagatgaag gaggagaggt tccaaaagtg     180
gccgattttc tcggtgtgag caaaccggac gaaaaccaat ccaaccacct agtagcttac     240
aacgactcag actactactt ccataccaat agcttgatgc ctagcgtcca atcaaacgat     300
gtcgttgtag cagcttgtga ctccaatact cctaacaaca gtagctatca tgagcttcaa     360
gagagtgctc acaatctaca gtcacttact ttgtccatgg ggaccaccgc tggtaataat     420
gttgtagaca aagcttcacc atccgagacc accggggata acgctagcgg tggagcacta     480
gccgttgttg agacggccac gccaagacgt gcattggaca ctttcggaca acgaacctcg     540
atctatcgtg gtgtcacaag acatcgatgg actggtcgat atgaggctca tctatgggat     600
aatagttgta gaagggaagg ccagtctagg aaaggaagac aagtttactt gggtggatat     660
gataaagaag ataaagcagc aagatcatat gatctagctg cacttaagta ctggggtccc     720
tcaactacta ctaatttccc cattacaaac tacgagaaag aagtagagga aatgaagcac     780

atgacgaggc aagagttcgt ggctgccatt agaaggaaaa gtagtggatt ttcgagaggc 840
gcttcgatgt atcgaggagt tacaaggcac caccaacatg gaagatggca agcaaggatc 900
ggccgagtcg ccgggaacaa agacctctac ttgggaactt ttagcactga ggaagaagca 960
gcagaagctt acgatatagc tgcaataaag tttagaggac ttaatgcagt gaccaacttc 1020
gagatcaacc ggtacgacgt gaaagccatt ctagagagta gcactcttcc catcggagga 1080
ggcgcagcta aacggctcaa agaagctcaa gctcttgagt cttcaaggaa acgcgaggcg 1140
gagatgatag cccttggttc aagtttccag tacggtggtg gctcgagcac aggctctggc 1200
tccacctcat caagacttca gcttcaacct taccctctaa gcattcaaca accattagag 1260
ccttttctat ctcttcagaa caatgacatc tctcattaca acaacaacaa tgctcacgat 1320
tcctcctctt ttaatcacca tagctatatc cagacacaac ttcatctcca ccaacagacc 1380
aacaattact tgcagcaaca gtcgagccag aactctcagc agctctacaa tgcgtatctt 1440
catagcaatc cggctctgct tcatggactt gtctctacct ctatcgttga caacaataat 1500
aacaatggag gctctagtgg gagctacaac actgcagcat ttcttgggaa ccacggtatt 1560
ggtattgggt ccagctcgac tgttggatcg accgaggagt ttccaaccgt taaaacagat 1620
tacgatatgc cttccagtga tggaaccgga gggtatagtg gttggaccag tgagtctgtt 1680
caggggtcaa accctggtgg tgttttcact atgtggaatg agtaa 1725

<210> 176
<211> 574
<212> PRT
<213> Arabidopsis thaliana

<400> 176
Met Asn Ser Asn Asn Trp Leu Gly Phe Pro Leu Ser Pro Asn Asn Ser
1               5                   10                  15
Ser Leu Pro Pro His Glu Tyr Asn Leu Gly Leu Val Ser Asp His Met
            20                  25                  30
Asp Asn Pro Phe Gln Thr Gln Glu Trp Asn Met Ile Asn Pro His Gly
        35                  40                  45
Gly Gly Gly Asp Glu Gly Gly Glu Val Pro Lys Val Ala Asp Phe Leu
    50                  55                  60
Gly Val Ser Lys Pro Asp Glu Asn Gln Ser Asn His Leu Val Ala Tyr
65                  70                  75                  80
Asn Asp Ser Asp Tyr Tyr Phe His Thr Asn Ser Leu Met Pro Ser Val
                85                  90                  95
Gln Ser Asn Asp Val Val Val Ala Ala Cys Asp Ser Asn Thr Pro Asn
            100                 105                 110
Asn Ser Ser Tyr His Glu Leu Gln Glu Ser Ala His Asn Leu Gln Ser
        115                 120                 125
Leu Thr Leu Ser Met Gly Thr Thr Ala Gly Asn Asn Val Val Asp Lys
    130                 135                 140
Ala Ser Pro Ser Glu Thr Thr Gly Asp Asn Ala Ser Gly Gly Ala Leu
145                 150                 155                 160
Ala Val Val Glu Thr Ala Thr Pro Arg Arg Ala Leu Asp Thr Phe Gly
                165                 170                 175
Gln Arg Thr Ser Ile Tyr Arg Gly Val Thr Arg His Arg Trp Thr Gly
            180                 185                 190
Arg Tyr Glu Ala His Leu Trp Asp Asn Ser Cys Arg Arg Glu Gly Gln
        195                 200                 205
Ser Arg Lys Gly Arg Gln Val Tyr Leu Gly Gly Tyr Asp Lys Glu Asp
    210                 215                 220
Lys Ala Ala Arg Ser Tyr Asp Leu Ala Ala Leu Lys Tyr Trp Gly Pro
225                 230                 235                 240
Ser Thr Thr Thr Asn Phe Pro Ile Thr Asn Tyr Glu Lys Glu Val Glu
                245                 250                 255
Glu Met Lys His Met Thr Arg Gln Glu Phe Val Ala Ala Ile Arg Arg
            260                 265                 270
Lys Ser Ser Gly Phe Ser Arg Gly Ala Ser Met Tyr Arg Gly Val Thr
        275                 280                 285

Arg His His Gln His Gly Arg Trp Gln Ala Arg Ile Gly Arg Val Ala
    290                 295                 300
Gly Asn Lys Asp Leu Tyr Leu Gly Thr Phe Ser Thr Glu Glu Glu Ala
305                 310                 315                 320
Ala Glu Ala Tyr Asp Ile Ala Ala Ile Lys Phe Arg Gly Leu Asn Ala
                325                 330                 335
Val Thr Asn Phe Glu Ile Asn Arg Tyr Asp Val Lys Ala Ile Leu Glu
            340                 345                 350
Ser Ser Thr Leu Pro Ile Gly Gly Gly Ala Ala Lys Arg Leu Lys Glu
        355                 360                 365
Ala Gln Ala Leu Glu Ser Ser Arg Lys Arg Glu Ala Glu Met Ile Ala
    370                 375                 380
Leu Gly Ser Ser Phe Gln Tyr Gly Gly Gly Ser Ser Thr Gly Ser Gly
385                 390                 395                 400
Ser Thr Ser Ser Arg Leu Gln Leu Gln Pro Tyr Pro Leu Ser Ile Gln
                405                 410                 415
Gln Pro Leu Glu Pro Phe Leu Ser Leu Gln Asn Asn Asp Ile Ser His
            420                 425                 430
Tyr Asn Asn Asn Asn Ala His Asp Ser Ser Ser Phe Asn His His Ser
        435                 440                 445
Tyr Ile Gln Thr Gln Leu His Leu His Gln Gln Thr Asn Asn Tyr Leu
    450                 455                 460
Gln Gln Gln Ser Ser Gln Asn Ser Gln Gln Leu Tyr Asn Ala Tyr Leu
465                 470                 475                 480
His Ser Asn Pro Ala Leu Leu His Gly Leu Val Ser Thr Ser Ile Val
                485                 490                 495
Asp Asn Asn Asn Asn Asn Gly Gly Ser Ser Gly Ser Tyr Asn Thr Ala
            500                 505                 510
Ala Phe Leu Gly Asn His Gly Ile Gly Ile Gly Ser Ser Ser Thr Val
        515                 520                 525
Gly Ser Thr Glu Glu Phe Pro Thr Val Lys Thr Asp Tyr Asp Met Pro
    530                 535                 540
Ser Ser Asp Gly Thr Gly Gly Tyr Ser Gly Trp Thr Ser Glu Ser Val
545                 550                 555                 560
Gln Gly Ser Asn Pro Gly Gly Val Phe Thr Met Trp Asn Glu
                565                 570

<210>  177
<211>  1707
<212>  DNA
<213>  Arabidopsis thaliana

<400>  177
atgaattcta acaactggct cgcgttccct ctatcaccaa ctcactcttc tttgccgcct      60
cacattcact cttcacaaaa ttctcatttc aatctaggtt tggtcaacga caatatcgac     120
aacccttttc aaaaccaagg atggaatatg atcaatccac atggtggagg cggcgaaggt     180
ggagaggttc caaaagtggc tgatttctta ggagtgagca aatcggggga tcatcacacc     240
gatcacaacc tcgtacctta taacgacatt catcaaacca acgcctccga ctactacttt     300
caaaccaata gcttgttacc tacagtcgtc acttgtgcct ctaatgctcc taataattat     360
gagcttcaag agagtgcaca caatttgcaa tctctcactc tctctatggg aagtactgga     420
gctgccgctg cagaagtcgc cactgtgaaa gcctcgccgg ctgagactag tgccgataat     480
agtagcagca ctaccaacac aagcggagga gccatcgttg aggctacacc gagacggact     540
ttggaaactt ttggacaacg aacctctatc tatcgtggag ttacaagaca tagatggacc     600
ggtagatatg aagctcatct ttgggataat agctgtagaa gagaaggaca atcaaggaaa     660
ggaagacaag tctacttagg tgggtatgac aaagaagaga aagcagccag agcatatgat     720
ctagctgcac ttaaatattg gggtccctct actactacca actttccgat aactaactac     780
gagaaggaag tagaggagat gaaaaacatg acgagacaag agtttgtggc ttctataaga     840
aggaagagta gcggattctc gcgtggtgca tccatgtatc gtggagtaac aaggcatcat     900
caacatggaa gatggcaagc aaggatcggc cgagttgctg gaaacaaaga tctctacttg     960

ggaacattca gcacggagga agaagcagca gaagcttatg acatagctgc gataaagttt 1020
cgaggtctaa acgcggttac aaactttgag ataaatcggt atgatgtgaa agccatcctg 1080
gagagcaaca cacttcctat aggaggtggt gcggctaaac ggctcaaaga agctcaagct 1140
ctagaatcat caagaaaacg agaggaaatg atagccctcg gatcaaattt ccatcaatat 1200
ggtgcagcga gcggctcgag ctctgttgct tccagctcta ggcttcagct tcaaccttac 1260
cctctaagca ttcaacaacc ttttgagcat cttcatcatc atcagccttt acttactcta 1320
cagaacaaca acgatatctc tcagtatcat gattccttta gttacattca gacgcagctt 1380
catcttcacc aacaacaaac caacaattac ttgcagtctt ctagtcacac ttcacagctc 1440
tacaatgctt atcttcagag taaccctggt ctgcttcatg gatttgtctc tgataataac 1500
aacacttcag ggtttcttgg aaacaatggg attggtattg ggtcaagctc taccgttgga 1560
tcatcggctg aggaagagtt tccagccgtg aaagtcgatt acgatatgcc tccttccggt 1620
ggagctacag ggtatggagg atggaatagt ggagagtctg ctcaaggatc gaatccagga 1680
ggtgttttca cgatgtggaa tgaataa 1707

<210> 178
<211> 568
<212> PRT
<213> Arabidopsis thaliana

<400> 178
   Met Asn Ser Asn Asn Trp Leu Ala Phe Pro Leu Ser Pro Thr His Ser
   1 5 10 15
   Ser Leu Pro Pro His Ile His Ser Ser Gln Asn Ser His Phe Asn Leu
   20 25 30
   Gly Leu Val Asn Asp Asn Ile Asp Asn Pro Phe Gln Asn Gln Gly Trp
   35 40 45
   Asn Met Ile Asn Pro His Gly Gly Gly Gly Glu Gly Gly Glu Val Pro
   50 55 60
   Lys Val Ala Asp Phe Leu Gly Val Ser Lys Ser Gly Asp His His Thr
   65 70 75 80
   Asp His Asn Leu Val Pro Tyr Asn Asp Ile His Gln Thr Asn Ala Ser
   85 90 95
   Asp Tyr Tyr Phe Gln Thr Asn Ser Leu Leu Pro Thr Val Val Thr Cys
   100 105 110
   Ala Ser Asn Ala Pro Asn Asn Tyr Glu Leu Gln Glu Ser Ala His Asn
   115 120 125
   Leu Gln Ser Leu Thr Leu Ser Met Gly Ser Thr Gly Ala Ala Ala Ala
   130 135 140
   Glu Val Ala Thr Val Lys Ala Ser Pro Ala Glu Thr Ser Ala Asp Asn
   145 150 155 160
   Ser Ser Ser Thr Thr Asn Thr Ser Gly Gly Ala Ile Val Glu Ala Thr
   165 170 175
   Pro Arg Arg Thr Leu Glu Thr Phe Gly Gln Arg Thr Ser Ile Tyr Arg
   180 185 190
   Gly Val Thr Arg His Arg Trp Thr Gly Arg Tyr Glu Ala His Leu Trp
   195 200 205
   Asp Asn Ser Cys Arg Arg Glu Gly Gln Ser Arg Lys Gly Arg Gln Val
   210 215 220
   Tyr Leu Gly Gly Tyr Asp Lys Glu Glu Lys Ala Ala Arg Ala Tyr Asp
   225 230 235 240
   Leu Ala Ala Leu Lys Tyr Trp Gly Pro Ser Thr Thr Thr Asn Phe Pro
   245 250 255
   Ile Thr Asn Tyr Glu Lys Glu Val Glu Glu Met Lys Asn Met Thr Arg
   260 265 270
   Gln Glu Phe Val Ala Ser Ile Arg Arg Lys Ser Ser Gly Phe Ser Arg
   275 280 285
   Gly Ala Ser Met Tyr Arg Gly Val Thr Arg His His Gln His Gly Arg
   290 295 300
   Trp Gln Ala Arg Ile Gly Arg Val Ala Gly Asn Lys Asp Leu Tyr Leu
   305                 310                 315                 320
   Gly Thr Phe Ser Thr Glu Glu Glu Ala Ala Glu Ala Tyr Asp Ile Ala
                   325                 330                 335
   Ala Ile Lys Phe Arg Gly Leu Asn Ala Val Thr Asn Phe Glu Ile Asn
               340                 345                 350
   Arg Tyr Asp Val Lys Ala Ile Leu Glu Ser Asn Thr Leu Pro Ile Gly
           355                 360                 365
   Gly Gly Ala Ala Lys Arg Leu Lys Glu Ala Gln Ala Leu Glu Ser Ser
       370                 375                 380
   Arg Lys Arg Glu Glu Met Ile Ala Leu Gly Ser Asn Phe His Gln Tyr
   385                 390                 395                 400
   Gly Ala Ala Ser Gly Ser Ser Ser Val Ala Ser Ser Ser Arg Leu Gln
                   405                 410                 415
   Leu Gln Pro Tyr Pro Leu Ser Ile Gln Gln Pro Phe Glu His Leu His
               420                 425                 430
   His His Gln Pro Leu Leu Thr Leu Gln Asn Asn Asn Asp Ile Ser Gln
           435                 440                 445
   Tyr His Asp Ser Phe Ser Tyr Ile Gln Thr Gln Leu His Leu His Gln
       450                 455                 460
   Gln Gln Thr Asn Asn Tyr Leu Gln Ser Ser Ser His Thr Ser Gln Leu
   465                 470                 475                 480
   Tyr Asn Ala Tyr Leu Gln Ser Asn Pro Gly Leu Leu His Gly Phe Val
                   485                 490                 495
   Ser Asp Asn Asn Asn Thr Ser Gly Phe Leu Gly Asn Asn Gly Ile Gly
               500                 505                 510
   Ile Gly Ser Ser Ser Thr Val Gly Ser Ser Ala Glu Glu Glu Phe Pro
           515                 520                 525
   Ala Val Lys Val Asp Tyr Asp Met Pro Pro Ser Gly Gly Ala Thr Gly
       530                 535                 540
   Tyr Gly Gly Trp Asn Ser Gly Glu Ser Ala Gln Gly Ser Asn Pro Gly
   545                 550                 555                 560
   Gly Val Phe Thr Met Trp Asn Glu
                   565

<210>  179
<211>  1662
<212>  DNA
<213>  Glycine max

<400>  179
atgaacaaca actggctttc gttccctctt tctcctactc attcttcctt accagctcat      60
gatcttcaag caactcaata tcatcaattt tcccttgggt tagtgaacga gaacatggat     120
aaccctttcc aaaatcatga ttggaatctg attaacaccc atagtagcaa cgaaattcca     180
aaagtggctg attttctagg agtgagcaag tctgaaaatc agtcagacct tgcagcctta     240
aacgaaattc attcaaatga ttcagattat ctgttcacaa acaacagtct ggtgcctatg     300
caaaaccctg tgttggacac acctagcaat gagtatcaag aaaatgctaa tagtaatttg     360
caatcattga cattatccat gggaagtggt aaggattcaa catgtgaaac cagtggtgaa     420
aatagcacaa acactactgt tgaagttgca cctagaagaa ctttggatac attcgggcag     480
agaacatcca tatatcgtgg agtaactcga catagatgga ctggaaggta tgaagctcat     540
ctttgggata atagctgtag aagggaaggc caatcaagaa aaggacgcca agtttatttg     600
ggtggatatg ataaagaaga gaaagcagct agagcttatg atttagctgc actgaagtac     660
tgggggacat ccaccactac caactttcca attagcaact atgagaagga attggatgaa     720
atgaaacaca tgacgagaca agaatttgtt gccgccatta gaaggaaaag cagtggtttc     780
tccaggggtg catcaatgta tcgtggagtt acaaggcatc accaacacgg aagatggcaa     840
gcaaggattg gcagagttgc aggaaacaaa gatctttact tgggaacttt cagtactgag     900
gaagaggctg cagaagcata cgacatagca gcgataaagt tcagaggtct caacgctgtc     960
acaaactttg acatgagccg ctacgacgtg aaagccattc ttgaaagcaa cactctccca    1020
ataggaggag gcgctgcaaa gcgtctgaaa gaagctcaag ctctagaatc ttcgagaaaa    1080
cgcgaagaga tgattgcact aggctcatct tccacgttcc aatacggaac ctcagcaagc    1140

tcttctaggc ttcacgctta ccctctaatg cagcaccacc accagttcga gcaacctcaa 1200
cctctgctaa ctcttcaaaa ccacgacata agttcttctc acttctctca ccagcaagac 1260
cctttgcatc atcaggttta catccaaacg cagcttcagt tgcaccagca gagtggcgct 1320
tcttcttata gctttcagaa taatgctcag ttctacaatg gttaccttca gaaccaccct 1380
gcattgcttc agggaatgat gaacatgggg tcttcttctt cttcctcatc tgtgttggag 1440
aataataata gtaacaataa taataataat gttggtgggt ttgtgggaag tgggtttggt 1500
atggcttcga atgcaacggc ggggaacacg gtggggacag cggaggagtt agggctggtg 1560
aaggtggact atgacatgcc ggctggaggt tacggtggct ggtcggcggc ggactccatg 1620
cagacgtcaa atggtggggt gttcacaatg tggaatgatt aa 1662

<210> 180
<211> 553
<212> PRT
<213> Glycine max

<400> 180
Met Asn Asn Asn Trp Leu Ser Phe Pro Leu Ser Pro Thr His Ser Ser
1               5                   10                  15
Leu Pro Ala His Asp Leu Gln Ala Thr Gln Tyr His Gln Phe Ser Leu
            20                  25                  30
Gly Leu Val Asn Glu Asn Met Asp Asn Pro Phe Gln Asn His Asp Trp
        35                  40                  45
Asn Leu Ile Asn Thr His Ser Ser Asn Glu Ile Pro Lys Val Ala Asp
    50                  55                  60
Phe Leu Gly Val Ser Lys Ser Glu Asn Gln Ser Asp Leu Ala Ala Leu
65                  70                  75                  80
Asn Glu Ile His Ser Asn Asp Ser Asp Tyr Leu Phe Thr Asn Asn Ser
                85                  90                  95
Leu Val Pro Met Gln Asn Pro Val Leu Asp Thr Pro Ser Asn Glu Tyr
            100                 105                 110
Gln Glu Asn Ala Asn Ser Asn Leu Gln Ser Leu Thr Leu Ser Met Gly
        115                 120                 125
Ser Gly Lys Asp Ser Thr Cys Glu Thr Ser Gly Glu Asn Ser Thr Asn
    130                 135                 140
Thr Thr Val Glu Val Ala Pro Arg Arg Thr Leu Asp Thr Phe Gly Gln
145                 150                 155                 160
Arg Thr Ser Ile Tyr Arg Gly Val Thr Arg His Arg Trp Thr Gly Arg
                165                 170                 175
Tyr Glu Ala His Leu Trp Asp Asn Ser Cys Arg Arg Glu Gly Gln Ser
            180                 185                 190
Arg Lys Gly Arg Gln Val Tyr Leu Gly Gly Tyr Asp Lys Glu Glu Lys
        195                 200                 205
Ala Ala Arg Ala Tyr Asp Leu Ala Ala Leu Lys Tyr Trp Gly Thr Ser
    210                 215                 220
Thr Thr Thr Asn Phe Pro Ile Ser Asn Tyr Glu Lys Glu Leu Asp Glu
225                 230                 235                 240
Met Lys His Met Thr Arg Gln Glu Phe Val Ala Ala Ile Arg Arg Lys
                245                 250                 255
Ser Ser Gly Phe Ser Arg Gly Ala Ser Met Tyr Arg Gly Val Thr Arg
            260                 265                 270
His His Gln His Gly Arg Trp Gln Ala Arg Ile Gly Arg Val Ala Gly
        275                 280                 285
Asn Lys Asp Leu Tyr Leu Gly Thr Phe Ser Thr Glu Glu Glu Ala Ala
    290                 295                 300
Glu Ala Tyr Asp Ile Ala Ala Ile Lys Phe Arg Gly Leu Asn Ala Val
305                 310                 315                 320
Thr Asn Phe Asp Met Ser Arg Tyr Asp Val Lys Ala Ile Leu Glu Ser
                325                 330                 335
Asn Thr Leu Pro Ile Gly Gly Gly Ala Ala Lys Arg Leu Lys Glu Ala

            340                 345                 350
Gln Ala Leu Glu Ser Ser Arg Lys Arg Glu Glu Met Ile Ala Leu Gly
        355                 360                 365
Ser Ser Ser Thr Phe Gln Tyr Gly Thr Ser Ala Ser Ser Ser Arg Leu
    370                 375                 380
His Ala Tyr Pro Leu Met Gln His His His Gln Phe Glu Gln Pro Gln
385                 390                 395                 400
Pro Leu Leu Thr Leu Gln Asn His Asp Ile Ser Ser Ser His Phe Ser
                405                 410                 415
His Gln Gln Asp Pro Leu His His Gln Gly Tyr Ile Gln Thr Gln Leu
            420                 425                 430
Gln Leu His Gln Gln Ser Gly Ala Ser Ser Tyr Ser Phe Gln Asn Asn
        435                 440                 445
Ala Gln Phe Tyr Asn Gly Tyr Leu Gln Asn His Pro Ala Leu Leu Gln
    450                 455                 460
Gly Met Met Asn Met Gly Ser Ser Ser Ser Ser Ser Ser Val Leu Glu
465                 470                 475                 480
Asn Asn Asn Ser Asn Asn Asn Asn Asn Asn Val Gly Gly Phe Val Gly
                485                 490                 495
Ser Gly Phe Gly Met Ala Ser Asn Ala Thr Ala Gly Asn Thr Val Gly
            500                 505                 510
Thr Ala Glu Glu Leu Gly Leu Val Lys Val Asp Tyr Asp Met Pro Ala
        515                 520                 525
Gly Gly Tyr Gly Gly Trp Ser Ala Ala Asp Ser Met Gln Thr Ser Asn
    530                 535                 540
Gly Gly Val Phe Thr Met Trp Asn Asp
545                 550

<210> 181
<211> 1674
<212> DNA
<213> Glycine max

<400> 181
atgaacaaca actggctttc gttccctctt tctcctactc attcttcctt accagctcat 60
gatcttcaag caactcaata tcatcaattt tcccttgggt tagtgaacga gaacatggat 120
aaccctttcc aaaatcatga ttggaatctg attaacaccc atagtagcaa cgaaattcca 180
aaagtggctg attttctagg agtgagcaag tctgaaaatc agtcagacct tgcagcctta 240
aacgaaattc attcaaatga ttcagattat ctgttcacaa acaacagtct ggtgcctatg 300
caaaaccctg tgttggacac acctagcaat gagtatcaag aaaatgctaa tagtaatttg 360
caatcattga cattatccat gggaagtggt aaggattcaa catgtgaaac cagtggtgaa 420
aatagcacaa acactactgt tgaagttgca cctagaagaa ctttggatac attcgggcag 480
agaacatcca tatatcgtgg agtaactcga catagatgga ctggaaggta tgaagctcat 540
ctttgggata atagctgtag aagggaaggc caatcaagaa aaggacgcca agtttatttg 600
ggtggatatg ataaagaaga gaaagcagct agggcttatg atttagctgc actgaagtac 660
tgggggacat ccaccactac caactttcca attagtaact atgagaagga attggatgaa 720
atgaaacaca tgacgcgaca agaatttgtt gctgccatta gaaggaaaag cagtggtttc 780
tccaggggtg catcaatgta tcgtggagtt acaaggcatc accaacacgg aagatggcaa 840
gcaagaattg gcagagttgc aggaaacaaa gatctttact tgggaacttt cagtactgaa 900
gaagaggctg ctgaagcata cgacatagct gcgataaagt tcagaggtct caacgctgtc 960
acaaactttg acatgagccg ctacgacgtg aaagccatcc ttgaaagcaa cactctccca 1020
ataggaggag gagctgcaaa gcgtctgaaa gaagctcaag ctctagaatc ttcgagaaag 1080
cgcgaagaga tgattgcact aggatcatcc acattccaat atggaaccac aagctctaat 1140
tctaggctac atgcttaccc tctaatgcag caccaccacc agtttgaaca acctcaacct 1200
ctgctaactt tgcaaaacca tgatatcagt tctcacttct ctcaccagca agaccctttg 1260
catcagggtt acatccaaac gcagcttcag ttgcaccagc agcagagtgg tggttcttct 1320
tcttatagct ttcagaataa taatataaat aatgctcagt tctataatgg ttataatctt 1380
cagaaccacc ctgcattgct tcagggaatg attaacatgg ggtcttcatc ttcttcatct 1440
gtgttggaga ataataatag taccaataat aatgttggtg ggtttgtggg aagtgggttt 1500

ggtatggctt ctaatgcaac gtcggggaac acggtgggga cggcggagga gctagggctg 1560
gtgaaggtgg actatgacat gccgactggt ggttacggtg gatggtcggc ggcggcggcg 1620
gcggagtcca tgcagacgtc gaatagtggg gtgttcacaa tgtggaatga ctga 1674

<210> 182
<211> 557
<212> PRT
<213> Glycine max

<400> 182
Met Asn Asn Asn Trp Leu Ser Phe Pro Leu Ser Pro Thr His Ser Ser
1               5                   10                  15
Leu Pro Ala His Asp Leu Gln Ala Thr Gln Tyr His Gln Phe Ser Leu
            20                  25                  30
Gly Leu Val Asn Glu Asn Met Asp Asn Pro Phe Gln Asn His Asp Trp
        35                  40                  45
Asn Leu Ile Asn Thr His Ser Ser Asn Glu Ile Pro Lys Val Ala Asp
    50                  55                  60
Phe Leu Gly Val Ser Lys Ser Glu Asn Gln Ser Asp Leu Ala Ala Leu
65                  70                  75                  80
Asn Glu Ile His Ser Asn Asp Ser Asp Tyr Leu Phe Thr Asn Asn Ser
                85                  90                  95
Leu Val Pro Met Gln Asn Pro Val Leu Asp Thr Pro Ser Asn Glu Tyr
            100                 105                 110
Gln Glu Asn Ala Asn Ser Asn Leu Gln Ser Leu Thr Leu Ser Met Gly
        115                 120                 125
Ser Gly Lys Asp Ser Thr Cys Glu Thr Ser Gly Glu Asn Ser Thr Asn
    130                 135                 140
Thr Thr Val Glu Val Ala Pro Arg Arg Thr Leu Asp Thr Phe Gly Gln
145                 150                 155                 160
Arg Thr Ser Ile Tyr Arg Gly Val Thr Arg His Arg Trp Thr Gly Arg
                165                 170                 175
Tyr Glu Ala His Leu Trp Asp Asn Ser Cys Arg Arg Glu Gly Gln Ser
            180                 185                 190
Arg Lys Gly Arg Gln Val Tyr Leu Gly Gly Tyr Asp Lys Glu Glu Lys
        195                 200                 205
Ala Ala Arg Ala Tyr Asp Leu Ala Ala Leu Lys Tyr Trp Gly Thr Ser
    210                 215                 220
Thr Thr Thr Asn Phe Pro Ile Ser Asn Tyr Glu Lys Glu Leu Asp Glu
225                 230                 235                 240
Met Lys His Met Thr Arg Gln Glu Phe Val Ala Ala Ile Arg Arg Lys
                245                 250                 255
Ser Ser Gly Phe Ser Arg Gly Ala Ser Met Tyr Arg Gly Val Thr Arg
            260                 265                 270
His His Gln His Gly Arg Trp Gln Ala Arg Ile Gly Arg Val Ala Gly
        275                 280                 285
Asn Lys Asp Leu Tyr Leu Gly Thr Phe Ser Thr Glu Glu Glu Ala Ala
    290                 295                 300
Glu Ala Tyr Asp Ile Ala Ala Ile Lys Phe Arg Gly Leu Asn Ala Val
305                 310                 315                 320
Thr Asn Phe Asp Met Ser Arg Tyr Asp Val Lys Ala Ile Leu Glu Ser
                325                 330                 335
Asn Thr Leu Pro Ile Gly Gly Gly Ala Ala Lys Arg Leu Lys Glu Ala
            340                 345                 350
Gln Ala Leu Glu Ser Ser Arg Lys Arg Glu Glu Met Ile Ala Leu Gly
        355                 360                 365
Ser Ser Thr Phe Gln Tyr Gly Thr Thr Ser Ser Asn Ser Arg Leu His
    370                 375                 380
Ala Tyr Pro Leu Met Gln His His His Gln Phe Glu Gln Pro Gln Pro

385                 390                 395                 400
Leu Leu Thr Leu Gln Asn His Asp Ile Ser Ser His Phe Ser His Gln
                405                 410                 415
Gln Asp Pro Leu His Gln Gly Tyr Ile Gln Thr Gln Leu Gln Leu His
            420                 425                 430
Gln Gln Gln Ser Gly Gly Ser Ser Ser Tyr Ser Phe Gln Asn Asn Asn
        435                 440                 445
Ile Asn Asn Ala Gln Phe Tyr Asn Gly Tyr Asn Leu Gln Asn His Pro
    450                 455                 460
Ala Leu Leu Gln Gly Met Ile Asn Met Gly Ser Ser Ser Ser Ser Ser
465                 470                 475                 480
Val Leu Glu Asn Asn Asn Ser Thr Asn Asn Asn Val Gly Gly Phe Val
                485                 490                 495
Gly Ser Gly Phe Gly Met Ala Ser Asn Ala Thr Ser Gly Asn Thr Val
            500                 505                 510
Gly Thr Ala Glu Glu Leu Gly Leu Val Lys Val Asp Tyr Asp Met Pro
        515                 520                 525
Thr Gly Gly Tyr Gly Gly Trp Ser Ala Ala Ala Ala Ala Glu Ser Met
    530                 535                 540
Gln Thr Ser Asn Ser Gly Val Phe Thr Met Trp Asn Asp
545                 550                 555

<210>  183
<211>  1632
<212>  DNA
<213>  Medicago truncatula

<400>  183
atgaacaata actggctttc attccctctc tcaccttctc attcttcctt accttctaat      60
gatcttcaag caactcaata tcatcacttt cctcttggat tagtcaatga caacatggaa     120
aaccctttcc aaaatcatga ttggaatctg atgaacacac acaacagcaa tgaagttcca     180
aaggttgcgg attttctcgg tgtatgcaag tctgaaaatc actcagatct tgctacaccg     240
aacgaaattc aatctaatga ttcagattat ctgtttacaa ataacaatac tctcatgcca     300
atgcaaaacc aaatggttac aacatgcacc aatgagtatc aagaaaaggc tagtaatagt     360
aatttgcagt ctttgacatt atccatggga agtggtaaag attcaacatg tgaaactagt     420
ggtgaaaata gtacaaacac tgttgaagtt gctgttccta aaagaacttc agagacattt     480
ggacaaagaa cttcgatata tcgcggtgta acaaaacata gatggactgg aaggtatgaa     540
gctcaccttt gggataacag ctgtagaagg gaaggtcagt cgagaaaagg ccgccaaggt     600
ggatatgata aagaagagaa agctgctagg tcttatgatt tagctgcact taagtactgg     660
gggacatcca ccactaccaa ctttccagtt agcaactatg agaaggaaat agatgaaatg     720
aagcacatga caagacaaga atttgttgcc tctattagaa ggaaaagcag cggtttctct     780
aggggtgcat caatgtaccg tggagttaca aggcatcacc aacatggaag atggcaagca     840
aggattggca gagttgcagg aaataaagat ctatacttgg gaactttcag cactgaagaa     900
gaggctgcag aagcatacga catagcagca ataaaattca gaggactcaa cgctgtaaca     960
aactttgaca tgactcgtta cgacgtgaaa gccattctcg aaagcaacac actgccaatt    1020
ggaggaggag ctgcaaaaag actaaaagaa gcacaagctc tagaaacttc gagaaaacgc    1080
gaagaaatgc ttgcacttaa ctcatcatct ttccaatatg gaacatcaag ctctagtaac    1140
actagactcc aaccctaccc tctcatgcaa tatcatcacc aatttgaaca acctcaacca    1200
ttgctaacat tacaaaacaa ccatgaaagc ttgaattctc aacaattctc tcaacaccaa    1260
ggtggtggtt atttccaaac acagcttgag ttgtgtcaac aacaaaacca acaaccatct    1320
cagaatagta acataggttc attctacaat ggttattatc agaatcatcc tggtttgttt    1380
cagatgaata atataggatc ttcttcttca tcttcggtga tgggaaataa tggtggtggt    1440
tctagtggga tttatagtaa tagtggaggg ttaattagta ataatgctgt tgaggaattt    1500
gtgccggtta aggttgatta tgacatgcaa ggtgatggaa gtggttttgg cggctggtcg    1560
gcggcaggag agaacatgca gactgctgat ttgtttacaa tgtggaatga ctatgagaca    1620
agagagaatt ag                                                        1632

<210>  184
<211>  557

<212>  PRT
<213>  Medicago truncatulata

<400>  184
Met Asn Asn Asn Trp Leu Ser Phe Pro Leu Ser Pro Thr His Ser Ser
1               5                   10                  15
Leu Pro Ala His Asp Leu Gln Ala Thr Gln Tyr His Gln Phe Ser Leu
            20                  25                  30
Gly Leu Val Asn Glu Asn Met Asp Asn Pro Phe Gln Asn His Asp Trp
        35                  40                  45
Asn Leu Ile Asn Thr His Ser Ser Asn Glu Ile Pro Lys Val Ala Asp
    50                  55                  60
Phe Leu Gly Val Ser Lys Ser Glu Asn Gln Ser Asp Leu Ala Ala Leu
65                  70                  75                  80
Asn Glu Ile His Ser Asn Asp Ser Asp Tyr Leu Phe Thr Asn Asn Ser
                85                  90                  95
Leu Val Pro Met Gln Asn Pro Val Leu Asp Thr Pro Ser Asn Glu Tyr
            100                 105                 110
Gln Glu Asn Ala Asn Ser Asn Leu Gln Ser Leu Thr Leu Ser Met Gly
        115                 120                 125
Ser Gly Lys Asp Ser Thr Cys Glu Thr Ser Gly Glu Asn Ser Thr Asn
    130                 135                 140
Thr Thr Val Glu Val Ala Pro Arg Arg Thr Leu Asp Thr Phe Gly Gln
145                 150                 155                 160
Arg Thr Ser Ile Tyr Arg Gly Val Thr Arg His Arg Trp Thr Gly Arg
                165                 170                 175
Tyr Glu Ala His Leu Trp Asp Asn Ser Cys Arg Arg Glu Gly Gln Ser
            180                 185                 190
Arg Lys Gly Arg Gln Val Tyr Leu Gly Gly Tyr Asp Lys Glu Glu Lys
        195                 200                 205
Ala Ala Arg Ala Tyr Asp Leu Ala Ala Leu Lys Tyr Trp Gly Thr Ser
    210                 215                 220
Thr Thr Thr Asn Phe Pro Ile Ser Asn Tyr Glu Lys Glu Leu Asp Glu
225                 230                 235                 240
Met Lys His Met Thr Arg Gln Glu Phe Val Ala Ala Ile Arg Arg Lys
                245                 250                 255
Ser Ser Gly Phe Ser Arg Gly Ala Ser Met Tyr Arg Gly Val Thr Arg
            260                 265                 270
His His Gln His Gly Arg Trp Gln Ala Arg Ile Gly Arg Val Ala Gly
        275                 280                 285
Asn Lys Asp Leu Tyr Leu Gly Thr Phe Ser Thr Glu Glu Glu Ala Ala
    290                 295                 300
Glu Ala Tyr Asp Ile Ala Ala Ile Lys Phe Arg Gly Leu Asn Ala Val
305                 310                 315                 320
Thr Asn Phe Asp Met Ser Arg Tyr Asp Val Lys Ala Ile Leu Glu Ser
                325                 330                 335
Asn Thr Leu Pro Ile Gly Gly Gly Ala Ala Lys Arg Leu Lys Glu Ala
            340                 345                 350
Gln Ala Leu Glu Ser Ser Arg Lys Arg Glu Glu Met Ile Ala Leu Gly
        355                 360                 365
Ser Ser Thr Phe Gln Tyr Gly Thr Thr Ser Ser Asn Ser Arg Leu His
    370                 375                 380
Ala Tyr Pro Leu Met Gln His His His Gln Phe Glu Gln Pro Gln Pro
385                 390                 395                 400
Leu Leu Thr Leu Gln Asn His Asp Ile Ser Ser His Phe Ser His Gln
                405                 410                 415
Gln Asp Pro Leu His Gln Gly Tyr Ile Gln Thr Gln Leu Gln Leu His
            420                 425                 430
Gln Gln Gln Ser Gly Gly Ser Ser Ser Tyr Ser Phe Gln Asn Asn Asn

435                 440                 445
Ile Asn Asn Ala Gln Phe Tyr Asn Gly Tyr Asn Leu Gln Asn His Pro
    450                 455                 460
Ala Leu Leu Gln Gly Met Ile Asn Met Gly Ser Ser Ser Ser Ser Ser
465                 470                 475                 480
Val Leu Glu Asn Asn Asn Ser Thr Asn Asn Asn Val Gly Gly Phe Val
                485                 490                 495
Gly Ser Gly Phe Gly Met Ala Ser Asn Ala Thr Ser Gly Asn Thr Val
            500                 505                 510
Gly Thr Ala Glu Glu Leu Gly Leu Val Lys Val Asp Tyr Asp Met Pro
        515                 520                 525
Thr Gly Gly Tyr Gly Gly Trp Ser Ala Ala Ala Ala Ala Glu Ser Met
    530                 535                 540
Gln Thr Ser Asn Ser Gly Val Phe Thr Met Trp Asn Asp
545                 550                 555

<210>  185
<211>  2079
<212>  DNA
<213>  Oryza sativa

<400>  185
atggccacca tgaacaactg gctggccttc tccctctccc cgcaggatca gctcccgccg      60
tctcagacca actccactct catctccgcc gccgccacca ccaccaccgc cggcgactcc     120
tccaccggcg acgtctgctt caacatcccc caagattgga gcatgagggg atcggagctc     180
tcggcgctcg tcgccgagcc gaagctggag gacttcctcg gcggcatctc cttctcggag     240
cagcagcatc atcacggcgg caagggcggc gtgatcccga gcagcgccgc cgcttgctac      300
gcgagctccg gcagcagcgt cggctacctg taccctcctc caagctcatc ctcgctccag     360
ttcgccgact ccgtcatggt ggccacctcc tcgcccgtcg tcgcccacga cggcgtcagc     420
ggcggcggca tggtgagcgc cgccgccgcc gcggcggcca gtggcaacgg cggcattggc     480
ctgtccatga tcaagaactg gctccggagc cagccggcgc cgcagccggc gcaggcgctg     540
tctctgtcca tgaacatggc ggggacgacg acggcgcagg gcggcggcgc catggcgctc     600
ctcgccggcg caggggagcg aggccggacg acgcccgcgt cagagagcct gtccacgtcg     660
gcgcacggag cgacgacggc gacgatggct ggtggtcgca aggagattaa cgaggaaggc     720
agcggcagcg ccggcgccgt ggttgccgtc ggctcggagt caggcggcag cggcgccgtg     780
gtggaggccg gcgcggcggc ggcggcggcg aggaagtccg tcgacacgtt cggccagaga     840
acatcgatct accgcggcgt gacaaggcat agatggacag ggaggtatga ggctcatctt     900
tgggacaaca gctgcagaag agagggccaa actcgcaagg gtcgtcaagg tggttatgac     960
aaagaggaaa aagctgctag agcttatgat ttggctgctc tcaaatactg gggcccgacg     1020
acgacgacaa attttccggt aaataactat gaaaaggagc tggaggagat gaagcacatg    1080
acaaggcagg agttcgtagc ctctttgaga aggaagagca gtggtttctc cagaggtgca    1140
tccatttacc gtggagtaac taggcatcac cagcatggga gatggcaagc aaggatagga    1200
agagttgcag ggaacaagga cctctacttg ggcaccttca gcacgcagga ggaggcggcg    1260
gaggcgtacg acatcgcggc gatcaagttc cgggggctca acgccgtcac caacttcgac     1320
atgagccgct acgacgtcaa gagcatcctc gacagcgctg ccctccccgt cggcaccgcc    1380
gccaagcgcc tcaaggacgc cgaggccgcc gccgcctacg acgtcggccg catcgcctcg    1440
cacctcggcg gcgacggcgc ctacgccgcg cattacggcc accaccacca ctcggccgcc    1500
gccgcctggc cgaccatcgc gttccaggcg gcggcggcgc cgccgccgca cgccgccggg    1560
ctttaccacc cgtacgcgca gccgctgcgt gggtggtgca agcaggagca ggaccacgcc    1620
gtgatcgcgg cggcgcacag cctgcaggat ctccaccacc tcaacctcgg cgccgccgcc    1680
gccgcgcatg acttcttctc gcaggcgatg cagcagcagc acggcctcgg cagcatcgac    1740
aacgcgtcgc tcgagcacag caccggctcc aactccgtcg tctacaacgg cgacaatggc    1800
ggcggaggcg gcggctacat catggcgccg atgagcgccg tgtcggccac ggccaccgcg    1860
gtggcgagca gccacgatca cggcggcgac ggcgggaagc aggtgcagat ggggtacgac    1920
agctacctcg tcggcgcaga cgcctacggc ggcggcggcg ccgggaggat gccatcctgg    1980
gcgatgacgc cggcgtcggc gccggccgcc acgagcagca gcgacatgac cggagtctgc    2040
catggcgcac agctcttcag cgtctggaac gacacataa                           2079

<210>  186

<211>  692
<212>  PRT
<213>  Oryza sativa

<400>  186
   Met Ala Thr Met Asn Asn Trp Leu Ala Phe Ser Leu Ser Pro Gln Asp
   1               5                   10                  15
   Gln Leu Pro Pro Ser Gln Thr Asn Ser Thr Leu Ile Ser Ala Ala Ala
               20                  25                  30
   Thr Thr Thr Thr Ala Gly Asp Ser Ser Thr Gly Asp Val Cys Phe Asn
           35                  40                  45
   Ile Pro Gln Asp Trp Ser Met Arg Gly Ser Glu Leu Ser Ala Leu Val
       50                  55                  60
   Ala Glu Pro Lys Leu Glu Asp Phe Leu Gly Gly Ile Ser Phe Ser Glu
   65                  70                  75                  80
   Gln Gln His His His Gly Gly Lys Gly Gly Val Ile Pro Ser Ser Ala
                   85                  90                  95
   Ala Ala Cys Tyr Ala Ser Ser Gly Ser Ser Val Gly Tyr Leu Tyr Pro
               100                 105                 110
   Pro Pro Ser Ser Ser Ser Leu Gln Phe Ala Asp Ser Val Met Val Ala
           115                 120                 125
   Thr Ser Ser Pro Val Val Ala His Asp Gly Val Ser Gly Gly Gly Met
       130                 135                 140
   Val Ser Ala Ala Ala Ala Ala Ala Ala Ser Gly Asn Gly Gly Ile Gly
   145                 150                 155                 160
   Leu Ser Met Ile Lys Asn Trp Leu Arg Ser Gln Pro Ala Pro Gln Pro
                   165                 170                 175
   Ala Gln Ala Leu Ser Leu Ser Met Asn Met Ala Gly Thr Thr Thr Ala
               180                 185                 190
   Gln Gly Gly Gly Ala Met Ala Leu Leu Ala Gly Ala Gly Glu Arg Gly
           195                 200                 205
   Arg Thr Thr Pro Ala Ser Glu Ser Leu Ser Thr Ser Ala His Gly Ala
       210                 215                 220
   Thr Thr Ala Thr Met Ala Gly Gly Arg Lys Glu Ile Asn Glu Glu Gly
   225                 230                 235                 240
   Ser Gly Ser Ala Gly Ala Val Val Ala Val Gly Ser Glu Ser Gly Gly
                   245                 250                 255
   Ser Gly Ala Val Val Glu Ala Gly Ala Ala Ala Ala Ala Ala Arg Lys
               260                 265                 270
   Ser Val Asp Thr Phe Gly Gln Arg Thr Ser Ile Tyr Arg Gly Val Thr
           275                 280                 285
   Arg His Arg Trp Thr Gly Arg Tyr Glu Ala His Leu Trp Asp Asn Ser
       290                 295                 300
   Cys Arg Arg Glu Gly Gln Thr Arg Lys Gly Arg Gln Gly Gly Tyr Asp
   305                 310                 315                 320
   Lys Glu Glu Lys Ala Ala Arg Ala Tyr Asp Leu Ala Ala Leu Lys Tyr
                   325                 330                 335
   Trp Gly Pro Thr Thr Thr Thr Asn Phe Pro Val Asn Asn Tyr Glu Lys
               340                 345                 350
   Glu Leu Glu Glu Met Lys His Met Thr Arg Gln Glu Phe Val Ala Ser
           355                 360                 365
   Leu Arg Arg Lys Ser Ser Gly Phe Ser Arg Gly Ala Ser Ile Tyr Arg
       370                 375                 380
   Gly Val Thr Arg His His Gln His Gly Arg Trp Gln Ala Arg Ile Gly
   385                 390                 395                 400
   Arg Val Ala Gly Asn Lys Asp Leu Tyr Leu Gly Thr Phe Ser Thr Gln
                   405                 410                 415
   Glu Glu Ala Ala Glu Ala Tyr Asp Ile Ala Ala Ile Lys Phe Arg Gly
               420                 425                 430
   Leu Asn Ala Val Thr Asn Phe Asp Met Ser Arg Tyr Asp Val Lys Ser
           435                 440                 445
   Ile Leu Asp Ser Ala Ala Leu Pro Val Gly Thr Ala Ala Lys Arg Leu
       450                 455                 460
   Lys Asp Ala Glu Ala Ala Ala Ala Tyr Asp Val Gly Arg Ile Ala Ser
   465                 470                 475                 480
   His Leu Gly Gly Asp Gly Ala Tyr Ala Ala His Tyr Gly His His His
                   485                 490                 495
   His Ser Ala Ala Ala Ala Trp Pro Thr Ile Ala Phe Gln Ala Ala Ala
               500                 505                 510
   Ala Pro Pro Pro His Ala Ala Gly Leu Tyr His Pro Tyr Ala Gln Pro
           515                 520                 525
   Leu Arg Gly Trp Cys Lys Gln Glu Gln Asp His Ala Val Ile Ala Ala
       530                 535                 540
   Ala His Ser Leu Gln Asp Leu His His Leu Asn Leu Gly Ala Ala Ala
   545                 550                 555                 560
   Ala Ala His Asp Phe Phe Ser Gln Ala Met Gln Gln Gln His Gly Leu
                   565                 570                 575
   Gly Ser Ile Asp Asn Ala Ser Leu Glu His Ser Thr Gly Ser Asn Ser
               580                 585                 590
   Val Val Tyr Asn Gly Asp Asn Gly Gly Gly Gly Gly Gly Tyr Ile Met
           595                 600                 605
   Ala Pro Met Ser Ala Val Ser Ala Thr Ala Thr Ala Val Ala Ser Ser
       610                 615                 620
   His Asp His Gly Gly Asp Gly Gly Lys Gln Val Gln Met Gly Tyr Asp
   625                 630                 635                 640
   Ser Tyr Leu Val Gly Ala Asp Ala Tyr Gly Gly Gly Gly Ala Gly Arg
                   645                 650                 655
   Met Pro Ser Trp Ala Met Thr Pro Ala Ser Ala Pro Ala Ala Thr Ser
               660                 665                 670
   Ser Ser Asp Met Thr Gly Val Cys His Gly Ala Gln Leu Phe Ser Val
           675                 680                 685
   Trp Asn Asp Thr
       690

<210>  187
<211>  2133
<212>  DNA
<213>  Zea mays

<400>  187
atggccactg tgaacaactg gctcgctttc tccctctccc cgcaggagct gccgccctcc      60
cagacgacgg actccacact catctcggcc gccaccgccg accatgtctc cggcgatgtc     120
tgcttcaaca tcccccaaga ttggagcatg aggggatcag agctttcggc gctcgtcgcg     180
gagccgaagc tggaggactt cctcggcggc atctccttct ccgagcagca tcacaaggcc     240
aactgcaaca tgatacccag cactagcagc acagtttgct acgcgagctc aggtgctagc     300
accggctacc atcaccagct gtaccaccag cccaccagct cagcgctcca cttcgcggac     360
tccgtaatgg tggcctcctc ggccggtgtc cacgacggcg gtgccatgct cagcgcggcc     420
gccgctaacg gtgtcgctgg cgctgccagt gccaacggcg gcggcatcgg gctgtccatg     480
attaagaact ggctgcggag ccaaccggcg cccatgcagc cgagggtggc ggcggctgag     540
ggcgcgcagg ggctctcttt gtccatgaac atggcgggga cgacccaagg cgctgctggc     600
atgccacttc tcgctggaga gcgcgcacgg gcgcccgaga gtgtatcgac gtcagcacag     660
ggtggagccg tcgtcgtcac ggcgccgaag gaggatagcg gtggcagcgg tgttgccggc     720
gctctagtag ccgtgagcac ggacacgggt ggcagcggcg gcgcgtcggc tgacaacacg     780
gcaaggaaga cggtggacac gttcgggcag cgcacgtcga tttaccgtgg cgtgacaagg     840
catagatgga ctgggagata tgaggcacat ctttgggata acagttgcag aaggaagggg     900
caaactcgta agggtcgtca agtctattta ggtggctatg ataaagagga gaaagctgct     960
agggcttatg atcttgctgc tctgaagtac tggggtgcca caacaacaac aaatttccca    1020
gtgagtaact acgaaaagga gctcgaggac atgaagcaca tgacaaggca ggagtttgta    1080

gcgtctctga gaaggaagag cagtggtttc tccagaggtg catccattta caggggagtg 1140
actaggcatc accaacatgg aagatggcaa gcacggattg gacgagttgc agggaacaag 1200
gatctttact tgggcacctt cagcacccag gaggaggcag cggaggcgta cgacatcgcg 1260
gcgatcaagt tccgcggcct caacgccgtc accaacttcg acatgagccg ctacgacgtg 1320
aagagcatcc tggacagcag cgccctcccc atcggcagcg ccgccaagcg cctcaaggag 1380
gccgaggccg cagcgtccgc gcagcaccac cacgccggcg tggtgagcta cgacgtcggc 1440
cgcatcgcct cgcagctcgg cgacggcgga gccctggcgg cggcgtacgg cgcgcactac 1500
cacggcgccg cctggccgac catcgcgttc cagccgggcg ccgccagcac aggcctgtac 1560
cacccgtacg cgcagcagcc aatgcgcggc ggcgggtggt gcaagcagga gcaggaccac 1620
gcggtgatcg cggccgcgca cagcctgcag gacctccacc acctgaacct gggcgcggcc 1680
ggcgcgcacg actttttctc ggcagggcag caggccgccg ccgctgcgat gcacggcctg 1740
ggtagcatcg acagtgcgtc gctcgagcac agcaccggct ccaactccgt cgtctacaac 1800
ggcggggtcg gcgacagcaa cggcgccagc gccgtcggcg gcagtggcgg tggctacatg 1860
atgccgatga gcgctgccgg agcaaccact acatcggcaa tggtgagcca cgagcaggtg 1920
catgcacggg cctacgacga agccaagcag gctgctcaga tggggtacga gagctacctg 1980
gtgaacgcgg agaacaatgg tggcggaagg atgtctgcat gggggactgt cgtgtctgca 2040
gccgcggcgg cagcagcaag cagcaacgac aacatggccg ccgacgtcgg ccatggcggc 2100
gcgcagctct tcagtgtctg gaacgacact taa 2133

<210> 188
<211> 710
<212> PRT
<213> Zea mays

<400> 188
Met Ala Thr Val Asn Asn Trp Leu Ala Phe Ser Leu Ser Pro Gln Glu
1               5                   10                  15
Leu Pro Pro Ser Gln Thr Thr Asp Ser Thr Leu Ile Ser Ala Ala Thr
            20                  25                  30
Ala Asp His Val Ser Gly Asp Val Cys Phe Asn Ile Pro Gln Asp Trp
        35                  40                  45
Ser Met Arg Gly Ser Glu Leu Ser Ala Leu Val Ala Glu Pro Lys Leu
    50                  55                  60
Glu Asp Phe Leu Gly Gly Ile Ser Phe Ser Glu Gln His His Lys Ala
65                  70                  75                  80
Asn Cys Asn Met Ile Pro Ser Thr Ser Ser Thr Val Cys Tyr Ala Ser
                85                  90                  95
Ser Gly Ala Ser Thr Gly Tyr His His Gln Leu Tyr His Gln Pro Thr
            100                 105                 110
Ser Ser Ala Leu His Phe Ala Asp Ser Val Met Val Ala Ser Ser Ala
        115                 120                 125
Gly Val His Asp Gly Gly Ala Met Leu Ser Ala Ala Ala Ala Asn Gly
    130                 135                 140
Val Ala Gly Ala Ala Ser Ala Asn Gly Gly Gly Ile Gly Leu Ser Met
145                 150                 155                 160
Ile Lys Asn Trp Leu Arg Ser Gln Pro Ala Pro Met Gln Pro Arg Val
                165                 170                 175
Ala Ala Ala Glu Gly Ala Gln Gly Leu Ser Leu Ser Met Asn Met Ala
            180                 185                 190
Gly Thr Thr Gln Gly Ala Ala Gly Met Pro Leu Leu Ala Gly Glu Arg
        195                 200                 205
Ala Arg Ala Pro Glu Ser Val Ser Thr Ser Ala Gln Gly Gly Ala Val
    210                 215                 220
Val Val Thr Ala Pro Lys Glu Asp Ser Gly Gly Ser Gly Val Ala Gly
225                 230                 235                 240
Ala Leu Val Ala Val Ser Thr Asp Thr Gly Gly Ser Gly Gly Ala Ser
                245                 250                 255
Ala Asp Asn Thr Ala Arg Lys Thr Val Asp Thr Phe Gly Gln Arg Thr
            260                 265                 270

Ser Ile Tyr Arg Gly Val Thr Arg His Arg Trp Thr Gly Arg Tyr Glu
        275                 280                 285
Ala His Leu Trp Asp Asn Ser Cys Arg Arg Glu Gly Gln Thr Arg Lys
    290                 295                 300
Gly Arg Gln Val Tyr Leu Gly Gly Tyr Asp Lys Glu Glu Lys Ala Ala
305                 310                 315                 320
Arg Ala Tyr Asp Leu Ala Ala Leu Lys Tyr Trp Gly Ala Thr Thr Thr
                325                 330                 335
Thr Asn Phe Pro Val Ser Asn Tyr Glu Lys Glu Leu Glu Asp Met Lys
            340                 345                 350
His Met Thr Arg Gln Glu Phe Val Ala Ser Leu Arg Arg Lys Ser Ser
        355                 360                 365
Gly Phe Ser Arg Gly Ala Ser Ile Tyr Arg Gly Val Thr Arg His His
    370                 375                 380
Gln His Gly Arg Trp Gln Ala Arg Ile Gly Arg Val Ala Gly Asn Lys
385                 390                 395                 400
Asp Leu Tyr Leu Gly Thr Phe Ser Thr Gln Glu Glu Ala Ala Glu Ala
                405                 410                 415
Tyr Asp Ile Ala Ala Ile Lys Phe Arg Gly Leu Asn Ala Val Thr Asn
            420                 425                 430
Phe Asp Met Ser Arg Tyr Asp Val Lys Ser Ile Leu Asp Ser Ser Ala
        435                 440                 445
Leu Pro Ile Gly Ser Ala Ala Lys Arg Leu Lys Glu Ala Glu Ala Ala
    450                 455                 460
Ala Ser Ala Gln His His His Ala Gly Val Val Ser Tyr Asp Val Gly
465                 470                 475                 480
Arg Ile Ala Ser Gln Leu Gly Asp Gly Gly Ala Leu Ala Ala Ala Tyr
                485                 490                 495
Gly Ala His Tyr His Gly Ala Ala Trp Pro Thr Ile Ala Phe Gln Pro
            500                 505                 510
Gly Ala Ala Ser Thr Gly Leu Tyr His Pro Tyr Ala Gln Gln Pro Met
        515                 520                 525
Arg Gly Gly Gly Trp Cys Lys Gln Glu Gln Asp His Ala Val Ile Ala
    530                 535                 540
Ala Ala His Ser Leu Gln Asp Leu His His Leu Asn Leu Gly Ala Ala
545                 550                 555                 560
Gly Ala His Asp Phe Phe Ser Ala Gly Gln Gln Ala Ala Ala Ala Ala
                565                 570                 575
Met His Gly Leu Gly Ser Ile Asp Ser Ala Ser Leu Glu His Ser Thr
            580                 585                 590
Gly Ser Asn Ser Val Val Tyr Asn Gly Gly Val Gly Asp Ser Asn Gly
        595                 600                 605
Ala Ser Ala Val Gly Gly Ser Gly Gly Gly Tyr Met Met Pro Met Ser
    610                 615                 620
Ala Ala Gly Ala Thr Thr Thr Ser Ala Met Val Ser His Glu Gln Val
625                 630                 635                 640
His Ala Arg Ala Tyr Asp Glu Ala Lys Gln Ala Ala Gln Met Gly Tyr
                645                 650                 655
Glu Ser Tyr Leu Val Asn Ala Glu Asn Asn Gly Gly Gly Arg Met Ser
            660                 665                 670
Ala Trp Gly Thr Val Val Ser Ala Ala Ala Ala Ala Ala Ala Ser Ser
        675                 680                 685
Asn Asp Asn Met Ala Ala Asp Val Gly His Gly Gly Ala Gln Leu Phe
    690                 695                 700
Ser Val Trp Asn Asp Thr
705                 710

<210>  189
<211>  1374

<212>  DNA
<213>  Lotus corniculatus

<400>  189
agatctctct ctgcagtgca caatccagtg gtaagcacat gtagtaatgg taatgagtct      60
caagaaaatg gtaatggtaa tttgcaatcg ttgacattat ccatgggaag tggtaaggat     120
tcaacatgtg aaaccagtgg tgacactagt accaacacta tcgaagctgt gcctagaaga     180
accttggaga catttgggca aagaacatct atatatcgag gtgtaacaag acatagatgg     240
accggaaggt atgaagctca cctttgggac aatagctgta gaagggaagg acagtcaagg     300
aaaggtcgcc aaggtggata tgataaagaa gacaaagcag ctaggcctta tgatttagct     360
gcccttaagt actgggggac atccactact accaactttc cggttagcaa ctatgagaag     420
gaagtggatg acatgaagca tatgacaaga caagaatttg tggcttccat tagaaggaaa     480
agcagtggtt tctcgagggg tgcttcaatg tatcgtggag ttacaaggca tcatcaacat     540
gggaggtggc aagcaaggat tggaagagtt gcaggaaaca aagatcttta cttgggaact     600
ttcggtactg aggaagaggc ggcagaagct tacgacatag ctgcgataaa gttcagaggc     660
cttaacgcca tcaccaactt tgacatgaac cgttacgatg tgaaagccat tctagagagc     720
aacaccctcc caatcggagg aggagcttca aaaaggctaa aagaaactca agctctggaa     780
tcttcaagaa aacgtgaaga tcagatgatt gcactcggct caacatttca tcaatacgga     840
attgcaaccc catcaagctc tacaaggcca caaccttacc cgctaaacct aatgcatcat     900
cacaatcagt ttgaacaaca gcctcaacca tttctaacct tacaaaacca tgacattaat     960
tctcaatact cccatcatca gcaggaccct tcgtttcagc agagttacat tcaaacacag    1020
cttcagttgc agttgaatca acacggtggt ggtggttcta gttatgctca agaaacagct    1080
cctcatcaga acagtgagtt ctataatggt ggaaattatt accttcagaa ccttcaggga    1140
atgatgatga acaatagtat ggggtcttgt tcttcatcgt ctgtgttgga gaatgatcat    1200
aatgctgctg ctggtggggc ttcttttgtg ggtcccgcag cggaggaact tgggttggtt    1260
aaggttgatt atgacatgga tgctgctgct ggcggtggtt atggtggttg gtcagcggcg    1320
gagtccatgc acacgtcggc ggctggtggt ttgtttacta tgtggaatga gtga          1374

<210>  190
<211>  457
<212>  PRT
<213>  Lotus corniculatus

<400>  190
Arg Ser Leu Ser Ala Val His Asn Pro Val Val Ser Thr Cys Ser Asn
1               5                   10                  15
Gly Asn Glu Ser Gln Glu Asn Gly Asn Gly Asn Leu Gln Ser Leu Thr
            20                  25                  30
Leu Ser Met Gly Ser Gly Lys Asp Ser Thr Cys Glu Thr Ser Gly Asp
        35                  40                  45
Thr Ser Thr Asn Thr Ile Glu Ala Val Pro Arg Arg Thr Leu Glu Thr
    50                  55                  60
Phe Gly Gln Arg Thr Ser Ile Tyr Arg Gly Val Thr Arg His Arg Trp
65                  70                  75                  80
Thr Gly Arg Tyr Glu Ala His Leu Trp Asp Asn Ser Cys Arg Arg Glu
                85                  90                  95
Gly Gln Ser Arg Lys Gly Arg Gln Gly Gly Tyr Asp Lys Glu Asp Lys
            100                 105                 110
Ala Ala Arg Ala Tyr Asp Leu Ala Ala Leu Lys Tyr Trp Gly Thr Ser
        115                 120                 125
Thr Thr Thr Asn Phe Pro Val Ser Asn Tyr Glu Lys Glu Val Asp Asp
    130                 135                 140
Met Lys His Met Thr Arg Gln Glu Phe Val Ala Ser Ile Arg Arg Lys
145                 150                 155                 160
Ser Ser Gly Phe Ser Arg Gly Ala Ser Met Tyr Arg Gly Val Thr Arg
                165                 170                 175
His His Gln His Gly Arg Trp Gln Ala Arg Ile Gly Arg Val Ala Gly
            180                 185                 190
Asn Lys Asp Leu Tyr Leu Gly Thr Phe Gly Thr Glu Glu Glu Ala Ala

195                 200                 205
Glu Ala Tyr Asp Ile Ala Ala Ile Lys Phe Arg Gly Leu Asn Ala Ile
    210                 215                 220
Thr Asn Phe Asp Met Asn Arg Tyr Asp Val Lys Ala Ile Leu Glu Ser
225                 230                 235                 240
Asn Thr Leu Pro Ile Gly Gly Gly Ala Ser Lys Arg Leu Lys Glu Thr
                245                 250                 255
Gln Ala Leu Glu Ser Ser Arg Lys Arg Glu Asp Gln Met Ile Ala Leu
            260                 265                 270
Gly Ser Thr Phe His Gln Tyr Gly Ile Ala Thr Pro Ser Ser Ser Thr
        275                 280                 285
Arg Pro Gln Pro Tyr Pro Leu Asn Leu Met His His His Asn Gln Phe
    290                 295                 300
Glu Gln Gln Pro Gln Pro Phe Leu Thr Leu Gln Asn His Asp Ile Asn
305                 310                 315                 320
Ser Gln Tyr Ser His His Gln Gln Asp Pro Ser Phe Gln Gln Ser Tyr
                325                 330                 335
Ile Gln Thr Gln Leu Gln Leu Gln Leu Asn Gln His Gly Gly Gly Gly
            340                 345                 350
Ser Ser Tyr Ala Gln Glu Thr Ala Pro His Gln Asn Ser Glu Phe Tyr
        355                 360                 365
Asn Gly Gly Asn Tyr Tyr Leu Gln Asn Leu Gln Gly Met Met Met Asn
    370                 375                 380
Asn Ser Met Gly Ser Cys Ser Ser Ser Ser Val Leu Glu Asn Asp His
385                 390                 395                 400
Asn Ala Ala Ala Gly Gly Ala Ser Phe Val Gly Pro Ala Ala Glu Glu
                405                 410                 415
Leu Gly Leu Val Lys Val Asp Tyr Asp Met Asp Ala Ala Ala Gly Gly
            420                 425                 430
Gly Tyr Gly Gly Trp Ser Ala Ala Glu Ser Met His Thr Ser Ala Ala
        435                 440                 445
Gly Gly Leu Phe Thr Met Trp Asn Glu
    450                 455

<210>  191
<211>  202
<212>  PRT
<213>  Artificial sequence

<220>
<223>  AP2 domain of Arath_PLT1 and Arath_PLT2

<220>
<221>  VARIANT
<222>  (4)..(4)
<223>  /replace = "Thr"

<220>
<221>  VARIANT
<222>  (6)..(6)
<223>  /replace = "Glu"

<220>
<221>  VARIANT
<222>  (57)..(57)
<223>  /replace = "Glu"

<220>
<221>  VARIANT

<222>  (62)..(62)
<223>  /replace = "Ala"

<220>
<221>  VARIANT
<222>  (93)..(93)
<223>  /replace = "Asn"

<220>
<221>  VARIANT
<222>  (102)..(102)
<223>  /replace = "Ser"

<220>
<221>  VARIANT
<222>  (187)..(187)
<223>  /replace = "Asn"

<400>  191
Pro Arg Arg Ala Leu Asp Thr Phe Gly Gln Arg Thr Ser Ile Tyr Arg
1               5                   10                  15
Gly Val Thr Arg His Arg Trp Thr Gly Arg Tyr Glu Ala His Leu Trp
            20                  25                  30
Asp Asn Ser Cys Arg Arg Glu Gly Gln Ser Arg Lys Gly Arg Gln Val
        35                  40                  45
Tyr Leu Gly Gly Tyr Asp Lys Glu Asp Lys Ala Ala Arg Ser Tyr Asp
    50                  55                  60
Leu Ala Ala Leu Lys Tyr Trp Gly Pro Ser Thr Thr Thr Asn Phe Pro
65                  70                  75                  80
Ile Thr Asn Tyr Glu Lys Glu Val Glu Glu Met Lys His Met Thr Arg
                85                  90                  95
Gln Glu Phe Val Ala Ala Ile Arg Arg Lys Ser Ser Gly Phe Ser Arg
            100                 105                 110
Gly Ala Ser Met Tyr Arg Gly Val Thr Arg His His Gln His Gly Arg
        115                 120                 125
Trp Gln Ala Arg Ile Gly Arg Val Ala Gly Asn Lys Asp Leu Tyr Leu
    130                 135                 140
Gly Thr Phe Ser Thr Glu Glu Glu Ala Ala Glu Ala Tyr Asp Ile Ala
145                 150                 155                 160
Ala Ile Lys Phe Arg Gly Leu Asn Ala Val Thr Asn Phe Glu Ile Asn
                165                 170                 175
Arg Tyr Asp Val Lys Ala Ile Leu Glu Ser Ser Thr Leu Pro Ile Gly
            180                 185                 190
Gly Gly Ala Ala Lys Arg Leu Lys Glu Ala
        195                 200

<210>  192
<211>  8
<212>  PRT
<213>  Artificial sequence

<220>
<223>  motif 1

<220>
<221>  VARIANT
<222>  (3)..(3)
<223>  /replace = "Leu"

<220>
<221> VARIANT
<222> (4)..(4)
<223> /replace = "Glu"

<400> 192
Pro Lys Val Ala Asp Phe Leu Gly
1               5

<210> 193
<211> 7
<212> PRT
<213> Artificial sequence

<220>
<223> motif 2

<220>
<221> VARIANT
<222> (1)..(1)
<223> /replace = "Leu"

<220>
<221> VARIANT
<222> (3)..(3)
<223> /replace = "Ser"

<220>
<221> VARIANT
<222> (4)..(4)
<223> /replace = "Val"

<220>
<221> VARIANT
<222> (7)..(7)
<223> /replace = "Glu"

<400> 193
Val Phe Thr Met Trp Asn Asp
1               5

<210> 194
<211> 2193
<212> DNA
<213> Oryza sativa

<400> 194
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720

aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga attttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 195
<211> 54
<212> DNA
<213> Artificial sequence

<220>
<223> primer: PRM08180

<400> 195
ggggacaagt ttgtacaaaa aagcaggctt aaacaatgat caatccacac ggtg 54

<210> 196
<211> 50
<212> DNA
<213> Artificial sequence

<220>
<223> primer: PRM08181

<400> 196
ggggaccact ttgtacaaga aagctgggtt ccttgtttac tcattccaca 50

<210> 197
<211> 56
<212> DNA
<213> Artificial sequence

<220>
<223> primer: PRM08182

<400> 197
ggggacaagt ttgtacaaaa aagcaggctt aaacaatgaa ttctaacaac tggctc 56

<210> 198
<211> 50
<212> DNA
<213> Artificial sequence

<220>
<223> primer: PRM08183

<400> 198
ggggaccact ttgtacaaga aagctgggtt catcttttat tcattccaca 50

<210> 199
<211> 1638
<212> DNA
<213> Populus tremuloides

<400> 199
atgaattcta acaactggct ttcatttcct ctttctccta ctcatccttc cttgcctgct 60
catctacatg catcccaccc tcatcaattc tctctagggt tagtcaatga taatatggaa 120
aacccatttc aaactcaaga gtggagtctt cttaacactc atcaaggcaa caatgaagtg 180
ccaaaggttg cagactttct tggtgtgagc aaatctgaga atcaatcaga tcttgtagcc 240
ttcaatgaaa ttcaagctaa tgaatctgac tatctctttt caaacaatag tctagtacca 300
gtccaaaatg ctgttgtagg cgccaataat acctttgagt ttcaagaaaa tgctagcaat 360
ttgcagtcat taacattgtc tatgggcagt gctagtggta aaggttctac atgtgaaccc 420
agtggggata atagcactaa tactgttgaa gctgctgcac caagaagaac tttggataca 480
tttgggcaaa gaacatccat atatcgtggt gtaacaaggc atcgatggac aggaaggtat 540
gaagctcatt tatgggataa tagttgcaga agagaaggtc aatctaggaa aggaagacag 600
ggtggctatg acaaagaaga aaaggcagct agggcttatg atcttgctgc acttaagtac 660
tggggaacat ccaccactac caatttccca atcagcaact acgagaaaga aatagaggaa 720
atgaagcaca tgaccaggca agaatttgtg gcctccatta gaaggaagag tagtggcttc 780
tctaggggtg catccatgta tcgtggagtt acaaggcatc accagcatgg tagatggcaa 840
gcaaggatag gcagagttgc aggaaacaaa gatctctact tgggaacttt tagcactgag 900
gaggaggctg cagaagctta tgacatagca gcaataaagt ttagagggct taatgcagtg 960
actaactttg acatgaatcg atatgatgtg aagagcattc ttgaaagcaa tactttgcca 1020
attggaggag gggcagccaa acggctaaag gaggctcaag caattgaatc atcacgaaaa 1080
agagaagaaa tgattgctct tggctcaagt tttccatatg gatcaacttc aagctctagc 1140
aggctacaag cttaccctct aatgcagaca ccatttgagc aacctcaacc tttacttact 1200
ctacaaaatc aagacatttc tcagtacact caggattcct catcattcca ccaaaatttc 1260
cttcaaacac agcttcattt gcaccagcaa tctacagggt ctaatttcct gcataaccaa 1320
tcaaaccaaa accctcaata ttacaatagt tatatccaaa acaatccagc tttacttcat 1380
ggattgtgga acatgggttc ttcatcatct gtaatggaga ataatggaag ttctagtggg 1440
agctatagta ctggaggtta tctgggaaat gggctgggaa tggcttccaa ttcaacaggg 1500
tctaatgcag taggatcagc cgaggaactt gcacttgtca aagttgatta tgatatgcct 1560
tctagtggct atggaagctg gtctggggac tcagtccagg gatccaatcc aggtgttttc 1620
actatgtgga atgagtga 1638

<210> 200
<211> 545
<212> PRT
<213> Populus tremuloides

<400> 200
   Met Asn Ser Asn Asn Trp Leu Ser Phe Pro Leu Ser Pro Thr His Pro
   1 5 10 15
   Ser Leu Pro Ala His Leu His Ala Ser His Pro His Gln Phe Ser Leu
   20 25 30
   Gly Leu Val Asn Asp Asn Met Glu Asn Pro Phe Gln Thr Gln Glu Trp
   35 40 45
   Ser Leu Leu Asn Thr His Gln Gly Asn Asn Glu Val Pro Lys Val Ala
       50                  55                  60
   Asp Phe Leu Gly Val Ser Lys Ser Glu Asn Gln Ser Asp Leu Val Ala
   65                  70                  75                  80
   Phe Asn Glu Ile Gln Ala Asn Glu Ser Asp Tyr Leu Phe Ser Asn Asn
                   85                  90                  95
   Ser Leu Val Pro Val Gln Asn Ala Val Val Gly Ala Asn Asn Thr Phe
               100                 105                 110
   Glu Phe Gln Glu Asn Ala Ser Asn Leu Gln Ser Leu Thr Leu Ser Met
           115                 120                 125
   Gly Ser Ala Ser Gly Lys Gly Ser Thr Cys Glu Pro Ser Gly Asp Asn
       130                 135                 140
   Ser Thr Asn Thr Val Glu Ala Ala Ala Pro Arg Arg Thr Leu Asp Thr
   145                 150                 155                 160
   Phe Gly Gln Arg Thr Ser Ile Tyr Arg Gly Val Thr Arg His Arg Trp
                   165                 170                 175
   Thr Gly Arg Tyr Glu Ala His Leu Trp Asp Asn Ser Cys Arg Arg Glu
               180                 185                 190
   Gly Gln Ser Arg Lys Gly Arg Gln Gly Gly Tyr Asp Lys Glu Glu Lys
           195                 200                 205
   Ala Ala Arg Ala Tyr Asp Leu Ala Ala Leu Lys Tyr Trp Gly Thr Ser
       210                 215                 220
   Thr Thr Thr Asn Phe Pro Ile Ser Asn Tyr Glu Lys Glu Ile Glu Glu
   225                 230                 235                 240
   Met Lys His Met Thr Arg Gln Glu Phe Val Ala Ser Ile Arg Arg Lys
                   245                 250                 255
   Ser Ser Gly Phe Ser Arg Gly Ala Ser Met Tyr Arg Gly Val Thr Arg
               260                 265                 270
   His His Gln His Gly Arg Trp Gln Ala Arg Ile Gly Arg Val Ala Gly
           275                 280                 285
   Asn Lys Asp Leu Tyr Leu Gly Thr Phe Ser Thr Glu Glu Glu Ala Ala
       290                 295                 300
   Glu Ala Tyr Asp Ile Ala Ala Ile Lys Phe Arg Gly Leu Asn Ala Val
   305                 310                 315                 320
   Thr Asn Phe Asp Met Asn Arg Tyr Asp Val Lys Ser Ile Leu Glu Ser
                   325                 330                 335
   Asn Thr Leu Pro Ile Gly Gly Gly Ala Ala Lys Arg Leu Lys Glu Ala
               340                 345                 350
   Gln Ala Ile Glu Ser Ser Arg Lys Arg Glu Glu Met Ile Ala Leu Gly
           355                 360                 365
   Ser Ser Phe Pro Tyr Gly Ser Thr Ser Ser Ser Ser Arg Leu Gln Ala
       370                 375                 380
   Tyr Pro Leu Met Gln Thr Pro Phe Glu Gln Pro Gln Pro Leu Leu Thr
   385                 390                 395                 400
   Leu Gln Asn Gln Asp Ile Ser Gln Tyr Thr Gln Asp Ser Ser Ser Phe
                   405                 410                 415
   His Gln Asn Phe Leu Gln Thr Gln Leu His Leu His Gln Gln Ser Thr
               420                 425                 430
   Gly Ser Asn Phe Leu His Asn Gln Ser Asn Gln Asn Pro Gln Tyr Tyr
           435                 440                 445
   Asn Ser Tyr Ile Gln Asn Asn Pro Ala Leu Leu His Gly Leu Trp Asn
       450                 455                 460
   Met Gly Ser Ser Ser Ser Val Met Glu Asn Asn Gly Ser Ser Ser Gly
   465                 470                 475                 480
   Ser Tyr Ser Thr Gly Gly Tyr Leu Gly Asn Gly Leu Gly Met Ala Ser
                   485                 490                 495
   Asn Ser Thr Gly Ser Asn Ala Val Gly Ser Ala Glu Glu Leu Ala Leu
               500                 505                 510
   Val Lys Val Asp Tyr Asp Met Pro Ser Ser Gly Tyr Gly Ser Trp Ser
           515                 520                 525
   Gly Asp Ser Val Gln Gly Ser Asn Pro Gly Val Phe Thr Met Trp Asn
       530                 535                 540
   Glu
   545

<210>  201
<211>  1515
<212>  DNA
<213>  Populus tremuloides

<400>  201
atgttttttt ttttgtttcc tttagagtgg agtcttctta acactcaagg caacaatgaa      60
gtgccaaagg ttgcagattt tcttggtgta agtaaatctg agaatcaatc agatctcgta     120
gccttcaatg aaattcaagc cagtgattct gagtatctct tttcaagcaa tagtctgttg     180
ccggtccaaa atgctgtggt agccgccagt actaactacg aatttcaaga aaatcctagc     240
aatttgcagt cattaacatt gtctatgggc agtgctagtg gtaagggttc taaatgtgaa     300
accagtggtg ataatagtac taattctgtc gaagctgctg ctccaagaag gactttggat     360
acatttggtc aaagaacatc catctatcgt ggtgtaacaa ggcatcgatg gacaggaagg     420
tatgaagctc atttatggga taatagttgc agaagagaag gtcaatccag gaaaggaaga     480
caaggtggct atgacaaaga agacaaggct gctagggctt atgatcttgc tgcacttaag     540
tactggggaa catcgaccac taccaatttt cctatcagca actatgagaa agaactagag     600
gacatgaaga acatgaccag acaagaattt gtggcctcca ttagaaggaa gagtagtggc     660
ttctctaggg gtgcatccat gtatcgtgga gtcacaaggc atcaccaaca tggaagatgg     720
caagcaagaa ttggtagagt tgcaggaaac aaagatctct acttgggaac ttttagcact     780
gaggaggagg ctgcagaagc ttatgacata gcagcaataa agtttagagg gcttaatgca     840
gtgactaatt ttgacatgaa tcgatatgat gtgaaaagca ttcttgagag caatagtttg     900
ccaattggag gaggggcagc caaaaggcta aaggaggctc aagcaatcga atcgtcacaa     960
aaacgagaag aaatgattgc tcttggatca agttatccat atggatcaac ttcaagctct    1020
agtcgacaac aagcttactc tctaatgcag aaaccatttg agcaacctca acctttactt    1080
actctacaaa atcaagacat ttctcagtac actcaagatt cttcatttca gcaaaattac    1140
cttcaaacac agcttcattt gcaccagcta tctgcagggt ctaatttcct gcataataac    1200
caatcaagcc aaaatcctca gtattacaac agctatatcc aaaacaatcc cactttgctt    1260
catggattgt ggaacatggg ttcttcatca tctctaatgg agaataatgg cagttctagt    1320
gggagttata gtactgtcgg ttatctggga aatgggttgg gaatggctac caattcaaca    1380
gggtctaatg cagtagctga ggaacttcca cttgttaaga tagattatga tatgccttct    1440
ggtggctatg gaagttggtc tggggaatca gttcagggat ccaacccagg tgtttttaca    1500
atgtggaatg agtga                                                     1515

<210>  202
<211>  504
<212>  PRT
<213>  Populus tremuloides

<400>  202
Met Phe Phe Phe Leu Phe Pro Leu Glu Trp Ser Leu Leu Asn Thr Gln
1               5                   10                  15
Gly Asn Asn Glu Val Pro Lys Val Ala Asp Phe Leu Gly Val Ser Lys
            20                  25                  30
Ser Glu Asn Gln Ser Asp Leu Val Ala Phe Asn Glu Ile Gln Ala Ser
        35                  40                  45
Asp Ser Glu Tyr Leu Phe Ser Ser Asn Ser Leu Leu Pro Val Gln Asn
    50                  55                  60
Ala Val Val Ala Ala Ser Thr Asn Tyr Glu Phe Gln Glu Asn Pro Ser
65                  70                  75                  80
Asn Leu Gln Ser Leu Thr Leu Ser Met Gly Ser Ala Ser Gly Lys Gly
                85                  90                  95
Ser Lys Cys Glu Thr Ser Gly Asp Asn Ser Thr Asn Ser Val Glu Ala
            100                 105                 110
Ala Ala Pro Arg Arg Thr Leu Asp Thr Phe Gly Gln Arg Thr Ser Ile

115                 120                 125
Tyr Arg Gly Val Thr Arg His Arg Trp Thr Gly Arg Tyr Glu Ala His
    130                 135                 140
Leu Trp Asp Asn Ser Cys Arg Arg Glu Gly Gln Ser Arg Lys Gly Arg
145                 150                 155                 160
Gln Gly Gly Tyr Asp Lys Glu Asp Lys Ala Ala Arg Ala Tyr Asp Leu
                165                 170                 175
Ala Ala Leu Lys Tyr Trp Gly Thr Ser Thr Thr Thr Asn Phe Pro Ile
            180                 185                 190
Ser Asn Tyr Glu Lys Glu Leu Glu Asp Met Lys Asn Met Thr Arg Gln
        195                 200                 205
Glu Phe Val Ala Ser Ile Arg Arg Lys Ser Ser Gly Phe Ser Arg Gly
    210                 215                 220
Ala Ser Met Tyr Arg Gly Val Thr Arg His His Gln His Gly Arg Trp
225                 230                 235                 240
Gln Ala Arg Ile Gly Arg Val Ala Gly Asn Lys Asp Leu Tyr Leu Gly
                245                 250                 255
Thr Phe Ser Thr Glu Glu Glu Ala Ala Glu Ala Tyr Asp Ile Ala Ala
            260                 265                 270
Ile Lys Phe Arg Gly Leu Asn Ala Val Thr Asn Phe Asp Met Asn Arg
        275                 280                 285
Tyr Asp Val Lys Ser Ile Leu Glu Ser Asn Ser Leu Pro Ile Gly Gly
    290                 295                 300
Gly Ala Ala Lys Arg Leu Lys Glu Ala Gln Ala Ile Glu Ser Ser Gln
305                 310                 315                 320
Lys Arg Glu Glu Met Ile Ala Leu Gly Ser Ser Tyr Pro Tyr Gly Ser
                325                 330                 335
Thr Ser Ser Ser Ser Arg Gln Gln Ala Tyr Ser Leu Met Gln Lys Pro
            340                 345                 350
Phe Glu Gln Pro Gln Pro Leu Leu Thr Leu Gln Asn Gln Asp Ile Ser
        355                 360                 365
Gln Tyr Thr Gln Asp Ser Ser Phe Gln Gln Asn Tyr Leu Gln Thr Gln
    370                 375                 380
Leu His Leu His Gln Leu Ser Ala Gly Ser Asn Phe Leu His Asn Asn
385                 390                 395                 400
Gln Ser Ser Gln Asn Pro Gln Tyr Tyr Asn Ser Tyr Ile Gln Asn Asn
                405                 410                 415
Pro Thr Leu Leu His Gly Leu Trp Asn Met Gly Ser Ser Ser Ser Leu
            420                 425                 430
Met Glu Asn Asn Gly Ser Ser Ser Gly Ser Tyr Ser Thr Val Gly Tyr
        435                 440                 445
Leu Gly Asn Gly Leu Gly Met Ala Thr Asn Ser Thr Gly Ser Asn Ala
    450                 455                 460
Val Ala Glu Glu Leu Pro Leu Val Lys Ile Asp Tyr Asp Met Pro Ser
465                 470                 475                 480
Gly Gly Tyr Gly Ser Trp Ser Gly Glu Ser Val Gln Gly Ser Asn Pro
                485                 490                 495
Gly Val Phe Thr Met Trp Asn Glu
            500

<210>  203
<211>  785
<212>  DNA
<213>  Vitis vinifera

<400>  203
tcttgataaa tcccaaaact gactgtgtag gtactgagga ggaagctgca gaagcctatg      60
atattgcagc aataaagttc agaggcctta atgcagtgac caactttgac atgaatcgat     120
acgatgtgaa gagcattctt gaaagcaaca ctcttccgat aggtggggga gcggccaagc     180

ggctaaagga ggctcaagca attgaatcat caagaaaacg ggaagaaatg atagccctag 240
gttcaagttt ccaatatggg agctcgagct ctagcaggtt acagacatat cctctaatgc 300
agcagcagtt tgagcaacct cagcctttac taacattaca gaaccaagaa ccattactaa 360
ctttgcaaaa ccctgaaatt tctcagtacc cccaagactc ccagtttcac caaaactaca 420
tccaaactca gttgcagttg caccagcaat ctgggtcgta cctgaaccat tcaagccaaa 480
gtcctcagtt ctacaacagt tacctccaca acaacccggc tcttcttcat gggctgatga 540
gtatgggctc ttcttcatct gtcatggaga ataatgggag ttctagtggg agttacaatg 600
gaggmtactt caataatgga cttggggttg cttcgaattc tacggtggct agtgcagtag 660
gatcagcaga ggagcttccc ctcatcaagg ttgattacga tatgccggcc gcaggctatg 720
gcagctggtc aggtgactca gttcagggac agaatgctgg agtttttaca atgtggaatg 780
actga 785

<210> 204
<211> 260
<212> PRT
<213> Vitis vinifera

<400> 204
Leu Ile Asn Pro Lys Thr Asp Cys Val Gly Thr Glu Glu Glu Ala Ala
1               5                   10                  15
Glu Ala Tyr Asp Ile Ala Ala Ile Lys Phe Arg Gly Leu Asn Ala Val
            20                  25                  30
Thr Asn Phe Asp Met Asn Arg Tyr Asp Val Lys Ser Ile Leu Glu Ser
        35                  40                  45
Asn Thr Leu Pro Ile Gly Gly Gly Ala Ala Lys Arg Leu Lys Glu Ala
    50                  55                  60
Gln Ala Ile Glu Ser Ser Arg Lys Arg Glu Glu Met Ile Ala Leu Gly
65                  70                  75                  80
Ser Ser Phe Gln Tyr Gly Ser Ser Ser Ser Ser Arg Leu Gln Thr Tyr
                85                  90                  95
Pro Leu Met Gln Gln Gln Phe Glu Gln Pro Gln Pro Leu Leu Thr Leu
            100                 105                 110
Gln Asn Gln Glu Pro Leu Leu Thr Leu Gln Asn Pro Glu Ile Ser Gln
        115                 120                 125
Tyr Pro Gln Asp Ser Gln Phe His Gln Asn Tyr Ile Gln Thr Gln Leu
    130                 135                 140
Gln Leu His Gln Gln Ser Gly Ser Tyr Leu Asn His Ser Ser Gln Ser
145                 150                 155                 160
Pro Gln Phe Tyr Asn Ser Tyr Leu His Asn Asn Pro Ala Leu Leu His
                165                 170                 175
Gly Leu Met Ser Met Gly Ser Ser Ser Ser Val Met Glu Asn Asn Gly
            180                 185                 190
Ser Ser Ser Gly Ser Tyr Asn Gly Gly Tyr Phe Asn Asn Gly Leu Gly
        195                 200                 205
Val Ala Ser Asn Ser Thr Val Ala Ser Ala Val Gly Ser Ala Glu Glu
    210                 215                 220
Leu Pro Leu Ile Lys Val Asp Tyr Asp Met Pro Ala Ala Gly Tyr Gly
225                 230                 235                 240
Ser Trp Ser Gly Asp Ser Val Gln Gly Gln Asn Ala Gly Val Phe Thr
                245                 250                 255
Met Trp Asn Asp
            260

<210> 205
<211> 150
<212> DNA
<213> brassica napus

<400> 205

ggaacatcgg ccgaggaaga gtttcccacg gttaaagttg attacgatat gcctccttta 60
ggtggagcca cagggtgtga acgatggact aatggagaga atggtcaggg gtcaaatcca 120
ggaggtgtct tcacaatgtg gaatgaataa 150

<210> 206
<211> 49
<212> PRT
<213> Brassica napus

<400> 206
Gly Thr Ser Ala Glu Glu Glu Phe Pro Thr Val Lys Val Asp Tyr Asp
1               5                   10                  15
Met Pro Pro Leu Gly Gly Ala Thr Gly Cys Glu Arg Trp Thr Asn Gly
            20                  25                  30
Glu Asn Gly Gln Gly Ser Asn Pro Gly Gly Val Phe Thr Met Trp Asn
        35                  40                  45
Glu

<210> 207
<211> 162
<212> DNA
<213> Phaseolus coccineus

<400> 207
tcgaatgcat cgtcgggtaa tgcggtgggc acggcggagg aacttggatt ggtgaaagtt 60
gactatgaca tgccggccgg aggttacggt ggttggtcgg cggcggcggc ggaatccatg 120
cagacgtcaa atggtggggt gttcacaatg tggaatgagt ga 162

<210> 208
<211> 53
<212> PRT
<213> Phaseolus coccineus

<400> 208
Ser Asn Ala Ser Ser Gly Asn Ala Val Gly Thr Ala Glu Glu Leu Gly
1               5                   10                  15
Leu Val Lys Val Asp Tyr Asp Met Pro Ala Gly Gly Tyr Gly Gly Trp
            20                  25                  30
Ser Ala Ala Ala Ala Glu Ser Met Gln Thr Ser Asn Gly Gly Val Phe
        35                  40                  45
Thr Met Trp Asn Glu
    50

<210> 209
<211> 7
<212> PRT
<213> Artificial sequence

<220>
<223> Motif 2

<220>
<221> VARIANT
<222> (1)..(1)
<223> /replace = "Leu"

<220>
<221> UNSURE

<222>  (3)..(3)
<223>  Xaa can be any naturally occurring amino acid

<220>
<221>  VARIANT
<222>  (4)..(4)
<223>  /replace = "Val"

<220>
<221>  VARIANT
<222>  (7)..(7)
<223>  /replace = "Glu"

<400>  209
Val Phe Xaa Met Trp Asn Asp
1               5

<210>  210
<211>  2194
<212>  DNA
<213>  Oryza sativa

<400>  210
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt      420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata     1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct     1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt     1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct     2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160

ttggtgtagc ttgccacttt caccagcaaa gttc 2194

<210> 211
<211> 1245
<212> DNA
<213> Oryza sativa

<400> 211
cttgttgttg atctgtgccc ccaagaagaa taacactcta ctcttacttg ttggaaaaaa 60
atagtattag caaccacgca tatgcaaatt ttaatgcagt aataataaga gatggatcga 120
tcgttttcca gctcttgtat atgtgactgg ccctgcttta tgtgtgtagt gttaatttca 180
gctttagcag tacgtgatta gtgatggaca ataattgtcg cagacgtatc tatcaattgc 240
tcctgttgtg tgatgcttta actgttggaa tcaaagttgc gttgcctttg ttgttatgag 300
gaggaatata tatgttgggg caggaaaaga atggaggaga gatcgttctc catatcctta 360
tcatcggcct cgtcactgct cgcagtttaa ctttttggtg atgcgagcga tggtcagcca 420
tatatatact cccatgctgc atgctagtaa tcaatatacg ccttgtaaaa gtaaacgatc 480
gtctagtaat tgcaatatca taggggtagc cattgacaga gatctacata gatagagggg 540
gaacaagaat tgacactcca cagatgctcc actcattcac ctttactaat ttatatcttt 600
tgatgtttga tcgatcgatc gatccgtccg tcggtgtctc gacgaataaa aactgcaaat 660
cgaactgtat gtatataata tagcgtcgta aattaaatta aattaaatcg aactgaatac 720
tacatgtcga agcaagaatt agttcaacta aaagatttag tttttccggt tgcaatatct 780
gtgaaattaa ttgaagaaat taagaagaaa actggagaga tatatatatg gatgagacaa 840
aatgagataa gacgcatgat ggtccctcgg atgatgtcgt ccgttcctta tttccattcc 900
atggcagctg ctatcgctat ctagtgcgcg cggcatctcc aatcccatcc attctagtgg 960
tcgatctagc tactactgag tattgttttt tcttcttttt actactgttg attattctgc 1020
aactgcagtt agatgcttgc tactcctaca tcgatctctc tcgcgcgggc gtatgcattg 1080
cattcactac tgatgatccg tgggtgtagt gtgggtggct ataaataggg cagggtgcgg 1140
ttgccattgc tcctcaggcc agcaactgag aagctccata caagtaagca gcagctagtt 1200
gccgacaagg ccagagaagg aagaagaagc tctcatcatc atcac 1245

<210> 212
<211> 675
<212> DNA
<213> Oryza sativa

<400> 212
atgaagagca ggaagaacag cacgacgagc acaaaagcag caggcagctg ccacaccagc 60
agcagcggag gaggaggagg cggcggcaac tgctatagca gcagcagtag caagatggag 120
cgcaaggatg tggagaagaa tcggcgcctc cacatgaagg gtctctgcct caagctctcc 180
tccctcatcc ccgccgccgc tccccgccgc catcaccacc actactccac ctcctcctcc 240
tcatcgccgc cctcctccac caaggaggct gtgacgcagc tggatcacct ggagcaggcg 300
gcggcgtaca tcaagcagct caaggggagg atcgacgagc tgaagaagag gaagcagcag 360
gcggcggcac tcaccaccag caccagcaat ggcggcggcg gcgggatgcc ggtggtggag 420
gtgcggtgcc aggatgggac gctggacgtg gtggtggtga gcgaggcgat cagggaggag 480
agggagaggg cggtgcggct gcacgaggtg atcggcgtgc tggaggaaga aggcgcggag 540
gtggtgaacg ccagcttctc cgtcgtcggc gacaagatct tctacactct ccactcccag 600
gcgctctgct ccaggatcgg cctcgacgcc tccagggtct cccacaggct gcgcaacctc 660
ctcctccaat attaa 675

<210> 213
<211> 224
<212> PRT
<213> Oryza sativa

<400> 213
Met Lys Ser Arg Lys Asn Ser Thr Thr Ser Thr Lys Ala Ala Gly Ser
1               5                   10                  15
Cys His Thr Ser Ser Ser Gly Gly Gly Gly Gly Gly Gly Asn Cys Tyr
            20                  25                  30

Ser Ser Ser Ser Ser Lys Met Glu Arg Lys Asp Val Glu Lys Asn Arg
        35                  40                  45
Arg Leu His Met Lys Gly Leu Cys Leu Lys Leu Ser Ser Leu Ile Pro
    50                  55                  60
Ala Ala Ala Pro Arg Arg His His His His Tyr Ser Thr Ser Ser Ser
65                  70                  75                  80
Ser Ser Pro Pro Ser Ser Thr Lys Glu Ala Val Thr Gln Leu Asp His
                85                  90                  95
Leu Glu Gln Ala Ala Ala Tyr Ile Lys Gln Leu Lys Gly Arg Ile Asp
            100                 105                 110
Glu Leu Lys Lys Arg Lys Gln Gln Ala Ala Ala Leu Thr Thr Ser Thr
        115                 120                 125
Ser Asn Gly Gly Gly Gly Gly Met Pro Val Val Glu Val Arg Cys Gln
    130                 135                 140
Asp Gly Thr Leu Asp Val Val Val Val Ser Glu Ala Ile Arg Glu Glu
145                 150                 155                 160
Arg Glu Arg Ala Val Arg Leu His Glu Val Ile Gly Val Leu Glu Glu
                165                 170                 175
Glu Gly Ala Glu Val Val Asn Ala Ser Phe Ser Val Val Gly Asp Lys
            180                 185                 190
Ile Phe Tyr Thr Leu His Ser Gln Ala Leu Cys Ser Arg Ile Gly Leu
        195                 200                 205
Asp Ala Ser Arg Val Ser His Arg Leu Arg Asn Leu Leu Leu Gln Tyr
    210                 215                 220

<210>  214
<211>  696
<212>  DNA
<213>  Oryza sativa

<400>  214
atggatcagg agaaggcgga cggcggcggc aggaggagga ggagcagggc gacgagtagc      60
agcggcagcg gcgcgagcag cacggcggcg gcggcggcgg cggagaggaa ggagatggag     120
cgcaggaggc ggcaggacat gaagggcctc tgcgtcaagc tcgcctctct catccccaaa     180
gaacactgct ccatgtccaa gatgcaggcg gcgtctagga cccagctggg cagcctggac     240
gaagcggcgg cctacatcaa gaagctcaag gaaagggtgg acgagctgca ccacaagagg     300
agcatgatga gtatcacatc atcgcgctgc cgctcaggag gaggaggagg accagctgct     360
gctgctggcc agtcgacgag cggcggcggc ggcggggaag aagaagaaga agatatgacg     420
aggacgacgg cggcggcggc ggtggtggag gtgcggcagc acgtgcagga ggggtcgctg     480
atcagcttgg acgtggtgct gatctgcagc gcagcgaggc cggtcaagtt ccacgacgtc     540
atcaccgtcc tcgaggaaga aggcgccgac atcatctccg ccaacttctc cctcgccgcc     600
cacaatttct actacaccat ctactccagg gcctttagct caagaattgg catagaggct     660
tcgaggattt ctgagagact acgggcattg gtatga                               696

<210>  215
<211>  231
<212>  PRT
<213>  Oryza sativa

<400>  215
Met Asp Gln Glu Lys Ala Asp Gly Gly Gly Arg Arg Arg Arg Ser Arg
1               5                   10                  15
Ala Thr Ser Ser Ser Gly Ser Gly Ala Ser Ser Thr Ala Ala Ala Ala
            20                  25                  30
Ala Ala Glu Arg Lys Glu Met Glu Arg Arg Arg Arg Gln Asp Met Lys
        35                  40                  45
Gly Leu Cys Val Lys Leu Ala Ser Leu Ile Pro Lys Glu His Cys Ser
    50                  55                  60
Met Ser Lys Met Gln Ala Ala Ser Arg Thr Gln Leu Gly Ser Leu Asp

65                  70                  75                  80
Glu Ala Ala Ala Tyr Ile Lys Lys Leu Lys Glu Arg Val Asp Glu Leu
                85                  90                  95
His His Lys Arg Ser Met Met Ser Ile Thr Ser Ser Arg Cys Arg Ser
            100                 105                 110
Gly Gly Gly Gly Gly Pro Ala Ala Ala Ala Gly Gln Ser Thr Ser Gly
        115                 120                 125
Gly Gly Gly Gly Glu Glu Glu Glu Glu Asp Met Thr Arg Thr Thr Ala
    130                 135                 140
Ala Ala Ala Val Val Glu Val Arg Gln His Val Gln Glu Gly Ser Leu
145                 150                 155                 160
Ile Ser Leu Asp Val Val Leu Ile Cys Ser Ala Ala Arg Pro Val Lys
                165                 170                 175
Phe His Asp Val Ile Thr Val Leu Glu Glu Glu Gly Ala Asp Ile Ile
            180                 185                 190
Ser Ala Asn Phe Ser Leu Ala Ala His Asn Phe Tyr Tyr Thr Ile Tyr
        195                 200                 205
Ser Arg Ala Phe Ser Ser Arg Ile Gly Ile Glu Ala Ser Arg Ile Ser
    210                 215                 220
Glu Arg Leu Arg Ala Leu Val
225                 230

<210>  216
<211>  633
<212>  DNA
<213>  Oryza sativa

<400>  216
atggagatcc cgccgccgtc agctggtggc ggaggcggcg gcggcaagcc cgaccggaag      60
acgacggagc gcatccgccg cgagcagatg aacaagctct actcccacct cgactccctc     120
gtccgctccg ctccccccac agttaattcc attccttcac attcaaattc aaattcaaaa     180
taccatcaaa gaaaattacg gattctcggt ggggcggcgg cggcgacgac gaggccggac     240
aggttggggg tggcggcgga gtacataagg cagacgcagg agagggtgga catgctgagg     300
gagaagaaga gggagctcac cggcggcggc ggcggcggct cctcgtcgtc gtccggcgcc     360
ggggcggcca cggccgccgc gccggaggtg gaggtgcagc acctggggtc cggcctgcac     420
gccatcctct tcaccggcgc gccgcccacc gacggcgcct ccttccaccg cgccgtccgc     480
gccgtcgagg acgccggcgg ccaggtgcag aacgcgcact tctccgtcgc cggcgccaag     540
gccgtctaca ccatccacgc catgattgga gatggatatg gaggcattga gagggtggtg     600
cagagattaa aggaagcaat acggagcaac taa                                  633

<210>  217
<211>  210
<212>  PRT
<213>  Oryza sativa

<400>  217
Met Glu Ile Pro Pro Pro Ser Ala Gly Gly Gly Gly Gly Gly Gly Lys
1               5                   10                  15
Pro Asp Arg Lys Thr Thr Glu Arg Ile Arg Arg Glu Gln Met Asn Lys
            20                  25                  30
Leu Tyr Ser His Leu Asp Ser Leu Val Arg Ser Ala Pro Pro Thr Val
        35                  40                  45
Asn Ser Ile Pro Ser His Ser Asn Ser Asn Ser Lys Tyr His Gln Arg
    50                  55                  60
Lys Leu Arg Ile Leu Gly Gly Ala Ala Ala Ala Thr Thr Arg Pro Asp
65                  70                  75                  80
Arg Leu Gly Val Ala Ala Glu Tyr Ile Arg Gln Thr Gln Glu Arg Val
                85                  90                  95
Asp Met Leu Arg Glu Lys Lys Arg Glu Leu Thr Gly Gly Gly Gly Gly

        100                 105                 110
Gly Ser Ser Ser Ser Ser Gly Ala Gly Ala Ala Thr Ala Ala Ala Pro
        115                 120                 125
Glu Val Glu Val Gln His Leu Gly Ser Gly Leu His Ala Ile Leu Phe
    130                 135                 140
Thr Gly Ala Pro Pro Thr Asp Gly Ala Ser Phe His Arg Ala Val Arg
145                 150                 155                 160
Ala Val Glu Asp Ala Gly Gly Gln Val Gln Asn Ala His Phe Ser Val
                165                 170                 175
Ala Gly Ala Lys Ala Val Tyr Thr Ile His Ala Met Ile Gly Asp Gly
            180                 185                 190
Tyr Gly Gly Ile Glu Arg Val Val Gln Arg Leu Lys Glu Ala Ile Arg
        195                 200                 205
Ser Asn
    210

<210>  218
<211>  546
<212>  DNA
<213>  Arabidopsis thaliana

<400>  218
atggggagag caagagaaat aggagaagga aactcatcgt cgttaaggga acaacgaaac      60
ctcagagaga aggatcgaag gatgcgcatg aaacatctct tctctatact ttcttctcat     120
gtttctccca ctcgcaagtt accagtgcct caccttatag atcaagcgac atcatacatg     180
atccaattga aagagaatgt aaattatttg aaagagaaga aaaggacatt gttacaagga     240
gaactcggga atctctacga agggtcgttt cttctaccca aactcagtat tcgttcgcgg     300
gattcgacca tagaaatgaa tctgatcatg gatctaaaca tgaaaagagt aatgttacac     360
gagcttgtga gtatttttga agaagaagga gctcaagtaa tgagtgctaa tcttcagaac     420
ttgaatgata ggaccactta cacaatcata gcccaggcta tcattagtcg gattggcatt     480
gatccatcaa ggatagaaga gagagtacgg aaaatcatct atggatatat atattttgaa      540
gcatga                                                                546

<210>  219
<211>  188
<212>  PRT
<213>  Arabidopsis thaliana

<400>  219
Met Gly Arg Ala Arg Glu Ile Gly Glu Gly Asn Ser Ser Ser Leu Arg
1               5                   10                  15
Glu Gln Arg Asn Leu Arg Glu Lys Asp Arg Arg Met Arg Met Lys His
            20                  25                  30
Leu Phe Ser Ile Leu Ser Ser His Val Ser Pro Thr Arg Lys Leu Pro
        35                  40                  45
Val Pro His Leu Ile Asp Gln Ala Thr Ser Tyr Met Ile Gln Leu Lys
    50                  55                  60
Glu Asn Val Asn Tyr Leu Lys Glu Lys Lys Arg Thr Leu Leu Gln Gly
65                  70                  75                  80
Glu Leu Gly Asn Leu Tyr Glu Gly Ser Phe Leu Leu Pro Lys Leu Ser
                85                  90                  95
Ile Arg Ser Arg Asp Ser Thr Ile Glu Met Asn Leu Ile Met Asp Leu
            100                 105                 110
Asn Met Lys Arg Val Met Leu His Glu Leu Val Ser Ile Phe Glu Glu
        115                 120                 125
Glu Gly Ala Gln Val Met Ser Ala Asn Leu Gln Asn Leu Asn Asp Arg
    130                 135                 140
Thr Thr Tyr Thr Ile Ile Ala Gln Val Pro His Pro His Ala Tyr Ala
145                 150                 155                 160

Ile Ile Ser Arg Ile Gly Ile Asp Pro Ser Arg Ile Glu Glu Arg Val
                165                 170                 175
Arg Lys Ile Ile Tyr Gly Tyr Ile Tyr Phe Glu Ala
            180                 185

<210>  220
<211>  525
<212>  DNA
<213>  Arabidopsis thaliana

<400>  220
atggaaagag cgagagaaat aggagaagga agcgcatcgt cattacggga acaacgaaac      60
ctcagagaga aagaacgacg aatgcgcatg aaacatctct tctccatact ctcttctcat     120
gtttctccca ctcgtaggtt gccagtgcct caacttatag atcaagcggt atcatacatg     180
atccaactga aagagaaggt aaactatttg aatgagatga aaaggagaat gttaggagga     240
gaagtcaaaa atcgctctga agggtcgtct cttctgccaa aactcagtat tcgttcactg     300
gattcgatca tagaaatgaa tctggttatg gatctaaaca tgaaaggagt aatgttacac     360
aagcttgtga gtgtttttga agaagaagga gctcaagtga tgagtgctaa tcttcagaac     420
ttgaatgata ggacctttta tacaatcata gcccaggcta tcatatgtcg gatcgggatt     480
gatccatcaa ggatagaaga gagattaagg gatataatct catga                     525

<210>  221
<211>  174
<212>  PRT
<213>  Arabidopsis thaliana

<400>  221
Met Glu Arg Ala Arg Glu Ile Gly Glu Gly Ser Ala Ser Ser Leu Arg
1               5                   10                  15
Glu Gln Arg Asn Leu Arg Glu Lys Glu Arg Arg Met Arg Met Lys His
            20                  25                  30
Leu Phe Ser Ile Leu Ser Ser His Val Ser Pro Thr Arg Arg Leu Pro
        35                  40                  45
Val Pro Gln Leu Ile Asp Gln Ala Val Ser Tyr Met Ile Gln Leu Lys
    50                  55                  60
Glu Lys Val Asn Tyr Leu Asn Glu Met Lys Arg Arg Met Leu Gly Gly
65                  70                  75                  80
Glu Val Lys Asn Arg Ser Glu Gly Ser Ser Leu Leu Pro Lys Leu Ser
                85                  90                  95
Ile Arg Ser Leu Asp Ser Ile Ile Glu Met Asn Leu Val Met Asp Leu
            100                 105                 110
Asn Met Lys Gly Val Met Leu His Lys Leu Val Ser Val Phe Glu Glu
        115                 120                 125
Glu Gly Ala Gln Val Met Ser Ala Asn Leu Gln Asn Leu Asn Asp Arg
    130                 135                 140
Thr Phe Tyr Thr Ile Ile Ala Gln Ala Ile Ile Cys Arg Ile Gly Ile
145                 150                 155                 160
Asp Pro Ser Arg Ile Glu Glu Arg Leu Arg Asp Ile Ile Ser
                165                 170

<210>  222
<211>  369
<212>  DNA
<213>  Arabidopsis thaliana

<400>  222
atgatccaat tgaaagagaa tgtaaattat ttgaaagaga agaaaaggac attgttacaa      60
ggagaactcg ggaatctcta cgaagggtcg tttcttctac ccaaactcag tattcgttcg     120
cgggattcga ccatagaaat gaatctgatc atggatctaa acatgaaaag agtaatgtta     180

cacgagcttg tgagtatttt tgaagaagaa ggagctcaag taatgagtgc taatcttcag 240
aacttgaatg ataggaccac ttacacaatc atagcccagg ctatcattag tcggattggc 300
attgatccat caaggataga agagagagta cggaaaatca tctatggata tatatatttt 360
gaagcatga 369

<210> 223
<211> 122
<212> PRT
<213> Arabidopsis thaliana

<400> 223
Met Ile Gln Leu Lys Glu Asn Val Asn Tyr Leu Lys Glu Lys Lys Arg
1               5                   10                  15
Thr Leu Leu Gln Gly Glu Leu Gly Asn Leu Tyr Glu Gly Ser Phe Leu
            20                  25                  30
Leu Pro Lys Leu Ser Ile Arg Ser Arg Asp Ser Thr Ile Glu Met Asn
        35                  40                  45
Leu Ile Met Asp Leu Asn Met Lys Arg Val Met Leu His Glu Leu Val
    50                  55                  60
Ser Ile Phe Glu Glu Glu Gly Ala Gln Val Met Ser Ala Asn Leu Gln
65                  70                  75                  80
Asn Leu Asn Asp Arg Thr Thr Tyr Thr Ile Ile Ala Gln Ala Ile Ile
                85                  90                  95
Ser Arg Ile Gly Ile Asp Pro Ser Arg Ile Glu Glu Arg Val Arg Lys
            100                 105                 110
Ile Ile Tyr Gly Tyr Ile Tyr Phe Glu Ala
        115                 120

<210> 224
<211> 573
<212> DNA
<213> Arabidopsis thaliana

<400> 224
atggagccga gccattcaaa cacaggtcaa tcaagatctg tagatcgcaa aacggttgag 60
aaaaatagaa ggatgcaaat gaagtctctc tactcagaac tcatctctct tcttcctcat 120
cattcttcta cggagccttt aacactacct gatcagctag atgaagctgc aaactacatc 180
aagaagctac aagtgaacgt ggagaaaaag agagaaagga aaaggaacct cgttgcgact 240
acaactttgg agaaactgaa ttccgtcgga tcttcatcgg tttcgtcgag cgtcgatgtc 300
tccgtgccaa gaaagctgcc aaaaatcgag attcaagaaa ctggttccat ttttcacatc 360
tttcttgtga caagcttgga acacaagttt atgttttgtg agatcattcg tgttctcacc 420
gaggaattag gagctgagat cactcatgct ggatactcca ttgttgatga tgctgtcttc 480
cacacccttc actgcaaggt ggaagaacac gattatggag ctaggagtca aattcctgaa 540
agactggaga agattgtgaa cagtgttcac taa 573

<210> 225
<211> 190
<212> PRT
<213> Arabidopsis thaliana

<400> 225
Met Glu Pro Ser His Ser Asn Thr Gly Gln Ser Arg Ser Val Asp Arg
1               5                   10                  15
Lys Thr Val Glu Lys Asn Arg Arg Met Gln Met Lys Ser Leu Tyr Ser
            20                  25                  30
Glu Leu Ile Ser Leu Leu Pro His His Ser Ser Thr Glu Pro Leu Thr
        35                  40                  45
Leu Pro Asp Gln Leu Asp Glu Ala Ala Asn Tyr Ile Lys Lys Leu Gln
    50                  55                  60

Val Asn Val Glu Lys Lys Arg Glu Arg Lys Arg Asn Leu Val Ala Thr
65                  70                  75                  80
Thr Thr Leu Glu Lys Leu Asn Ser Val Gly Ser Ser Ser Val Ser Ser
                85                  90                  95
Ser Val Asp Val Ser Val Pro Arg Lys Leu Pro Lys Ile Glu Ile Gln
            100                 105                 110
Glu Thr Gly Ser Ile Phe His Ile Phe Leu Val Thr Ser Leu Glu His
        115                 120                 125
Lys Phe Met Phe Cys Glu Ile Ile Arg Val Leu Thr Glu Glu Leu Gly
    130                 135                 140
Ala Glu Ile Thr His Ala Gly Tyr Ser Ile Val Asp Asp Ala Val Phe
145                 150                 155                 160
His Thr Leu His Cys Lys Val Glu Glu His Asp Tyr Gly Ala Arg Ser
                165                 170                 175
Gln Ile Pro Glu Arg Leu Glu Lys Ile Val Asn Ser Val His
            180                 185                 190

<210>  226
<211>  423
<212>  DNA
<213>  Medicago truncatula

<400>  226
atgacatctc taacaaagga ggcgatttca gtgccggatc agctaaagga agcaacaaac      60
tacataaaga aattgcagat caacctggag aaaatgaagg aaaagaagaa ttttctacta     120
ggaattcaaa ggccaaatgt gaatttgaat agaaaccaga agatgggatt aaagtctcca     180
aaaattaaga tacaacaaat tggtttagtc ttagaggttg ttctaataac tggattggag     240
tctcagtttt tgttcagcga aacctttcga gttcttcatg aagaaggagt tgatattgtt     300
aatgctagtt ataaggtcaa tgaagattct gttttccatt caatacactg ccaggtagga     360
gaatttggca atgaagctgc aagaatatct gagagattga agaagtttat gcaagactat     420
tag                                                                   423

<210>  227
<211>  140
<212>  PRT
<213>  Medicago truncatula

<400>  227
Met Thr Ser Leu Thr Lys Glu Ala Ile Ser Val Pro Asp Gln Leu Lys
1               5                   10                  15
Glu Ala Thr Asn Tyr Ile Lys Lys Leu Gln Ile Asn Leu Glu Lys Met
            20                  25                  30
Lys Glu Lys Lys Asn Phe Leu Leu Gly Ile Gln Arg Pro Asn Val Asn
        35                  40                  45
Leu Asn Arg Asn Gln Lys Met Gly Leu Lys Ser Pro Lys Ile Lys Ile
    50                  55                  60
Gln Gln Ile Gly Leu Val Leu Glu Val Val Leu Ile Thr Gly Leu Glu
65                  70                  75                  80
Ser Gln Phe Leu Phe Ser Glu Thr Phe Arg Val Leu His Glu Glu Gly
                85                  90                  95
Val Asp Ile Val Asn Ala Ser Tyr Lys Val Asn Glu Asp Ser Val Phe
            100                 105                 110
His Ser Ile His Cys Gln Val Gly Glu Phe Gly Asn Glu Ala Ala Arg
        115                 120                 125
Ile Ser Glu Arg Leu Lys Lys Phe Met Gln Asp Tyr
    130                 135                 140

<210>  228
<211>  882

<212>  DNA
<213>  Hordeum vulgare

<400>  228
atgaatgcct gcgctctgaa ctcgatggag ccggtgaagg cgaagccggc gaggggcggg      60
aagaggagca gggagagcgg cggcacggcg gtggtgctgc tggagaagaa ggagtcggag     120
aaggagagga ggaagcgcat gaaggcgctc tgcgagaagc tcgcatccct catcccaagg     180
gaacactgct gctccaccac tgatacaatg acccagctag gcagcctgga tgtgggggca     240
tcgtatatca agaagctgaa ggagagggtc gatgagctac aacgtaggat gacctctgcg     300
cagaccttgg ataccttcag aggagacact agcatcccaa cgcccactac caccactacc     360
acgaaaagcg ttgtagggtc gctggaagaa gagaaagctc gggaggcatc ggcacccgta     420
ttgcaggtgc ggcaacacga cgattcaagc atggaggtga gattgatatg ctgcatgaag     480
aggccgatca agctccatga ggtgatcacc atccatgagg aagaaggtgc tgagatcatc     540
aacgccaatc actctgttgc tggccaaaaa atgttctaca ctatacactc tcgggcctct     600
agctcgagaa ttggcataga tgttccaagg gtttctgaac gactgcgagc attgctccaa     660
ctttattcgc atgaaaatca ggcaccgtcg tgcatgcaca acaatcaagt cctcaggtac     720
aatgatgggg ccaacgcgac tcctcccaag gagctcgtcg gcaacaatga tataggtgga     780
accaacaagg tggcaaacca cgagtgtgag tcttgggttg agcaagacca agtgatgtgg     840
agttccctgc ttgcgtcaat atcaccagag ttgctcgagt ag                        882

<210>  229
<211>  293
<212>  PRT
<213>  Hordeum vulgare

<400>  229
Met Asn Ala Cys Ala Leu Asn Ser Met Glu Pro Val Lys Ala Lys Pro
1               5                   10                  15
Ala Arg Gly Gly Lys Arg Ser Arg Glu Ser Gly Gly Thr Ala Val Val
            20                  25                  30
Leu Leu Glu Lys Lys Glu Ser Glu Lys Glu Arg Arg Lys Arg Met Lys
        35                  40                  45
Ala Leu Cys Glu Lys Leu Ala Ser Leu Ile Pro Arg Glu His Cys Cys
    50                  55                  60
Ser Thr Thr Asp Thr Met Thr Gln Leu Gly Ser Leu Asp Val Gly Ala
65                  70                  75                  80
Ser Tyr Ile Lys Lys Leu Lys Glu Arg Val Asp Glu Leu Gln Arg Arg
                85                  90                  95
Met Thr Ser Ala Gln Thr Leu Asp Thr Phe Arg Gly Asp Thr Ser Ile
            100                 105                 110
Pro Thr Pro Thr Thr Thr Thr Thr Thr Lys Ser Val Val Gly Ser Leu
        115                 120                 125
Glu Glu Glu Lys Ala Arg Glu Ala Ser Ala Pro Val Leu Gln Val Arg
    130                 135                 140
Gln His Asp Asp Ser Ser Met Glu Val Arg Leu Ile Cys Cys Met Lys
145                 150                 155                 160
Arg Pro Ile Lys Leu His Glu Val Ile Thr Ile His Glu Glu Glu Gly
                165                 170                 175
Ala Glu Ile Ile Asn Ala Asn His Ser Val Ala Gly Gln Lys Met Phe
            180                 185                 190
Tyr Thr Ile His Ser Arg Ala Ser Ser Ser Arg Ile Gly Ile Asp Val
        195                 200                 205
Pro Arg Val Ser Glu Arg Leu Arg Ala Leu Leu Gln Leu Tyr Ser His
    210                 215                 220
Glu Asn Gln Ala Pro Ser Cys Met His Asn Asn Gln Val Leu Arg Tyr
225                 230                 235                 240
Asn Asp Gly Ala Asn Ala Thr Pro Pro Lys Glu Leu Val Gly Asn Asn
                245                 250                 255
Asp Ile Gly Gly Thr Asn Lys Val Ala Asn His Glu Cys Glu Ser Trp

260                 265                 270
Val Glu Gln Asp Gln Val Met Trp Ser Ser Leu Leu Ala Ser Ile Ser
        275                 280                 285
Pro Glu Leu Leu Glu
    290

<210>  230
<211>  2193
<212>  DNA
<213>  Oryza sativa

<400>  230
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga attttttttc ccaaatatct taaaaagtca    1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccatttttaa ttatatgaaa tgaactgtag cataagcagt attcatttgg    1920
attatttttt ttattagctc tcaccccttc attattctga gctgaaagtc tggcatgaac    1980
tgtcctcaat tttgttttca aattcacatc gattatctat gcattatcct cttgtatcta    2040
cctgtagaag tttctttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193

<210>  231
<211>  56
<212>  DNA
<213>  Artificial sequence

<220>
<223>  primer: prm06808

<400>  231

ggggacaagt ttgtacaaaa aagcaggctt aaacaatgaa gagcaggaag aacagc 56

<210> 232
<211> 50
<212> DNA
<213> Artificial sequence

<220>
<223> primer: prm06809

<400> 232
ggggaccact ttgtacaaga aagctgggtg cagagtgaaa gagtggtgtg 50

<210> 233
<211> 2194
<212> DNA
<213> Oryza sativa

<400> 233
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 234
<211> 1065

<212>  DNA
<213>  Arabidopsis thaliana

<400>  234
atggagttgt taatgtgttc gggtcaggcc gagtcaggtg gttcctcttc caccgagtct      60
tcttcactca gtggtggact caggtttggt cagaagatct acttcgagga tggatccgga     120
tccagaagca agaaccgggt caataccgtt cgtaagtcgt ctaccacggc gaggtgccaa     180
gtggaaggtt gtagaatgga tctaagcaat gttaaagctt attactcgag acacaaagtt     240
tgttgcattc actctaaatc atctaaagtc attgtctctg gtcttcatca aaggttttgt     300
caacaatgta gcaggtttca ccagctttct gagtttgact tggagaaaag aagttgtcgc     360
agaagactcg cttgtcataa cgaacgacga agaaaaccac aacccacaac ggctcttttc     420
acttctcatt actctcgaat cgctccatct ctttacggaa accccaatgc tgcaatgatt     480
aaaagcgttt tgggagatcc tactgcgtgg tcaaccgcaa gatcagtgat gcagcggcct     540
ggaccgtggc agattaatcc agttagggaa acccatccac acatgaatgt tttatcacat     600
ggaagctcaa gctttactac atgtccagag atgataaaca acaatagcac agattcaagc     660
tgtgctctct ctcttctgtc aaactcatac ccaattcatc agcagcaact tcagacacca     720
acaaatacat ggcgaccatc ttctggtttc gactcgatga tctcattctc cgataaggtt     780
acaatggctc agccaccgcc catttcaacc catcagccgc ccatctcaac acatcagcag     840
tacctcagcc aaacttggga agtcatcgcg ggcgaaaaga gcaattcaca ttatatgtct     900
cctgtgagtc aaatctcgga gccagcagat ttccagataa gcaatggcac cacaatgggt     960
ggatttgagc tgtatcttca tcagcaggtt ctgaagcaat acatggaacc cgagaacaca    1020
agagcttatg actcctctcc tcaacatttc aattggtctc tttga                    1065

<210>  235
<211>  354
<212>  PRT
<213>  Arabidopsis thaliana

<400>  235
Met Glu Leu Leu Met Cys Ser Gly Gln Ala Glu Ser Gly Gly Ser Ser
1               5                   10                  15
Ser Thr Glu Ser Ser Ser Leu Ser Gly Gly Leu Arg Phe Gly Gln Lys
            20                  25                  30
Ile Tyr Phe Glu Asp Gly Ser Gly Ser Arg Ser Lys Asn Arg Val Asn
        35                  40                  45
Thr Val Arg Lys Ser Ser Thr Thr Ala Arg Cys Gln Val Glu Gly Cys
    50                  55                  60
Arg Met Asp Leu Ser Asn Val Lys Ala Tyr Tyr Ser Arg His Lys Val
65                  70                  75                  80
Cys Cys Ile His Ser Lys Ser Ser Lys Val Ile Val Ser Gly Leu His
                85                  90                  95
Gln Arg Phe Cys Gln Gln Cys Ser Arg Phe His Gln Leu Ser Glu Phe
            100                 105                 110
Asp Leu Glu Lys Arg Ser Cys Arg Arg Arg Leu Ala Cys His Asn Glu
        115                 120                 125
Arg Arg Arg Lys Pro Gln Pro Thr Thr Ala Leu Phe Thr Ser His Tyr
    130                 135                 140
Ser Arg Ile Ala Pro Ser Leu Tyr Gly Asn Pro Asn Ala Ala Met Ile
145                 150                 155                 160
Lys Ser Val Leu Gly Asp Pro Thr Ala Trp Ser Thr Ala Arg Ser Val
                165                 170                 175
Met Gln Arg Pro Gly Pro Trp Gln Ile Asn Pro Val Arg Glu Thr His
            180                 185                 190
Pro His Met Asn Val Leu Ser His Gly Ser Ser Ser Phe Thr Thr Cys
        195                 200                 205
Pro Glu Met Ile Asn Asn Asn Ser Thr Asp Ser Ser Cys Ala Leu Ser
    210                 215                 220
Leu Leu Ser Asn Ser Tyr Pro Ile His Gln Gln Gln Leu Gln Thr Pro
225                 230                 235                 240

Thr Asn Thr Trp Arg Pro Ser Ser Gly Phe Asp Ser Met Ile Ser Phe
                245                 250                 255
Ser Asp Lys Val Thr Met Ala Gln Pro Pro Pro Ile Ser Thr His Gln
                260                 265                 270
Pro Pro Ile Ser Thr His Gln Gln Tyr Leu Ser Gln Thr Trp Glu Val
        275                 280                 285
Ile Ala Gly Glu Lys Ser Asn Ser His Tyr Met Ser Pro Val Ser Gln
    290                 295                 300
Ile Ser Glu Pro Ala Asp Phe Gln Ile Ser Asn Gly Thr Thr Met Gly
305                 310                 315                 320
Gly Phe Glu Leu Tyr Leu His Gln Gln Val Leu Lys Gln Tyr Met Glu
                325                 330                 335
Pro Glu Asn Thr Arg Ala Tyr Asp Ser Ser Pro Gln His Phe Asn Trp
                340                 345                 350
Ser Leu

<210>  236
<211>  1128
<212>  DNA
<213>  Arabidopsis thaliana

<400>  236
atggagatgg gttccaactc gggtccgggt catggtccgg gtcaggcaga gtcgggtggt      60
tcctccactg agtcatcctc tttcagtgga gggctcatgt ttggccagaa gatctacttc     120
gaggacggtg gtggtggatc cgggtcttct tcctcaggtg gtcgttcaaa cagacgtgtc     180
cgtggaggcg ggtcgggtca gtcgggtcag ataccaaggt gccaagtgga aggttgtggg     240
atggatctaa ccaatgcaaa aggttattac tcgagacacc gagtttgtgg agtgcactct     300
aaaacaccta aagtcactgt ggctggtatc gaacagaggt tttgtcaaca gtgcagcagg     360
tttcatcagc ttccggaatt tgacctagag aaaaggagtt gccgcaggag actcgctggt     420
cataatgagc gacgaaggaa gccacagcct gcgtctctct ctgtgttagc ttctcgttac     480
gggaggatcg caccttcgct ttacgaaaat ggtgatgctg gaatgaatgg aagctttctt     540
gggaaccaag agataggatg gccaagttca agaacattgg atacaagagt gatgaggcgg     600
ccagtgtcgt caccgtcatg gcagatcaat ccaatgaatg tatttagtca aggttcagtt     660
ggtggaggag ggacaagctt ctcatctcca gagattatgg acactaaact agagagctac     720
aagggaattg gcgactcaaa ctgtgctctc tctcttctgt caaatccaca tcaaccacat     780
gacaacaaca acaacaacaa caacaacaac aacaacaaca acaatacatg gcgagcttct     840
tcaggttttg gcccgatgac ggttacaatg gctcaaccac cacctgcacc tagccagcat     900
cagtatctga acccgccttg ggtattcaag gacaatgata atgatatgtc tcctgttttg     960
aatttaggtc gatacaccga gccagataat tgtcagataa gtagtggcac ggcaatgggt    1020
gagttcgagt tatctgatca ccatcatcaa agtaggagac agtacatgga agatgagaac    1080
acaagggctt atgactcttc ttctcaccat accaactggt ccctctga                 1128

<210>  237
<211>  375
<212>  PRT
<213>  Arabidopsis thaliana

<400>  237
Met Glu Met Gly Ser Asn Ser Gly Pro Gly His Gly Pro Gly Gln Ala
1               5                   10                  15
Glu Ser Gly Gly Ser Ser Thr Glu Ser Ser Ser Phe Ser Gly Gly Leu
            20                  25                  30
Met Phe Gly Gln Lys Ile Tyr Phe Glu Asp Gly Gly Gly Gly Ser Gly
        35                  40                  45
Ser Ser Ser Ser Gly Gly Arg Ser Asn Arg Arg Val Arg Gly Gly Gly
    50                  55                  60
Ser Gly Gln Ser Gly Gln Ile Pro Arg Cys Gln Val Glu Gly Cys Gly
65                  70                  75                  80

Met Asp Leu Thr Asn Ala Lys Gly Tyr Tyr Ser Arg His Arg Val Cys
                85                  90                  95
Gly Val His Ser Lys Thr Pro Lys Val Thr Val Ala Gly Ile Glu Gln
            100                 105                 110
Arg Phe Cys Gln Gln Cys Ser Arg Phe His Gln Leu Pro Glu Phe Asp
        115                 120                 125
Leu Glu Lys Arg Ser Cys Arg Arg Arg Leu Ala Gly His Asn Glu Arg
    130                 135                 140
Arg Arg Lys Pro Gln Pro Ala Ser Leu Ser Val Leu Ala Ser Arg Tyr
145                 150                 155                 160
Gly Arg Ile Ala Pro Ser Leu Tyr Glu Asn Gly Asp Ala Gly Met Asn
                165                 170                 175
Gly Ser Phe Leu Gly Asn Gln Glu Ile Gly Trp Pro Ser Ser Arg Thr
            180                 185                 190
Leu Asp Thr Arg Val Met Arg Arg Pro Val Ser Ser Pro Ser Trp Gln
        195                 200                 205
Ile Asn Pro Met Asn Val Phe Ser Gln Gly Ser Val Gly Gly Gly Gly
    210                 215                 220
Thr Ser Phe Ser Ser Pro Glu Ile Met Asp Thr Lys Leu Glu Ser Tyr
225                 230                 235                 240
Lys Gly Ile Gly Asp Ser Asn Cys Ala Leu Ser Leu Leu Ser Asn Pro
                245                 250                 255
His Gln Pro His Asp Asn Asn Asn Asn Asn Asn Asn Asn Asn Asn Asn
            260                 265                 270
Asn Asn Asn Thr Trp Arg Ala Ser Ser Gly Phe Gly Pro Met Thr Val
        275                 280                 285
Thr Met Ala Gln Pro Pro Pro Ala Pro Ser Gln His Gln Tyr Leu Asn
    290                 295                 300
Pro Pro Trp Val Phe Lys Asp Asn Asp Asn Asp Met Ser Pro Val Leu
305                 310                 315                 320
Asn Leu Gly Arg Tyr Thr Glu Pro Asp Asn Cys Gln Ile Ser Ser Gly
                325                 330                 335
Thr Ala Met Gly Glu Phe Glu Leu Ser Asp His His His Gln Ser Arg
            340                 345                 350
Arg Gln Tyr Met Glu Asp Glu Asn Thr Arg Ala Tyr Asp Ser Ser Ser
        355                 360                 365
His His Thr Asn Trp Ser Leu
    370                 375

<210>  238
<211>  1149
<212>  DNA
<213>  Aquilegia formosa x Aquilegia pubescens

<400>  238
atggaaatgg gttccagctc tttcgctggt ggtgggggta agggtgcttc tggttcatct      60
gattcttcac tgaatggttt gaaatttggg aagaaaatct actttgaaga tgtgggtatt     120
ggagttttag gcaagtcaag tgttgggtct ccgtcaattt tggtttctga agctcggttg     180
ccaccggctt cgtcggtgaa gaagggtaga ggggttttgc aaggacaacc acctagatgt     240
tctgttgaag gttgtaagct tgatcttact gatgctaagc cttattactc aaggcacaaa     300
gtctgtggta tgcactctaa atctcctaaa gtaattgttg gtggtttgga gcagaggttt     360
tgccagcagt gtagcagatt tcatctactc tgtgaatttg accaaggcaa gcgaagctgt     420
cgtagacgtc tagctggcca caatgagcgt cgaagaaaac cacaacctgg atcaatattt     480
tcaccgcgct atggtcgtgt gtcaccatct ttccaagaaa atagcaccag aggaggaggt     540
tttctaatgg acttcacagc gtacccaagg ctggcaggaa gggatgcatg gcaaacagta     600
aaagccggca attgggcaga tggaaaccaa acctctccta ttaagaagct tctcccacat     660
caatggcaag gcaattcaga gaatcctcct cctattgtct attctcaggc acctcacccg     720
tatctgcaag gtgtggctag tggatcaatt ttttccagta caagaatacc ttcagccgag     780
tgttttgctg gtgtctctga ctccagctgt gctctctctc ttctgtcaaa tcaaccatgg     840

aaccccagaa accagacttc cgatttggag gtgaataaca tagtgaacgg tgaaggggta 900
tccatggcaa gatctatagc acctcatagt gctgtagtca ccaacttcac gaacaacaca 960
tggaatttta agggcaacag tgaagttagt ggcagttccc atgaaattcc acgtcaggtg 1020
tcgcagccag tcaccaatca tttctccagt gagctcgatc tagctcagca ggggaacagg 1080
cagtacatgg agatccggca ttcaagggat tttggttctt ccactcacca gatgcactgg 1140
tctctttga 1149

<210> 239
<211> 382
<212> PRT
<213> Aquilegia formosa x Aquilegia pubescens

<400> 239
Met Glu Met Gly Ser Ser Ser Phe Ala Gly Gly Gly Gly Lys Gly Ala
1               5                   10                  15
Ser Gly Ser Ser Asp Ser Ser Leu Asn Gly Leu Lys Phe Gly Lys Lys
            20                  25                  30
Ile Tyr Phe Glu Asp Val Gly Ile Gly Val Leu Gly Lys Ser Ser Val
        35                  40                  45
Gly Ser Pro Ser Ile Leu Val Ser Glu Ala Arg Leu Pro Pro Ala Ser
    50                  55                  60
Ser Val Lys Lys Gly Arg Gly Val Leu Gln Gly Gln Pro Pro Arg Cys
65                  70                  75                  80
Ser Val Glu Gly Cys Lys Leu Asp Leu Thr Asp Ala Lys Pro Tyr Tyr
                85                  90                  95
Ser Arg His Lys Val Cys Gly Met His Ser Lys Ser Pro Lys Val Ile
            100                 105                 110
Val Gly Gly Leu Glu Gln Arg Phe Cys Gln Gln Cys Ser Arg Phe His
        115                 120                 125
Leu Leu Cys Glu Phe Asp Gln Gly Lys Arg Ser Cys Arg Arg Arg Leu
    130                 135                 140
Ala Gly His Asn Glu Arg Arg Arg Lys Pro Gln Pro Gly Ser Ile Phe
145                 150                 155                 160
Ser Pro Arg Tyr Gly Arg Val Ser Pro Ser Phe Gln Glu Asn Ser Thr
                165                 170                 175
Arg Gly Gly Gly Phe Leu Met Asp Phe Thr Ala Tyr Pro Arg Leu Ala
            180                 185                 190
Gly Arg Asp Ala Trp Gln Thr Val Lys Ala Gly Asn Trp Ala Asp Gly
        195                 200                 205
Asn Gln Thr Ser Pro Ile Lys Lys Leu Leu Pro His Gln Trp Gln Gly
    210                 215                 220
Asn Ser Glu Asn Pro Pro Pro Ile Val Tyr Ser Gln Ala Pro His Pro
225                 230                 235                 240
Tyr Leu Gln Gly Val Ala Ser Gly Ser Ile Phe Ser Ser Thr Arg Ile
                245                 250                 255
Pro Ser Ala Glu Cys Phe Ala Gly Val Ser Asp Ser Ser Cys Ala Leu
            260                 265                 270
Ser Leu Leu Ser Asn Gln Pro Trp Asn Pro Arg Asn Gln Thr Ser Asp
        275                 280                 285
Leu Glu Val Asn Asn Ile Val Asn Gly Glu Gly Val Ser Met Ala Arg
    290                 295                 300
Ser Ile Ala Pro His Ser Ala Val Val Thr Asn Phe Thr Asn Asn Thr
305                 310                 315                 320
Trp Asn Phe Lys Gly Asn Ser Glu Val Ser Gly Ser Ser His Glu Ile
                325                 330                 335
Pro Arg Gln Val Ser Gln Pro Val Thr Asn His Phe Ser Ser Glu Leu
            340                 345                 350
Asp Leu Ala Gln Gln Gly Asn Arg Gln Tyr Met Glu Ile Arg His Ser
        355                 360                 365

Arg Asp Phe Gly Ser Ser Thr His Gln Met His Trp Ser Leu
    370                 375                 380

<210> 240
<211> 1170
<212> DNA
<213> Gossypium hirsutum

<400> 240
atggaaatgg gttcgggctc tttgactgag tcgggcggtt ctcccaccaa ctcttccgcc 60
gagtcactca acggcttgaa gttcggtaaa aagatctact ttgaggatac agccgctgtc 120
gccgccgccg ctggtggtaa aagtgttggt ggtggtgcaa acatagggac tccatccaag 180
tccggtccag catcttcaag cttagccggg tcgtgtagga aagccagggt tgatggaatg 240
gcgcaagggg ttctgccttc taggtgtcaa gtagaagggt gtaaagtgga tctgagtgat 300
gctaaggctt actattcaag gcataaggtt tgttgtatgc actctaagtc atctaaagtc 360
attgttgctg gtctcgagca aagattttgt cagcagtgta gcagatttca tcagctttct 420
gaatttgaca aagggaaacg gagttgtcgt agacggcttg caggtcacaa tgagcgacgc 480
aggaaaccac cacctggatc attattttcc tctccttatg gccggctttc ttcctctatt 540
attgaaaatg gcagtagagg tggaagcttt atagtggatt tctcagcata cccaaggctt 600
tcaggaaggg atgcatggcc agcagctcga tcgttagaat gcataactgg aaatcgaagc 660
acagccactg gaagctcatt ttcacatcca cggcaaaaca actccagcaa acctcctcat 720
gaccatttct tgcaaggttc accatgtggg actagtttct ccagcactgg aatttctcca 780
ggagaatgct tcacagggtc tggtgactca agctgtgctc tctctcttct gtcaaatcaa 840
ccgtggggct ccaggaacca ggctttgaat ttttctgtaa atggcgtgat aagtactgaa 900
gggtcctctg cggcacaacc aacaacgctt catggtgcag ttgtgaaccc ttattcaaat 960
gcctctttgg atttcaatgg cagtgacact gttcgcagtt ctcacaagat gctgccacac 1020
ctagatttgg gtcaaatccc agaccctgtt aactgtcaat tctctagtga ccttgagttg 1080
tctcaacaaa gctggaggtc atatatcgaa catgagcagt ccggggcagc ctatgacgac 1140
tccatgcagc atatccactg gacgctctaa 1170

<210> 241
<211> 389
<212> PRT
<213> Gossypium hirsutum

<400> 241
Met Glu Met Gly Ser Gly Ser Leu Thr Glu Ser Gly Gly Ser Pro Thr
1               5                   10                  15
Asn Ser Ser Ala Glu Ser Leu Asn Gly Leu Lys Phe Gly Lys Lys Ile
            20                  25                  30
Tyr Phe Glu Asp Thr Ala Ala Val Ala Ala Ala Ala Gly Gly Lys Ser
        35                  40                  45
Val Gly Gly Gly Ala Asn Ile Gly Thr Pro Ser Lys Ser Gly Pro Ala
    50                  55                  60
Ser Ser Ser Leu Ala Gly Ser Cys Arg Lys Ala Arg Val Asp Gly Met
65                  70                  75                  80
Ala Gln Gly Val Leu Pro Ser Arg Cys Gln Val Glu Gly Cys Lys Val
                85                  90                  95
Asp Leu Ser Asp Ala Lys Ala Tyr Tyr Ser Arg His Lys Val Cys Cys
            100                 105                 110
Met His Ser Lys Ser Ser Lys Val Ile Val Ala Gly Leu Glu Gln Arg
        115                 120                 125
Phe Cys Gln Gln Cys Ser Arg Phe His Gln Leu Ser Glu Phe Asp Lys
    130                 135                 140
Gly Lys Arg Ser Cys Arg Arg Arg Leu Ala Gly His Asn Glu Arg Arg
145                 150                 155                 160
Arg Lys Pro Pro Pro Gly Ser Leu Phe Ser Ser Pro Tyr Gly Arg Leu
                165                 170                 175
Ser Ser Ser Ile Ile Glu Asn Gly Ser Arg Gly Gly Ser Phe Ile Val

180                 185                 190
Asp Phe Ser Ala Tyr Pro Arg Leu Ser Gly Arg Asp Ala Trp Pro Ala
        195                 200                 205
Ala Arg Ser Leu Glu Cys Ile Thr Gly Asn Arg Ser Thr Ala Thr Gly
    210                 215                 220
Ser Ser Phe Ser His Pro Arg Gln Asn Asn Ser Ser Lys Pro Pro His
225                 230                 235                 240
Asp His Phe Leu Gln Gly Ser Pro Cys Gly Thr Ser Phe Ser Ser Thr
                245                 250                 255
Gly Ile Ser Pro Gly Glu Cys Phe Thr Gly Ser Gly Asp Ser Ser Cys
            260                 265                 270
Ala Leu Ser Leu Leu Ser Asn Gln Pro Trp Gly Ser Arg Asn Gln Ala
        275                 280                 285
Leu Asn Phe Ser Val Asn Gly Val Ile Ser Thr Glu Gly Ser Ser Ala
    290                 295                 300
Ala Gln Pro Thr Thr Leu His Gly Ala Val Val Asn Pro Tyr Ser Asn
305                 310                 315                 320
Ala Ser Leu Asp Phe Asn Gly Ser Asp Thr Val Arg Ser Ser His Lys
                325                 330                 335
Met Leu Pro His Leu Asp Leu Gly Gln Ile Pro Asp Pro Val Asn Cys
            340                 345                 350
Gln Phe Ser Ser Asp Leu Glu Leu Ser Gln Gln Ser Trp Arg Ser Tyr
        355                 360                 365
Ile Glu His Glu Gln Ser Gly Ala Ala Tyr Asp Asp Ser Met Gln His
    370                 375                 380
Ile His Trp Thr Leu
385

<210>  242
<211>  1017
<212>  DNA
<213>  Ipomoea nil

<400>  242
atggaactgg gttcttcttc ttcttctacc tctacctccg ccgactcctc atccgacggc      60
ttgaagttcg gcaaaaaagt ctactttgaa gatgcgggtg gtgggagtgg tgggtcgtcg     120
ctgccggcca agagagggag gagcgcggtg gcgcaaggcg gacagccacc caggtgccag     180
gtggaagggt gcaaggcaga tctgagtgaa tttaaggctt attactcaaa gcataaagtt     240
tgtggtatgc actccaagtc tcctaaggtc attgtttctg ggcttgaaca gagattctgc     300
cagcagtgca gcaggtttca tcaattgagt gaatttgatc aagtaaaaag gagctgccgt     360
aggcgtttgg ctggtcataa tgagcgtcgt aggaagcccc cacttggatc catattgtcc     420
acacattatg ggactctttc ttcttcaatg tttggaaaca atggccactt tgtgatggat     480
ttcgcctcat acccatatcc gggtggtaag ggctcatggc ggccaagtac caatggcgtg     540
gggaactttc ttcaacaacc atggcagaga aactctgaag atcccccacc caagcttctt     600
ttgctaggtt cggatgctgc tgctagggct acttatccca gtccttgtgg agaatacttc     660
aatggggtct cggattccac ccgtgctctc tctcttctgt caaactcaaa tcagccctgg     720
ggctcgagaa accaacaacc ctctggtctc ggggttaata gcttacttaa cactgatgga     780
acgcttgctg ttcatccatc cggttcccat gctgccgtta tcaatgaatt ttcttcaagt     840
ccatggggtt ttaaaggcaa tcaagccact accagctcag ataagatcct tcctgatagt     900
cactattctg gtgagctcga gatgatggct catcaacaaa ctggacgagc atacatggga     960
atggagtact cgacgggtta tgattcttct gtccagaatg tgcactggac tctctga       1017

<210>  243
<211>  338
<212>  PRT
<213>  Ipomoea nil

<400>  243
Met Glu Leu Gly Ser Ser Ser Ser Ser Thr Ser Thr Ser Ala Asp Ser

1               5                   10                  15
Ser Ser Asp Gly Leu Lys Phe Gly Lys Lys Val Tyr Phe Glu Asp Ala
            20                  25                  30
Gly Gly Gly Ser Gly Gly Ser Ser Leu Pro Ala Lys Arg Gly Arg Ser
        35                  40                  45
Ala Val Ala Gln Gly Gly Gln Pro Pro Arg Cys Gln Val Glu Gly Cys
    50                  55                  60
Lys Ala Asp Leu Ser Glu Phe Lys Ala Tyr Tyr Ser Lys His Lys Val
65                  70                  75                  80
Cys Gly Met His Ser Lys Ser Pro Lys Val Ile Val Ser Gly Leu Glu
                85                  90                  95
Gln Arg Phe Cys Gln Gln Cys Ser Arg Phe His Gln Leu Ser Glu Phe
            100                 105                 110
Asp Gln Val Lys Arg Ser Cys Arg Arg Arg Leu Ala Gly His Asn Glu
        115                 120                 125
Arg Arg Arg Lys Pro Pro Leu Gly Ser Ile Leu Ser Thr His Tyr Gly
    130                 135                 140
Thr Leu Ser Ser Ser Met Phe Gly Asn Asn Gly His Phe Val Met Asp
145                 150                 155                 160
Phe Ala Ser Tyr Pro Tyr Pro Gly Gly Lys Gly Ser Trp Arg Pro Ser
                165                 170                 175
Thr Asn Gly Val Gly Asn Phe Leu Gln Gln Pro Trp Gln Arg Asn Ser
            180                 185                 190
Glu Asp Pro Pro Pro Lys Leu Leu Leu Leu Gly Ser Asp Ala Ala Ala
        195                 200                 205
Arg Ala Thr Tyr Pro Ser Pro Cys Gly Glu Tyr Phe Asn Gly Val Ser
    210                 215                 220
Asp Ser Thr Arg Ala Leu Ser Leu Leu Ser Asn Ser Asn Gln Pro Trp
225                 230                 235                 240
Gly Ser Arg Asn Gln Gln Pro Ser Gly Leu Gly Val Asn Ser Leu Leu
                245                 250                 255
Asn Thr Asp Gly Thr Leu Ala Val His Pro Ser Gly Ser His Ala Ala
            260                 265                 270
Val Ile Asn Glu Phe Ser Ser Ser Pro Trp Gly Phe Lys Gly Asn Gln
        275                 280                 285
Ala Thr Thr Ser Ser Asp Lys Ile Leu Pro Asp Ser His Tyr Ser Gly
    290                 295                 300
Glu Leu Glu Met Met Ala His Gln Gln Thr Gly Arg Ala Tyr Met Gly
305                 310                 315                 320
Met Glu Tyr Ser Thr Gly Tyr Asp Ser Ser Val Gln Asn Val His Trp
                325                 330                 335
Thr Leu

<210>  244
<211>  1035
<212>  DNA
<213>  Lactuca sativa

<400>  244
atggagatgg gtggttcgag tggttcttcg gagtcgcaac tgctcaaaat tggtttgcaa      60
ttcgggaaag aaatctattt tgaggatgtg ggagttggag ctcaggttaa atccgatgat     120
gggttgtctc cggcgagcgg cggcgatgct gctggagggc cgcagaagaa agggagaact     180
gctggtgggg tggtgagtgg ttttggacaa cagcaaccac cgaggtgtca ggtggaaggt     240
tgtaatctgg atctgagtga tgctaaaagt tactattcaa ggcacaaagt ttgtggtgct     300
cattcgaaaa cggctaaggt cattgttaat ggccttgaac agagattctg ccaacagtgc     360
agcaggttcc atcaactacc agagtttgac cagggaaaaa gaagctgcag gagacgattg     420
gctgggcaca atgaacgtag aagaaagcca tctctgctat ccactcgcta tggaactgtc     480
tcctcctcaa tctttgaaaa caatgggaat tctggaggct ttctaatgga cttttcgtca     540

tgctcaagag gaaggattca gtggccagga acaagggcgg caccaccgcc tcgagccgcc 600
atcgacctcc caattgccgg agaaaagttc ccaccgcttc catggcaaag caacctggat 660
aatccacctc cttatgttcc accaggaggg tgttttaatg gagtccatga ggactccaac 720
tgtgctctct ctcttctgtc aaatcactca tctggctcaa ggaaccaatc cctgagccat 780
gagtactata tcaaccctga agctggtgca tatgtgcacc agcttcatca accaacaacc 840
ggaaccggag ctgggttcgg aaccatggtt gaggtcaccg ccactggctg ggtatacgag 900
acccatgatg cccatttggg tttgggtcac gtcccacagt ctggtggtgg ctactctggt 960
gaggttggac ttggtccaca tggtggggga aggcgatatg actcatctgt tgaccacatt 1020
gactggtcac tttga 1035

<210> 245
<211> 344
<212> PRT
<213> Lactuca sativa

<400> 245
Met Glu Met Gly Gly Ser Ser Gly Ser Ser Glu Ser Gln Leu Leu Lys
1               5                   10                  15
Ile Gly Leu Gln Phe Gly Lys Glu Ile Tyr Phe Glu Asp Val Gly Val
            20                  25                  30
Gly Ala Gln Val Lys Ser Asp Asp Gly Leu Ser Pro Ala Ser Gly Gly
        35                  40                  45
Asp Ala Ala Gly Gly Pro Gln Lys Lys Gly Arg Thr Ala Gly Gly Val
    50                  55                  60
Val Ser Gly Phe Gly Gln Gln Gln Pro Pro Arg Cys Gln Val Glu Gly
65                  70                  75                  80
Cys Asn Leu Asp Leu Ser Asp Ala Lys Ser Tyr Tyr Ser Arg His Lys
                85                  90                  95
Val Cys Gly Ala His Ser Lys Thr Ala Lys Val Ile Val Asn Gly Leu
            100                 105                 110
Glu Gln Arg Phe Cys Gln Gln Cys Ser Arg Phe His Gln Leu Pro Glu
        115                 120                 125
Phe Asp Gln Gly Lys Arg Ser Cys Arg Arg Arg Leu Ala Gly His Asn
    130                 135                 140
Glu Arg Arg Arg Lys Pro Ser Leu Leu Ser Thr Arg Tyr Gly Thr Val
145                 150                 155                 160
Ser Ser Ser Ile Phe Glu Asn Asn Gly Asn Ser Gly Gly Phe Leu Met
                165                 170                 175
Asp Phe Ser Ser Cys Ser Arg Gly Arg Ile Gln Trp Pro Gly Thr Arg
            180                 185                 190
Ala Ala Pro Pro Pro Arg Ala Ala Ile Asp Leu Pro Ile Ala Gly Glu
        195                 200                 205
Lys Phe Pro Pro Leu Pro Trp Gln Ser Asn Leu Asp Asn Pro Pro Pro
    210                 215                 220
Tyr Val Pro Pro Gly Gly Cys Phe Asn Gly Val His Glu Asp Ser Asn
225                 230                 235                 240
Cys Ala Leu Ser Leu Leu Ser Asn His Ser Ser Gly Ser Arg Asn Gln
                245                 250                 255
Ser Leu Ser His Glu Tyr Tyr Ile Asn Pro Glu Ala Gly Ala Tyr Val
            260                 265                 270
His Gln Leu His Gln Pro Thr Thr Gly Thr Gly Ala Gly Phe Gly Thr
        275                 280                 285
Met Val Glu Val Thr Ala Thr Gly Trp Val Tyr Glu Thr His Asp Ala
    290                 295                 300
His Leu Gly Leu Gly His Val Pro Gln Ser Gly Gly Gly Tyr Ser Gly
305                 310                 315                 320
Glu Val Gly Leu Gly Pro His Gly Gly Gly Arg Arg Tyr Asp Ser Ser
                325                 330                 335
Val Asp His Ile Asp Trp Ser Leu

            340

<210> 246
<211> 1137
<212> DNA
<213> Malus domestica

<400> 246
atggaaatgg gctcgagttc taagaccgag tcagcgagct cttcctcctc ttcgccgccc 60
aactcctccg ctgagtcact caacggcttg aaattcggcc ggaaaatcta ctttgaggat 120
gggggttttg gagctctgca caaatcatca tgcgggtctg ctgctgggtc ttcctccgcc 180
ggggctacgc cgccaaagaa gcaaaggggc ggcggaaatt tgggtcagcc gccgcggtgt 240
caggtggagg gctgcgaggt agatctgagt ggtgccaaag cttactattc caggcacaaa 300
gtctgtggct tgcactctaa aactcccact gtcattgttg ctggtcttga acagaggttt 360
tgccaacagt gtagcaggtt tcatttactt cctgaatttg atcaaggaaa acgtagttgt 420
cgtagacgct tggctgggca taatgagcgt cgtagaaaac cacctccagg atccatactg 480
tctacgcgtg gcagactttc ttcgtctctc tacgaaaaca gcagcagaat tggaagcttt 540
ctgatggact tcactgcata cccaaggttt tctgggaggg atacatggac aacaagaacc 600
tctgagcgag cacctgttaa tcaaaatgcc aatgacgcag ggaagtttct acaacagccg 660
tggcagagca actctgatat ttctacatcc ggcttttacc tacaaggttc agcaggcggg 720
actagttatc ctggtcctgg aattcctcca ggagaatgtg tcacagtagt cactgactca 780
agctgtgctc tctctcttct gtcaaatcag ccatggggct ctcgaaaccg agtattgggt 840
gctgggatga attccttgat gaacactcaa ggggtacctg tggctcaacc agtccctcat 900
tctgcaacct ccaatcactt tccgaccact tcgtggggtt tcaaaggaaa tgaaaatggt 960
agcagctcac acgggatgct tccagatctg ggtctcggtc aaatctcgca gccgcttagc 1020
agtcagtact ctggtgtgct ggagctgtct caacagggta ggaggcagca acacatggaa 1080
ctcggacaca ccaggggcta tgactccacc agtcagcaga tgcactggtc actttaa 1137

<210> 247
<211> 378
<212> PRT
<213> Malus domestica

<400> 247
Met Glu Met Gly Ser Ser Ser Lys Thr Glu Ser Ala Ser Ser Ser Ser
1               5                   10                  15
Ser Ser Pro Pro Asn Ser Ser Ala Glu Ser Leu Asn Gly Leu Lys Phe
            20                  25                  30
Gly Arg Lys Ile Tyr Phe Glu Asp Gly Gly Phe Gly Ala Leu His Lys
        35                  40                  45
Ser Ser Cys Gly Ser Ala Ala Gly Ser Ser Ser Ala Gly Ala Thr Pro
    50                  55                  60
Pro Lys Lys Gln Arg Gly Gly Gly Asn Leu Gly Gln Pro Pro Arg Cys
65                  70                  75                  80
Gln Val Glu Gly Cys Glu Val Asp Leu Ser Gly Ala Lys Ala Tyr Tyr
                85                  90                  95
Ser Arg His Lys Val Cys Gly Leu His Ser Lys Thr Pro Thr Val Ile
            100                 105                 110
Val Ala Gly Leu Glu Gln Arg Phe Cys Gln Gln Cys Ser Arg Phe His
        115                 120                 125
Leu Leu Pro Glu Phe Asp Gln Gly Lys Arg Ser Cys Arg Arg Arg Leu
    130                 135                 140
Ala Gly His Asn Glu Arg Arg Arg Lys Pro Pro Pro Gly Ser Ile Leu
145                 150                 155                 160
Ser Thr Arg Gly Arg Leu Ser Ser Ser Leu Tyr Glu Asn Ser Ser Arg
                165                 170                 175
Ile Gly Ser Phe Leu Met Asp Phe Thr Ala Tyr Pro Arg Phe Ser Gly
            180                 185                 190
Arg Asp Thr Trp Thr Thr Arg Thr Ser Glu Arg Ala Pro Val Asn Gln

195                 200                 205
Asn Ala Asn Asp Ala Gly Lys Phe Leu Gln Gln Pro Trp Gln Ser Asn
    210                 215                 220
Ser Asp Ile Ser Thr Ser Gly Phe Tyr Leu Gln Gly Ser Ala Gly Gly
225                 230                 235                 240
Thr Ser Tyr Pro Gly Pro Gly Ile Pro Pro Gly Glu Cys Val Thr Val
                245                 250                 255
Val Thr Asp Ser Ser Cys Ala Leu Ser Leu Leu Ser Asn Gln Pro Trp
                260                 265                 270
Gly Ser Arg Asn Arg Val Leu Gly Ala Gly Met Asn Ser Leu Met Asn
        275                 280                 285
Thr Gln Gly Val Pro Val Ala Gln Pro Val Pro His Ser Ala Thr Ser
    290                 295                 300
Asn His Phe Pro Thr Thr Ser Trp Gly Phe Lys Gly Asn Glu Asn Gly
305                 310                 315                 320
Ser Ser Ser His Gly Met Leu Pro Asp Leu Gly Leu Gly Gln Ile Ser
                325                 330                 335
Gln Pro Leu Ser Ser Gln Tyr Ser Gly Val Leu Glu Leu Ser Gln Gln
                340                 345                 350
Gly Arg Arg Gln Gln His Met Glu Leu Gly His Thr Arg Gly Tyr Asp
        355                 360                 365
Ser Thr Ser Gln Gln Met His Trp Ser Leu
    370                 375

<210>  248
<211>  1014
<212>  DNA
<213>  Medicago truncatula

<400>  248
atggattcag gaggcaactc ttcttcagaa gagtcctcac ttaatggctt gaaatttggc      60
caacgaatct atttcgaaga tacagctctt actgctgctt ctgctgctgc tgctagtact     120
accattgctg ctggttctcc ttcttcttct ggttcaaaga aaggaagagg tgggtcagtt     180
caacattctc aaccacctag gtgtcaagtt gaaggatgta aactagatct gactgatgct     240
aaagcttact attctagaca caaagtttgt agcatgcact ctaaatcccc tactgttact     300
gtttctggtc ttcaacaaag gttttgtcaa caatgtagca gatttcatca gcttgctgag     360
tttgatcaag gaaaaagaag ttgtcggaga cgactagctg gtcataatga gcgtcgcaga     420
aagcccccac ccagctctct cttaacctca cgttttgcca ggctttcttc gtctgttttt     480
ggtaacagcg acagaggtgg cagctttttg atggaatttg cttcaaatcc gaaacatagt     540
ctgaggaatt cacccggaaa tcaaaccaca gcaatcggtt ggccttggcc ggggaacacg     600
gagtcgccat ctagcaacct tttcttgcaa ggttcggtgg gtgggacaag cttccctggt     660
gccaggcatc ctcccgagga aacttacact ggagtcacag attcaaactg tgctctctct     720
cttctgtcaa atcaaacatg gggttctcaa aacacagaac caagtcctgg attgaataac     780
atgctgaatt tcaacgggac acccatgaca caacttggta catcttctca tggtgtagcc     840
atgcatcaaa ttccaaacaa ttacgaggtt gtccctgatc ttggtcgggg tcacattttg      900
catcctcttg gtagccaaca ctctggcgag cttgatctgt tgcagcaggg aaggaggcat     960
tatatggatg tagaacattc cagggcctat gaatcttctc agtggtcact gtaa           1014

<210>  249
<211>  337
<212>  PRT
<213>  Medicago truncatula

<400>  249
Met Asp Ser Gly Gly Asn Ser Ser Ser Glu Glu Ser Ser Leu Asn Gly
1               5                   10                  15
Leu Lys Phe Gly Gln Arg Ile Tyr Phe Glu Asp Thr Ala Leu Thr Ala
            20                  25                  30
Ala Ser Ala Ala Ala Ala Ser Thr Thr Ile Ala Ala Gly Ser Pro Ser

        35                  40                  45
Ser Ser Gly Ser Lys Lys Gly Arg Gly Gly Ser Val Gln His Ser Gln
    50                  55                  60
Pro Pro Arg Cys Gln Val Glu Gly Cys Lys Leu Asp Leu Thr Asp Ala
65                  70                  75                  80
Lys Ala Tyr Tyr Ser Arg His Lys Val Cys Ser Met His Ser Lys Ser
                85                  90                  95
Pro Thr Val Thr Val Ser Gly Leu Gln Gln Arg Phe Cys Gln Gln Cys
            100                 105                 110
Ser Arg Phe His Gln Leu Ala Glu Phe Asp Gln Gly Lys Arg Ser Cys
        115                 120                 125
Arg Arg Arg Leu Ala Gly His Asn Glu Arg Arg Arg Lys Pro Pro Pro
    130                 135                 140
Ser Ser Leu Leu Thr Ser Arg Phe Ala Arg Leu Ser Ser Ser Val Phe
145                 150                 155                 160
Gly Asn Ser Asp Arg Gly Gly Ser Phe Leu Met Glu Phe Ala Ser Asn
                165                 170                 175
Pro Lys His Ser Leu Arg Asn Ser Pro Gly Asn Gln Thr Thr Ala Ile
            180                 185                 190
Gly Trp Pro Trp Pro Gly Asn Thr Glu Ser Pro Ser Ser Asn Leu Phe
        195                 200                 205
Leu Gln Gly Ser Val Gly Gly Thr Ser Phe Pro Gly Ala Arg His Pro
    210                 215                 220
Pro Glu Glu Thr Tyr Thr Gly Val Thr Asp Ser Asn Cys Ala Leu Ser
225                 230                 235                 240
Leu Leu Ser Asn Gln Thr Trp Gly Ser Gln Asn Thr Glu Pro Ser Pro
                245                 250                 255
Gly Leu Asn Asn Met Leu Asn Phe Asn Gly Thr Pro Met Thr Gln Leu
            260                 265                 270
Gly Thr Ser Ser His Gly Val Ala Met His Gln Ile Pro Asn Asn Tyr
        275                 280                 285
Glu Val Val Pro Asp Leu Gly Arg Gly His Ile Leu His Pro Leu Gly
    290                 295                 300
Ser Gln His Ser Gly Glu Leu Asp Leu Leu Gln Gln Gly Arg Arg His
305                 310                 315                 320
Tyr Met Asp Val Glu His Ser Arg Ala Tyr Glu Ser Ser Gln Trp Ser
                325                 330                 335
Leu

<210> 250
<211> 1143
<212> DNA
<213> Nicotiana bentamiana

<400> 250
atggaacttc tggcttctgc ttcttcttct acttctacta attctacttc ccctgactct 60
cctcccaaca ctttaaaatt tggtcaaaaa atctactttg agcatgttgg acttcagcac 120
ctcaaatcag caactgggtc gtcgtcgtca tcgccgccgg tcatcggaac tccggcgccg 180
gcgatgtcca agaaaggaag agggggtggt gtagttcaag gtcggcaacc acctaggtgt 240
caagttgaag ggtgtgaagc agatctgagt gatgttaagg cttactattc aaggcacaaa 300
gtctgtgcta cacattctaa gtctcctgtg gtcattgttg ctggtcttga acaaagattt 360
tgtcaacagt gtagcaggtt tcatcggttg ccagaatttg accaaggaaa acgcagttgc 420
cgcaggcgcc tagcaggcca taatgagcgt cggaggaaac ctccacctgg atctcttttg 480
tctaatcgct atggaagtct ttcttcatca atatttgaaa acaatggcag atctggaagt 540
tttctggttg acttcactgc atatccgaat ctcactggag gtgcatggcc aaatactaga 600
tcatctgatc gaggatggga taatcaatcc actgcgtcag ggaagcttct ccaaagtcat 660
tggctgaaca gttctgaaaa tccgacatcc gaccttgttc tgcaaggttc agttgctagg 720
ggtgccaatt attctggtcc tggtattatt ccttccggaa actgcttctc tggagtctca 780

gattccaatg gtgctctctc tcttctgtca aatgagccat ggggctcgag gaaccaatcc 840
tctagcctcg gggttaacgg cttggtcaac actgatggcg gacataccgt tcacccatcg 900
ggttcccatg ctgctcctgt caatcactac tcaggccctc tatggggatt taaaggaaat 960
gaagctagta gcagttcaca tgcaatacct cctgatctcg ggctgggtca catttctcaa 1020
catgctgtca atcagtactc tggtgagcct gggatggctc agcacagtgg aagacagtac 1080
atgggactgg agcattcaaa gggttataat tcttctgttc agaatgtgca ctggacactt 1140
tga 1143

<210> 251
<211> 380
<212> PRT
<213> Nicotiana bentamiana

<400> 251
Met Glu Leu Leu Ala Ser Ala Ser Ser Ser Thr Ser Thr Asn Ser Thr
1               5                   10                  15
Ser Pro Asp Ser Pro Pro Asn Thr Leu Lys Phe Gly Gln Lys Ile Tyr
            20                  25                  30
Phe Glu His Val Gly Leu Gln His Leu Lys Ser Ala Thr Gly Ser Ser
        35                  40                  45
Ser Ser Ser Pro Pro Val Ile Gly Thr Pro Ala Pro Ala Met Ser Lys
    50                  55                  60
Lys Gly Arg Gly Gly Gly Val Val Gln Gly Arg Gln Pro Pro Arg Cys
65                  70                  75                  80
Gln Val Glu Gly Cys Glu Ala Asp Leu Ser Asp Val Lys Ala Tyr Tyr
                85                  90                  95
Ser Arg His Lys Val Cys Ala Thr His Ser Lys Ser Pro Val Val Ile
            100                 105                 110
Val Ala Gly Leu Glu Gln Arg Phe Cys Gln Gln Cys Ser Arg Phe His
        115                 120                 125
Arg Leu Pro Glu Phe Asp Gln Gly Lys Arg Ser Cys Arg Arg Arg Leu
    130                 135                 140
Ala Gly His Asn Glu Arg Arg Arg Lys Pro Pro Pro Gly Ser Leu Leu
145                 150                 155                 160
Ser Asn Arg Tyr Gly Ser Leu Ser Ser Ser Ile Phe Glu Asn Asn Gly
                165                 170                 175
Arg Ser Gly Ser Phe Leu Val Asp Phe Thr Ala Tyr Pro Asn Leu Thr
            180                 185                 190
Gly Gly Ala Trp Pro Asn Thr Arg Ser Ser Asp Arg Gly Trp Asp Asn
        195                 200                 205
Gln Ser Thr Ala Ser Gly Lys Leu Leu Gln Ser His Trp Leu Asn Ser
    210                 215                 220
Ser Glu Asn Pro Thr Ser Asp Leu Val Leu Gln Gly Ser Val Ala Arg
225                 230                 235                 240
Gly Ala Asn Tyr Ser Gly Pro Gly Ile Ile Pro Ser Gly Asn Cys Phe
                245                 250                 255
Ser Gly Val Ser Asp Ser Asn Gly Ala Leu Ser Leu Leu Ser Asn Glu
            260                 265                 270
Pro Trp Gly Ser Arg Asn Gln Ser Ser Ser Leu Gly Val Asn Gly Leu
        275                 280                 285
Val Asn Thr Asp Gly Gly His Thr Val His Pro Ser Gly Ser His Ala
    290                 295                 300
Ala Pro Val Asn His Tyr Ser Gly Pro Leu Trp Gly Phe Lys Gly Asn
305                 310                 315                 320
Glu Ala Ser Ser Ser Ser His Ala Ile Pro Pro Asp Leu Gly Leu Gly
                325                 330                 335
His Ile Ser Gln His Ala Val Asn Gln Tyr Ser Gly Glu Pro Gly Met
            340                 345                 350
Ala Gln His Ser Gly Arg Gln Tyr Met Gly Leu Glu His Ser Lys Gly

355                 360                 365
Tyr Asn Ser Ser Val Gln Asn Val His Trp Thr Leu
    370                 375                 380

<210>  252
<211>  1254
<212>  DNA
<213>  Oryza sativa

<400>  252
atggagatgg ccagtggagg aggcgccgcc gccgccgccg gcggcggagt aggcggcagc      60
ggcggcggtg gtggtggagg ggacgagcac cgccagctgc acggtctcaa gttcggcaag     120
aagatctact tcgaggacgc cgccgcggca gcaggcggcg gcggcactgg cagtggcagt     180
ggcagcgcga gcgccgcgcc gccgtcctcg tcttccaagg cggcgggtgg tggacgcggc     240
ggagggggca agaacaaggg gaagggcgtg gccgcggcgg cgccaccgcc gccgccgccg     300
ccgccgcggt gccaggtgga ggggtgcggc gcggatctga gcgggatcaa gaactactac     360
tgccgccaca aggtgtgctt catgcattcc aaggctcccc gcgtcgtcgt cgccggcctc     420
gagcagcgct tctgccagca gtgcagcagg ttccacctgc tgcctgaatt tgaccaagga     480
aaacgcagct gccgcagacg ccttgcaggt cataatgagc gccggaggag gccgcaaacc     540
cctttggcat cacgctacgg tcgactagct gcatctgttg gtgagcatcg caggttcaga     600
agctttacgt tggatttctc ctacccaagg gttccaagca gcgtaaggaa tgcatggcca     660
gcaattcaac caggcgatcg gatctccggt ggtatccagt ggcacaggaa cgtagctcct     720
catggtcact ctagtgcagt ggcgggatat ggtgccaaca catacagcgg ccaaggtagc     780
tcttcttcag ggccaccggt gttcgctggc ccaaatctcc ctccaggtgg atgtctcgca     840
ggggtcggtg ccgccaccga ctcgagctgt gctctctctc ttctgtcaac ccagccatgg     900
gatactacta cccacagtgc cgctgccagc cacaaccagg ctgcagccat gtccactacc     960
accagctttg atggcaatcc tgtggcaccc tccgccatgg cgggtagcta catggcacca    1020
agcccctgga caggttctcg ggccatgagg gtggtggtcc ggagcgtggc gcaccagcta    1080
ccacatgaag tctcacttga tgaggtgcac cctggtccta gccatcatgc ccacttctcc    1140
ggtgagcttg agcttgctct gcaggggaac ggtccagccc cagcaccacg catcgatcct    1200
gggtccggca gcaccttcga ccaaaccagc aacacgatgg attggtctct gtag          1254

<210>  253
<211>  417
<212>  PRT
<213>  Oryza sativa

<400>  253
Met Glu Met Ala Ser Gly Gly Gly Ala Ala Ala Ala Ala Gly Gly Gly
1               5                   10                  15
Val Gly Gly Ser Gly Gly Gly Gly Gly Gly Gly Asp Glu His Arg Gln
            20                  25                  30
Leu His Gly Leu Lys Phe Gly Lys Lys Ile Tyr Phe Glu Asp Ala Ala
        35                  40                  45
Ala Ala Ala Gly Gly Gly Gly Thr Gly Ser Gly Ser Gly Ser Ala Ser
    50                  55                  60
Ala Ala Pro Pro Ser Ser Ser Ser Lys Ala Ala Gly Gly Gly Arg Gly
65                  70                  75                  80
Gly Gly Gly Lys Asn Lys Gly Lys Gly Val Ala Ala Ala Ala Pro Pro
                85                  90                  95
Pro Pro Pro Pro Pro Pro Arg Cys Gln Val Glu Gly Cys Gly Ala Asp
            100                 105                 110
Leu Ser Gly Ile Lys Asn Tyr Tyr Cys Arg His Lys Val Cys Phe Met
        115                 120                 125
His Ser Lys Ala Pro Arg Val Val Val Ala Gly Leu Glu Gln Arg Phe
    130                 135                 140
Cys Gln Gln Cys Ser Arg Phe His Leu Leu Pro Glu Phe Asp Gln Gly
145                 150                 155                 160
Lys Arg Ser Cys Arg Arg Arg Leu Ala Gly His Asn Glu Arg Arg Arg

165                 170                 175
Arg Pro Gln Thr Pro Leu Ala Ser Arg Tyr Gly Arg Leu Ala Ala Ser
            180                 185                 190
Val Gly Glu His Arg Arg Phe Arg Ser Phe Thr Leu Asp Phe Ser Tyr
        195                 200                 205
Pro Arg Val Pro Ser Ser Val Arg Asn Ala Trp Pro Ala Ile Gln Pro
    210                 215                 220
Gly Asp Arg Ile Ser Gly Gly Ile Gln Trp His Arg Asn Val Ala Pro
225                 230                 235                 240
His Gly His Ser Ser Ala Val Ala Gly Tyr Gly Ala Asn Thr Tyr Ser
                245                 250                 255
Gly Gln Gly Ser Ser Ser Ser Gly Pro Pro Val Phe Ala Gly Pro Asn
            260                 265                 270
Leu Pro Pro Gly Gly Cys Leu Ala Gly Val Gly Ala Ala Thr Asp Ser
        275                 280                 285
Ser Cys Ala Leu Ser Leu Leu Ser Thr Gln Pro Trp Asp Thr Thr Thr
    290                 295                 300
His Ser Ala Ala Ala Ser His Asn Gln Ala Ala Ala Met Ser Thr Thr
305                 310                 315                 320
Thr Ser Phe Asp Gly Asn Pro Val Ala Pro Ser Ala Met Ala Gly Ser
                325                 330                 335
Tyr Met Ala Pro Ser Pro Trp Thr Gly Ser Arg Gly His Glu Gly Gly
            340                 345                 350
Gly Arg Ser Val Ala His Gln Leu Pro His Glu Val Ser Leu Asp Glu
        355                 360                 365
Val His Pro Gly Pro Ser His His Ala His Phe Ser Gly Glu Leu Glu
    370                 375                 380
Leu Ala Leu Gln Gly Asn Gly Pro Ala Pro Ala Pro Arg Ile Asp Pro
385                 390                 395                 400
Gly Ser Gly Ser Thr Phe Asp Gln Thr Ser Asn Thr Met Asp Trp Ser
                405                 410                 415
Leu

<210>  254
<211>  1182
<212>  DNA
<213>  Oryza sativa

<400>  254
atggcgaccg gcggcagcgg cggcggcggc ggaggtggag gtggtggtga cgatgtccac      60
gggctcaagt tcggcaagaa gatctacttc gagcaggacg cggcggcgtc ggcgtcggcg     120
gcggcggtgg agtcgtcgtc gacgtcgtcg ggcggaggcg gcaagaaggg gaagggcgtg     180
gcggcggcgg cggcgccccc gccgccgctg ccgccgaggt gccaggtgga gggttgcggc     240
gtggatctga gcggcgtcaa gccgtactac tgccgccaca aggtgtgcta catgcacgcc     300
aaggagccca tcgtcgtcgt cgccggcctc gagcagcgct tctgccaaca gtgcagcagg     360
ttccaccaat tacctgaatt tgatcaagaa aaaaaaagct gccgcagacg ccttgcaggt     420
cacaatgaac gccggaggaa gccgacacct ggacctcttt cttctcgcta tggccggctt     480
gctgcatcct ttcatgaaga gccaggcagg tccagaagct ttgtggtaga tttctcatac     540
ccaagggttc caagcagtgt gagggatgcg tggcctgcta ttcagcccag cgatcgcatg     600
tccggttcaa tccagtggca agggggccat gaactccatc ctcaccgcag cgcagttgcg     660
ggatacagtg atcaccatgc gttcagcagc catggtggct cagcggctgg ggcaccaatg     720
ctccaccacc cagcctttga gctcacctca ggtggatgtc tcgcgggagt cgccaccgac     780
tccagctgtg ctctctctct tctgtcaact cagccatggg atactaccca aagcaccagc     840
agccacaacc ggtccccgcc aatgtcgtca acggccagcg ccttcggagg cggcaacaac     900
ccggtgtcgc cctcggtcat ggcaagcaac tacatggcgg cgagccccgg ctggaacagc     960
tccagccggg gccatgacgg cgccaggaac gtgcacctgc cgccaccgca cggggttgtg    1020
ctgaacgagg tccctccggg ctctgtccac cacggccatt tctccggcga gctcgagctc    1080
gcactgcagg gaggtgcccc gtccaaccgg ccggaagccg agcatggctc cggcagcggc    1140

gccttcagcc actccaccaa tgccatgaac tggtctctgt ag                    1182

<210>  255
<211>  393
<212>  PRT
<213>  Oryza sativa

<400>  255
Met Ala Thr Gly Gly Ser Gly Gly Gly Gly Gly Gly Gly Gly Gly Gly
1               5                   10                  15
Asp Asp Val His Gly Leu Lys Phe Gly Lys Lys Ile Tyr Phe Glu Gln
            20                  25                  30
Asp Ala Ala Ala Ser Ala Ser Ala Ala Ala Val Glu Ser Ser Ser Thr
        35                  40                  45
Ser Ser Gly Gly Gly Gly Lys Lys Gly Lys Gly Val Ala Ala Ala Ala
    50                  55                  60
Ala Pro Pro Pro Pro Leu Pro Pro Arg Cys Gln Val Glu Gly Cys Gly
65                  70                  75                  80
Val Asp Leu Ser Gly Val Lys Pro Tyr Tyr Cys Arg His Lys Val Cys
                85                  90                  95
Tyr Met His Ala Lys Glu Pro Ile Val Val Val Ala Gly Leu Glu Gln
            100                 105                 110
Arg Phe Cys Gln Gln Cys Ser Arg Phe His Gln Leu Pro Glu Phe Asp
        115                 120                 125
Gln Glu Lys Lys Ser Cys Arg Arg Arg Leu Ala Gly His Asn Glu Arg
    130                 135                 140
Arg Arg Lys Pro Thr Pro Gly Pro Leu Ser Ser Arg Tyr Gly Arg Leu
145                 150                 155                 160
Ala Ala Ser Phe His Glu Glu Pro Gly Arg Ser Arg Ser Phe Val Val
                165                 170                 175
Asp Phe Ser Tyr Pro Arg Val Pro Ser Ser Val Arg Asp Ala Trp Pro
            180                 185                 190
Ala Ile Gln Pro Ser Asp Arg Met Ser Gly Ser Ile Gln Trp Gln Gly
        195                 200                 205
Gly His Glu Leu His Pro His Arg Ser Ala Val Ala Gly Tyr Ser Asp
    210                 215                 220
His His Ala Phe Ser Ser His Gly Gly Ser Ala Ala Gly Ala Pro Met
225                 230                 235                 240
Leu His His Pro Ala Phe Glu Leu Thr Ser Gly Gly Cys Leu Ala Gly
                245                 250                 255
Val Ala Thr Asp Ser Ser Cys Ala Leu Ser Leu Leu Ser Thr Gln Pro
            260                 265                 270
Trp Asp Thr Thr Gln Ser Thr Ser Ser His Asn Arg Ser Pro Pro Met
        275                 280                 285
Ser Ser Thr Ala Ser Ala Phe Gly Gly Gly Asn Asn Pro Val Ser Pro
    290                 295                 300
Ser Val Met Ala Ser Asn Tyr Met Ala Ala Ser Pro Gly Trp Asn Ser
305                 310                 315                 320
Ser Ser Arg Gly His Asp Gly Ala Arg Asn Val His Leu Pro Pro Pro
                325                 330                 335
His Gly Val Val Leu Asn Glu Val Pro Pro Gly Ser Val His His Gly
            340                 345                 350
His Phe Ser Gly Glu Leu Glu Leu Ala Leu Gln Gly Gly Ala Pro Ser
        355                 360                 365
Asn Arg Pro Glu Ala Glu His Gly Ser Gly Ser Gly Ala Phe Ser His
    370                 375                 380
Ser Thr Asn Ala Met Asn Trp Ser Leu
385                 390

<210> 256
<211> 1119
<212> DNA
<213> Solanum tuberosum

<400> 256
atggaactgg gttcagtttc ttcttctggt aattcaagct catctgattc tttgaatggt 60
ttgaagtttg gtaagaaaat ctactttgga aatgtgggtg ttggagttca ggtcaagaat 120
ggaagtgggt cgtcgccggt gaccggagat gggaacatgc caccggctcc ggcgacgact 180
aagaggggga ggggtgggtt ggtgcagggt ggtcatccac ctaggtgtca agttgaaggt 240
tgtcaggcag atctgagtga tgctaaggct tactattcaa ggcataaagt ttgtggtatg 300
cactctaagt ctcctactgt tgttgttgct ggtcttgaac agaggttttg ccaacagtgt 360
agcaggttcc atcaattaac tgaattcgac caggggaaaa ggagttgccg caggagactg 420
gcatgccata atgagcgtcg taggaagcct ccatctggat ctcttttctc tacacactac 480
gggaatcttt cttcatcaat atttgaaaat aatagcagca gatccggaag ctttctggtc 540
gacttcagct cacaccaaaa tgtcaatgat agttcatggc caaatactcg agcatctgaa 600
caaggatggg atcatcaatc atcagggaag ttccttcaac gtccttggct gaataactct 660
gaaaatgctg ccagtgagct tgttttgcaa ggttcagcta ccaggaccag ttatcctagt 720
gttccttctg gagactattt tcctggagtc tcagattcaa gtggtgctct ctctcttctg 780
tcaaatcggt cctggggatc aaggaatcga tctccaagtc ttggggttaa cagccaagtt 840
cacattgatg gggtacacac cattcaacct tcaggttctc atggtgcacc taccaatcac 900
ttctcaagcc cttcattgag ttttaaagga aatgaagcta gcagcagttc acatgagatg 960
cctcctgatc tcggtttggg tcaaatgtta caagcttctg ataatccata ctgtggcgag 1020
cttgggatgg ctcagcatgg tgatggacga caatacatgg aactggacca gtccaagggt 1080
tatcatcctt ctgttcagaa tgtgcactgg actctttga 1119

<210> 257
<211> 372
<212> PRT
<213> Solanum tuberosum

<400> 257
Met Glu Leu Gly Ser Val Ser Ser Ser Gly Asn Ser Ser Ser Ser Asp
1               5                   10                  15
Ser Leu Asn Gly Leu Lys Phe Gly Lys Lys Ile Tyr Phe Gly Asn Val
            20                  25                  30
Gly Val Gly Val Gln Val Lys Asn Gly Ser Gly Ser Ser Pro Val Thr
        35                  40                  45
Gly Asp Gly Asn Met Pro Pro Ala Pro Ala Thr Thr Lys Arg Gly Arg
    50                  55                  60
Gly Gly Leu Val Gln Gly Gly His Pro Pro Arg Cys Gln Val Glu Gly
65                  70                  75                  80
Cys Gln Ala Asp Leu Ser Asp Ala Lys Ala Tyr Tyr Ser Arg His Lys
                85                  90                  95
Val Cys Gly Met His Ser Lys Ser Pro Thr Val Val Val Ala Gly Leu
            100                 105                 110
Glu Gln Arg Phe Cys Gln Gln Cys Ser Arg Phe His Gln Leu Thr Glu
        115                 120                 125
Phe Asp Gln Gly Lys Arg Ser Cys Arg Arg Arg Leu Ala Cys His Asn
    130                 135                 140
Glu Arg Arg Arg Lys Pro Pro Ser Gly Ser Leu Phe Ser Thr His Tyr
145                 150                 155                 160
Gly Asn Leu Ser Ser Ser Ile Phe Glu Asn Asn Ser Ser Arg Ser Gly
                165                 170                 175
Ser Phe Leu Val Asp Phe Ser Ser His Gln Asn Val Asn Asp Ser Ser
            180                 185                 190
Trp Pro Asn Thr Arg Ala Ser Glu Gln Gly Trp Asp His Gln Ser Ser
        195                 200                 205
Gly Lys Phe Leu Gln Arg Pro Trp Leu Asn Asn Ser Glu Asn Ala Ala

210                 215                 220
Ser Glu Leu Val Leu Gln Gly Ser Ala Thr Arg Thr Ser Tyr Pro Ser
225                 230                 235                 240
Val Pro Ser Gly Asp Tyr Phe Pro Gly Val Ser Asp Ser Ser Gly Ala
                245                 250                 255
Leu Ser Leu Leu Ser Asn Arg Ser Trp Gly Ser Arg Asn Arg Ser Pro
            260                 265                 270
Ser Leu Gly Val Asn Ser Gln Val His Ile Asp Gly Val His Thr Ile
        275                 280                 285
Gln Pro Ser Gly Ser His Gly Ala Pro Thr Asn His Phe Ser Ser Pro
    290                 295                 300
Ser Leu Ser Phe Lys Gly Asn Glu Ala Ser Ser Ser Ser His Glu Met
305                 310                 315                 320
Pro Pro Asp Leu Gly Leu Gly Gln Met Leu Gln Ala Ser Asp Asn Pro
                325                 330                 335
Tyr Cys Gly Glu Leu Gly Met Ala Gln His Gly Asp Gly Arg Gln Tyr
            340                 345                 350.
Met Glu Leu Asp Gln Ser Lys Gly Tyr His Pro Ser Val Gln Asn Val
        355                 360                 365
His Trp Thr Leu
    370

<210>  258
<211>  1140
<212>  DNA
<213>  Vitis vinifera

<400>  258
atggaaaggg gttcgagctc tttgaccgtt tccagctctt cggccaactc gtctgagtcg      60
ctcaacgggt tgaaatttgg gcagaagata tattttgaag atttgggcgt tggagctccg     120
gccaaatcgg gaaccggctc ctcctcctcc tcctctgccg ccggctccgg tggtcgccca     180
cctccggcgc cgccaaagaa ggtaagaggt agtggggttg ttcagggagg ccaaccaccg     240
aggtgtcaag ttgaagggtg taaactagat ctgagtgatg ccaaagctta ctattcaagg     300
cataaagtgt gtggtatgca ttcgaagtct ccaacggtca ttgttgcggg ccttgagcag     360
aggttttgcc agcagtgtag cagatttcat cagcttgccg aatttgacca aggaaaacga     420
agttgtcgta ggcgcctggc tggtcataat gagcgtcgca ggaagccacc acctggatct     480
ttattgtcct cacgctatgg gcgactttct tcatccattt ttgaaaacag cagcagggtg     540
ggaggaggct ttctgatgga ctttgctgca tacccaaggc atcccgagag ggatacttgg     600
ccaactacaa gagcatctga tcgggtacct ggaaatcaaa ccactgcgat gggaaggttt     660
cttccacatc catggcagag caactctgag aatcctctct ttctgcaagg ttcagccggc     720
gggaccagct ttcatggtcc tggaattcct tcaggagaat gtttcacagg ggcctccgac     780
tcaagctgtg ctctctctct tctgtcaaat cagccatgga gctccaggaa tcgagcatct     840
ggtcttggag caaacagctt catgaatcct gaaggggcat ccatggcgca acccacagct     900
cctcatagtg cagctatcaa tcacttccca agcacctcgt gggatttcaa gggcaatgaa     960
ggtagtagca gttcgcagga gatgccacct gatcttggtc ttggtcaaat ttcacagcct    1020
attaatagcc agttctcagg tgggggcgag ttgccccaac agagtggaag gcaatacatg    1080
gaactcgagc actccagggc ttatgacact tccactcagc agatgcactg gtcactttag    1140

<210>  259
<211>  379
<212>  PRT
<213>  Vitis vinifera

<400>  259
Met Glu Arg Gly Ser Ser Ser Leu Thr Val Ser Ser Ser Ser Ala Asn
1               5                   10                  15
Ser Ser Glu Ser Leu Asn Gly Leu Lys Phe Gly Gln Lys Ile Tyr Phe
            20                  25                  30
Glu Asp Leu Gly Val Gly Ala Pro Ala Lys Ser Gly Thr Gly Ser Ser

35                  40                  45
Ser Ser Ser Ser Ala Ala Gly Ser Gly Gly Arg Pro Pro Pro Ala Pro
    50                  55                  60
Pro Lys Lys Val Arg Gly Ser Gly Val Val Gln Gly Gly Gln Pro Pro
65                  70                  75                  80
Arg Cys Gln Val Glu Gly Cys Lys Leu Asp Leu Ser Asp Ala Lys Ala
                85                  90                  95
Tyr Tyr Ser Arg His Lys Val Cys Gly Met His Ser Lys Ser Pro Thr
            100                 105                 110
Val Ile Val Ala Gly Leu Glu Gln Arg Phe Cys Gln Gln Cys Ser Arg
        115                 120                 125
Phe His Gln Leu Ala Glu Phe Asp Gln Gly Lys Arg Ser Cys Arg Arg
    130                 135                 140
Arg Leu Ala Gly His Asn Glu Arg Arg Arg Lys Pro Pro Pro Gly Ser
145                 150                 155                 160
Leu Leu Ser Ser Arg Tyr Gly Arg Leu Ser Ser Ser Ile Phe Glu Asn
                165                 170                 175
Ser Ser Arg Val Gly Gly Gly Phe Leu Met Asp Phe Ala Ala Tyr Pro
            180                 185                 190
Arg His Pro Glu Arg Asp Thr Trp Pro Thr Thr Arg Ala Ser Asp Arg
        195                 200                 205
Val Pro Gly Asn Gln Thr Thr Ala Met Gly Arg Phe Leu Pro His Pro
    210                 215                 220
Trp Gln Ser Asn Ser Glu Asn Pro Leu Phe Leu Gln Gly Ser Ala Gly
225                 230                 235                 240
Gly Thr Ser Phe His Gly Pro Gly Ile Pro Ser Gly Glu Cys Phe Thr
                245                 250                 255
Gly Ala Ser Asp Ser Ser Cys Ala Leu Ser Leu Leu Ser Asn Gln Pro
            260                 265                 270
Trp Ser Ser Arg Asn Arg Ala Ser Gly Leu Gly Ala Asn Ser Phe Met
        275                 280                 285
Asn Pro Glu Gly Ala Ser Met Ala Gln Pro Thr Ala Pro His Ser Ala
    290                 295                 300
Ala Ile Asn His Phe Pro Ser Thr Ser Trp Asp Phe Lys Gly Asn Glu
305                 310                 315                 320
Gly Ser Ser Ser Ser Gln Glu Met Pro Pro Asp Leu Gly Leu Gly Gln
                325                 330                 335
Ile Ser Gln Pro Ile Asn Ser Gln Phe Ser Gly Gly Gly Glu Leu Pro
            340                 345                 350
Gln Gln Ser Gly Arg Gln Tyr Met Glu Leu Glu His Ser Arg Ala Tyr
        355                 360                 365
Asp Thr Ser Thr Gln Gln Met His Trp Ser Leu
    370                 375

<210>  260
<211>  1209
<212>  DNA
<213>  Zea mays

<400>  260
atggagtccg gcggtggcgg ggacgaccag ctgcacggcc tcaagttcgg caagaagatc      60
tacttcgagg acgccgccgg ctccagcagc ggcagcagca gcggcggtgg cagcgcgccc     120
gcgcctccag cgacgcagca gccgtcgccg ccggccgctt cgcctagggc cccggccggc     180
ggcggcagga ggggcagggc cgcggccggc ggcgcgggcc cctcgacggc gcccgcgccc     240
gcgcgctgcc aggtcgacgg ctgcaacgtt gacctcaccg acgtcaagcc cgcctactac     300
tgccgccaca aggtgtgcaa aatgcactcc aaggagcccc gcgtcctcgt caacggcctc     360
gagcagcgct tctgccagca gtgcagcagg ttccaccagc tgcctgaatt cgaccagcta     420
aagaagagct gccgcaaacg cctcgcaggc cacaacgagc gccggaggag gccgccgcct     480
ggaccccttg cgtcacgata cggccgtcac gctgcgtcgc tcggcgagcc cggcaggctc     540

agaagcttca tgctggattt ctcgtacccg agggtctcaa gcgccatgag gggtgggttt 600
cccgcggtga gggccggtgg tgaaagggtg cctggcggga tccagtggca agcgggcttg 660
gatcctcgtc accaccaagg cgcggtcgcg ggatacggcg cccactatgg gagcgagggt 720
ggtagctcgt cgtcggcgag gccgccggtg ttccctggcc cggagctgcc cccaggtgga 780
tgccttgcag gagtccccgc ggactccagc tgtgctctct ctcttctgtc aactcagcca 840
tgggatgctg cccacagcca cagccacagc cacgctgcgc caacagcggg tttcgacggc 900
ggcagccctg tggcgccctc cctcatggcg gcgagtagct acatcgcgcc gagcccctgg 960
accgagaccg actcctgggg ccacgaaggc gggcggagcg tgcctcagct gccacctgac 1020
gacgtccccc tcggcgaggt gcactccggc tcgagcagcc accacggcca gttctcaggt 1080
gagctcgagc tcgccctgca gggaaacagg ccagcgccag ggtcggcggc accgccagcg 1140
ccgcgcaata atcaggctcc cgcgggcacg ttcgaccagg ctggcaacac gatggactgg 1200
tcgctctag 1209

<210> 261
<211> 402
<212> PRT
<213> Zea mays

<400> 261
Met Glu Ser Gly Gly Gly Gly Asp Asp Gln Leu His Gly Leu Lys Phe
1               5                   10                  15
Gly Lys Lys Ile Tyr Phe Glu Asp Ala Ala Gly Ser Ser Ser Gly Ser
            20                  25                  30
Ser Ser Gly Gly Gly Ser Ala Pro Ala Pro Pro Ala Thr Gln Gln Pro
        35                  40                  45
Ser Pro Pro Ala Ala Ser Pro Arg Ala Pro Ala Gly Gly Gly Arg Arg
    50                  55                  60
Gly Arg Ala Ala Ala Gly Gly Ala Gly Pro Ser Thr Ala Pro Ala Pro
65                  70                  75                  80
Ala Arg Cys Gln Val Asp Gly Cys Asn Val Asp Leu Thr Asp Val Lys
                85                  90                  95
Pro Ala Tyr Tyr Cys Arg His Lys Val Cys Lys Met His Ser Lys Glu
            100                 105                 110
Pro Arg Val Leu Val Asn Gly Leu Glu Gln Arg Phe Cys Gln Gln Cys
        115                 120                 125
Ser Arg Phe His Gln Leu Pro Glu Phe Asp Gln Leu Lys Lys Ser Cys
    130                 135                 140
Arg Lys Arg Leu Ala Gly His Asn Glu Arg Arg Arg Arg Pro Pro Pro
145                 150                 155                 160
Gly Pro Leu Ala Ser Arg Tyr Gly Arg His Ala Ala Ser Leu Gly Glu
                165                 170                 175
Pro Gly Arg Leu Arg Ser Phe Met Leu Asp Phe Ser Tyr Pro Arg Val
            180                 185                 190
Ser Ser Ala Met Arg Gly Gly Phe Pro Ala Val Arg Ala Gly Gly Glu
        195                 200                 205
Arg Val Pro Gly Gly Ile Gln Trp Gln Ala Gly Leu Asp Pro Arg His
    210                 215                 220
His Gln Gly Ala Val Ala Gly Tyr Gly Ala His Tyr Gly Ser Glu Gly
225                 230                 235                 240
Gly Ser Ser Ser Ser Ala Arg Pro Pro Val Phe Pro Gly Pro Glu Leu
                245                 250                 255
Pro Pro Gly Gly Cys Leu Ala Gly Val Pro Ala Asp Ser Ser Cys Ala
            260                 265                 270
Leu Ser Leu Leu Ser Thr Gln Pro Trp Asp Ala Ala His Ser His Ser
        275                 280                 285
His Ser His Ala Ala Pro Thr Ala Gly Phe Asp Gly Gly Ser Pro Val
    290                 295                 300
Ala Pro Ser Leu Met Ala Ala Ser Ser Tyr Ile Ala Pro Ser Pro Trp
305                 310                 315                 320

Thr Glu Thr Asp Ser Trp Gly His Glu Gly Gly Arg Ser Val Pro Gln
                325                 330                 335
Leu Pro Pro Asp Asp Val Pro Leu Gly Glu Val His Ser Gly Ser Ser
            340                 345                 350
Ser His His Gly Gln Phe Ser Gly Glu Leu Glu Leu Ala Leu Gln Gly
        355                 360                 365
Asn Arg Pro Ala Pro Gly Ser Ala Ala Pro Pro Ala Pro Arg Asn Asn
    370                 375                 380
Gln Gly Ser Ala Gly Thr Phe Asp Gln Ala Gly Asn Thr Met Asp Trp
385                 390                 395                 400
Ser Leu

<210>  262
<211>  1137
<212>  DNA
<213>  Zea mays

<400>  262
atggcgaccg gcggcggcag cagcaggagc gacgacgtgc gcgggctcaa gtttggcaag      60
aagatctact tcgagcagga cggcgggagc gggagcgggg cgggggcggt gggcggcagg     120
aaggggaagg gcgtggccac cggtggcgcg aggccggcgt ccgccgcctc cgcagcccag     180
ccgccgaggt gccaggtgga cgggtgcggc gtggatctga gcgccgtcaa gcagtactac     240
tgccggcaca aggtgtgcaa catgcactcc aaggagccgc gcgtcttcgt cgccggcatc     300
gagcagcgct tctgccaaca gtgcagcagg ttccaccagc tacatgaatt tgaccaaggg     360
aaacgtagct gccgccgccg cctcatcggt cacaacgagc gccggaggaa gccaccacct     420
ggacctctca cttcacgata tggccggctc gctgcatcac ttcaagagcc tggcaggttc     480
agaagcttcc tgctcgactt ctcgtaccca agggttccaa gcagcgtgag ggatgcgtgg     540
ccaggaatcc agcacggtgg cgacaggatg ctgggcaccg tccagtggca tgggcaccaa     600
gaacctcctc acccacaccg cagtgcagct gctggctatg gcaaccatgc tgcatacaac     660
tgccatggcg gcttggtagc aggcggggcc ccaatgctct cctctgccgc ctttgagctc     720
ccgcctggcg gatgtgtcgc gggagttgcc gccgactcca gctgtgctct ctctcttctg     780
tcaactcagc catgggacac gacctcccac gaccaccggt ccccagcaat gcccgcggcc     840
ggcgccttcg acggcacccc ggtggcgccg tccgtcatgg cgagcagcta cgcggcgtcg     900
agcgcctgga cgggctcgcg ggaccccgct gccgacggcg ccaggaacgc gcagcgtctc     960
gacgatgctc tgcacctggt ccacccaggc tccgcggcgg tccacttctc cggcgagctc    1020
gagctcgccc tgcagggaag cggcgggccg ccacacctgc cgcgcgtcga ccatggcggc    1080
tccggcggcg gcaccttcaa ccattccacc accagcgcga tgaactggtc cctgtag       1137

<210>  263
<211>  378
<212>  PRT
<213>  Zea mays

<400>  263
Met Ala Thr Gly Gly Gly Ser Ser Arg Ser Asp Asp Val Arg Gly Leu
1               5                   10                  15
Lys Phe Gly Lys Lys Ile Tyr Phe Glu Gln Asp Gly Gly Ser Gly Ser
            20                  25                  30
Gly Ala Gly Ala Val Gly Gly Arg Lys Gly Lys Gly Val Ala Thr Gly
        35                  40                  45
Gly Ala Arg Pro Ala Ser Ala Ala Ser Ala Ala Gln Pro Pro Arg Cys
    50                  55                  60
Gln Val Asp Gly Cys Gly Val Asp Leu Ser Ala Val Lys Gln Tyr Tyr
65                  70                  75                  80
Cys Arg His Lys Val Cys Asn Met His Ser Lys Glu Pro Arg Val Phe
                85                  90                  95
Val Ala Gly Ile Glu Gln Arg Phe Cys Gln Gln Cys Ser Arg Phe His
            100                 105                 110

Gln Leu His Glu Phe Asp Gln Gly Lys Arg Ser Cys Arg Arg Arg Leu
        115                 120                 125
Ile Gly His Asn Glu Arg Arg Arg Lys Pro Pro Pro Gly Pro Leu Thr
    130                 135                 140
Ser Arg Tyr Gly Arg Leu Ala Ala Ser Leu Gln Glu Pro Gly Arg Phe
145                 150                 155                 160
Arg Ser Phe Leu Leu Asp Phe Ser Tyr Pro Arg Val Pro Ser Ser Val
                165                 170                 175
Arg Asp Ala Trp Pro Gly Ile Gln His Gly Gly Asp Arg Met Leu Gly
            180                 185                 190
Thr Val Gln Trp His Gly His Gln Glu Pro Pro His Pro His Arg Ser
        195                 200                 205
Ala Ala Ala Gly Tyr Gly Asn His Ala Ala Tyr Asn Cys His Gly Gly
    210                 215                 220
Leu Val Ala Gly Gly Ala Pro Met Leu Ser Ser Ala Ala Phe Glu Leu
225                 230                 235                 240
Pro Pro Gly Gly Cys Val Ala Gly Val Ala Ala Asp Ser Ser Cys Ala
                245                 250                 255
Leu Ser Leu Leu Ser Thr Gln Pro Trp Asp Thr Thr Ser His Asp His
            260                 265                 270
Arg Ser Pro Ala Met Pro Ala Ala Gly Ala Phe Asp Gly Thr Pro Val
        275                 280                 285
Ala Pro Ser Val Met Ala Ser Ser Tyr Ala Ala Ser Ser Ala Trp Thr
    290                 295                 300
Gly Ser Arg Asp Pro Ala Ala Asp Gly Ala Arg Asn Ala Gln Arg Leu
305                 310                 315                 320
Asp Asp Ala Leu His Leu Val His Pro Gly Ser Ala Ala Val His Phe
                325                 330                 335
Ser Gly Glu Leu Glu Leu Ala Leu Gln Gly Ser Gly Gly Pro Pro His
            340                 345                 350
Leu Pro Arg Val Asp His Gly Gly Ser Gly Gly Gly Thr Phe Asn His
        355                 360                 365
Ser Thr Thr Ser Ala Met Asn Trp Ser Leu
    370                 375

<210>  264
<211>  403
<212>  DNA
<213>  Sorghum propinquum

<400>  264
atggagtccg gtggcgccag tggcggcggc ggcggggacg accagctgca cggcctcaag      60
ttcggcaaga agatctactt cgaggacgcc gccgcggtcg gctccagcag cggtggcggt     120
ggcggtggca gtggcagtgc tagcgcgacc cccgcgcctc cagcgacgca gcagccgtcc     180
ccgccgcagg ccgcttcgcc cagggcagcc agcggcggcg gcggcaggag gggcagggcc     240
gcgggcggcc cctccccggc gcccgcgccc gcgcgctgcc aggtcgacgg ctgcaacgtg     300
gacctcaccg acgtcaagcc ctactactgc cgccacaagg tctgcaaaat gcactccaag     360
gagccccgcg tcgtcgtcaa cggcctcgag cagcgcttct gcc                       403

<210>  265
<211>  134
<212>  PRT
<213>  Sorghum propinquum

<400>  265
   Met Glu Ser Gly Gly Ala Ser Gly Gly Gly Gly Gly Asp Asp Gln Leu
   1               5                   10                  15
   His Gly Leu Lys Phe Gly Lys Lys Ile Tyr Phe Glu Asp Ala Ala Ala
               20                  25                  30
   Val Gly Ser Ser Ser Gly Gly Gly Gly Gly Gly Ser Gly Ser Ala Ser
           35                  40                  45
   Ala Thr Pro Ala Pro Pro Ala Thr Gln Gln Pro Ser Pro Pro Gln Ala
       50                  55                  60
   Ala Ser Pro Arg Ala Ala Ser Gly Gly Gly Gly Arg Arg Gly Arg Ala
   65                  70                  75                  80
   Ala Gly Gly Pro Ser Pro Ala Pro Ala Pro Ala Arg Cys Gln Val Asp
                   85                  90                  95
   Gly Cys Asn Val Asp Leu Thr Asp Val Lys Pro Tyr Tyr Cys Arg His
               100                 105                 110
   Lys Val Cys Lys Met His Ser Lys Glu Pro Arg Val Val Val Asn Gly
           115                 120                 125
   Leu Glu Gln Arg Phe Cys
       130

<210>  266
<211>  732
<212>  DNA
<213>  Allium cepa

<400>  266
atggaaatgg gttcgagctc tgagccaatt tctgactctc tcgatgggct cacattcggc      60
gaaaaaattt actttgaaga ttccactacc agtactgcaa gtactactag caatacaatt     120
gctgctcctc ccaaaaaggg aaaatcttta gcttcttcgt ccactaatat caactctaat     180
cagcaacaaa taagcatacc tagatgccag gttgaaggat gcaaagttga tttgactgga     240
gctaaagcct attactgcag gcataaggtt tgcggagttc attcaaaatc accaaaagtt     300
gtggttgctg gaattgaaca gaggttttgt cagcagtgca gcaggttcca ccagttgcaa     360
gaatttgacc aaggaaaaag aagctgccgc agacgcctag ctggacacaa tgagcgtcgt     420
agaaagccgc ctccaggccc ttcccgttat ggaaggatgt catcatctgt ctatgatggc     480
agtaacagat tcagaggatt cctgatggac ttcagttacc cgaggcccat acagcctcct     540
ccaggagatc atctatggcc tagactgtcc aacattccat catacccagc aaacccaaac     600
actgcatcta acattcatgt tgctcaaccg tacatgcatg caatgaatca ctacagttcc     660
gagcttccac acaggaatac atggtctggc gtctgcgact tgagctgtgc tctctctctt     720
ctgtcactca ac                                                         732

<210>  267
<211>  244
<212>  PRT
<213>  Allium cepa

<400>  267
Met Glu Met Gly Ser Ser Ser Glu Pro Ile Ser Asp Ser Leu Asp Gly
1               5                   10                  15
Leu Thr Phe Gly Glu Lys Ile Tyr Phe Glu Asp Ser Thr Thr Ser Thr
            20                  25                  30
Ala Ser Thr Thr Ser Asn Thr Ile Ala Ala Pro Pro Lys Lys Gly Lys
        35                  40                  45
Ser Leu Ala Ser Ser Ser Thr Asn Ile Asn Ser Asn Gln Gln Gln Ile
    50                  55                  60
Ser Ile Pro Arg Cys Gln Val Glu Gly Cys Lys Val Asp Leu Thr Gly
65                  70                  75                  80
Ala Lys Ala Tyr Tyr Cys Arg His Lys Val Cys Gly Val His Ser Lys
                85                  90                  95
Ser Pro Lys Val Val Val Ala Gly Ile Glu Gln Arg Phe Cys Gln Gln
            100                 105                 110
Cys Ser Arg Phe His Gln Leu Gln Glu Phe Asp Gln Gly Lys Arg Ser
        115                 120                 125
Cys Arg Arg Arg Leu Ala Gly His Asn Glu Arg Arg Arg Lys Pro Pro
    130                 135                 140

Pro Gly Pro Ser Arg Tyr Gly Arg Met Ser Ser Ser Val Tyr Asp Gly
145                 150                 155                 160
Ser Asn Arg Phe Arg Gly Phe Leu Met Asp Phe Ser Tyr Pro Arg Pro
                165                 170                 175
Ile Gln Pro Pro Pro Gly Asp His Leu Trp Pro Arg Leu Ser Asn Ile
            180                 185                 190
Pro Ser Tyr Pro Ala Asn Pro Asn Thr Ala Ser Asn Ile His Val Ala
        195                 200                 205
Gln Pro Tyr Met His Ala Met Asn His Tyr Ser Ser Glu Leu Pro His
    210                 215                 220
Arg Asn Thr Trp Ser Gly Val Cys Asp Leu Ser Cys Ala Leu Ser Leu
225                 230                 235                 240
Leu Ser Leu Asn

<210>  268
<211>  969
<212>  DNA
<213>  Antirrhinum majus

<400>  268
tcagctgctg gtggagcaga ggaatctctc aatgggttga agtttggcaa gaaaatatac      60
tttgaggagg ctaaggcaaa gaaagggaag agtaccggtg gggtggttag gtgccaggtg     120
gaggggtgtg aggtagatct gagtgatgct aaggcttact atttgagaca caaagtttgt     180
agtatgcatt caaagtctcc aaaggtcatt gttgctggaa tagaacaaag gttttgccag     240
cagtgcagca ggttccatca attgcctgaa tttgaccaag gaaaacggag ttgccgcaga     300
cgccttgcag gccacaacga acgtcggagg aagccatctc caggatctat gatgtctcct     360
tactatggaa gtctttctcc aaccttattt gataaccaaa atagaactgg aggctttttg     420
atggacttca gcacttaccc aaatctcgct gggaaagatt catggccaaa tacaataccc     480
gaacgaggat tgggaggtcc agcaagtcca tggcagagcg acatgcaaaa tcctgtacct     540
gagtttttgc gaggtacaac aaataggcca agtttttctg gtcttggagt atcttccgaa     600
gaatgtttta gcggagtctc taattccagc actgctctct ctcttctgtc aaatcagtcc     660
tggggctcca gaaactcgaa caatttcttg ggtaccaatg gaaacgggcc aaccatagtt     720
cagccgtcta ttaaccctgg tgccacaatt ggacagttta cctgtccctc ttggggtttt     780
ggaggcaacc cagctgataa cacctcccat gatatgcctc ccaatctgaa tttaggacaa     840
ttttctcact ccagtaacag tcactatact ggagagcctg gggtagtcca actgagccac     900
ggacaattcc aggacctcga tcactcaaga ggctatgatt cttccgttca ggacatgcat     960
tggtcactt                                                             969

<210>  269
<211>  323
<212>  PRT
<213>  Antirrhinum majus

<400>  269
Ser Ala Ala Gly Gly Ala Glu Glu Ser Leu Asn Gly Leu Lys Phe Gly
1               5                   10                  15
Lys Lys Ile Tyr Phe Glu Glu Ala Lys Ala Lys Lys Gly Lys Ser Thr
            20                  25                  30
Gly Gly Val Val Arg Cys Gln Val Glu Gly Cys Glu Val Asp Leu Ser
        35                  40                  45
Asp Ala Lys Ala Tyr Tyr Leu Arg His Lys Val Cys Ser Met His Ser
    50                  55                  60
Lys Ser Pro Lys Val Ile Val Ala Gly Ile Glu Gln Arg Phe Cys Gln
65                  70                  75                  80
Gln Cys Ser Arg Phe His Gln Leu Pro Glu Phe Asp Gln Gly Lys Arg
                85                  90                  95
Ser Cys Arg Arg Arg Leu Ala Gly His Asn Glu Arg Arg Arg Lys Pro
            100                 105                 110

Ser Pro Gly Ser Met Met Ser Pro Tyr Tyr Gly Ser Leu Ser Pro Thr
        115                 120                 125
Leu Phe Asp Asn Gln Asn Arg Thr Gly Gly Phe Leu Met Asp Phe Ser
    130                 135                 140
Thr Tyr Pro Asn Leu Ala Gly Lys Asp Ser Trp Pro Asn Thr Ile Pro
145                 150                 155                 160
Glu Arg Gly Leu Gly Gly Pro Ala Ser Pro Trp Gln Ser Asp Met Gln
                165                 170                 175
Asn Pro Val Pro Glu Phe Leu Arg Gly Thr Thr Asn Arg Pro Ser Phe
            180                 185                 190
Ser Gly Leu Gly Val Ser Ser Glu Glu Cys Phe Ser Gly Val Ser Asn
        195                 200                 205
Ser Ser Thr Ala Leu Ser Leu Leu Ser Asn Gln Ser Trp Gly Ser Arg
    210                 215                 220
Asn Ser Asn Asn Phe Leu Gly Thr Asn Gly Asn Gly Pro Thr Ile Val
225                 230                 235                 240
Gln Pro Ser Ile Asn Pro Gly Ala Thr Ile Gly Gln Phe Thr Cys Pro
                245                 250                 255
Ser Trp Gly Phe Gly Gly Asn Pro Ala Asp Asn Thr Ser His Asp Met
            260                 265                 270
Pro Pro Asn Leu Asn Leu Gly Gln Phe Ser His Ser Ser Asn Ser His
        275                 280                 285
Tyr Thr Gly Glu Pro Gly Val Val Gln Leu Ser His Gly Gln Phe Gln
    290                 295                 300
Asp Leu Asp His Ser Arg Gly Tyr Asp Ser Ser Val Gln Asp Met His
305                 310                 315                 320
Trp Ser Leu

<210>  270
<211>  633
<212>  DNA
<213>  Brassica napus

<400>  270
gacttggaga aaagaagttg tagaagacgt ctagcttgtc ataacgaaag aagaagaaag      60
ccccaagcaa caacagcagc tcttttggct tctggttact ctagaatcgc tccatctctt     120
tacggaagcg ttttgggaga tcctacaacg tggtcaaccg caagatctgt gatgggacgg     180
tccgcaccgt gggatagcca tcaactgatg aacgttttgt cacagggaag ttcaaggttt     240
agtataacat acccagagat ggtgaacaat aatagcacag actcaagctg tgctctctct     300
cttctgtcaa actcaaacac aactcagcag cagcagcaga catcaaccaa tgcttacttg     360
atggacgcag aaagggttac aatggctaag tcaccgcctg tttcagtaca caatcagtac     420
tcgaaacaaa cctgggagtt catgtcaggc gaaaagagca attggccttg tgtgtcgtcc     480
cctgttttgg gactgagaca aatctctgag ccagatgatg acctccagtt cctgatgagc     540
aatggcacca caatgggtgg attcgagctg aacctacagc aggagcaggt tctgaggcaa     600
tactcttcta ctcaaaattt tacttggcct ctt                                  633

<210>  271
<211>  211
<212>  PRT
<213>  Brassica napus

<400>  271
Asp Leu Glu Lys Arg Ser Cys Arg Arg Arg Leu Ala Cys His Asn Glu
1               5                   10                  15
Arg Arg Arg Lys Pro Gln Ala Thr Thr Ala Ala Leu Leu Ala Ser Gly
            20                  25                  30
Tyr Ser Arg Ile Ala Pro Ser Leu Tyr Gly Ser Val Leu Gly Asp Pro
        35                  40                  45

Thr Thr Trp Ser Thr Ala Arg Ser Val Met Gly Arg Ser Ala Pro Trp
    50                  55                  60
Asp Ser His Gln Leu Met Asn Val Leu Ser Gln Gly Ser Ser Arg Phe
65                  70                  75                  80
Ser Ile Thr Tyr Pro Glu Met Val Asn Asn Asn Ser Thr Asp Ser Ser
                85                  90                  95
Cys Ala Leu Ser Leu Leu Ser Asn Ser Asn Thr Thr Gln Gln Gln Gln
            100                 105                 110
Gln Thr Ser Thr Asn Ala Tyr Leu Met Asp Ala Glu Arg Val Thr Met
        115                 120                 125
Ala Lys Ser Pro Pro Val Ser Val His Asn Gln Tyr Ser Lys Gln Thr
    130                 135                 140
Trp Glu Phe Met Ser Gly Glu Lys Ser Asn Trp Pro Cys Val Ser Ser
145                 150                 155                 160
Pro Val Leu Gly Leu Arg Gln Ile Ser Glu Pro Asp Asp Asp Leu Gln
                165                 170                 175
Phe Leu Met Ser Asn Gly Thr Thr Met Gly Gly Phe Glu Leu Asn Leu
            180                 185                 190
Gln Gln Glu Gln Val Leu Arg Gln Tyr Ser Ser Thr Gln Asn Phe Thr
        195                 200                 205
Trp Pro Leu
    210

<210>  272
<211>  852
<212>  DNA
<213>  Saccharum officinarum

<400>  272
ctcgtcgtca acggcctcga gcagcgcttc tgccagcagt gcagcaggtt ccaccagctg      60
cctgaatttg accaactaaa gaaaagctgc cgcagacgtc ttgcaggcca caatgaacgc     120
cggaggaggc cacctcctgg acctcttgca tcacgatatg gtcgccttgc tgcatcattt     180
ggtgagcccg gcaggttccg aagctttatg ttggatttct catacccaag ggttccaggc     240
accatgaggg atgggtttcc ggcagttcga cctggcgaaa gggtgcctgg tagtatccag     300
tggcaagcgg gcttagatcc tcatcatcat caaagcgcgg tcgcaggata cggtgcccac     360
tcatatggga gccagggtag ctcgtcgtcg tcaaggccac cggtgttccc tggtccagag     420
ctccccccag gtggatgtct tgcaggagtc ccctcggact ctagctgtgc tctctctctt     480
ctgtcaactc agccatggga tactacccac agcgccggcc acagccatgc tggatcaatg     540
cctgcaacag caggttttga cggcaaccct gtggcaccct ccctcatggc gagtagctac     600
attgcgccaa gcccctggac tgactcccgg ggccatgaag gcgggcggaa cgtgcctcag     660
ttgccacctg acgtccccct cagcgaggtg cactctggct caagcagcca tcacggccag     720
ttctcaggtg agctcgagct tgccctgcag ggaaacaggc cagcaccagg gtcagcgcca     780
gcgccgcgca atgatcaggg ctccacgggc acgttcgacc agtccggcaa cacaatggac     840
tggtcgctct ag                                                         852

<210>  273
<211>  283
<212>  PRT
<213>  Saccharum officinarum

<400>  273
Leu Val Val Asn Gly Leu Glu Gln Arg Phe Cys Gln Gln Cys Ser Arg
1               5                   10                  15
Phe His Gln Leu Pro Glu Phe Asp Gln Leu Lys Lys Ser Cys Arg Arg
            20                  25                  30
Arg Leu Ala Gly His Asn Glu Arg Arg Arg Arg Pro Pro Pro Gly Pro
        35                  40                  45
Leu Ala Ser Arg Tyr Gly Arg Leu Ala Ala Ser Phe Gly Glu Pro Gly
    50                  55                  60

Arg Phe Arg Ser Phe Met Leu Asp Phe Ser Tyr Pro Arg Val Pro Gly
65                  70                  75                  80
Thr Met Arg Asp Gly Phe Pro Ala Val Arg Pro Gly Glu Arg Val Pro
                85                  90                  95
Gly Ser Ile Gln Trp Gln Ala Gly Leu Asp Pro His His His Gln Ser
            100                 105                 110
Ala Val Ala Gly Tyr Gly Ala His Ser Tyr Gly Ser Gln Gly Ser Ser
        115                 120                 125
Ser Ser Ser Arg Pro Pro Val Phe Pro Gly Pro Glu Leu Pro Pro Gly
    130                 135                 140
Gly Cys Leu Ala Gly Val Pro Ser Asp Ser Ser Cys Ala Leu Ser Leu
145                 150                 155                 160
Leu Ser Thr Gln Pro Trp Asp Thr Thr His Ser Ala Gly His Ser His
                165                 170                 175
Ala Gly Ser Met Pro Ala Thr Ala Gly Phe Asp Gly Asn Pro Val Ala
            180                 185                 190
Pro Ser Leu Met Ala Ser Ser Tyr Ile Ala Pro Ser Pro Trp Thr Asp
        195                 200                 205
Ser Arg Gly His Glu Gly Gly Arg Asn Val Pro Gln Leu Pro Pro Asp
    210                 215                 220
Val Pro Leu Ser Glu Val His Ser Gly Ser Ser Ser His His Gly Gln
225                 230                 235                 240
Phe Ser Gly Glu Leu Glu Leu Ala Leu Gln Gly Asn Arg Pro Ala Pro
                245                 250                 255
Gly Ser Ala Pro Ala Pro Arg Asn Asp Gln Gly Ser Thr Gly Thr Phe
            260                 265                 270
Asp Gln Ser Gly Asn Thr Met Asp Trp Ser Leu
        275                 280

<210>  274
<211>  647
<212>  DNA
<213>  Festuca arundinacea

<400>  274
ggcacgaggg tggatctgag cggctccaag acctactact gccgccacaa ggtctgctcc      60
atgcactcca aggcgccccg cgtcgtcgtc gccggcctcg agcagcgctt ctgccagcag     120
tgcagcaggt tccaccagtt gcctgaattt gacaatggaa aacgcagctg ccgcagacgt     180
ctcgcaggtc acaatgaacg ccgtaggaag ccgcctcctg gccctctggc gtcacgctat     240
ggccgactcg ctgcatcctt tgaagaaccg ggcaggtaca gaagctttct gttagatttc     300
tcctacccaa gggttccgag cagcgtgcgg gatgcttggc ctgcagttcg accaggctac     360
cgtatgccca gtgaaatcca gtggcaaggg aacctagacc tgcgtcctca cacgggttat     420
ggcccacatg catatggcag ccacggcttc cccggtccag agctccctcc aggcgggtgt     480
ctcacagggg tcgccaccga ctccagctgt gctctctctc ttctgtcaac tcagccatgg     540
gataccacca cccacggtgc cagccacgac catcggtctg cggccatgtc cgcggccgcg     600
ggcttcgacg gcagccctgc ggcagtgtca ccctccatca tggcgag                   647

<210>  275
<211>  215
<212>  PRT
<213>  Festuca arundinacea

<400>  275
Gly Thr Arg Val Asp Leu Ser Gly Ser Lys Thr Tyr Tyr Cys Arg His
1               5                   10                  15
Lys Val Cys Ser Met His Ser Lys Ala Pro Arg Val Val Val Ala Gly
            20                  25                  30
Leu Glu Gln Arg Phe Cys Gln Gln Cys Ser Arg Phe His Gln Leu Pro
        35                  40                  45

Glu Phe Asp Asn Gly Lys Arg Ser Cys Arg Arg Arg Leu Ala Gly His
    50                  55                  60
Asn Glu Arg Arg Arg Lys Pro Pro Pro Gly Pro Leu Ala Ser Arg Tyr
65                  70                  75                  80
Gly Arg Leu Ala Ala Ser Phe Glu Glu Pro Gly Arg Tyr Arg Ser Phe
                85                  90                  95
Leu Leu Asp Phe Ser Tyr Pro Arg Val Pro Ser Ser Val Arg Asp Ala
            100                 105                 110
Trp Pro Ala Val Arg Pro Gly Tyr Arg Met Pro Ser Glu Ile Gln Trp
        115                 120                 125
Gln Gly Asn Leu Asp Leu Arg Pro His Thr Gly Tyr Gly Pro His Ala
    130                 135                 140
Tyr Gly Ser His Gly Phe Pro Gly Pro Glu Leu Pro Pro Gly Gly Cys
145                 150                 155                 160
Leu Thr Gly Val Ala Thr Asp Ser Ser Cys Ala Leu Ser Leu Leu Ser
                165                 170                 175
Thr Gln Pro Trp Asp Thr Thr Thr His Gly Ala Ser His Asp His Arg
            180                 185                 190
Ser Ala Ala Met Ser Ala Ala Ala Gly Phe Asp Gly Ser Pro Ala Ala
        195                 200                 205
Val Ser Pro Ser Ile Met Ala
    210                 215

<210>  276
<211>  11
<212>  PRT
<213>  Artificial sequence

<220>
<223>  motif 1

<220>
<221>  VARIANT
<222>  (1)..(1)
<223>  /replace = "Thr"

<220>
<221>  VARIANT
<222>  (3)..(3)
<223>  /replace = "Arg" /replace = "Met" /replace = "Gln"

<220>
<221>  VARIANT
<222>  (6)..(6)
<223>  /replace = "Gln" /replace = "Arg"

<220>
<221>  VARIANT
<222>  (7)..(7)
<223>  /replace = "Arg" /replace = "Glu"

<220>
<221>  VARIANT
<222>  (8)..(8)
<223>  /replace = "Val"

<220>
<221>  VARIANT
<222>  (11)..(11)

<223>  /replace = "Gly"

<400>  276
Gly Leu Lys Phe Gly Lys Lys Ile Tyr Phe Glu
1               5                   10

<210>  277
<211>  78
<212>  PRT
<213>  Artificial sequence

<220>
<223>  SPL DNA-binding domain (DBD) of Arath_SPL15 transcription factor

<400>  277
Thr Ala Arg Cys Gln Val Glu Gly Cys Arg Met Asp Leu Ser Asn Val
1               5                   10                  15
Lys Ala Tyr Tyr Ser Arg His Lys Val Cys Cys Ile His Ser Lys Ser
            20                  25                  30
Ser Lys Val Ile Val Ser Gly Leu His Gln Arg Phe Cys Gln Gln Cys
        35                  40                  45
Ser Arg Phe His Gln Leu Ser Glu Phe Asp Leu Glu Lys Arg Ser Cys
    50                  55                  60
Arg Arg Arg Leu Ala Cys His Asn Glu Arg Arg Arg Lys Pro
65                  70                  75

<210>  278
<211>  11
<212>  PRT
<213>  Artificial sequence

<220>
<223>  motif 2

<220>
<221>  VARIANT
<222>  (3)..(3)
<223>  /replace = "Asn" /replace = "Thr"

<220>
<221>  VARIANT
<222>  (4)..(4)
<223>  /replace = "Gly" /replace = "Arg"

<220>
<221>  VARIANT
<222>  (11)..(11)
<223>  /replace = "Thr"

<400>  278
Asp Ser Ser Cys Ala Leu Ser Leu Leu Ser Asn
1               5                   10

<210>  279
<211>  1130
<212>  DNA
<213>  Oryza sativa

<400>  279

catgcggcta atgtagatgc tcactgcgct agtagtaagg tactccagta cattatggaa 60
tatacaaagc tgtaatactc gtatcagcaa gagagaggca cacaagttgt agcagtagca 120
caggattaga aaaacgggac gacaaatagt aatggaaaaa caaaaaaaaa caaggaaaca 180
catggcaata taaatggaga aatcacaaga ggaacagaat ccgggcaata cgctgcgaaa 240
gtactcgtac gtaaaaaaaa gaggcgcatt catgtgtgga cagcgtgcag cagaagcagg 300
gatttgaaac cactcaaatc caccactgca aaccttcaaa cgaggccatg gtttgaagca 360
tagaaagcac aggtaagaag cacaacgccc tcgctctcca ccctcccacc caatcgcgac 420
gcacctcgcg gatcggtgac gtggcctcgc cccccaaaaa tatcccgcgg cgtgaagctg 480
acaccccggg cccacccacc tgtcacgttg gcacatgttg gttatggttc ccggccgcac 540
caaaatatca acgcggcgcg gcccaaaatt tccaaaatcc cgcccaagcc cctggcgcgt 600
gccgctcttc cacccaggtc cctctcgtaa tccataatgg cgtgtgtacc ctcggctggt 660
tgtacgtggg cgggttaccc tgggggtgtg ggtggatgac gggtgggccc ggaggaggtc 720
cggccccgcg cgtcatcgcg gggcggggtg tagcgggtgc gaaaaggagg cgatcggtac 780
gaaaattcaa attaggaggt gggggcgggg gcccttggag aataagcgga atcgcagata 840
tgcccctgac ttggcttggc tcctcttctt cttatccctt gtcctcgcaa ccccgcttcc 900
ttctctcctc tcctcttctc ttctcttctc tggtggtgtg ggtgtgtccc tgtctcccct 960
ctccttcctc ctctcctttc ccctcctctc ttcccccctc tcacaagaga gagagcgcca 1020
gactctcccc aggtgaggtg agaccagtct ttttgctcga ttcgacgcgc ctttcacgcc 1080
gcctcgcgcg gatctgaccg cttccctcgc ccttctcgca ggattcagcc 1130

<210> 280
<211> 56
<212> DNA
<213> Artificial sequence

<220>
<223> primer: prm07277

<400> 280
ggggacaagt ttgtacaaaa aagcaggctt aaacaatgga gttgttaatg tgttcg 56

<210> 281
<211> 51
<212> DNA
<213> Artificial sequence

<220>
<223> primer: prm07278

<400> 281
ggggaccact ttgtacaaga aagctgggtt gatgaagatc ttaaaaggtg a 51

<210> 282
<211> 1110
<212> DNA
<213> Brassica rapa

<400> 282
atggataggg gttccaactc gggtcttggt cttggtccag gccagacaga gtcgggtggt 60
tcatccactg agtcatcctc tttaagtgga gggctcatgt ttggccagag gatctacttc 120
gaggacgctg gaggtggaac cgggtcttct tcctccggcg ggtcaaacag aagggtacgt 180
ggaagcgggt cgggtccttc gggtcagata ccaaggtgtc aagtggaagg ttgtggaatg 240
gatctaacca atgcaaaggg ttattacacg aggcatagag tttgtggaat gcactctaaa 300
acaccaaaag tcattgtcgc tggtatagaa caaaggtttt gtcaacagtg cagcaggttt 360
catcagcttc cggaatttga cctagagaaa aggagttgcc gtaggagact cgctggtcat 420
aatgagagac gaaggaagcc acagcctgcg tctctatctg tgttgtcttc tcgttatggg 480
aggatcactc cttctctata cggaaatggt gaaactacaa tgaatgggag ctttcttggt 540
tcccaagaaa tgggttggaa tagtgcaaga acgttggata caagagtgat gagacggcca 600
ccgtcgtggc agatcaatcc tatgaatgtg tttagtcatg gatcagtaag tggaggagga 660

ggaggaggga taagcttctc atctccagag attatggaca ctaaaccaga gagctacaag 720
ggaattggca gcgactcaaa ctgtgctctc tctcttctgt caaacccaca tcagccacat 780
gacaacaaca acagcaacaa cacatggaga acttcttcgg gttttggtcc gatgacggtt 840
acaatggctc agccaccacc tgcacctagc cagcagcatc agtatctgaa cccgccttgg 900
gtattcaagg acgatgataa tagctgtccg aatgatatgt ctcctgtttt gaatcttggt 960
cggttcaccg agacagagat aagcggtgga acgactttgg gtgagttcga gttatctgac 1020
catcatcatc agaataggag gcagtacatg gaaagtgaga acacaagggc ttatggctct 1080
tcttcacacc ataacaactg gtctctctga 1110

<210> 283
<211> 369
<212> PRT
<213> Brassica rapa

<400> 283
   Met Asp Arg Gly Ser Asn Ser Gly Leu Gly Leu Gly Pro Gly Gln Thr
   1 5 10 15
   Glu Ser Gly Gly Ser Ser Thr Glu Ser Ser Ser Leu Ser Gly Gly Leu
   20 25 30
   Met Phe Gly Gln Arg Ile Tyr Phe Glu Asp Ala Gly Gly Gly Thr Gly
   35 40 45
   Ser Ser Ser Ser Gly Gly Ser Asn Arg Arg Val Arg Gly Ser Gly Ser
   50 55 60
   Gly Pro Ser Gly Gln Ile Pro Arg Cys Gln Val Glu Gly Cys Gly Met
   65 70 75 80
   Asp Leu Thr Asn Ala Lys Gly Tyr Tyr Thr Arg His Arg Val Cys Gly
   85 90 95
   Met His Ser Lys Thr Pro Lys Val Ile Val Ala Gly Ile Glu Gln Arg
   100 105 110
   Phe Cys Gln Gln Cys Ser Arg Phe His Gln Leu Pro Glu Phe Asp Leu
   115 120 125
   Glu Lys Arg Ser Cys Arg Arg Arg Leu Ala Gly His Asn Glu Arg Arg
   130 135 140
   Arg Lys Pro Gln Pro Ala Ser Leu Ser Val Leu Ser Ser Arg Tyr Gly
   145 150 155 160
   Arg Ile Thr Pro Ser Leu Tyr Gly Asn Gly Glu Thr Thr Met Asn Gly
   165 170 175
   Ser Phe Leu Gly Ser Gln Glu Met Gly Trp Asn Ser Ala Arg Thr Leu
   180 185 190
   Asp Thr Arg Val Met Arg Arg Pro Pro Ser Trp Gln Ile Asn Pro Met
   195 200 205
   Asn Val Phe Ser His Gly Ser Val Ser Gly Gly Gly Gly Gly Gly Ile
   210 215 220
   Ser Phe Ser Ser Pro Glu Ile Met Asp Thr Lys Pro Glu Ser Tyr Lys
   225 230 235 240
   Gly Ile Gly Ser Asp Ser Asn Cys Ala Leu Ser Leu Leu Ser Asn Pro
   245 250 255
   His Gln Pro His Asp Asn Asn Asn Ser Asn Asn Thr Trp Arg Thr Ser
   260 265 270
   Ser Gly Phe Gly Pro Met Thr Val Thr Met Ala Gln Pro Pro Pro Ala
   275 280 285
   Pro Ser Gln Gln His Gln Tyr Leu Asn Pro Pro Trp Val Phe Lys Asp
   290 295 300
   Asp Asp Asn Ser Cys Pro Asn Asp Met Ser Pro Val Leu Asn Leu Gly
   305 310 315 320
   Arg Phe Thr Glu Thr Glu Ile Ser Gly Gly Thr Thr Leu Gly Glu Phe
   325 330 335
   Glu Leu Ser Asp His His His Gln Asn Arg Arg Gln Tyr Met Glu Ser
   340 345 350
   Glu Asn Thr Arg Ala Tyr Gly Ser Ser Ser His His Asn Asn Trp Ser
           355                 360                 365
   Leu

<210>  284
<211>  1080
<212>  DNA
<213>  Glycine max

<400>  284
atggcttcag acgccaaact ctcctcttct gagtccctca acggtttgaa attcggccaa      60
aaaatctatt ttgaggatgt tggtcttgct actccagcca cctctcttac ttcttcttct     120
tcttcttctt ctgctgctac tgttacttct tcttcttctt caaggaaagg aagaggtggg     180
tctgttcaac cagctcaacc tcccaggtgt caagttgaag ggtgcaaagt agatctgagt     240
gatgcaaaag cttactattc tagacacaag gtctgtggca tgcactctaa atccccttca     300
gtcattgttg ctggtcttca acaaaggttt tgtcaacagt gtagcaggtt tcatcagctt     360
cctgagtttg atcaaggaaa aagaagttgc cgtaggcgac tagctggcca taatgaacgt     420
cggagaaagc ccccaacaag ctccctctta acctctcgct atgccagact ttcttcgtct     480
gcttttgata atagtggcag aggtagcaac tttctgatgg aattgacttc atacccaaag     540
cttagtctga gaaattcact tccaactcct agatcatctg agctagctcc tggaaatcaa     600
acctccacac ttagctggaa tggcaactca gagacatcat ctgacctttt cttgcaaggt     660
tcggtgggtg ggacaagctt cgccagcccg ggacatcctc caggggaaag ttacattggg     720
gtcaccgaca cgagctgtgc tctctctctt ctgtcaaatc aaacatgggg ttctagaaac     780
acagcaccaa gtcttgggtt gagtaacatg ataaatttca acgggacacc cttgacacaa     840
cttgctgcat catctcatgg tgcatcaatc catcaacttc caaatacctc gtggtttttc     900
aagggcattg attctggtaa ctgttcgccc gaggtggtcc ctgatctagg tctcggtcag     960
atttcacagc ctctcaatag ccaacttcat ggtgagctgg acctgtccca acagggcagg    1020
aggcattata tggatctaga acagtccagg gcatatgaat ctgctcattg gtcactttaa    1080

<210>  285
<211>  359
<212>  PRT
<213>  Glycine max

<400>  285
Met Ala Ser Asp Ala Lys Leu Ser Ser Ser Glu Ser Leu Asn Gly Leu
1               5                   10                  15
Lys Phe Gly Gln Lys Ile Tyr Phe Glu Asp Val Gly Leu Ala Thr Pro
            20                  25                  30
Ala Thr Ser Leu Thr Ser Ser Ser Ser Ser Ser Ser Ala Ala Thr Val
        35                  40                  45
Thr Ser Ser Ser Ser Ser Arg Lys Gly Arg Gly Gly Ser Val Gln Pro
    50                  55                  60
Ala Gln Pro Pro Arg Cys Gln Val Glu Gly Cys Lys Val Asp Leu Ser
65                  70                  75                  80
Asp Ala Lys Ala Tyr Tyr Ser Arg His Lys Val Cys Gly Met His Ser
                85                  90                  95
Lys Ser Pro Ser Val Ile Val Ala Gly Leu Gln Gln Arg Phe Cys Gln
            100                 105                 110
Gln Cys Ser Arg Phe His Gln Leu Pro Glu Phe Asp Gln Gly Lys Arg
        115                 120                 125
Ser Cys Arg Arg Arg Leu Ala Gly His Asn Glu Arg Arg Arg Lys Pro
    130                 135                 140
Pro Thr Ser Ser Leu Leu Thr Ser Arg Tyr Ala Arg Leu Ser Ser Ser
145                 150                 155                 160
Ala Phe Asp Asn Ser Gly Arg Gly Ser Asn Phe Leu Met Glu Leu Thr
                165                 170                 175
Ser Tyr Pro Lys Leu Ser Leu Arg Asn Ser Leu Pro Thr Pro Arg Ser

180                 185                 190
Ser Glu Leu Ala Pro Gly Asn Gln Thr Ser Thr Leu Ser Trp Asn Gly
        195                 200                 205
Asn Ser Glu Thr Ser Ser Asp Leu Phe Leu Gln Gly Ser Val Gly Gly
    210                 215                 220
Thr Ser Phe Ala Ser Pro Gly His Pro Pro Gly Glu Ser Tyr Ile Gly
225                 230                 235                 240
Val Thr Asp Thr Ser Cys Ala Leu Ser Leu Leu Ser Asn Gln Thr Trp
                245                 250                 255
Gly Ser Arg Asn Thr Ala Pro Ser Leu Gly Leu Ser Asn Met Ile Asn
            260                 265                 270
Phe Asn Gly Thr Pro Leu Thr Gln Leu Ala Ala Ser Ser His Gly Ala
        275                 280                 285
Ser Ile His Gln Leu Pro Asn Thr Ser Trp Phe Phe Lys Gly Ile Asp
    290                 295                 300
Ser Gly Asn Cys Ser Pro Glu Val Val Pro Asp Leu Gly Leu Gly Gln
305                 310                 315                 320
Ile Ser Gln Pro Leu Asn Ser Gln Leu His Gly Glu Leu Asp Leu Ser
                325                 330                 335
Gln Gln Gly Arg Arg His Tyr Met Asp Leu Glu Gln Ser Arg Ala Tyr
            340                 345                 350
Glu Ser Ala His Trp Ser Leu
        355

<210>  286
<211>  1128
<212>  DNA
<213>  Populus tremuloides

<400>  286
atggaaatgg attcaggctc cctaaccgag tcagctactt ccaatgcaac ttctccgcca      60
gctgagtctg ttaatggatt gaaatttggt aagaagattt actttgagga tcacgtgggg     120
gtcggtgctc cggctaagag cggaactggg tcatcctcat ccggttccgg gtcagggtca     180
tctaggaagg ctcaaggtgg acagcaccag cagccaccaa ggtgtcaagt tgaagggtgc     240
aaagtagatc tgagtgatgc taagacttac tattcaaggc acaaagtttg tagtatgcac     300
tccaagtctc ctagagttat tgttgctggt ttggtgcaaa gattttgcca gcaatgtagc     360
agatttcatc tacttcctga atttgaccaa ggaaaacgaa gttgccgcag gcgcctagct     420
ggccataatg agcgacggag gaagccacca tctggatcgg tgttgtccgc tcgccatggc     480
cgattctctc cctctttgtt tgataatagc agcagagctg gaggccttct tgtggacttt     540
agtgcatatc caaggcatac tgggagagat ggatggcctg cagcaaggtc ttctgagctt     600
acccccggga atgatactgc tgccacagga aggtctatat ctcatatgtg gcagataagc     660
tcccagaatc ctccatccaa cctttgcttg caaggctcaa ctggcgggac tggccttttc     720
agttcaggaa ttcctccggg agaatgcttc acaggagttg ctgtttcaga ctcgagctgt     780
gctctctctc ttctgtcaaa tcaaccatgg ggctccacaa accgagcatc aagtcttgcg     840
gtgaatgact tgtttagtgc cgaagaggca cccgtggttc aatcaacagc tcaccatggt     900
gcggctgtca atcagtatcc aatcccttgg agcttcaaga gcaatgaagg aagtaacagt     960
tcacatgaga tgtgccctga tctaggtctg ggtcaaattt caatgcctct caacagtcaa    1020
cttgctggtc agctcgagca gtctcaacag aataggaggc aatacatgga cctcgagcat    1080
tccagggctt atgactcttc aacccagcac atccactggt cactttaa                 1128

<210>  287
<211>  375
<212>  PRT
<213>  Populus tremuloides

<400>  287
Met Glu Met Asp Ser Gly Ser Leu Thr Glu Ser Ala Thr Ser Asn Ala
1               5                   10                  15
Thr Ser Pro Pro Ala Glu Ser Val Asn Gly Leu Lys Phe Gly Lys Lys

20                          25                          30
Ile Tyr Phe Glu Asp His Val Gly Val Gly Ala Pro Ala Lys Ser Gly
        35                          40                          45
Thr Gly Ser Ser Ser Ser Gly Ser Gly Ser Gly Ser Ser Arg Lys Ala
    50                          55                          60
Gln Gly Gly Gln His Gln Gln Pro Pro Arg Cys Gln Val Glu Gly Cys
65                          70                          75                          80
Lys Val Asp Leu Ser Asp Ala Lys Thr Tyr Tyr Ser Arg His Lys Val
                        85                          90                          95
Cys Ser Met His Ser Lys Ser Pro Arg Val Ile Val Ala Gly Leu Val
                100                         105                         110
Gln Arg Phe Cys Gln Gln Cys Ser Arg Phe His Leu Leu Pro Glu Phe
        115                         120                         125
Asp Gln Gly Lys Arg Ser Cys Arg Arg Arg Leu Ala Gly His Asn Glu
    130                         135                         140
Arg Arg Arg Lys Pro Pro Ser Gly Ser Val Leu Ser Ala Arg His Gly
145                         150                         155                         160
Arg Phe Ser Pro Ser Leu Phe Asp Asn Ser Ser Arg Ala Gly Gly Leu
                        165                         170                         175
Leu Val Asp Phe Ser Ala Tyr Pro Arg His Thr Gly Arg Asp Gly Trp
                180                         185                         190
Pro Ala Ala Arg Ser Ser Glu Leu Thr Pro Gly Asn Asp Thr Ala Ala
        195                         200                         205
Thr Gly Arg Ser Ile Ser His Met Trp Gln Ile Ser Ser Gln Asn Pro
    210                         215                         220
Pro Ser Asn Leu Cys Leu Gln Gly Ser Thr Gly Gly Thr Gly Leu Phe
225                         230                         235                         240
Ser Ser Gly Ile Pro Pro Gly Glu Cys Phe Thr Gly Val Ala Val Ser
                        245                         250                         255
Asp Ser Ser Cys Ala Leu Ser Leu Leu Ser Asn Gln Pro Trp Gly Ser
                260                         265                         270
Thr Asn Arg Ala Ser Ser Leu Ala Val Asn Asp Leu Phe Ser Ala Glu
        275                         280                         285
Glu Ala Pro Val Val Gln Ser Thr Ala His His Gly Ala Ala Val Asn
    290                         295                         300
Gln Tyr Pro Ile Pro Trp Ser Phe Lys Ser Asn Glu Gly Ser Asn Ser
305                         310                         315                         320
Ser His Glu Met Cys Pro Asp Leu Gly Leu Gly Gln Ile Ser Met Pro
                        325                         330                         335
Leu Asn Ser Gln Leu Ala Gly Gln Leu Glu Gln Ser Gln Gln Asn Arg
                340                         345                         350
Arg Gln Tyr Met Asp Leu Glu His Ser Arg Ala Tyr Asp Ser Ser Thr
        355                         360                         365
Gln His Ile His Trp Ser Leu
    370                         375

<210>  288
<211>  1059
<212>  DNA
<213>  Citrus clementina

<400>  288
atggaactgg gttcagacta tttggctgaa tcaggtggtg gctccggctc cggctcaggc      60
tccggcttat catccgctga gccatcactt aatggtttga agtttggcaa aaaaatctat     120
tttgaggatg tcggtactgc cggagctcca tttccaggat ctgggtcatc atctgggtcc     180
gggtcagggt caggttcagg gtcagggagg aaggtgaggg gtgttggcgg tggtatggtt     240
actagtgggc agcagccacc aaggtgccaa gtggaggggt gtaaagttga tctgagtgat     300
gccaaagctt actattcaag gcacaaagtt tgtggcatgc attcaaagtc tcctgttgtc     360
actgttgccg gccttgagca gaggttttgc cagcaatgta gcagatttca tcagcttccg     420

gagtttgacc aaggaaaacg aagttgccgc aggcgcctgg caggccataa tgagcgccgg 480
aggaagccaa cttctggacc atttttgggc actcgttatg gcaggctctc ttcctctgtc 540
attgagaaca gcagccaagg tggaggattt ctgatcgact tcagtgcata tcagatggtt 600
ggtgggaggg atggatggcc agtgacaagt gtctccaagc aggtatctgg aaatcaaacc 660
actgtcacag caaggcatct tcctcagcca ctatggcaaa accactctca ggatcctcca 720
cctgatcgtt accttcagtg ttcaacagct gggactggtt tctctggtcc tggaattcct 780
tgtggaggat gcttcacagg agttgctgac tcaaactgtg ctctctctct tctgtcaaat 840
caaccatggg gctctaagaa cccgacaccg ggtcatggag tgggtgactt aatgcatgcc 900
cataccaaat ccgtcactca accagtatcg ccccatggag cagctattaa tcaatatcca 960
aacatgtcat ggggggttca agggcaatgc aacctggtag cagttccaca ccaaatggcc 1020
ccccaaatgg ggtttgggtt caacattccg gcccattaa 1059

<210> 289
<211> 352
<212> PRT
<213> Citrus clementina

<400> 289
Met Glu Leu Gly Ser Asp Tyr Leu Ala Glu Ser Gly Gly Gly Ser Gly
1               5                   10                  15
Ser Gly Ser Gly Ser Gly Leu Ser Ser Ala Glu Pro Ser Leu Asn Gly
            20                  25                  30
Leu Lys Phe Gly Lys Lys Ile Tyr Phe Glu Asp Val Gly Thr Ala Gly
        35                  40                  45
Ala Pro Phe Pro Gly Ser Gly Ser Ser Ser Gly Ser Gly Ser Gly Ser
    50                  55                  60
Gly Ser Gly Ser Gly Arg Lys Val Arg Gly Val Gly Gly Gly Met Val
65                  70                  75                  80
Thr Ser Gly Gln Gln Pro Pro Arg Cys Gln Val Glu Gly Cys Lys Val
                85                  90                  95
Asp Leu Ser Asp Ala Lys Ala Tyr Tyr Ser Arg His Lys Val Cys Gly
            100                 105                 110
Met His Ser Lys Ser Pro Val Val Thr Val Ala Gly Leu Glu Gln Arg
        115                 120                 125
Phe Cys Gln Gln Cys Ser Arg Phe His Gln Leu Pro Glu Phe Asp Gln
    130                 135                 140
Gly Lys Arg Ser Cys Arg Arg Arg Leu Ala Gly His Asn Glu Arg Arg
145                 150                 155                 160
Arg Lys Pro Thr Ser Gly Pro Phe Leu Gly Thr Arg Tyr Gly Arg Leu
                165                 170                 175
Ser Ser Ser Val Ile Glu Asn Ser Ser Gln Gly Gly Gly Phe Leu Ile
            180                 185                 190
Asp Phe Ser Ala Tyr Gln Met Val Gly Gly Arg Asp Gly Trp Pro Val
        195                 200                 205
Thr Ser Val Ser Lys Gln Val Ser Gly Asn Gln Thr Thr Val Thr Ala
    210                 215                 220
Arg His Leu Pro Gln Pro Leu Trp Gln Asn His Ser Gln Asp Pro Pro
225                 230                 235                 240
Pro Asp Arg Tyr Leu Gln Cys Ser Thr Ala Gly Thr Gly Phe Ser Gly
                245                 250                 255
Pro Gly Ile Pro Cys Gly Gly Cys Phe Thr Gly Val Ala Asp Ser Asn
            260                 265                 270
Cys Ala Leu Ser Leu Leu Ser Asn Gln Pro Trp Gly Ser Lys Asn Pro
        275                 280                 285
Thr Pro Gly His Gly Val Gly Asp Leu Met His Ala His Thr Lys Ser
    290                 295                 300
Val Thr Gln Pro Val Ser Pro His Gly Ala Ala Ile Asn Gln Tyr Pro
305                 310                 315                 320
Asn Met Ser Trp Gly Val Gln Gly Gln Cys Asn Leu Val Ala Val Pro

325                 330                 335
His Gln Met Ala Pro Gln Met Gly Phe Gly Phe Asn Ile Pro Ala His
            340                 345                 350

<210>  290
<211>  565
<212>  DNA
<213>  Beta vulgaris

<400>  290
atggatacgg gttcaaatta tccgaccgtt aaggggtcat catcaacctc ttcatcatct      60
gggttgtcgg attctttaaa tgggctgaaa tttgggcaaa aaatatactt tgaagatgtg     120
ggtggtgttg gaacttccgg caagtcttcc gtcgccggaa gtggtggtgc tccggcgaaa     180
agggccggaa aaggggtggt gcaaagtggg caaccaccaa ggtgtcaagt agaagggtgt     240
aagatagatc ttagtgatgc taaaacttat tattctaggc ataaagtttg tggtatgcac     300
tctaaatctt ctgttgttat tgttgctggt cttgagcaac gtttttgcca gcagtgcagc     360
agatttcatc ggcttcctga gtttgaccaa gggaaacgaa gttgtcgcag acgccttgct     420
ggtcataatg agcgtcgaag aaaaccacca cctggtcttt tgttatcatc acgtttggga     480
cgtctctctt catccctttt tggtgataac accggcggaa gtggtggatt cttattggac     540
ttctcttcgt atccacggca ttctg                                           565

<210>  291
<211>  188
<212>  PRT
<213>  Beta vulgaris

<400>  291
Met Asp Thr Gly Ser Asn Tyr Pro Thr Val Lys Gly Ser Ser Ser Thr
1               5                   10                  15
Ser Ser Ser Ser Gly Leu Ser Asp Ser Leu Asn Gly Leu Lys Phe Gly
            20                  25                  30
Gln Lys Ile Tyr Phe Glu Asp Val Gly Gly Val Gly Thr Ser Gly Lys
        35                  40                  45
Ser Ser Val Ala Gly Ser Gly Gly Ala Pro Ala Lys Arg Ala Gly Lys
    50                  55                  60
Gly Val Val Gln Ser Gly Gln Pro Pro Arg Cys Gln Val Glu Gly Cys
65                  70                  75                  80
Lys Ile Asp Leu Ser Asp Ala Lys Thr Tyr Tyr Ser Arg His Lys Val
                85                  90                  95
Cys Gly Met His Ser Lys Ser Ser Val Val Ile Val Ala Gly Leu Glu
            100                 105                 110
Gln Arg Phe Cys Gln Gln Cys Ser Arg Phe His Arg Leu Pro Glu Phe
        115                 120                 125
Asp Gln Gly Lys Arg Ser Cys Arg Arg Arg Leu Ala Gly His Asn Glu
    130                 135                 140
Arg Arg Arg Lys Pro Pro Pro Gly Ser Leu Leu Ser Ser Arg Leu Gly
145                 150                 155                 160
Arg Leu Ser Ser Ser Leu Phe Gly Asp Asn Thr Gly Gly Ser Gly Gly
                165                 170                 175
Phe Leu Leu Asp Phe Ser Ser Tyr Pro Arg His Ser
            180                 185

<210>  292
<211>  536
<212>  DNA
<213>  Hevea brasiliensis

<400>  292
atggaaatgg gttcgggctc tttgacagag tcaggtacct ccaacgccac ttctccacct      60

gctgagtcaa taaatgggtt gaaatttggc caaaaaatct attttgagaa tgcgggggct 120
aagactccgg ccaaatctgc acccgggtct tcatcttccg ggtccggggc cccgtccagg 180
aaggttcacg gtgggcagca gcagcagcca cccaggtgtc aagttgaggg atgtaaagtg 240
gatctgagtg atgctaaggc ttattattcg aggcacaaag tttgtggtat gcactctaag 300
tctcctaagg tcattgttgc tggtttggag caaagatttt gccagcagtg tagtagattt 360
catcagcttc ctgaatttga ccaaggaaaa cgaagttgcc gcagacgcct agctggtcat 420
aatgaacggc ggaggaagcc accaactgga tcagtgctgt catctcgcta taacagactt 480
ttttcaacaa tttttgataa cagcagccga gctgggggca ttcttgtgga tttcag 536

<210> 293
<211> 177
<212> PRT
<213> Hevea brasiliensis

<400> 293
Met Glu Met Gly Ser Gly Ser Leu Thr Glu Ser Gly Thr Ser Asn Ala
1               5                   10                  15
Thr Ser Pro Pro Ala Glu Ser Ile Asn Gly Leu Lys Phe Gly Gln Lys
            20                  25                  30
Ile Tyr Phe Glu Asn Ala Gly Ala Lys Thr Pro Ala Lys Ser Ala Pro
        35                  40                  45
Gly Ser Ser Ser Ser Gly Ser Gly Ala Pro Ser Arg Lys Val His Gly
    50                  55                  60
Gly Gln Gln Gln Gln Pro Pro Arg Cys Gln Val Glu Gly Cys Lys Val
65                  70                  75                  80
Asp Leu Ser Asp Ala Lys Ala Tyr Tyr Ser Arg His Lys Val Cys Gly
                85                  90                  95
Met His Ser Lys Ser Pro Lys Val Ile Val Ala Gly Leu Glu Gln Arg
            100                 105                 110
Phe Cys Gln Gln Cys Ser Arg Phe His Gln Leu Pro Glu Phe Asp Gln
        115                 120                 125
Gly Lys Arg Ser Cys Arg Arg Arg Leu Ala Gly His Asn Glu Arg Arg
    130                 135                 140
Arg Lys Pro Pro Thr Gly Ser Val Leu Ser Ser Arg Tyr Asn Arg Leu
145                 150                 155                 160
Phe Ser Thr Ile Phe Asp Asn Ser Ser Arg Ala Gly Gly Ile Leu Val
                165                 170                 175
Asp

<210> 294
<211> 1130
<212> DNA
<213> Oryza sativa

<400> 294
catgcggcta atgtagatgc tcactgcgct agtagtaagg tactccagta cattatggaa 60
tatacaaagc tgtaatactc gtatcagcaa gagagaggca cacaagttgt agcagtagca 120
caggattaga aaaacgggac gacaaatagt aatggaaaaa caaaaaaaaa caaggaaaca 180
catggcaata taaatggaga aatcacaaga ggaacagaat ccgggcaata cgctgcgaaa 240
gtactcgtac gtaaaaaaaa gaggcgcatt catgtgtgga cagcgtgcag cagaagcagg 300
gatttgaaac cactcaaatc caccactgca aaccttcaaa cgaggccatg gtttgaagca 360
tagaaagcac aggtaagaag cacaacgccc tcgctctcca ccctcccacc caatcgcgac 420
gcacctcgcg gatcggtgac gtggcctcgc cccccaaaaa tatcccgcgg cgtgaagctg 480
acaccccggg cccacccacc tgtcacgttg gcacatgttg gttatggttc ccggccgcac 540
caaaatatca acgcggcgcg gcccaaaatt tccaaaatcc cgcccaagcc cctggcgcgt 600
gccgctcttc cacccaggtc cctctcgtaa tccataatgg cgtgtgtacc ctcggctggt 660
tgtacgtggg cgggttaccc tgggggtgtg ggtggatgac gggtgggccc ggaggaggtc 720
cggccccgcg cgtcatcgcg gggcggggtg tagcgggtgc gaaaaggagg cgatcggtac 780

gaaaattcaa attaggaggt ggggggcggg gcccttggag aataagcgga atcgcagata 840
tgcccctgac ttggcttggc tcctcttctt cttatccctt gtcctcgcaa ccccgcttcc 900
ttctctcctc tcctcttctc ttctcttctc tggtggtgtg ggtgtgtccc tgtctcccct 960
ctccttcctc ctctcctttc ccctcctctc ttcccccctc tcacaagaga gagagcgcca 1020
gactctcccc aggtgaggtg agaccagtct ttttgctcga ttcgacgcgc ctttcacgcc 1080
gcctcgcgcg gatctgaccg cttccctcgg ccttctcgca ggattcagcc 1130

<210> 295
<211> 2194
<212> DNA
<213> Oryza sativa

<400> 295
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 296
<211> 1149
<212> DNA
<213> Zea mays

<220>
<221> misc_feature
<222> (157)..(157)

<223>  n is a, c, g, or t

<220>
<221>  misc_feature
<222>  (894)..(894)
<223>  n is a, c, g, or t

<220>
<221>  misc_feature
<222>  (954)..(954)
<223>  n is a, c, g, or t

<400>  296
atgcacaact gcaaagaaac cagcaagaat gcgccggcgg tttatagttc cccgctactg      60
caccgctctc tgctgcaggc tgcagctttg actaccaact catacagtac tagtactata     120
ctagagtact actacgccat tagcacggtg gttgcantag gccgtggtct ggaggtctct     180
ctcagctcag acgaggatcg tcggagcaag cagcagcgcg tgcagcctct gggagaccct     240
ggcggcgtcg acgccgatcc tggggctgag cgcctgcgag tggacggtgt agaatatctt     300
gtccccgatg acggagaagc tggcgctgac cacctcggcg ccctcctgct ccagcacggt     360
gatgacctcg tgcagcttga agggcctggc ggcctcgctg atgagcacca cgtccagcgt     420
cccgtcctgg caccgcacct cgatgaccgg catgcgcacg cctccgccgc cgccggtgga     480
cccggccgcc gccgttgcag cctccgtggc cgcgccgccg ccgttgcagc agcccgcctt     540
ccgctgcttc agcgcctcga tccgctcctt gagctgcttg atgtacgccg ccgcgctgtc     600
cagctggtcc agctgcgtca ccgcgtcctg cttgttgccg ggattggagg acaccgccgc     660
cgacgcggcg tcggagagga gggaggcgtg cgtggcggcg gcggcggcgg cgggagggat     720
gagggaggag agcttgaggc agaggccctt catgtgcagc ctccggttct tctccacgtc     780
cttcctctcc agcttgcacc ccccgccgct gctgtgcgcg ttcctctccc ccgcggcgca     840
ggcgcccgag ctgcccccgc tgctctgcct ccggctcttc atctcgatcg accngaccgg     900
tctgcagatc tctccttggc tgttctcgtc gctgctcctg ccgggcgtga agcngcgcag     960
cctttggttg ggacttggga gggacaagtt gcaacagcac ccacggccca cggcacggag    1020
ggggcgaaag gcaaggaggc caggacaaga cgagagaaat acaggccggg ggagatggct    1080
ccgtggcgcg tacgtgtgtc tacctgcatg ttggttgatc cgattgcatc tgctgtaacc    1140
atatattaa                                                            1149

<210>  297
<211>  382
<212>  PRT
<213>  Zea mays

<220>
<221>  UNSURE
<222>  (53)..(53)
<223>  Xaa can be any naturally occurring amino acid

<400>  297
Met His Asn Cys Lys Glu Thr Ser Lys Asn Ala Pro Ala Val Tyr Ser
1               5                   10                  15
Ser Pro Leu Leu His Arg Ser Leu Leu Gln Ala Ala Ala Leu Thr Thr
            20                  25                  30
Asn Ser Tyr Ser Thr Ser Thr Ile Leu Glu Tyr Tyr Tyr Ala Ile Ser
        35                  40                  45
Thr Val Val Ala Xaa Gly Arg Gly Leu Glu Val Ser Leu Ser Ser Asp
    50                  55                  60
Glu Asp Arg Arg Ser Lys Gln Gln Arg Val Gln Pro Leu Gly Asp Pro
65                  70                  75                  80
Gly Gly Val Asp Ala Asp Pro Gly Ala Glu Arg Leu Arg Val Asp Gly
                85                  90                  95
Val Glu Tyr Leu Val Pro Asp Asp Gly Glu Ala Gly Ala Asp His Leu
            100                 105                 110

Gly Ala Leu Leu Leu Gln His Gly Asp Asp Leu Val Gln Leu Glu Gly
        115                 120                 125
Pro Gly Gly Leu Ala Asp Glu His His Val Gln Arg Pro Val Leu Ala
    130                 135                 140
Pro His Leu Asp Asp Arg His Ala His Ala Ser Ala Ala Ala Gly Gly
145                 150                 155                 160
Pro Gly Arg Arg Arg Cys Ser Leu Arg Gly Arg Ala Ala Ala Val Ala
                165                 170                 175
Ala Ala Arg Leu Pro Leu Leu Gln Arg Leu Asp Pro Leu Leu Glu Leu
            180                 185                 190
Leu Asp Val Arg Arg Arg Ala Val Gln Leu Val Gln Leu Arg His Arg
        195                 200                 205
Val Leu Leu Val Ala Gly Ile Gly Gly His Arg Arg Arg Arg Gly Val
    210                 215                 220
Gly Glu Glu Gly Gly Val Arg Gly Gly Gly Gly Gly Gly Gly Arg Asp
225                 230                 235                 240
Glu Gly Gly Glu Leu Glu Ala Glu Ala Leu His Val Gln Pro Pro Val
                245                 250                 255
Leu Leu His Val Leu Pro Leu Gln Leu Ala Pro Pro Ala Ala Ala Val
            260                 265                 270
Arg Val Pro Leu Pro Arg Gly Ala Gly Ala Arg Ala Ala Pro Ala Ala
        275                 280                 285
Leu Pro Pro Ala Leu His Leu Asp Arg Pro Asp Arg Ser Ala Asp Leu
    290                 295                 300
Ser Leu Ala Val Leu Val Ala Ala Pro Ala Gly Arg Glu Ala Ala Gln
305                 310                 315                 320
Pro Leu Val Gly Thr Trp Glu Gly Gln Val Ala Thr Ala Pro Thr Gly
                325                 330                 335
His Gly Thr Glu Gly Ala Lys Gly Lys Glu Ala Arg Thr Arg Arg Glu
            340                 345                 350
Lys Tyr Arg Pro Gly Glu Met Ala Pro Trp Arg Val Arg Val Ser Thr
        355                 360                 365
Cys Met Leu Val Asp Pro Ile Ala Ser Ala Val Thr Ile Tyr
    370                 375                 380

<210>  298
<211>  660
<212>  DNA
<213>  Zea mays

<400>  298
atggagatga gagcgaagaa gagcagcaga agcagcacga gcaacaccag cggcagcacg      60
acgaccacgg cggtggagag gaaggagatc gagaggagga ggaggcagca gatgaagagc     120
ctctgcgcca agctcgcctc cctcatcccg aaagaacact actcctccaa ggatgctatg     180
acccagctgg gtagcctaga cgaggcagcc acgtacataa agagactcaa ggagagggtg     240
gaggagctgc ggcacaagag cgcctctgca cggctcttgg ccgctggcag tggcacgaga     300
cgaggcggag gaggaggagg cgcctccaca tcgtcggcag cgacgaccac ggcgagcggt     360
ggcgcaggat catctgaaga agccggccgg cgggaggacg acatgccgcc ggcggtggta     420
gaggttcggc agcacaatga cgggtcaagc ctggacgtgg tgctcatcag cagcgcggcg     480
cgacccttca agctgcacga ggtggtcacc gtgctggagg aagaaggcgc cgagaccgac     540
aacgccaacc tctccgtcgc cggcaccaaa atcttctaca ccatccactg caaggccttt     600
tgcccaagaa tcggtataga tgtttcaaga gtttctgaaa gattaagagc attgggatga     660

<210>  299
<211>  219
<212>  PRT
<213>  Zea mays

<400>  299

Met Glu Met Arg Ala Lys Lys Ser Ser Arg Ser Ser Thr Ser Asn Thr
1               5                   10                  15
Ser Gly Ser Thr Thr Thr Thr Ala Val Glu Arg Lys Glu Ile Glu Arg
            20                  25                  30
Arg Arg Arg Gln Gln Met Lys Ser Leu Cys Ala Lys Leu Ala Ser Leu
        35                  40                  45
Ile Pro Lys Glu His Tyr Ser Ser Lys Asp Ala Met Thr Gln Leu Gly
    50                  55                  60
Ser Leu Asp Glu Ala Ala Thr Tyr Ile Lys Arg Leu Lys Glu Arg Val
65                  70                  75                  80
Glu Glu Leu Arg His Lys Ser Ala Ser Ala Arg Leu Leu Ala Ala Gly
                85                  90                  95
Ser Gly Thr Arg Arg Gly Gly Gly Gly Gly Gly Ala Ser Thr Ser Ser
            100                 105                 110
Ala Ala Thr Thr Thr Ala Ser Gly Gly Ala Gly Ser Ser Glu Glu Ala
        115                 120                 125
Gly Arg Arg Glu Asp Asp Met Pro Pro Ala Val Val Glu Val Arg Gln
    130                 135                 140
His Asn Asp Gly Ser Ser Leu Asp Val Val Leu Ile Ser Ser Ala Ala
145                 150                 155                 160
Arg Pro Phe Lys Leu His Glu Val Val Thr Val Leu Glu Glu Glu Gly
                165                 170                 175
Ala Glu Thr Asp Asn Ala Asn Leu Ser Val Ala Gly Thr Lys Ile Phe
            180                 185                 190
Tyr Thr Ile His Cys Lys Ala Phe Cys Pro Arg Ile Gly Ile Asp Val
        195                 200                 205
Ser Arg Val Ser Glu Arg Leu Arg Ala Leu Gly
    210                 215

<210>  300
<211>  669
<212>  DNA
<213>  Glycine max

<400>  300
atgggtcaac aacaacaggg aagtcaacct tctccaacca aagtcgaaag aaagattgta      60
gagaaaaaca ggagaagcca gatgaagaac ctctattccg aactcaactc tcttctccct     120
acccgtaatc ccaaggaagc gatgtcactg cctgatcaaa tagatgaagc aatcaactat     180
atcaaaagcc tagagacaaa agtgaagctg gagcaggaga agaaagaaag gttaaaggaa     240
aggaagagaa ctcgtggtgg ctgttcgagt tcttctgaag cacaaggaag cctgaaatcg     300
ccaaatatcc agattcacga aacgggaaat ttgcttgaag tcattctaac atgtggggtc     360
gatagccagt tcatgttctg tgaaattatt cgaattttgc atgaagagaa cgtcgaggtc     420
atcaatgcca attcttcaat ggtcggagat ttagtgattc atgttgtgca cggggaggtt     480
gagccatcta tctatcaatt cggagcgacc aaagtgagtg agaagctgaa atggtttatg     540
aacggatcct tcagtgatgt ggaaatggag cctgaattaa tgtggaattt taaaattgat     600
gctactgagc cgtgggggct tctagatgat cttacactgg acaatgtctt accaccaaat     660
actttgtaa                                                             669

<210>  301
<211>  222
<212>  PRT
<213>  Glycine max

<400>  301
Met Gly Gln Gln Gln Gln Gly Ser Gln Pro Ser Pro Thr Lys Val Glu
1               5                   10                  15
Arg Lys Ile Val Glu Lys Asn Arg Arg Ser Gln Met Lys Asn Leu Tyr
            20                  25                  30
Ser Glu Leu Asn Ser Leu Leu Pro Thr Arg Asn Pro Lys Glu Ala Met

            35                  40                  45
Ser Leu Pro Asp Gln Ile Asp Glu Ala Ile Asn Tyr Ile Lys Ser Leu
    50                  55                  60
Glu Thr Lys Val Lys Leu Glu Gln Glu Lys Lys Glu Arg Leu Lys Glu
65                  70                  75                  80
Arg Lys Arg Thr Arg Gly Gly Cys Ser Ser Ser Ser Glu Ala Gln Gly
                85                  90                  95
Ser Leu Lys Ser Pro Asn Ile Gln Ile His Glu Thr Gly Asn Leu Leu
            100                 105                 110
Glu Val Ile Leu Thr Cys Gly Val Asp Ser Gln Phe Met Phe Cys Glu
        115                 120                 125
Ile Ile Arg Ile Leu His Glu Glu Asn Val Glu Val Ile Asn Ala Asn
    130                 135                 140
Ser Ser Met Val Gly Asp Leu Val Ile His Val Val His Gly Glu Val
145                 150                 155                 160
Glu Pro Ser Ile Tyr Gln Phe Gly Ala Thr Lys Val Ser Glu Lys Leu
                165                 170                 175
Lys Trp Phe Met Asn Gly Ser Phe Ser Asp Val Glu Met Glu Pro Glu
            180                 185                 190
Leu Met Trp Asn Phe Lys Ile Asp Ala Thr Glu Pro Trp Gly Leu Leu
        195                 200                 205
Asp Asp Leu Thr Leu Asp Asn Val Leu Pro Pro Asn Thr Leu
    210                 215                 220

## Description

The present invention relates generally to the field of molecular biology and concerns a method for improving various plant growth characteristics by modulating expression in a plant of a nucleic acid encoding a GRP (Growth-Related Protein). The present invention also concerns plants having modulated expression of a nucleic acid encoding a GRP, which plants have improved growth characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigor has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various growth characteristics may be improved in plants by modulating expression in a plant of a nucleic acid encoding a GRP (Growth-Related Protein) in a plant. The GRP may be one of the following: a MADS-box transcription factor (OsMADS15), a PLT transcription factor, a bHLH transcription factor, an SPL15 transcription factor, and a halotolerance protein (HAL3).

### BACKGROUND

### Halotolerance proteins

Many enzymatic processes in a cell require the involvement of Coenzyme A (CoA or CoASH). Coenzyme A (CoASH) itself is a highly polar molecule, consisting of adenosine 3',5'-diphosphate linked to 4-phosphopantethenic acid (Vitamin B5) and thence to β-mercaptoethylamine. The free SH group may be esterified, depending on the process in which CoA is involved. The pathway of CoA synthesis has been elucidated in bacteria, animals and plants. In plants, the conversion of the CoA precursor 4'-phosphopantothenoyl-cysteine (PPC) into 4'-phosphopantetheine is catalysed by the flavoprotein 4'-phosphopantothenoyl-cysteine decarboxylase (PPCDC or HAL3, Kupke et al. J. Biol. Chem. 276, 19190-19196, 2001). The gene encoding HAL3 proteins may be part of a small gene family: the *Arabidopsis* genome comprises two isoforms (AtHAL3a and AtHAL3b; Espinoza-Ruiz et al. Plant J. 20, 529-539, 1999), in tobacco 3 HAL3 genes are present (Yonamine et al. J. Exp. Bot. 55, 387-395, 2004), though in rice HAL3 is a single copy gene. It is suggested that AtHAL3 and other HAL3 proteins function as a trimer, with each monomer having a flavin mononucleotide (FMN) bound (Albert et al. Structure 8, 961-969, 2000). The FMN cofactor is postulated to play a role in the redox reaction generating 4'-phosphopantetheine.

The molecular function of HAL3 proteins is not fully elucidated yet. The HAL3 proteins show homology to the yeast SIS2 protein, which is involved in halotolerance (Ferrando et al., Mol. Cell. Biol. 15, 5470-5481). Plants or plant cells ectopically expressing *HAL3* show improved salt, osmotic or lithium stress tolerance (Espinoza-Ruiz et al., 1999; Yonamine et al., 2004). Tobacco HAL3a overexpression in BY2 cells reportedly caused an increase in intracellular proline content, which may contribute to the salt tolerant phenotype (Yonamine et al., 2004). Furthermore, *Arabidopsis* plants overexpressing *AtHAL3a* displayed a faster growth rate than the wild type plants (Espinoza-Ruiz et al., 1999). The AtHAL3b protein was shown to interact with the cell cycle protein CDKB1;1 (WO 00/36124), therefore it was postulated that AtHAL3b is useful for conferring salt stress tolerance to plants and for increasing the growth rate of plants under salt stress conditions Surprisingly, it has now been found that preferentially increasing expression of a nucleic acid encoding a HAL3 polypeptide in expanding tissues gives plants having increased yield relative to control plants, which yield increase is at least 10% compared to the control plants.

### Transcription factors

Transcription factors are usually defined as proteins that bind to a cis-regulatory element (eg. an enhancer, a TATA box) and that, in association with RNA polymerase, are capable of activating and/or repressing transcription. The *Arabidopsis* genome codes for at least 1533 transcriptional regulators, which account for ~5.9% of its estimated total number of genes (Riechmann et al., 2000 (Science Vol. 290, 2105-2109)).

### MADS15

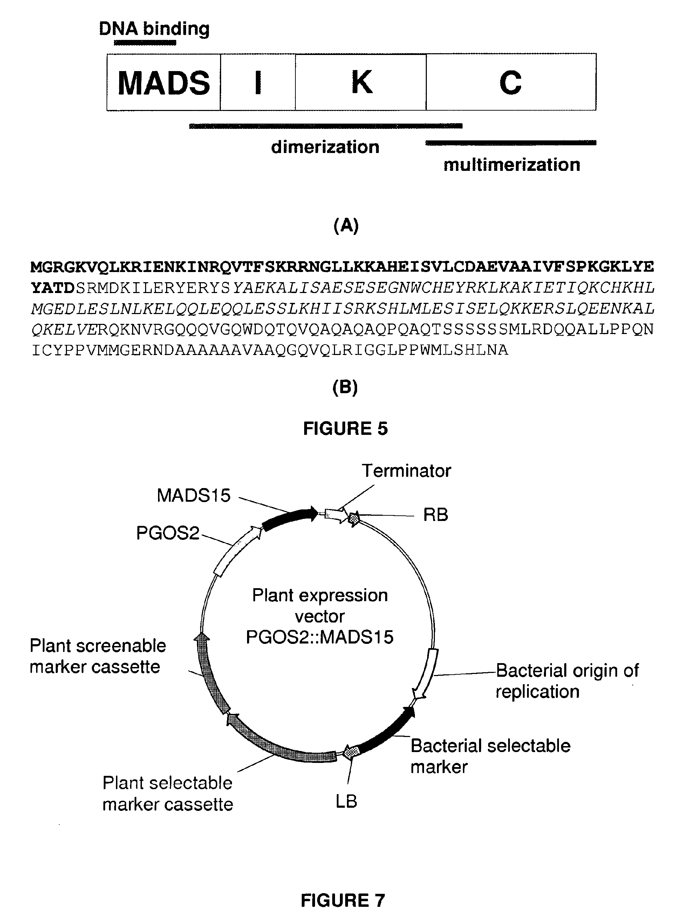

The MADS-box genes constitute a large gene family of eukaryotic transcriptional regulators involved in diverse aspects of yeast, plant and animal development. MADS-box genes encode a strongly conserved MADS domain responsible for DNA binding to specific boxes in the regulatory region of their target genes. The gene family can be divided into two main lineages, type I and type II. Type II genes are also named MIKC-type proteins, referring to the four functional domains they possess (Figure 5, (Jack, Plant Mol. Biol. 46, 515-520, 2001)):
- MADS for DNA binding, about 60 amino acids (highly conserved) located at the N-terminal end of the protein;
- I for intervening domain (less conserved), involved in the selective formation of MADS dimer;
- K for keratin domain (well conserved) responsible for dimerisation;
- C for C-terminal region (variable in sequence and length) involved in transcriptional activation, or in the formation of a multimeric transcription factor complex.

Over 100 MADS-box genes have been identified in *Arabidopsis,* and have been phylogenetically classified into 12 clades, each clade having specific deviations from the MADS consensus (Thiessen et al. J Mol. Evol. 43, 484-516, 1996). *OsMADS15* belongs to the SQUA clade (for SQUAMOSA, from *Antirrhinum majus*). Genes of the SQUA clade are classified as A function organ identity genes with reference to the ABC floral organ identity specification model, proposed by Coen and Meyerowitz in 1991 (Nature 353, 31-7). Besides *OsMADS15*, rice *OsMADS14*, *OsMADS18*, and *OsMADS20* are also part of the SQUA clade.

The SQUA clade in dicotyledonous plants (dicots) is subdivided into two subgroups, the AP1 and the FUL subclades (in which OsMADS15 clusters). These subclades diverge essentially with respect to the specific amino acid motifs located at the C-terminus of their respective proteins (Litt and Irish, Genetics 165, 821-833, 2003). In addition to the presence of a specific AP1 amino acid motif, the dicot AP1 clade related proteins usually comprise a farnesylation motif at their C-terminus (this motif is CAAX, where C is cysteine, A is usually an aliphatic amino acid, and X is methionine, glutamine, serine, cysteine or alanine). In monocotyledonous plants (monocots), the SQUA clade proteins are also subdivided into two main groups, which may be distinguished based on conserved C-terminal motifs located within the last 15 amino acids of the proteins: LPPWMLS (for example OsMADS15, SEQ ID NO: 117) and LPPWMLR (for example OsMADS18). In contrast to dicot sequences of the SQUA clade, monocot sequences of the SQUA clade do not possess a farnesylation motif at their C-terminus. OsMADS15 was postulated to function in a complex with other proteins to control organ formation. However, so far no mutants with a visible phenotype have been identified; no experimental data have been presented relating to ectopic expression or downregulated expression of *OsMADS15,* except for the data presented in WO 01/14559 (EP1209232, US6,995,302). In this disclosure, transgenic *Fagopyrum esculentum* plants expressing *OsMADS15* in sense direction showed increased branching, whereas transgenics expressing the antisense construct had decreased growth and suppressed branching. *Kalanchoë daigremontiana* transformed with the sense construct had leaf development around the roots, which was taken as an indication of increased branching. The authors stated that no changes were observed in terms of flower development and structure of these flowers. The orthologue of OsMADS15 in *Arabidopsis* is APETALA1 (AP1). Ectopic expression of *AP1* results in a decrease of flowering time, and *AP1* mutants exhibit delayed flowering and have abnormal flowers.

### PLT

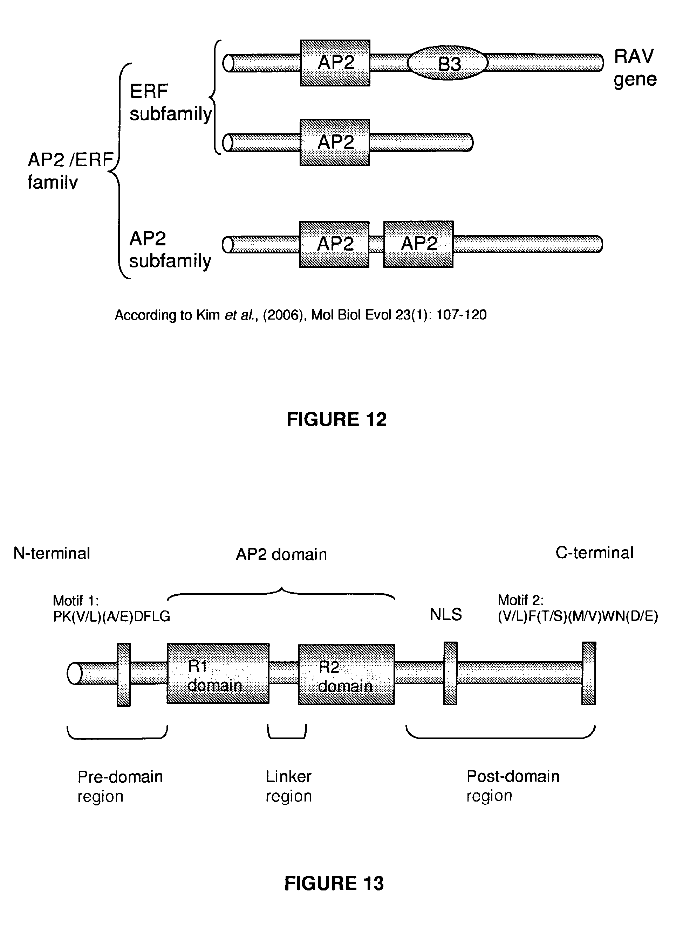

The AP2(apetala2)/ERF (ethylene-responsive responsive element-binding factor) family comprises transcription factors with at least one highly conserved DNA binding domain, the AP2 domain. The AP2 domain was originally described in APETALA2 (AP2), an *Arabidopsis* protein involved in developmental programs such as meristem identity regulation and floral organ specification (Jofuku et al., (1994) Plant Cell 6, 1211-1225). AP2/ERFproteins are divided into subfamilies based on whether they contain one (ERF subfamily) or two (AP2 subfamily) DNA binding domains (Figure 12). More than 140 AP2/ERF genes have been identified in the *Arabidopsis thaliana* genome (Reichmann et al. (2000) Science 290: 2105-2110), amongst which up to 18 belong to the AP2 subfamily (Kim et al. (2006) Mol Bio Evol 23(1): 107-120).

Two *Arabidopsis* AP2 subfamily transcription factor polypeptides, encoded by the Plethora 1 (PLT1) and Plethora 2 (PLT2) genes, collectively called PLT genes, have been shown to be required for stem cell specification and maintenance specifically in the root meristem. In RT-PCR analysis, PLT transcripts were mainly detected in roots, indicating that PLT expression is strongly associated with root identity. Ectopic PLT expression in the *Arabidopsis* embryo induces homeotic transformation of apical domains into root stem cells, roots, or hypocotyls (Aida et al (2004) Cell 119:109-120).

US patent application 2004/0045049 and international patent application WO03/013227 provide the nucleic acid sequence encoding the *Arabidopsis* PLT1 transcription factor (referred to in the applications as G1793). Overexpression of G1793 (using the CaMV 35S promoter) in *Arabidopsis* was reported to produce alterations in cotyledon morphology, a mild reduction in overall plant size and thin inflorescences (possibly with abnormal flowers) compared to wild type controls. G1793 overexpressors produced more seed oil than control plants. Nucleic acid sequences encoding potential *Glycine max, Oryza sativa* and *Zea mays* orthologs are provided.

International patent application WO 03/002751 provides for a *Glycine max* nucleic acid sequence encoding a PLT polypeptide, with sequence similarity to a corn nucleic acid sequence identified by gene profiling (by DNA microarray).

International patent application WO 05/075655 provides for *Oryza sativa* and Zea *mays* nucleic acid sequences with sequence similarity to the *Arabidopsis* PLT genes.

### bHLH

The basic/helix-loop-helix (bHLH) proteins are a superfamily of transcription factors that bind as dimers to specific DNA target sites and that have been well characterized in non-plant eukaryotes as important regulatory components in diverse biological processes. The distinguishing characteristic of the bHLH family is a bipartite domain consisting of approximately 60 amino acids. This bipartite domain is comprised of a DNA-binding basic region, which binds to a consensus hexanucleotide E-box and two α-helices separated by a variable loop region. The two α-helices promote dimerization, allowing the formation of homo- and heterodimers between different family members. While the bHLH domain is evolutionarily conserved, there is little sequence similarity between clades beyond the domain.

Bailey et al., 2003 (The Plant Cell, Vol. 15, 2497-2501) report the total number of detected bHLH genes in *Arabidopsis thaliana* to be 162, making bHLH genes one of the largest families of transcription factors in *Arabidopsis*; the rice genome reportedly contains 131 bHLH genes (Buck and Atchley, 2003 (J. Mol. Evol. 56:742-750)). Heim et al., 2003 (Mol. Biol. Evol. 20(5):735-747) identified 12 subfamilies of bHLH genes from *Arabidopsis thaliana* based on structural similarities.

The bHLH proteins from plants that have been characterized have been reported to function in anthocyanin biosynthesis, phytochrome signaling, globulin expression, fruit dehiscence, carpel and epidermal development (Buck and Atchley, 2003).

### SPL

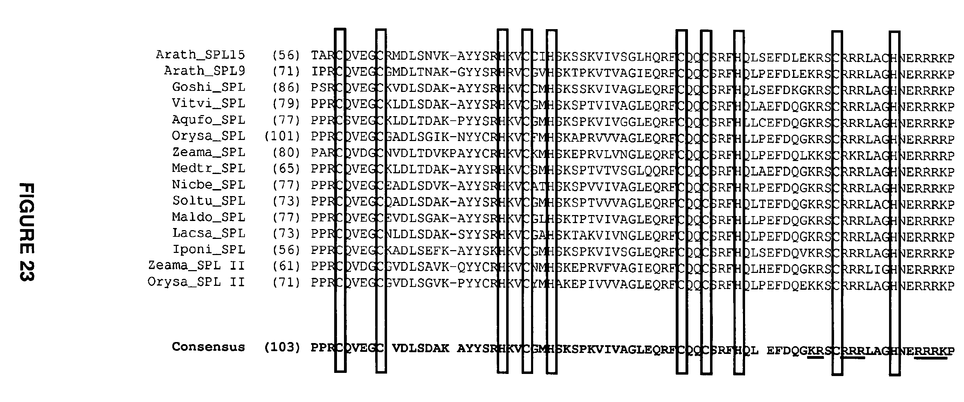

The Squamosa promoter binding protein-like (SPL) transcription factor polypeptides are structurally diverse proteins that share a highly conserved DNA binding domain (DBD) of about 80 amino acid residues in length (Klein et al. (1996) Mol Gen Genet 259: 7-16; Cardon et al. (1999) Gene 237: 91-104). The SPL transcription factor DNA consensus sequence binding site in the promoter of target genes is 5'-TNCGTACAA-3' where N represents any base. Within the SPL DBD are ten conserved cysteine (Cys) or histidine (His) residues (see Figure 23) of which eight are zinc coordinating residues binding two zinc ions necessary for the formation of SPL specific zinc finger tertiary structure (Yamasaki et al. (2004) J Mol Biol 337: 49-63). A second conserved feature within the SPL DBD is a bipartite nuclear localisation signal. Outside of the DBD, a micro RNA (miRNA) target motif (miR156) is found in most of the nucleic acid sequences encoding SPL transcription factor polypeptides (either in the coding region, or the 3' UTR) across the plant kingdom (Rhoades et al. (2002) Cell 110: 513-520). miRNAs control SPL gene expression post-transcriptionally by targeting SPL encoding mRNAs for degradation or by translational repression.

Riechmann et al. (Science 290: 2105-2109, 2000) report 16 SPL transcription factor polypeptides in *Arabidopsis thaliana,* with little sequence similarity between themselves (apart from the abovementioned features), the size of the deduced SPL polypeptide ranging from 131 to 927 amino acids. Nevertheless, pairs of SPL transcription factor polypeptides sharing higher sequence homology were detected within the SPL family of this plant (Cardon *et al.* (1999)).

The SPL transcription factor polypeptides (only found in plants) characterized to date have been shown to function in plant development, in particular in flower development. Transgenic plants overexpressing an SPL3 transcription factor polypeptide were reported to flower earlier (Cardon et al. (1997) Plant J 12: 367-377).

In European patent application EP1033405, the nucleic acid and deduced polypeptide sequences of the SPL15 transcription factor polypeptide are presented.

In international patent application WO03013227, a nucleic acid sequence (and deduced polypeptide sequence; internal reference G2346) is presented that encodes part of the SPL15 transcription factor polypeptide however 38 amino acids from the C-terminal end of the SPL15 transcription factor. Transgenic *Arabidopsis thaliana* plants constitutively overexpressing the modified SPL15 transcription factor (or G2346) polypeptide have slightly enlarged cotyledons. At later stages of development, the same plants are reported to show no consistent differences from control plants.

### SUMMARY OF THE INVENTION

Surprisingly, it has now been found that preferentially increasing expression of a nucleic acid encoding a HAL3 polypeptide in expanding tissues gives plants having enhanced yield-related traits, in particular increased early vigour and increased seed yield relative to control plants, which seed yield increase is at least 10% compared to the control plants.

According to another embodiment of present invention, there is provided a method for enhancing yield-related traits, in particular for increasing early vigour of a plant and increasing seed yield of a plant, relative to control plants, comprising preferentially increasing expression of a nucleic acid encoding a HAL3 polypeptide in expanding tissues of a plant.

Surprisingly, it has now been found that modulating expression of a nucleic acid encoding a MADS15 polypeptide gives plants having enhanced yield-related traits, in particular increased yield relative to control plants.

According one embodiment, there is provided a method for increasing plant yield relative to control plants, comprising increasing expression of a nucleic acid encoding a MADS15 polypeptide in a plant. The increased yield comprised increased vegetative biomass but not increased seed yield.

According to another embodiment, the present invention provides methods for increasing yield of a plant relative to control plants, comprising decreasing the level and/or activity of an endogenous MADS15 polypeptide. The increased yield comprised higher seed yield, but did not comprise increased vegetative biomass.

Surprisingly, it has now been found that increasing expression of a nucleic acid encoding a PLT transcription factor polypeptide gives plants having enhanced yield related traits, in particular increased yield relative to control plants.

According to a further embodiment, there is provided a method for increasing plant yield relative to control plants, comprising increasing expression in a plant of a nucleic acid encoding a PLT transcription factor polypeptide.

Surprisingly, it has now been found that modulating expression in a plant of a nucleic acid encoding a particular class of bHLH transcription factor gives plants having enhanced yield-related traits relative to control plants. The particular class of bHLH transcription factor suitable for enhancing yield-related traits in plants is described in detail below.

According to a further embodiment, the present invention provides a method for enhancing yield-related traits in plants relative to control plants, comprising modulating expression in a plant of a nucleic acid encoding a particular class of bHLH transcription factor.

Surprisingly, it has now been found that increasing expression in a plant of a nucleic acid encoding an SPL15 transcription factor polypeptide gives plants having enhanced yield related traits, in particular increased yield relative to control plants.

According to a further embodiment, the invention provides a method for increasing yield in plants relative to control plants, comprising increasing expression in a plant of a nucleic acid encoding an SPL15 transcription factor polypeptide.

### DEFINITIONS

### Polypeptide(s)/Protein(s)

The terms "polypeptide" and "protein" are used interchangeably herein and refer to amino acids in a polymeric form of any length.

### Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)" are used interchangeably herein and refer to nucleotides, either ribonucleotides or deoxyribonucleotides or a combination of both, in a polymeric form of any length.

### Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

### Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, IacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company and Table 1 below).

**Table 1: Examples of conserved amino acid substitutions**

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

### Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

### Orthologue(s)/Paralogue(s)

Orthologues and paralogues encompass evolutionary concepts used to describe the ancestral relationships of genes. Paralogues are genes within the same species that have originated through duplication of an ancestral gene and orthologues are genes from different organisms that have originated through speciation.

### Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

### Motif/Consensus sequence/Signature

The term "motif' or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

### Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tₘ is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tₘ decreases about 1 °C per % base mismatch. The Tₘ may be calculated using the following equations, depending on the types of hybrids:
1) DNA-DNA hybrids (Meinkoth and Wahl, Anal. Biochem., 138: 267-284, 1984): ${T}_{m} = 81.5 ⁢ °C + 16.6 ⁢ {xlog}_{10} ⁢ {\left[{Na}^{+}\right]}^{a} + 0.41 ⁢ x % \left[G/{C}^{b}\right] - 500 ⁢ x ⁢ {\left[{L}^{c}\right]}^{- 1} - 0.61 ⁢ x % formamide$
2) DNA-RNA or RNA-RNA hybrids: ${T}_{m} = 79.8 + 18.5 ⁢ \left({log}_{10}⁢{\left[{Na}^{+}\right]}^{a}\right) + 0.58 \left(%G/{C}^{b}\right) + 11.8 ⁢ {\left(%G/{C}^{b}\right)}^{2} - 820 / {L}^{c}$
3) oligo-DNA or oligo-RNA^{d} hybrids:
   For <20 nucleotides: Tₘ= 2 (Iₙ)
   For 20-35 nucleotides: Tₘ= 22 + 1.46 (Iₙ)
      ^{a} or for other monovalent cation, but only accurate in the 0.01-0.4 M range.
      ^{b} only accurate for %GC in the 30% to 75% range.
      ^{c} L = length of duplex in base pairs.
      ^{d} Oligo, oligonucleotide; Iₙ, effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5 × Denhardt's reagent, 0.5-1.0% SDS, 100 µg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989 and yearly updates).

### Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex, BMC Bioinformatics. 2005; 6: 25).

### Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

### Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

### Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1,000 transcripts per cell.

### Operably linked

The term "operably linked" as used herein refers to a functional linkage between the promoter sequence and the gene of interest, such that the promoter sequence is able to initiate transcription of the gene of interest.

### Constitutive promoter

A "constitutive promoter" refers to a promoter that is transcriptionally active during most, but not necessarily all, phases of growth and development and under most environmental conditions, in at least one cell, tissue or organ. Table 2 below gives examples of constitutive promoters.

**Table 2: Examples of constitutive promoters**

| Gene Source | Reference |
|---|---|
| Actin | McElroy et al, Plant Cell, 2: 163-171, 1990 |
| HMGB | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

### Ubiquitous promoter

A ubiquitous promoter is active in substantially all tissues or cells of an organism.

### Developmentally-regulated promoter

A developmentally-regulated promoter is active during certain developmental stages or in parts of the plant that undergo developmental changes.

### Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

### Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 3 below.

**Table 3: Examples of green tissue-specific promoters**

| **Gene** | **Expression** | **Reference** |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts

### Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

### Modulation

The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

### Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

### Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene.

### Decreased expression

Reference herein to "reduction or substantial elimination" is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, a polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, Schwab R, 2005. Convenient tools for design and generation of amiRNAs and their precursors are also available to the public, Schwab et al., 2006.

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

### Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta^{®}; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

### Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

### Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ und Bent, AF (1998). The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

### T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to modified expression of genes near the inserted T-DNA. The resulting transgenic plants show dominant phenotypes due to modified expression of genes close to the introduced promoter.

### TILLING

TILLING (Targeted Induced Local Lesions In Genomes) is a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

### Homologous recombination

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8).

### Yield

The term "yield" in general means a measurable produce of economic value, typically related to a specified crop, to an area, and to a period of time. Individual plant parts directly contribute to yield based on their number, size and/or weight, or the actual yield is the yield per acre for a crop and year, which is determined by dividing total production (includes both harvested and appraised production) by planted acres.

### Early vigour

Early vigour (active healthy well-balanced growth especially during early stages of plant growth) may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

### Increase/Improve/Enhance

The terms "increase", "improve" or "enhance" are interchangeable and shall mean in the sense of the application at least a 5%, 6%, 7%, 8%, 9% or 10%, preferably at least 15% or 20%, more preferably 25%, 30%, 35% or 40% more yield and/or growth in comparison to control plants as defined herein.

### Seed yield

Increased seed yield may manifest itself as one or more of the following: a) an increase in seed biomass (total seed weight) which may be on an individual seed basis and/or per plant and/or per hectare or acre; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

### Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., Cocos spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma* cacao, *Trifolium* spp., *Triticosecale rimpaui, Triticum* spp. *(e.g. Triticum aestivum, Triticum durum, Triticum turgidum, Triticum hybemum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

### DETAILED DESCRIPTION OF THE INVENTION

### Detailed description for HAL3

According to a first embodiment of the present invention, there is provided a method for increasing plant yield relative to control plants, comprising preferentially increasing expression of a nucleic acid encoding a HAL3 polypeptide in expanding tissues of a plant.

Any reference hereinafter to a "protein useful in the methods of the invention" is taken to mean a HAL3 polypeptide as defined herein. Any reference hereinafter to a "nucleic acid useful in the methods of the invention" is taken to mean a nucleic acid capable of encoding such an HAL3 polypeptide. The nucleic acid to be introduced into a plant (and therefore useful in performing the methods of the invention) is any nucleic acid encoding the type of protein which will now be described, hereafter also named *"HAL3* nucleic acid" or *"HAL3* gene".

A "reference", "reference plant", "control", "control plant", "wild type" or "wild type plant" is in particular a cell, a tissue, an organ, a plant, or a part thereof, which was not produced according to the method of the invention. Accordingly, the terms "wild type", "control" or "reference" are exchangeable and can be a cell or a part of the plant such as an organelle or tissue, or a plant, which was not modified or treated according to the herein described method according to the invention. Accordingly, the cell or a part of the plant such as an organelle or a plant used as wild type, control or reference corresponds to the cell, plant or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property. That means in other words that the wild type denotes (1) a plant, which carries the unaltered or not modulated form of a gene or allele or (2) the starting material/plant from which the plants produced by the process or method of the invention are derived.

Preferably, any comparison between the wild type plants and the plants produced by the method of the invention is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, a plant, which was not modulated, modified or treated according to the herein described process of the invention and is in any other property as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or plant, relates to an organelle, cell, tissue or plant, which is nearly genetically identical to the organelle, cell, tissue or plant, of the present invention or a part thereof preferably 95%, more preferred are 98%, even more preferred are 99,00%, in particular 99,10%, 99,30%, 99,50%, 99,70%, 99,90%, 99,99%, 99, 999% or more. Most preferable the "reference", "control", or "wild type" is preferably a subject, e.g. an organelle, a cell, a tissue, a plant, which is genetically identical to the plant, cell organelle used according to the method of the invention except that nucleic acid molecules or the gene product encoded by them are changed, modulated or modified according to the inventive method.

In case, a control, reference or wild type differing from the subject of the present invention only by not being subject of the method of the invention can not be provided, a control, reference or wild type can be a plant in which the cause for the modulation of the activity conferring the increase of the metabolites as described under examples.

The increase referred to the activity of the polypeptide amounts in a cell, a tissue, a organelle, an organ or an organism or a part thereof preferably to at least 5%, preferably to at least 10% or at to least 15%, especially preferably to at least 20%, 25%, 30% or more, very especially preferably are to at least 40%, 50% or 60%, most preferably are to at least 70% or more in comparison to the control, reference or wild type.

The term "increased yield" is taken to mean an increase in biomass (weight) of one or more parts of a plant, which may include aboveground (harvestable) parts and/or (harvestable) parts below ground. Preferably, the increase in yield is at least 10% over the yield of corresponding wild type plants.

In particular, such harvestable parts are seeds, and performance of the methods of the invention results in plants having increased seed yield relative to the seed yield of control plants.

The term "expression" or "gene expression" means the appearance of a phenotypic trait as a consequence of the transcription of a specific gene or specific genes. The term "expression" or "gene expression" in particular means the transcription of a gene or genes into structural RNA (rRNA, tRNA) or mRNA with subsequent translation of the latter into a protein. The process includes transcription of DNA, processing of the resulting mRNA product and its translation into an active protein.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of suitable control plants.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

According to a preferred feature of the present invention, performance of the methods of the invention gives plants having increased seed yield relative to control plants. Therefore according to the present invention, there is provided a method for increasing seed yield in plants relative to the seed yield of control plants, the method comprising preferentially increasing expression of a nucleic acid encoding a HAL3 polypeptide in shoot tissues, preferably in expanding tissues of a plant shoot.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a HAL3 polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to suitable control plants grown under comparable conditions. Therefore, according to the present invention, there is provided a method for increasing yield in plants grown under non-stress conditions or under mild drought conditions, which method comprises increasing expression in a plant of a nucleic acid encoding a HAL3 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a HAL3 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

According to a second preferred feature of the invention, performance of the methods of the invention gives plants having increased plant vigour relative to control plants, particularly during the early stages of plant development (typically three, four weeks post germination in the case of rice and maize, but this will vary from species to species) leading to early vigour. Therefore, according to the present invention, there is provided a method for increasing the plant early vigour, which method comprises modulating, preferably increasing expression in a plant of a nucleic acid encoding a HAL3 polypeptide. Preferably the increase in seedling vigour is achieved by expressing the nucleic acid encoding the HAL3 polypeptide under the control of a shoot specific promoter. There is also provided a method for producing plants having early vigour relative to control plants, which method comprises modulating, preferably increasing, expression in a plant of a nucleic acid encoding a HAL3 polypeptide.

Early vigour may also result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera *Helianthus, Tagetes* e.g. the species *Helianthus annus* [sunflower], *Tagetes lucida, Tagetes erecta* or *Tagetes tenuifolia* [Marigold], *Brassicaceae* such as the genera *Brassica, Arabadopsis* e.g. the species *Brassica napus, Brassica rapa* ssp. [canola, oilseed rape, turnip rape] or *Arabidopsis thaliana. Fabaceae* such as the genera *Glycine* e.g. the species *Glycine max, Soja hispida* or *Soja max* [soybean]. *Linaceae* such as the genera *Linum* e.g. the species *Linum usitatissimum,* [flax, linseed]; Poaceae such as the genera *Hordeum, Secale, Avena, Sorghum, Oryza, Zea, Triticum* e.g. the species *Hordeum vulgare* [barley]; *Secale cereale* [rye], *Avena sativa, Avena fatua, Avena byzantina, Avena fatua* var. *sativa, Avena hybrida* [oat], *Sorghum bicolor* [Sorghum, millet], *Oryza sativa, Oryza latifolia* [rice], *Zea mays* [corn, maize] *Triticum aestivum, Triticum durum, Triticum turgidum, Triticum hybernum, Triticum* macha, *Triticum sativum* or *Triticum vulgare* [wheat, bread wheat, common wheat]; *Solanaceae* such as the genera *Solanum, Lycopersicon* e.g. the species *Solanum tuberosum* [potato], *Lycopersicon esculentum, Lycopersicon lycopersicum, Lycopersicon pyriforme, Solanum integrifolium* or *Solanum lycopersicum* [tomato].

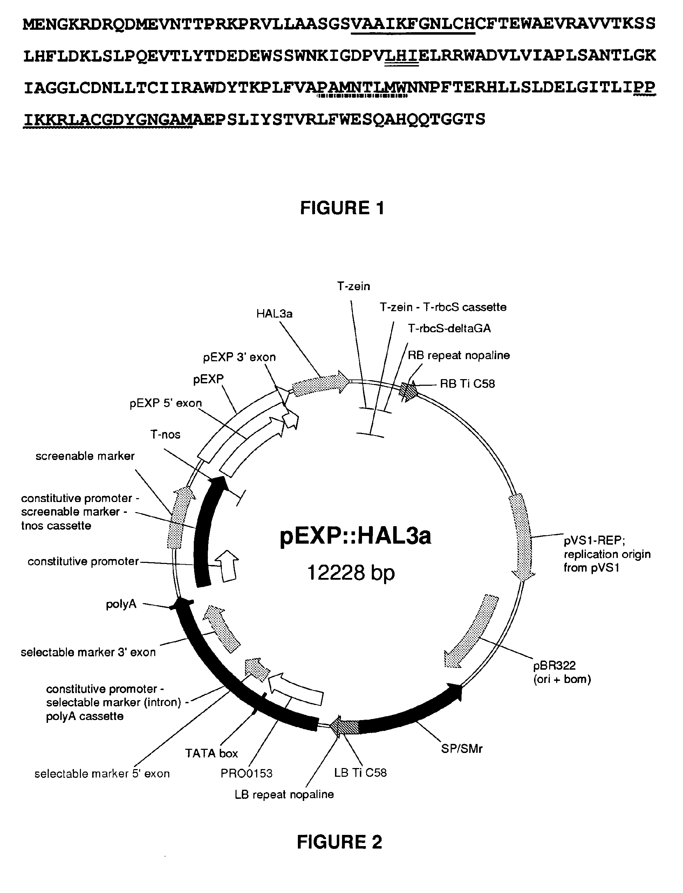

The term "HAL3 polypeptide" as defined herein refers to a flavoprotein belonging to the superfamily HFCD (Homo-oligomeric Flavin containing Cys Decarboxylases; Kupke J. Biol.Chem. 276, 27597-27604, 2001). These proteins share a flavin-binding motif, conserved active-site residues and are trimeric or dodecameric enzymes. HAL3 proteins preferably comprise from N-terminus to C-terminus (i) a substrate binding helix, (ii) an insertion His motif, (iii) a PXMNXXMW motif and (iv) a substrate recognition clamp (Figure 1). These four domains are predicted to be involved in substrate binding, the insertion His motif comprises a conserved His residue that is involved in the active site whereas the sequence in the substrate recognition clamp may be somewhat variable in sequence and length (Blaesse et al., EMBO J. 19, 6299-6310, 2000). The structural features (i) to (iv) are also described in Kupke et al.(2001), which disclosure is incorporated herein by reference. Typically, HAL3 polypeptides are capable of binding of FMN cofactors.

Typically the substrate binding helix is comprised in sequence motif 1 with the following consensus sequence (SEQ ID NO: 6): 
(K/R)PR(V/I)(I/L)LAA(S/T)GSVA(A/S)(I/M/V)KF(G/E/A/S)(N/S/I)L(C/V/A)(H
/R/G)(C/S/I)(F/L)(T/S/C)(E/D/Q)WA(E/D)V(R/K)AV(V/A/S).
 The insertion His motif, the PXMNXXMW motif and the substrate recognition clamp are part of sequence motif 2 with the following consensus sequence (SEQ ID NO: 7): 
VLHIELR(R/K/Q)WAD(V/I/A)(L/M)(V/I)IAPLSANTL(G/A)KIAGG(L/M)CDNLLTC(I/V
)(I/V)RAWD(Y/F)(T/S/N/D/K)KP(L/F/M/I)F(V/A)APAMNT(L/F)MW(N/S/T)NPFT(E
/S/Q/A)(R/K)H(L/F/I)(X1)(X2)(L/I/M)(D/N/S)(E/L/Q)(L/M)G(I/V/L)(T/S/A/I
)L(I/V)PP(I/V/T)(K/T/S)K(R/T/K)LACGD(Y/H)G(N/T)GAM(A/S)E
 wherein X₁ may be any amino acid, preferably x₁ is one of L, V, E, H, D, M, I or Q and wherein X₂ may be any amino acid, preferably X₂ is one of S, L, T, A, or V.

These motifs form part of a larger conserved region in the protein, as an example, the conserved region of *Arabidopsis* HAL3a is given as SEQ ID NO: 8. HAL3 polypeptides useful in the methods of the present invention have in increasing order of preference at least 65%, 70%, 75%, 80%, 85%, 90% or 95% sequence identity to the conserved region represented by SEQ ID NO: 8.

The conserved region in HAL3 proteins, as exemplified in SEQ ID NO: 8 and comprising the above described features (i) to (iv), encompasses a Flavoprotein domain which may be identified using specialised databases e.g. SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)). This FMN-binding domain (Pfam entry PF02441, InterPro entry IPR003382) is typically found in flavoprotein enzymes. The terms "domain" and "motif" are defined in the definitions section above.

The conserved region in SEQ ID NO: 2, as represented by SEQ ID NO: 8, may also in other HAL3 proteins be identified, using methods for the alignment of sequences for comparison as described hereinabove. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified, including the conserved motifs 1 and 2 (SEQ ID NO: 6 and 7), or matches with one or more conservative change at any position.

HAL3 polypeptides (at least in their native form) and their homologues typically catalyse the decarboxylation of 4'-phosphopantothenoylcysteine to 4'-phosphopantetheine, a step in coenzyme A biosynthesis, which reaction may be tested in a biochemical assay; alternatively, HAL3 activity may be assayed by a complementation test with a *dfp* mutant E. coli strain (Kupke et al 2001; Yonamine et al 2004). The enzyme is also able to decarboxylate pantothenoylcysteine to pantothenoylcysteamine. Furthermore, the protein is involved in conferring salt and osmotic stress tolerance to plants, which feature may be useful in a bioassay for HAL3.

SEQ ID NO: 2 (encoded by SEQ ID NO: 1) is an example of a HAL3 polypeptide comprising from N-terminus to C-terminus features (i) a substrate binding helix, (ii) an insertion His motif, (iii) a PXMNXXMW motif and (iv) a substrate recognition clamp; and having in increasing order of preference at least 65%, 70%, 75%, 80%, 85%, 90% or 95% sequence identity to the conserved region represented by SEQ ID NO: 8. Further examples of HAL3 polypeptides comprising features (i) to (iv) are given in Table A in the examples section below:
Homologues of a HAL3 polypeptide may also be used to perform the methods of invention. Homologues (or homologous proteins) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art.

The sequence identity values may be determined over the entire conserved domain (as indicated above) or over the full length nucleic acid or amino acid sequence using the programs mentioned above using the default parameters.

Homologues also include orthologues and paralogues. Orthologues and paralogues may easily be found by performing a so-called reciprocal blast search. This may be done by a first BLAST involving submitting a query sequence (for example, SEQ ID NO: 1 or SEQ ID NO: 2) for a BLAST search against any sequence database, such as the publicly available NCBI database. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then submitted to a second BLAST search (BLAST back) against sequences from the organism from which the query sequence is derived (where the query sequence is SEQ ID NO: 1 or SEQ ID NO: 2 the second BLAST would therefore be against *Arabidopsis* sequences). The results of the first and second BLAST searches are then compared. A paralogue is identified if a high-ranking hit from the first BLAST is from the same species as from which the query sequence is derived; an orthologue is identified if a high-ranking hit is not from the same species as from which the query sequence is derived. Preferred orthologues are orthologues of AtHAL3a (SEQ ID NO: 2), AtHAL3b (SEQ ID NO: 10) or of the long form of AtHAL3b (SEQ ID NO: 40). High-ranking hits are those having a low E-value. The lower the E-value, the more significant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues. In addition to E-values, comparisons are also scored by percentage identity. Percentage identity refers to the number of identical nucleotides (or amino acids) between the two compared nucleic acid (or polypeptide) sequences over a particular length. Preferably, HAL3 polypeptide homologues have in increasing order of preference at least 60%, 70%, 80%, 85%, 90%, 95%, 96%, 97%, 98% or 99% or more sequence identity or similarity (functional identity) to an unmodified HAL3 polypeptide as represented by SEQ ID NO: 2. Preferably, HAL3 polypeptide homologues are as represented by the sequences referred to in Table A.

The HAL3 polypeptide useful in the methods of the present invention may be a derivative of SEQ ID NO: 2. "Derivatives" include peptides, oligopeptides, polypeptides which may, compared to the amino acid sequence of the naturally-occurring form of the protein, such as the one presented in SEQ ID NO: 2, comprise substitutions of amino acids with non-naturally occurring amino acid residues, or additions of non-naturally occurring amino acid residues. Derivatives of the proteins as represented by the sequences listed in Table A are further examples which may be suitable for use in the methods of the invention

Examples of nucleic acids encoding HAL3 polypeptides include but are not limited to those represented by the sequences listed in Table A. Variants of nucleic acids encoding HAL3 polypeptides may be suitable for use in the methods of the invention. Suitable variants include portions of nucleic acids encoding HAL3 polypeptides and/or nucleic acids capable of hybridising with nucleic acids/genes encoding HAL3 polypeptides. Further variants include splice variants and allelic variants of nucleic acids encoding HAL3 polypeptides.

The term "portion" as defined herein refers to a piece of DNA encoding a polypeptide comprising from N-terminus to C-terminus features (i) a substrate binding helix, (ii) an insertion His motif, (iii) a PXMNXXMW motif and (iv) a substrate recognition clamp; and having in increasing order of preference at least 65%, 70%, 75%, 80%, 85%, 90% or 95% sequence identity to the conserved region represented by SEQ ID NO: 8.

A portion may be prepared, for example, by making one or more deletions to a nucleic acid encoding a HAL3 polypeptide. The portions may be used in isolated form or they may be fused to other coding (or non coding) sequences in order to, for example, produce a protein that combines several activities. When fused to other coding sequences, the resulting polypeptide produced upon translation may be bigger than that predicted for the HAL3 portion. Preferably, the portion codes for a polypeptide with substantially the same biological activity as the HAL3 polypeptide of SEQ ID NO: 2. The portion is typically at least 50, 100, 150 or 200 nucleotides in length, preferably at least 250, 300, 350 or 400 nucleotides in length, more preferably at least 450, 500, 550, 600 or 650 nucleotides in length.

Preferably, the portion is a portion of a nucleic acid as represented by the sequences listed in Table A. Most preferably the portion is a portion of a nucleic acid as represented by SEQ ID NO: 1.

The terms "fragment", "fragment of a sequence" or "part of a sequence" "portion" or "portion thereof' mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybidizing with the nucleic acid molecule of the invention or used in the process of the invention under stringend conditions, while the maximum size is not critical. In some applications, the maximum size usually is not substantially greater than that required to provide the desired activity and/or function(s) of the original sequence. A comparable function means at least 40%, 45% or 50%, preferably at least 60%, 70%, 80% or 90% or more of the original sequence.

Another variant of a nucleic acid encoding a HAL3 polypeptide, useful in the methods of the present invention, is a nucleic acid capable of hybridising under reduced stringency conditions, preferably under stringent conditions, with a probe derived from the nucleic acid as defined hereinbefore, which hybridising sequence encodes a polypeptide comprising from N-terminus to C-terminus features (i) a substrate binding helix, (ii) an insertion His motif, (iii) a PXMNXXMW motif and (iv) a substrate recognition clamp; and has in increasing order of preference at least 65%, 70%, 75%, 80%, 85%, 90% or 95% sequence identity to the conserved region represented by SEQ ID NO: 8.

Preferably, the hybridising sequence is one that is capable of hybridising to a nucleic acid as represented by (or to probes derived from) the sequences listed in Table A, or to a portion of any of these sequences (the target sequence). Most preferably the hybridising sequence is capable of hybridising to SEQ ID NO: 1 (or to probes derived therefrom). Probes are generally less than 700 bp or 600 bp in length, preferably less than 500, 400 bp, 300 bp 200 bp or 100 bp in length. Commonly, probe lengths for DNA-DNA hybridisations such as Southern blotting, vary between 100 and 500 bp, whereas the hybridising region in probes for DNA-DNA hybridisations such as in PCR amplification generally are shorter than 50 but longer than 10 nucleotides, preferably they are 15, 20, 25, 30, 35, 40, 45 or 50 bp in length.

The HAL3 polypeptide may be encoded by a splice variant. The term "splice variant" as used herein encompasses variants of a nucleic acid sequence in which selected introns and/or exons have been excised, replaced, displaced or added, or in which introns have been shortened or lengthened. Such variants will be ones in which the substantial biological activity of the protein is retained, which may be achieved by selectively retaining functional segments of the protein. Such splice variants may be found in nature or may be manmade. Methods for making such splice variants are well known in the art.

Preferred splice variants are splice variants of the nucleic acid encoding a HAL3 polypeptide comprising from N-terminus to C-terminus (i) a substrate binding helix, (ii) an insertion His motif, (iii) a PXMNXXMW motif and (iv) a substrate recognition clamp; and having in increasing order of preference at least 65%, 70%, 75%, 80%, 85%, 90% or 95% sequence identity to the conserved region represented by SEQ ID NO: 8.

Further preferred splice variants of nucleic acids encoding HAL3 polypeptides comprising features as defined hereinabove are splice variants of a nucleic acid as represented by any one of the sequences listed in Table A. Most preferred is a splice variant of a nucleic acid sequence as represented by SEQ ID NO: 1.

The HAL3 polypeptide may also be encoded by an allelic variant of a nucleic acid encoding a polypeptide comprising from N-terminus to C-terminus (i) a substrate binding helix, (ii) an insertion His motif, (iii) a PXMNXXMW motif and (iv) a substrate recognition clamp; and having in increasing order of preference at least 65%, 70%, 75%, 80%, 85%, 90% or 95% sequence identity to the conserved region represented by SEQ ID NO: 8.

Preferred allelic variants of nucleic acids encoding HAL3 polypeptides comprising features as defined hereinabove are allelic variants of a nucleic acid as represented by any one of the sequences listed in Table A. Most preferred is an allelic variant of a nucleic acid sequence as represented by SEQ ID NO: 1.

Directed evolution (or gene shuffling) may also be used to generate variants of nucleic acids encoding HAL3 polypeptides. Site-directed mutagenesis may be used to generate variants of nucleic acids encoding HAL3 polypeptides. Several methods are available to achieve site-directed mutagenesis, the most common being PCR based methods (Current Protocols in Molecular Biology. Wiley Eds.).

Nucleic acids encoding HAL3 polypeptides may be derived from any natural or artificial source. The nucleic acid may be modified from its native form in composition and/or genomic environment through deliberate human manipulation. Preferably the *HAL3* nucleic acid is from a plant, further preferably from a dicotyledonous plant, more preferably from the family Brassicaceae, most preferably the nucleic acid is from *Arabidopsis thaliana.*

The increased expression of a nucleic acid encoding a HAL3 polypeptide, preferentially in shoots of a plant, may be performed by introducing a genetic modification (preferably in the locus of a *HAL3* gene). The locus of a gene as defined herein is taken to mean a genomic region, which includes the gene of interest and 10KB up- or downstream of the coding region.

The genetic modification may be introduced, for example, by any one (or more) of the following methods: T-DNA activation, TILLING and homologous recombination (as described in the definitions section) or by introducing and increasing expression a nucleic acid encoding a HAL3 polypeptide preferentially in expanding tissues of a plant. Following introduction of the genetic modification, there follows an optional step of selecting for increased expression of a nucleic acid encoding a HAL3 factor polypeptide preferentially in expanding tissues, which increased expression gives plants having increased yield.

T-DNA activation tagging results in transgenic plants that show dominant phenotypes due to modified expression of genes close to the introduced promoter. The promoter to be introduced may be any promoter capable of increasing expression of the nucleic acid encoding a HAL3 polypeptide preferentially in expanding tissues of a plant.

A genetic modification may also be introduced in the locus of a gene encoding a HAL3 polypeptide using the technique of TILLING (Targeted Induced Local Lesions In Genomes). Plants carrying such mutant variants have increased expression of a nucleic acid encoding a HAL3 polypeptide preferentially in expanding plant tissues.

T-DNA activation and TILLING are examples of technologies that enable the generation of genetic modifications comprising preferentially increasing expression of a nucleic acid encoding a HAL3 polypeptide in expanding tissues of plants.

The effects of the invention may also be reproduced using homologous recombination. The nucleic acid to be targeted is preferably the region controlling the natural expression of a nucleic acid encoding a HAL3 polypeptide in a plant. A weak promoter specific for expression in shoots is introduced into this region, replacing it partly or substantially all of it.

A preferred method for introducing a genetic modification (which in this case need not be in the locus of a *HAL3* gene) is to introduce and express preferentially in the shoot of a plant a nucleic acid encoding a HAL3 polypeptide, as defined hereinabove. The nucleic acid to be introduced into a plant may be a full-length nucleic acid or may be a portion or a hybridising sequence or another nucleic acid variant as hereinbefore defined.

The methods of the invention rely on preferentially increased expression of a nucleic acid encoding a HAL3 polypeptide in shoot tissue of a plant, preferably in the cell expansion zone of vegetative shoots.

The invention also provides genetic constructs and vectors to facilitate introduction and/or preferential expression of the nucleic acid sequences useful in the methods according to the invention, in shoots, preferably in expanding tissues of a plant shoots.

Therefore, there is provided a gene construct comprising:
(i) a nucleic acid encoding a HAL3 polypeptide as defined hereinabove;
(ii) one or more control sequences, of which at least one is a shoot-specific promoter, operably linked to the nucleic acid of (i).

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding a HAL3 polypeptide). The skilled artisan is well aware of the genetic elements that must be present on the vector in order to successfully transform, select and propagate host cells containing the sequence of interest. The sequence of interest is operably linked to one or more control sequences (at least to a promoter).

Suitable promoters, which are functional in plants, are generally known. They may take the form of constitutive or inducible promoters. Suitable promoters can enable the development- and/or tissue-specific expression in multi-celled eukaryotes; thus, leaf-, root-, flower-, seed-, stomata-, tuber- or fruit-specific promoters may advantageously be used in plants.

Different plant promoters usable in plants are promoters such as, for example, the USP, the LegB4-, the DC3 promoter or the ubiquitin promoter from parsley.

For expression in plants, the nucleic acid molecule must, as described above, be linked operably to or comprise a suitable promoter which expresses the gene at the right point in time and in a cell- or tissue-specific manner. Usable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268. Stable, constitutive expression of the proteins according to the invention a plant can be advantageous. However, inducible expression of the polypeptide of the invention is advantageous, if a late expression before the harvest is of advantage, as metabolic manipulation may lead to plant growth retardation.

The expression of plant genes can also be facilitated via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which bring gene expression in tissues and organs, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3): 459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or α-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

In a preferred embodiment, the nucleic acid sequence encoding a HAL3 polypeptide is operably linked to a shoot a shoot specific promoter. A shoot-specific promoter refers to any promoter able to drive expression of the gene of interest in vegetative plant shoot tissues. Preferably, the shoot specific promoter is able to preferentially drive expression of the gene of interest in expanding tissues of vegetative shoots, that is in the cell expansion zone of vegetative shoots. Reference herein to "preferentially" driving expression in the shoot is taken to mean driving expression of any sequence operably linked thereto in the cell expansion zone of vegetative shoots substantially to the exclusion of driving expression elsewhere in the plant, apart from any residual expression due to leaky promoter expression. The shoot-specific promoter may be either a natural or a synthetic promoter.

Preferably, the shoot-specific promoter is a promoter isolated from a beta-expansin gene, such as a rice beta expansin EXBP9 promoter (WO 2004/070039), as represented by SEQ ID NO: 5 or a promoter of similar strength and/or a promoter with a similar expression pattern as the rice beta expansin promoter (i.e. a functionally equivalent promoter).

It should be clear that the applicability of the present invention is not restricted to the nucleic acid encoding a HAL3 polypeptide represented by SEQ ID NO: 1, nor is the applicability of the invention restricted to expression of a nucleic acid encoding a HAL3 polypeptide when driven by a beta expansin promoter.

Optionally, one or more terminator sequences (also a control sequence) may be used in the construct introduced into a plant. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

Other control sequences (besides promoter, enhancer, silencer, intron sequences, 3'UTR and/or 5'UTR regions) may be protein and/or RNA stabilizing elements.

For the detection and/or selection of the successful transfer of the nucleic acid sequences as depicted in the sequence protocol and used in the process of the invention, it is advantageous to use marker genes (= reporter genes). These marker genes enable the identification of a successful transfer of the nucleic acid molecules via a series of different principles, for example via visual identification with the aid of fluorescence, luminescence or in the wavelength range of light which is discernible for the human eye, by a resistance to herbicides or antibiotics, via what are known as nutritive markers (auxotrophism markers) or antinutritive markers, via enzyme assays or via phytohormones. Examples of such markers which may be mentioned are GFP (= green fluorescent protein); the luciferin/luceferase system, the β-galactosidase with its colored substrates, for example X-Gal, the herbicide resistances to, for example, imidazolinone, glyphosate, phosphinothricin or sulfonylurea, the antibiotic resistances to, for example, bleomycin, hygromycin, streptomycin, kanamycin, tetracyclin, chloramphenicol, ampicillin, gentamycin, geneticin (G418), spectinomycin or blasticidin, to mention only a few, nutritive markers such as the utilization of mannose or xylose, or antinutritive markers such as the resistance to 2-deoxyglucose. This list is a small number of possible markers. The skilled worker is very familiar with such markers. Different markers are preferred, depending on the organism and the selection method.

The present invention also encompasses plants obtainable by the methods according to the present invention. The present invention therefore provides plants, plant parts or plant cells thereof obtainable by the method according to the present invention, which plants or parts or cells thereof comprise a nucleic acid transgene encoding a HAL3 polypeptide under the control of a shoot-specific promoter.

The invention also provides a method for the production of transgenic plants having increased yield relative to control plants, comprising introduction and preferential expression of a nucleic acid encoding a HAL3 polypeptide in the shoot of a plant.

Host plants for the nucleic acids or the vector used in the method according to the invention, the expression cassette or construct or vector are, in principle, advantageously all plants, which are capable of synthesizing the polypeptides used in the inventive method.

More specifically, the present invention provides a method for the production of transgenic plants having increased yield which method comprises:
(i) introducing and preferentially expressing a nucleic acid encoding a HAL3 polypeptide in the shoot of a plant; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The transfer of foreign genes into the genome of a plant is called transformation. In doing this the methods described for the transformation and regeneration of plants from plant tissues or plant cells are utilized for transient or stable transformation. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Further advantageous transformation methods, in particular for plants, are known to the skilled worker and are described herein below.

Generally after transformation, plant cells or cell groupings are selected for the presence of one or more markers which are encoded by plant-expressible genes co-transferred with the gene of interest, following which the transformed material is regenerated into a whole plant.

As mentioned Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (*Carthamus tinctorius*) or cocoa beans, for example by bathing scarified leaves or leaf segments in an agrobacterial solution and subsequently growing them in suitable media.

In addition to the transformation of somatic cells, which then has to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the influorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). C R Acad Sci Paris Life Sci, 316: 1194-1199], while in the case of the"floral dip" method the developing floral tissue is incubated briefly with a surfactant-treated agrobacterial suspension [Clough, SJ und Bent, AF (1998). The Plant J. 16, 735-743]. A certain proportion of transgenic seeds are harvested in both cases, and these seeds can be distinguished from nontransgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process, which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview can be taken from Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient cointegrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a HAL3 polypeptide. Preferred host cells according to the invention are plant cells.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

The present invention also encompasses use of nucleic acids encoding HAL3 polypeptides and use of HAL3 polypeptides in increasing plant yield as defined hereinabove in the methods of the invention.

Nucleic acids encoding HAL3 polypeptides, or HAL3 polypeptides, may find use in breeding programmes in which a DNA marker is identified which may be genetically linked to a *HAL3* gene. The nucleic acids/genes, or the HAL3 polypeptides may be used to define a molecular marker. This DNA or protein marker may then be used in breeding programmes to select plants having increased yield as defined hereinabove in the methods of the invention.

Allelic variants of a *HAL3* nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

A nucleic acid encoding a HAL3 polypeptide may also be used as probes for genetically and physically mapping the genes that they are a part of, and as markers for traits linked to those genes. Such information may be useful in plant breeding in order to develop lines with desired phenotypes. Such use of *HAL3* nucleic acids requires only a nucleic acid sequence of at least 15 nucleotides in length. The *HAL3* nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the HAL3 nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the *HAL3* nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield, as described hereinbefore. This increased yield may also be combined with other economically advantageous traits, such as further yield-enhancing traits, tolerance to other abiotic and biotic stresses, traits modifying various architectural features and/or biochemical and/or physiological features.

### Detailed description for MADS15 up

Surprisingly, it has now been found that modulating expression in a plant of a nucleic acid encoding a MADS15 polypeptide gives plants having enhanced yield-related traits relative to control plants. The particular class of MADS15 polypeptides suitable for enhancing yield-related traits in plants is described in detail below.

The present invention provides a method for enhancing yield-related traits in plants relative to control plants, comprising modulating expression in a plant of a nucleic acid encoding a MADS15 polypeptide.

Any reference hereinafter to a "protein useful in the methods of the invention" is taken to mean a MADS15 polypeptide as defined herein. Any reference hereinafter to a "nucleic acid useful in the methods of the invention" is taken to mean a nucleic acid capable of encoding such a MADS15 polypeptide.

A preferred method for modulating (preferably, increasing) expression of a nucleic acid encoding a protein useful in the methods of the invention is by introducing and expressing in a plant a nucleic acid encoding a protein useful in the methods of the invention as defined below.

The nucleic acid to be introduced into a plant (and therefore useful in performing the methods of the invention) is any nucleic acid encoding the type of protein which will now be described, hereafter also named "*MADS15* nucleic acid" or "*MADS15* gene".

The term "MADS15 polypeptide" as defined herein refers to a protein that falls in the group of FUL-like MADS box proteins as delineated by Adam et al.(J. Mol. Evol. 62, 15-31). FUL-like MADS box proteins are part of the SQUAMOSA subfamily and have the typical MIKC^{c} architecture: a MADS domain (M) at the N-terminus, followed by an intervening domain (I) involved in dimerisation, a highly conserved keratin domain (K) and a variable domain (C) at the C-terminus, which is responsible for binding of interacting proteins or transcriptional activation (De Bodt et al. Trends in Plant Science 8, 475-483).

Plant MADS15 polypeptides may also be identified by the presence of certain conserved motifs. The presence of these conserved motifs may be identified using methods for the alignment of sequences for comparison as described hereinabove. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified. Upon identification of a MADS15 polypeptide by the presence of these motifs, a person skilled in the art may easily derive the corresponding nucleic acid encoding the polypeptide comprising the relevant motifs, and use a sufficient length of contiguous nucleotides of the same to perform any one or more of the gene silencing methods described above (for the reduction or substantial elimination of an endogenous *MADS15* gene expression).

Typically, the presence of at least one of the motifs 1 to 4 should be sufficient to identify any query sequence as a MADS15, however, the presence of at least motifs 1 and 2 is preferred. The consensus sequence provided is based on the monocot sequences given in the sequence listing. A person skilled in the art would be well aware that the consensus sequence may vary somewhat if further or different sequences (for example from dicotyledonous plants) were used for comparison.

Motif 1, located in the MADS domain (SEQ ID NO: 48):

L(L/V)KKA(H/N)EIS(VI)L(C/Y)DAE(V/I/L)(A/G)(L/A/V)(I/V)(I/V)FS(T/P/A/N
)KGKLYE(Y/F)(A/S)(T/S)(D/N/E)S(K/C/R/S)M(D/E)(N/I/R/K)IL(E/D)R.

Preferably, this conserved sequence motif 1 has the sequence:
LLKKAHEISVLCDAEVA (L/A/V) I (I/V) FS (T/P) KGKLYEYATDS (C/R) M (D/E) (R/K) ILER,
   most preferably the conserved sequence motif 1 has the sequence:
LLKKAHEISVLCDAEVAAIVFSPKGKLYEYATDSRMDKILER

Motif 2, located in the K domain (SEQ ID NO: 49):
KLK (A/S) (K/R) (V/I) E (A/T/S) (L/I) (Q/N) (K/R/N) (S/C/R) (Q/H) (R/K) HLMGE
   Preferably, this conserved sequence motif 2 has the sequence:
KLKAK (V/I) E (A/T) (L/I) QK (S/C) (Q/H) (R/K) HLMGE
   More preferably, this conserved sequence motif 2 has the sequence:
KLKAKIETIQKCHKHLMGE

Optionally, the MADS15 polypeptide or its homologue may comprise in the C domain motif 3 and/or motif 4:
motif 3 (SEQ ID NO: 50):
   Q (P/Q/V/A) QTS (S/F) (S/F) (S/F) (S/F) (S/C/F) (F/M)
motif 4 (SEQ ID NO: 51):
   (G/A/V/L) (L/P) XWMX (S/H)
   wherein the first X residue in motif 4 may be any amino acid, but preferably L, P or H; and wherein the second X residue may be any amino acid, but preferably V or L.

Alternatively, the C-domain (starting behind the keratin domain, i.e. at R175 in SEQ ID NO: 44) may be characterised by the increased occurrence of glutamine (normally 3,93%, here at least 8,77% with a maximum of 26,73%), in addition, the content in alanine, proline, and/or serine may also be increased (above 7,8%, 4,85% and 6,89% respectively).

Alternatively formulated, a preferred MADS15 protein useful in the methods of the present invention comprises at least an N-terminal MADS domain corresponding to the SMART domain SM00432 (Pfam PF00319) followed by a Keratin domain corresponding to the K-box region defined in Pfam as PF01486, more preferably, the MADS15 protein has in its MADS domain the conserved signature of SEQ ID NO: 48 and in its K-domain the conserved signature of SEQ ID NO: 49, most preferably, the MADS15 protein has the sequence given in SEQ ID NO: 44.

Examples of proteins useful in the methods of the invention and nucleic acids encoding the same are given below in table D of Example 8.

Also useful in the methods of the invention are homologues of any one of the amino acid sequences given in table D.

Also useful in the methods of the invention are derivatives of any one of the polypeptides given in table D or orthologues or paralogues of any of the aforementioned SEQ ID NOs. A preferred derivative is a derivative of SEQ ID NO: 44. Derivatives of the polypeptides given in table D are further examples which may be suitable for use in the methods of the invention. Derivatives useful in the methods of the present invention preferably have similar biological and functional activity as the unmodified protein from which they are derived.

The invention is illustrated by transforming plants with the *Oryza sativa* nucleic acid sequence represented by SEQ ID NO: 43, encoding the polypeptide sequence of SEQ ID NO: 44, however performance of the invention is not restricted to these sequences. The methods of the invention may advantageously be performed using any nucleic acid encoding a protein useful in the methods of the invention as defined herein, including orthologues and paralogues, such as any of the nucleic acid sequences given in table D. The amino acid sequences given in table D may be considered to be orthologues and paralogues of the MADS15 polypeptide represented by SEQ ID NO: 44.

Orthologues and paralogues may easily be found by performing a so-called reciprocal blast search. Typically, this involves a first BLAST involving BLASTing a query sequence (for example using any of the sequences listed in table D) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 43 or SEQ ID NO: 44, the second BLAST would therefore be against *Oryza sativa* sequences). The results of the first and second BLASTs are then compared. A paralogue is identified if a high-ranking hit from the first blast is from the same species as from which the query sequence is derived, a BLAST back then ideally results in the query sequence as highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Table D gives examples of orthologues and paralogues of the MADS15 protein represented by SEQ ID NO 44. Further orthologues and paralogues may readily be identified using the BLAST procedure described above.

The proteins of the invention are identifiable by the presence of the conserved MADS and/or keratin domain(s) (shown in Figure 5). Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)).

Domains may also be identified using routine techniques, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the MADS or keratin domain, or one of the motifs defined above) may be used as well. The sequence identity values, which are indicated below in Example 10 as a percentage were determined over the entire nucleic acid or amino acid sequence, and/or over selected domains or conserved motif(s), using the programs mentioned above using the default parameters.

Furthermore, a MADS15 protein may also be identifiable by its ability or inability to bind DNA and to interact with other proteins. DNA-binding activity and protein-protein interactions may readily be determined *in vitro* or *in vivo* using techniques well known in the art. Examples of in vitro assays for DNA binding activity include: gel retardation analysis using known MADS-box DNA binding domains (West et al. (1998) Nucl Acid Res 26(23): 5277-87), or yeast one-hybrid assays. An example of an *in vitro* assay for protein-protein interactions is the yeast two-hybrid analysis (Fields and Song (1989) Nature 340:245-6). Proteins known to interact with OsMADS15 include other MADS proteins (such as those involved in the flowering signalling complex), receptor like kinases such a Clavata1 and Clavata2, Erecta, BRI1 or RSK. Further details are provided in Example 13.

Nucleic acids encoding proteins useful in the methods of the invention need not be full-length nucleic acids, since performance of the methods of the invention does not rely on the use of full-length nucleic acid sequences. Examples of nucleic acids suitable for use in performing the methods of the invention include the nucleic acid sequences given in table D, but are not limited to those sequences. Nucleic acid variants may also be useful in practising the methods of the invention. Examples of such nucleic acid variants include portions of nucleic acids encoding a protein useful in the methods of the invention, nucleic acids hybridising to nucleic acids encoding a protein useful in the methods of the invention, splice variants of nucleic acids encoding a protein useful in the methods of the invention, allelic variants of nucleic acids encoding a protein useful in the methods of the invention and variants of nucleic acids encoding a protein useful in the methods of the invention that are obtained by gene shuffling. The terms portion, hybridising sequence, splice variant, allelic variant and gene shuffling will now be described.

According to the present invention, there is provided a method for enhancing yield-related traits in plants, comprising introducing and expressing in a plant a portion of any one of the nucleic acid sequences given in table D, or a portion of a nucleic acid encoding an orthologue, paralogue or homologue of any of the amino acid sequences given in table D.

Portions useful in the methods of the invention, encode a polypeptide falling within the definition of a nucleic acid encoding a protein useful in the methods of the invention as defined herein and having substantially the same biological activity as the amino acid sequences given in table D. Preferably, the portion is a portion of any one of the nucleic acids given in table D. The portion is typically at least 400 consecutive nucleotides in length, preferably at least 600 consecutive nucleotides in length, more preferably at least 700 consecutive nucleotides in length and most preferably at least 800 consecutive nucleotides in length, the consecutive nucleotides being of any one of the nucleic acid sequences given in table D. Most preferably the portion is a portion of the nucleic acid of SEQ ID NO: 43. Preferably, the portion encodes an amino acid sequence comprising (any one or more of) the MADS and keratin domain as defined herein.

A portion of a nucleic acid encoding a MADS15 protein as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the MADS 15 protein portion.

Another nucleic acid variant useful in the methods of the invention is a nucleic acid capable of hybridising, under reduced stringency conditions, preferably under stringent conditions, with a nucleic acid encoding a MADS15 protein as defined herein, or with a portion as defined herein.

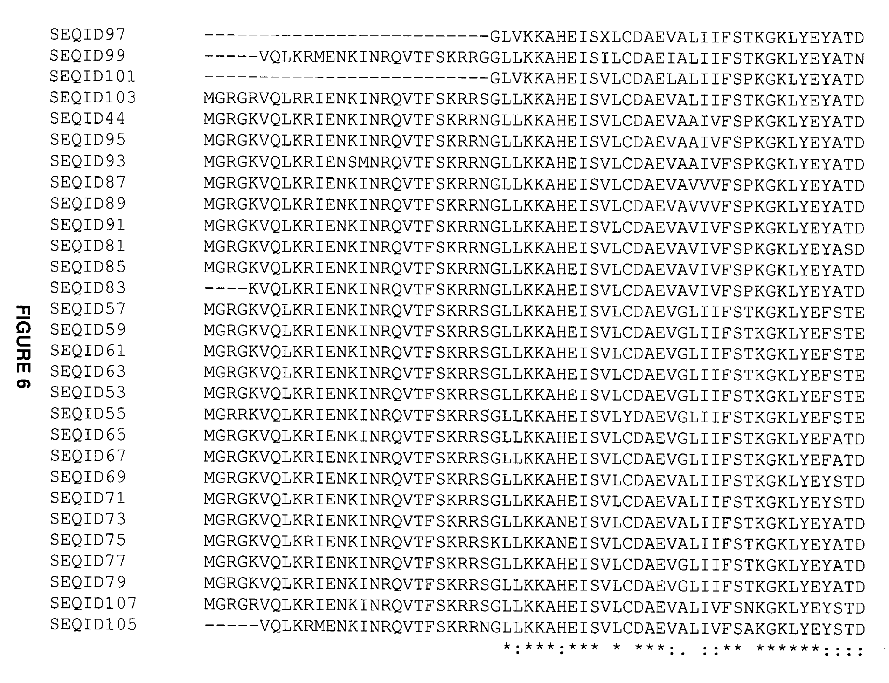

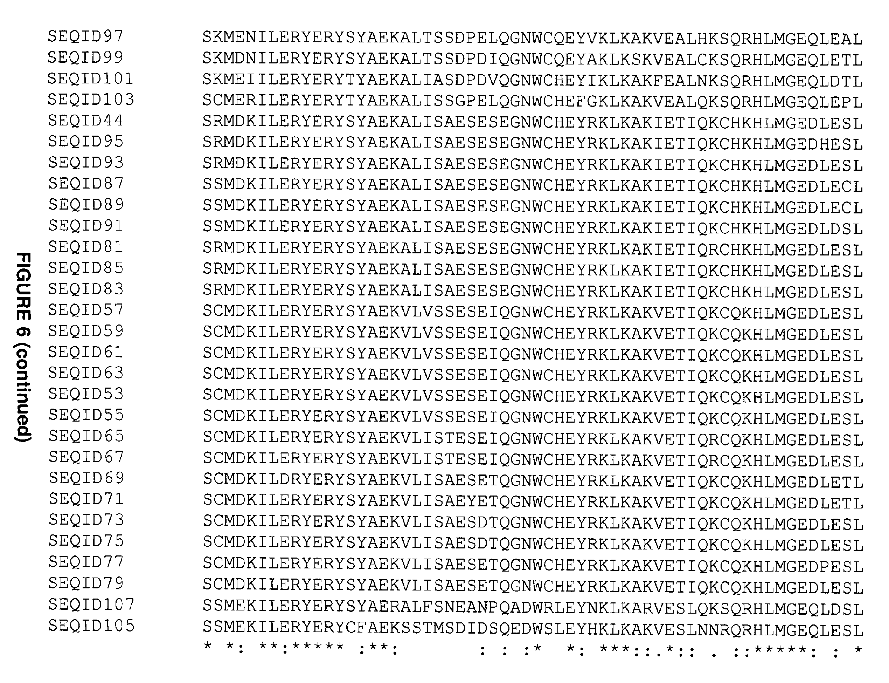

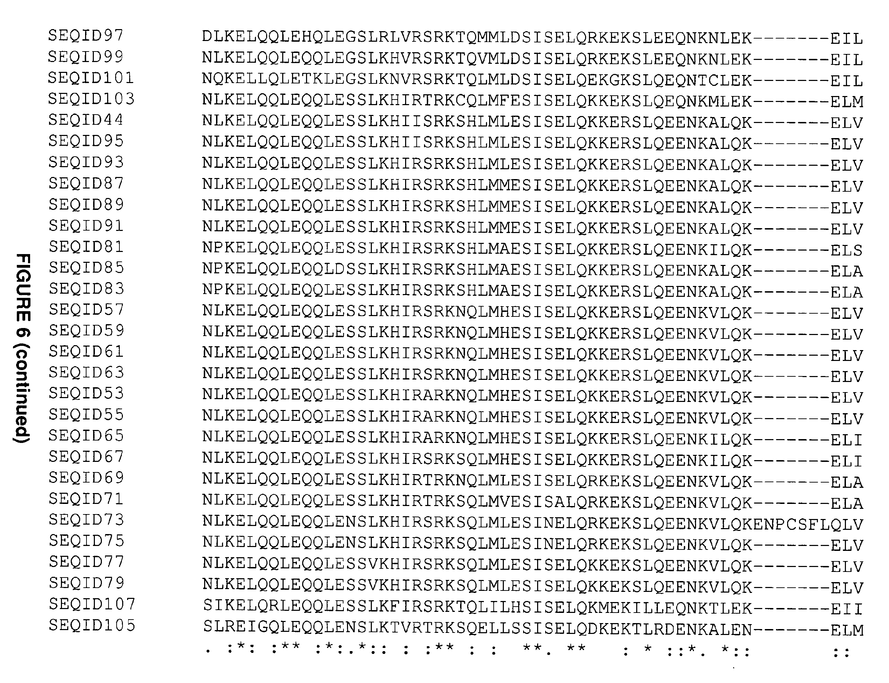

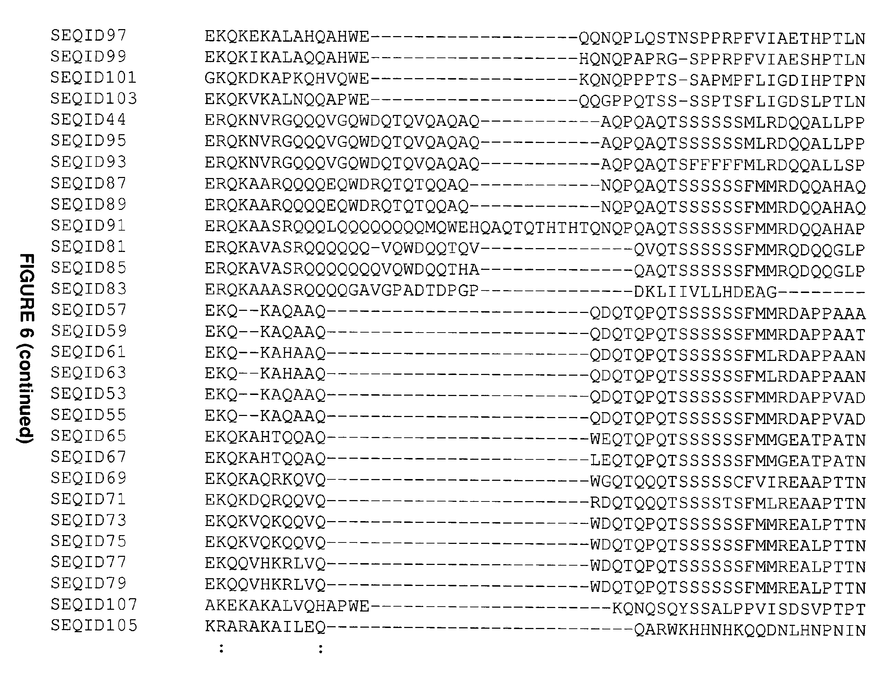

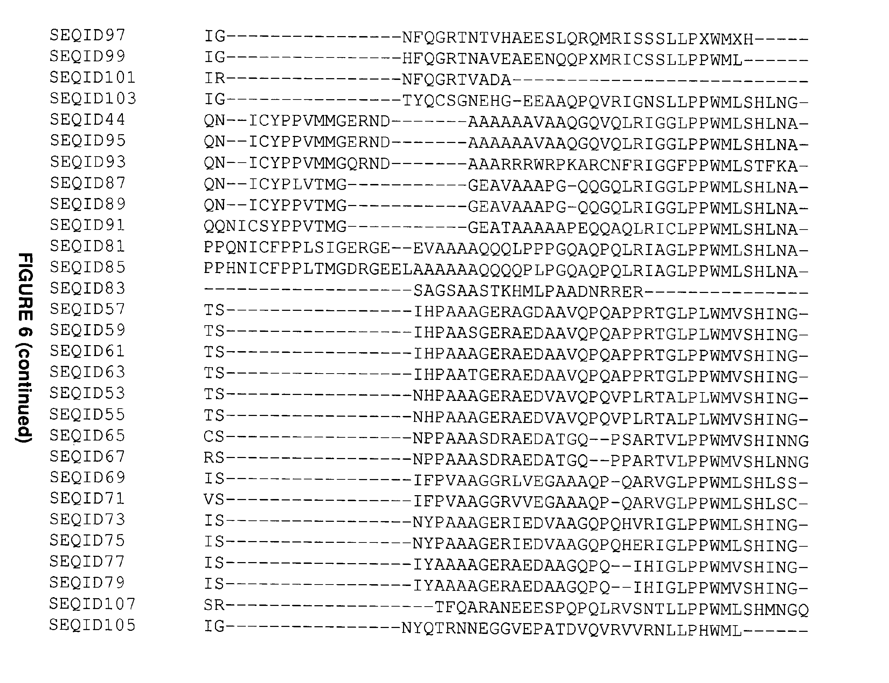

Hybridising sequences useful in the methods of the invention, encode a polypeptide having a MADS and/or keratin domain (see the alignment of Figure 6) and having substantially the same biological activity as the MADS15 protein represented by any of the amino acid sequences given in table D. The hybridising sequence is typically at least 400 consecutive nucleotides in length, preferably at least 600 consecutive nucleotides in length, more preferably at least 700 consecutive nucleotides in length and most preferably at least 800 consecutive nucleotides in length, the consecutive nucleotides being of any one of the nucleic acid sequences given in table D. Preferably, the hybridising sequence is one that is capable of hybridising to any of the nucleic acids given in table D, or to a portion of any of these sequences, a portion being as defined above. Most preferably, the hybridising sequence is capable of hybridising to a nucleic acid as represented by SEQ ID NO: 43 or to a portion thereof. Preferably, the hybridising sequence encodes an amino acid sequence comprising any one or more of the motifs or domains as defined herein.

According to the present invention, there is provided a method for enhancing yield-related traits in plants, comprising introducing and expressing in a plant a nucleic acid capable of hybridizing to any one of the nucleic acids given in table D, or comprising introducing and expressing in a plant a nucleic acid capable of hybridising to a nucleic acid encoding an orthologue, paralogue or homologue of any of the nucleic acid sequences given in table D.

Another nucleic acid variant useful in the methods of the invention is a splice variant encoding a MADS15 protein as defined hereinabove.

According to the present invention, there is provided a method for enhancing yield-related traits in plants, comprising introducing and expressing in a plant a splice variant of any one of the nucleic acid sequences given in table D, or a splice variant of a nucleic acid encoding an orthologue, paralogue or homologue of any of the amino acid sequences given in table D.

Preferred splice variants are splice variants of a nucleic acid represented by SEQ ID NO: 43 or a splice variant of a nucleic acid encoding an orthologue or paralogue of SEQ ID NO: 44. Preferably, the amino acid sequence encoded by the splice variant comprises any one or more of the motifs or domains as defined herein.

Another nucleic acid variant useful in performing the methods of the invention is an allelic variant of a nucleic acid encoding a MADS15 protein as defined hereinabove. The allelic variants useful in the methods of the present invention have substantially the same biological activity as the MADS15 protein of SEQ ID NO: 44.

According to the present invention, there is provided a method for enhancing yield-related traits in plants, comprising introducing and expressing in a plant an allelic variant of any one of the nucleic acids given in table D, or comprising introducing and expressing in a plant an allelic variant of a nucleic acid encoding an orthologue, paralogue or homologue of any of the amino acid sequences given in table D.

Preferably, the allelic variant is an allelic variant of SEQ ID NO: 43 or an allelic variant of a nucleic acid encoding an orthologue or paralogue of SEQ ID NO: 44. Preferably, the amino acid sequence encoded by the allelic variant comprises any one or more of the motifs or domains as defined herein.

A further nucleic acid variant useful in the methods of the invention is a nucleic acid variant obtained by gene shuffling. Gene shuffling or directed evolution may also be used to generate variants of nucleic acids encoding MADS15 proteins as defined above.

According to the present invention, there is provided a method for enhancing yield-related traits in plants, comprising introducing and expressing in a plant a variant of any one of the nucleic acid sequences given in table D, or comprising introducing and expressing in a plant a variant of a nucleic acid encoding an orthologue, paralogue or homologue of any of the amino acid sequences given in table D, which variant nucleic acid is obtained by gene shuffling.

Preferably, the variant nucleic acid obtained by gene shuffling encodes an amino acid sequence comprising any one or more of the motifs or domains as defined herein.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding MADS15 proteins may be derived from any natural or artificial source. The nucleic acid may be modified from its native form in composition and/or genomic environment through deliberate human manipulation. Preferably the MADS15-encoding nucleic acid is from a plant, further preferably from a monocotyledonous plant, more preferably from the Poaceae family, most preferably the nucleic acid is from *Oryza sativa.*

Any reference herein to a MADS15 protein is therefore taken to mean a MADS15 protein as defined above. Any nucleic acid encoding such a MADS15 protein is suitable for use in performing the methods of the invention.

The present invention also encompasses plants or parts thereof (including seeds) obtainable by the methods according to the present invention. The plants or parts thereof comprise a nucleic acid transgene encoding a MADS15 protein as defined above.

The invention also provides genetic constructs and vectors to facilitate introduction and/or expression of the nucleic acid sequences useful in the methods according to the invention, in a plant. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising
(a) nucleic acid encoding MADS15 protein as defined above;
(b) one or more control sequences capable of driving expression of the nucleic acid sequence of (a); and optionally
(c) a transcription termination sequence.

Plants are transformed with a vector comprising the sequence of interest (i.e., a nucleic acid encoding a MADS15 polypeptide as defined herein. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter may be used to drive expression of the nucleic acid sequence.

The promoter may be a constitutive promoter; alternatively, the promoter may be an inducible promoter, i.e. having induced or increased transcription initiation in response to a chemical or a stress-inducible promoter, or a pathogen-induced promoter.

Additionally or alternatively, the promoter may be an organ-specific or tissue-specific promoter, or the promoter may be a ubiquitous promoter, or the promoter may be developmentally regulated. Furthermore, the promoter may be organ-specific or tissue-specific or cell-specific.

Preferably, the *MADS15* nucleic acid or variant thereof is operably linked to a constitutive promoter. A preferred constitutive promoter is one that is also substantially ubiquitously expressed. Further preferably the promoter is derived from a plant, more preferably a monocotyledonous plant. Most preferred is use of a GOS2 promoter (for example from rice, SEQ ID NO: 47 or SEQ ID NO: 108). It should be clear that the applicability of the present invention is not restricted to the *MADS15* nucleic acid represented by SEQ ID NO: 43, nor is the applicability of the invention restricted to expression of a *MADS15* nucleic acid when driven by a GOS2 promoter. Examples of other constitutive promoters or functionally equivalent promters which may also be used to drive expression of a *MADS15* nucleic acid are shown in the definitions section.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

An intron sequence may also be added to the 5' untranslated region (UTR) or in the coding sequence to increase the amount of the mature message that accumulates in the cytosol.

Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene.

The invention also provides a method for the production of transgenic plants having enhanced yield-related traits relative to control plants, comprising introduction and expression in a plant of any nucleic acid encoding a MADS15 protein as defined hereinabove.

More specifically, the present invention provides a method for the production of transgenic plants having increased yield, which method comprises:
(i) introducing and expressing in a plant or plant cell a *MADS15* nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a MADS15 protein as defined hereinabove. Preferred host cells according to the invention are plant cells.

Host plants for the nucleic acids or the vector used in the method according to the invention, the expression cassette or construct or vector are, in principle, advantageously all plants, which are capable of synthesizing the polypeptides used in the inventive method.

The invention also extends to harvestable parts of a plant such as, but not limited to seeds, leaves, fruits, flowers, stems, rhizomes, roots, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression.

As mentioned above, a preferred method for modulating (preferably, increasing) expression of a nucleic acid encoding a MADS15 protein is by introducing and expressing in a plant a nucleic acid encoding a MADS15 protein; however the effects of performing the method, i.e. enhancing yield-related traits may also be achieved using other well known techniques. A description of some of these techniques will now follow.

One such technique is T-DNA activation tagging. The resulting transgenic plants show dominant phenotypes due to modified expression of genes close to the introduced promoter.

The effects of the invention may also be reproduced using the technique of TILLING (Targeted Induced Local Lesions In Genomes), or by homologous recombination.

Reference herein to enhanced yield-related traits is taken to mean an increase in biomass (weight) of one or more parts of a plant, which may include aboveground (harvestable) parts and/or (harvestable) parts below ground.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

In a particular embodiment, such harvestable parts are roots, and performance of the methods of the invention results in plants having increased root growth relative to the root growth of suitable control plants. Root development is an essential determinant of plant growth and crop yield since the root is the main channel to extract nutrients from the environment.

Increased root yield may for example manifest itself as one of more of the following: a) increased amount of root biomass (whereby a discrimination between thick roots and thin roots may be made by defining a certain treshold), b) increased average root diameter, c) increased total root biomass, and d) increased root biomass/shoot biomass ratio. For the purpose of this invention, it should be understood that the term 'root growth' encompasses all aspects of growth of the different parts that make up the root system at different stages of its development, both in monocotyledonous and dicotyledonous plants. It is to be understood that enhanced growth of the root can result from enhanced growth of one or more of its parts including the primary root, lateral roots, adventitious roots, etc. all of which fall within the scope of this invention.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a MADS15 protein as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a MADS15 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a MADS15 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

In a preferred embodiment of the invention, the increase in yield and/or growth rate occurs according to the methods of the present invention under non-stress conditions.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The present invention also encompasses use of nucleic acids encoding the MADS15 protein described herein and use of these MADS15 proteins in enhancing yield-related traits in plants.

Nucleic acids encoding the MADS15 protein described herein, or the MADS15 proteins themselves, may find use in breeding programmes in which a DNA marker is identified which may be genetically linked to a MADS15-encoding gene. The nucleic acids/genes, or the MADS15 proteins themselves may be used to define a molecular marker. This DNA or protein marker may then be used in breeding programmes to select plants having enhanced yield-related traits as defined hereinabove in the methods of the invention.

Allelic variants of a MADS15 protein-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding MADS15 proteins may also be used as probes for genetically and physically mapping the genes that they are a part of, and as markers for traits linked to those genes. Such information may be useful in plant breeding in order to develop lines with desired phenotypes. Such use of MADS15 protein-encoding nucleic acids requires only a nucleic acid sequence of at least 15 nucleotides in length. The MADS15 protein-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the MADS15 protein-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the MADS15 protein-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Detailed description of MADS15 down

It has now surprisingly been found that decreasing the level and/or activity of an endogenous MADS15 polypeptide gives plants having enhanced yield-related traits, in particular increased yield relative, to corresponding wild type plants. The present invention therefore provides methods for enhancing yield related traits, particularly for increasing yield of a plant relative to control plants, comprising decreasing the level and/or activity of an endogenous MADS15 polypeptide. Reference herein to "control plants" is taken to mean corresponding wild type plants in which there is no reduction in activity of the endogenous MADS15 polypeptide.

Advantageously, performance of the methods according to the present invention results in plants having increased yield, particularly increased seed yield and/or increased biomass, relative to corresponding wild type plants.

The term "increased yield" as defined herein is taken to mean an increase in biomass (weight) of one or more parts of a plant, which may include aboveground (harvestable) parts and/or (harvestable) parts below ground.

In particular, such harvestable parts include vegetative biomass and/or seeds, and performance of the methods of the invention results in plants having increased yield (in vegetative biomass and/or seed) relative to the yield of control plants.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The increase in seed yield may also be manifested as an increase in seed size and/or seed volume. This may increase the amount, or change the composition of, substances in the seed, such as oils, proteins and carbohydrates.

According to a preferred feature, performance of the methods of the invention result in plants having increased yield, particularly increased biomass and/or seed yield. Therefore, according to the present invention, there is provided a method for increasing plant yield, which method comprises decreasing the level of activity of a MADS15 polypeptide or a homologue thereof, preferably by downregulating expression of a *MADS15* gene or a homologue thereof.

Since the transgenic plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, flowering time and speed of seed maturation. An increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate. Therefore, according to the present invention, there is provided a method for increasing the growth rate of plants relative to control plants, which method comprises preferentially reducing the expression level and/or activity of an endogenous *MADS15* gene in a plant.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises reducing expression of an endogenous MADS15 gene in a plant.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises decreasing expression in a plant of a nucleic acid encoding a MADS15 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

In a preferred embodiment of the invention, the increase in yield and/or growth rate occurs according to the methods of the present invention under non-stress conditions.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats. Reference herein to a "decrease" in level of an endogenous MADS15 protein in a plant is taken to mean a reduction in protein concentration or substantial elimination of an endogenous MADS15 protein relative to endogenous MADS15 protein levels found in corresponding wild type plants. This reduction or substantial elimination may result in reduced or substantially abolished MADS15 protein activity in a plant.

Reference herein to a "decrease" in activity of an endogenous MADS15 protein in a plant is taken to mean a reduction in MADS15 protein activity or substantial elimination of activity of an endogenous MADS15 protein relative to endogenous MADS15 protein activity levels found wild type plants.

Preferably, the reduction in endogenous MADS15 protein level and/or activity is obtained by downregulating the expression of the endogenous *MADS15* gene.

Reference herein to an "endogenous" *MADS15* gene not only refers to *MADS15* genes as found in a plant in its natural form (i.e., without there being any human intervention), but also refers to isolated *MADS15* genes subsequently introduced into a plant. For example, a transgenic plant containing a *MADS15* transgene may encounter a reduction or substantial elimination of the *MADS15* transgene expression and/or reduced or substantial elimination of expression of an endogenous *MADS15* gene.

This reduction (or substantial elimination) of endogenous *MADS15* gene expression may be achieved using any one or more of several well-known gene silencing methods. "Gene silencing" or "downregulation" of expression, as used herein, refers to a reduction or the substantial elimination of *MADS15* gene expression and/or MADS15 polypeptide levels and/or MADS 15 polypeptide activity.

One such method for reduction or substantial elimination of endogenous *MADS15* gene expression is RNA-mediated downregulation of gene expression (RNA silencing). Silencing in this case is triggered in a plant by a double stranded RNA molecule (dsRNA) that is substantially homologous to a target *MADS15* gene. This dsRNA is further processed by the plant into about 21 to about 26 nucleotides called short interfering RNAs (siRNAs). The siRNAs are incorporated into an RNA-induced silencing complex (RISC) that cleaves the mRNA of a *MADS15* target gene, thereby reducing or substantially eliminating the number of *MADS15* mRNAs to be translated into a MADS15 protein.

One example of an RNA silencing method involves the introduction of coding sequences or parts thereof in a sense orientation into a plant. The additional gene, or part thereof, will silence an endogenous *MADS15* gene, giving rise to a phenomenon known as co-suppression. The reduction of *MADS15* gene expression will be more pronounced if several additional copies are introduced into the plant, as there is a positive correlation between high transcript levels and the triggering of co-suppression.

Another example of an RNA silencing method involves the use of antisense *MADS15* nucleic acid sequences.

The antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection). Preferably, production of antisense nucleic acids in plants occurs by means of a stably integrated transgene comprising a promoter operative for constitutive expression in plants, an antisense oligonucleotide, and a terminator.

A preferred method for reduction or substantial elimination of endogenous *MADS15* gene expression via RNA silencing is by using an expression vector into which a *MADS15* gene or fragment thereof has been cloned as an inverted repeat (in part or completely), separated by a spacer (non-coding DNA).

In still another embodiment, the reduction or substantial elimination of endogenous *MADS15* exprssion may be obtained by using ribozymes. For example, a derivative of a Tetrahymena L-19 IVS RNA can be constructed in which the nucleotide sequence of the active site is complementary to the nucleotide sequence to be cleaved in an MADS15-encoding mRNA.

Gene silencing may also be achieved by insertion mutagenesis or if there is a mutation on the endogenous *MADS15* gene and/or a mutation on an isolated *MADS15* gene subsequently introduced into a plant. The reduction or substantial elimination of MADS15 protein activity may be caused by a non-functional MADS15. For example, MADS15 binds to various interacting proteins; one or more mutation(s) and/or truncation(s) within the MADS box of a MADS15 may therefore provide for a MADS15 protein that is still able to bind interacting proteins but that cannot exhibit its normal function as transcription factor.

A further approach to gene silencing is by targeting nucleotide sequences complementary to the regulatory region of the *MADS15* gene (e.g., the *MADS15* promoter and/or enhancers) to form triple helical structures that prevent transcription of the *MADS15* gene in target cells.

Still another approach to gene silencing is described by Hiratsu et al. (Plant J. 34, 733-739, 2003). This method does not depend on sequence homology to the targeted gene but involves the use of a repression sequence domain in transcriptional gene fusions, and has been used to modify traits of agronomic interest (Fujita et al., Plant Cell 17, 3470-3488, 2005 and Mitsuda at al., Plant Cell 17, 2993-3006, 2005). Typically, a nucleotide chimeric fusion is made between a gene encoding a protein capable of positively influencing the expression of the targeted gene (such as a transcription activator), and a nucleotide fragment encoding a repression domain. Upon expression of the chimeric gene fusion, the expression of the targeted gene is repressed, usually in a dominant negative fashion. Repression domains are well known in the art, for example the EAR motif present in some AP2 and Zinc finger transcription factor. Methods based on repression domains are well suited to overcome gene redundancy for the targeted gene in the plant species of choice.

Described above are examples of various methods for gene silencing (for the reduction or substantial elimination of endogenous *MADS15* gene expression. The methods of the invention rely on the reduction of expression of an endogenous *MADS15* gene in a plant. A person skilled in the art would readily be able to adapt the aforementioned methods for silencing so as to achieve gene silencing in a whole plant or in parts thereof through the use of an appropriate promoter, for example.

It should be noted that the essence of the present invention resides in the advantageous and surprising results found upon reduction or substantial elimination of endogenous *MADS15* gene expression in a plant, and is not limited to any particular method for such reduction or substantial elimination of endogenous MADS15 protein activity. The activity of a MADS15 polypeptide may also be decreased or eliminated by introducing a genetic modification (preferably in the locus of a *MADS15* gene). The locus of a gene as defined herein is taken to mean a genomic region, which includes the gene of interest and 10 kb up- or down stream of the coding region.

The genetic modification may be introduced, for example, by any one (or more) of the following methods: T-DNA inactivation, TILLING, site-directed mutagenesis, directed evolution, homologous recombination. Following introduction of the genetic modification, there follows a step of selecting for decreased activity of a MADS15 polypeptide, which decrease in activity gives plants having increased yield.

T-DNA inactivation tagging involves insertion of a T-DNA, in the genomic region of the gene of interest or 10 kb up- or downstream of the coding region of a gene in a configuration such that the T-DNA inhibits expression of the targeted gene. Typically, regulation of expression of the targeted gene by its natural promoter is disrupted. The T-DNA is randomly inserted into the plant genome, for example, through *Agrobacterium* infection and leads to downregulated expression of genes near the inserted T-DNA. The resulting transgenic plants show phenotypes due to inhibited expression of genes close to the introduced T-DNA.

A genetic modification may also be introduced in the locus of a *MADS15* gene using the technique of TILLING (Targeted Induced Local Lesions In Genomes).

Site-directed mutagenesis and random mutagenesis may be used to generate variants of *MADS15* nucleic acids. Several methods are available to achieve site-directed mutagenesis, the most common being PCR based methods (current protocols in molecular biology. Wiley Eds.).

Directed evolution may also be used to generate variants of *MADS15* nucleic acids. This consists of iterations of DNA shuffling followed by appropriate screening and/or selection to generate variants of *MADS15* nucleic acids or variants thereof encoding MADS15 polypeptides having a modified (here decreased or abolished) biological activity (Castle et al., (2004) Science 304(5674): 1151-4; US patents 5,811,238 and 6,395,547).

T-DNA activation, TILLING, site-directed mutagenesis and directed evolution are examples of technologies that enable the generation of novel alleles and *MADS15* variants.

The effects of the invention may also be reproduced using homologous recombination. The nucleic acid to be targeted may be an allele encoding an inactive protein or a protein with decreased activity, used to replace the endogenous gene or may be introduced in addition to the endogenous gene, and needs to be targeted to the locus of the MADS15 gene.

Other methods, such as the use of antibodies directed to the endogenous MADS15 for inhibiting its function *in planta,* or interference in the signalling pathway in which MADS15 is involved, will be well known to the skilled man. Alternatively, a screening program may be set up to identify natural variants of a MADS15 gene, which variants have reduced MADS15 activity, or no MADS15 activity at all. Such natural variants may also be used in the methods of the present invention.

For optimal performance, the gene silencing techniques used for the reduction or substantial elimination of endogenous *MADS15* gene expression requires the use of *MADS15* nucleic acid sequences from monocotyledonous plants for transformation into monocotyledonous plants. Preferably, a *MADS15* nucleic acid from any given plant species is introduced into that same species. For example, a *MADS15* nucleic acid from rice (be it a full length *MADS15* sequence or a fragment) is transformed into a rice plant. The *MADS15* nucleic acid need not be introduced into the same plant variety.

Reference herein to a *"MADS15* gene" or a *"MADS15* nucleic acid" is taken to mean a polymeric form of a deoxyribonucleotide or a ribonucleotide polymer of any length, either double- or single-stranded, or analogues thereof, that have the essential characteristic of a natural ribonucleotide in that they can hybridise to nucleic acids in a manner similar to naturally occurring polynucleotides. A "*MADS15* gene" or a "*MADS15* nucleic acid" refers to a sufficient length of substantially contiguous nucleotides of a MADS15-encoding gene to perform gene silencing; this may be as little as 20 or fewer nucleotides. A gene encoding a (functional) protein is not a requirement for the various methods discussed above for the reduction or substantial elimination of expression of an endogenous *MADS15* gene.

The methods of the invention may be performed using a sufficient length of substantially contiguous nucleotides of a *MADS15* gene/nucleic acid, which may consist of 20 or fewer nucleotides, which may be from any part of the *MADS15* gene/nucleic acid, such as the 5' end of the coding region that is well conserved amongst the *MADS15* gene family, or encoding one of the conserved motifs described below.

*MADS15* genes are well known in the art and useful in the methods of the invention are substantially contiguous nucleotides of the plant *MADS15* genes/nucleic acid described in Moon et al. (Plant Physiol. 120, 1193-1204, 1999).

Other *MADS15* gene/nucleic acid sequences may also be used in the methods of the invention, and may readily be identified by a person skilled in the art. MADS15 polypeptides may be identified by the presence of one or more of several well-known features (see below). Upon identification of a MADS15 polypeptide, a person skilled in the art could easily derive, using routine techniques, the corresponding encoding nucleic acid sequence and use a sufficient length of contiguous nucleotides of the same to perform any one or more of the gene silencing methods described above.

The term "MADS15 polypeptide or homologue thereof" as defined herein refers to a protein that falls in the group of FUL-like MADS box proteins as delineated by Adam et al.(J. Mol. Evol. 62, 15-31). FUL-like MADS box proteins are part of the SQUAMOSA subfamily and have the typical MIKC^{c} architecture: a MADS domain (M) at the N-terminus, followed by an intervening domain (I) involved in dimerisation, a highly conserved keratin domain (K) and a variable domain (C) at the C-terminus, which is responsible for binding of interacting proteins or transcriptional activation (De Bodt et al. Trends in Plant Science 8, 475-483).

Plant MADS15 polypeptides may also be identified by the presence of certain conserved motifs. The presence of these conserved motifs may be identified using methods for the alignment of sequences for comparison as described hereinabove. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified. Upon identification of a MADS15 polypeptide by the presence of these motifs, a person skilled in the art may easily derive the corresponding nucleic acid encoding the polypeptide comprising the relevant motifs, and use a sufficient length of contiguous nucleotides of the same to perform any one or more of the gene silencing methods described above (for the reduction or substantial elimination of an endogenous *MADS15* gene expression).

Typically, the presence of at least one of the motifs 1 to 4 should be sufficient to identify any query sequence as a MADS15, however the presence of at least motifs 1 and 2 is preferred. The consensus sequence provided is based on the monocot sequences given in the sequence listing. A person skilled in the art would be well aware that the consensus sequence may vary somewhat if further or different sequences (for example from dicot sequences) were used for comparison.

Motif 1, located in the MADS domain (SEQ ID NO: 114): 
L(L/V)KKA(H/N)EIS(VI)L(C/Y)DAE(V/I/L) (A/G) (L/A/V) (I/V) (I/V)FS(T/P/A/N
)KGKLYE(Y/F) (A/S) (T/S) (D/N/E)S(K/C/R/S)M(D/E) (N/I/R/K)IL(E/D)R.
 Preferably, this conserved sequence motif 1 has the sequence:
LLKKAHEISVLCDAEVA (L/A/V) I (I/V) FS (T/P) KGKLYEYATDS (C/R) M (D/E) (R/K) ILER,
   most preferably the conserved sequence motif 1 has the sequence:
   LLKKAHEISVLCDAEVAAIVFSPKGKLYEYATDSRMDKILER
Motif 2, located in the K domain (SEQ ID NO: 115):
KLK (A/S) (K/R) (V/I) E (A/T/S) (L/I) (Q/N) (K/R/N) (S/C/R) (Q/H) (R/K) HLMGE
   Preferably, this conserved sequence motif 2 has the sequence:
KLKAK (V/I) E (A/T) (L/I) QK (S/C) (Q/H) (R/K) HLMGE
   More preferably, this conserved sequence motif 2 has the sequence:
KLKAKIETIQKCHKHLMGE

Optionally, the MADS15 polypeptide or its homologue may comprise in the C domain motif 3 and/or motif 4:
motif 3 (SEQ ID NO: 116):
   Q (P/Q/V/A) QTS (S/F) (S/F) (S/F) (S/F) (S/C/F) (F/M)
motif 4 (SEQ ID NO: 117):
   (G/A/V/L) (L/P) XWMX (S/H)
   wherein the first X residue in motif 4 may be any amino acid, but preferably L, P or H; and wherein the second X residue may be any amino acid, but preferably V or L.

Alternatively, the C-domain (starting behind the keratin domain, i.e. at R175 in SEQ ID NO: 110) may be characterised by the increased occurrence of glutamine (normally 3,93%, here at least 8,77% with a maximum of 26,73%), in addition, the content in alanine, proline, and/or serine may also be increased (above 7,8%, 4,85% and 6,89% respectively).

Alternatively formulated, a preferred MADS15 protein useful in the methods of the present invention comprises at least an N-terminal MADS domain corresponding to the SMART domain SM00432 (Pfam PF00319) followed by a Keratin domain corresponding to the K-box region defined in Pfam as PF01486, more preferably, the MADS15 protein has in its MADS domain the conserved signature of SEQ ID NO: 114 and in its K-domain the conserved signature of SEQ ID NO: 115, most preferably, the MADS15 protein has the sequence given in SEQ ID NO: 110.

Homologues, as defined above, may readily be identified using routine techniques well known in the art, such as by sequence alignment; homologues of OsMADS15 may have been named differently in various plant species, therefore the gene/protein names should not be used for identifying orthologues or paralogues. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologous sequences may readily be identified using, for example, the ClustalW multiple sequence alignment algorithm (version 1.83), with the default pairwise alignment parameters, and a scoring method in percentage. Minor manual editing may be performed to optimise alignment between conserved motifs (see below), as would be apparent to a person skilled in the art.

The various structural domains in a MADS15 protein may be identified using specialised databases e.g. SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318;), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)).

Furthermore, a MADS15 protein may also be identifiable by its ability to bind DNA and to interact with other proteins. DNA-binding activity and protein-protein interactions may readily be determined in vitro or in vivo using techniques well known in the art. Examples of in vitro assays for DNA binding activity include: gel retardation analysis using known MADS-box DNA binding domains (West et al. (1998) Nucl Acid Res 26(23): 5277-87), or yeast one-hybrid assays. An example of an in vitro assay for protein-protein interactions is the yeast two-hybrid analysis (Fields and Song (1989) Nature 340:245-6). Proteins known to interact with OsMADS15 include other MADS proteins (such as those involved in the flowering signalling complex), receptor like kinases such a Clavata1 and Clavata2, Erecta, BRI1 or RSK.

Therefore upon identification of a MADS15 polypeptide using one or several of the features described above, a person skilled in the art may easily derive the corresponding nucleic acid encoding the polypeptide, and use a sufficient length of substantially contiguous nucleotides of the same to perform any one or more of the gene silencing methods described above (for the reduction or substantial elimination of an endogenous *MADS15* gene expression).

Preferred for use in the methods of the invention is a sufficient length of substantially contiguous nucleotides of SEQ ID NO: 109 (*OsMADS15*), or the use of a sufficient length of substantially contiguous nucleotides of a nucleic acid sequence encoding an orthologue or paralogue of OsMADS15 (SEQ ID NO: 109). Examples of such orthologues and paralogues of OsMADS15 are provided in Table D below. Close homologues of OsMADS15 are the proteins represented by SEQ ID NO: 147, 149, 151, 153, 155, 157, 159, 161, 163, 165, 167, 169, 171 and 173.

Orthologues in, for example, monocot plant species may easily be found by performing a so-called reciprocal blast search. This may be done by a first blast involving blasting a query sequence (for example, SEQ ID NO: 109 or SEQ ID NO: 110) against any sequence database, such as the publicly available NCBI database. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 109 or SEQ ID NO: 110 the second blast would therefore be against rice sequences). The results of the first and second BLASTs are then compared. A paralogue is identified if a high-ranking hit from the first blast is from the same species as from which the query sequence is derived; an orthologue is identified if a high-ranking hit is not from the same species as from which the query sequence is derived. High-ranking hits are those having a low E-value. The lower the E-value, the more significant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues.

The source of the substantially contiguous nucleotides of a *MADS15* gene/nucleic acid may be any plant source or artificial source. For optimal performance, the gene silencing techniques used for the reduction or substantial elimination of endogenous *MADS15* gene expression requires the use of *MADS15* sequences from monocotyledonous plants for transformation into monocotyledonous plants. Preferably, *MADS15* sequences from the family Poaceae are transformed into plants of the family Poaceae. Further preferably, a *MADS15* nucleic acid from rice (be it a full length *MADS15* sequence or a fragment) is transformed into a rice plant. The *MADS15* nucleic acid need not be introduced into the same plant variety. Most preferably, the *MADS15* nucleic acid from rice is a sufficient length of substantially contiguous nucleotides of SEQ ID NO: 109 (*OsMADS15*) or a sufficient length of substantially contiguous nucleotides of a nucleic acid sequence encoding an orthologue or paralogue of OsMADS15 (SEQ ID NO: 110). As mentioned above, a person skilled in the art would be well aware of what would constitute a sufficient length of substantially contiguous nucleotides to perform any of the gene silencing methods defined hereinabove, this may be as little as 20 or fewer substantially contiguous nucleotides in some cases.

The invention also provides genetic constructs and vectors to facilitate introduction and/or expression of the nucleotide sequences useful in the methods according to the invention.

Therefore, there is provided a gene construct comprising one or more control sequences capable of driving expression of a sense and/or antisense *MADS15* nucleic acid sequence in a plant so as to silence an endogenous *MADS15* gene in the plant; and optionally a transcription termination sequence. Preferably, the control sequence is a constitutive and ubiquitous promoter.

A preferred construct for gene silencing is one comprising an inverted repeat of a *MADS15* gene or fragment thereof, preferably capable of forming a hairpin structure, which inverted repeat is under the control of a constitutive promoter.

Therefore, the invention provides a construct comprising:
(a) a *MADS15* nucleic acid capable of forming a hairpin structure;
(b) one or more control sequences capable of driving expression of the nucleic acid sequence of (a); and optionally
(c) a transcription termination sequence.

Constructs useful in the methods according to the present invention may be created using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

The sequence of interest is operably linked to one or more control sequences (at least to a promoter) capable of increasing expression in a plant.

Advantageously, any type of promoter may be used to drive expression of the nucleic acid sequence. The promoter may be an inducible promoter, i.e. having induced or increased transcription initiation in response to a developmental, chemical, environmental or physical stimulus. Additionally or alternatively, the promoter may be a tissue-preferred or cell-preferred promoter, i.e. one that is capable of preferentially initiating transcription in certain tissues, such as the leaves, roots, seed tissue etc, or even in specific cells. Promoters able to initiate transcription in certain tissues or cells only are referred to herein as "tissue-specific", respectively "cell-specific".

Preferably, the *MADS15* nucleic acid or functional variant thereof is operably linked to a constitutive promoter. Preferably the promoter is a ubiquitous promoter and is expressed predominantly throughout the plant. Preferably, the constitutive promoter capable of preferentially expressing the nucleic acid throughout the plant has a comparable expression profile to a GOS2 promoter. More preferably, the constitutive promoter has the same expression profile as the rice GOS2 promoter, most preferably, the promoter capable of preferentially expressing the nucleic acid throughout the plant is the GOS2 promoter from rice (SEQ ID NO: 113 or SEQ ID NO: 174). It should be clear that the applicability of the present invention is not restricted to the *MADS15* nucleic acid represented by SEQ ID NO: 109, nor is the applicability of the invention restricted to expression of a *MADS15* nucleic acid when driven by a GOS2 promoter. An alternative constitutive promoter that is useful in the methods of the present invention is the high mobility group protein promoter (PRO0170, SEQ ID NO: 40 in WO2004070039). Examples of other constitutive promoters that may also be used to drive expression of a *MADS15* nucleic acid are shown in the definitions section.

Optionally, one or more terminator sequences may also be used in the construct introduced into a plant. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1. The genetic construct may optionally comprise a selectable marker gene.

The present invention also encompasses plants including plant parts obtainable by the methods according to the present invention having increased yield relative to control plants and which have reduced or substantially eliminated expression of an endogenous *MADS15* gene.

The invention furthermore provides a method for the production of transgenic plants having increased yield relative to control plants, which transgenic plants have reduced or substantially eliminated expression of an endogenous *MADS15* gene.

More specifically, the present invention provides a method for the production of transgenic plants having increased seed yield which method comprises:
- introducing and expressing in a plant, plant part or plant cell a gene construct comprising one or more control sequences capable of preferentially driving expression of an inverted repeat *MADS15* nucleic acid sequence in a plant so as to silence an endogenous *MADS15* gene in the plant; and
- cultivating the plant, plant part or plant cell under conditions promoting plant growth and development.

Preferably, the construct introduced into a plant is one comprising an inverted repeat (in part or complete) of a *MADS15* gene or fragment thereof, preferably capable of forming a hairpin structure.

According to a preferred feature of the present invention, the construct is introduced into a plant by transformation.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also extends to harvestable parts of a plant such as seeds and products derived, preferably directly derived, from a harvestable part of such a plant, such as dry pellets or powders, oil, fat and fatty acids, starch or proteins.

The present invention also encompasses use of *MADS15* nucleic acids for the reduction or substantial elimination of endogenous *MADS15* gene expression in a plant for increasing plant seed yield as defined hereinabove.

Nucleic acids encoding the MADS15 protein described herein, or the MADS15 proteins themselves, may find use in breeding programmes in which a DNA marker is identified which may be genetically linked to a MADS15-encoding gene. The nucleic acids/genes, or the MADS15 proteins themselves may be used to define a molecular marker. This DNA or protein marker may then be used in breeding programmes to select plants having enhanced yield-related traits as defined hereinabove in the methods of the invention.

Allelic variants of a MADS15 protein-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features. Nucleic acids encoding MADS15 proteins may also be used as probes for genetically and physically mapping the genes that they are a part of, and as markers for traits linked to those genes. Such information may be useful in plant breeding in order to develop lines with desired phenotypes. Such use of MADS15 protein-encoding nucleic acids requires only a nucleic acid sequence of at least 15 nucleotides in length. The MADS15 protein-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the MADS15 protein-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the MADS15 protein-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Detailed description of PLT

Surprisingly, it has now been found that increasing expression of a nucleic acid encoding a PLT transcription factor polypeptide gives plants having enhanced yield-related traits, in particular increased yield relative to control plants.

According to the present invention, there is provided a method for increasing plant yield relative to control plants, comprising increasing expression in a plant of a nucleic acid encoding a PLT transcription factor polypeptide.

The term "increased yield" as defined herein is taken to mean an increase in biomass (weight) of one or more parts of a plant, which may include aboveground (harvestable) parts and/or (harvestable) parts below ground.

In particular, such harvestable parts are seeds, and performance of the methods of the invention results in plants having increased seed yield relative to the seed yield of control plants.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

According to a preferred feature of the present invention, performance of the methods of the invention gives plants having increased seed yield relative to control plants. Therefore according to the present invention, there is provided a method for increasing seed yield in plants relative to the seed yield of control plants, the method comprising increasing expression in a plant of a nucleic acid encoding a PLT transcription factor polypeptide.

Since the transgenic plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour (increased seedling vigor at emergence). The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate. Therefore, according to the present invention, there is provided a method for increasing the growth rate of plants, which method comprises increasing expression in a plant of a nucleic acid encoding a PLT transcription factor polypeptide.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions enhanced yield-related traits relative to control plants grown under comparable conditions. Therefore, according to the present invention, there is provided a method for enhancing yield-related traits , in particular for increasing yield, in plants grown under non-stress conditions or under mild drought conditions, which method comprises increasing expression in a plant of a nucleic acid encoding a PLT transcription factor polypeptide.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Examples of typical crop plants grown for oil production include soybean, sunflower, cotton, canola, peanuts or palm. Examples of typical crop plants grown for starch production include rice, wheat, barley, corn or potato. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The term "PLT transcription factor polypeptide" as defined herein refers to any polypeptide comprising in increasing order of preference at least 70%, 75%, 80%, 85%, 90%, 95%, 96%, 97%, 98% or 99% sequence identity to the AP2 domain represented by SEQ ID NO: 191.

Preferably the PLT transcription factor polypeptide of choice is a PLT transcription factor polypeptide that in its natural genetic environment is essentially expressed in the roots (below ground parts) of the plant.

Further preferably, the PLT transcription factor polypeptide comprises either one motif but preferably both of motif1 as represented by SEQ ID NO: 192 PK(V/L)(A/E)DFLG and motif 2 as represented by SEQ ID NO: 209: (V/L)FX(M/V)WN(D/E), wherein X may be any amino acid; preferably motif 2 has the sequence of SEQ ID NO: 193 (V/L)F(T/S/N)(M/V)WN(D/E).

Examples of PLT transcription factor polypeptides comprising (i) in increasing order of preference at least 70%, 75%, 80%, 85%, 90%, 95%, 96%, 97%, 98% or 99% sequence identity to the AP2 domain represented by SEQ ID NO: 191 are proteins represented by the sequences given in Table I in the examples section. Preferred examples are represented by SEQ ID NO: 176 and SEQ ID NO: 178.

The AP2 domain in a PLT transcription factor polypeptide may be identified using specialised databases e.g. SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)). The AP2 domain of a PLT transcription factor comprises two repeats R1 and R2, each of about 68 amino acids and separated by a linker region (Figure 13).

The AP2 domain as represented by SEQ ID NO: 191 may be identified using methods for the alignment of sequences for comparison as described hereinabove. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified, including motif1 as represented by SEQ ID NO: 192 and motif 2 as represented by SEQ ID NO: 209, preferably as represented by SEQ ID NO: 193.

Homologues of a PLT transcription factor polypeptide may also be used to perform the methods of invention. Homologues (or homologous proteins) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Minor manual editing may be performed to optimise alignment between conserved motifs, as would be apparent to a person skilled in the art.

Homologues also include orthologues and paralogues. Orthologues and paralogues may easily be found by performing a so-called reciprocal blast search. This may be done by a first BLAST involving BLASTing a query sequence (for example, SEQ ID NO: 175, SEQ ID NO: 176, SEQ ID NO: 177 or SEQ ID NO: 178) against any sequence database, such as the publicly available NCBI database. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 175, SEQ ID NO: 176, SEQ ID NO: 177 or SEQ ID NO: 178, the second BLAST would therefore be against *Arabidopsis thaliana* sequences). The results of the first and second BLASTs are then compared. A paralogue is identified if a high-ranking hit from the first BLAST is from the same species as from which the query sequence is derived; an orthologue is identified if a high-ranking hit is not from the same species as from which the query sequence is derived. High-ranking hits are those having a low E-value. The lower the E-value, the more significant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues. In addition to E-values, comparisons are also scored by percentage identity. Percentage identity refers to the number of identical nucleotides (or amino acids) between the two compared nucleic acid (or polypeptide) sequences over a particular length. PLT transcription factor polypeptide homologues have in increasing order of preference at least 25%, 30%, 40%, 50%, 60%, 70%, 80%, 85%, 90%, 95%, 96%, 97%, 98% or 99% or more sequence identity or similarity (functional identity) to an unmodified PLT transcription factor polypeptide as represented by SEQ ID NO: 176 or SEQ ID NO: 178. Percentage identity between PLT transcription factor polypeptide homologues outside of the AP2 domain is reputedly low. Preferably, PLT transcription factor polypeptide homologues comprise an AP2 domain with in increasing order of preference at least 70%, 75%, 80%, 85%, 90%, 95%, 96%, 97%, 98% or 99% sequence identity to the AP2 domain represented by SEQ ID NO: 191. Further preferably, PLT transcription factor polypeptide homologues are as represented by SEQ ID NO: 180, SEQ ID NO: 182, SEQ ID NO: 184, SEQ ID NO: 186, SEQ ID NO: 188, SEQ ID NO: 200 and SEQ ID NO: 202.

The PLT polypeptide may be a derivative of SEQ ID NO: 176 or SEQ ID NO: 178. "Derivatives" include peptides, oligopeptides, polypeptides which may, compared to the amino acid sequence of the naturally-occurring form of the protein, such as the one presented in SEQ ID NO: 176 or SEQ ID NO: 178, comprise substitutions of amino acids with non-naturally occurring amino acid residues, or additions of non-naturally occurring amino acid residues. Derivatives of SEQ ID NO: 180, SEQ ID NO: 182, SEQ ID NO: 184, SEQ ID NO: 186, SEQ ID NO: 188, SEQ ID NO: 200 and SEQ ID NO: 202 are further examples which may be suitable for use in the methods of the invention provided that they have the same or similar biological activity.

Furthermore, PLT transcription factor polypeptides (at least in their native form) typically have DNA-binding activity and an activation domain. A person skilled in the art may easily determine the presence of an activation domain and DNA-binding activity using routine techniques and procedures. Proteins interacting with PLT transcription factor polypeptides (as, for example, in transcriptional complexes) may easily be identified using standard techniques for a person skilled in the art.

Examples of nucleic acids encoding PLT transcription factor polypeptides include but are not limited to those represented by any one of: SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 199 and SEQ ID NO: 201. Variants of nucleic acids encoding PLT transcription factor polypeptides may be suitable for use in the methods of the invention provided that they have the same or similar biological activity. Suitable variants include portions of nucleic acids encoding PLT transcription factor polypeptides and/or nucleic acids capable of hybridising with nucleic acids/genes encoding PLT transcription factor polypeptides. Further variants include splice variants and allelic variants of nucleic acids encoding PLT transcription factor polypeptides. The term "portion" as defined herein refers to a piece of DNA encoding a polypeptide comprising in increasing order of preference at least 70%, 75%, 80%, 85%, 90%, 95%, 96%, 97%, 98% or 99% sequence identity to the AP2 domain represented by SEQ ID NO: 191. The portion may further comprise either one motif but preferably both of motif1 as represented by SEQ ID NO: 192 and/or motif 2 as represented by SEQ ID NO: 209; preferably, motif 2 has the sequences as represented by SEQ ID NO: 193.

A portion may be prepared, for example, by making one or more deletions to a nucleic acid encoding a PLT transcription factor polypeptide. The portions may be used in isolated form or they may be fused to other coding (or non coding) sequences in order to, for example, produce a protein that combines several activities. When fused to other coding sequences, the resulting polypeptide produced upon translation may be bigger than that predicted for the PLT transcription factor portion. Preferably, the portion codes for a polypeptide with substantially the same biological activity as the PLT transcription factor polypeptides of SEQ ID NO: 176 and SEQ ID NO: 178.

Preferably, the portion is a portion of a nucleic acid as represented by any one of the sequences listed in Table I of the Examples. Most preferably the portion is a portion of a nucleic acid as represented by SEQ ID NO: 175 and SEQ ID NO: 177.

Another variant of a nucleic acid encoding a PLT transcription factor polypeptide, useful in the methods of the present invention, is a nucleic acid capable of hybridising under reduced stringency conditions, preferably under stringent conditions, with a probe derived from the nucleic acid as defined hereinbefore, which hybridising sequence encodes a polypeptide comprising in increasing order of preference at least 70%, 75%, 80%, 85%, 90%, 95%, 96%, 97%, 98% or 99% sequence identity to the AP2 domain represented by SEQ ID NO: 191. The hybridizing sequence may further comprise either one motif but preferably both of motif1 as represented by SEQ ID NO: 192 and/or motif 2 as represented by SEQ ID NO: 209, preferably as represented by SEQ ID NO: 193.

Preferably, the hybridising sequence is one that is capable of hybridising to a nucleic acid as represented by (or to probes derived from) any one of the sequences listed in Table I of the Examples, or to a portion of any of the aforementioned sequences (the target sequence). Most preferably the hybridising sequence is capable of hybridising to SEQ ID NO: 175 or to SEQ ID NO: 177 (or to probes derived therefrom). Probes are generally less than 1000 bp in length, preferably less than 500 bp in length. Commonly, probe lengths for DNA-DNA hybridisations such as Southern blotting, vary between 100 and 500 bp, whereas the hybridising region in probes for DNA-DNA hybridisations such as in PCR amplification generally are shorter than 50 but longer than 10 nucleotides .

The PLT transcription factor polypeptide may be encoded by a splice variant. The term "splice variant" as used herein encompasses variants of a nucleic acid sequence in which selected introns and/or exons have been excised, replaced, displaced or added, or in which introns have been shortened or lengthened. Such variants will be ones in which the substantial biological activity of the protein is retained, which may be achieved by selectively retaining functional segments of the protein. Such splice variants may be found in nature or may be manmade. Methods for making such splice variants are well known in the art.

Preferred splice variants are splice variants of the nucleic acid encoding a PLT transcription factor polypeptide comprising in increasing order of preference at least 70%, 75%, 80%, 85%, 90%, 95%, 96%, 97%, 98% or 99% sequence identity to the AP2 domain represented by SEQ ID NO: 191. Splice variants may further comprise either one motif but preferably both of motif1 as represented by SEQ ID NO: 192 and/or motif 2 as represented by SEQ ID NO: 209, preferably as represented by SEQ ID NO: 193.

Further preferred splice variants of nucleic acids encoding PLT transcription factor polypeptides comprising features as defined hereinabove are splice variants of a nucleic acid as represented by any one of the sequences listed in Table I of the Examples. Most preferred is a splice variant of a nucleic acid sequence as represented by SEQ ID NO: 175 and SEQ ID NO: 177.

The PLT transcription factor polypeptide may also be encoded by an allelic variant of a nucleic acid encoding a polypeptide comprising from comprising in increasing order of preference at least 70%, 75%, 80%, 85%, 90%, 95%, 96%, 97%, 98% or 99% sequence identity to the AP2 domain represented by SEQ ID NO: 191. The allelic variant may further comprise either one motif but preferably both of motif1 as represented by SEQ ID NO: 192 and/or motif 2 as represented by SEQ ID NO: 209, preferably as represented by SEQ ID NO: 193.

Preferred allelic variants of nucleic acids encoding PLT transcription factor polypeptides comprising features as defined hereinabove are splice variants of a nucleic acid as represented by any one of the sequences listed in Table I of the Examples. Most preferred is an allelic variant of a nucleic acid sequence as represented by SEQ ID NO: 175 and SEQ ID NO: 177.

The increase in expression of a nucleic acid encoding a PLT transcription factor polypeptide leads to raised corresponding mRNA or polypeptide levels, which could equate to raised activity of the PLT transcription factor polypeptide; or the activity may also be raised when there is no change in polypeptide levels, or even when there is a reduction in polypeptide levels. This may occur when the intrinsic properties of the polypeptide are altered, for example, by making mutant versions that are more active that the wild type polypeptide.

Directed evolution (or gene shuffling) may be used to generate variants of nucleic acids encoding PLT transcription factor polypeptides. This consists of iterations of DNA shuffling followed by appropriate screening and/or selection to generate variants of nucleic acids or portions thereof encoding PLT transcription factor polypeptides or homologues or portions thereof having an modified biological activity (Castle et al., (2004) Science 304(5674): 1151-4; US patents 5,811,238 and 6,395,547).

Site-directed mutagenesis may be used to generate variants of nucleic acids encoding PLT transcription factor polypeptides. Several methods are available to achieve site-directed mutagenesis, the most common being PCR based methods (current protocols in molecular biology. Wiley Eds.).

Directed evolution and site-directed mutagenesis are examples of technologies that enable the generation of variants of nucleic acids encoding PLT transcription factor polypeptides with modified activity useful to perform the methods of the invention.

Nucleic acids encoding PLT transcription factor polypeptides may be derived from any natural or artificial source. The nucleic acid may be modified from its native form in composition and/or genomic environment through deliberate human manipulation. Preferably the PLT transcription factor nucleic acid is from a plant, preferably from a dicotyledonous plant, further preferably from the *Brassicaceae* family, more preferably from the *Arabidopsis* genus, most preferably the nucleic acid is from *Arabidopsis thaliana.*

The methods of the invention rely on increased expression of a nucleic acid encoding a PLT transcription factor polypeptide in a plant. The nucleic acid may be a full-length nucleic acid or may be a portion or a hybridising sequence or another nucleic acid variant as hereinbefore defined.

The methods of the invention rely on increased expression of a nucleic acid encoding a PLT transcription factor polypeptide in a plant.

The invention also provides genetic constructs and vectors to facilitate introduction and/or expression in a plant of the nucleic acid sequences useful in the methods according to the invention.

Therefore, there is provided a gene construct comprising:
(i) A nucleic acid encoding a PLT transcription factor polypeptide as defined hereinabove;
(ii) One or more control sequences, of which at least one is a medium strength constitutive promoter.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding a PLT transcription factor polypeptide). The skilled artisan is well aware of the genetic elements that must be present on the vector in order to successfully transform, select and propagate host cells containing the sequence of interest. The sequence of interest is operably linked to one or more control sequences (at least to a promoter).

The nucleic acid encoding a PLT transcription factor polypeptide or variant thereof is operably linked to a constitutive promoter, preferably a medium strength constitutive promoter. A constitutive promoter is transcriptionally active during most, but not necessarily all, phases of its growth and development and is substantially ubiquitously expressed at moderate levels. Promoter strength and/or expression pattern can be analysed as described in the definitions section. The promoter strength and/or expression pattern can for example be compared to that of a well-characterised shoot preferred reference promoter, such as the Cab27 promoter (weak expression, GenBank AP004700) or the putative protochlorophyllid reductase promoter (strong expression, GenBank AL606456). Preferably the promoter used in the methods of the present invention is derived from a plant, further preferably a monocotyledonous plant. Most preferred is use of a GOS2 promoter (from rice) (SEQ ID NO: 194 or alternatively SEQ ID NO: 210). It should be clear that the applicability of the present invention is not restricted to the nucleic acid represented by SEQ ID NO: 175 or SEQ ID NO: 177, nor is the applicability of the invention restricted to expression of a nucleic acid encoding a PLT transcription factor polypeptide when driven by a GOS2 promoter. Examples of other constitutive promoters that may also be used to drive expression of a nucleic acid encoding a PLT transcription factor polypeptide are shown in the definitions section.

Optionally, one or more terminator sequences (also a control sequence) may be used in the construct introduced into a plant. Additional regulatory elements may include transcriptional as well as translational enhancers. Those skilled in the art will be aware of terminator and enhancer sequences that may be suitable for use in performing the invention. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1. The genetic construct may optionally comprise a selectable marker gene.

The present invention also encompasses plants obtainable by the methods according to the present invention. The present invention therefore provides plants, plant parts or plant cells thereof obtainable by the method according to the present invention, which plants or parts or cells thereof comprise a nucleic acid transgene encoding a PLT transcription factor polypeptide under the control of a constitutive promoter, preferably a non-viral constitutive promoter.

The invention also provides a method for the production of transgenic plants having increased yield relative to control plants, comprising introduction and preferential expression of a nucleic acid encoding a PLT transcription factor polypeptide in a plant.

More specifically, the present invention provides a method for the production of transgenic plants having increased yield which method comprises:
(i) introducing and expressing a nucleic acid encoding a PLT transcription factor polypeptide in a plant; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

Generally after transformation, plant cells or cell groupings are selected for the presence of one or more markers which are encoded by plant-expressible genes co-transferred with the gene of interest, following which the transformed material is regenerated into a whole plant. Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a PLT transcription factor polypeptide. Preferred host cells according to the invention are plant cells.

The invention furthermore extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

The present invention also encompasses use of nucleic acids encoding PLT transcription factor polypeptides and use of PLT transcription factor polypeptides in increasing plant yield as defined hereinabove in the methods of the invention.

The increased expression of a nucleic acid encoding a PLT transcription factor polypeptide may be performed by introducing a genetic modification (preferably in the locus of a PLT transcription factor gene). The locus of a gene as defined herein is taken to mean a genomic region, which includes the gene of interest and 10KB up- or downstream of the coding region.

The genetic modification may be introduced by alternative methods as the one described hereinabove, for example, by any one (or more) of the following: T-DNA activation, TILLING and homologous recombination. Following introduction of the genetic modification, there follows an optional step of selecting for increased expression of a nucleic acid encoding a PLT transcription factor polypeptide, which increased expression gives plants having increased yield.

T-DNA activation tagging results in transgenic plants that show dominant phenotypes due to modified expression of genes close to the introduced promoter. The promoter to be introduced a medium stength constitutive promoter capable of increasing expression of the nucleic acid encoding a PLT transcription factor polypeptide in a plant.

A genetic modification may also be introduced in the locus of a gene encoding a PLT transcription factor polypeptide using the technique of TILLING (Targeted Induced Local Lesions In Genomes).

The effects of the invention may also be reproduced using homologous recombination. The nucleic acid to be introduced (which may be a nucleic acid encoding a PLT transcription factor polypeptide or variant thereof as hereinbefore defined) is targeted to the locus of a PLT gene. The nucleic acid to be targeted may be an improved allele used to replace the endogenous gene or may be introduced in addition to the endogenous gene.

T-DNA activation, TILLING and homologous recombination are examples of technologies that enable the generation of genetic modifications comprising increasing expression of a nucleic acid encoding a PLT transcription factor polypeptide in plants.

Nucleic acids encoding PLT transcription factor polypeptides, or PLT transcription factor polypeptides, may find use in breeding programmes in which a DNA marker is identified which may be genetically linked to a PLT transcription factor gene. The nucleic acids/genes, or the PLT transcription factor polypeptides may be used to define a molecular marker. This DNA or protein marker may then be used in breeding programmes to select plants having increased yield as defined hereinabove in the methods of the invention.

Allelic variants of a PLT transcription factor nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

A nucleic acid encoding a PLT transcription factor polypeptide may also be used as probes for genetically and physically mapping the genes that they are a part of, and as markers for traits linked to those genes. Such information may be useful in plant breeding in order to develop lines with desired phenotypes. Such use of PLT transcription factor nucleic acids requires only a nucleic acid sequence of at least 15 nucleotides in length. The PLT transcription factor nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the PLT transcription factor nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the PLT transcription factor nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield, as described hereinbefore. This increased yield may also be combined with other economically advantageous traits, such as further yield-enhancing traits, tolerance to other abiotic and biotic stresses, traits modifying various architectural features and/or biochemical and/or physiological features.

### Detailed description of bHLH

Surprisingly, it has now been found that modulating expression in a plant of a nucleic acid encoding a particular class of bHLH transcription factor gives plants having enhanced yield-related traits relative to control plants. The particular class of bHLH transcription factor suitable for enhancing yield-related traits in plants is described in detail below.

The present invention provides a method for enhancing yield-related traits in plants relative to control plants, comprising modulating expression in a plant of a nucleic acid encoding a particular class of bHLH transcription factor.

A preferred method for modulating (preferably, increasing) expression of a nucleic acid encoding a bHLH transcription factor is by introducing and expressing in a plant a nucleic acid encoding a particular class of bHLH transcription factor as further defined below.

The nucleic acid to be introduced into a plant (and therefore useful in performing the methods of the invention) is any nucleic acid encoding the type of bHLH transcription factor which will now be described. A bHLH transcription factor as defined herein refers to a polypeptide represented by any one of SEQ ID NO 213, SEQ ID NO 215, SEQ ID NO: 217, SEQ ID NO: 219, SEQ ID NO 225, SEQ ID NO: 297, SEQ ID NO: 299 and SEQ ID NO: 301. The invention is illustrated by transforming plants with the *Oryza sativa* sequence represented by SEQ ID NO: 212, encoding the polypeptide of SEQ ID NO: 213. SEQ ID NO: 215 from *Oryza sativa* (encoded by SEQ ID NO: 214) and SEQ ID NO: 217 from *Oryza sativa* (encoded by SEQ ID NO: 216) are paralogues of the polypeptide of SEQ ID NO: 213. SEQ ID NO: 219 from *Arabidopsis thaliana* (encoded by SEQ ID NO: 218) and SEQ ID NO: 225 from *Arabidopsis thaliana* (encoded by SEQ ID NO: 224) are orthologues of the polypeptide of SEQ ID NO: 213. SEQ ID NO: 221 from *Arabidopsis thaliana* (encoded by SEQ ID NO: 220) and SEQ ID NO: 223 from *Arabidopsis thaliana* (encoded by SEQ ID NO: 222) are variants of SEQ ID NO: 218 and SEQ ID NO: 219.

Orthologues and paralogues may easily be found by performing a so-called reciprocal blast search. Typically this involves a first BLAST involving BLASTing a query sequence (for example, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 224 or SEQ ID NO: 225) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 212, SEQ ID NO: 213, SEQ ID NO: 214, SEQ ID NO: 215, SEQ ID NO: 216 or SEQ ID NO: 217, the second BLAST would therefore be against *Oryza* sequences; where the query sequence is SEQ ID NO: 218, SEQ ID NO: 219, SEQ ID NO: 224 or SEQ ID NO: 225, the second BLAST would therefore be against *Arabidopsis* sequences). The results of the first and second BLASTs are then compared. A paralogue is identified if a high-ranking hit from the first blast is from the same species as from which the query sequence is derived, a BLAST back then ideally results in the query sequence as highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. Preferably the score is greater than 50, more preferably greater than 100; and preferably the E-value is less than e-5, more preferably less than e-6. An example detailing the identification of orthologues and paralogues is given in Example 31 and Example 30 respectively herein. In the case of large families, ClustalW may be used, followed by a neighbour joining tree, to help visualize clustering of related genes and to identify orthologues and paralogues.

Examples of orthologues obtained by the BLAST procedure mentioned above are SEQ ID NO: 227 from *Medicago truncatula* (encoded by SEQ ID NO: 226) and SEQ ID NO: 229 from *Hordeum vulgare* (encoded by SEQ ID NO: 228). Further orthologues and paralogues may readily be identified using the BLAST procedure described above and by following the procedure given in the Examples section.

The proteins represented by SEQ ID NO: 297, 299 and 301 were hitherto unknown. Therefore, the invention also provides hitherto unknown bHLH transcription factors and bHLH transcription factor-encoding nucleic acids.

According to a further embodiment of the present invention, there is therefore provided an isolated nucleic acid molecule comprising:
(i) a nucleic acid represented by any one of SEQ ID NO: 296, SEQ ID NO: 298 and SEQ ID NO: 300;
(ii) the complement of a nucleic acid represented by any one of SEQ ID NO: 296, SEQ ID NO: 298 and SEQ ID NO: 300;
(iii) a nucleic acid encoding a bHLH transcription factor having (a) in increasing order of preference, at least 50%, 55%, 60%, 65%, 70%, 75%, 80%, 85%, 90%, 95%, 96%, 97%, 98%, 99% or more sequence identity to the amino acid sequence represented by any one of SEQ ID NO: 297, SEQ ID NO: 299 and SEQ ID NO: 301, and (b) a bHLH domain.

According to a further embodiment of the present invention, there is also provided an isolated polypeptide comprising:
(i) an amino acid sequence represented by any one of SEQ ID NO: 297, SEQ ID NO: 299 and SEQ ID NO: 301;
(ii) an amino acid sequence having (a) in increasing order of preference, at least 50%, 55%, 60%, 65%, 70%, 75%, 80%, 85%, 90%, 95%, 96%, 97%, 98%, 99% or more sequence identity to any one of the amino acid sequences represented by SEQ ID NO: 297, SEQ ID NO: 299 and SEQ ID NO: 301, and (b) a bHLH domain.;
(iii) derivatives of any of the amino acid sequences given in (i) or (ii) above.

The polypeptides represented by any one of SEQ ID NO 213, SEQ ID NO 215, SEQ ID NO: 217, SEQ ID NO: 219, SEQ ID NO 221, SEQ ID NO 223, SEQ ID NO 225, SEQ ID NO: 227, SEQ ID NO: 229, SEQ ID NO: 297, SEQ ID NO: 299, SEQ ID NO: 301, or orthologues or paralogues of any of the aforementioned SEQ ID NOs, all comprise a bHLH domain.

bHLH domains are well known in the art and may readily be identified by persons skilled in the art. The family is defined by a bHLH signature domain, which consists of 60 or so amino acids with two functionally distinct regions. A basic region, located at the N-terminal end of the domain, is involved in DNA binding and consists of 15 or so amino acids with a high number of basic residues. An HLH region, at the C-terminal end, functions as a dimerization domain and mainly comprises hydrophobic residues that form two amphipathic helices separated by a loop region of variable sequence and length.

A bHLH domain may be identified using methods for the alignment of sequences for comparison. In some instances, default parameters may be adjusted to modify the stringency of the search. For example using BLAST, the statistical significance threshold (called E-value) for reporting matches against database sequences may be increased to show less stringent matches. In this way, short nearly exact matches may be identified.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole the sequence) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Minor manual editing may be performed to optimise alignment between conserved motifs, as would be apparent to a person skilled in the art.

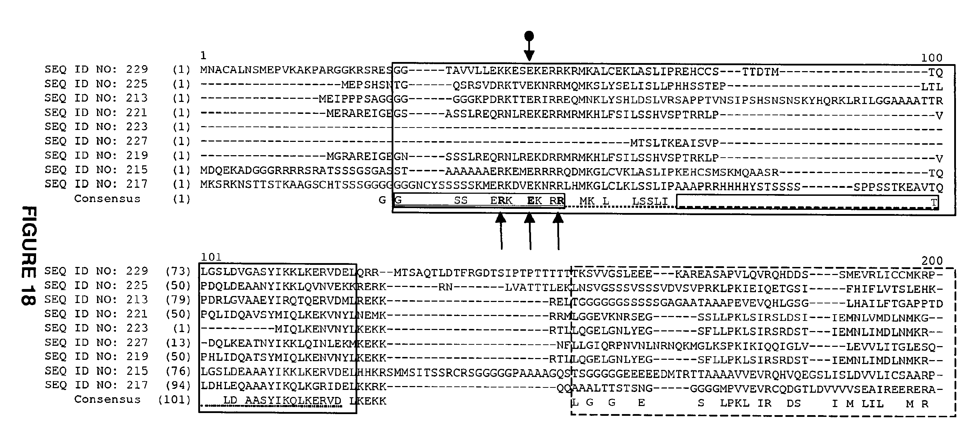

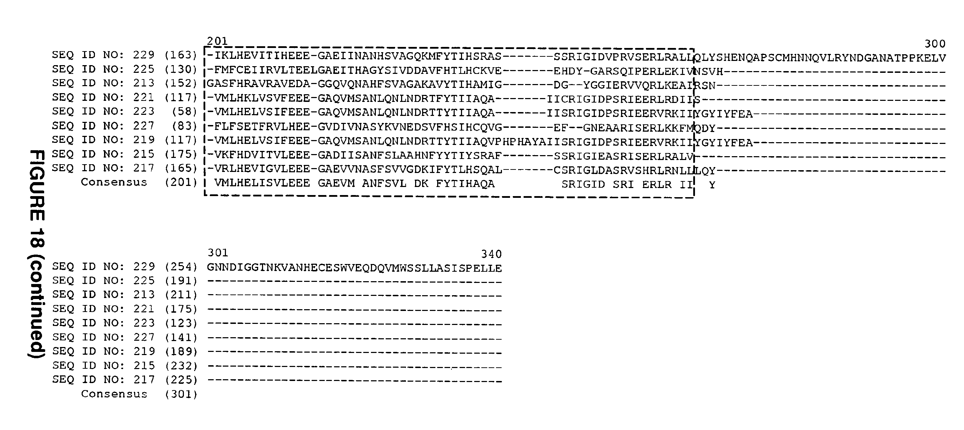

Specialist databases also exist for the identification of domains. The bHLH domain in a bHLH transcription factor may be identified using, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)). The HLH domain structure in the InterPro database is designated IPR001092 and IPR011598; PF00010 in the Pfam database; SM00353 in the SMART database and PS50888 in the PROSITE database. Furthermore, the alignment shown in Figure 18 highlights the bHLH domain of bHLH transcription factors useful in the methods of the invention.

The polypeptides represented by any one of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or orthologues or paralogues of any of the aforementioned SEQ ID NOs, typically exhibit considerable sequence divergence outside of the conserved bHLH domain.

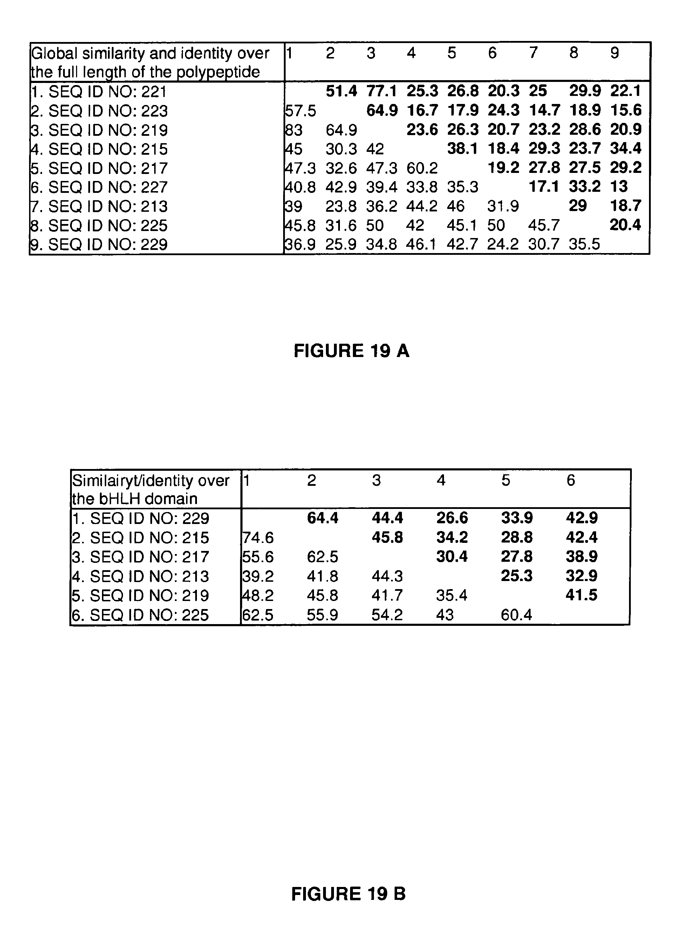

Figure 19a is a matrix showing the overall similarities and identities (in bold) of the bHLH type proteins described above. Even though the identities appear to be relatively low, polypeptides having sequence identity falling within the ranges shown in the matrix may be taken to be orthologues or paralogues of any of SEQ ID NO 213, SEQ ID NO 215, SEQ ID NO: 217, SEQ ID NO: 219 or SEQ ID NO 225; this may be confirmed by the reciprocal blast procedure described above. Typically, nucleic acids encoding bHLH transcription factors useful in the methods of the invention have, in increasing order of preference, at least 13%, 15%, 20%, 25%, 30%, 35%, 40%, 45%, 50%, 55%, 60%, 65%, 70%, 75%, 80%, 85%, 90%, 95% or more sequence identity to the bHLH transcription factors represented by any one of SEQ ID NO 213, SEQ ID NO 215, SEQ ID NO: 217, SEQ ID NO: 219 or SEQ ID NO 225.

The matrix shown in Figure 19b shows similarities and identities (in bold) over the bHLH domain, where of course the values are higher than when considering full length proteins. Typically, nucleic acids encoding bHLH transcription factors useful in the methods of the invention have bHLH domains having, in increasing order of preference, at least 25%, 30%, 35%, 40%, 45%, 50%, 55%, 60%, 65%, 70%, 75%, 80%, 85%, 90%, 95% or more sequence identity to the bHLH domain of any one of the polypeptides represented by any one of SEQ ID NO 213, SEQ ID NO 215, SEQ ID NO: 217, SEQ ID NO: 219 or SEQ ID NO 225.

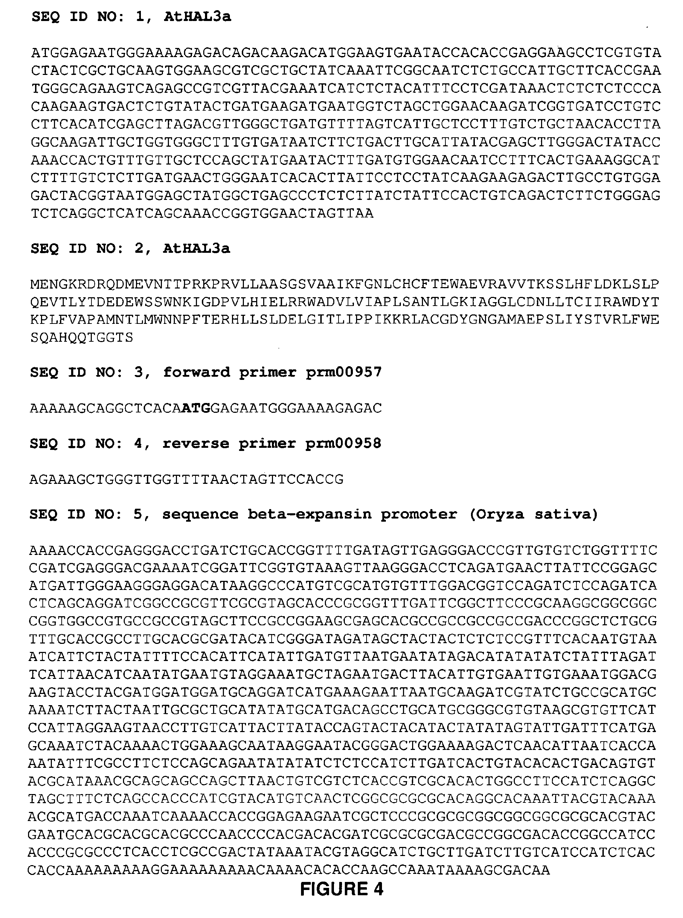

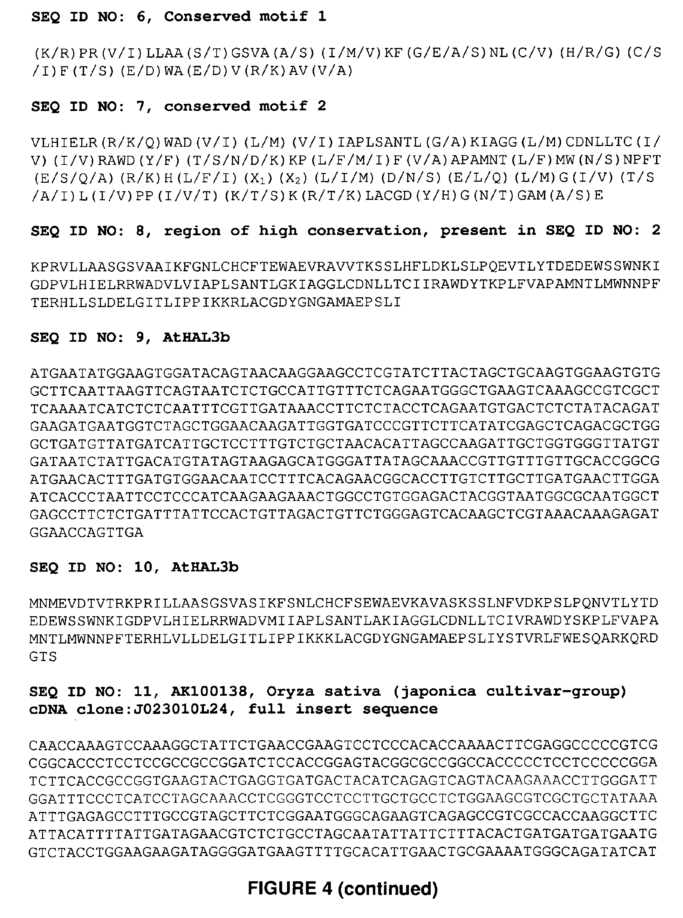

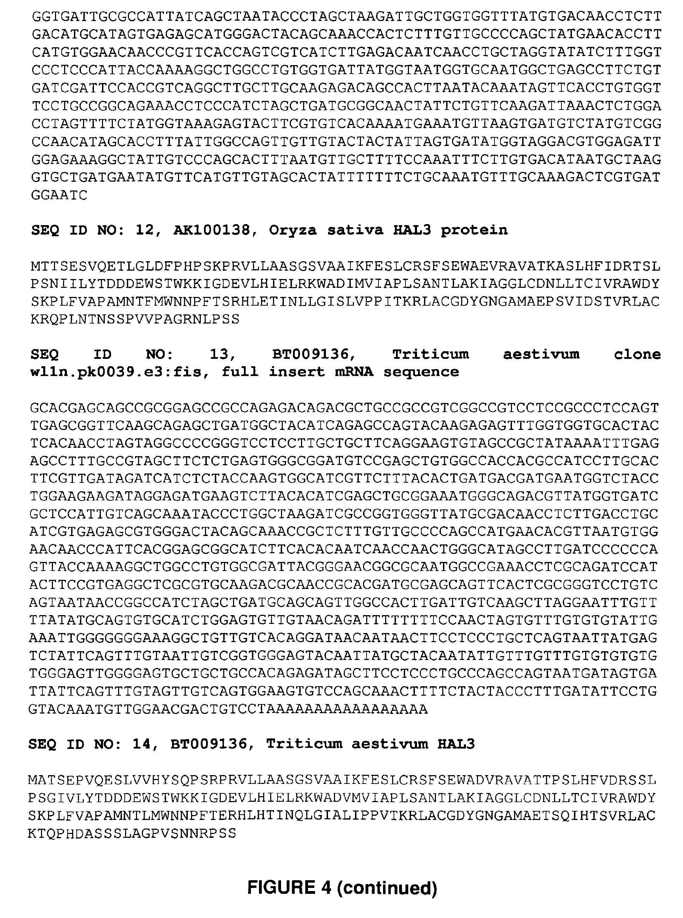

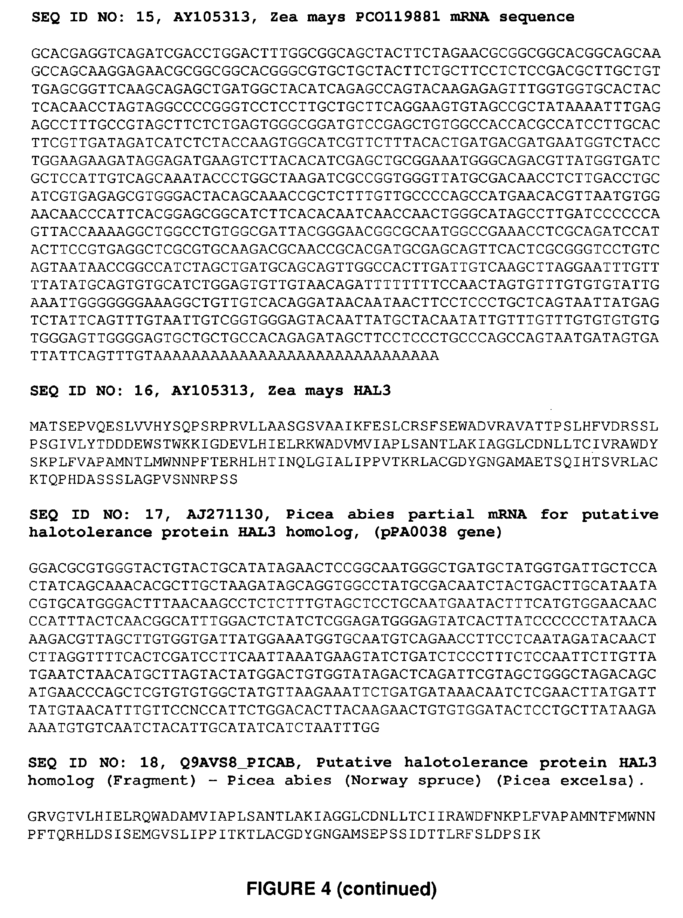

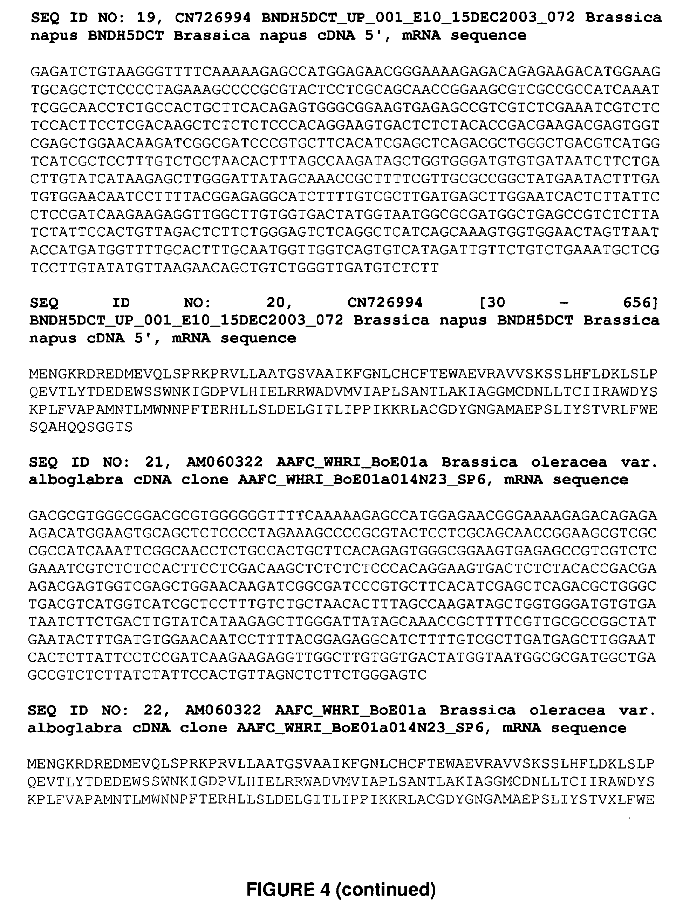

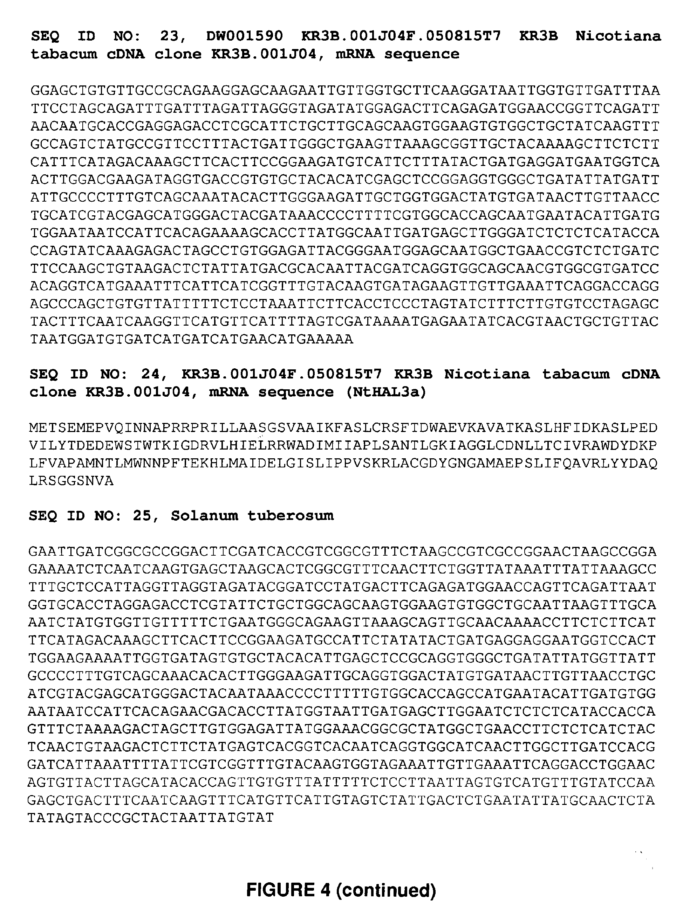

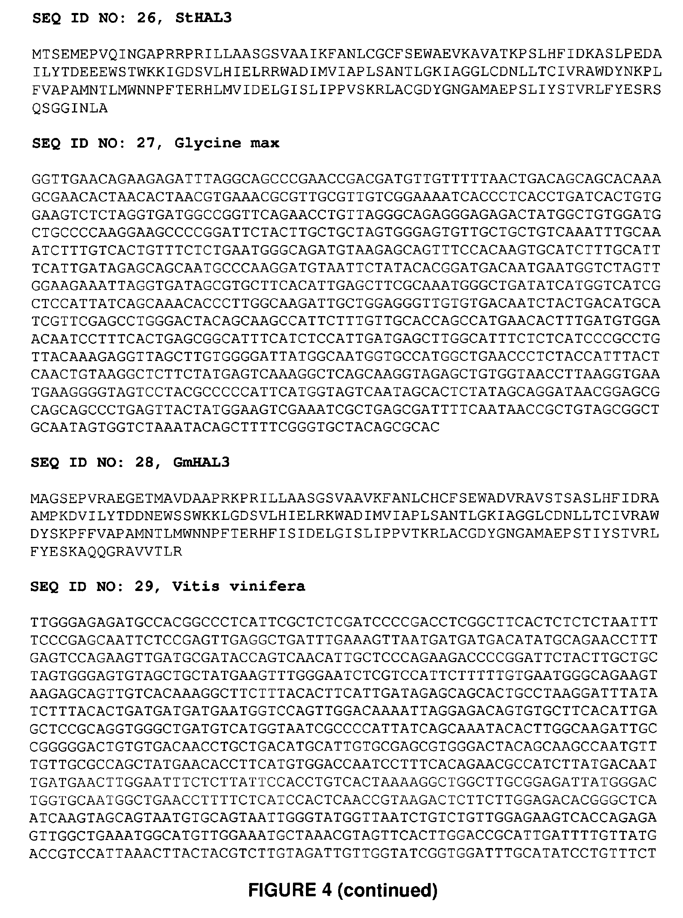

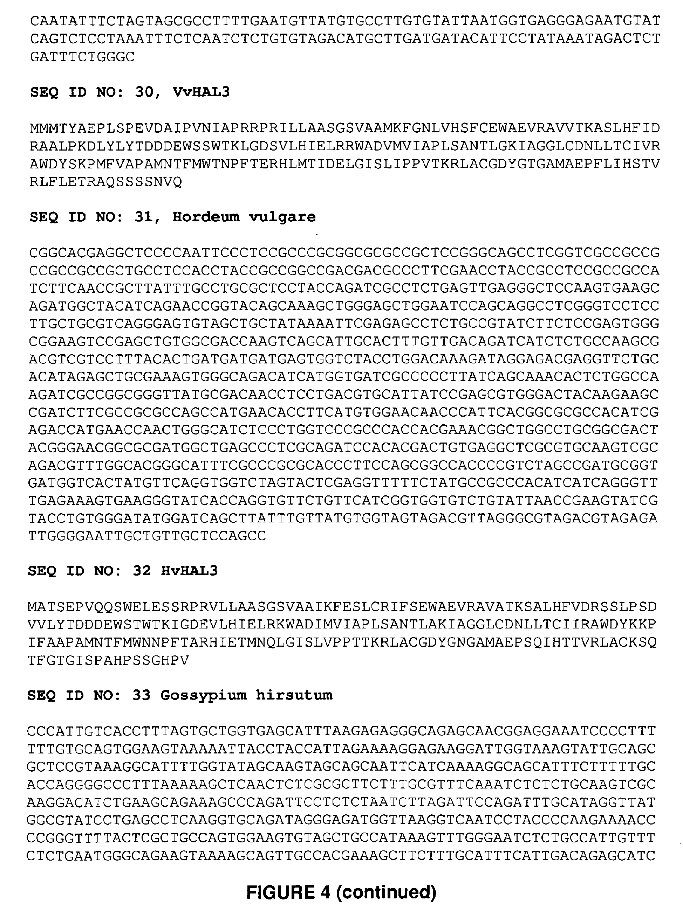

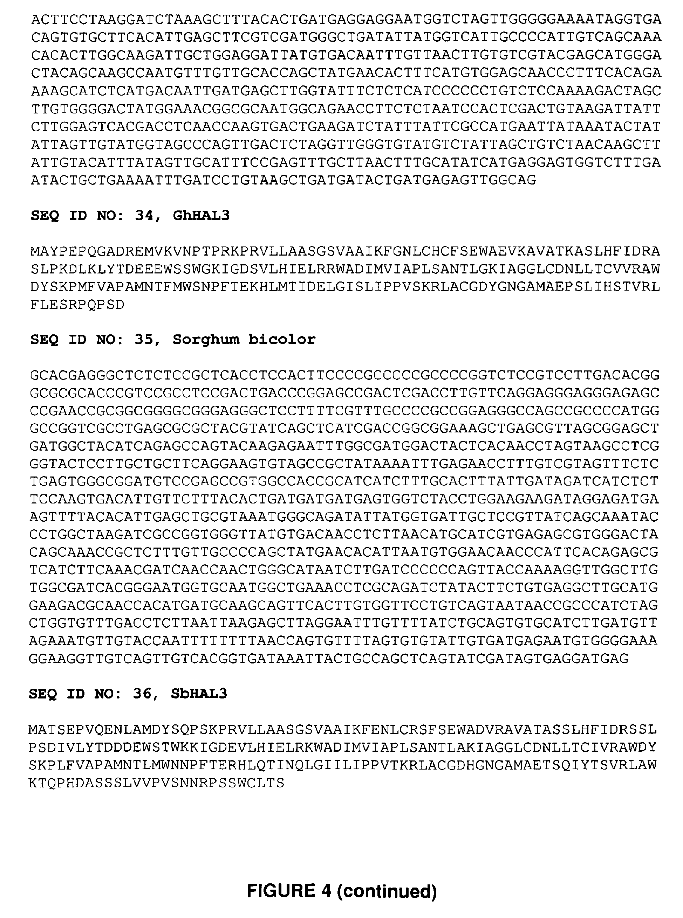

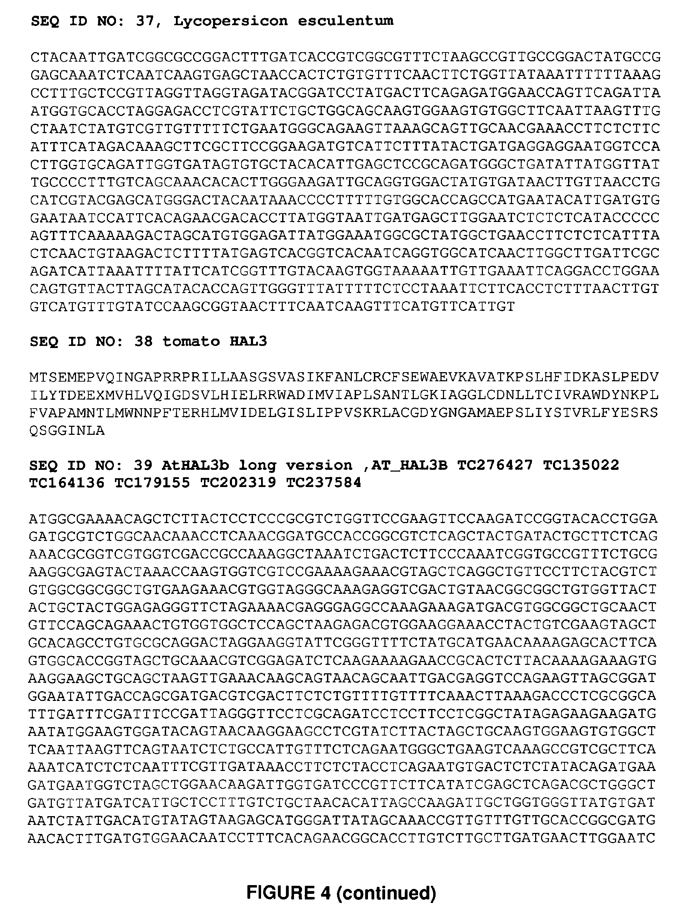

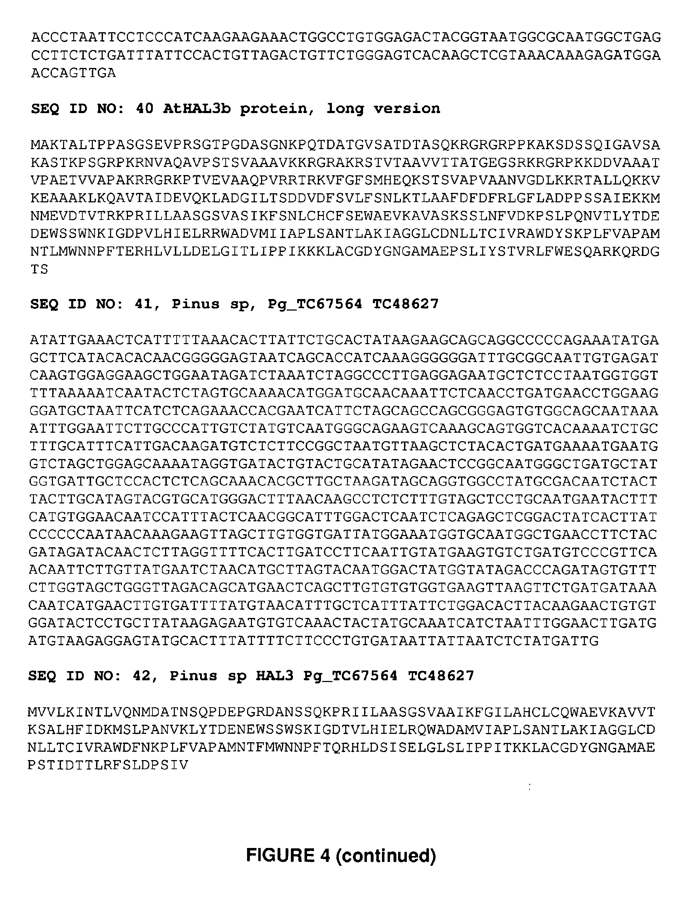

A pattern of amino acids, termed a 5-9-13 configuration, may be found at three positions within the basic region of the bHLH domain (see Fig. 4 of Heim et al., 2003 (Mol. Biol. Evol. 20(5):735-747) and Figure 18 herein where three upwardly pointing arrows show the configuration). The polypeptides represented by any one of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or orthologues or paralogues of any of the aforementioned SEQ ID NOs, preferably comprise a K/R ER configuration, more preferably an RER configuration, within the bHLH domain, typically within the basic region of the domain. The presence of this configuration is not a prerequisite to performing the methods of the invention, therefore some variation in the K/R ER configuration is acceptable. *Arabidopsis* bHLH polypeptides were grouped into twelve subfamilies according to structural similarities (see Fig. 4 of Heim *et al.,* 2003). Members of group IX constitute bHLH polypeptides having an RER configuration. Furthermore, one member of Group VI comprises an RER configuration.

The polypeptides represented by any one of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or orthologues or paralogues of any of the aforementioned SEQ ID NOs, may also comprise (in addition to a bHLH domain and optionally a K/R ER 5-9-13 motif, preferably an RER motif) a domain designated PFB26111 (see Pfam database). The domain is also indicated in Figure 18.

Typically, nucleic acids encoding bHLH transcription factors (as defined above) having a bHLH domain and having, in increasing order of preference, at least 50%, 55%, 60%, 65%, 70%, 75%, 80%, 85%, 90%, 95% or more sequence identity to the PFB26111 domain (see Figure 18) of any one of the polypeptides represented by any one of SEQ ID NO 213, SEQ ID NO 215, SEQ ID NO: 217, SEQ ID NO: 219, SEQ ID NO 225, SEQ ID NO: 297, SEQ ID NO: 299, or SEQ ID NO: 301 are useful in the methods of the invention.

Nucleic acids encoding the polypeptides represented by any one of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301, or orthologues or paralogues of any of the aforementioned SEQ ID NOs, need not be full-length nucleic acids, since performance of the methods of the invention does not rely on the use of full length nucleic acid sequences. Examples of nucleic acids suitable for use in performing the methods of the invention include but are not limited to those represented by any one of: SEQ ID NO: 212, SEQ ID NO: 214, SEQ ID NO: 216, SEQ ID NO: 218, SEQ ID NO: 220, SEQ ID NO: 222, SEQ ID NO: 224, SEQ ID NO: 226, SEQ ID NO: 228, SEQ ID NO: 296, SEQ ID NO: 298, and SEQ ID NO: 300. Nucleic acid variants may also be useful in practising the methods of the invention. Examples of such nucleic acid variants include portions of nucleic acids encoding a bHLH transcription factor as defined herein, splice variants of nucleic acids encoding a bHLH transcription factor as defined herein, allelic variants of nucleic acids encoding a bHLH transcription factor as defined herein and variants of nucleic acids encoding a bHLH transcription factor as defined herein that are obtained by gene shuffling. The terms portion, splice variant, allelic variant and gene shuffling will now be described.

A portion of a nucleic acids encoding a bHLH transcription factor as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the bHLH transcription factor portion.

According to the present invention, there is provided a method for enhancing yield-related traits in plants, comprising introducing and expressing in a plant a portion of a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301, or a portion of a nucleic acid encoding orthologues, paralogues or homologues of any of the aforementioned SEQ ID NOs.

Portions useful in the methods of the invention, encode a polypeptide having a bHLH domain (as described above) and having substantially the same biological activity as the bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301, or orthologues or paralogues of any of the aforementioned SEQ ID NOs. The portion is typically at least 150 consecutive nucleotides in length, preferably at least 300 consecutive nucleotides in length, more preferably at least 400 consecutive nucleotides in length and most preferably at least 500 consecutive nucleotides in length. Preferably, the portion is a portion of a nucleic acid as represented by any one of SEQ ID NO: 212, SEQ ID NO: 214, SEQ ID NO: 216, SEQ ID NO: 218, SEQ ID NO: 220, SEQ ID NO: 222, SEQ ID NO: 224, SEQ ID NO: 226, SEQ ID NO: 228, SEQ ID NO: 296, SEQ ID NO: 298, and SEQ ID NO: 300. Most preferably the portion is a portion of a nucleic acid as represented by SEQ ID NO: 212.

Another nucleic acid variant useful in the methods of the invention, is a nucleic acid capable of hybridising under reduced stringency conditions, preferably under stringent conditions, with a nucleic acid encoding a bHLH transcription factor as defined herein, or a with a portion as defined herein.

Hybridising sequences useful in the methods of the invention, encode a polypeptide having a bHLH domain (as described above) and having substantially the same biological activity as the bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or having substantially the same biological activity as orthologues or paralogues of any of the aforementioned SEQ ID NOs. The hybridising sequence is typically at least 150 consecutive nucleotides in length, preferably at least 300 consecutive nucleotides in length, more preferably at least 400 consecutive nucleotides in length and most preferably at least 500 consecutive nucleotides in length. Preferably, the hybridising sequence is one that is capable of hybridising to any of the nucleic acids represented by SEQ ID NO: 212, SEQ ID NO: 214, SEQ ID NO: 216, SEQ ID NO: 218, SEQ ID NO: 220, SEQ ID NO: 222, SEQ ID NO: 224, SEQ ID NO: 226, SEQ ID NO: 228, SEQ ID NO: 296, SEQ ID NO: 298, and SEQ ID NO: 300 or to a portion of any of the aforementioned sequences, a portion being as defined above. Most preferably the hybridising sequence is capable of hybridising to a nucleic acid as represented by SEQ ID NO: 212, or to portions thereof.

According to the present invention, there is provided a method for enhancing yield-related traits in plants, comprising introducing and expressing in a plant a nucleic acid capable of hybridizing to a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301, or comprising introducing and expressing in a plant a nucleic acid capable of hybridising to a nucleic acid encoding an orthologue, paralogue or homologue of any of the aforementioned SEQ ID NOs.

Another nucleic acid variant useful in the methods of the invention is a splice variant encoding a bHLH transcription factor as defined hereinabove.

According to the present invention, there is provided a method for enhancing yield-related traits in plants, comprising introducing and expressing in a plant a splice variant of a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, and SEQ ID NO: 301, or a splice variant of a nucleic acid encoding an orthologue, paralogue or homologue of any of the aforementioned SEQ ID NOs.

Preferred splice variants are splice variants of a nucleic acid encoding bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, and SEQ ID NO: 301, or splice variants encoding orthologues or paralogues of any of the aforementioned SEQ ID NOs. Further preferred are splice variants of nucleic acids represented by any one of SEQ ID NO: 212, SEQ ID NO: 214, SEQ ID NO: 216, SEQ ID NO: 218, SEQ ID NO: 220, SEQ ID NO: 222, SEQ ID NO: 224, SEQ ID NO: 226, SEQ ID NO: 228, SEQ ID NO: 296, SEQ ID NO: 298, and SEQ ID NO: 300. Most preferred is a splice variant of a nucleic acid as represented by SEQ ID NO: 212.

Another nucleic acid variant useful in performing the methods of the invention is an allelic variant of a nucleic acid encoding a bHLH transcription factor as defined hereinabove. According to the present invention, there is provided a method for enhancing yield-related traits in plants, comprising introducing and expressing in a plant an allelic variant of a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, and SEQ ID NO: 301, or comprising introducing and expressing in a plant an allelic variant of a nucleic acid encoding an orthologue, paralogue or homologue of any of the aforementioned SEQ ID NOs.

The allelic variant may be an allelic variant of a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, and SEQ ID NO: 301, or an allelic variants of a nucleic acid encoding orthologues or paralogues of any of the aforementioned SEQ ID NOs. Further preferred are allelic variants of nucleic acids represented by any one of SEQ ID NO: 212, SEQ ID NO: 214, SEQ ID NO: 216, SEQ ID NO: 218, SEQ ID NO: 220, SEQ ID NO: 222, SEQ ID NO: 224, SEQ ID NO: 226 and SEQ ID NO: 228, SEQ ID NO: 296, SEQ ID NO: 298, SEQ ID NO: 300. Most preferred is an allelic variant of a nucleic acid as represented by SEQ ID NO: 212.

A further nucleic acid variant useful in the methods of the invention is a nucleic acid variant obtained by gene shuffling. Gene shuffling or directed evolution may also be used to generate variants of nucleic acids encoding bHLH transcription factors as defined above.

According to the present invention, there is provided a method for enhancing yield-related traits in plants, comprising introducing and expressing in a plant a variant of a nucleic acid encoding a bHLH transcription factor represented by any of SEQ ID NO 213, SEQ ID NO 215, SEQ ID NO: 217, SEQ ID NO: 219, SEQ ID NO 225, SEQ ID NO: 297, SEQ ID NO: 299, SEQ and ID NO: 301, or comprising introducing and expressing in a variant of a nucleic acid encoding an orthologue, paralogue or homologue of any of the aforementioned SEQ ID NOs, which nucleic acid is obtained by gene shuffling.

Furthermore, nucleic acid variants may also be obtained by site-directed mutagenesis. Several methods are available to achieve site-directed mutagenesis, the most common being PCR based methods (current protocols in molecular biology. Wiley Eds.).

Also useful in the methods of the invention are nucleic acids encoding homologues of any one of the amino acids represented by SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or orthologues or paralogues of any of the aforementioned SEQ ID NOs.

Also useful in the methods of the invention are nucleic acids encoding derivatives of any one of the amino acids represented by SEQ ID NO 213, SEQ ID NO 215, SEQ ID NO: 217, SEQ ID NO: 219, SEQ ID NO 225, SEQ ID NO: 297, SEQ ID NO: 299, SEQ ID NO: 301 or orthologues or paralogues of any of the aforementioned SEQ ID NOs. Derivatives of SEQ ID NO: 215, SEQ ID NO: 217, SEQ ID NO: 219, SEQ ID NO: 221, SEQ ID NO: 223, SEQ ID NO: 225, SEQ ID NO: 227 and SEQ ID NO: 229, SEQ ID NO: 297, SEQ ID NO: 299, SEQ ID NO: 301 are further examples which may be suitable for use in the methods of the invention

Furthermore, bHLH transcription factors (at least in their native form) typically have DNA-binding activity and an activation domain. A person skilled in the art may easily determine the presence of an activation domain and DNA-binding activity using routine tools and techniques.

Nucleic acids encoding bHLH transcription factors may be derived from any natural or artificial source. The nucleic acid may be modified from its native form in composition and/or genomic environment through deliberate human manipulation. Preferably the bHLH transcription factor-encoding nucleic acid is from a plant, further preferably from a monocot, more preferably from the *Poaceae* family, most preferably the nucleic acid is from *Oryza sativa.*

Any reference herein to a bHLH transcription factor is therefore taken to mean a bHLH transcription factor as defined above. Any nucleic acid encoding such a bHLH transcription factor is suitable for use in performing the methods of the invention.

The present invention also encompasses plants or parts thereof (including plant cells) obtainable by the methods according to the present invention. The plants or parts thereof comprise a nucleic acid transgene encoding a bHLH transcription factor as defined above.

The invention also provides genetic constructs and vectors to facilitate introduction and/or expression of the nucleic acid sequences useful in the methods according to the invention, in a plant.

Therefore, there is provided a gene construct comprising:
(i) Any nucleic acid encoding a bHLH-type transcription factor as defined hereinabove;
(ii) One or more control sequences operably liked to the nucleic acid of (i).

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding a bHLH-type transcription factor). The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. The promoter may be an inducible promoter, i.e. having induced or increased transcription initiation in response to a developmental, chemical, environmental or physical stimulus. The promoter may be a tissue-specific promoter, i.e. one that is capable of preferentially initiating transcription in certain tissues, such as the leaves, roots, seed tissue etc.

According to one preferred feature of the invention, the nucleic acid encoding a bHLH-type transcription factor is operably linked to a constitutive promoter. A constitutive promoter is transcriptionally active during most but not necessarily all phases of its growth and development and is substantially ubiquitously expressed. The constitutive promoter is preferably a GOS2 promoter, more preferably the constitutive promoter is a rice GOS2 promoter, further preferably the constitutive promoter is represented by a nucleic acid sequence substantially similar to SEQ ID NO: 230 or SEQ ID NO: 233, most preferably the constitutive promoter is as represented by SEQ ID NO: 233.

It should be clear that the applicability of the present invention is not restricted to the bHLH transcription factor-encoding nucleic acid represented by SEQ ID NO: 212, nor is the applicability of the invention restricted to expression of a such a bHLH transcription factor-encoding nucleic acid when driven by a GOS2 promoter. Examples of other constitutive promoters which may also be used perform the methods of the invention are shown in the definitions section.

Optionally, one or more terminator sequences (also a control sequence) may be used in the construct introduced into a plant. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1. Other control sequences (besides promoter, enhancer, silencer, intron sequences, 3'UTR and/or 5'UTR regions) may be protein and/or RNA stabilizing elements. The genetic construct may optionally comprise a selectable marker gene.

The invention also provides a method for the production of transgenic plants having enhanced yield-related traits relative to control plants, comprising introduction and expression in a plant of any nucleic acid encoding a bHLH-type transcription factor polypeptide as defined hereinabove.

More specifically, the present invention provides a method for the production of transgenic plants having enhanced yield-related traits, which method comprises:
(i) introducing and expressing a nucleic acid encoding a bHLH-type transcription factor (as defined herein) in a plant cell; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a bHLH transcription factor as defined hereinabove. Preferred host cells according to the invention are plant cells.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression.

As mentioned above, a preferred method for modulating (preferably, increasing) expression of a nucleic acid encoding a bHLH transcription factor is by introducing and expressing in a plant a nucleic acid encoding a bHLH transcription factor; however the effects of performing the method, i.e. enhancing yield-related traits may also be achieved using other well known techniques. A description of some of these techniques will now follow.

One such technique is T-DNA activation tagging. The resulting transgenic plants show dominant phenotypes due to modified expression of genes close to the introduced promoter. The effects of the invention may also be reproduced using the technique of TILLING (Targeted Induced Local Lesions In Genomes). The effects of the invention may also be reproduced using homologous recombination.

Reference herein to the term enhanced yield-related traits is taken to mean an increase in biomass (weight) of one or more parts of a plant, which may include aboveground (harvestable) parts and/or (harvestable) parts below ground.

In particular, such harvestable parts are seeds, and performance of the methods of the invention results in plants having increased seed yield relative to the seed yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour (increased seedling vigor at emergence). The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a bHLH transcription factor.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a MADS15 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

According to a preferred feature of the present invention, performance of the methods of the invention gives plants having an increased growth rate relative to control plants, particularly during the early stages of plant development (typically three weeks post germination in the case of rice and maize, but this will vary from species to species) leading to early vigour. Therefore, according to the present invention, there is provided a method for increasing the growth rate of plants, which method comprises modulating, preferably increasing, expression in a plant of a nucleic acid encoding a bHLH transcription factor. The present invention therefore also provides a method for obtaining plants having early vigour relative to control plants, which method comprises modulating, preferably increasing, expression in a plant of a nucleic acid encoding a bHLH transcription factor.

Early vigour may also result from or be manifested as increased plant fitness relative to control plants due to, for example, the plants being better adapted to their environment (i.e. being more able to cope with various abiotic or biotic stress factors). Plants having early vigour also show better establishment of the crop (with the crop growing in uniform manner, i.e. with the majority of plants reaching the various stages of development at substantially the same time), and show better growth and often better yield. Early vigour may be determined by measuring various factors, such as seedling growth rate, thousand kernel weight, percentage germination, percentage emergence, seedling height, root length and shoot biomass and many more.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats. Plants in which early vigour is a particularly desirably trait include rice, maize, wheat, sunflower, sorghum.

The present invention also encompasses use of nucleic acids encoding bHLH transcription factors and use of bHLH transcription factor polypeptides in enhancing yield-related traits.

Nucleic acids encoding bHLH transcription factor polypeptides, or bHLH transcription factors themselves, may find use in breeding programmes in which a DNA marker is identified which may be genetically linked to a bHLH transcription factor-encoding gene. The nucleic acids/genes, or the bHLH transcription factors themselves may be used to define a molecular marker. This DNA or protein marker may then be used in breeding programmes to select plants having increased yield as defined hereinabove in the methods of the invention.

Allelic variants of a bHLH transcription factor-encoding acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

A nucleic acid encoding a bHLH transcription factor may also be used as probes for genetically and physically mapping the genes that they are a part of, and as markers for traits linked to those genes. Such information may be useful in plant breeding in order to develop lines with desired phenotypes. Such use of bHLH transcription factor encoding nucleic acids requires only a nucleic acid sequence of at least 15 nucleotides in length. The bHLH transcription factor encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the bHLH transcription factor encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the bHLH transcription factor encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhnaced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Detailed description of SPL15

Surprisingly, it has now been found that increasing expression in a plant of a nucleic acid encoding an SPL15 transcription factor polypeptide gives plants having enhanced yield related traits, particularly increased yield, relative to control plants. Therefore, the invention provides a method for enhancing yield related traits in particular for increasing yield in plants relative to control plants, comprising increasing expression in a plant of a nucleic acid encoding an SPL15 transcription factor polypeptide.

A preferred method for increasing expression of a nucleic acid encoding a SPL15 transcription factor polypeptide is by introducing and expressing in a plant a nucleic acid encoding a SPL15 transcription factor polypeptide.

The term "SPL15 transcription factor polypeptide" as defined herein refers to a polypeptide comprising from N-terminal to C-terminal: (i) Motif 1 as represented by SEQ ID NO: 276; and (ii) in increasing order of preference at least 65%, 70%, 75%, 80%, 85%, 90%, 95% or 98% sequence identity to the SPL DBD represented by SEQ ID NO: 277; and (iii) Motif 2 as represented by SEQ ID NO: 278.

The most conserved amino acids within Motif 1 are XLXFGXXXYFX, and within Motif 2 DSXXALSLLSX (where X is a specified subset of amino acids differing for each position, as presented in SEQ ID NO: 276 and SEQ ID NO: 277). Within Motif 1 and Motif 2, are allowed one or more conservative change at any position, and/or one, two or three non-conservative change(s) at any position.

Additionally, the SPL15 transcription factor polypeptide may comprise any one or both of the following: (a) a G/S rich stretch preceding the SPL DBD; and (b) the W(S/T)L tripeptide at the C-terminal end of the polypeptide.

An example of an SPL15 transcription polypeptide as defined hereinabove comprising from N-terminal to C-terminal: (i) Motif 1 as represented by SEQ ID NO: 276; and (ii) in increasing order of preference at least 65%, 70%, 75%, 80%, 85%, 90%, 95% or 98% sequence identity to the SPL DBD represented by SEQ ID NO: 277; and (iii) Motif 2 as represented by SEQ ID NO: 278; and additionally comprising: (a) a G/S rich stretch preceding the SPL DBD; and (b) the W(S/T)L tripeptide at the C-terminal end of the polypeptide, is represented as in SEQ ID NO: 235. Further such examples are represented by any one of SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287 or orthologues or paralogues of any of the aforementioned SEQ ID NOs. The invention is illustrated by transforming plants with the *Arabidopsis thaliana* sequence represented by SEQ ID NO: 234, encoding the polypeptide of SEQ ID NO: 235. SEQ ID NO: 237 from *Arabidopsis thaliana* (encoded by SEQ ID NO: 236) is a paralogue of the polypeptide of SEQ ID NO: 235. SEQ ID NO: 239 (encoded by SEQ ID NO: 238, from *Aquilegia formosa x Aquilegia pubescens*), SEQ ID NO: 241 (encoded by SEQ ID NO: 240, from *Gossypium hirsutum*), SEQ ID NO: 243 (encoded by SEQ ID NO: 242, from *Ipomoea nil*), SEQ ID NO: 245 (encoded by SEQ ID NO: 244, from *Lactuca sativa*), SEQ ID NO: 247 (encoded by SEQ ID NO: 246, from *Malus domestica*), SEQ ID NO: 249 (encoded by SEQ ID NO: 248, from *Medicago truncatula*), SEQ ID NO: 251 (encoded by SEQ ID NO: 250, from *Nicotiana bentamiana*), SEQ ID NO: 253 (encoded by SEQ ID NO: 252, from *Oryza sativa*), SEQ ID NO: 255 (encoded by SEQ ID NO: 254, from *Oryza sativa*), SEQ ID NO: 257 (encoded by SEQ ID NO: 256, from *Solanum tuberosum* SEQ ID NO: 259 (encoded by SEQ ID NO: 258, from *Vitis vinifera*), SEQ ID NO: 261 (encoded by SEQ ID NO: 260, from Zea *mays*), SEQ ID NO: 263 (encoded by SEQ ID NO: 262, *Zea mays*), SEQ ID NO: 283 (encoded by SEQ ID NO: 282, *Brassica rapa*), SEQ ID NO: 285 (encoded by SEQ ID NO: 284, *Glycine max*), and SEQ ID NO: 287 (encoded by SEQ ID NO: 286, *Populus tremuloides*) are orthologues of the polypeptide of SEQ ID NO: 235.

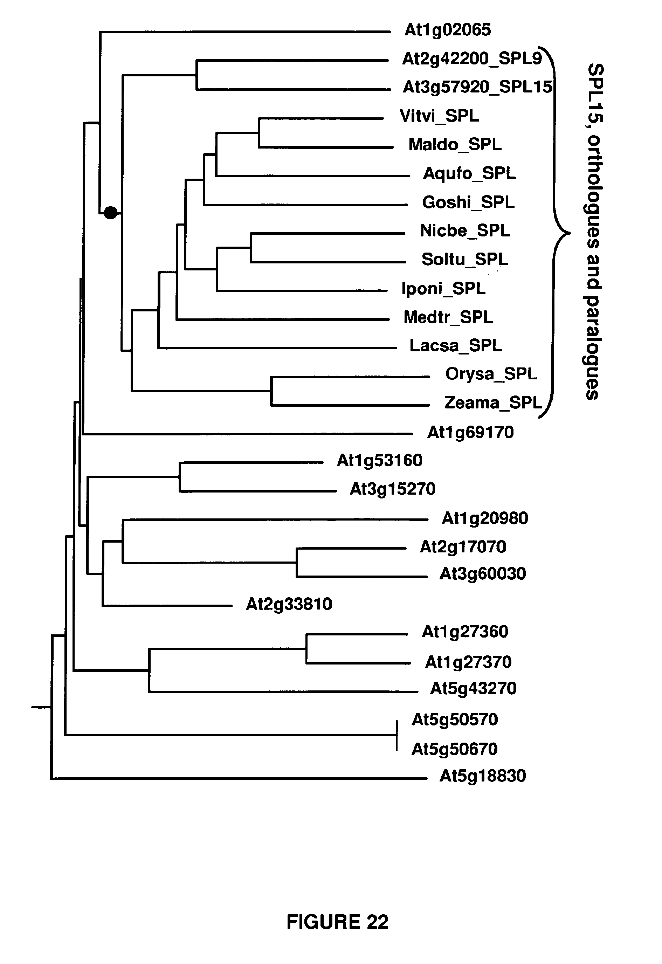

Orthologues and paralogues may easily be found by performing a so-called reciprocal blast search. This may be done by a first BLAST involving BLASTing a query sequence (for example, SEQ ID NO: 234 or SEQ ID NO: 235) against any sequence database, such as the publicly available NCBI database. BLASTN or TBLASTX (using standard default values) may be used when starting from a nucleotide sequence and BLASTP or TBLASTN (using standard default values) may be used when starting from a polypeptide sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 234 or SEQ ID NO: 235, the second BLAST would therefore be against *Arabidopsis* sequences). The results of the first and second BLASTs are then compared. A paralogue is identified if a high-ranking hit from the first BLAST is from the same species as from which the query sequence is derived, a BLAST back then ideally results in the query sequence as highest hit (besides itself); an orthologue is identified if a high-ranking hit in the first BLAST is not from the same species as from which the query sequence is derived and preferably results upon BLAST back in the query sequence amongst the highest hits. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. An example detailing the identification of orthologues and paralogues is given in Example 41. In the case of large families, ClustalW may be used, followed by a neighbour joining tree, to help visualize clustering of related genes and to identify orthologues and paralogues. In Figure 22, the SPL15 transcription factor polypeptide paralogues and orthologues cluster together.

The polypeptides represented by any one of SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287, or orthologues or paralogues of any of the aforementioned SEQ ID NOs, all comprise an SPL DBD having, in increasing order of preference, at least 65%, 70%, 75%, 80%, 85%, 90%, 95% or 98% sequence identity to the SPL15 DBD represented by SEQ ID NO: 277.

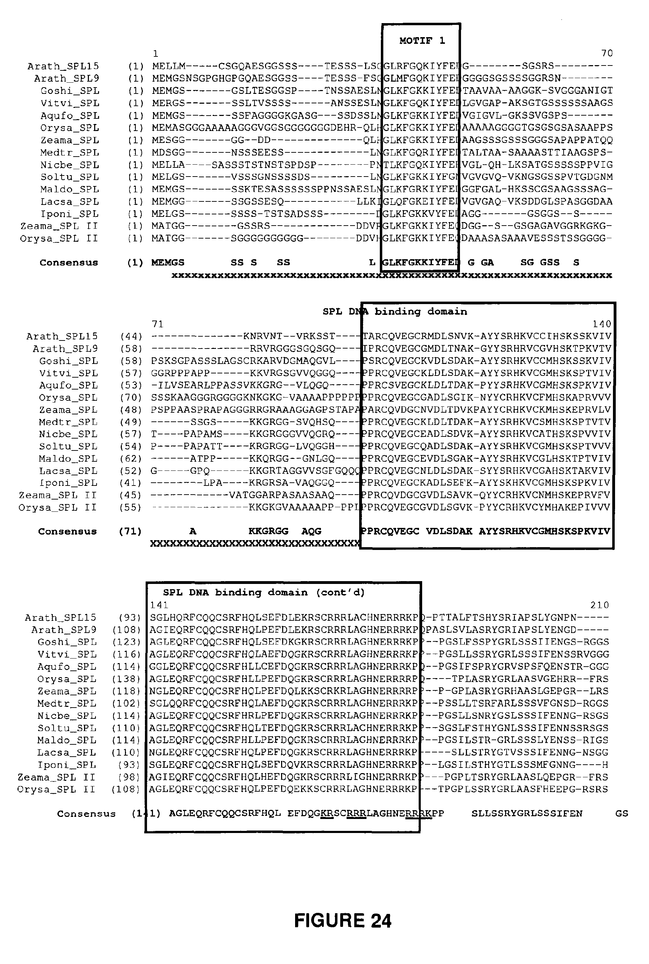

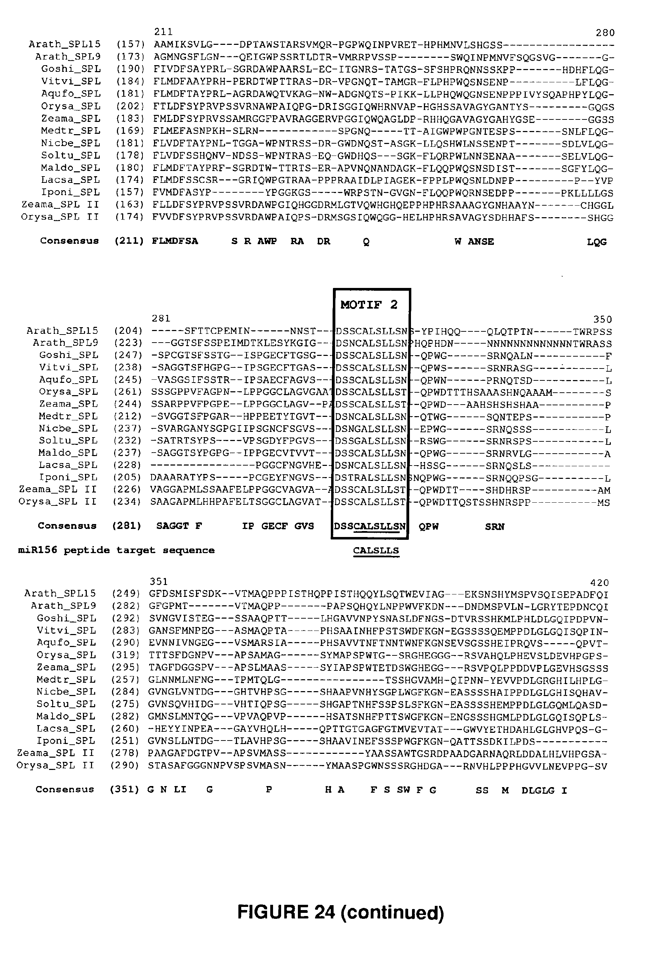

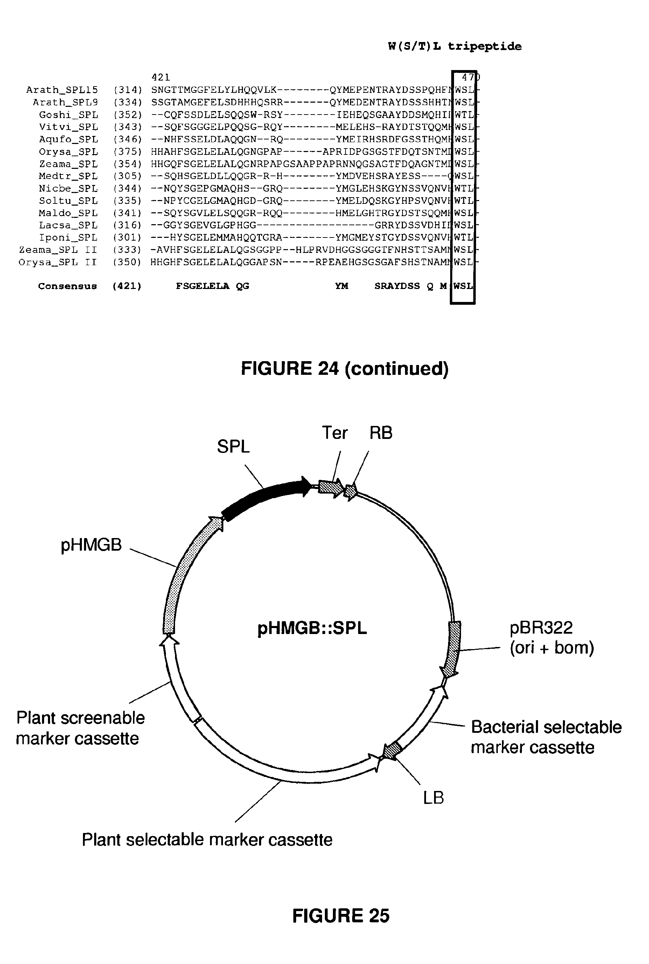

SPL DBDs are well known in the art and may readily be identified by persons skilled in the art. A SPL DBD may be identified using methods for the alignment of sequences for comparison. Methods for the alignment of sequences for comparison include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83) available at GenomeNet service at the Kyoto University Bioinformatics Center, with the default pairwise alignment parameters, and a scoring method in percentage. Minor manual editing may be performed to optimise alignment between conserved motifs, as would be apparent to a person skilled in the art. The alignment shown in Figures 23 and 24 highlight the SPL DBD in SPL15 transcription factor polypeptides. Preferably, SPL15 transcription factor polypeptides useful in the methods of the invention comprise an SPL DBD having, in increasing order of preference, at least 65%, 70%, 75%, 80%, 85%, 90%, 95% or 98% sequence identity to the SPL DBD represented by SEQ ID NO: 277.

In some instances, default parameters may be adjusted to modify the stringency of the search. For example using BLAST, the statistical significance threshold (called "expect" value) for reporting matches against database sequences may be increased to show less stringent matches. In this way, short nearly exact matches may be identified. Motif 1 as represented by SEQ ID NO: 276 and Motif 2 as represented by SEQ ID NO: 278 both comprised in the SPL15 transcription factor polypeptides useful in the methods of the invention may be identified this way (Figure 24). Within Motif 1 and Motif 2, are allowed one or more conservative change at any position, and/or one, two or three non-conservative change(s) at any position. The W(S/T)L tripeptide at the C-terminal end of the polypeptide may likewise be identified (Figure 24).

Special databases also exisit for the identification of domains. The SPL DBD in a SPL15 transcription factor polypeptide may be identified using, for example, SMART (Schultz et al. (1998) Proc. Natl. Acad. Sci. USA 95, 5857-5864; Letunic et al. (2002) Nucleic Acids Res 30, 242-244; hosted by the EMBL at Heidelberg, Germany), InterPro (Mulder et al., (2003) Nucl. Acids. Res. 31, 315-318; hosted by the European Bioinformatics Institute (EBI) in the United Kingdom), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32: D134-D137, (2004), The ExPASy proteomics server is provided as a service to the scientific community (hosted by the Swiss Institute of Bioinformatics (SIB) in Switzerland) or Pfam (Bateman et al., Nucleic Acids Research 30(1): 276-280 (2002), hosted by the Sanger Institute in the United Kingdom). The SPL DBD in the InterPro database is designated IPR004333, PF03110 in the Pfam database and PS51141 in the PROSITE database.

Furthermore, the presence of G/S rich stretch preceding the SPL DBD may also readily be identified (Figure 24). Primary amino acid composition (in %) to determine if a polypeptide domain is rich in specific amino acids may be calculated using software programs from the ExPASy server, in particular the ProtParam tool (Gasteiger E et al. (2003) ExPASy: the proteomics server for in-depth protein knowledge and analysis. Nucleic Acids Res 31:3784-3788). The composition of the protein of interest may then be compared to the average amino acid composition (in %) in the Swiss-Prot Protein Sequence data bank. Within this databank, the average Gly (G) and Ser (E) content are both of 6.9% (adding up to 13.8%). As an example, the G/S rich stretch preceding the SPL DBD of SEQ ID NO: 235 contains 14.5 % of G and 25.5 % of S (adding up to 40%). As defined herein, a G/S rich stretch has a combined G and S content (in % terms) above that found in the average amino acid composition (in % terms) of the proteins in the Swiss-Prot Protein Sequence. Both G and S belong to the category of very small amino acids.

The nucleic acid encoding the polypeptides represented by any one of SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287, or orthologues or paralogues of any of the aforementioned SEQ ID NOs, need not be full-length nucleic acids, since performance of the methods of the invention does not rely on the use of full length nucleic acid sequences. Furthermore, examples of nucleic acids suitable for use in performing the methods of the invention include but are not limited to those represented by any one of: SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286. Nucleic acid variants may also be useful in practising the methods of the invention. Examples of such variants include portions of nucleic acids, splice variants, allelic variants either naturally occurring or obtained by DNA manipulation.

A portion may be prepared, for example, by making one or more deletions to a nucleic acid encoding a SPL15 transcription factor polypeptide as defined hereinabove. The portions may be used in isolated form or they may be fused to other coding (or non coding) sequences in order to, for example, produce a protein that combines several activities. When fused to other coding sequences, the resultant polypeptide produced upon translation may be bigger than that predicted for the SPL15 transcription factor portion. Portions useful in the methods of the invention, encode an SPL15 transcription factor polypeptide (as described above) and having substantially the same biological activity as the SPL15 transcription factor polypeptide represented by any of SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287, or orthologues or paralogues of any of the aforementioned SEQ ID NOs. Examples of portions may include the nucleotides encoding Motif 1 as represented by SEQ ID NO: 276, in increasing order of preference at least 65%, 70%, 75%, 80%, 85%, 90%, 95% or 98% sequence identity to the SPL DBD represented by SEQ ID NO: 277, and Motif 2 as represented by SEQ ID NO: 278. Portions may additionally include nucleotides encoding the G/S rich stretch or the W(S/T)L tripeptide (but not necessarily the nucleotides encoding the amino acid sequences between any of these). The portion is typically at least 250 nucleotides in length, preferably at least 500 nucleotides in length, more preferably at least 750 nucleotides in length and most preferably at least 1000 nucleotides in length. Preferably, the portion is a portion of a nucleic acid as represented by any one of SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286. Most preferably the portion is a portion of a nucleic acid as represented by SEQ ID NO: 234.

Another nucleic acid variant useful in the methods of the invention, is a nucleic acid capable of hybridising under reduced stringency conditions, preferably under stringent conditions, with a nucleic acid encoding a SPL15 transcription factor polypeptide as defined hereinabove, or a with a portion as defined hereinabove.

Hybridising sequences useful in the methods of the invention, encode a polypeptide comprising from N-terminal to C-terminal: (i) Motif 1 as represented by SEQ ID NO: 276; and (ii) in increasing order of preference at least 65%, 70%, 75%, 80%, 85%, 90%, 95% or 98% sequence identity to the SPL DBD represented by SEQ ID NO: 277; and (iii) Motif 2 as represented by SEQ ID NO: 278, and having substantially the same biological activity as the SPL15 transcription factor polypeptides represented by SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287, or orthologues or paralogues of any of the aforementioned SEQ ID NOs. The hybridising sequence is typically at least 250 nucleotides in length, preferably at least 500 nucleotides in length, more preferably at least 750 nucleotides in length and most preferably at least 1000 nucleotides in length. Preferably, the hybridising sequence is one that is capable of hybridising to any of the nucleic acids represented by SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286, or to a portion of any of the aforementioned sequences, a portion being as defined above. Most preferably the hybridising sequence is capable of hybridising to SEQ ID NO: 234, or to portions thereof.

Another nucleic acid variant useful in the methods of the invention is a splice variant encoding a SPL15 transcription factor polypeptide as defined hereinabove.

Preferred splice variants are splice variants of a nucleic acid encoding SPL15 transcription factor polypeptide represented by any of SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, or SEQ ID NO: 287, or splice variants encoding orthologues or paralogues of any of the aforementioned SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286. Most preferred is a splice variant of a nucleic acid as represented by SEQ ID NO: 234.

Another nucleic acid variant useful in performing the methods of the invention is an allelic variant of a nucleic acid encoding a SPL15 transcription factor polypeptide as defined hereinabove.

The allelic variant may be an allelic variant of a nucleic acid encoding a SPL15 transcription factor polypeptide represented by any of SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, or SEQ ID NO: 287, or an allelic variant of a nucleic acid encoding orthologues or paralogues of any of the aforementioned SEQ ID NOs. Further preferred are allelic variants of nucleic acids represented by any one of SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286. Most preferred is an allelic variant of a nucleic acid as represented by SEQ ID NO: 234.

A further nucleic acid variant useful in the methods of the invention is a nucleic acid variant obtained by gene shuffling. Gene shuffling or directed evolution may also be used to generate variants of nucleic acids encoding SPL15 transcription factor polypeptides as defined above.

Furthermore, nucleic acid variants may also be obtained by site-directed mutagenesis. Several methods are available to achieve site-directed mutagenesis, the most common being PCR based methods (Current Protocols in Molecular Biology. Wiley (Eds)).

Also useful in the methods of the invention are nucleic acids encoding homologues of any one of the amino acids represented by SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261, SEQ ID NO: 263, SEQ ID NO: 283, SEQ ID NO: 285, SEQ ID NO: 287, or orthologues or paralogues of any of the aforementioned SEQ ID NOs.

Also useful in the methods of the invention are nucleic acids encoding derivatives of any one of the amino acids represented by SEQ ID NO: 235, SEQ ID NO: 237, SEQ ID NO: 239, SEQ ID NO: 241, SEQ ID NO: 243, SEQ ID NO: 245, SEQ ID NO: 247, SEQ ID NO: 249, SEQ ID NO: 251, SEQ ID NO: 253, SEQ ID NO: 255, SEQ ID NO: 257, SEQ ID NO: 259, SEQ ID NO: 261 SEQ ID NO: 263, SEQ ID NO: 283, SEQ ID NO: 285, SEQ ID NO: 287, or orthologues or paralogues of any of the aforementioned SEQ ID NOs. "Derivatives" include peptides, oligopeptides, polypeptides which may, compared to the amino acid sequence of the naturally-occurring form of the protein, such as the one presented in SEQ ID NO: 235, comprise substitutions of amino acids with non-naturally occurring amino acid residues, or additions of non-naturally occurring amino acid residues.

Furthermore, SPL15 transcription factor polypeptides (at least in their native form) typically have DNA-binding activity and an activation domain. A person skilled in the art may easily determine the presence of an activation domain and DNA-binding activity using routine tools and techniques.

Nucleic acids encoding SPL15 transcription factor polypeptides may be derived from any natural or artificial source. The nucleic acid may be modified from its native form in composition and/or genomic environment through deliberate human manipulation. Preferably the SPL15 transcription factor polypeptide-encoding nucleic acid is from a plant, further preferably from a dicot, more preferably from the *Brassicacea* family, most preferably the nucleic acid is from *Arabidopsis thaliana.*

Any reference herein to a SPL15 transcription factor polypeptide is therefore taken to mean a SPL15 transcription factor peptide as defined above. Any nucleic acid encoding such a SPL15 transcription factor polypeptide is suitable for use in performing the methods of the invention.

The invention also provides genetic constructs and vectors to facilitate introduction and/or expression of the nucleic acid sequences useful in the methods according to the invention, in a plant.

Therefore, there is provided a gene construct comprising:
(i) A nucleic acid encoding a SPL15 transcription factor polypeptide as defined hereinabove;
(ii) One or more control sequences operably liked to the nucleic acid of (i).

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding a SPL15 transcription factor polypeptide). The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. The promoter may be an inducible promoter, i.e. having induced or increased transcription initiation in response to a developmental, chemical, environmental or physical stimulus. The promoter may be a tissue-specific promoter, i.e. one that is capable of preferentially initiating transcription in certain tissues, such as the leaves, roots, seed tissue etc.

According to the invention, the nucleic acid encoding a SPL15 transcription factor polypeptide is operably linked to a constitutive promoter. The constitutive promoter is preferably a HMGB (high mobility group B) promoter, more preferably the constitutive promoter is a rice HMGB promoter, further preferably the constitutive promoter is represented by a nucleic acid sequence substantially similar to SEQ ID NO: 279, most preferably the constitutive promoter is as represented by SEQ ID NO: 279 or SEQ ID NO: 294.

It should be clear that the applicability of the present invention is not restricted to the nucleic acid encoding an SPL15 transcription factor polypeptide as represented by SEQ ID NO: 234, nor is the applicability of the invention restricted to expression of a such nucleic acid encoding an SPL15 transcription factor polypeptide when driven by a HMGB promoter. Examples of other constitutive promoters which may also be used perform the methods of the invention are shown in the definitions section.

Additional regulatory elements for increasing expression of nucleic acids or genes, or gene products, may include transcriptional as well as translational enhancers. Those skilled in the art will be aware of terminator and enhancer sequences that may be suitable for use in performing the invention. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art. Optionally, one or more terminator sequences (also a control sequence) may be used in the construct introduced into a plant.

An intron sequence may also be added to the 5' untranslated region (UTR) or the coding sequence of the partial coding sequence to increase the amount of the mature message that accumulates in the cytosol.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene.

The invention also provides a method for the production of transgenic plants having increased yield relative to control plants, comprising introduction and expression in a plant of a nucleic acid encoding a SPL15 transcription factor polypeptide as defined hereinabove.

More specifically, the present invention provides a method for the production of transgenic plants having increased yield relative to control plants, which method comprises:
(i) introducing and expressing a nucleic acid encoding a SPL15 transcription factor polypeptide in a plant cell; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation. Generally after transformation, plant cells or cell groupings are selected for the presence of one or more markers which are encoded by plant-expressible genes co-transferred with the gene of interest, following which the transformed material is regenerated into a whole plant. Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a SPL15 transcription factor polypeptide as defined hereinabove. Preferred host cells according to the invention are plant cells.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

As mentioned above, a preferred method for increasing expression of a nucleic acid encoding a SPL15 transcription factor polypeptide is by introducing and expressing in a plant a nucleic acid encoding a SPL15 transcription factor polypeptide; however the effects of performing the method, i.e. increasing yield, may also be achieved using other well known techniques. A description of some of these techniques will now follow.

One such technique is T-DNA activation tagging. The resulting transgenic plants show dominant phenotypes due to modified expression of genes close to the introduced promoter.

The effects of the invention may also be reproduced using the technique of TILLING (Targeted Induced Local Lesions In Genomes).

The effects of the invention may also be reproduced using homologous recombination.

"Increased yield" as defined herein is taken to mean an increase in biomass (weight) of one or more parts of a plant, which may include aboveground (harvestable) parts and/or (harvestable) parts below ground.

In particular, such harvestable parts include vegetative biomass and/or seeds, and performance of the methods of the invention results in plants having increased yield (in vegetative biomass and/or seed) relative to the yield of control plants.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises increasing expression in a plant of a nucleic acid encoding a SPL15 transcription factor polypeptide

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a MADS15 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a MADS15 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

The methods of the invention are advantageously applicable to any plant.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The present invention also encompasses plants obtainable by the methods according to the present invention. The present invention therefore provides plants, parts and cells from such plants obtainable by the methods according to the present invention, which plants or parts or cells comprise a nucleic acid transgene encoding a SPL15 transcription factor polypeptide as defined above.

The present invention also encompasses use of nucleic acids encoding SPL15 transcription factor polypeptides in increasing yield in a plant compared to yield in a control plant.

One such use relates to increasing yield of plants, yield being defined as defined herein above. Yield may in particular include one or more of the following: increased aboveground biomass, increased number of flowers per panicle, increased seed yield, increased total number of seeds, increased number of filled seeds, increased thousand kernel weight (TKW) and increased harvest index.

Nucleic acids encoding SPL15 transcription factor polypeptides may find use in breeding programmes in which a DNA marker is identified which may be genetically linked to a gene encoding SPL15 transcription factor polypeptide. Nucleic acids encoding SPL15 transcription factor polypeptides may be used to define a molecular marker. This marker may then be used in breeding programmes to select plants having increased seed yield. The nucleic acids encoding SPL15 transcription factor polypeptides may be, for example, a nucleic acid as represented by any one of SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286.

Allelic variants of a nucleic acid encoding an SPL15 transcription factor polypeptide may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased seed yield. Selection is typically carried out by monitoring growth performance of plants containing different allelic variants of the sequence in question, for example, different allelic variants of any one of SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 250, SEQ ID NO: 252, SEQ ID NO: 254, SEQ ID NO: 256, SEQ ID NO: 258, SEQ ID NO: 260, SEQ ID NO: 262, SEQ ID NO: 282, SEQ ID NO: 284 and SEQ ID NO: 286. Growth performance may be monitored in a greenhouse or in the field. Further optional steps include crossing plants, in which the superior allelic variant was identified, with another plant. This could be used, for example, to make a combination of interesting phenotypic features.

Nucleic acids encoding SPL15 transcription factor polypeptides may also be used as probes for genetically and physically mapping the genes that they are a part of, and as markers for traits linked to those genes. Such information may be useful in plant breeding in order to develop lines with desired phenotypes. Such use of nucleic acids encoding SPL15 transcription factor polypeptides requires only a nucleic acid sequence of at least 15 nucleotides in length. The nucleic acids encoding SPL15 transcription factor polypeptides may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with a nucleic acid encoding SPL15 transcription factor polypeptide. The resulting banding patterns may then be subjected to genetic analyses using computer programs such as MapMaker (Lander et al. (1987) Genomics 1: 174-181) in order to construct a genetic map. In addition, the nucleic acid may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding SPL15 transcription factor polypeptide in the genetic map previously obtained using this population (Botstein et al. (1980) Am. J. Hum. Genet. 32: 314-331).

The production and use of plant gene-derived probes for use in genetic mapping is described in Bernatzky and Tanksley (GENETICS 112 (4): 887-898, 1986). Numerous publications describe genetic mapping of specific cDNA clones using the methodology outlined above or variations thereof. For example, F2 intercross populations, backcross populations, randomly mated populations, near isogenic lines (NIL), and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridization (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et a/. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield, as described hereinbefore. These traits may also be combined with other economically advantageous traits, such as further yield-increasing traits, tolerance to other abiotic and biotic stresses, traits modifying various architectural features and/or biochemical and/or physiological features.

### Description of figures

The present invention will now be described with reference to the following figures in which:
Fig. 1 represents the schematic structure of the AtHAL3a polypeptide comprising from N-terminus to C-terminus (i) the substrate binding helix (single underlined), (ii) the insertion His motif (double underlined), (iii) the PXMNXXMW motif (dotted) and (iv) the substrate recognition clamp (wave underlined). The N-terminal and C-terminal ends of the HAL3 proteins are not very conserved.
**Fig. 2** shows a binary vector pEXP::HAL3, for increased expression in *Oryza sativa* of an *Arabidopsis thaliana* HAL3 nucleic acid under the control of a beta expansin promoter.

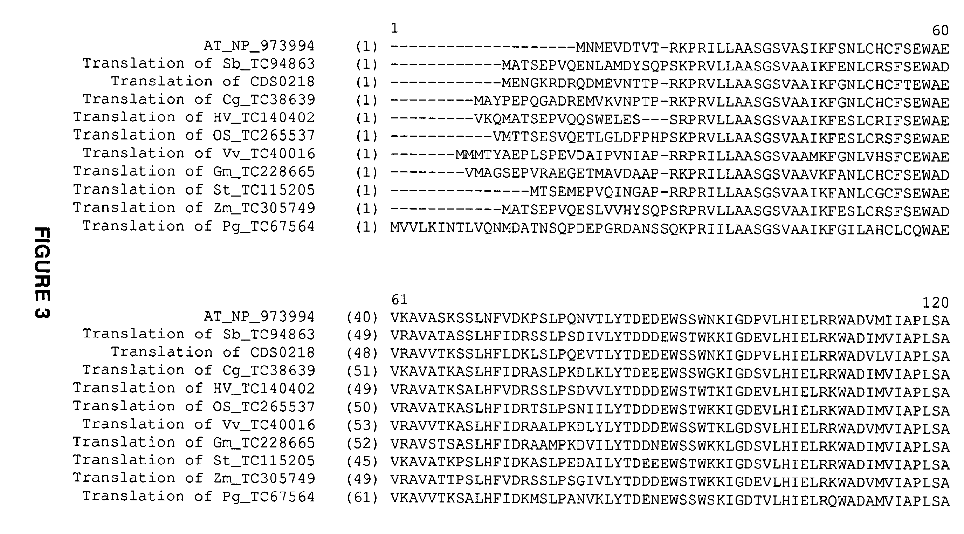

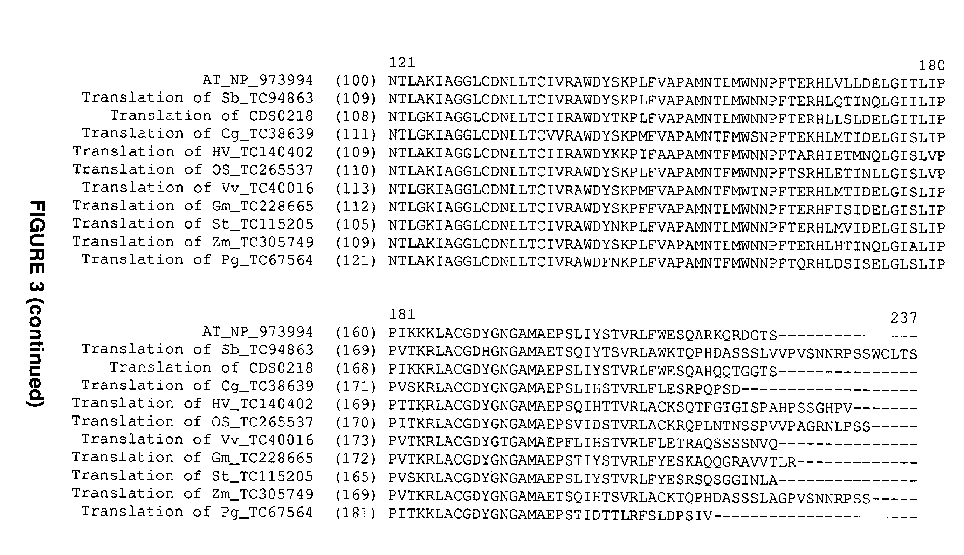

**Fig. 3** shows a multiple alignment of a number of HAL3 sequences from *Arabidopsis thaliana* AtHAL3b (At_NP_97S994), *Sorghum bicolor* (Sb), *Arabidopsis thaliana* AtHAL3a (Ath0218), *Gossipium hirsutum* (Cg), *Hordeum vulgare* (Hv), *Oryza sativa* (Os), *Vitis vinifera* (Vv), *Glycine max* (Gm), *Solanum tuberosum* (St), *Zea mays* (Zm), and *Pinus* sp (Pg).
**Fig. 4** details examples of sequences useful in performing the methods according to the present invention: SEQ ID NO: 1 and SEQ ID NO: 2 represent the nucleic acid sequence and the protein sequence of AtHAl3a. SEQ ID NO: 3 and SEQ ID NO: 4 are the sequences of the forward and reverse primers used to isolate the *AtHAL3* gene. SEQ ID NO: 5 is the sequence of the beta expansin promoter used in the methods of the present invention. SEQ ID NO: 9 to SEQ ID NO: 42 represent examples of full length or partial DNA/protein sequences, useful in the methods of the invention or for isolating corresponding full length sequences. In some cases, sequences were assembled from EST sequences, with lesser quality sequencing. As a consequence, a few nucleic acid substitutions may be expected.
**Fig. 5**(A) shows the domain structure of the MADS15 protein, (B) represents the sequence of SEQ ID NO: 44 with the MADS domain in bold and the keratin domain in italics. Between the MADS domain and the keratin, the intervening domain is located while the C-domain is located C-terminally of the keratin domain.
**Fig. 6** shows a multiple alignment of various MADS15 proteins. The asterisks indicate identical amino acids, the colons represent highly conservative substitutions, the dots represent less conserved substitutions.
**Fig. 7** shows the binary vector for increased expression in *Oryza sativa* of an *Oryza sativa* MADS 15 protein-encoding nucleic acid under the control of a GOS2 promoter.

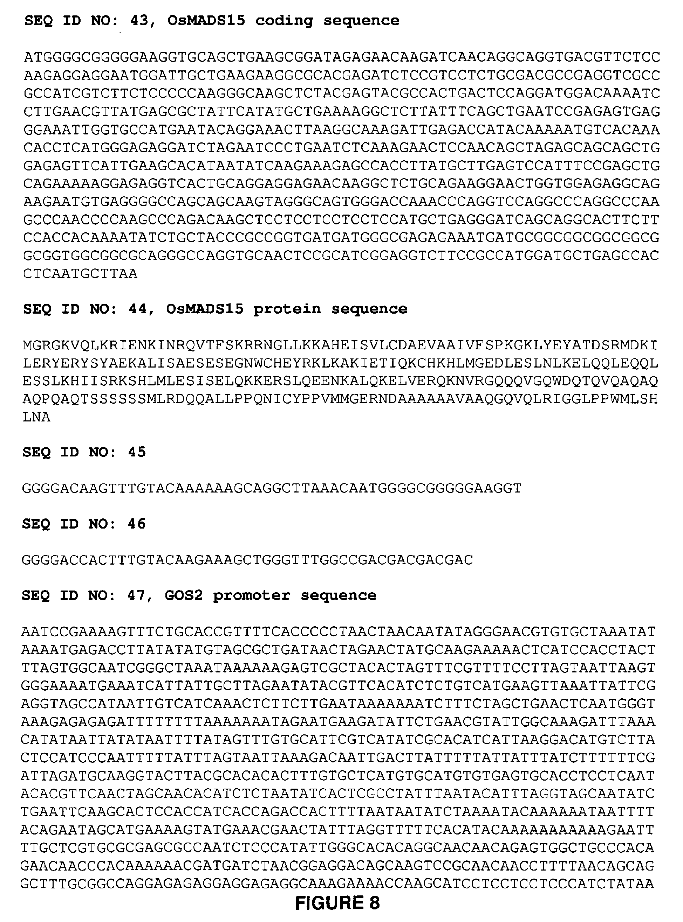

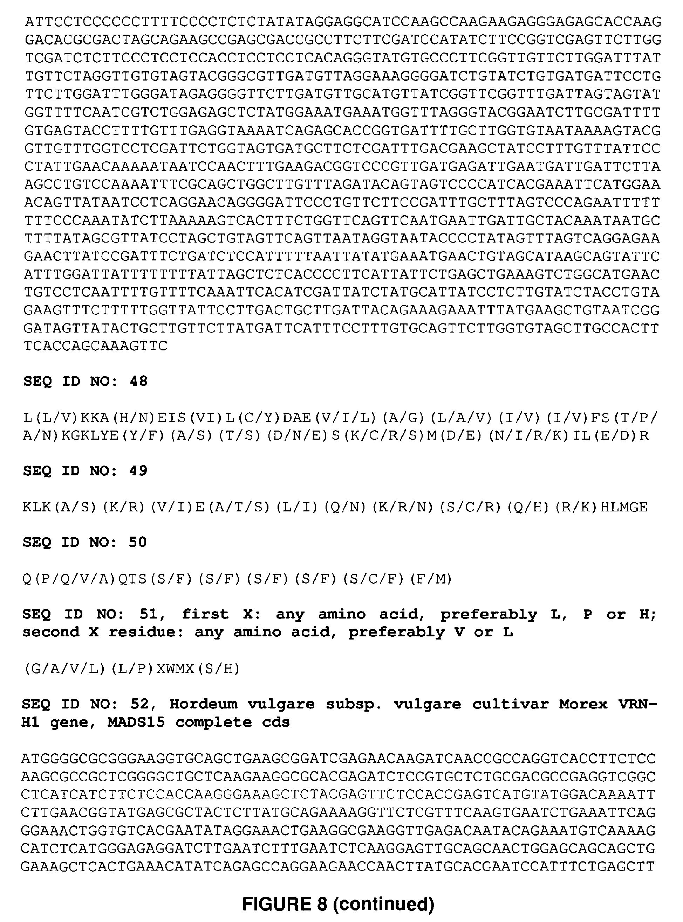

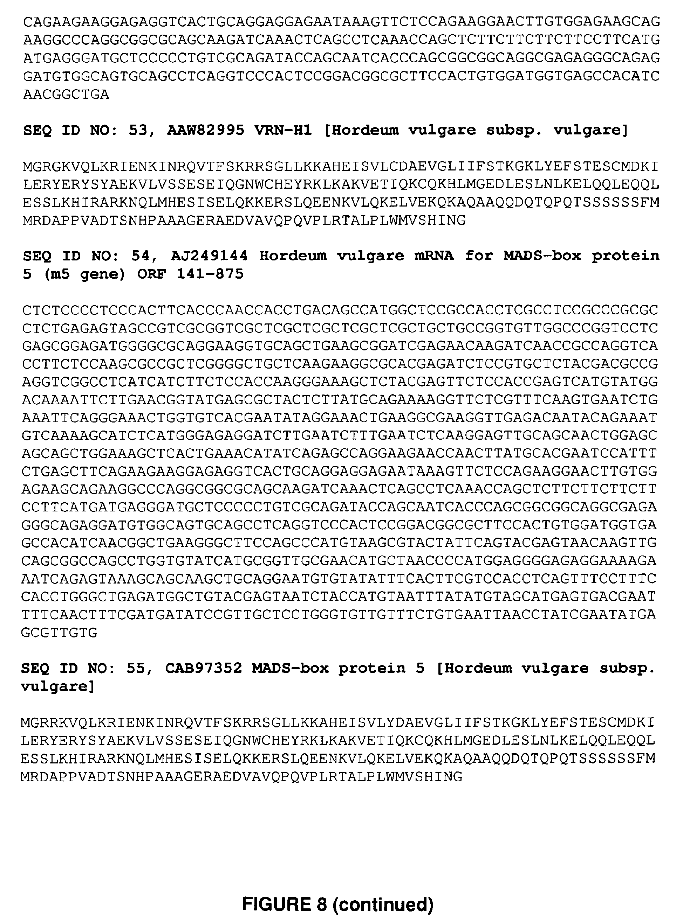

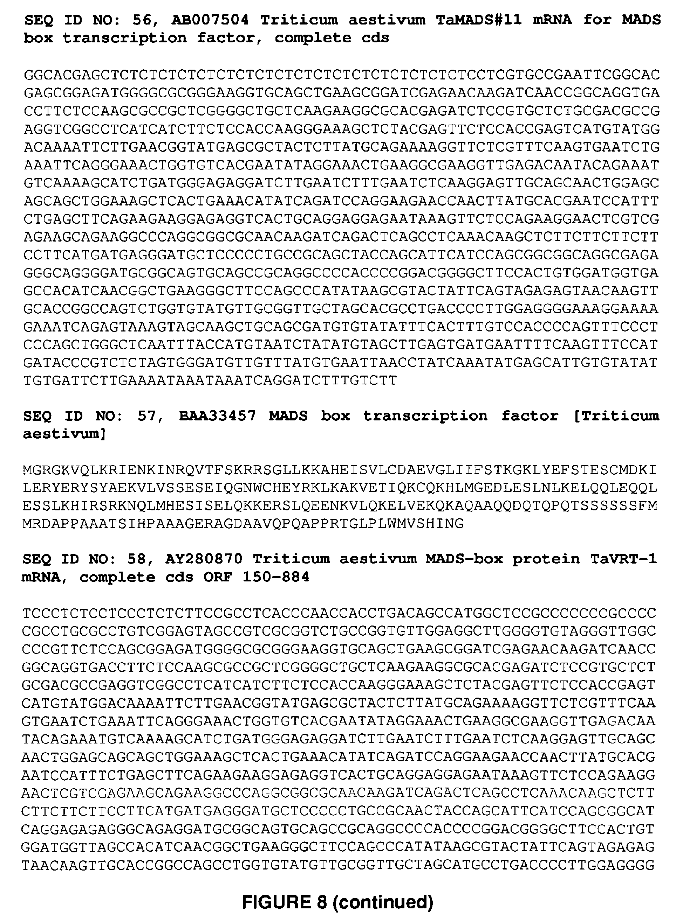

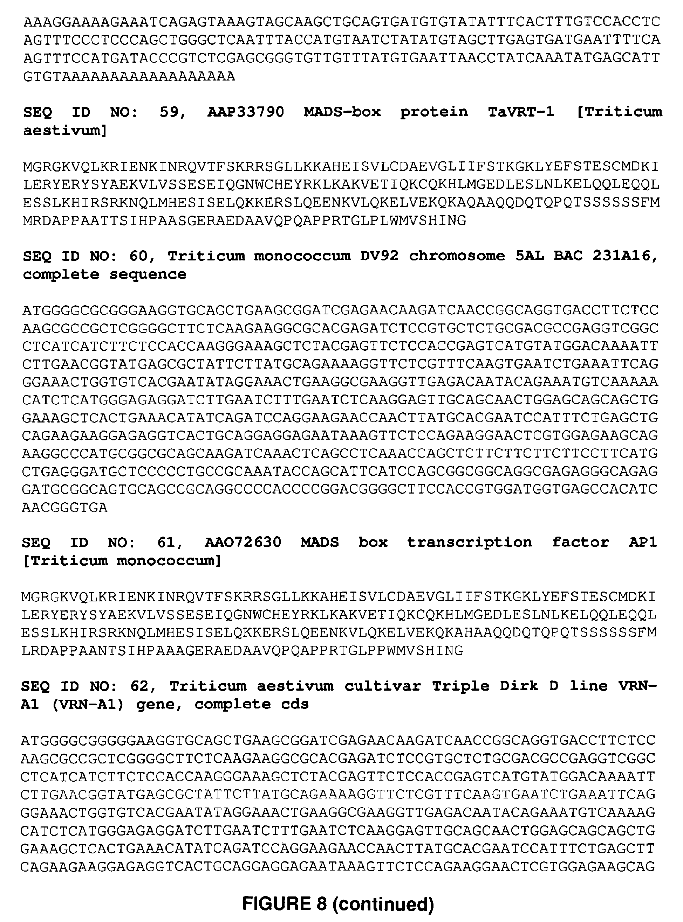

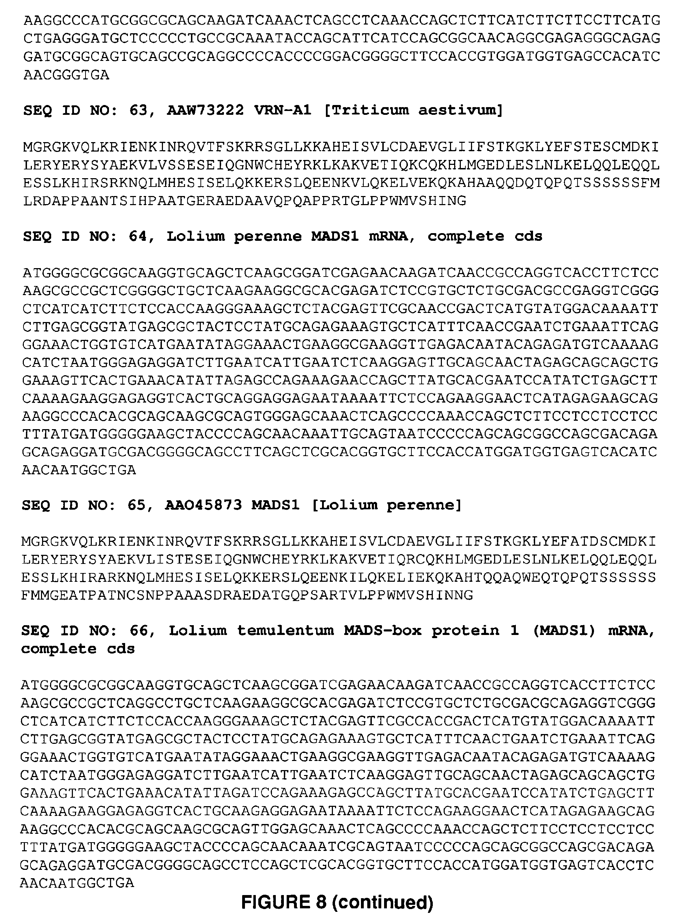

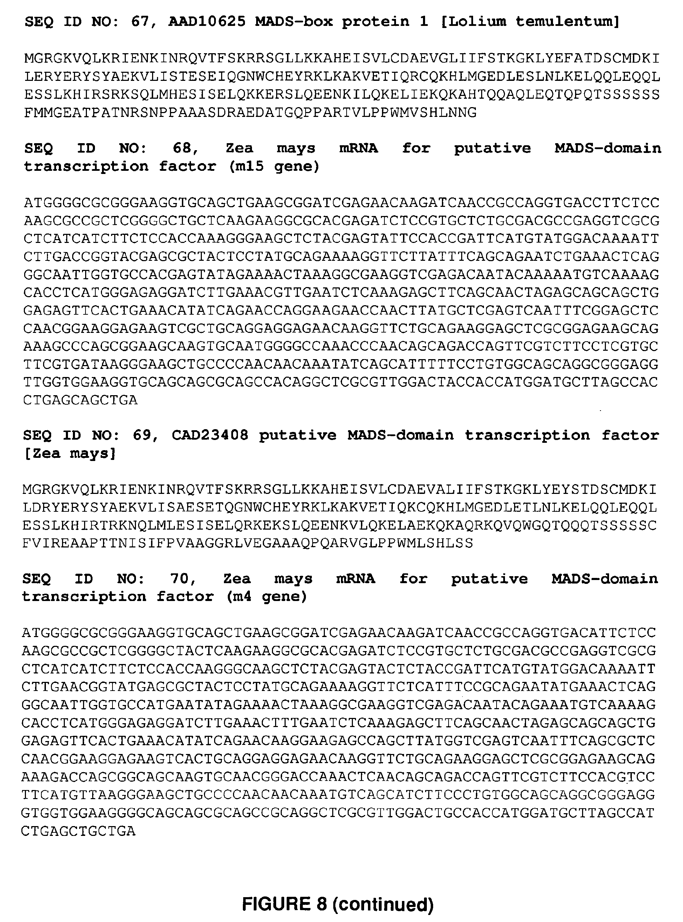

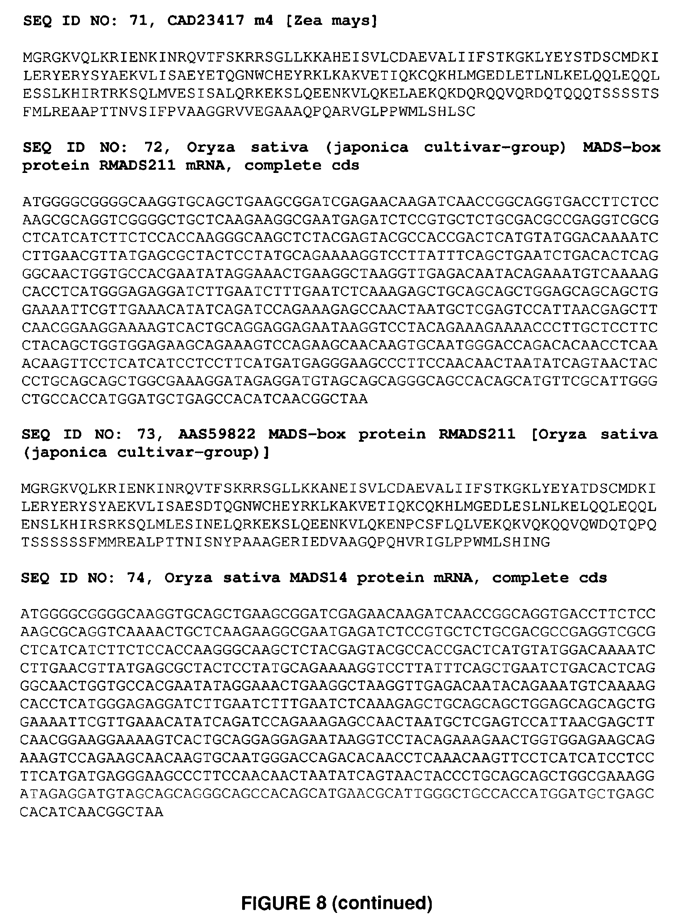

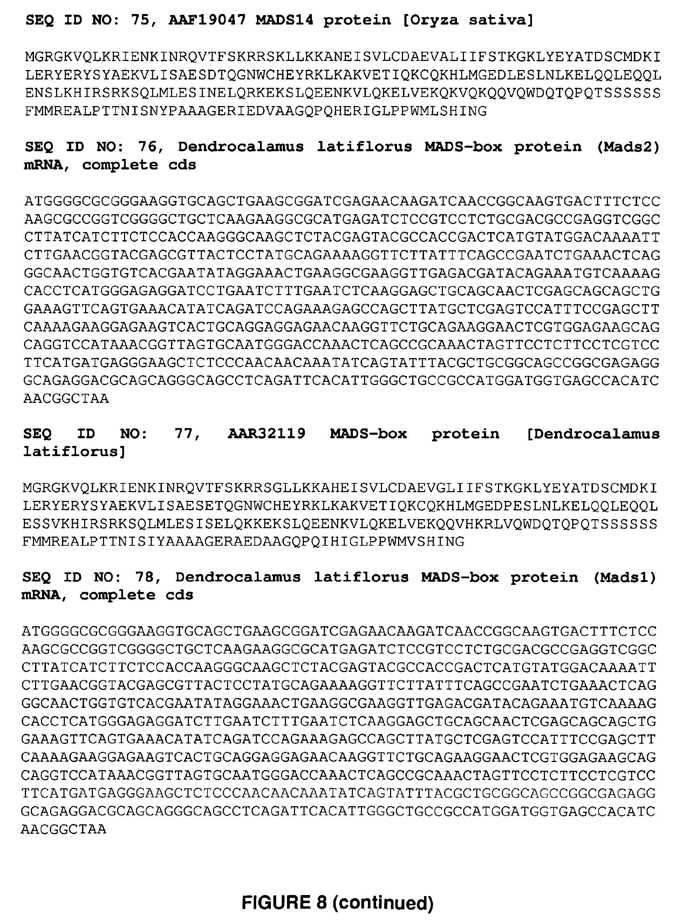

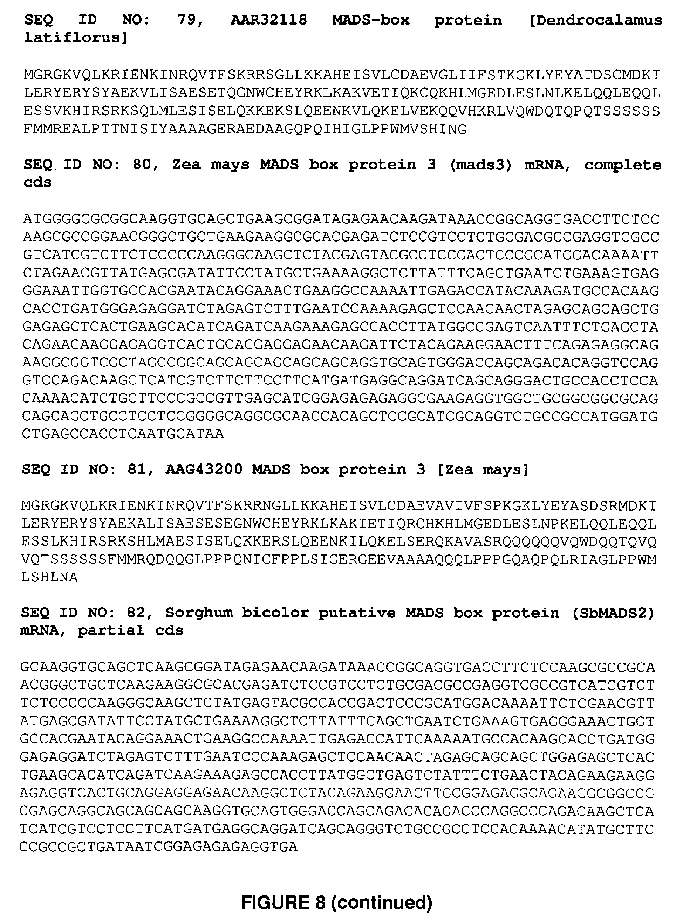

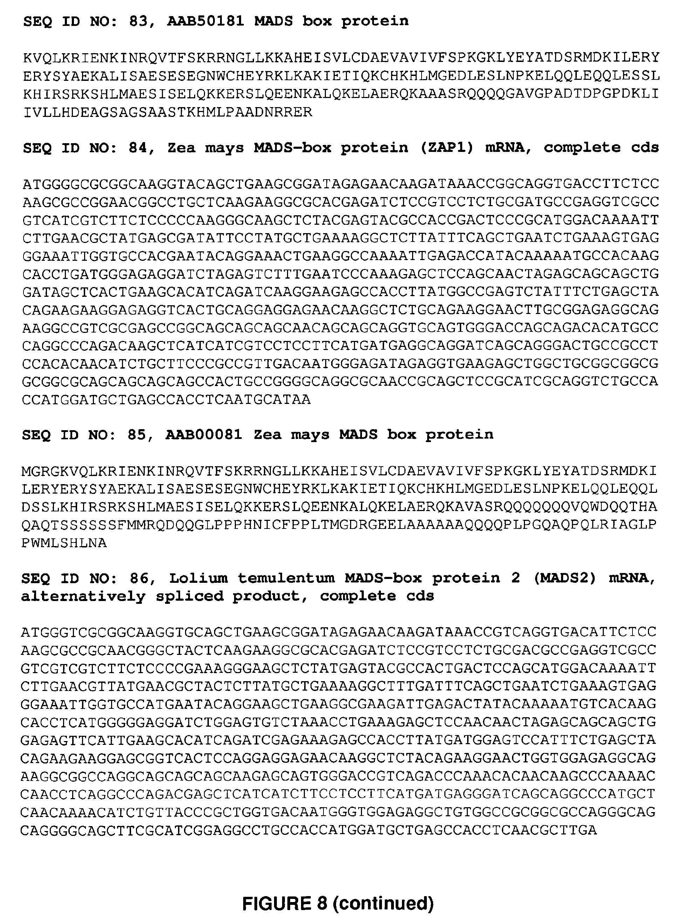

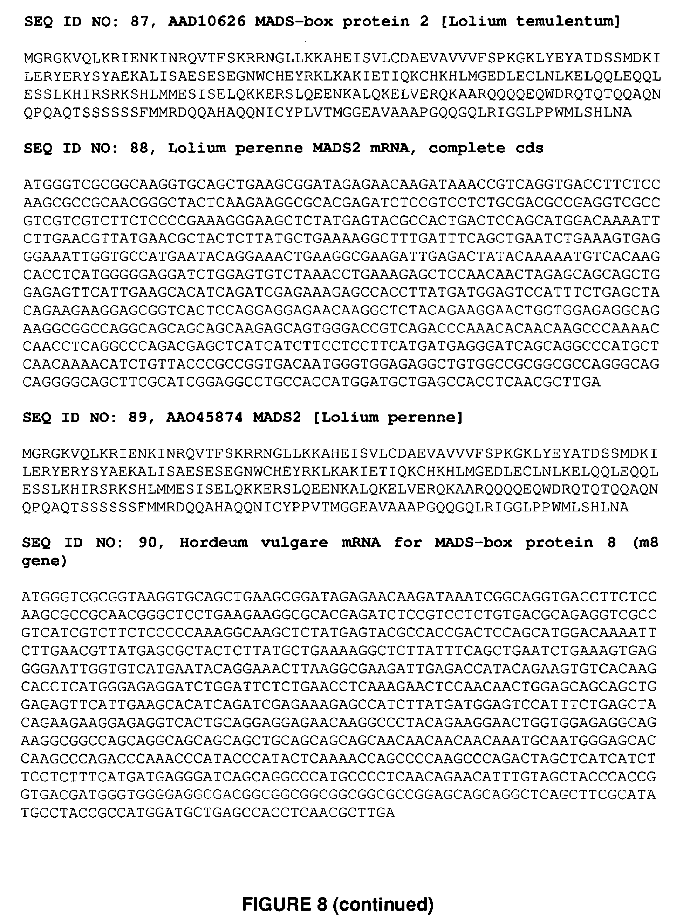

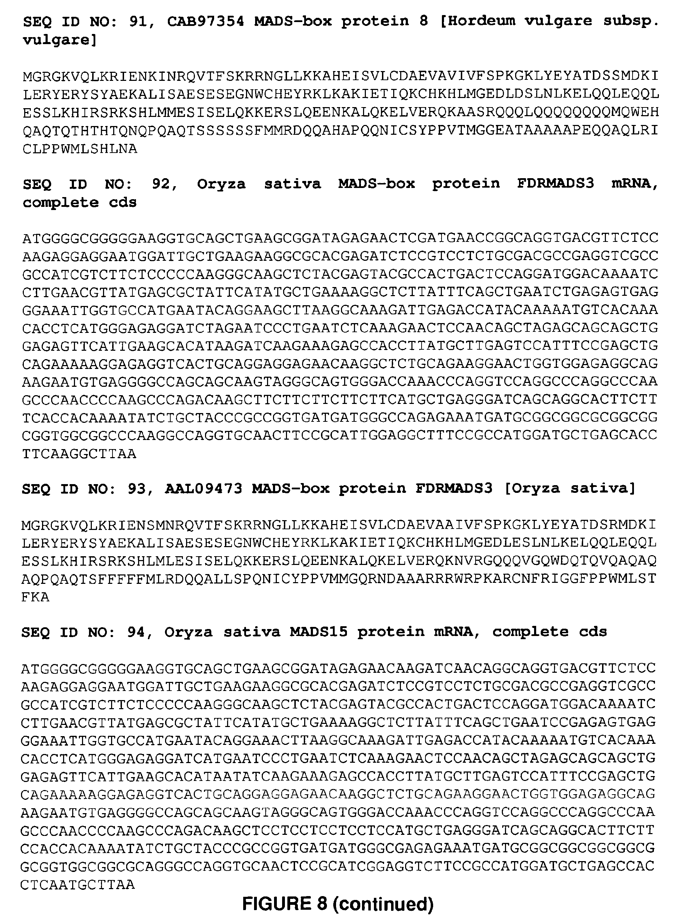

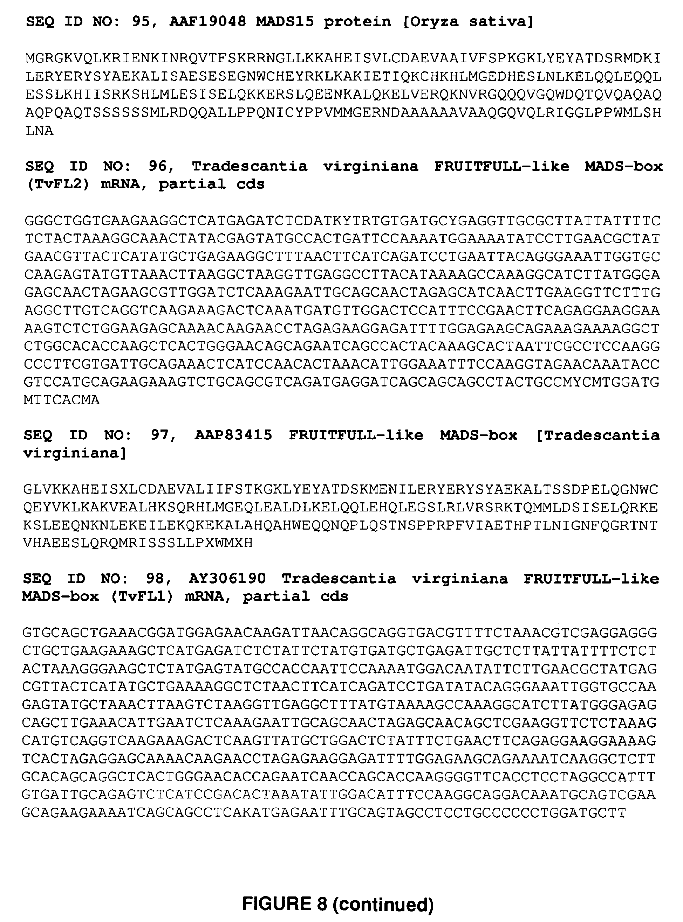

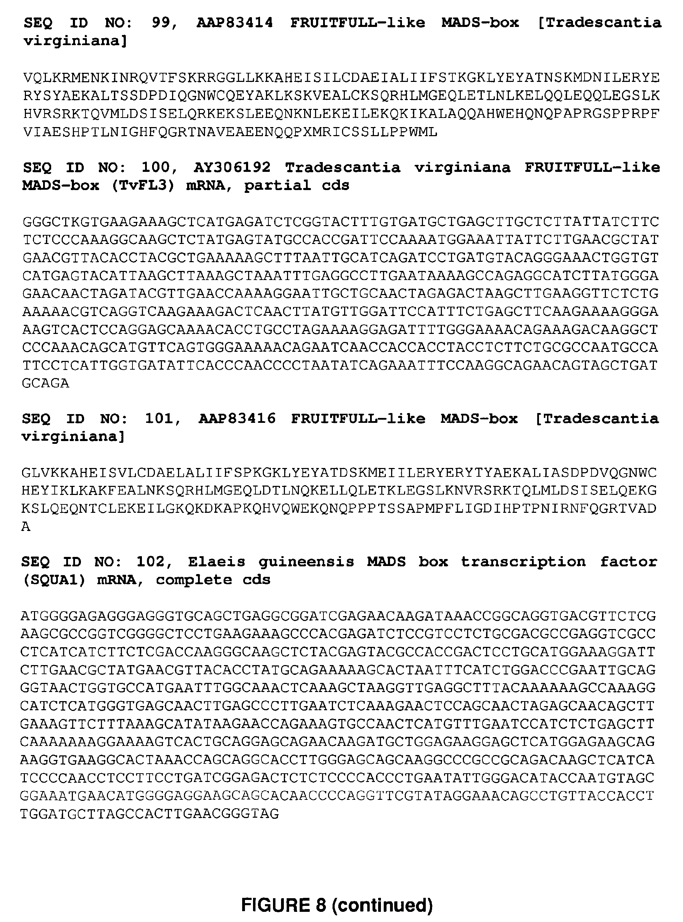

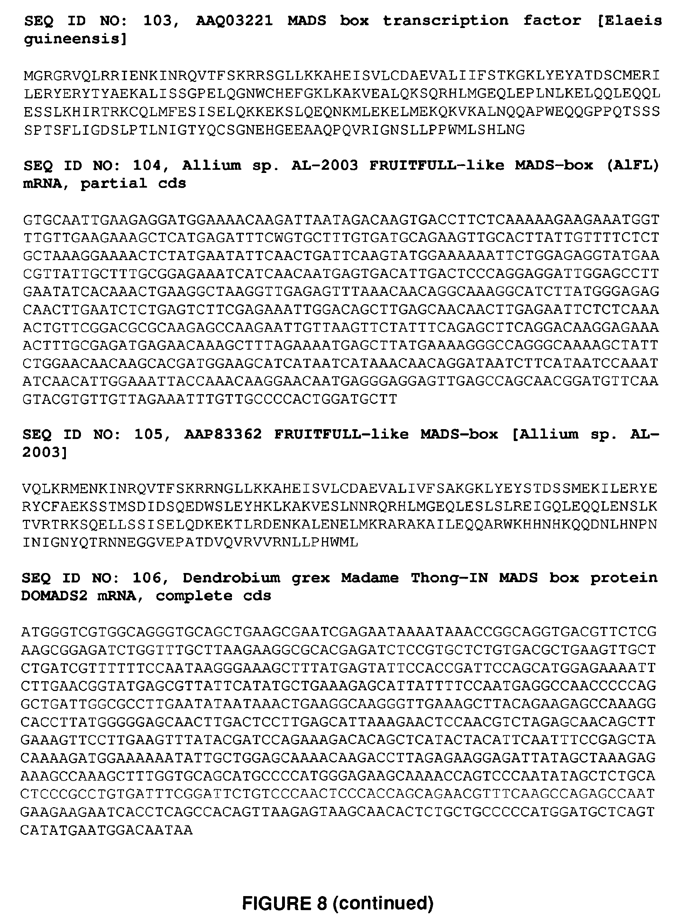

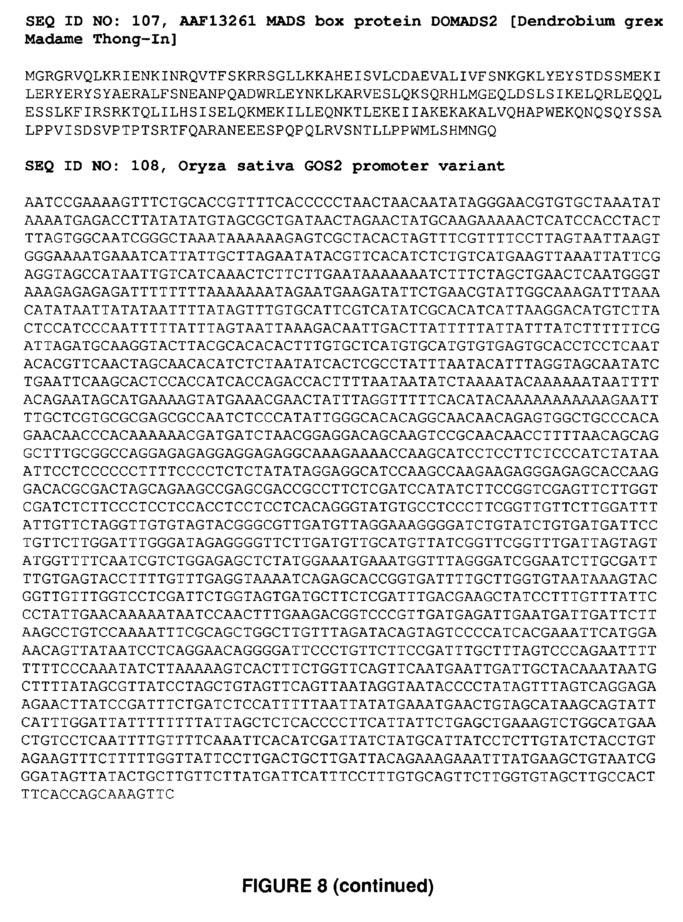

**Fig. 8** details examples of sequences useful in performing the methods according to the present invention.

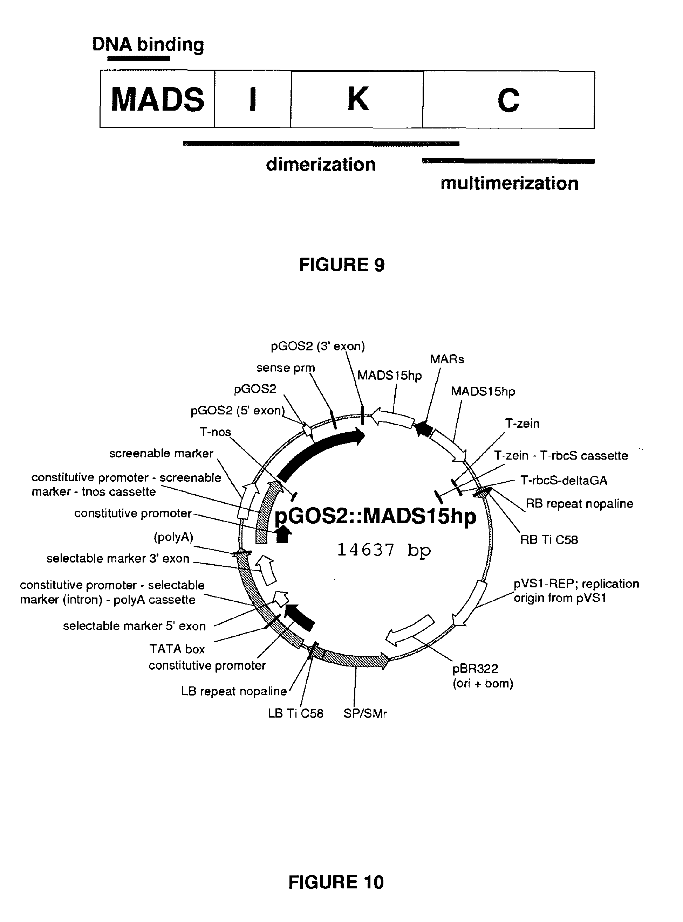

**Fig. 9** is a schematic representation of a full-length OsMADS15 polypeptide. The typical domains (M, MADS; I, intervening domain; K, keratin K-box region; C, variable C-terminal region) are indicated, the lines above and below the diagram show the regions of the protein involved in DNA binding, dimerisation and in multimerisation with interacting proteins.
**Fig. 10** shows the binary vector pGOS::MADS15hp for MADS15 RNA silencing in *Oryza sativa,* using a hairpin construct under the control of a constitutive promoter (GOS2).

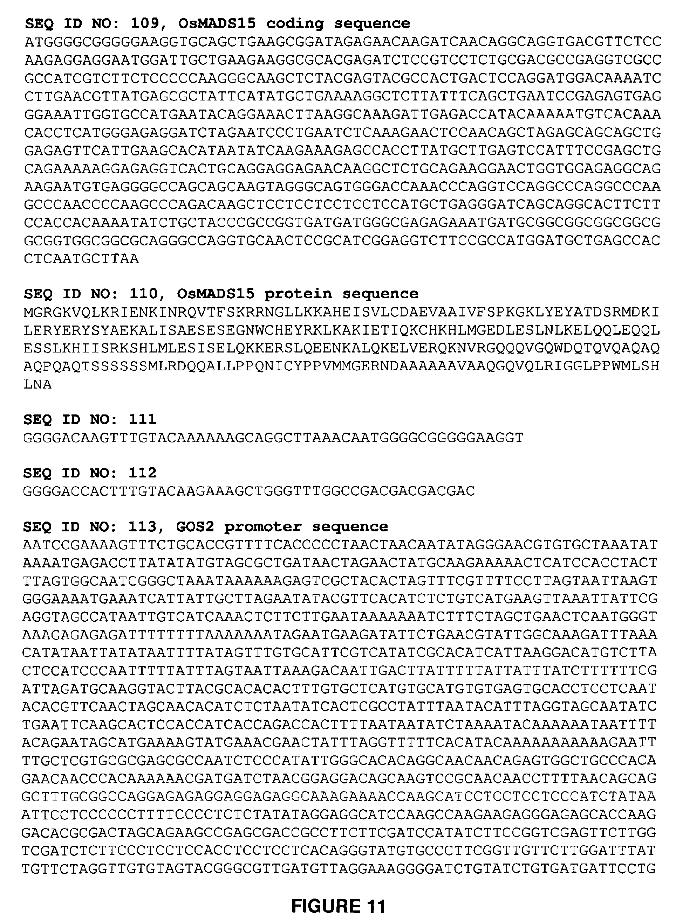

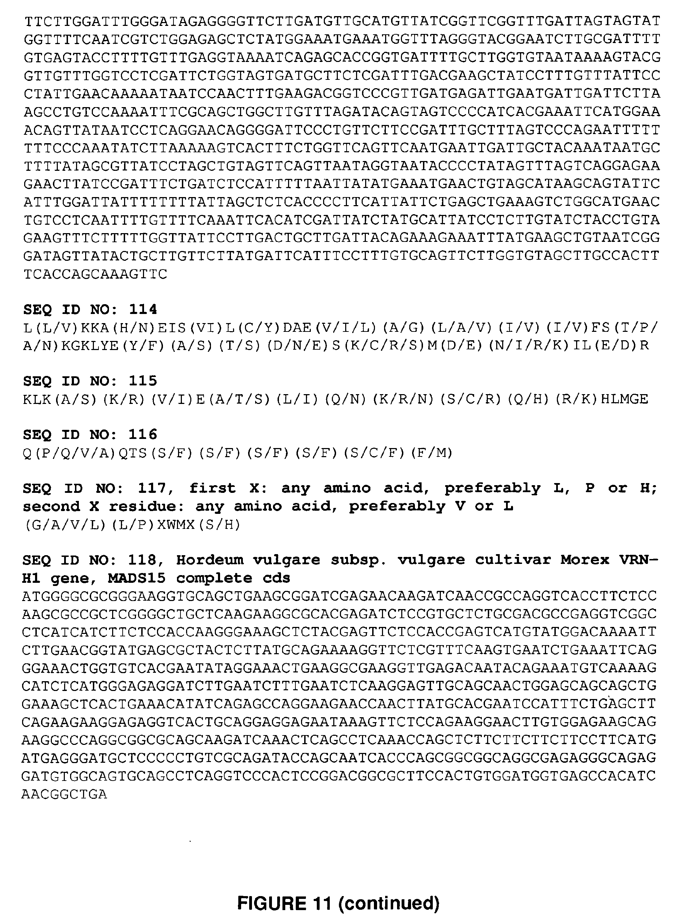

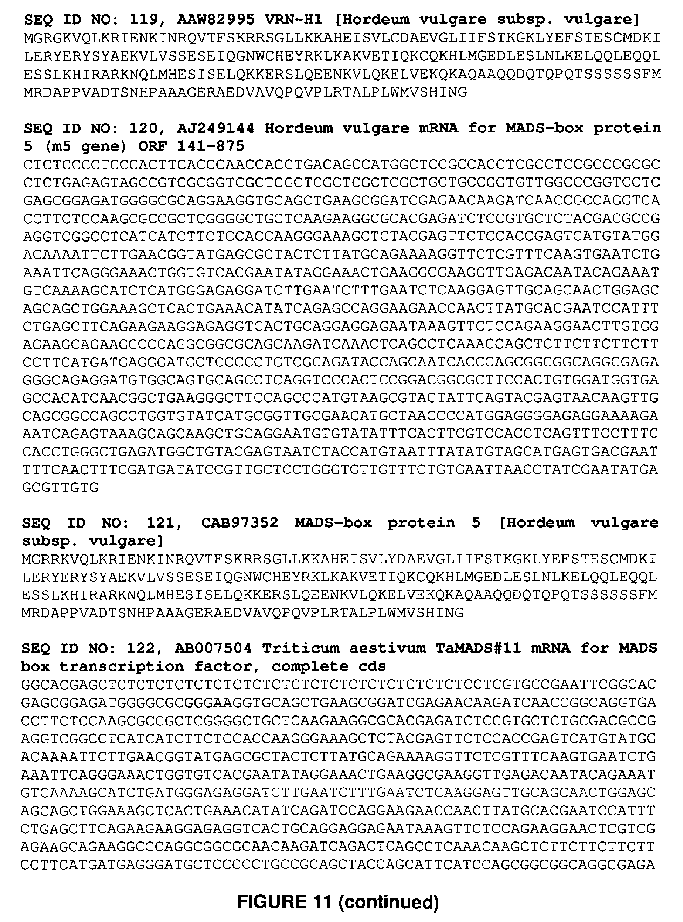

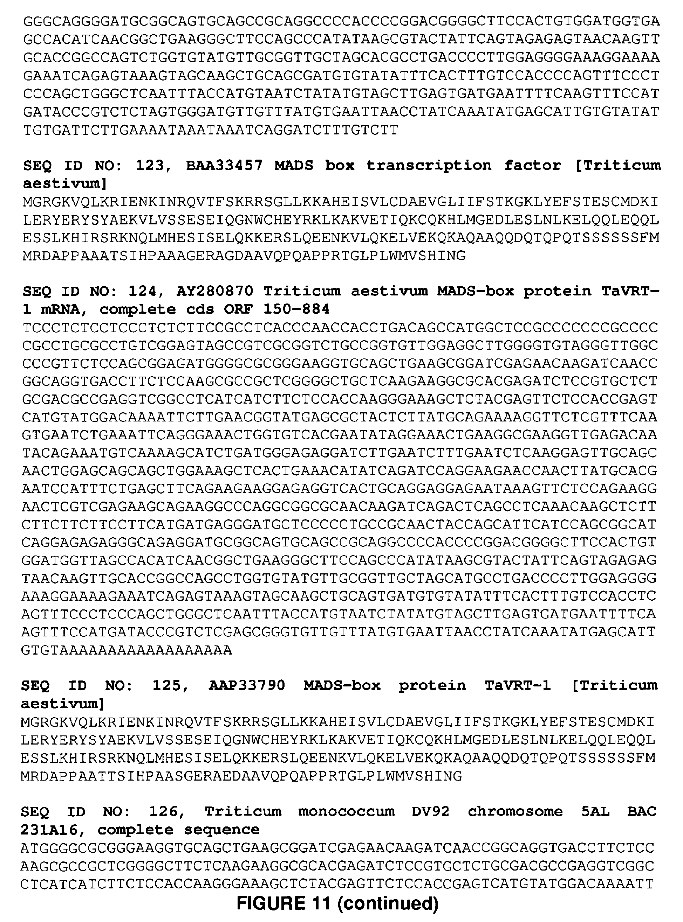

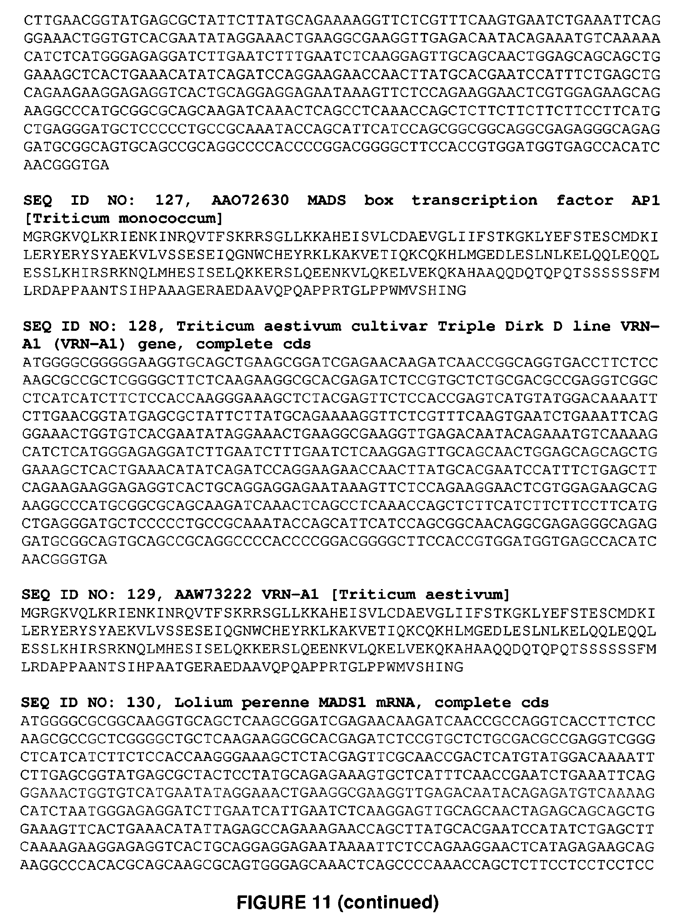

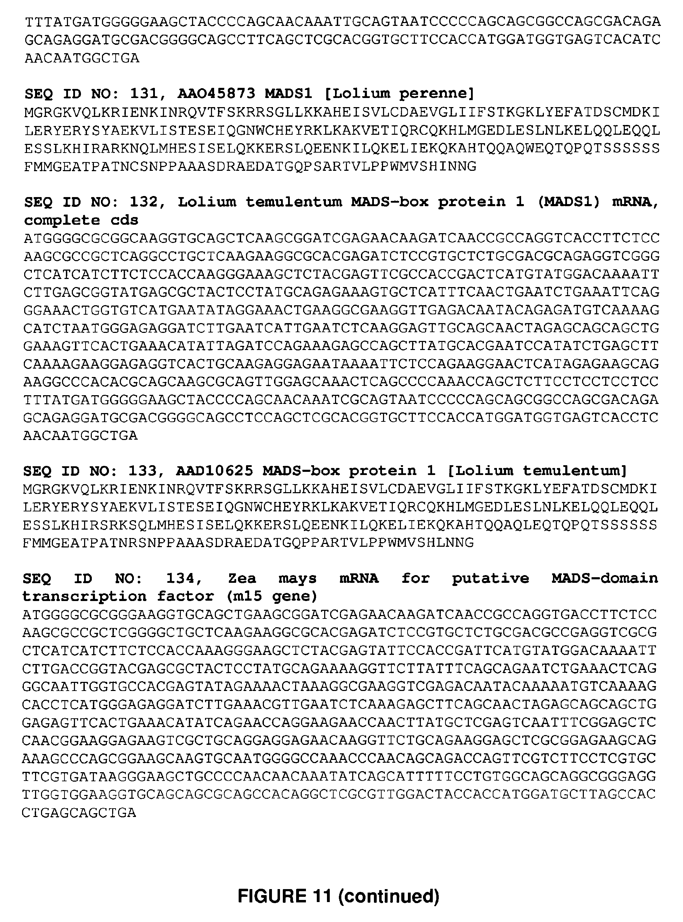

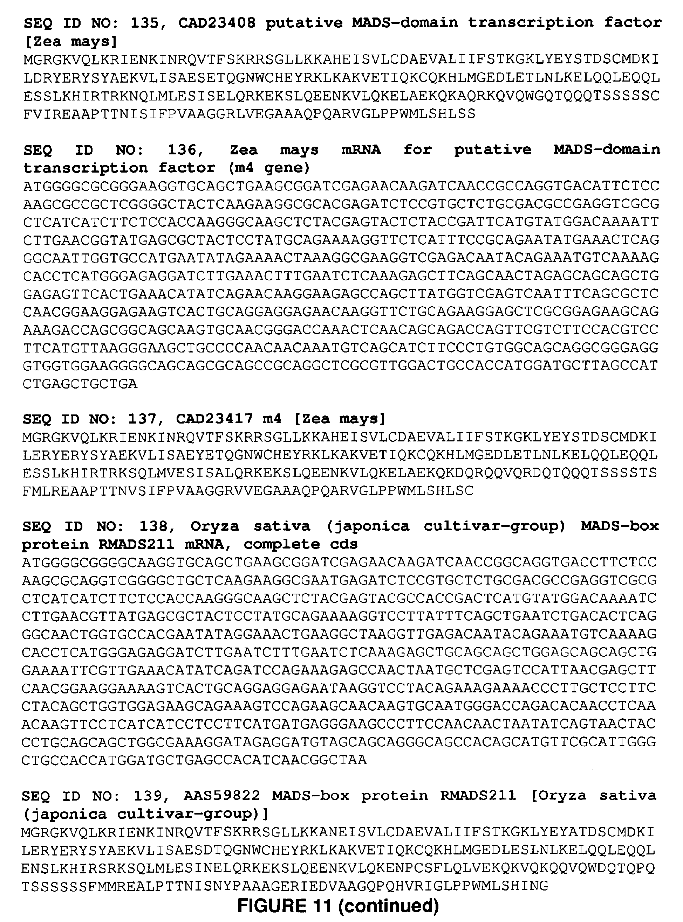

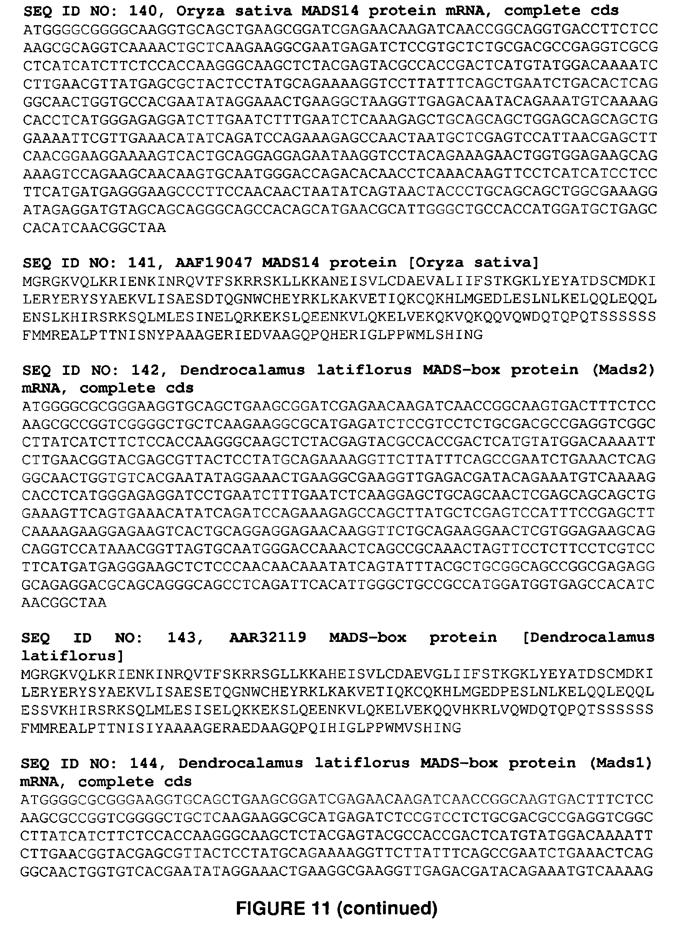

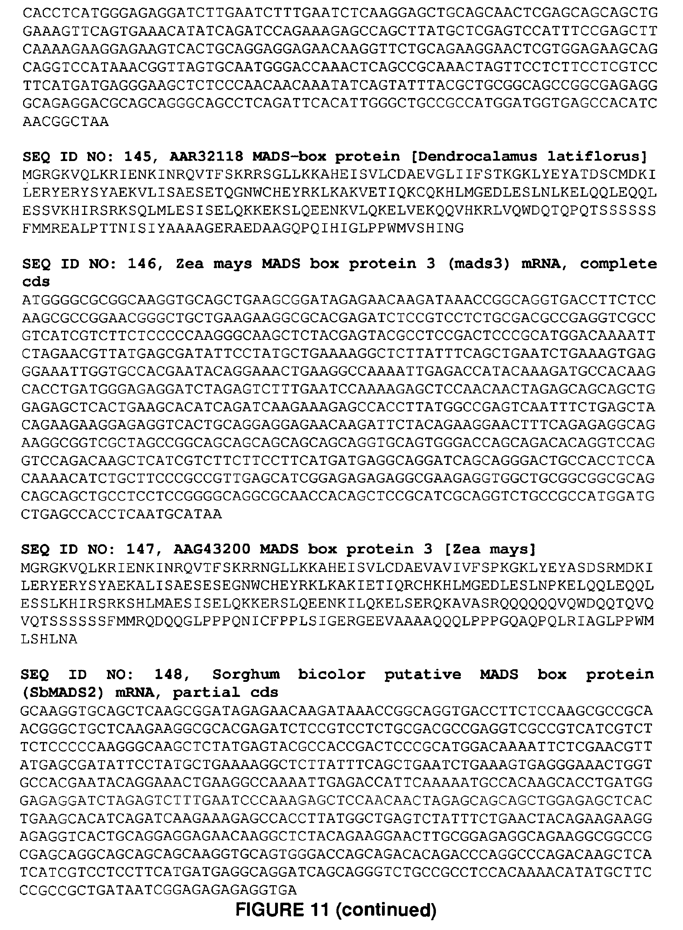

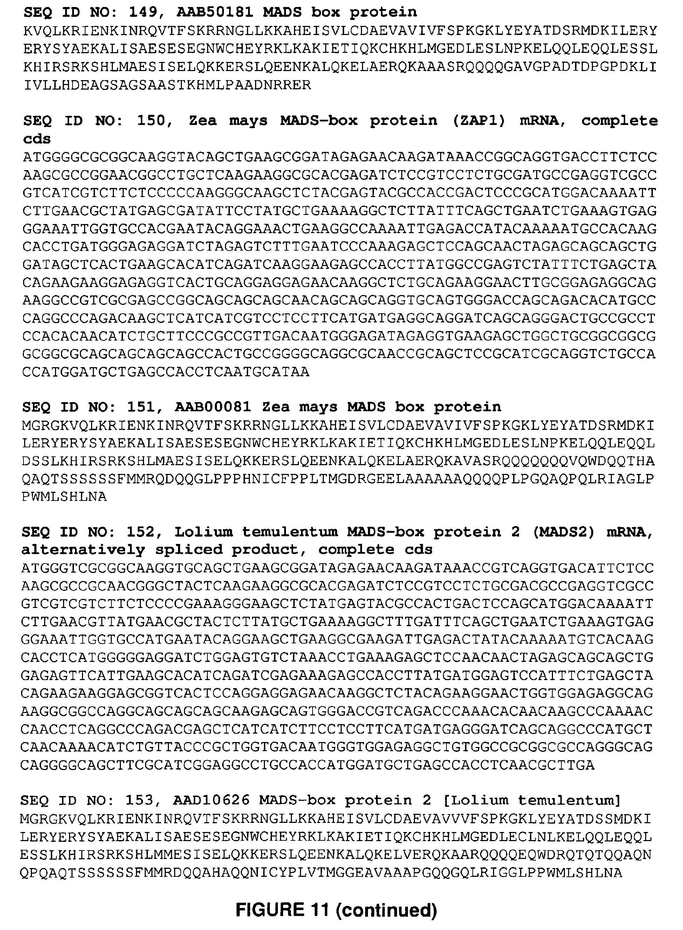

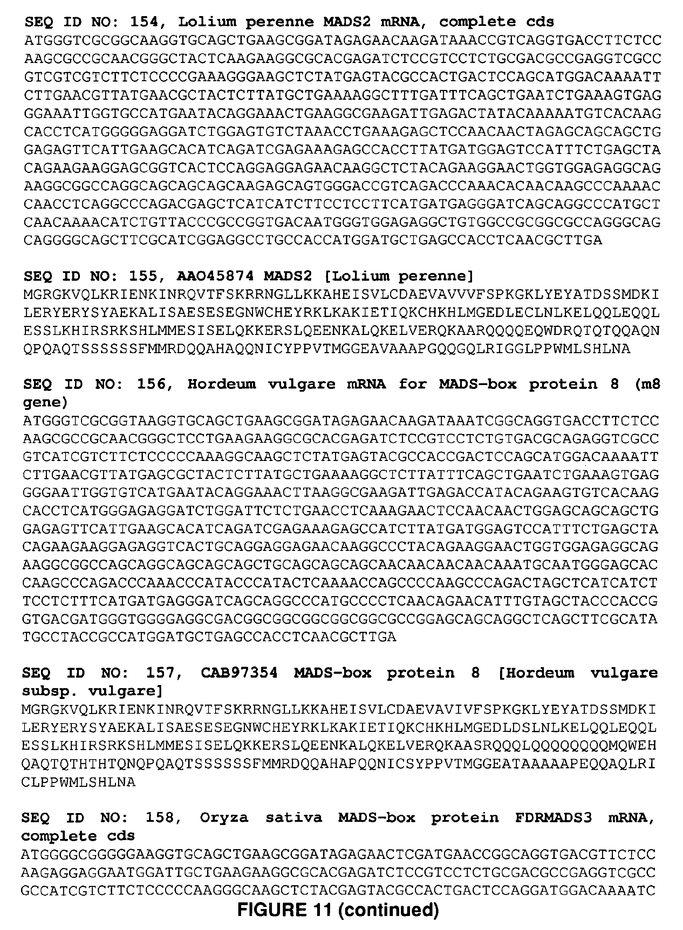

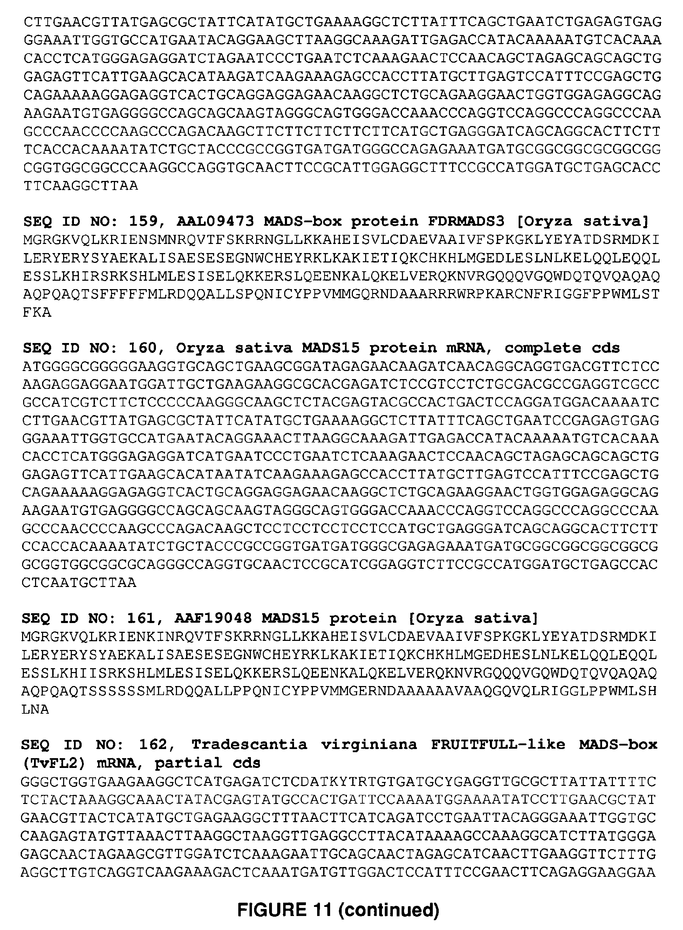

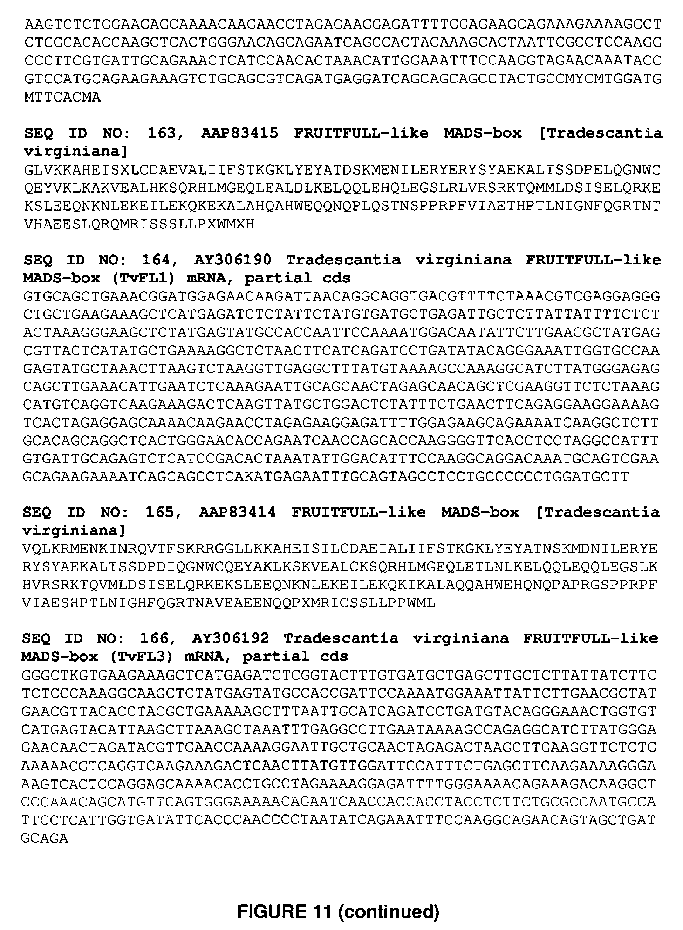

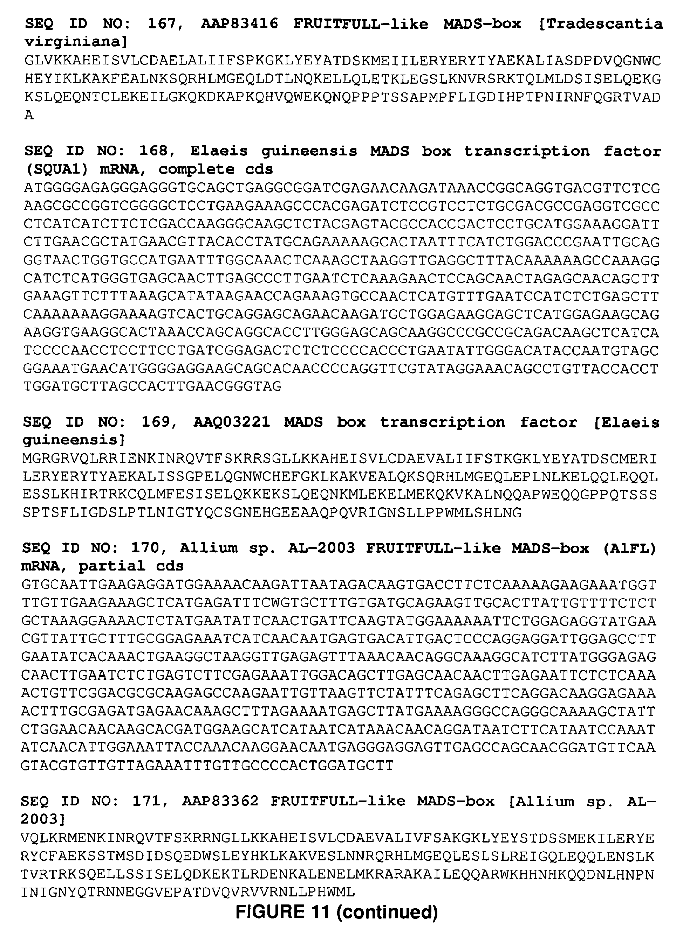

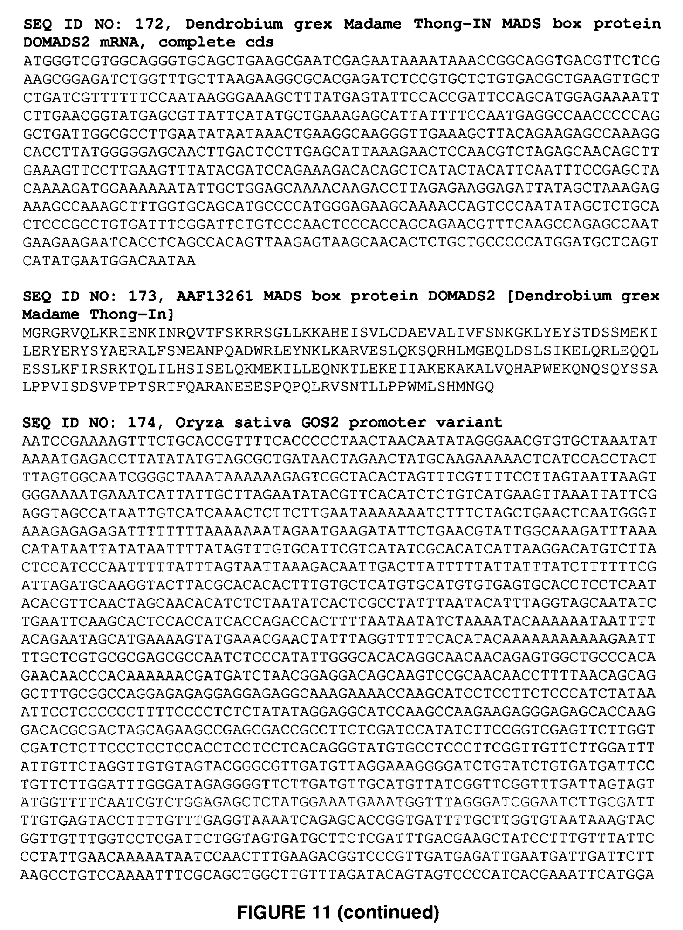

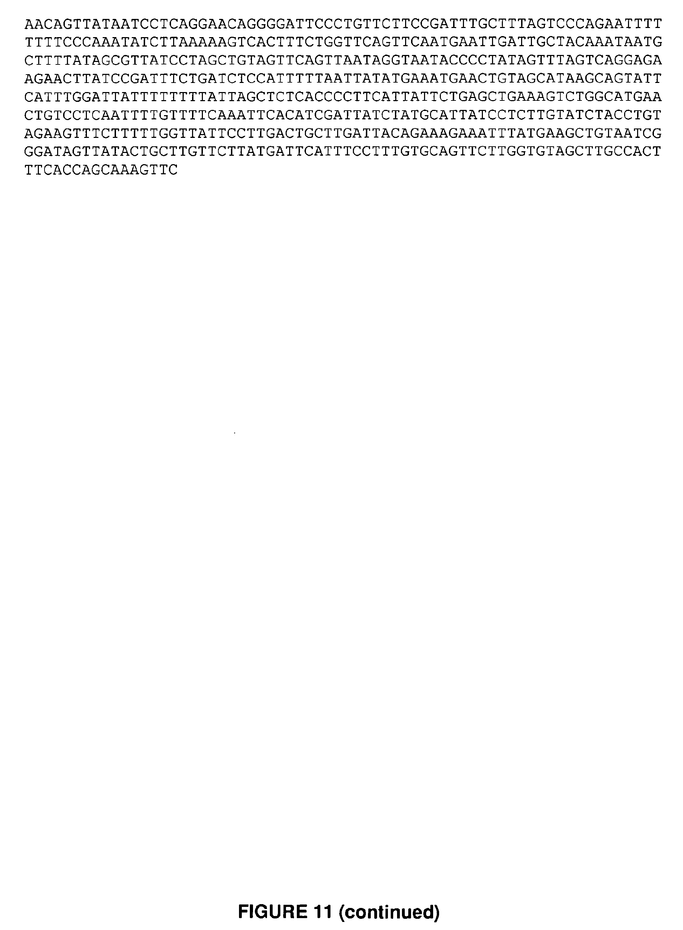

**Fig. 11** details examples of sequences useful in performing the methods according to the present invention, or useful in isolating such sequences. Sequences may result from public EST assemblies, with lesser quality sequencing. As a consequence, a few nucleic acid substitutions may be expected. The start (ATG) and stop codons delimit the nucleic acid sequences when these encode full-length MADS15 polypeptides. However both 5' and 3' UTR may also be used for the performing the methods of the invention.
**Fig. 12** shows the schematic classification of the AP2/ERF transcription factor polypeptides according to the domains present: the AP2 subfamily with two AP2 repeats and the ERF subfamily with only one AP2 repeat. The ERF subfamily is further subdivided into transcription factors comprising a B3 domain in addition to the AP2 repeat (called RAV), or not. The PLT transcription factor polypeptides belong to the AP2 subfamily with two AP2 repeats.
**Fig. 13** respresents a schematic presentation of the PLT transcription factor polypeptide structure. The AP2 domain comprises the two AP2 repeats (boxed) separated by a linker region. The approximate positions of the nuclear localisation signal (NLS), of motif 1 PK(V/L)(A/E)DFLG and of motif 2 (V/L)FX(M/V)WN(D/E) are marked as boxes.

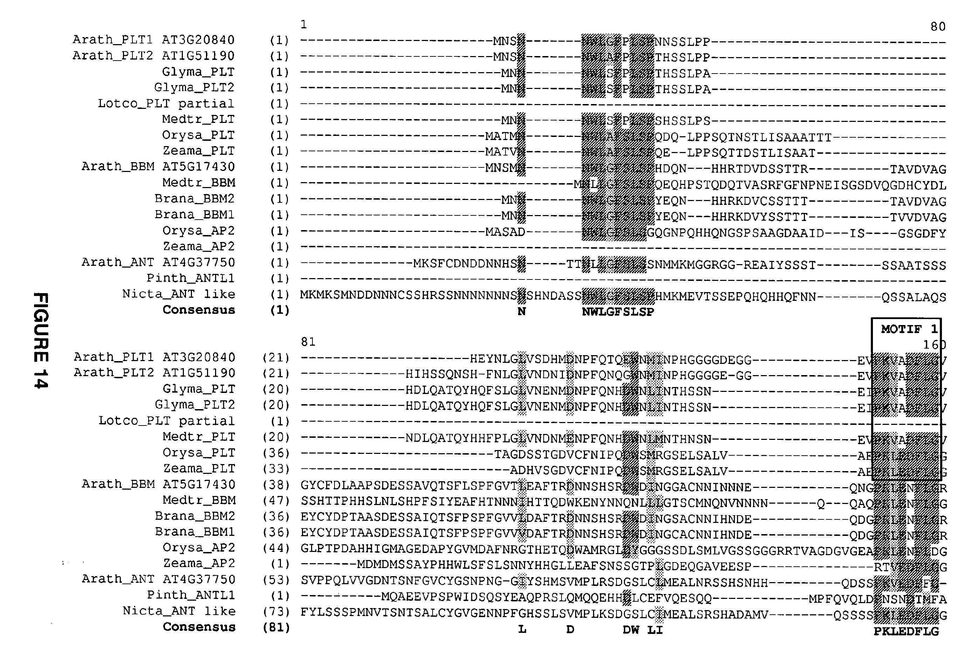

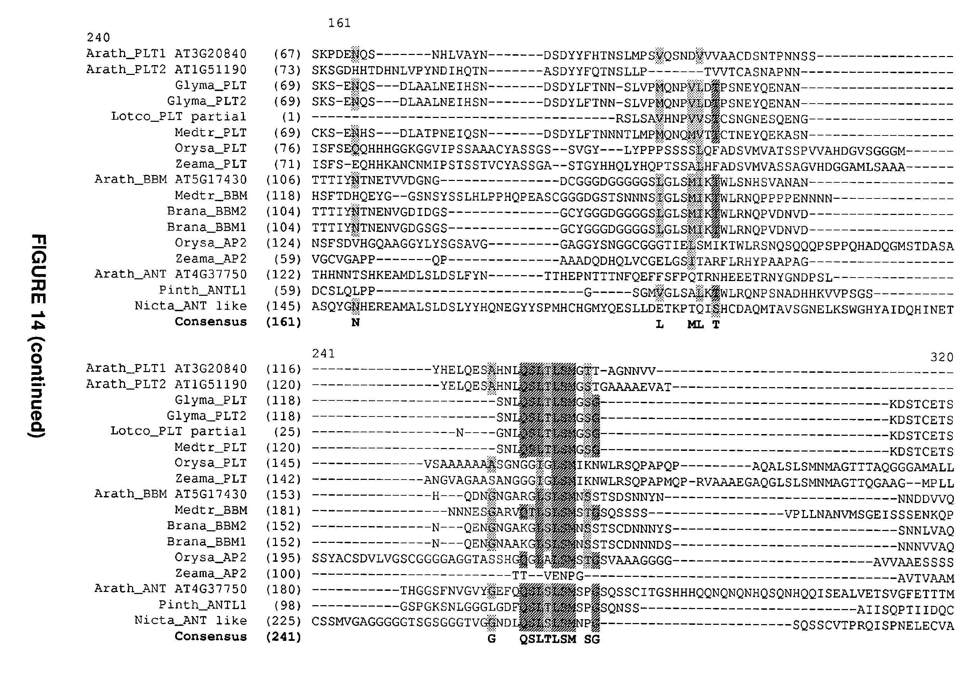

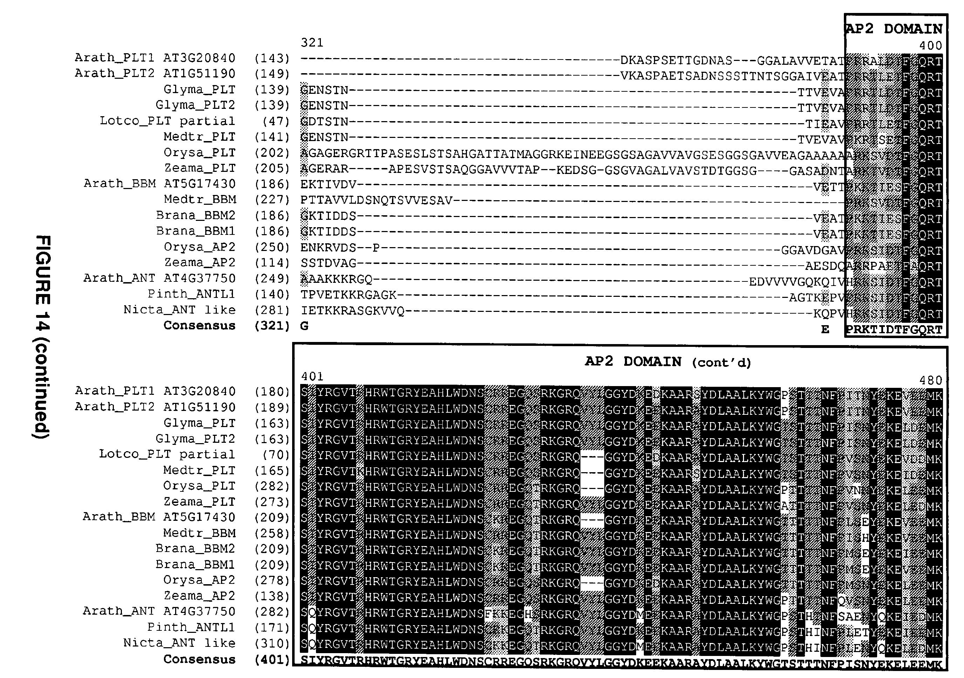

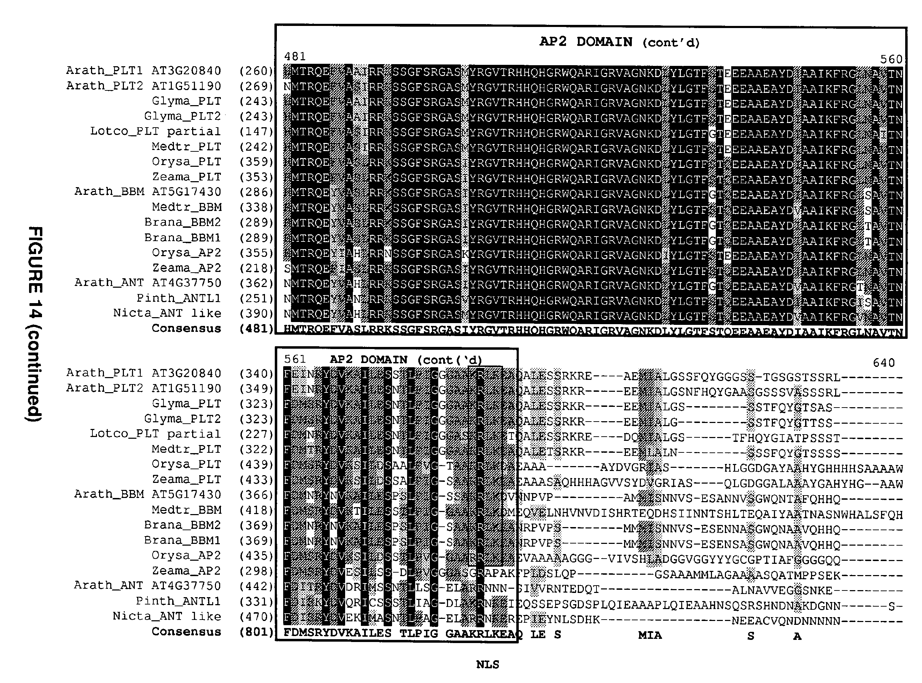

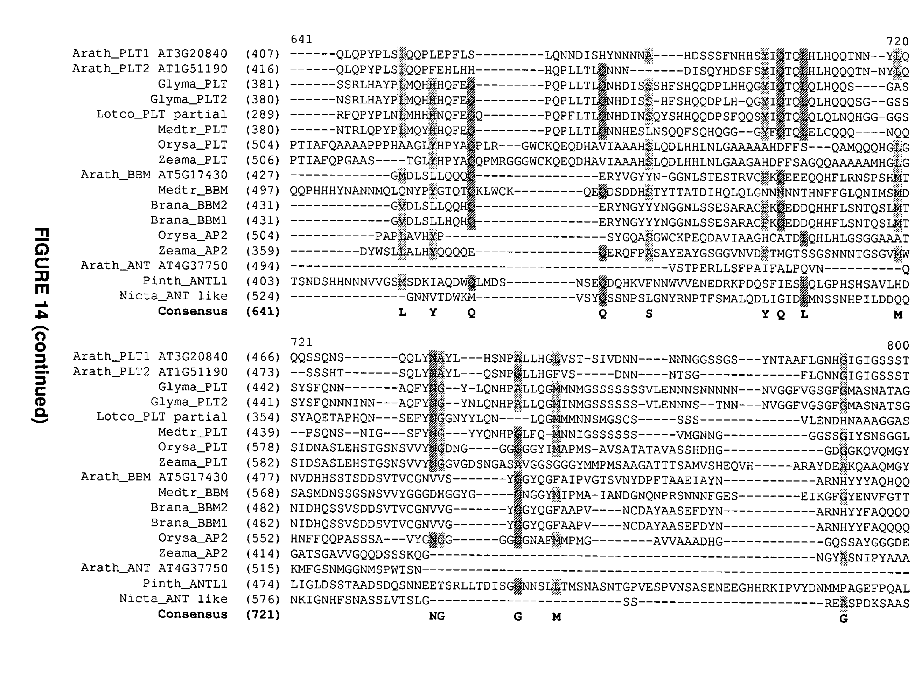

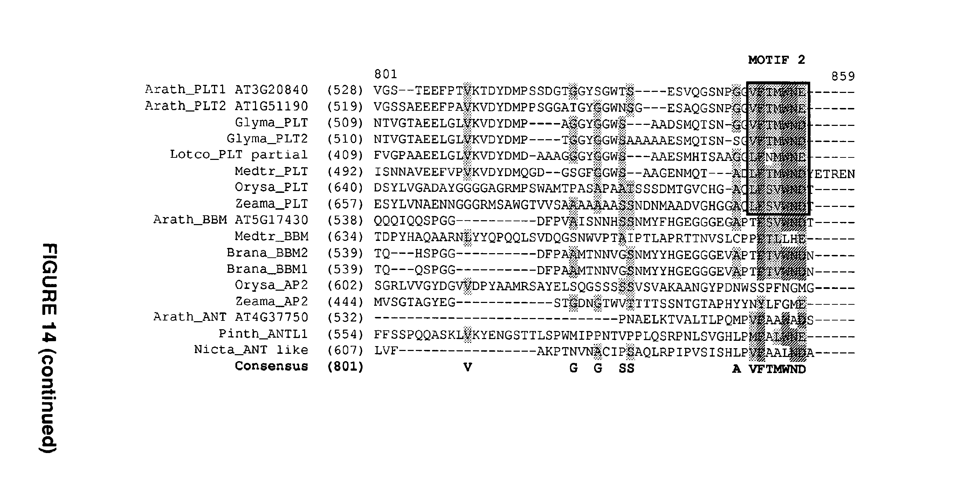

**Fig. 14** is an alignment of PLT transcription factor polypeptide sequences (from Table 4), compared to other AP2 domain transcription factor polypeptides (from Table 4 below). The nuclear localisation signal (NLS), motif 1 PK(V/L)(A/E)DFLG and motif 2 (V/L)FX(M/V)WN(D/E) are boxed. Identical residues are blackened, conservative residues are grayed.

**Table 4: AP2 domain transcription factor polypeptides aligned against the PLT transcription factor polypeptides used to perform the methods of the invention.**

| **Name** | **NCBI accession number** | **Source** |
|---|---|---|
| Arath_ANT | NM_119937 | *Arabidopsis thaliana* |
| Arath_BBM | NM_121749.1 | *Arabidopsis thaliana* |
| Brana_BBM1 | AF317904 | *Brassica napus* |
| Brana_BBM2 | AF317905 | *Brassica napus* |
| Medtr_AP2 BBM | AY899909 | *Medicago truncatula* |
| Nicta_ANT like | AY461432 | *Nicotiana tabacum* |
| Orysa_AP2 | XM_473084 | *Oryza sativa* |
| Pinth_ANTL1 | AB101585 | *Pinus thunbergii* |
| Zeama_AP2 | AY109146.1 | *Zea mays* |

**Fig. 15** is a photograph of 40 seeds from a control plant (left) compared to 40 seeds from a transgenic plant with increased expression of a PLT transcription factor polypeptide.
**Fig. 16** shows a binary vector pGOS2::PLT, for increased expression in *Oryza sativa* of an *Oryza sativa* PLT transcription factor nucleic acid under the control of a GOS2 promoter.

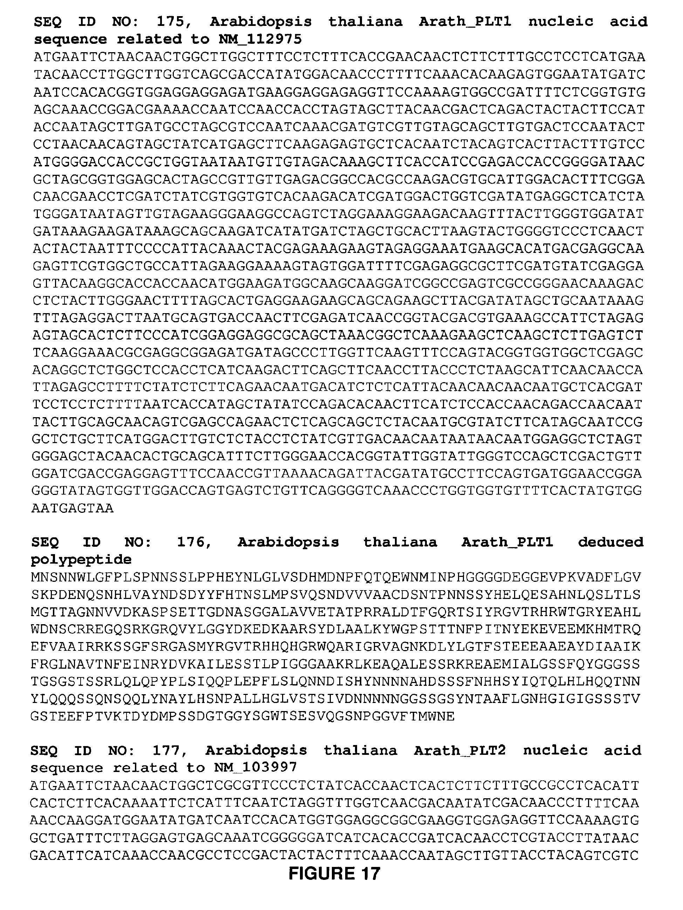

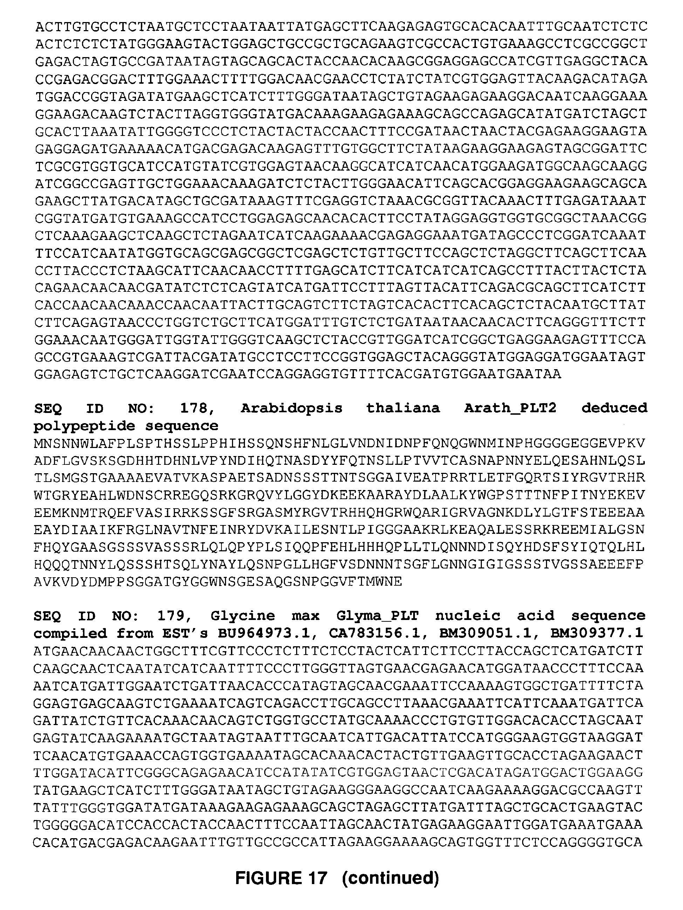

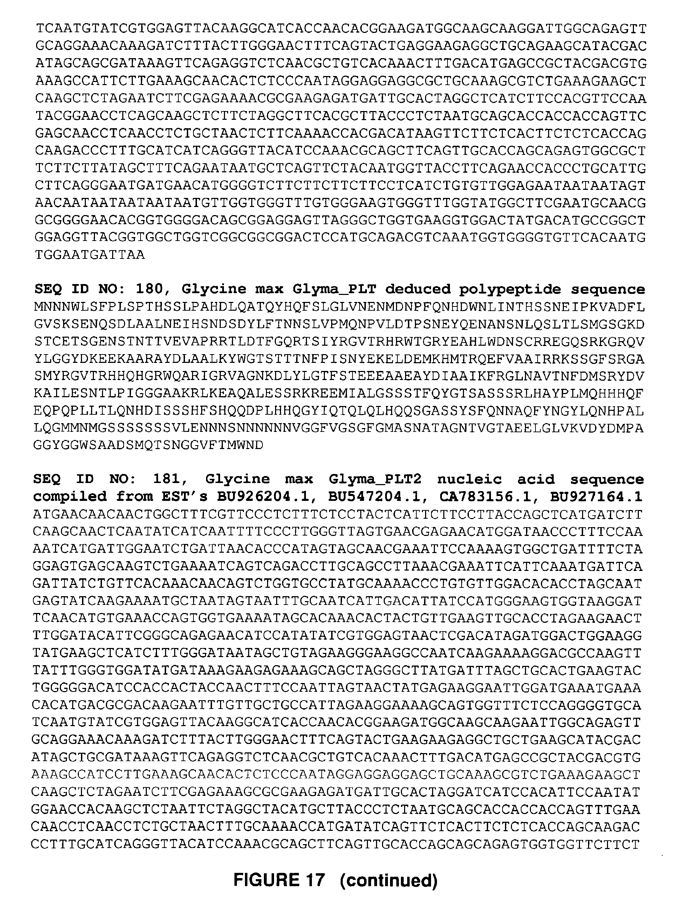

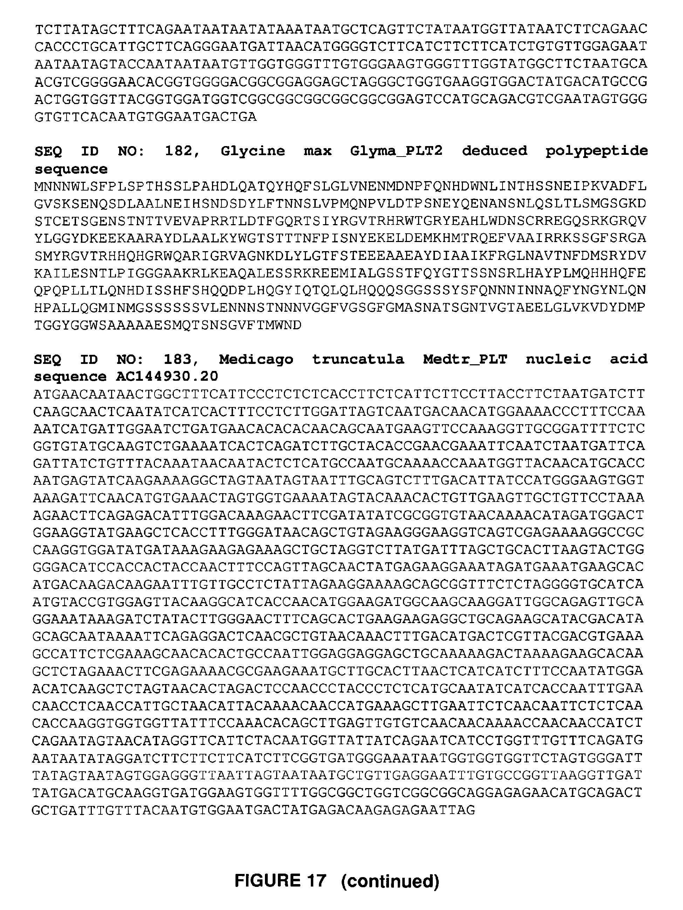

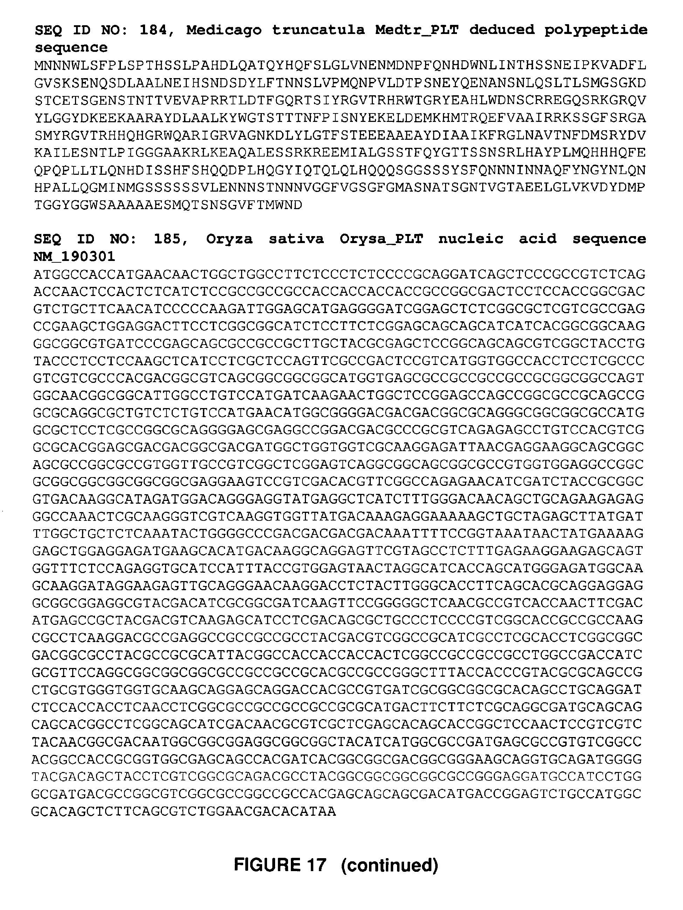

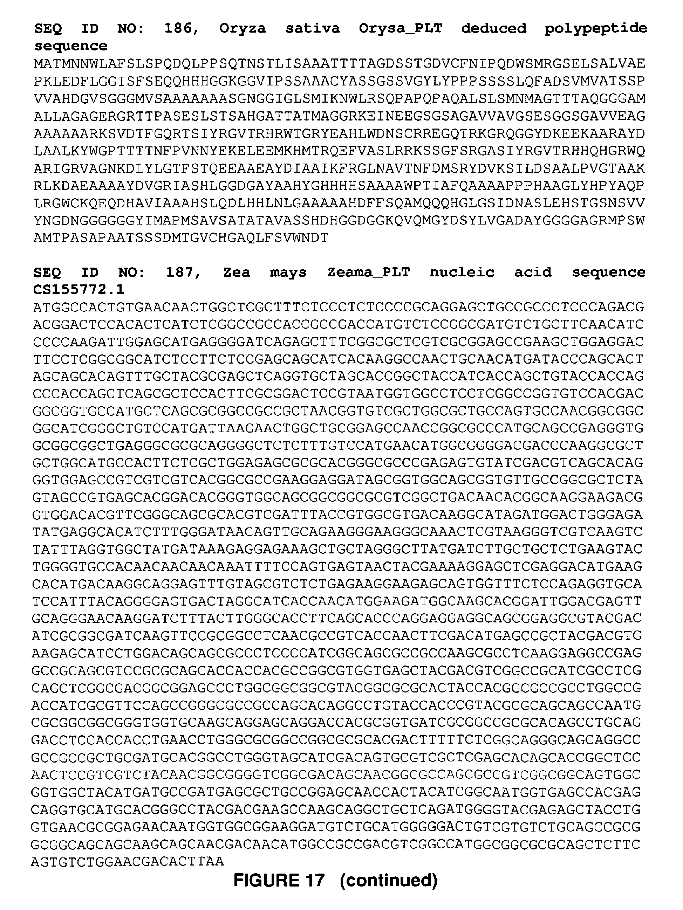

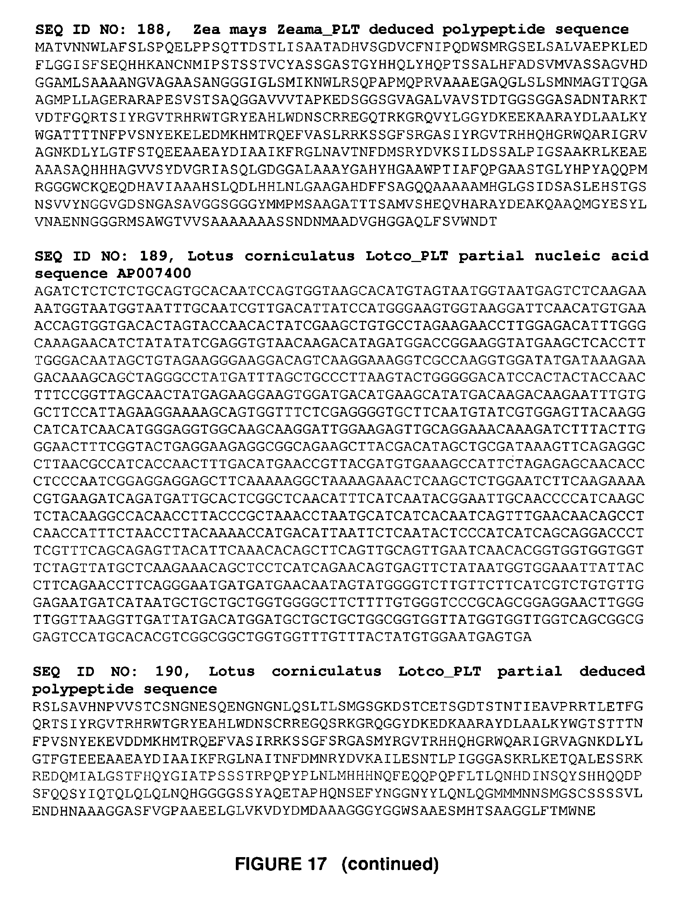

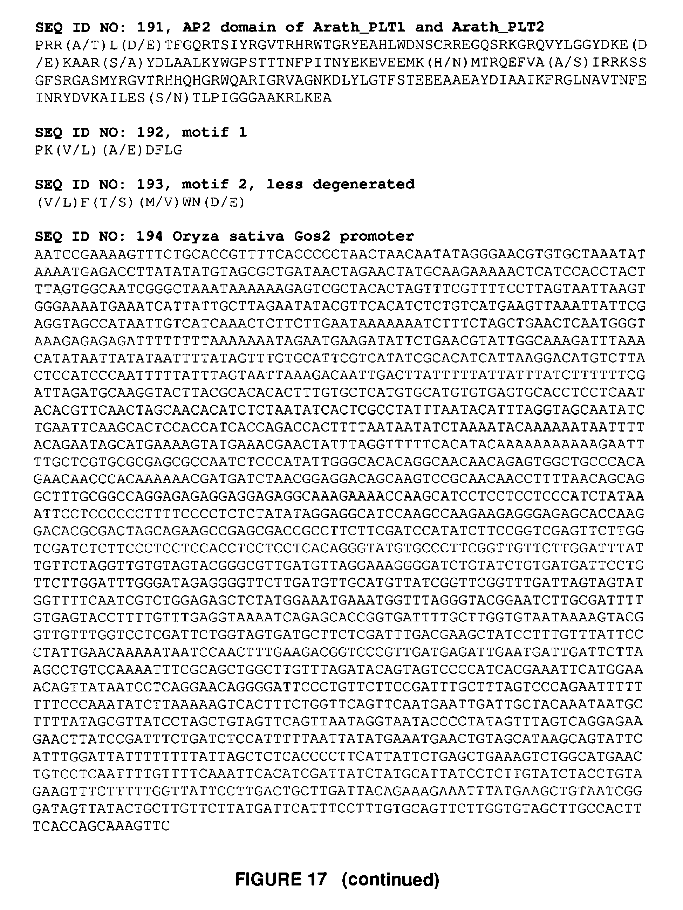

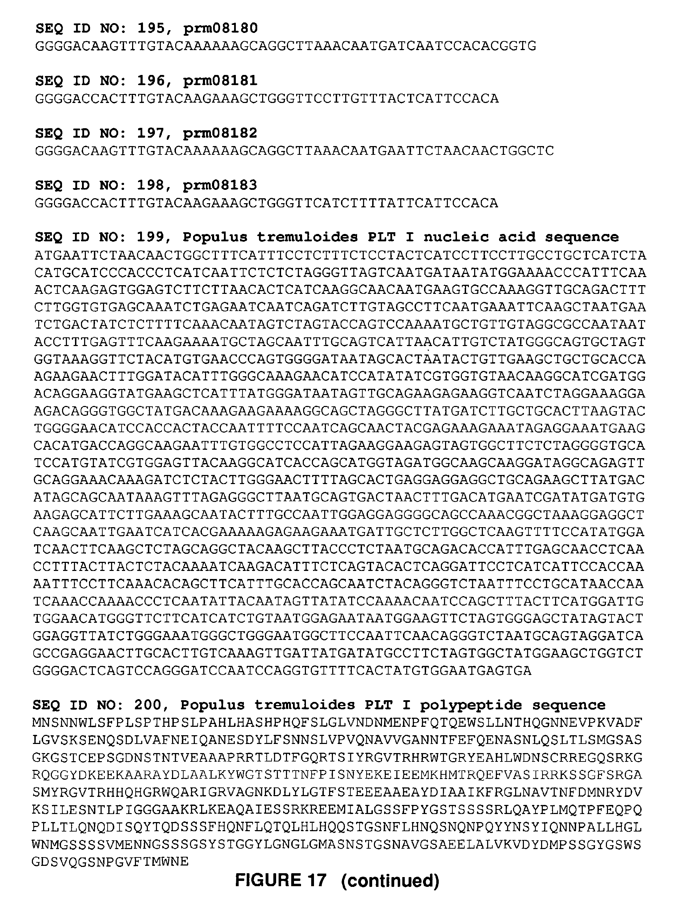

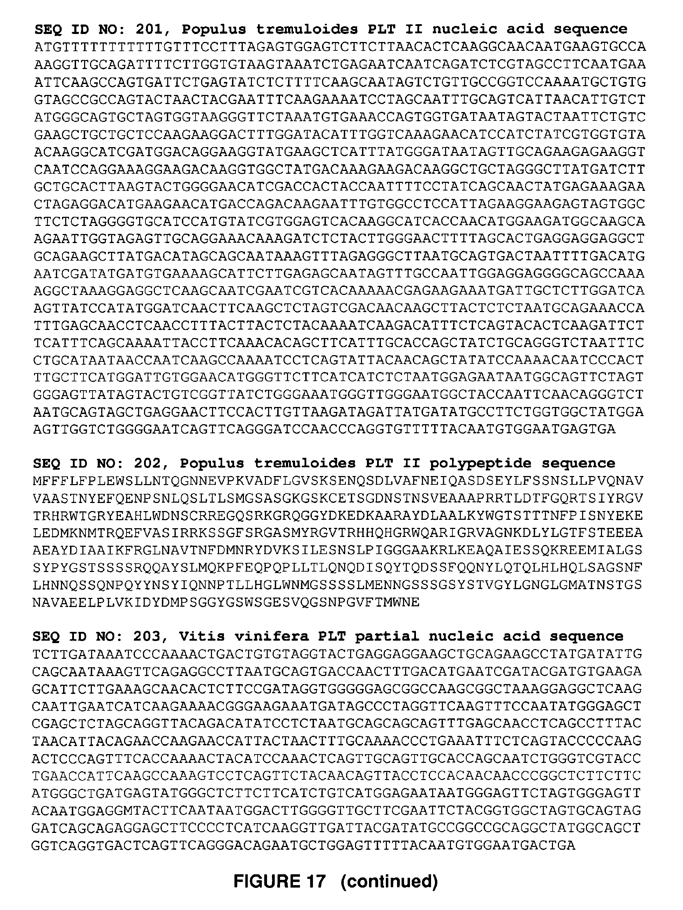

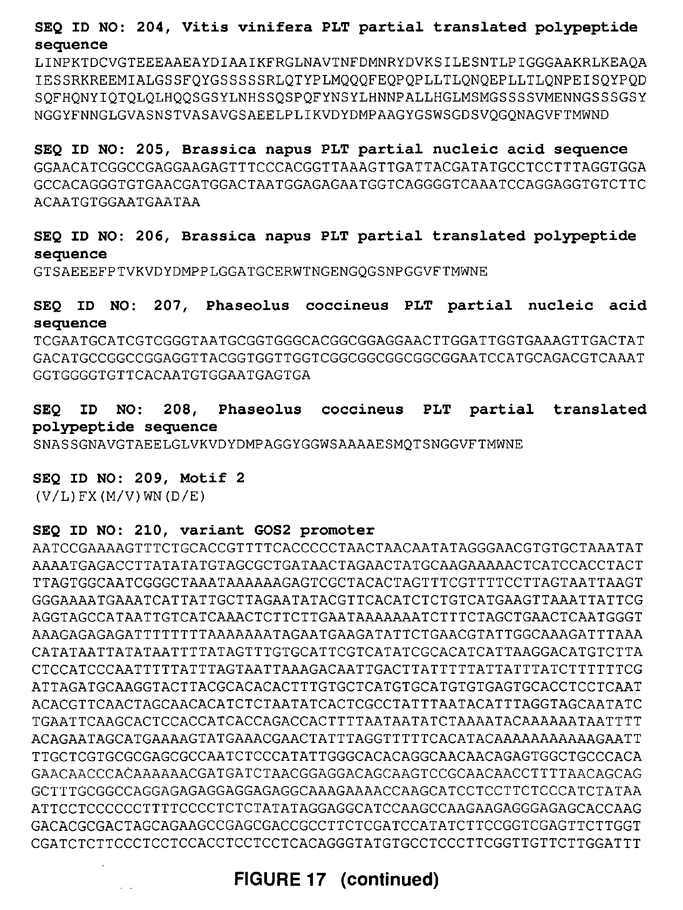

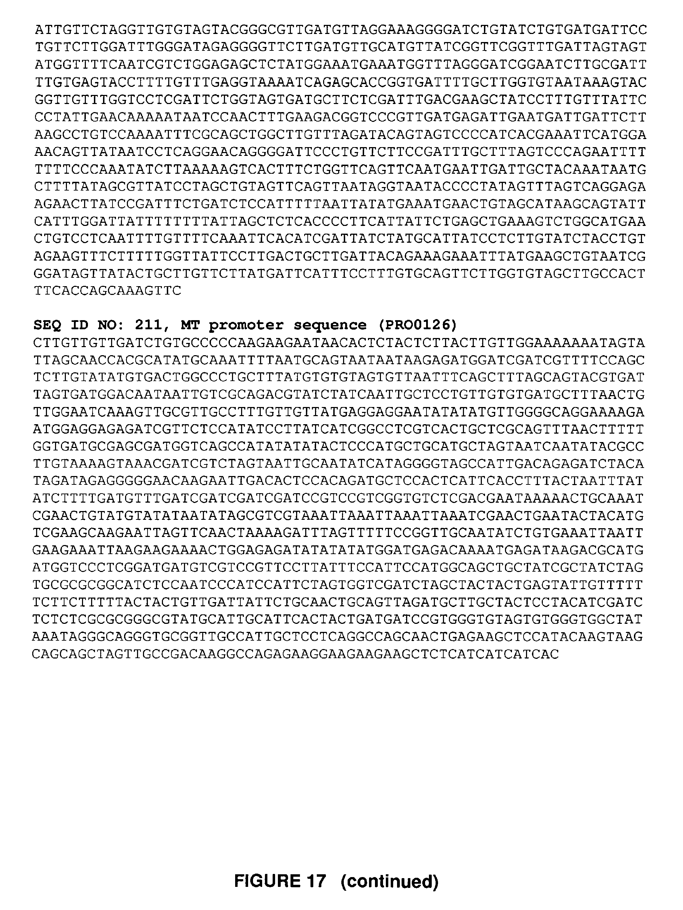

**Fig. 17** details examples of sequences useful in performing the methods according to the present invention (SEQ ID NO : 175 to SEQ ID NO : 188). Partial sequences (SEQ ID NO : 189 and SEQ ID NO : 190) useful in isolating corresponding full length sequences are also presented.
**Fig. 18** shows an alignment of bHLH transcription factors as defined hereinabove. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. The bHLH domain is indicated within the solid box and the PFB26111 domain is indicated within the dashed box. Also indicated by the three upwardly pointing arrows is the 5-9-13 configuration (K/R ER). The single downwardly pointing arrow indicates the glutamic acid reside for recognition of the E-BOX.
**Fig. 19** shows a matrix of similarity and identity between bHLH transcription factors from various species. Percentage identity is shown in bold. **Fig. 19a** is a matrix over full length sequences and **Fig. 19b** is a matrix over the bHLH domain only. More details are provided in Example 34.
**Fig. 20** shows a binary vector pGOS2::bHLH, for increased expression in *Oryza sativa* of an *Oryza sativa* bHLH transcription factor-encoding nucleic acid under the control of a GOS2 promoter.

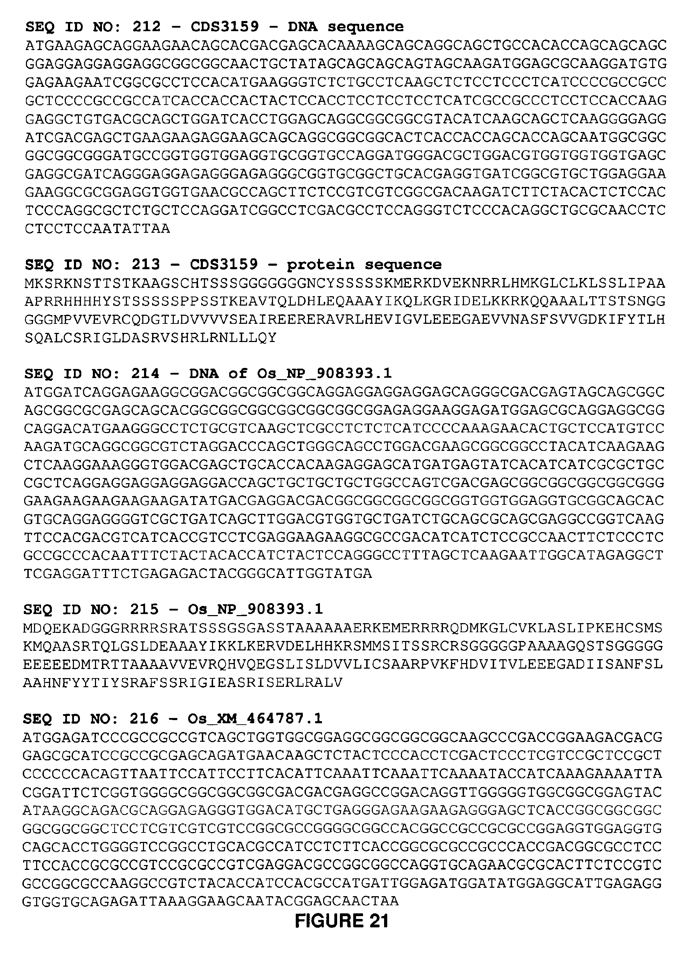

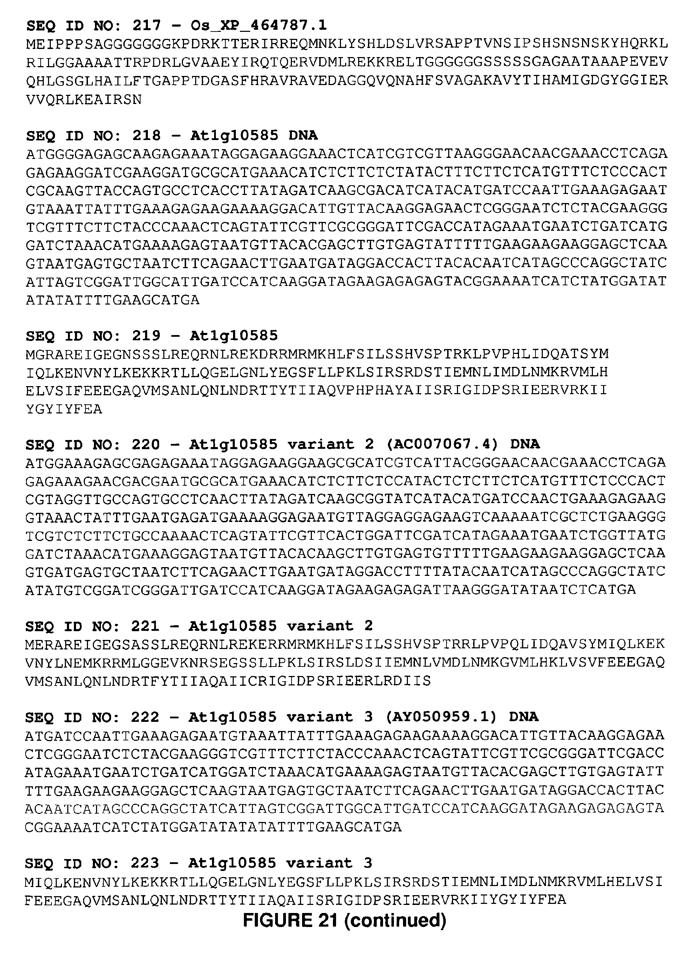

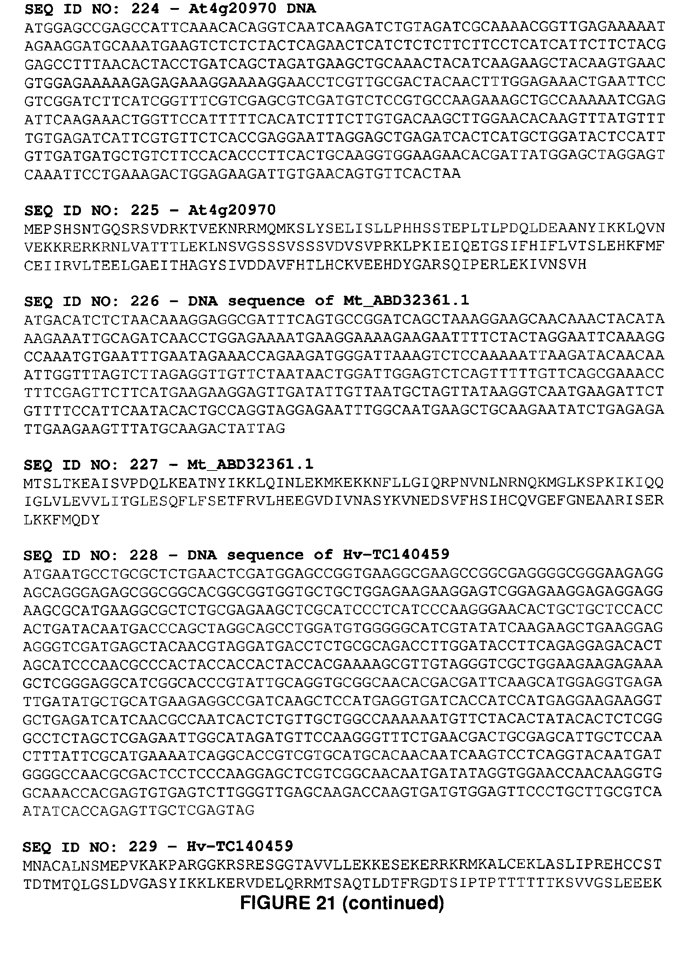

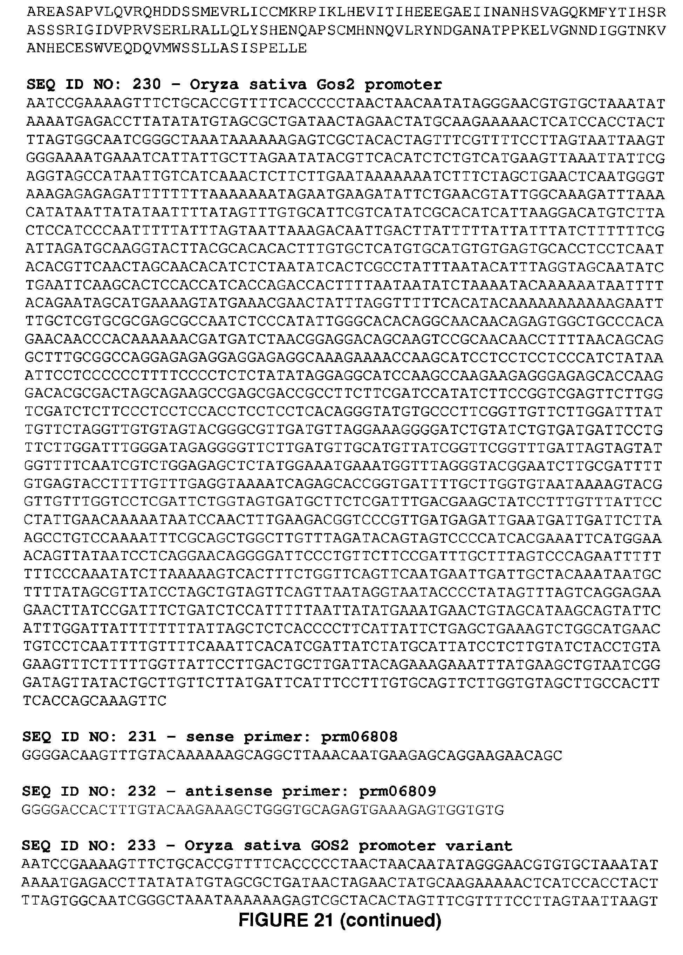

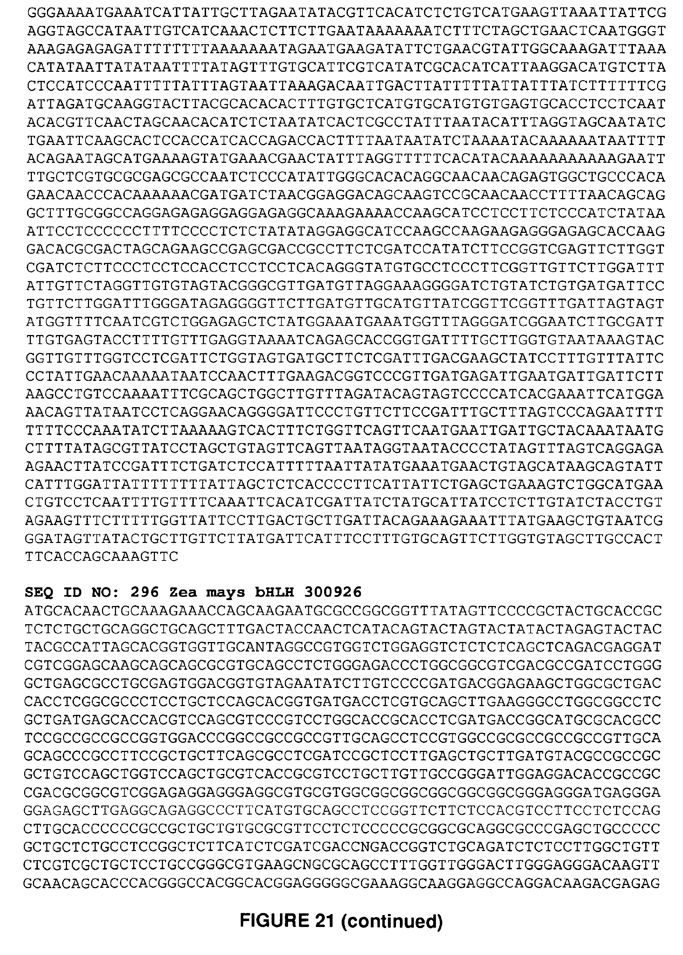

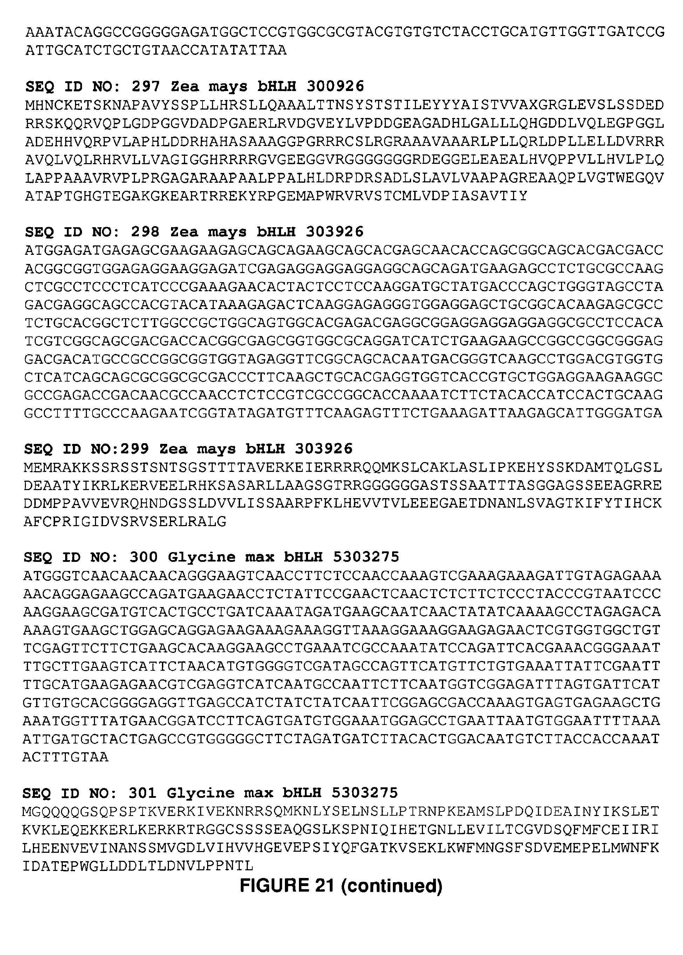

**Fig. 21** details examples of sequences useful in performing the methods according to the present invention.
**Fig. 22** Neighbour-joining tree output after a multiple sequence alignment of all *Arabidopsis thaliana* SPL transcription factor polypeptides and of SPL15 transcription factor polypeptide orthologues using CLUSTAL W (1.83) (at GenomeNet service at the Kyoto University Bioinformatics Center), and default values (Blosum 62 as weight matrix, gap open penalty of 10; gap extension penalty of 0.05). Arabidopsis thaliana SPL15 transcription factor polypeptide clusters with the other SPL15 transcription factor polypeptide orthologues and paralogues, as shown by the curly bracket.
**Fig. 23** shows an alignment of the DNA-binding domain (DBD) of SPL15 transcription factor polypeptide orthologues and paralogs. The sequences were aligned using AlignX program from Vector NTI suite (InforMax, Bethesda, MD). Multiple alignment was done with a gap opening penalty of 10 and a gap extension of 0.01. The conserved Cys and His residues involved in zinc ion binding are boxed. The bipartite nuclear localization signal (NLS) is underlined.
**Fig. 24** is an alignment of SPL15 transcription factor polypeptide orthologues and paralogues. The sequences were aligned using AlignX program from Vector NTI suite (InforMax, Bethesda, MD). Multiple alignment was done with a gap opening penalty of 10 and a gap extension of 0.01. Minor manual editing was also carried out where necessary to better position some conserved regions. The three main characterized domains, from N-terminal to C-terminal, are boxed and identified as Motif 1, the SPL DNA binding domain and Motif 2. Additionally, the G/S rich stretch preceding the SPL DBD is marked with Xs and the W(S/T)L tripeptide at the C-terminal end of the polypeptide also boxed.
**Fig. 25** shows a binary vector pHMGB::SPL15, for increased expression in *Oryza sativa* of an *Arabidopsis thaliana* nucleic acid encoding an SPL15 transcription factor polypeptide under the control of an HMGB promoter.
**Fig. 26** details examples of sequences useful in performing the methods according to the present invention.

### Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Example section A: HAL3

### Example 1: Identification of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to the nucleic acid sequence used in the methods of the present invention.

**Table A: Examples of HAL3 polypeptides:**

| ***Plant Source*** | ***Nucleic acid SEQ ID NO:*** | ***Protein SEQ ID NO:*** |
|---|---|---|
| *Arabidopsis thaliana* | 9 | 10 |
| *Oryza sativa* | 11 | 12 |
| *Triticum aestivum* | 13 | 14 |
| *Zea mays* | 15 | 16 |
| *Picea abies* | 17 | 18 |
| *Brassica napus* | 19 | 20 |
| *Brassica oleracea* | 21 | 22 |
| *Nicotiana tabacum* | 23 | 24 |
| *Solanum tuberosum* | 25 | 26 |
| *Glycine max* | 27 | 28 |
| *Vitis vinifera* | 29 | 30 |
| *Hordeum vulgare* | 31 | 32 |
| *Gossypium hirsutum* | 33 | 34 |
| *Sorghum bicolor* | 35 | 36 |
| *Lycopersicon esculentum* | *37* | *38* |
| *Arabidopsis thaliana* | *39* | *40* |
| *Pinus sp.* | *41* | *42* |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

### Example 2: Cloning of the nucleic acid sequence used in the methods of the invention

The nucleic acid sequence used in the methods of the invention was amplified by PCR using as template a custom-made *Arabidopsis thaliana* seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm00957 (SEQ ID NO: 3; sense, start codon in bold:
5' aaaaagcaggctcacaatggagaatgggaaaagagac 3')
   and prm00958 (SEQ ID NO: 4; reverse, complementary,:
5' agaaagctgggttggttttaactagttccaccg 3'),
which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pHAL3. Plasmid pDONR201 was purchased from Invitrogen, as part of the Gateway® technology.

### Example 3: Expression Vector Construction

The entry clone pHAL3 was subsequently used in an LR reaction with pEXP, a destination vector used for *Oryza sativa* transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice beta expansin promoter (SEQ ID NO: 5) for shoot-specific expression was located upstream of this Gateway cassette.

After the LR recombination step, the resulting expression vector pEXP::HAL3 (Figure 2) was transformed into *Agrobacterium* strain LBA4044 and subsequently to *Oryza sativa* plants.

### Example 4: Crop transformation

The transformed *Agrobacterium* icontaining the expression vectors were used independently to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2.4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25°C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K₂SO₄, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 5: Evaluation procedure

### 5.1 Evaluation setup

Approximately 30 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

### 5.2 Statistical analysis: t test and F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### Example 6: Evaluation results

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield (total seed weight) was measured by weighing all filled husks harvested from a plant. Total seed number per plant was measured by counting the number of husks harvested from a plant. The harvest index (Hl) in the present invention is defined as the ratio between the total seed yield and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

As presented in Table B, the seed yield, number of filled seeds and harvest index are increased in the transgenic plants preferentially expressing a nucleic acid encoding a HAL3 polypeptide in the shoot, compared to control plants.

Table B shows the average yield increase (total seed weight), increase in number of filled seeds and increase of harvest index in percent, calculated from the transgenic events compared to control plants, in the T1 generation

**Table B**

| ***parameters*** | ***% increase*** | ***p-value*** |
|---|---|---|
| Total seed weight | 14 | 0.0072 |
| Number of filled seeds | 15 | 0.0052 |
| Harvest Index | 10 | 0.0093 |

### Example 7: Early vigour evaluation results

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Plant early vigour (as determined by aboveground area) was seen in six out of seven events of the T1 generation, with an overall increase in aboveground area for transgenic seedlings of 27% compared to control plants. Four of these T1 events were further evaluated in the T2 generation, and all four of these events gave an increase in aboveground area for transgenic seedlings compared to control plants, with an overall increase in aboveground area for transgenic seedlings of 33% compared to control plants. The results were also shown to be statistically significant with the p-value from the F-test being lower than 0.0001 (T2 generation) indicating that the effect seen is likely due to the transgene rather than the position of the gene or a line effect, see table C.

**Table C**

| ***parameters*** | ***% increase*** | ***p-value*** |
|---|---|---|
| Early vigour | 33 | 0.0000 |

### Example Section B: MADS15 upregulated

### Example 8: Identification of sequences related to SEQ lD NO: 43 and SEQ ID NO: 44

Sequences (full length cDNA, ESTs or genomic) related to SEQ ID NO: 43 and/or protein sequences related to SEQ ID NO: 44 were identified amongst those maintained in the Entrez Nucleotides database at the National Center for Biotechnology Information (NCBI) using database sequence search tools, such as the Basic Local Alignment Tool (BLAST) (Altschul et a/. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. The polypeptide encoded by SEQ ID NO: 43 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search.

In addition to the publicly available nucleic acid sequences available at NCBI, proprietary sequence databases are also searched following the same procedure as described herein above.

Table D provides a list of nucleic acid and protein sequences related to the nucleic acid sequence as represented by SEQ ID NO: 43 and the protein sequence represented by SEQ ID NO: 44.

**Table D: Nucleic acid sequences related to the nucleic acid sequence (SEQ ID NO: 43) useful in the methods of the present invention, and the corresponding deduced polypeptides.**

| **name** | **source organism** | **SEQ ID NO: nucl/protein** | **database accession number** |
|---|---|---|---|
| VRN-H1 | *Hordeum vulgare* | 52/53 | AAW82995 |
| m5 | *Hordeum vulgare* | 54/55 | CAB97352 |
| TaMADS#11 | *Triticum aestivum* | 56/57 | BAA33457 |
| TaVRT-1 | *Triticum aestivum* | 58/59 | AAP33790 |
| AP1 | *Triticum monococcum* | 60/61 | AAO72630 |
| VRN-A1 | *Triticum aestivum* | 62/63 | AAW73222 |
| MADS1 | *Lolium perenne* | 64/65 | AAO45873 |
| MADS1 | *Lolium temulentum* | 66/67 | AAD10625 |
| m15 | *Zea mays* | 68/69 | CAD23408 |
| m4 | *Zea mays* | 70/71 | CAD23417 |
| RMADS211 | *Oryza sativa* | 72/73 | AAS59822 |
| MADS14 | *Oryza sativa* | 74/75 | AAF19047 |
| Mads2 | *Dendrocalamus latiflorus* | 76/77 | AAR32119 |
| Mads1 | *Dendrocalamus latiflorus* | 78/79 | AAR32118 |
| mads3 | *Zea mays* | 80/81 | AAG43200 |
| SbMADS2 | *Sorghum bicolor* | 82/83 | AAB50181 |
| ZAP1 | *Zea mays* | 84/85 | AAB00081 |
| MADS2 | *Lolium temulentum* | 86/87 | AAD10626 |
| MADS2 | *Lolium perenne* | 88/89 | AAO45874 |
| m8 | *Hordeum vulgare* | 90/91 | CAB97354 |
| FDRMADS3 | *Oryza sativa* | 92/93 | AAL09473 |
| MADS15 | *Oryza sativa* | 94/95 | AAL09473 |
| TvFL2 | *Tradescantia virginiana* | 96/97 | AAP83415 |
| TvFL1 | *Tradescantia virginiana* | 98/99 | AAP83414 |
| TvFL3 | *Tradescantia virginiana* | 100/101 | AAP83416 |
| SQUA1 | *Elaeis guineensis* | 102/103 | AAQ03221 |
| AIFL | *Allium sp.* | 104/105 | AAP83362 |
| DOMADS2 | *Dendrobium grex* | 106/107 | AAF13261 |

### Example 9: Alignment of relevant polypeptide sequences

AlignX from the Vector NTI (Invitrogen) is based on the popular Clustal algorithm of progressive alignment (Thompson et a/. (1997) Nucleic Acids Res 25:4876-4882; Chenna et a/. (2003). Nucleic Acids Res 31:3497-3500). A phylogenetic tree can be constructed using a neighbour-joining clustering algorithm. Default values are for the gap open penalty of 10, for the gap extension penalty of 0,1 and the selected weight matrix is Blosum 62 (if polypeptides are aligned).

The result of the multiple sequence alignment using polypeptides relevant in identifying the ones useful in performing the methods of the invention is shown in Figure 6.

### Example 10: Calculation of global percentage identity between polypeptide sequences useful in performing the methods of the invention

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:
***Scoring matrix: Blosum62***
First Gap: 12
Extending gap: 2

Results of the software analysis are shown in Table E for the global similarity and identity over the full length of the polypeptide sequences (excluding the partial polypeptide sequences). Percentage identity is given above the diagonal and percentage similarity is given below the diagonal.

The percentage identity between the polypeptide sequences useful in performing the methods of the invention can be as low as 40 % amino acid identity compared to SEQ ID NO: 44.

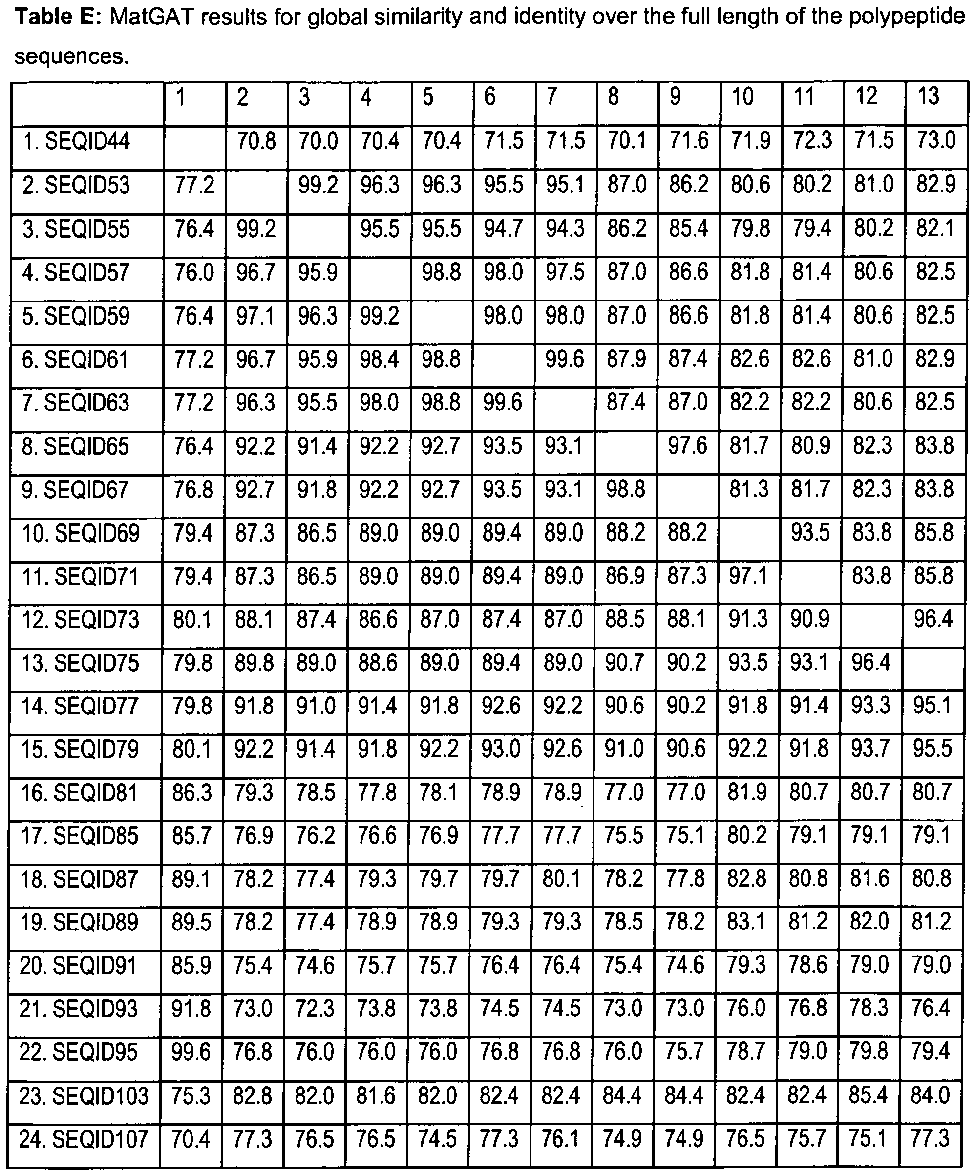

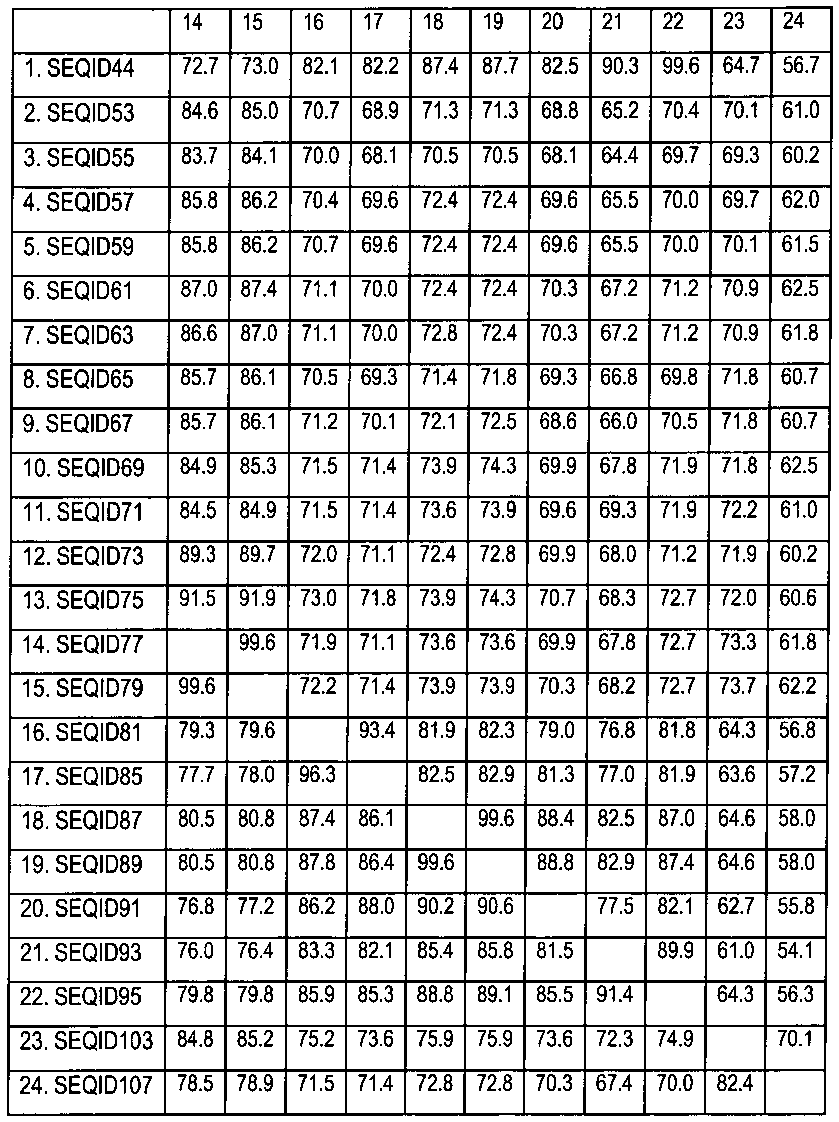

### Example 11: Identification of domains comprised in polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 44 are presented in Table F.

**Table F: InterPro scan results of the polypeptide sequence as represented by SEQ ID NO: 44**

| **Database** | **Accession number** | **Accession name** |
|---|---|---|
| PRINTS | PR00404 | MADSDOMAIN |
| PFAM | PF00319 | SRF-TF |
| SMART | SM00432 | MADS |
| PROFILE | PS50066 | MADS_BOX_2 |
| SUPERFAMILY | SSF55455 | SRF-like |
| PFAM | PF01486 | K-box |
| SUPERFAMILY | SSF46589 | tRNA-binding arm |
| PANTHER | PTHR11945 | MADS BOX PROTEIN |
| PANTHER | PTHR11945:SF19 | MADS BOX PROTEIN |

### Example 12: Topology prediction of the polypeptide sequences useful in performing the methods of the invention (subcellular localization, transmembrane...)

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 44 are presented Table G. The "plant" organism group has been selected, no cutoffs defined, and the predicted length of the transit peptide requested. The subcellular localization of the polypeptide sequence as represented by SEQ ID NO: 44 may be the cytoplasm or nucleus, no transit peptide is predicted.

**Table G: TargetP 1.1 analysis of the polypeptide sequence as represented by SEQ ID NO: 44**

| | |
|---|---|
| Length (AA) | 267 |
| Chloroplastic transit peptide | 0.088 |
| Mitochondrial transit peptide | 0.492 |
| Secretory pathway signal peptide | 0.046 |
| Other subcellular targeting | 0.655 |
| Predicted Location | Chloroplastic |
| Reliability class | 5 |
| Predicted transit peptide length | / |

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

### Example 13: Assay related to the polypeptide sequences useful in performing the methods of the invention

Lim et al. (PMB 44, 513-527, 2000) describe two assays for measuring protein-protein interactions that may be applied to MADS15. In the yeast two-hybrid assay, a truncated form of MADS1 (comprising the complete MADS box, the I- and K-domain but lacking the C-terminal part of the C-domain) was used as bait to test interaction with MADS15. In the *in vitro* pull-down assay, GST and GST-fused MADS1 proteins were produced and immobilised on glutathione Sepharose 4B. The resin-bound GST/GST-MADS1 was mixed with ³⁵S-labeled MADS15 proteins or fragments thereof. After washing, the interacting proteins were eluted and analysed by SDS-PAGE. A person skilled in the art will appreciate that the MADS15 protein may be used as bait in the two-hybrid screen or may be immobilised on glutathione resin as well. Furthermore, a MADS15 protein, when used according to the methods of the present invention, will result in increased root yield in rice, measured as an increased ratio of root biomass over shoot biomass.

### Example 14: Cloning of nucleic acid sequence as represented by SEQ ID NO: 43

The *Oryza sativa MADS15* gene was amplified by PCR using as template an *Oryza sativa* seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of 1.59 x 10⁷ cfu. Original titer was determined to be 9.6 x 10⁵ cfu/ml after first amplification of 6 x 10¹¹ cfu/ml. After plasmid extraction, 200 ng of template was used in a 50 µl PCR mix. Primers prm06892 (SEQ ID NO: 45; sense, start codon in bold, AttB1 site in italic: 5'-*ggggacaagtttgtacaaaaaagcaggcttaaaca* **atg**ggcgggggaaggt-3') and prm06893 (SEQ ID NO: 46; reverse, complementary, AttB2 site in italic: 5'-*ggggaccactttgtacaagaaagctgggt*ttggccgacgacgacgac-3'), which include the AttB sites for Gateway recombination, were used for PCR amplification. PCR was performed using Hifi Taq DNA polymerase in standard conditions. A PCR fragment of around 915 bp was amplified and purified also using standard methods. The first step of the Gateway procedure, the BP reaction, was then performed, during which the PCR fragment recombines in vivo with the pDONR201 plasmid to produce, according to the Gateway terminology, an "entry clone", pMADS15. Plasmid pDONR201 was purchased from Invitrogen, as part of the Gateway® technology.

### Example 15: Expression vector construction using the nucleic acid sequence as represented by SEQ ID NO: 43

The entry clone pMADS15 was subsequently used in an LR reaction with pGOS2, a destination vector used for *Oryza sativa* transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 108, alternatively, SEQ ID NO: 47 is equally useful) for constitutive expression was located upstream of this Gateway cassette.

After the LR recombination step, the resulting expression vector pGOS2::MADS15 (Figure 7) was transformed into *Agrobacterium* strain LBA4044 according to methods well known in the art.

### Example 16: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23°C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 ― 10 mm in length, they are cut and transferred to shoot elongation medium (MSBAP-0.5, containing 0.5 mg/I BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 17: Phenotypic evaluation procedure

### 17.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

### 17.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### 17.3 Parameters measured

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root biomass and shoot biomass in the period of active growth of root and shoot).

### Example 18: Results of the phenotypic evaluation of the transgenic plants

Upon analysis of the plants as described above, the inventors found that plants transformed with the *MADS15* gene construct had a higher root yield, expressed as root/shoot index, compared to plants lacking the *MADS15* transgene. The increase was 9.4% (p-value 0.0207) in T1 and 25.7% (p-value 0.0002) in T2. The p-values show that the increases were significant.

### Example section C: MADS15 downregulated

See also Examples 8 to 13 for the identification and characterisation of MADS15 related sequences.

### Example 19: Gene Cloning

The *Oryza sativa MADS15* gene was amplified by PCR using as template an *Oryza sativa* seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of 1.59 x 10⁷ cfu. Original titer was determined to be 9.6 x 10⁵ cfu/ml after first amplification of 6 x 10¹¹ cfu/ml. After plasmid extraction, 200 ng of template was used in a 50 µl PCR mix. Primers prm06892 (SEQ ID NO: 111; sense, start codon in bold, AttB1 site in italic: '-*ggggacaagtttgtacaaaaaagcaggcttaaaca***atg**gg cgggggaaggt-3') and prm06893 (SEQ ID NO: 112; reverse, complementary, AttB2 site in italic: 5'*-ggggaccactttgtacaagaaagctgggt*ttggccgacgacgacgac-3'), which include the AttB sites for Gateway recombination, were used for PCR amplification. PCR was performed using Hifi Taq DNA polymerase in standard conditions. A PCR fragment of around 915 bp was amplified and purified also using standard methods. The PCR fragment was subsequently used for the preparation of a hairpin construct, using techniques known in the art. The first step of the Gateway procedure, the BP reaction, was then performed, during which the hairpin construct recombines in vivo with the pDONR201 plasmid to produce, according to the Gateway terminology, an "entry clone", pMADS15hp. Plasmid pDONR201 was purchased from Invitrogen, as part of the Gateway® technology.

### Example 20: Vector Construction

The entry clone pMADS15hp was subsequently used in an LR reaction with p01519, a destination vector for the inverted repeat construct. This vector contains as functional elements within the T-DNA borders: a plant selectable marker; a screenable marker expression cassette; and a Gateway cassette intended for LR *in vivo* recombination such that the sequence of interest from the entry clone is integrated as an inverted repeat. A rice GOS2 promoter (SEQ ID NO: 174, alternatively, SEQ ID NO: 113 is equally useful) for constitutive expression was located upstream of this Gateway cassette.

After the LR recombination step, the resulting expression vector with the inverted repeat, Figure 10) were transformed into *Agrobacterium* strain LBA4044 and subsequently to *Oryza sativa* plants.

### Example 21: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (Zea *mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25°C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23°C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 22: Evaluation methods of plants transformed with the MADS15 inverted repeat under control of the rice GOS2 promoter

Approximately 15 to 20 independent T0 rice transformants were generated. The primary transformants were transferred from a tissue culture chamber to a greenhouse for growing and harvest of T1 seed. Eight events for the inverted repeat construct of which the T1 progeny segregated 3:1 for presence/absence of the transgene, were retained. For each of these events, approximately 10 T1 seedlings containing the transgene (hetero- and homozygotes) and approximately 10 T1 seedlings lacking the transgene (nullizygotes) were selected by monitoring visual marker expression. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The Areamax is the above ground area at the time point at which the plant had reached its maximal leafy biomass.

The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of the following seed-related parameters:
The flowers-per-panicle is a parameter estimating the average number of florets per panicle on a plant, derived from the number of total seeds divided by the number of first panicles. The tallest panicle and all the panicles that overlapped with the tallest panicle when aligned vertically, were considered as first panicles and were counted manually. The number of filled seeds was determined by counting the number of filled husks that remained after the separation step. The total seed yield (total seed weight) was measured by weighing all filled husks harvested from a plant. Total seed number per plant was measured by counting the number of husks harvested from a plant and corresponds to the number of florets per plant. These parameters were derived in an automated way from the digital images using image analysis software and were analysed statistically. Individual seed parameters (including width, length, area, weight) were measured using a custom-made device consisting of two main components, a weighing and imaging device, coupled to software for image analysis.

A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### Example 23: measurement of yield-related parameters for the inverted repeat construct transformants:

Upon analysis of the seeds as described above, the inventors found that plants transformed with the hairpin *MADS15* gene construct had a higher seed yield, expressed as number of filled seeds, total weight of seeds, total number of seeds, and flowers per panicle, compared to plants lacking the *MADS15* transgene, whereas the plants transformed with the sense *MADS15* gene construct showed opposite effects. The p-values show that the increases were significant.

The results obtained for plants in the T1 generation are summarised in Table H, which represent the mean values for all the tested lines:

**Table H:**

| | ***% difference*** | ***p-value*** |
|---|---|---|
| number of filled seeds | +122 | 0,0000 |
| total weight of seeds | +112 | 0,0000 |
| total number of seeds | +27 | 0,0000 |
| flowers per panicle | +25 | 0,0000 |

### Example Section D: PLT

### Example 24 : Identification of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et a/. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

The Tablel below provides a list of nucleic acid sequences related to the nucleic acid sequence used in the methods of the present invention.

**Table I: nucleic acid sequences related to the nucleic acid sequence used in the methods of the present invention**

| **Name** | **Nucleic acid SEQ ID NO** | **Polypeptide SEQ lD NO** | | **NCBI accession number** | **Source organism** |
|---|---|---|---|---|---|
| Arath_PLT1 | 175 | 176 | full length | NM_112975 (At3g20840) | *Arabidopsis thaliana* |
| Arath_PLT2 | 177 | 178 | full length | NM_103997 (At1g51190) | *Arabidopsis thaliana* |
| Glyma_PLT | 179 | 180 | full length | BU964973.1 CA783156.1 BM309051.1 BM309377.1 | *Glycine max* |
| Glyma_PLT2 | 181 | 182 | full length | BU926204.1 BU547204.1 CA783156.1 BU927164.1 | *Glycine max* |
| Medtr_PLT | 183 | 184 | full length | AC144930.20 | *Medicago truncatula* |
| Orysa_PLT | 185 | 186 | full length | NM_190301 | *Oryza sativa* |
| Zeama_PLT | 187 | 188 | full length | CS155772.1 | *Zea mays* |
| Lotco_PLT | 189 | 190 | partial | AP007400 | *Lotus comiculatus* |
| Poptr_PLT I | 199 | 200 | full length | scaff_III.1595 | *Populus tremuloides* |
| Poptr_PLT II | 201 | 202 | full length | scaff_I.328 | *Populus tremuloides* |
| Vitvi_PLT | 203 | 204 | partial | AM469514 | *Vitis vinifera* |
| Brana_PLT | 205 | 206 | partial | CN730825 | *Brassica napus* |
| Phaco_PLT | 207 | 208 | partial | CA902624.1] | *Phaseolus coccineus* |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

### Example 25: Cloning of the nucleic acid sequences used in the methods of the invention

The nucleic acid sequences used in the methods of the invention were amplified by PCR using as template a custom-made *Arabidopsis thaliana* cDNA library made starting from RNA extracted from different tissues (in pCMV Sport 6.0; Invitrogen, Paisley, UK). PCR was performed using Hifi Taq DNA polymerase in standard conditions, using 200 ng of template in a 50 µl PCR mix. The primers used to amplify SEQ ID NO: 175 (PLT1) were prm08180 (SEQ ID NO: 195; sense, start codon in bold, AttB1 site in italic: 5' *GGGGACAAGTTTGTA CAAAAAAGCAGGCTTAAACA***ATG**ATCAATCCACACGGTG 3') and prm08181 (SEQ ID NO: 196; reverse, complementary, AttB2 site in italic: 5' *GGGGACCACTTTGTACAAGAAAG CTGGGT*TCCTTGTTTACTCATTCCACA 3'), which include the AttB sites for Gateway recombination. The primers used to amplify SEQ ID NO: 177 (PLT2) were prm08182 (SEQ ID NO: 197; sense, start codon in bold, AttB1 site in italic: 5' *GGGGACAAGTTTGTACAAAAAAGCAGGCTTAAACA***ATG**AATTCTAACA ACTGGCTC 3') and prm08183 (SEQ ID NO: 198; reverse, complementary, AttB2 site in italic: 5' *GGGGACCACTTTGTACAAGAAAGCTGGGT*TCATCTTTTATTCATTCCACA 3'),

The amplified PCR fragments were purified also using standard methods. The first step of the Gateway procedure, the BP reaction, was then performed, during which the PCR fragment recombines in vivo with the pDONR201 plasmid to produce, according to the Gateway terminology, an "entry clone", pPLT1 for SEQ ID NO: 175 and pPLT2 for SEQ ID NO: 177. Plasmid pDONR201 was purchased from Invitrogen, as part of the Gateway® technology.

### Example 26: Expression Vector Construction

The entry clones pPLT1 and pPLT2 were subsequently used in an LR reaction with destination vectors used for *Oryza sativa* transformation. A first vector contains as functional elements within the T-DNA borders: a plant selectable marker; a screenable marker expression cassette; and a Gateway cassette intended for LR in vivo recombination with the nucleic acid sequence of interest already cloned in the entry clone. A rice constitutive promoter, a GOS2 promoter (SEQ ID NO: 210, alternatively SEQ ID NO: 194 is equally useful) was located upstream of this Gateway cassette.

A second vector contains the same functional elements within the T-DNA borders: a plant selectable marker; a screenable marker expression cassette; and a Gateway cassette intended for LR in vivo recombination with the nucleic acid sequence of interest already cloned in the entry clone. A rice promoter for expression in meristems, an MT promoter (SEQ ID NO: 211) (PRO0126) was located upstream of this Gateway cassette.

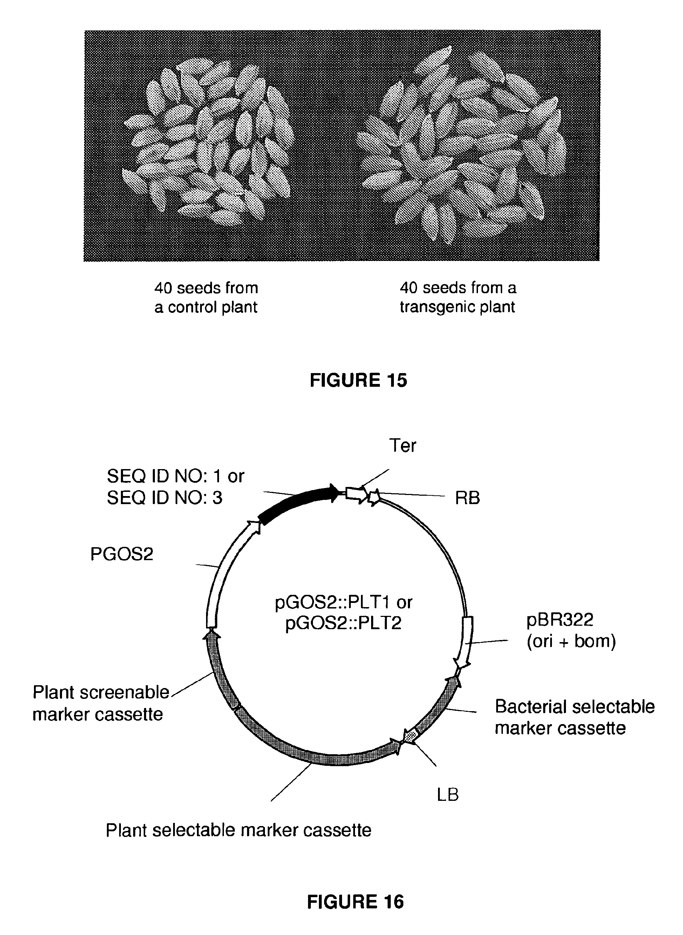

After the LR recombination step, the resulting expression vectors, pGOS2::PLT1, pGOS2; PLT2, pMT::PLT1 and pMT::PLT2 (Figure 16 shows the construct with the GOS2 promoter) were independently transformed into *Agrobacterium* strain LBA4044 and subsequently to *Oryza sativa* plants. Transformed rice plants were allowed to grow and were then examined for the parameters described below.

### Example 27: Crop transformation

The transformed *Agrobacterium* icontaining the expression vectors were used independently to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K₂SO₄, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 28: Phenotypic evaluation procedure

### 28.1 Evaluation setup

### Evaluation in normal growth conditions

The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

### Evaluation under reduced nitrogen availability

The rice plants were grown in potting soil as under normal conditions except for the nutrient solution. The pots were watered from transplantation to maturation with a specific nutrient solution containing reduced N nitrogen (N) content, usually between 7 to 8 times less. The rest of the cultivation (plant maturation, seed harvest) was the same as for plants not grown under abiotic stress.

### 28.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### 28.3 Parameters measured

### Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass, areamax) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

### Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Example 29: Results of the phenotypic evaluation of the transgenic plants

### 29.1 Results of the phenotypic evaluation of the transgenics plants grown under normal growth conditions, expressing either PLT1 or PLT2 nucleic acid sequence under the control of a constitutive promoter

The TKW measurement results of the T1 seeds of PLT1 and PLT2 transgenic rice plants grown under normal growth conditions, are shown in Table J, in absolute values (averaged events) and as a percentage compard to wild type plants. A substantial increase in TKW is observed for the transgenic seeds containing either construct, compared to wild type seeds.

**Table J : Results of TKW measurements of the T1 seeds of PLT1 and PLT2 transgenic plants grown under normal growth conditions.**

| | **TKW (g)** | **% increase** |
|---|---|---|
| PLT1 transgenics | 0.0290 | 16% |
| PLT2 transgenics | 0.0288 | 15% |
| WT | 0.0250 | |

Individual seed parameters (including width, length, area, weight) were measured using a custom-made device consisting of two main components, a weighing and imaging device, coupled to software for image analysis.

The seed area and seed length measurement results of T1 seeds of PLT1 and PLT2 transgenic rice plants are shown in Table K in absolute values (averaged events) and as a percentage compard to wild type plants. A clear increase in both seed area and seed length is observed for the transgenic seeds containing either construct, compared to wild type seeds.

**Table K : Results of seed area and seed length measurements of the T1 seeds of PLT1 and PLT2 transgenic plants grown under normal growth conditions.**

| | **Seed area (mm²)** | **% increase** | **Seed length (mm)** | **% increase** |
|---|---|---|---|---|
| PLT1 transgenics | 27,60 | 12% | 9,4 | 13% |
| PLT2 transgenics | 27,35 | 11% | 9,4 | 13% |
| WT | 24,56 | | 8,3 | |

Seed width was not significantly affected on the T1 seeds of the PLT and PLT2 transgenic plants (data not shown).

### 29.2 Results of the phenotypic evaluation of the transgenics plants grown under reduced nitrogen availability conditions, expressing either PLT1 or PLT2 nucleic acid sequence under the control of a constitutive promoter

The TKW measurement results of the T1 seeds of PLT1 and PLT2 transgenic rice plants grown under reduced nitrogen availability conditions, are shown in Table L, in absolute values (averaged events) and as a percentage compard to wild type plants. A substantial increase in TKW is observed for the transgenic seeds containing either construct, compared to wild type seeds.

**Table L : Results of TKW measurements of the T1 seeds of PLT1 and PLT2 transgenic plants grown under reduced nitrogen availability conditions.**

| | **TKW (g)** | **% increase** |
|---|---|---|
| **PLT1 transgenics** | 0.0295 | 12% |
| **PLT2 transgenics** | 0.0278 | 8% |
| **WT** | 0.0256 | |

### 29.3 Results of the phenotypic evaluation of the transgenics plants grown under normal growth conditions, expressing PLT2 nucleic acid sequence under the control of a meristem-specific promoter

The TKW measurement results of the T1 seeds PLT2 transgenic rice plants grown under normal growth conditions, expressing PLT2 nucleic acid sequence under the control of a meristem-specific promoter, are shown in Table M, in absolute values (averaged events) and as a percentage compard to wild type plants. A substantial increase in TKW is observed for the transgenic seeds containing the construct, compared to wild type seeds.

**Table M : Results of TKW measurements of the T1 seeds of PLT2 transgenic plants grown under normal growth conditions, expressing PLT2 nucleic acid sequence under the control of a mersitem-specific promoter.**

| | **TKW (g)** | **% increase** |
|---|---|---|
| PLT2 transgenics | 0.0263 | 3% |
| WT | 0.0254 | |

### Example Section E: bHLH

### Example 30: Identification of paralogues of the bHLH of SEQ ID NO: 213 in rice

SEQID 2 was used to search for paralogues in the rice genome using a BLASTP algorithm used to perform similarity searches of a protein query sequence to a given database of protein sequences. The protein database queried corresponded to the rice proteome of the MIPS institute, the MIPS Oryza sativa Database (MOsDB), comprising 59,712 sequences; 27,051,637 total letters. Results were ranked according to highest similarity as determined from highest score and lowest e-value. Hits identifying bHLH protein sequences paralogous to SEQID NO: 2 had a score of at least 50 and an e-value lower than e-05. Pairwise alignments between the query sequence and the *de novo* identified paralogues are shown below.

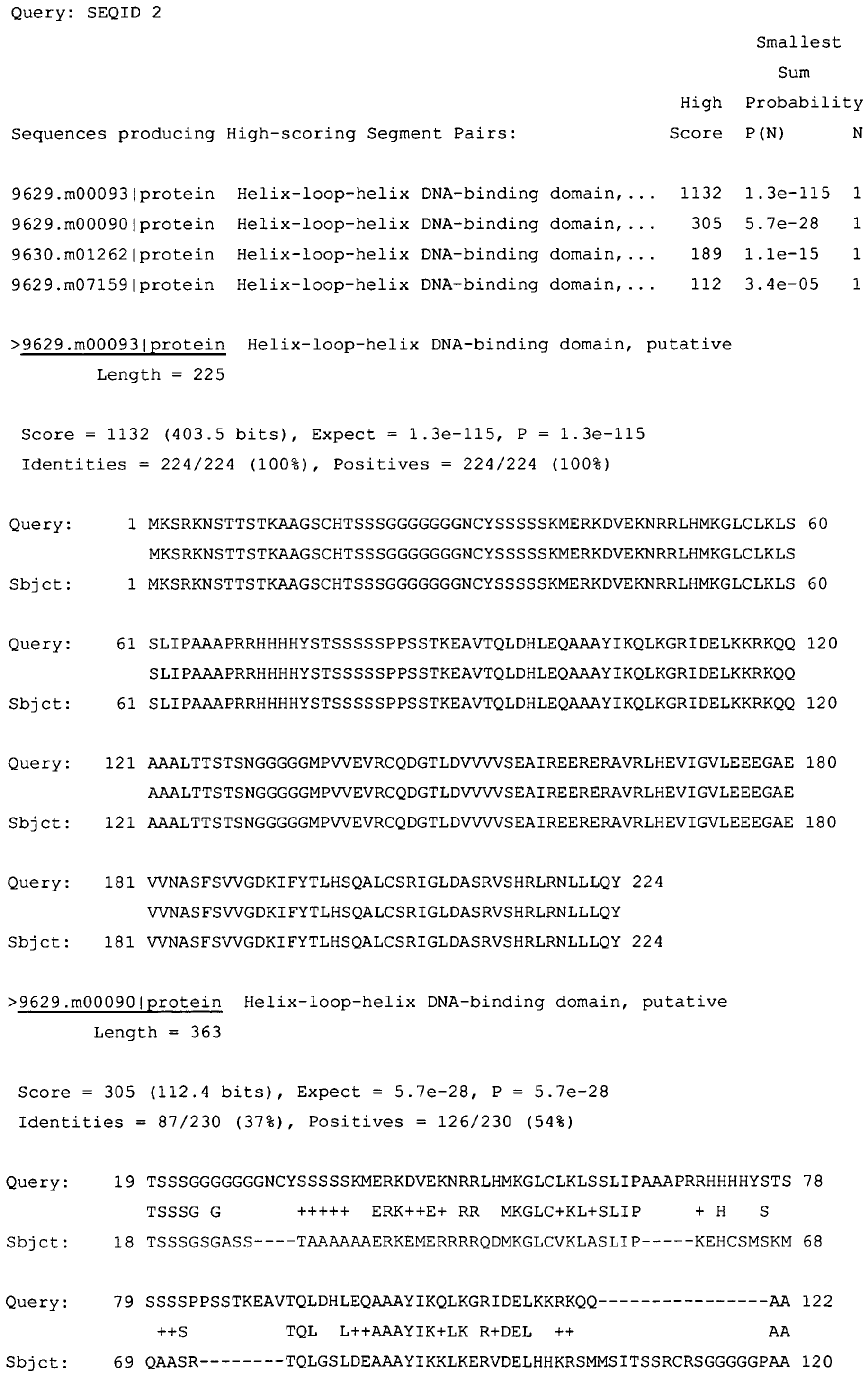

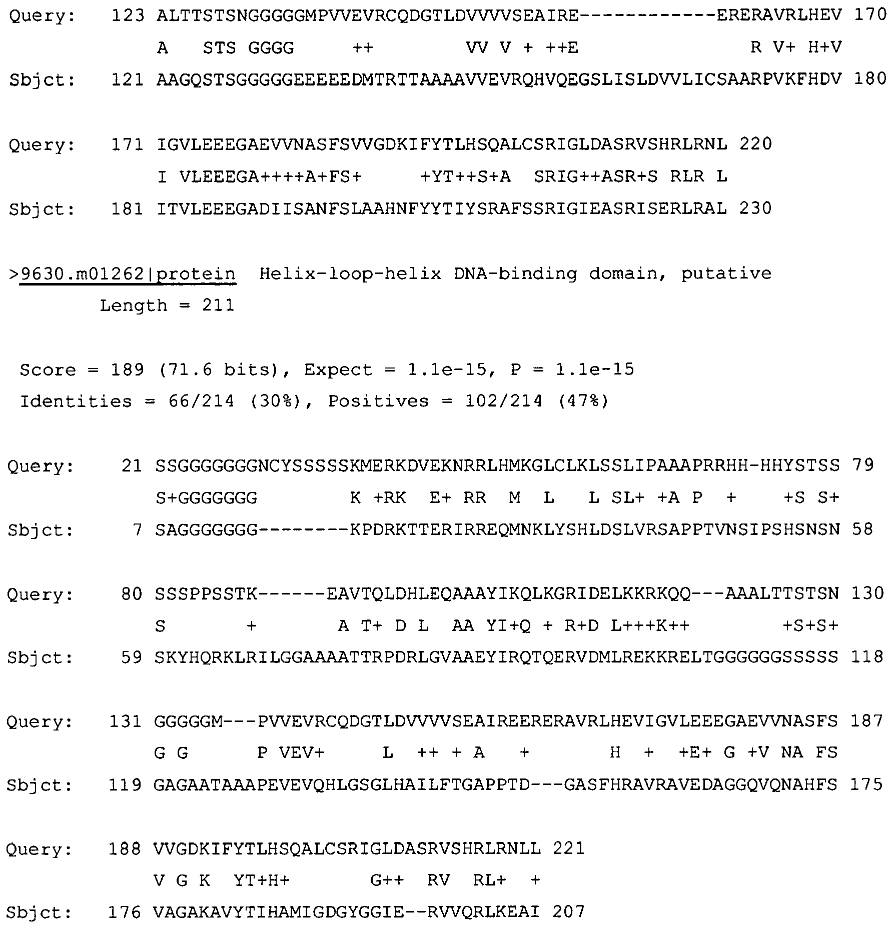

### Example 31: Identification of orthologues of the bHLH of SEQ ID NO: 213 in Arabidopsis thaliana

SEQID 2 was used to search for orthologues in the *Arabidopsis* genome using a BLASTP algorithm used to perform a similarity search of a protein query sequence to a given database of protein sequences. The protein database queried corresponded to the *Arabidopsis* proteome of the MIPS institute, the MIPS *Arabidopsis thaliana* database (MAtDB) comprising 26,735 sequences; 11,317,104 total letters. Results were ranked according to highest similarity as determined from highest score and lowest e-value. Hits identifying bHLH protein sequences orthologous to SEQID2 had a score of at least 50 and an e-value lower than e-05. Pairwise alignments between the query sequence and the *de novo* identified orthologue are shown below.

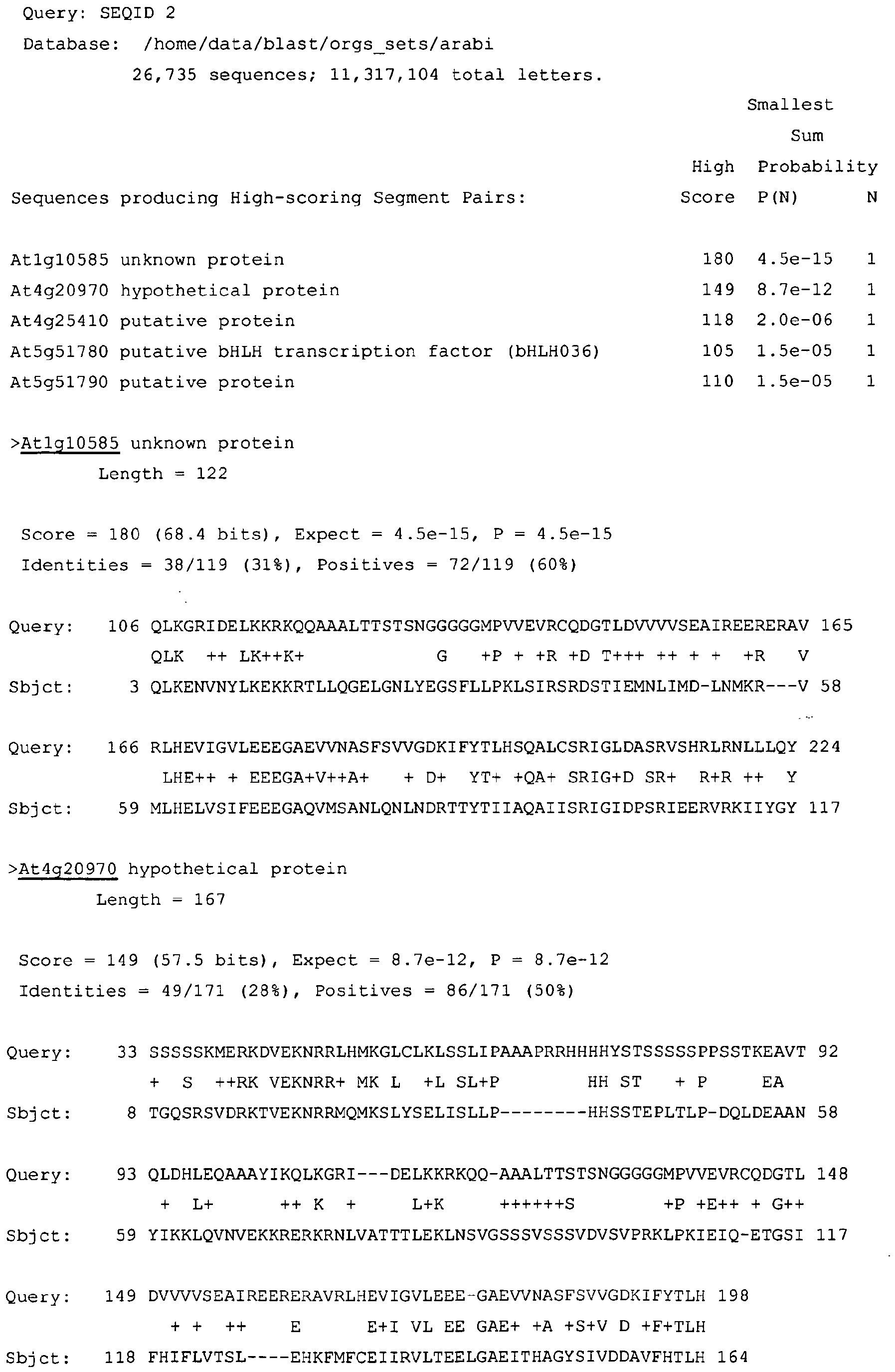

### Example 32: Identification of a bHLH from Medicago truncatula

The question to be addressed was whether the query sequence (SEQ ID NO: 227 from *Medicago truncatula*) was a bHLH polypeptide according to the definition applied herein. SEQ ID 227 was compared to the *Arabidopsis* proteome database of the MIPS institute.

Comparison was carried out using the BLASTP 2.0MP-WashU algorithm, which performs similarity searches of a protein query sequence to a given database of protein sequences. (Reference: Gish, W. (1996-2002)). The parameters used in the comparison were E value 10 Cutoff score (S2): 56

The protein database queried corresponded to the *Arabidopsis* proteome of the MIPS institute comprising 26,735 sequences (Database: /home/data/blast/orgs_sets/arabi 26,735 sequences; 11,317,104 total letters). Results were ranked according to highest similarity as determined from highest score and lowest e-value. The first Hit identified (underlined in the alignment) corresponded to SEQ ID NO: 225 (At4g20970) indicating that the sequence is a bHLH polypeptide according to the definition applied herein. Pairwise alignments between the query sequence from *Medicago* and the first hit corresponding to the *de novo* identified bHLH are shown below.

### BLASTP 2.0MP-WashU [09-Sep-2002] [decunix4.0-ev56-I32LPF64 2002-09-09T17:45:09]

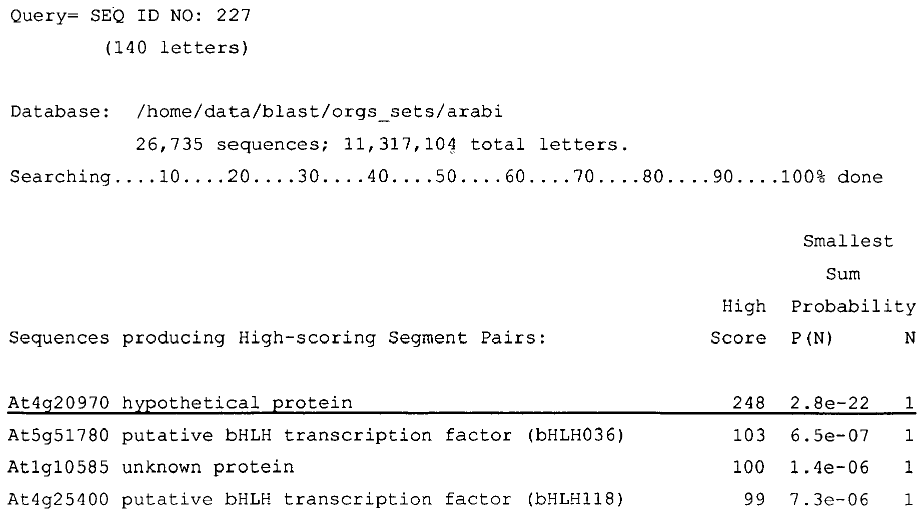

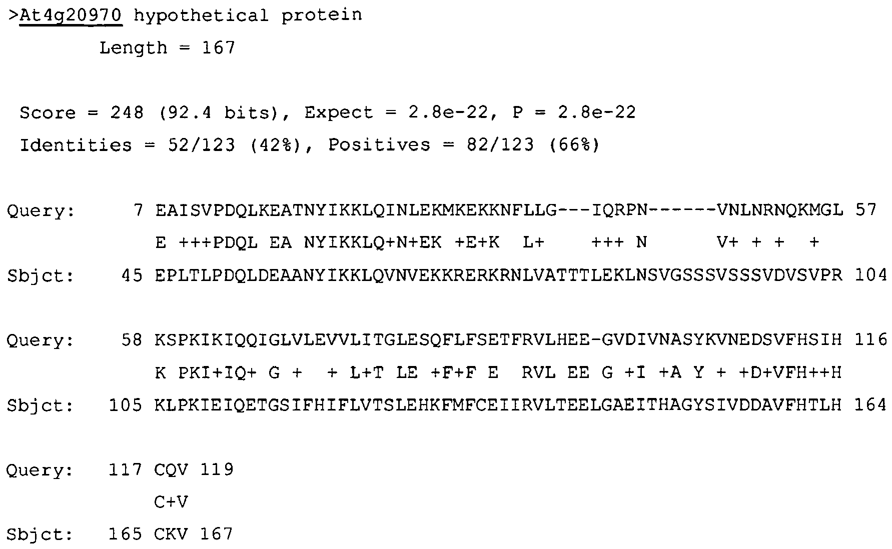

### Example 33: Identification of sequences related to the nucleic acid sequence used in the methods of the invention

bHLH Sequences, whether nucleotide (full length cDNA, ESTs or genomic) or protein (full length or partial polypeptides), were used to identified other bHLH nucleotide or proteins sequences amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program was used to find regions of local similarity between sequences by comparing nucleic acid or polypeptide sequences to sequence databases and by calculating the statistical significance of matches. For example, the polypeptide encoded by SEQ ID NO: 213 was used as query to the nr (non-redundant: (Database: All GenBank+EMBL+DDBJ+PDB sequences (but no EST, STS, GSS,environmental samples or phase 0, 1 or 2 HTGS sequences) 3,819,973 sequences; 16,928,533,343 total letters) database sequence using the TBLASTN algorithm, with default settings and the filter to ignore low complexity sequences. The output of the analysis (shown below) was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length.

Hits on the database identifying BHLH proteins produced a score of at least 50 and an e-value equal to or lower than e-05.

| Sequences producing significant alignments: | Score (Bits) | E Value |
|---|---|---|
| gi\|55765745\|ref\|NM_183508.2\| Oryza sativa (japonica cultivar-gro | 440 | 1e-121 |
| gi\|42821746\|dbj\|AK109432.2\| Oryza sativa (japonica cultivar-g... | 440 | 1e-121 |
| gi\|55769744\|ref\|XM_549862.1\| Oryza sativa (japonica cultivar-gro | 247 | 1e-105 |
| gi\|58530787\|dbj\|AP008207.1\| Oryza sativa (japonica cultivar-g... | 263 | 2e-68 |
| gi\|17385651\|dbj\|AP002845.3\| Oryza sativa (japonica cultivar-g... | 263 | 2e-68 |
| gi\|32980356\|dbj\|AK070332.1\| Oryza sativa (japonica cultivar-g... | 251 | 9e-65 |
| gi\|348941351\|ref\|NM_183504.1\| Oryza sativa (japonica cultivar-gro | 137 | 3e-30 |
| gi\|15293050\|gb\|AY050959.1\| Arabidopsis thaliana unknown prote... | 80.1 | 6e-16 |
| gi\|79340923\|ref\|NM_100934.2\| Arabidopsis thaliana transcripti... | 87.8 | 2e-15 |
| gi\|58531195\|dbj\|AP008214.1\| Oryza sativa (japonica cultivar-g... | 51.6 | 4e-13 |
| gi\|42408246\|dbj\|AP004557.3\| Oryza sativa (japonica cultivar-g... | 51.6 | 4e-13 |
| gi\|42408168\|dbj\|AP004376.3\| Oryza sativa (japonica cultivar-g... | 51.6 | 4e-13 |
| gi\|22328837\|ref\|NM_118215.2\| Arabidopsis thaliana DNA binding... | 78.2 | 1e-12 |
| gi\|7268888\|emb\|AL161554.2\|ATCHRIV54 Arabidopsis thaliana DNA chr | 77.8 | 2e-12 |
| gi\|5262774\|emb\|AL080282.1\|ATT13K14 Arabidopsis thaliana DNA c... | 77.8 | 2e-12 |
| gi\|50906596\|ref\|XM_464787.1\| Oryza sativa (japonica cultivar-gro | 77.0 | 3e-12 |

### Example 34: Determination of global similarity and identity between bHLH transcription factors

Global percentages similarity and identity between bHLH polypeptides was determined using the MatGAT software (BMC Bioinformatics. 2003 Jul 10;4:29. MatGAT: an application that generates similarity/identity matrices using protein or DNA sequences. Campanella JJ, Bitincka L, Smalley J.). MatGAT software generates similarity/identity matrices for DNA or protein sequences without needing pre-alignment of the data. The program performs a series of pair-wise alignments, calculates similarity and identity, and then places the results in a distance matrix.

Parameters used in the comparison were:
Scoring matrix: Blosum62
First Gap: 12
Extending gap: 2

Results are shown in Figure 19a.

### Example 35: Determination of global similarity and identity between bHLH domains in bHLH polypeptides

bHLH domains were mapped using the SMART software (Schultz et al. (1998) Proc. Natl. Acad. Sci. USA 95, 5857-5864; Letunic et al. (2006) Nucleic Acids Res 34, D257-D260). Smart software is available through EMBL institute(European Molecular Biology Laboratory **(EMBL).**

The sequences of the bHLH domain used are given below.
Hv_BHLH
   KESEKERRKRMKALCEKLASLIPREHCCSTTDTMTQLGSLDVGASYIKKLKERVDE
OS_NP_908393_BHLH
   KEMERRRRQDMKGLCVKLASLIPKEHCSMSKMQAASRTQLGSLDEAAAYIKKLKERVDE
OS_NP_908397.1_BHLH 
KDVEKNRRLHMKGLCLKLSSLIPAAAPRRHHHYSTSSSSSPPSSTKEAVTQLDHLEQAAAYIKQLKGR
IDE
Os_XP_464787.1_BHLH 
KTTERIRREQMNKLYSHLDSLVRSAPPTVNSIPSHSNSNSKYHQRKLRILGGAAAATTRPDRLGVAAEY
IRQTQERVDM
AT1G10585_bHLH
   nlrekdrrmrmkhlfsilsshvsptrklpvphlidqatsymiqlkenvny
At4g20970_bHLH
   KTVEKNRRMQMKSLYSELISLLPHHSSTEPLTLPDQLDEAANYIKKLQVNVEK

Global percentages of similarity and identity between bHLH domains of bHLH polypeptides were determined using the software and parameters described in Example 34.

Results are shown in Figure 19b.

### Example 36: Cloning of the nucleic acid sequence used in the methods of the invention

The nucleic acid sequence used in the methods of the invention was amplified by PCR using as template a custom-made Oryza *sativa* seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm06808 (SEQ ID NO: 231; sense, start codon in bold, AttB1 site in italic: 5'-ggggacaagtttgtacaaaaaagcaggcttaaacaatgaagagcaggaagaacagc 3') and prm06809 (SEQ ID NO: 232; reverse, complementary, AttB2 site in italic: 5' ggggaccactttgtacaagaaagctgggtgcagagtgaaagagtggtgtg 3'), which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pbHLH. Plasmid pDONR201 was purchased from Invitrogen, as part of the Gateway® technology.

### Example 37: Expression Vector Construction

The entry clone p076 was subsequently used in an LR reaction with pGOS2, a destination vector used for *Oryza sativa* transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 233, alternatively, SEQ ID NO: 230 is equally useful) for constitutive expression (internal reference PRO0129) was located upstream of this Gateway cassette.

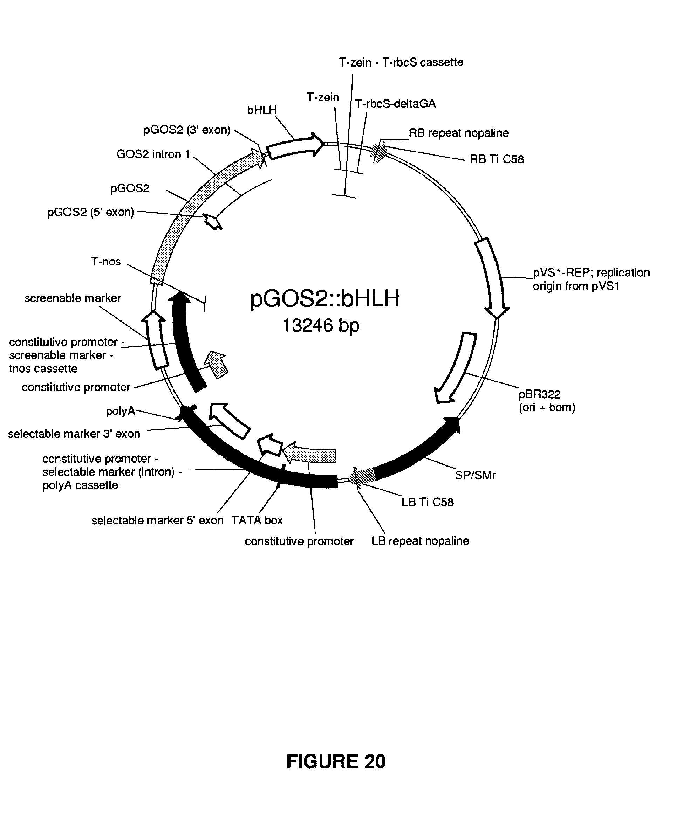

After the LR recombination step, the resulting expression vector pGOS2::bHLH (Figure 20) was transformed into *Agrobacterium* strain LBA4044 and subsequently to *Oryza sativa* plants.

### Example 38: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 30 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25°C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25°C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 ― 10 mm in length, they are cut and transferred to shoot elongation medium (MSBAP-0.5, containing 0.5 mg/l BAP). Shoots of about 2 cm in length are transferred to the rooting medium (MSO) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839―847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839―847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 39: Evaluation procedure

### 39.1 Evaluation setup

Approximately 30 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048×1536 pixels, 16 million colours) were taken of each plant from at least 6 different angles.

### 39.2 Statistical analysis: t-test and F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### Example 40: Evaluation results

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Early vigour (as determined by aboveground area) was seen in six out of seven events of the T1 generation, with an overall increase in aboveground area for transgenic seedlings of 22% compared to control plants. Four of these T1 events were further evaluated in the T2 generation, and all four of these events gave an increase in aboveground area for transgenic seedlings compared to control plants, with an overall increase in aboveground area for transgenic seedlings of 13% compared to control plants. The results were also shown to be statistically significant with the p-value from the F-test being 0.0007 (T2 generation) indicating that the effect seen is likely due to the transgene rather than the position of the gene or a line effect.

### Example Section F: SPL15

### Example 41: Identifcation of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. The polypeptide encoded by the nucleic acid of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search

The Table N below provides a list of nucleic acid sequences related to the nucleic acid sequence used in the methods of the present invention.

**Table N: nucleic acid sequences related to the nucleic acid sequence (SEQ ID NO: 234) used in the methods of the present invention, and the corresponding deduced polypeptides**

| **Name** | **Nucleic acid SEQ ID NO** | **Polypeptide SEQ ID NO** | **Sequence length** | **NCBI accession number** | **Source organism** |
|---|---|---|---|---|---|
| Arath_SPL15 (At3g57920) | 234 | 235 | Full length | NM_115654.1 | *Arabidopsis thaliana* |
| Arath_SPL9 (At2g42200) | 236 | 237 | Full length | AY150378 | *Arabidopsis thaliana* |
| Aqufo_SPL | 238 | 239 | Full length | contig of DR915312 DR949057.1 | *Aquilegia formosa x Aquilegia pubescens* |
| Goshi_SPL | 240 | 241 | Full length | DT566400 | *Gossypium hirsutum* |
| lponi_SPL | 242 | 243 | Full length | contig of BJ576204.1 BJ556115 BJ567301 | *Ipomoea nil* |
| Lacsa_SPL | 244 | 245 | Full length | contig of DY966949 DW119178 | *Lactuca sativa* |
| Maldo_SPL | 246 | 247 | Full length | contig of CN891102.1 C0868185.1 CV523507 | *Malus domestica* |
| Medtr_SPL | 248 | 249 | Full length | spliced from AC170989.2 | *Medicago truncatula* |
| Nicbe_SPL | 250 | 251 | Full length | contig of CK284078.1 CK294165 | *Nicotiana bentamiana* |
| Orysa_SPL | 252 | 253 | Full length | XM_483285 | *Oryza sativa* |
| Orysa_SPL II | 254 | 255 | Full length | spliced from AC108762 | *Oryza sativa* |
| Soltu_SPL | 256 | 257 | Full length | contig of CK246692.1 CK254420.1 | *Solanum tuberosum* |
| Vitvi_SPL | 258 | 259 | Full length | contig of CV098277 CV092812.1 | *Vitis vinifera* |
| Zeama_SPL | 260 | 261 | Full length | contig of EB160653 DY235599 DV029129 | *Zea mays* |
| Zeama_SPL II | 262 | 263 | Full length | contig of AJ011619 DV033513.1 DY532686.1 | *Zea mays* |
| Sorpr_SPL | 264 | 265 | Partial | BF422188 | *Sorghum propinquium* |
| Allce_SPL | 266 | 267 | Partial | CF444518.1 | *Allium cepa* |
| Antma_SPL | 268 | 269 | Partial | AMA011623 | *Antirrhinum majus* |
| Brana_SPL | 270 | 271 | Partial | CX189447 | *Brassica napus* |
| Sacof_SPL | 272 | 273 | Partial | contig of CA113070 CA254724 | *Saccharum officinarum* |
| Fesar_SPL | 274 | 275 | Partial | DT706587.1 | *Festuca arundinacea* |
| Brara_SPL | 282 | 283 | Full length | AC189445.1 | Brassica rapa |
| Glyma_SPL | 284 | 285 | Full length | CX708501.1 BG651519.1 | Glycine max |
| Poptr_SPL | 286 | 287 | Full length | scaff_XVI416 | Populus tremuloides |
| Citcl_SPL | 288 | 289 | Partial | DY293795 | Citrus clementina |
| Betvu_SPL | 290 | 291 | Partial | BQ594361.1 | Beta vulgaris |
| Hevbr_SPL | 292 | 293 | Partial | EC604947 | Hevea brasiliensis |

### Example 42: Determination of global similarity and identity between SPL15 transcription factors, and their SPL DBD.

Global percentages of similarity and identity between SPL15 transcription factors were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line. The sequence of SEQ ID NO: 235 is indicated as number 5 in the matrix.

Parameters used in the comparison were:
Scoring matrix: Blosum62
First Gap: 12
Extending gap: 2

Results of the software analysis are shown in Table O for the global similarity and identity over the full length of the SPL15 transcription factor polypeptides. Percentage identity is given above the diagonal and percentage similarity is given below the diagonal. Percentage identity between the SPL15 transcription factor paralogues and orthologues ranges between 30 and 70%, reflecting the relatively low sequence identity conservation between them outside of the SPL DBD.

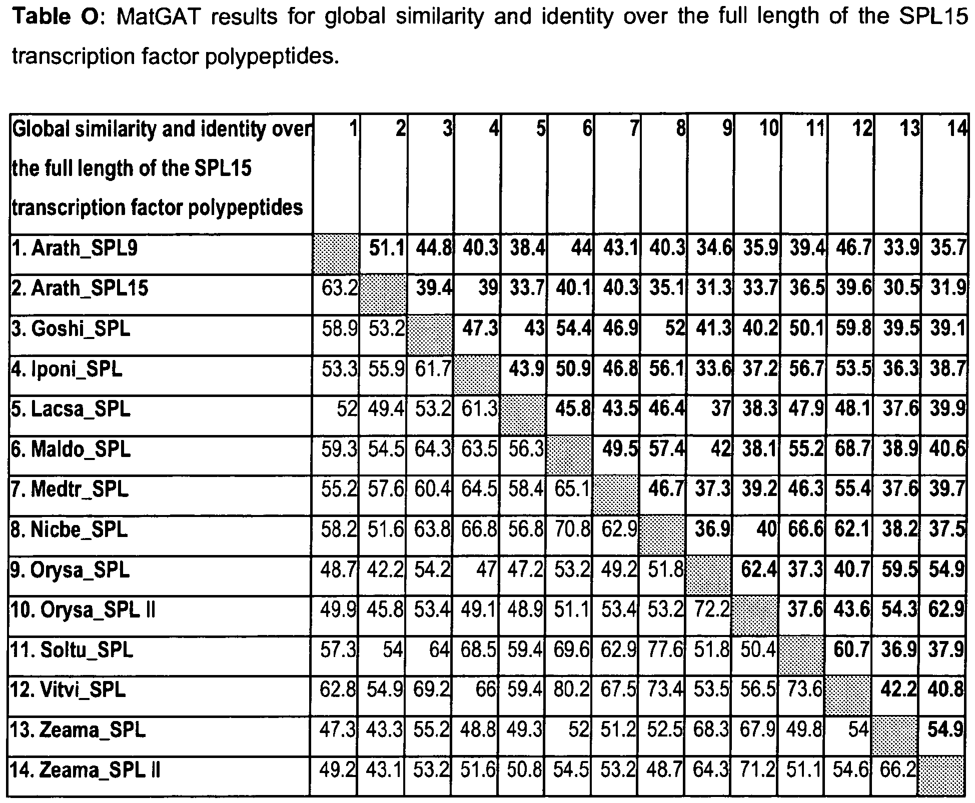

Results of the software analysis are shown in Table P for the **global similarity and identity over the SPL DBD of the** SPL15 transcription factor **polypeptides.** Percentage identity is given above the diagonal and percentage similarity is given below the diagonal. Percentage identity between the SPL DBD of SPL15 transcription factor paralogues and orthologues ranges between 70% and 100%.

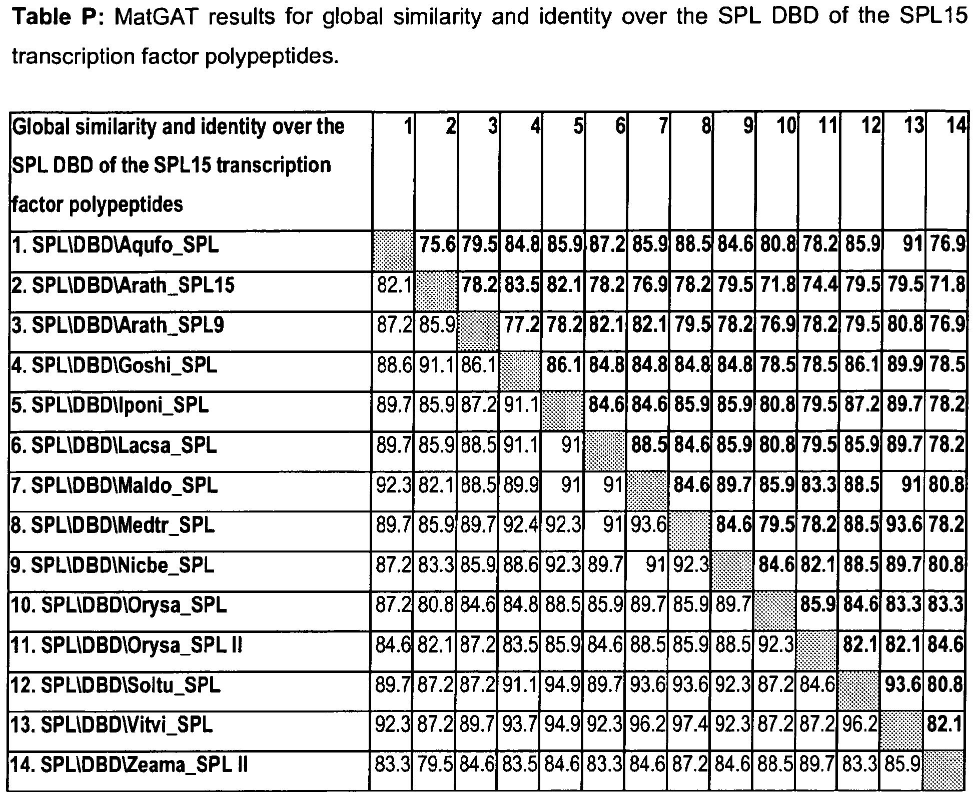

### Example 43: Cloning of the nucleic acid sequence used in the methods of the invention

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

The nucleic acid sequence used in the methods of the invention was amplified by PCR using as template a custom-made *Arabidopsis thaliana* mixed tissues cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm07277 (SEQ ID NO: 280; sense, start codon in bold, AttB1 site in lower case: 5'-*ggggacaagtttgtacaaaaaagcaggcttaaaca***ATG**GAGTTGTTAATGTGTTCG 3') and prm07278 (SEQ ID NO: 281; reverse, complementary, AttB2 site in lower case: 5' *ggggaccactttgtacaagaaagctgggt*TGATGAAGATCTTAAAAGGTGA 3'), which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pSPL15. Plasmid pDONR201 was purchased from Invitrogen, as part of the Gateway® technology.

### Example 44: Expression Vector Construction

The entry clone p13075 was subsequently used in an LR reaction with p06659, a destination vector used for *Oryza sativa* transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice HMGB promoter (SEQ ID NO: 294) for constitutive expression was located upstream of this Gateway cassette. Alternatively, the HMGB promoter represented by SEQ ID NO: 46 is equally useful. A similar construct was made with the *SPL15* coding sequence under control of the constitutive GOS2 promoter (SEQ ID NO: 295).

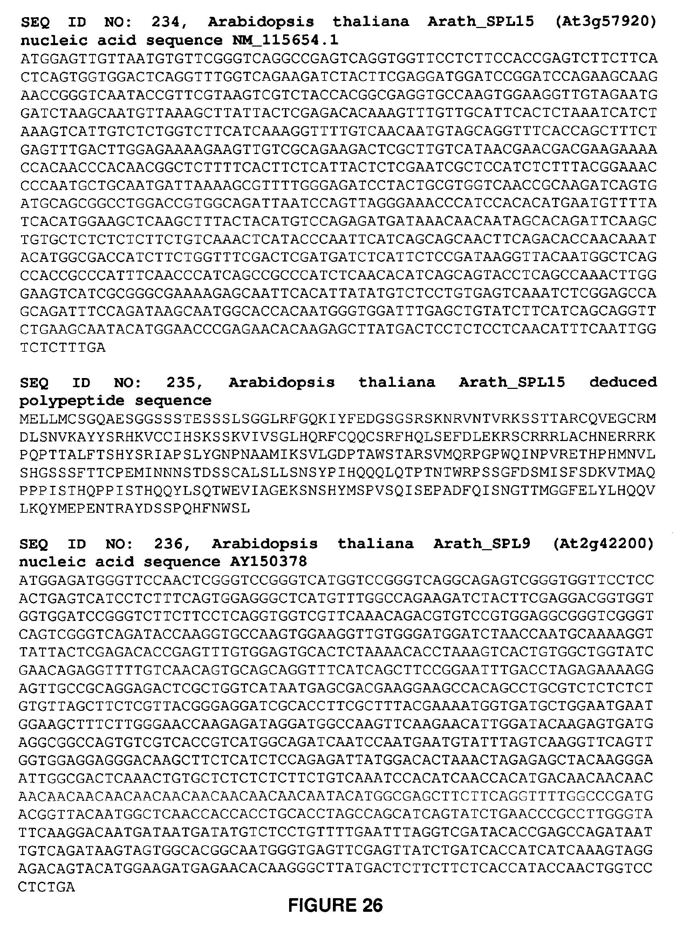

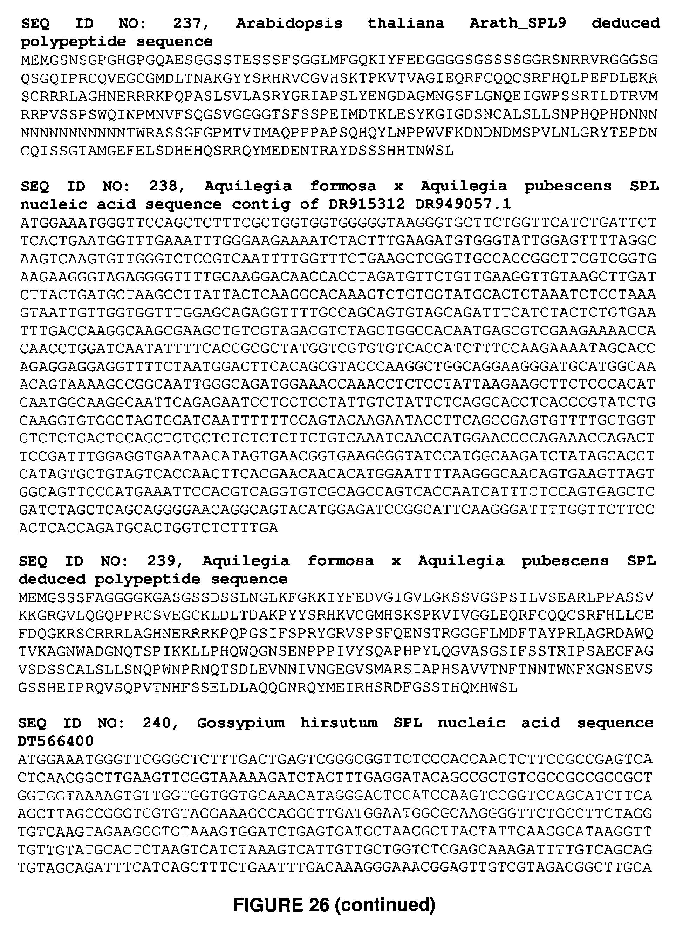

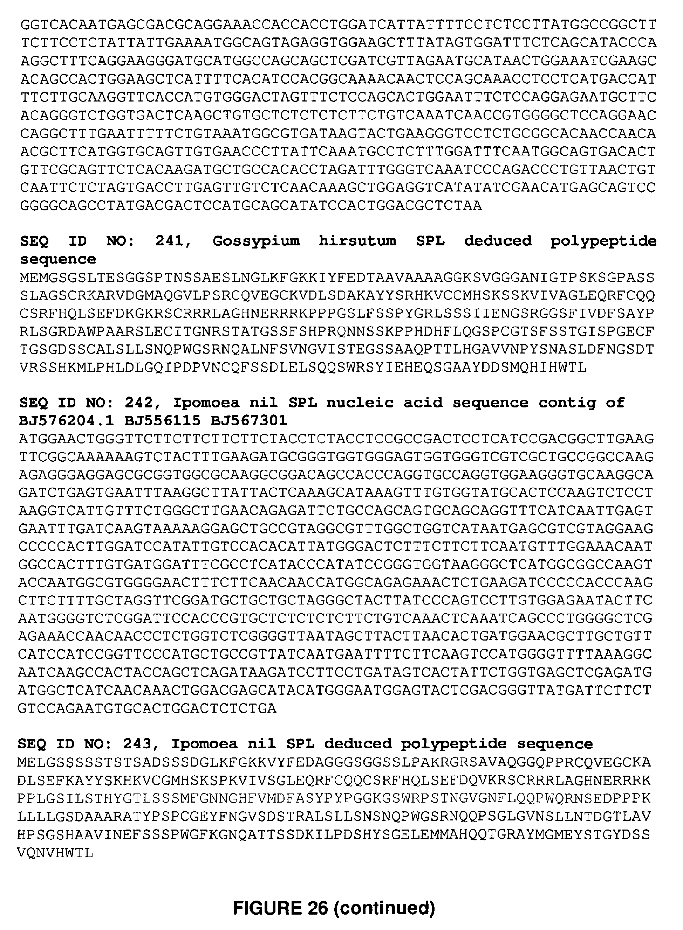

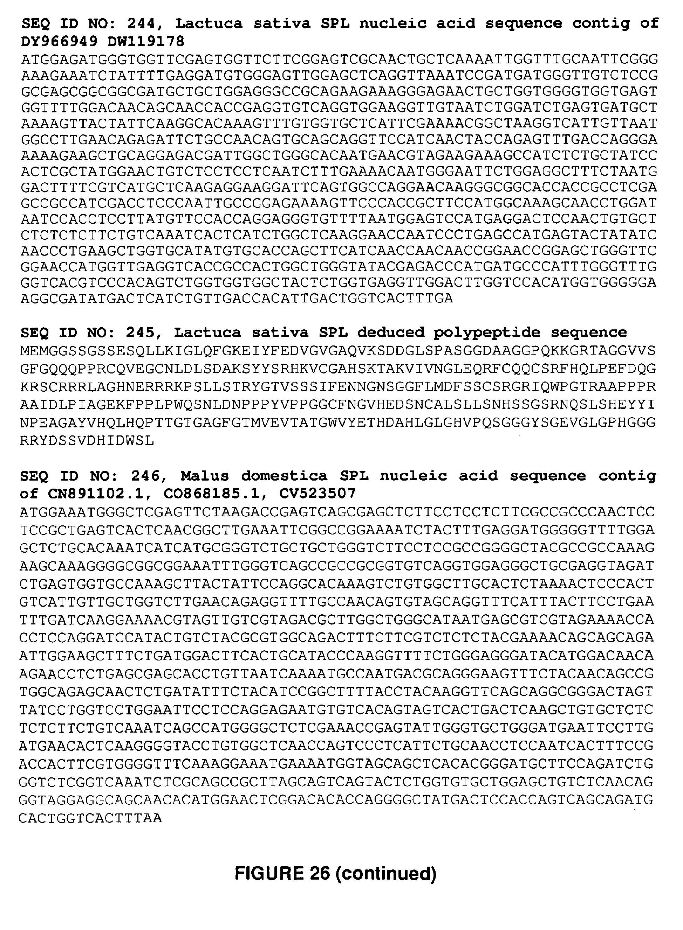

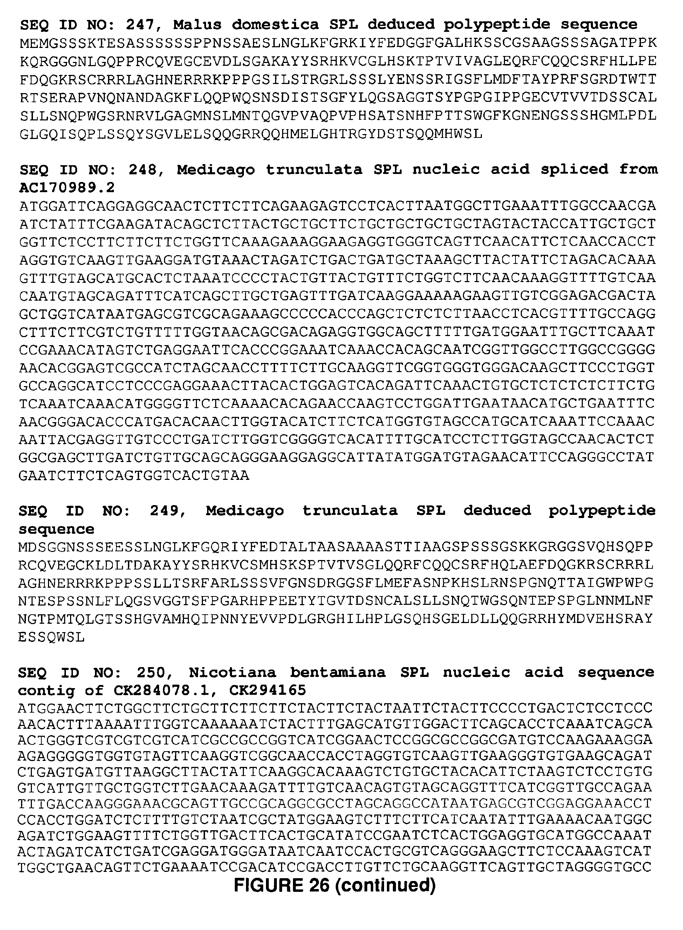

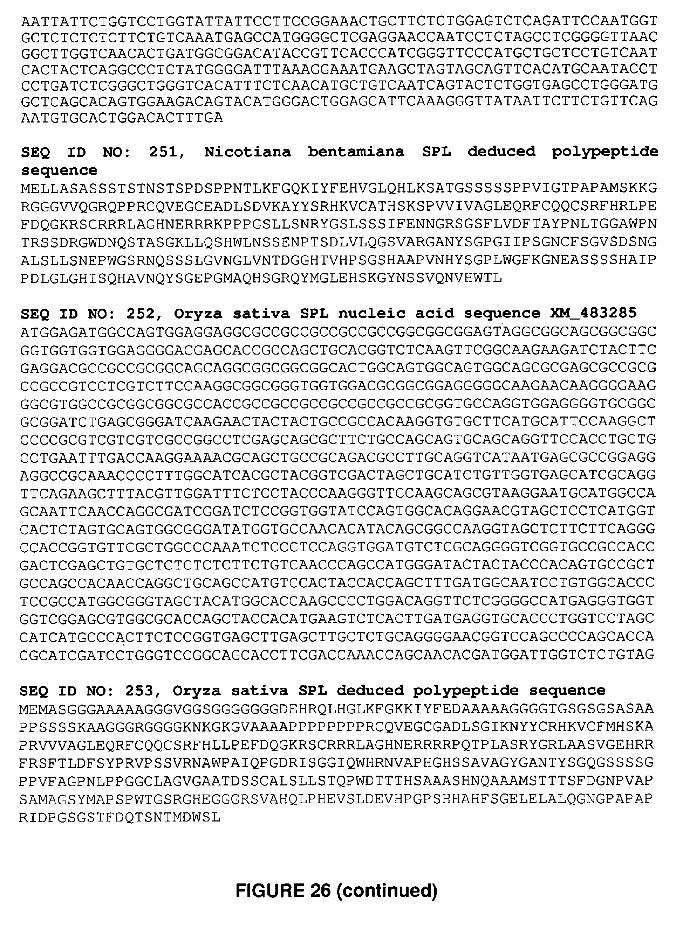

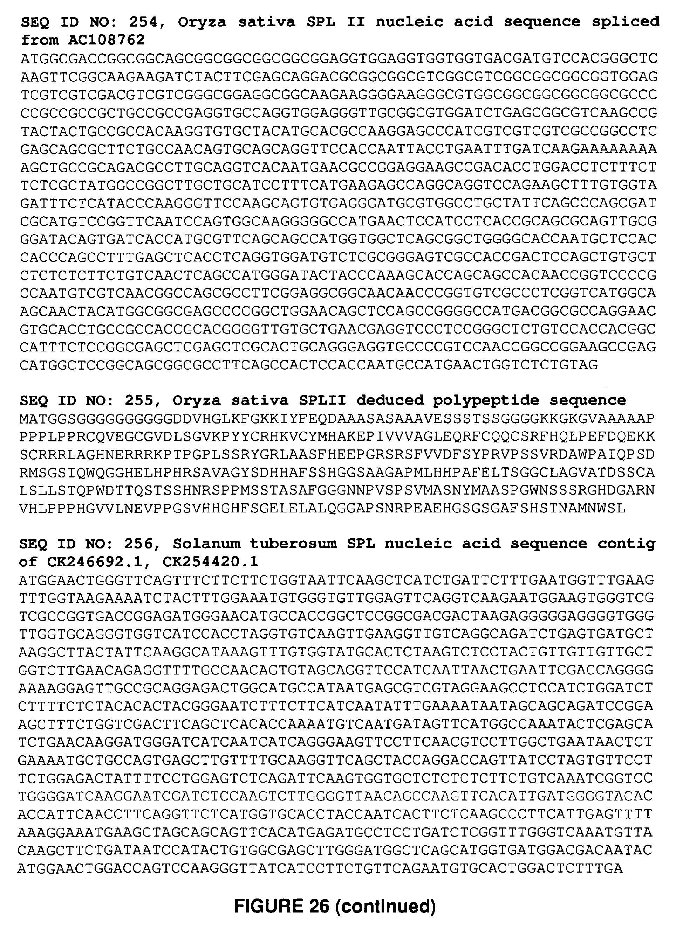

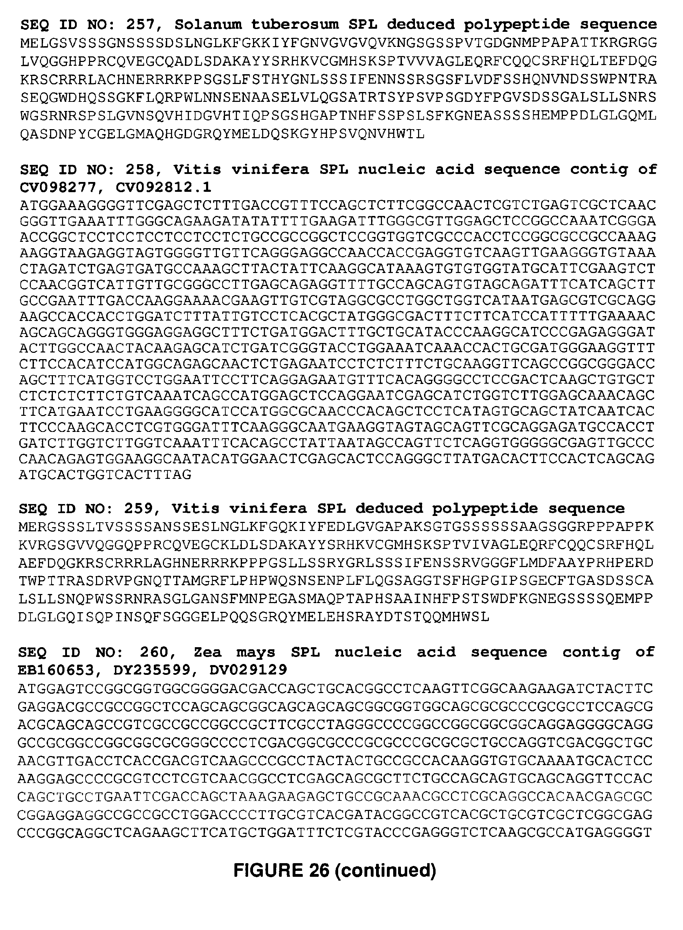

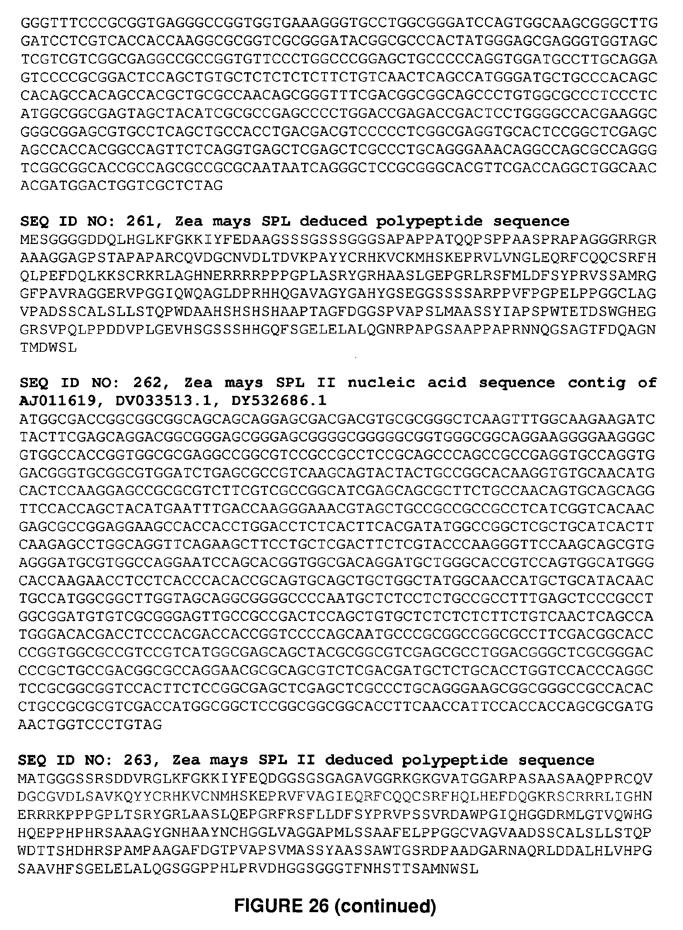

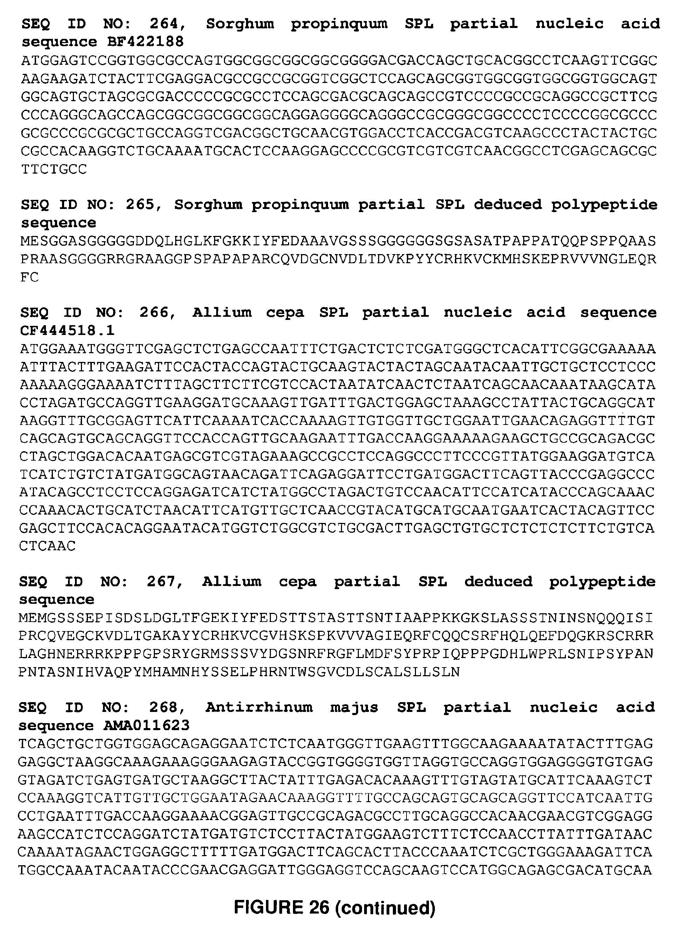

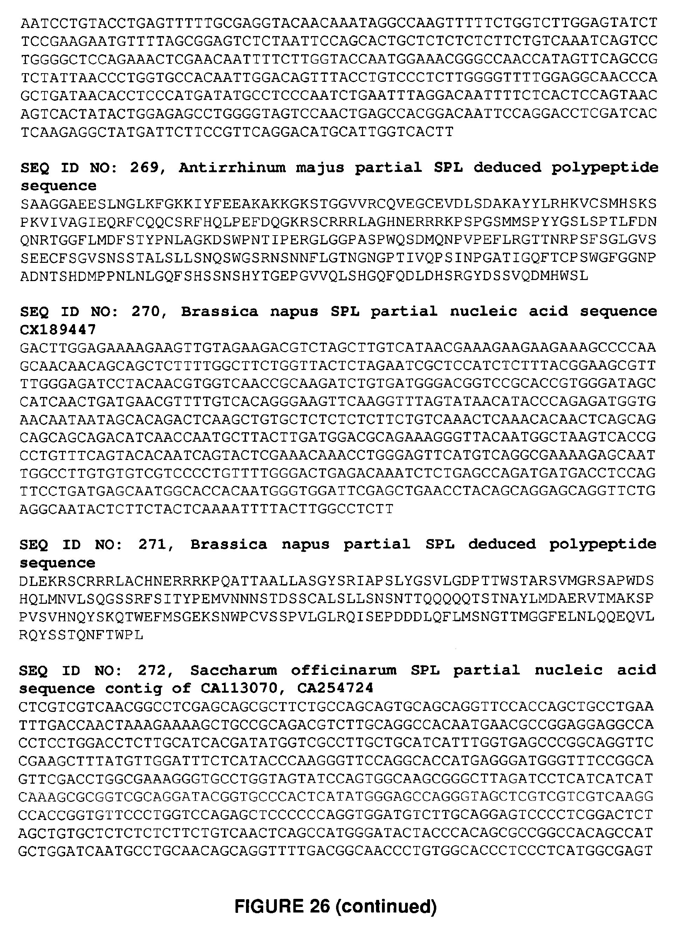

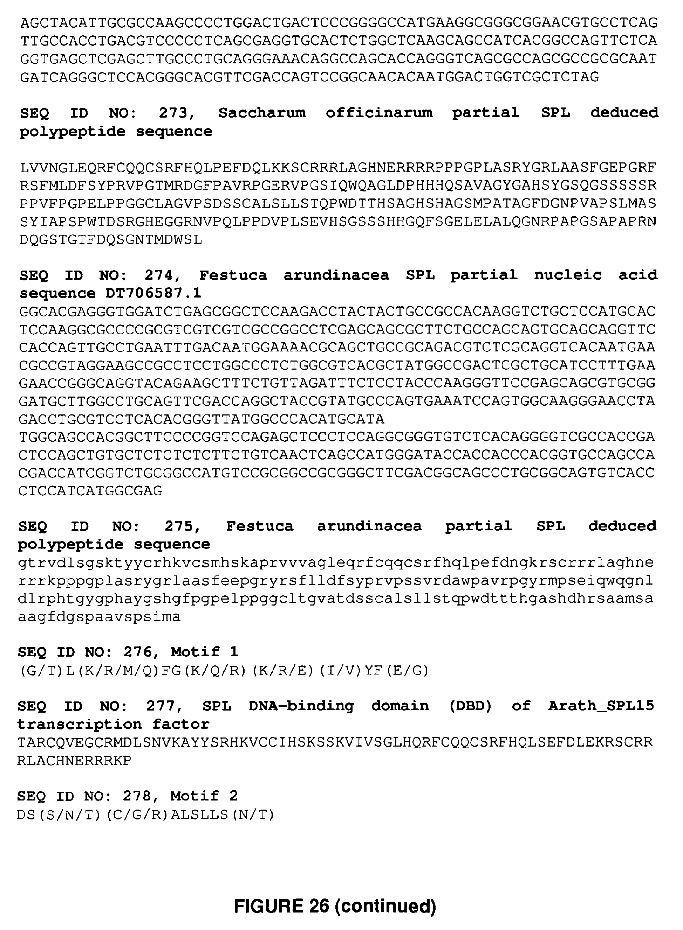

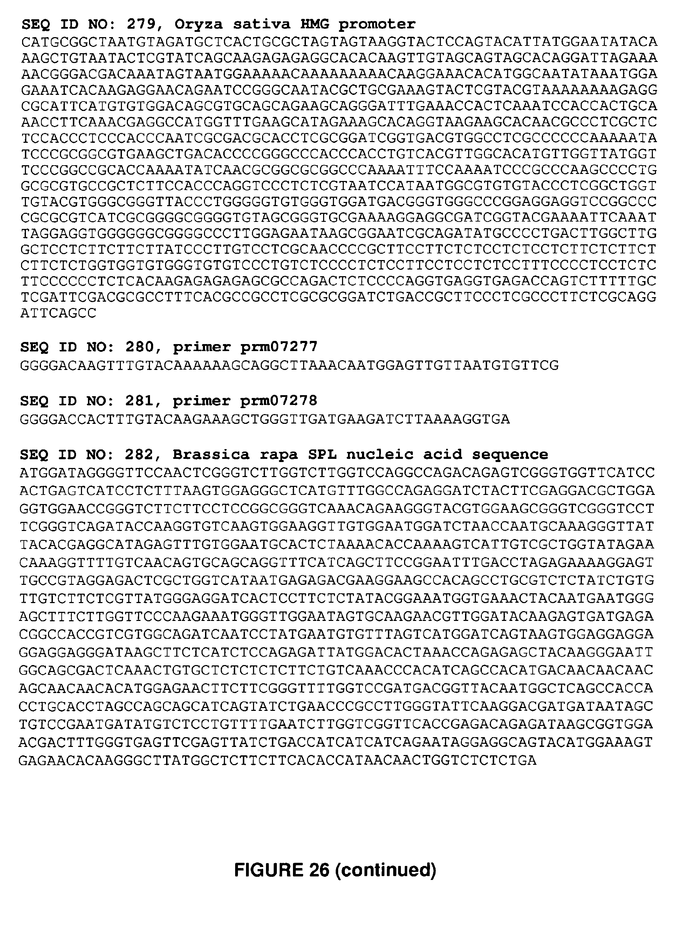

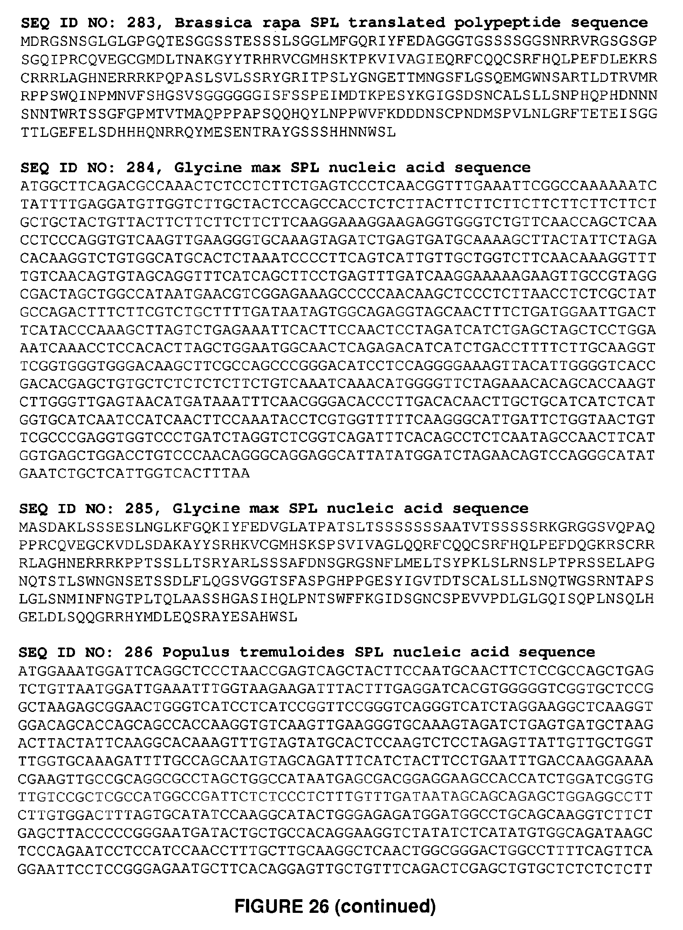

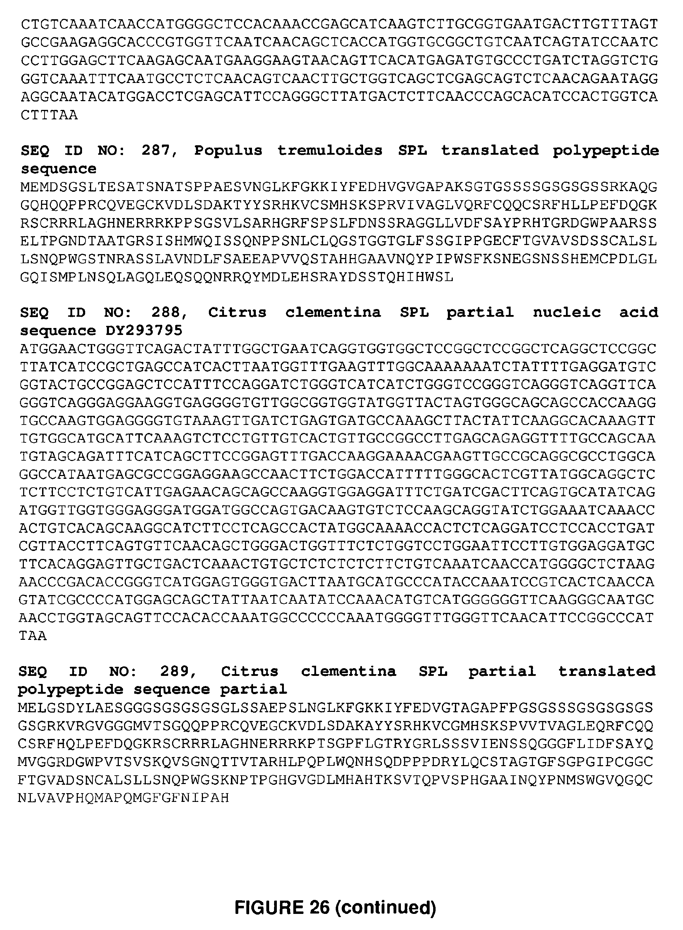

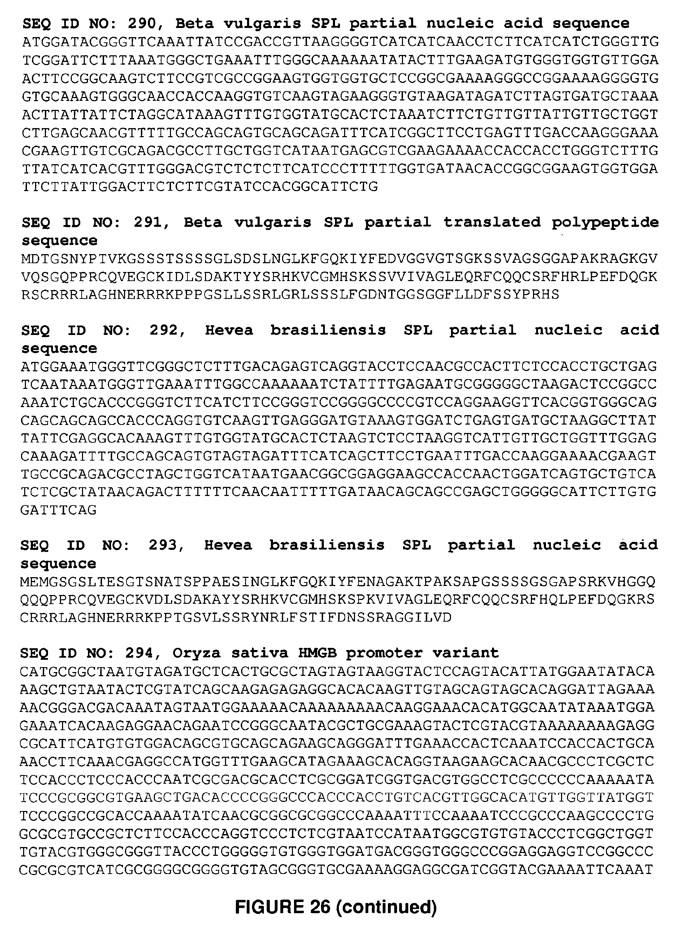

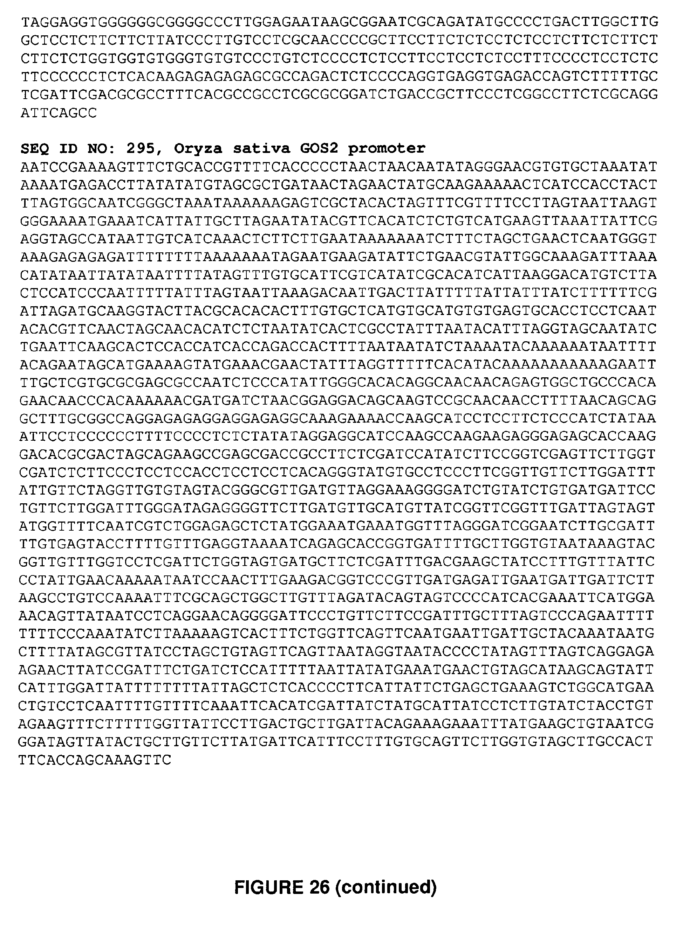

After the LR recombination step, the resulting expression vector pHMGB::SPL15 (Figure 26), or pGOS2::SPL15, was transformed into *Agrobacterium* strain LBA4044 and subsequently to *Oryza sativa* plants.

### Example 45: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 ― 10 mm in length, they are cut and transferred to shoot elongation medium (MSBAP-0.5, containing 0.5 mg/l BAP). Shoots of about 2 cm in length are transferred to the rooting medium (MSO) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839―847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839―847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Example 46: Evaluation procedure

### 46.1 Evaluation setup

Approximately 35 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

Five T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

### 46.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

### Example 47: Evaluation results

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass.

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand kernel weight (TKW) is extrapolated from the number of filled seeds counted and their total weight. The harvest index (Hl) in the present invention is defined as the ratio between the total seed yield and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

As presented in Tables Q to W, the aboveground biomass, the number of flowers per panicle, the seed yield, the total number of seeds, the number of filled seeds, the thousand kernel weight (TKW) and harvest index are increased in the transgenic plants with increased expression a nucleic acid encoding a SPL15 transcription factor polypeptide, compared to suitable control plants. Results from the T1 and the T2 generations are shown.

Table Q shows the number of transgenic events with an increase in aboveground biomass, the percentage of this increase, as well as the statistical relevance of this increase according to the F-test.

**Table Q: Number of transgenic events with an increase in aboveground biomass, the percentage of the increase, and P value of the F-test in T1 and T2 generation of transgenic rice with increased expression of a nucleic acid encoding an SPL15 transcription factor polypeptide.**

| **Aboveground biomass** | | | |
|---|---|---|---|
| | **Number of events showing an increase** | **% Difference** | **P value of F test** |
| T1 generation | 5 out of 7 | 5 | 0.0773 |
| T2 generation | 4 out of 5 | 7 | 0.0172 |

Table R shows the number of transgenic events with an increase in the total number of flowers per panicle, the percentage of this increase, as well as the statistical relevance of this increase according to the F-test.

**Table R: Number of transgenic events with an increase in flowers per panicle, the percentage of the increase, and P value of the F-test in T1 and T2 generation of transgenic rice with increased expression of a nucleic acid encoding an SPL15 transcription factor polypeptide.**

| **Flowers per panicle** | | | |
|---|---|---|---|
| | **Number of events showing an increase** | **% Difference** | **P value of F test** |
| T1 generation | 6 out of 7 | 4 | 0.0447 |
| T2 generation | 4 out of 5 | 10 | 0.0013 |

Table S shows the number of transgenic events with an increase in total seed yield (total seed weight), the percentage of this increase, as well as the statistical relevance of this increase according to the F-test.

**Table S: Number of transgenic events with an increase in seed yield, the percentage of the increase, and P value of the F-test in T1 and T2 generation of transgenic rice with increased expression of a nucleic acid encoding an SPL15 transcription factor polypeptide.**

| **Seed yield** | | | |
|---|---|---|---|
| | **Number of events showing an increase** | **% Difference** | **P value of F test** |
| T1 generation | 7 out of 7 | 15 | 0.0019 |
| T2 generation | 5 out of 5 | 21 | 0.0001 |

Table T shows the number of transgenic events with an increase in the total number of seeds, the percentage of this increase, as well as the statistical relevance of this increase according to the F-test.

**Table T: Number of transgenic events with an increase in total number of seeds, the percentage of the increase, and P value of the F-test in T1 and T2 generation of transgenic rice with increased expression of a nucleic acid encoding an SPL15 transcription factor polypeptide.**

| **Total number of seeds** | | | |
|---|---|---|---|
| | **Number of events showing an increase** | **% Difference** | **P value of F test** |
| T1 generation | 6 out of 7 | 6 | 0.07 |
| T2 generation | 4 out of 5 | 13 | 0.0023 |

Table U shows the number of transgenic events with an increase in the number of filled seeds, the percentage of this increase, as well as the statistical relevance of this increase according to the F-test.

**Table U: Number of transgenic events with an increase in number of filled seeds, the percentage of the increase, and P value of the F-test in T1 and T2 generation of transgenic rice with increased expression of a nucleic acid encoding an SPL15 transcription factor polypeptide.**

| **Number of filled seeds** | | | |
|---|---|---|---|
| | **Number of events showing an increase** | **% Difference** | **P value of F test** |
| T1 generation | 6 out of 7 | 14 | 0.0031 |
| T2 generation | 4 out of 5 | 19 | 0.0003 |

Table V shows the number of transgenic events with an increase in the harvest index, the percentage of this increase, as well as the statistical relevance of this increase according to the F-test.

**Table V Number of transgenic events with an increase in harvest index, the percentage of the increase, and P value of the F-test in T1 and T2 generation of transgenic rice with increased expression of a nucleic acid encoding an SPL15 transcription factor polypeptide.**

| **Harvest index** | | | |
|---|---|---|---|
| | **Number of events showing an increase** | **% Difference** | **P value of F test** |
| T1 generation | 7 out of 7 | 12 | 0.0003 |
| T2 generation | 4 out of 5 | 15 | 0.0001 |

Table W shows the number of transgenic events with an increase in the thousand kernel weight (TKW), the percentage of this increase, as well as the statistical relevance of this increase according to the F-test.

**Table W: Number of transgenic events with an increase in thousand kernel weight (TKW), the percentage of the increase, and P value of the F-test in T1 and T2 generation of transgenic rice with increased expression of a nucleic acid encoding an SPL15 transcription factor polypeptide.**

| **Thousand kernel weight** | | | |
|---|---|---|---|
| | **Number of events showing an increase** | **% Difference** | **P value of F test** |
| T1 generation | 4 out of 7 | 2 | 0.0041 |
| T2 generation | 4 out of 5 | 1 | 0.0259 |

### Example 48: Results of the phenotypic evaluation of the transgenic plants expressing SEQ ID NO: 234 under the control of a constitutive promoter

The results of the evaluation of transgenic rice plants expressing the nucleic acid sequence useful in performing the methods of the invention, under the control of the constitutive GOS2 promoter, are presented in Table X. The percentage difference between the transgenics and the corresponding nullizygotes is also shown.

The transgenic plants expressing the nucleic acid sequence useful in performing the methods of the invention, have increased early vigour and increased TKW compared to the control plants (in this case, the nullizygotes).

**Table X: Results of the evaluation of T1 generation transgenic rice plants expressing the nucleic acid sequence useful in performing the methods of the invention, under the control of a strong constitutive GOS2 promoter.**

| **Trait** | **% Increase of the best events in T1 generation** |
|---|---|
| Increased early vigour | 17% |
| Total seed yield (per plant) | 18% |
| Total number of filled seeds | 19% |
| Increased seed fill rate | 10% |
| Increased harvest index | 15% |

## Claims

1. Method for increasing plant yield and/or increasing abiotic stress resistance relative to suitable control plants, comprising increasing expression in a plant of a nucleic acid encoding a PLT transcription factor polypeptide, and optionally selecting for plants having increased yield and/or increased abiotic stress resistance.

2. Method according to claim 1, wherein said increased abiotic stress resistance is increased nutrient uptake efficiency, relative to control plants.

3. Method according to claim 1 or 2, wherein said increased expression is effected by introducing and expressing in a plant, plant part or plant cell a nucleic acid encoding a PLT transcription factor polypeptide.

4. Method according to claim 3, wherein said nucleic acid encoding a PLT transcription factor is any one of the nucleic acid SEQ ID NOs given in Table I or a portion thereof or a sequence capable of hybridising with any one of the nucleic acids SEQ ID NOs given in Table I; and/or wherein said nucleic acid encoding a PLT transcription factor encodes an orthologue or paralogue of any of the nucleic acid SEQ ID NOs given in Table I.

5. Method according to any of claims 1 to 4, wherein said increased yield is increased seed yield, preferably wherein said seed yield is selected from one or more of: a) increased seed biomass; b) increased number of flowers per plant; c) increased number of (filled) seeds; d) increased seed filling rate; e) increased harvest index; f) increased Thousand Kernel Weight (TKW); g) increased seed area; h) increased seed length; and i) increased seed width.

6. Method according to any one of claims 3 to 5, wherein said nucleic acid is operably linked to a constitutive promoter, preferably a GOS2 promoter.

7. Method according to any one of claims 1 to 6, wherein said nucleic acid encoding a PLT transcription factor polypeptide is of plant origin, preferably from a dicotyledonous plant, further preferably from the Brassicaceae family, more preferably from the genus *Arabidopsis,* most preferably from *Arabidopsis thaliana.*

8. Plant or part thereof, including seeds, obtainable by a method according to any one of claims 1 to 7, wherein said plant or part thereof comprises a nucleic acid encoding a PLT transcription factor polypeptide, which nucleic acid is operably linked to a constitutive promoter.

9. Construct comprising:
(i) a nucleic acid encoding a PLT transcription factor polypeptide;
(ii) one or more control sequences, capable of driving expression of the nucleic acid sequence of (i); and optionally
(iii) a transcription termination sequence.

10. Construct according to claim 9, wherein said constitutive promoter is a GOS2 promoter.

11. Use of a construct according to claim 9 or 10 for making plants having increased yield, particularly increased seed yield and/or increased abiotic stress resistance, relative to control plants; or use of a nucleic acid encoding a PLT transcription factor polypeptide or use of a PLT transcription factor polypeptide for increasing abiotic stress resistance and/or for improving plant yield, especially seed yield, relative to control plants.

12. Plant, plant part or plant cell transformed with a construct according to claim 9 or 10.

13. Method for the production of a transgenic plant having increased yield and/or increased abiotic stress resistance, which method comprises:
(i) introducing and expressing a nucleic acid encoding a PLT transcription factor polypeptide in a plant cell; and
(ii) cultivating the plant cell under conditions promoting plant growth and development.

14. Transgenic plant having increased yield and/or increased abiotic stress resistance relative to control plants, resulting from increased expression of a nucleic acid encoding a PLT transcription factor polypeptide, or plant cell derived from said transgenic plant.

15. Transgenic plant according to claim 8, 12 or 14, wherein said plant is a crop plant or a monocot or a cereal, such as rice, maize, wheat, barley, millet, rye, triticale, sorghum and oats, or a transgenic plant cell derived from said transgenic plant; and/or harvestable parts of said plant, wherein said harvestable parts are preferably seeds; and/or products derived from said plant or from said harvestable parts.
